# EUROPEAN PATENT APPLICATION

(11) **EP 1 698 626 A1**
(43) Date of publication of application: **06.09.2006**
(21) Application number: 04807937.0
(22) Date of filing: 27.12.2004
(51) Int. Cl.: C07D 401/04, C07D 401/14, C07D 405/14, C07D 417/14, A61K 31/4439, A61K 31/444, A61K 31/497, A61K 31/501, A61P 7/02, A61P 9/10

(54) **AMIDOPYRAZOLE DERIVATIVE**

(30) Priority: 26.12.2003 JP 2003434726; 20.01.2004 JP 2004012154; 04.11.2004 JP 2004321117
(71) Applicant: DAIICHI PHARMACEUTICAL CO., LTD., Chuo-ku, Tokyo 103-8234 (JP)
(72) Inventor: KANAYA, Naoaki, Daiichi Pharmaceutical Co., Ltd, Edogawa-ku, Tokyo 1348630 (JP); ISHIYAMA, Takashi, Daiichi Pharmaceutical Co., Ltd, Edogawa-ku, Tokyo 1348630 (JP); MUTO, Ryo, c/o Daiichi Pharmaceutical Co., Ltd., Edogawa-ku, Tokyo 1348630 (JP); OCHIAI, Yuichi, Daiichi Pharmaceutical Co., Ltd., Edo gawa-ku, Tokyo 1348630 (JP); WATANABE, Toshiyuki, Daiichi Pharmaceutical Co Ltd, Edogawa-ku, Tokyo 1348630 (JP); SHIMA Noriko, c/o Iwakura Hospital, Tokyo 133-0056 (JP)
(74) Representative: Hartz, Nikolai
(86) International application number: PCT/JP2004/019582
(87) International publication number: WO 2005/063737

(57) **Abstract**

A platelet coagulation inhibitor which inhibits neither COX-1 nor COX-2 is provided. The inhibitor is a compound represented by general formula (I): 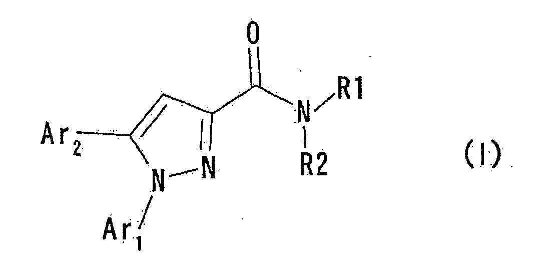 wherein Ar₁ and Ar₂ independently represent a 5- or 6-membered aromatic heterocyclic group optionally substituted with 1 to 3 substituents, or a phenyl group optionally substituted with 1 to 3 substituents; R1 represents a lower acyl group, carboxyl group, a lower alkoxycarbonyl group, a lower alkoxy group, a lower alkyl group optionally substituted with 1 or 2 substituents, a carbamoyl group optionally substituted with 1 or 2 substituents, an oxamoyl group optionally substituted with 1 or 2 substituents, an amino group optionally substituted with 1 or 2 substituents, a 4- to 7-membered alicyclic heterocyclic group optionally substituted with 1 or 2 substituents, a phenyl group optionally substituted with 1 to 3 substituents, or a 5- or 6-membered aromatic heterocyclic group optionally substituted with 1 to 3 substituents; and R2 represents hydrogen atom, a halogeno group, or the like.

## Description

### TECHNICAL FIELD

This invention relates to a pyrazole derivative which has platelet coagulation-inhibiting action.

### BACKGROUND ART

Platelets play an important role in deterring hemorrhage by way of coagulating and forming thrombus once the blood vessel is damaged. Platelets, however, are prone to aggregate and trigger thrombus and embolus when vascular endothelium is injured or the blood vessel is narrowed as in the case of arteriosclerosis, and thus are known to be responsible for the occurrence of ischemic diseases such as myocardial infarction, angina pectoris, ischemic cerebrovascular disorder and peripheral vascular disease. For such reasons, platelet coagulation inhibitors are administered for prevention and treatment of such ischemic diseases. Aspirin, among other things, has been used as a platelet coagulation inhibitor, and the effects of aspirin have been demonstrated by APT (Antiplatelet Trialists' Collaboration) in which clinical test results obtained by administering aspirin to 1, 000, 000 patients had been subjected to metaanalysis (See Non-patent Document 1). Aspirin, however, is known to cause side effects such as hemorrhage in gastrointestine and the like, namely, the so-called "aspirin-induced ulcer", and the side effect is not dose-dependent and occurs at a rate of about 1 per 100 patients (See Non-patent Document 2).

The inhibitory effect of aspirin on platelet coagulation is known to be based on the activity to inhibit cyclooxygenase. Cyclooxygenases include cyclooxygenase-1 (COX-1) and cyclooxygenase-2 (COX-2), and aspirin specifically inhibits COX-1 at a low dose resulting in the platelet coagulation. The inhibition of COX-1 also causes the aspirin-induced ulcer (See Non-patent Documents 3 and 4). In addition, nonsteroidal antiinflammatory drugs are known to exhibit antiinflammatory action by selectively inhibiting COX-2.

As described above, despite usefulness of the aspirin as a platelet coagulation inhibitor, it causes a gastrointestinal dysfunction as its side effect due to the COX-1-inhibiting action which is the action mechanism of inhibition of platelet coagulation, and there is a strong demand for new platelet coagulation inhibitors with no COX-1-inhibiting activity.

In the meanwhile, as pyrazole derivatives having antithrombotic activity have been known compound (A) (see Patent Document 1 and Non-patent Document 5) and compound (B) (see Patent Document 2).

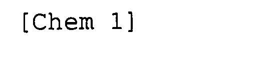

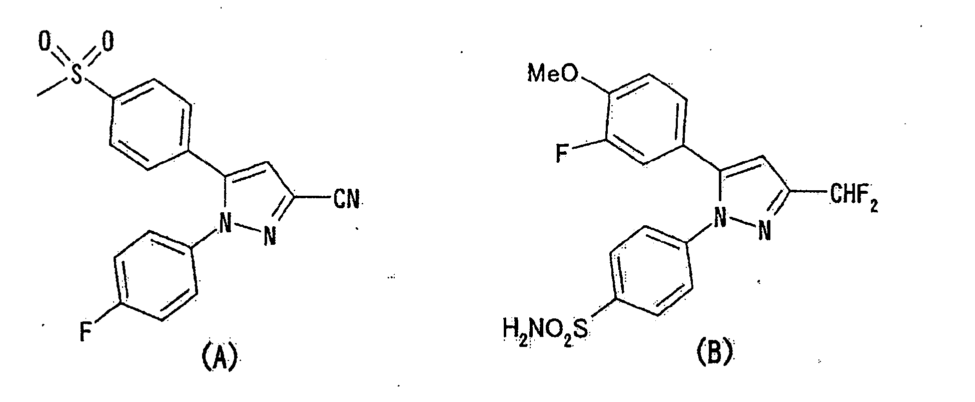

[Patent Document 1] Japanese Patent No. 2586713
[Patent Document 2] WO97-29774
[Non-patent Document 1] BMJ, vol.308, pages 81-106, 1994
[Non-patent Document 2] BMJ, vol.321, pages 1183-1187, 2000
[Non-patent Document 3] Neurology, vo1.57, Suppl.2, pages S5-S7, 2001
[Non-patent Document 4] Drugs Today, vol.35, pages 251-265, 1999
[Non-patent Document 5] Chem. Pharm. Bull., vol.45, pages 987-995, 1997

### [DISCLOSURE OF THE INVENTION]

### [PROBLEM TO BE SOLVED BY THE INVENTION]

Compound (A), however, exhibits IC₅₀ value of 5.3x10⁻⁶M against collagen-induced platelet coagulation with even stronger inhibitory activity against COX-2 (IC₅₀, 2.4x10⁻⁷M). The situation is similar for Compound (B). Theplatelet coagulation inhibitoryactivityof compound (B) is not so potent compared with its inhibitory activity against COX-2. As described above, inhibition of COX-2 may lead to an antiinflammatory action, and the inhibition of COX-2 is not necessarily favorable as a platelet coagulation inhibitor. In view of the situation as described above, an object of the present invention is to provide a strong inhibitor for platelet coagulation which does not inhibit COX-1 nor COX-2.

### [MEANS TO SOLVE THE PROBLEM]

The present inventors have made an extensive study in search of such platelet coagulation inhibitor, and found that a pyrazole derivative represented by the following general formula (I) exhibits strong platelet coagulation inhibitory activity without inhibiting COX-1 and COX-2 to complete the invention.
The present invention provides a compound represented by general formula (I):

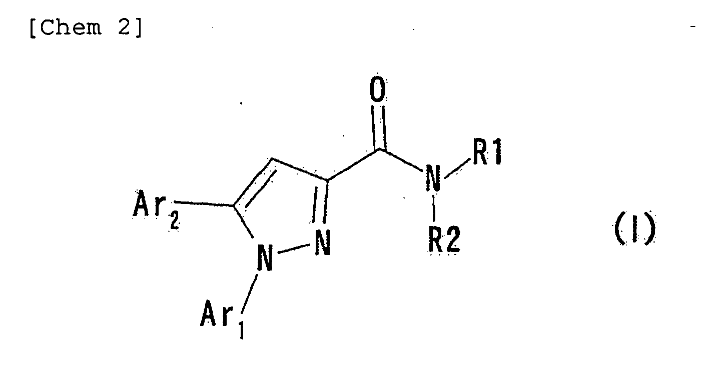

wherein Ar₁ and Ar₂ independently represent a 5- or 6-membered aromatic heterocyclic group optionally substituted with 1 to 3 substituents, or a phenyl group optionally substituted with 1 to 3 substituents, with the proviso that, when Ar₁ is a 5- or 6-membered aromatic heterocyclic group substituted with 1 to 3 substituents, Ar₂ is a 5- or 6-membered aromatic heterocyclic group optionally substituted with 1 to 3 substituents, and when Ar₁ is an unsubstituted 5- or 6-membered aromatic heterocyclic group, Ar₂ is a 5- or 6-membered aromatic heterocyclic group substituted with 1 to 3 substituents or a phenyl group substituted with a carbamoyl group having 1 or 2 substituents or with a lower alkyl group having 1 or 2 substituents, and when Ar₁ is a phenyl group optionally substituted with 1 to 3 substituents, Ar₂ is a 5- or 6-membered aromatic heterocyclic group substituted with 1 to 3 substituents;
R1 represents a lower acyl group, a lower alkoxycarbonyl group, a lower alkoxy group, a lower alkyl group optionally substituted with 1 or 2 substituents, a carbamoyl group optionally substituted with 1 or 2 substituents, an oxamoyl group optionally substituted with 1 or 2 substituents, an amino group optionally substituted with 1 or 2 substituents, a 4- to 7-membered alicyclic heterocyclic group optionally substituted with 1 or 2 substituents, a phenyl group optionally substituted with 1 to 3 substituents, or a 5- or 6-membered aromatic heterocyclic group optionally substituted with 1 to 3 substituents; and
R2 represents a hydrogen atom or a lower alkyl group optionally substituted with 1 or 2 substituents; or a salt thereof, or a solvate thereof.

This invention also provides a drug comprising the compound represented by the general formula (I), a salt thereof, or a solvate thereof.

This invention also provides a prophylactic and/or therapeutic composition for an ischemic disease comprising the compound represented by the general formula (I), a salt thereof, or a solvate thereof.

### [EFFECTS OF THE INVENTION]

The compound (I) of the present invention, the salt or the solvate thereof, or the solvate of such salt potently inhibit platelet coagulation, and hence, accordingly, also inhibits thrombogenesis without inhibiting COX-1 and COX-2. Therefore, it is useful as a prophylactic and/or therapeutic agent for ischemic diseases caused by thrombus or embolus such as myocardial infarction, angina pectoris (chronic stable angina, unstable angina, etc.), ischemic cerebrovascular disorder (transient ischemic attack (TIA), cerebral infarction, etc.), peripheral vascular disease, embolism after replacement with an artificial vessel, thrombotic embolism after coronary artery intervention (coronary artery bypass grafting(CAGB), percutaneous transluminal coronary angioplasty (PTCA), stent placement, etc.), diabetic retinopathy and nephropathy, and embolism after replacement with an artificial heart valve, and is also useful as a prophylactic and/or therapeutic agent for thrombus and embolus associated with vascular operation, blood extracorporeal circulation, and the like.

### [BEST MODE FOR CARRYING OUT THE INVENTION]

Next, the substituents in the general formula (I) as described above are described.

The aromatic heterocyclic groups represented by Ar₁ and Ar₂ are independently a 5- or 6-membered aromatic heterocyclic group, and examples of such groups include pyridyl group, pyridazinyl group, pyrimidinyl group, pyrazinyl group, furyl group, thienyl group, pyrrolyl group, pyrazolyl group, imidazolyl group, triazolyl group, oxazolyl group, isoxazolyl group, thiazolyl group, pyrazolyl group, and thiadiazolyl group.
Examples of substituents in the aromatic heterocyclic groups and the phenyl group represented by Ar₁ and Ar₂ are (1) a lower alkyl group optionally substituted with 1 or 2 substituents, (2) a halogeno group, (3) hydroxyl group, (4) cyano group, (5) an optionally substituted lower alkoxy group, (6) an aralkyloxy group, (7) a lower alkylthio group, (8) a lower alkoxycarbonyl group, (9) carboxyl group, (10) a lower alkylsulfonyl group, (11) an amino group optionally substituted with 1 or 2 substituents, (12) a carbamoyl group optionally substituted with 1 or 2 substituents, (13) an aminosulfonyl group optionally substituted with 1 or 2 lower alkyl groups, (14) a 4- to 7-membered alicyclic heterocyclic group optionally substituted with 1 or 2 substituents, (15) an optionally substituted lower alkenyl group, (16) an optionally substituted lower alkynyl group, and (17) an optionally substituted lower alkanoyl group, as described below.

(1) The lower alkyl group optionally substituted with 1 or 2' substituents which is the substituent of the aromatic heterocyclic group or the phenyl group means a straight, branched, or cyclic C₁₋₆ alkyl group which is optionally substituted with one group or the same or different two groups selected from the group consisting of (a) hydroxyl group, (b) halogeno groups, (c) straight, branched, or cyclic C₁₋₆ alkoxy groups, (d) amino groups optionally substituted with 1 or 2 substituents, (e) carbamoyl groups optionally substituted with C₁₋₆ alkyl group, (f) alkoxycarbonyl groups containing 2 to 7 carbon atoms in total, (g) carboxyl group, and (h) alicyclic-carbonyl groups. The cyclic alkyl group contains 3 to 6 carbon atoms.

Examples of the halogeno groups (b) include fluoro group, chloro group, and bromo group. Examples of the C₁₋₆ alkoxy group (c) include methoxy group, ethoxy group, propoxy group, isopropoxy group, butoxy group, isobutoxy group, pentoxy group, and cyclopentyloxy group.

Examples of the amino groups optionally substituted with 1 or 2 substituents (d) include, in addition to unsubstituted amino group, aminos group substituted with 1 or 2 substituents selected from (i) straight, branched, or cyclicC₁₋₆ alkyl groups, (ii) C₂₋₇ alkanoyl groups, (iii)arylcarbonylgroups, (iv) aliphatic heterocyclic-carbonyl groups, (v) alkoxycarbonyl groups containing 2 to 7 carbon atoms in total, (vi) aryloxycarbonyl groups, (vii) carbamoyls groups optionally substituted with a straight, branched or cyclic C₁₋₆ alkyl group or an alicyclic heterocyclic group, (viii) hydroxyl group, (ix) straight, branched or cyclic C₁₋₆ alkoxy groups, (x) straight, branched or cyclic C₁₋₆ alkylsulfonyl groups, and (xi) arylsulfonyl groups.

Examples of the C₁₋₆ alkyl group (i) include methyl group, ethyl group, propyl group, isopropyl group, primary, secondary, or tertiary butyl group, pentyl group, hexyl group, cyclopropyl group, cyclobutyl group, cyclopentyl group, cyclohexyl group, cyclopropylmethyl group, and cyclopentylmethyl group. Examples of the C₂₋₇ alkanoyl group (ii) include acetyl group, propionyl group, butylyl group, butanoyl group, and pentanoyl group. Examples of the arylcarbonyl group (iii) include arylcarbonyl groups containing 7 to 15 carbon atoms in total such as benzoyl group and naphtylcarbonyl group. Examples of the aliphatic heterocyclic-carbonyl group (iv) include 4- to 7-membered aliphatic heterocyclic-carbonyl groups such as azetidinecarbonyl group, pyrrolidinecarbonyl group, piperidinecarbonyl group, piperazinecarbonyl group, hexahydropyridazinecarbonyl group, tetrahydropyrimidinecarbonyl group, pyrazolidinecarbonyl group, imidazolidinecarbonyl group, homopiperazinecarbonyl group, morpholinecarbonyl group, and thiomorpholinecarbonyl group.

Examples of the alkoxycarbonyl group containing 2 to 7 carbon atoms in total (v) include methoxycarbonyl group, ethoxycarbonyl group, propoxycarbonyl group, and butoxycarbonyl group. Examples of the aryloxycarbonyl group (vi) include C₆₋₁₄ aryloxycarbonyl groups such as phenyloxycarbonyl group and naphthyloxycarbonyl group.

Examples of the carbamoyl group optionally substituted with an alkyl group or an alicyclic heterocyclic group include, in addition to unsubstituted carbamoyl group, methylcarbamoyl group, ethylcarbamoyl group, propylcarbamoyl group, isopropylcarbamoyl group, primary, secondary, or tertiary butylcarbamoyl group, pentylcarbamoyl group, hexylcarbamoyl group, cyclopropylcarbamoyl group, cyclobutylcarbamoyl group, cyclopentylcarbamoyl group, cyclohexylcarbamoyl group, cyclopropylmethylcarbamoyl group, cyclopentylmethylcarbamoyl group, dimethylcarbamoyl group, methylethylcarbamoyl group, diethylcarbamoyl group, methylpropylcarbamoyl group, methylisopropylcarbamoyl group, methyl cyclopropylcarbamoyl group, methylcyclopropylmethylcarbamoyl group, as well as 4- to 7-membered alicyclic heterocyclic carbamoyl groups. such as azetidine carbamoyl group, pyrrolidine carbamoyl group, piperidine carbamoyl group, piperazine carbamoyl group, hexahydropyridazine carbamoyl group, tetrahydropyrimidine carbamoyl group, pyrazolidine carbamoyl group, imidazolidine carbamoyl group, homopiperazine carbamoyl group, morpholine carbamoyl group, and thiomorpholine carbamoyl group. Examples of the alkoxy group (ix) include methoxy group, ethoxy group, propoxy group, isopropoxy group, butoxy group, isobutoxy group, pentoxy group, and cyclopentyloxy group. Examples of the alkylsulfonyl group (x) include methylsulfonyl group, ethylsulfonyl group, propylsulfonyl group, isopropylsulfonyl group, butylsulfonyl group, isobutylsulfonyl group, pentylsulfonyl group, and cyclopentylsulfonyl group. Examples of the arylsulfonyl group (xi) include C₆₋₁₄ arylsulfonyl groups such as phenylsulfonyl group, p-toluene sulfonyl group, and naphtylsulfonyl group.

Examples of the alkyl group which is optionally substituted with the groups (i) to (xi) include methyl group, ethyl group, propyl group, isopropyl group, primary, secondary, or tertiary butyl group, pentyl group, hexyl group, cyclopropyl group, cyclobutyl group, cyclopentyl group, cyclohexyl group, cyclopropylmethyl group, and cyclopentylmethyl group.

Examples of substituents (1) include methyl group, ethyl group, propyl group, isopropyl group, primary, secondary, or tertiary butyl group, pentyl group, hexyl group, cyclopropyl group, cyclobutyl group, cyclopentyl group, cyclohexyl group, cyclopropylmethyl group, cyclopentylmethyl group, hydroxymethyl group, 2-hydroxyethyl group, 3-hydroxypropyl group, 2-hydroxypropyl group, 2-fluoroethyl group, 3-fluoropropyl group, 2-fluoropropyl group, 2-fluorocyclopropyl group, 2-chloroethyl group, 3-chloropropyl group, 2-chloropropyl group, trifluoromethyl group, methoxymethyl group, ethoxymethyl group, propoxymethyl group, isopropoxymethyl group, 2-methoxyethyl group, 3-methoxypropyl group, aminomethyl group, 2-aminoethyl group, 1-aminoethyl group, 3-aminopropyl group, 2-aminopropyl group, methylaminomethyl group, 2-(methylamino)ethyl group, 1-(methylamino)ethyl group, 3-(methylamino)propyl group, 2-(methylamino)propyl group, dimethylaminomethyl group, 2-(dimethylamino)ethyl group, 1-(dimethylamino)ethyl group, 3-(dimethylamino)propyl group, 2-(dimethylamino)propyl group, 2-(methylethylamino)ethyl group, 1-(methylethylamino)ethyl group, 1-aminocyclopropyl group, 1-(methylamino)cyclopropyl group, 1-(ethylamino)cyclopropyl group, 1-(dimethylamino)cyclopropyl group, 1-(diethylamino)cyclopropyl group, 1-(methylethylamino)cyclopropyl group, acetylaminomethyl group, 2-acetylaminoethyl group, propionylaminomethyl group, 2-propionylaminoethyl group, benzoylaminomethyl group, 2-(benzoylamino)ethyl group, pyrrolidinecarbonylaminomethyl group, piperidinecarbonylaminomethyl group, 2-(pyrrolidinecarbonylamino)ethyl group, 2-(piperidinecarbonylamino)ethyl group, methoxycarbonylaminomethyl group, ethoxycarbonylaminomethyl group, 2-(methoxycarbonylamino)ethyl group, 2-(ethoxycarbonylamino)ethyl group, phenyloxycarbonylaminomethyl group, 2-(phenyloxycarbonyl)ethyl group, carbamoylaminomethyl group, N-methylcarbamoylaminomethyl group, N-ethylcarbamoylaminomethyl group, 2-(carbamoylamino)ethyl group, 2-(N-methylcarbamoylamino)ethyl group, 2-(N-ethylcarbamoylamino)ethyl group, hydroxyaminomethyl group, 2-(hydroxyamino)ethyl group, methoxyaminomethyl group, 2-(methoxyamino)ethyl group, 2-(ethoxy amino) ethyl group, N-methyl-N-methoxyaminomethyl group, 2-(N-methyl-N-methoxyamino)ethyl group, methylsulfonylaminomethyl group, ethylsulfonylaminomethyl group, 2-(methylsulfonylamino)ethyl group, 2-(ethylsulfonylamino)ethyl group, phenylsulfonylaminomethyl group, p-toluene sulfonylaminomethyl group, 2- (phenylsulfonylamino)ethyl group, 2- (p-toluenesulfonylamino)ethyl group, carbamoylmethyl group, methylcarbamoylmethyl group, 2-carbamoylethyl group, 2-(methylcarbamoyl)ethyl group, methoxycarbonylmethyl group, methoxycarbonylethyl group, carboxymethyl group, carboxyethyl group, pyrrolidinecarbonylmethyl group, and pyrrolidinecarbonylethyl group.

Examples of the halogeno groups (2) include fluoro group, chloro group, and bromo group.

Examples of the optionally substituted lower alkoxy group (5) include straight, branched or cyclic C₁₋₆ alkoxy groups optionally substituted with 1 or 2 substituents selected from carbamoyl group, lower alkoxycarbonyl groups, carboxyl group, and alicyclic heterocyclic carbonyl groups. Examples of the lower alkoxycarbonyl groups are as mentioned above in (v). Examples of the alicyclic heterocyclic carbonyl groups are as mentioned above in (iv). Examples of substituents (5) include methoxy group, ethoxy group, propoxy group, isopropoxy group, butoxy group, isobutoxy group, pentoxy group, cyclopentyloxy group, carbamoylmethoxy group, carbamoylethoxy group, methoxycarbonylmethoxy group, methoxycarbonylethoxy group, ethoxycarbonylmethoxy group, ethoxycarbonylethoxy group, carboxymethoxy group, carboxyethoxy group, pyrrolidinecarbonylmethoxy group, and pyrrolidinecarbonylethoxy group.

The aralkyloxy group (6) is a group consisting of a C₆₋₂₀ aryl group and a straight, branched or cyclic C₁₋₆ alkoxy group, and examples of the aralkyloxy groups include benzyloxy group and phenethyloxy group.
The lower alkylthio group (7) means a straight, branched or cyclic C₁₋₆ alkylthio group, and examples thereof include methylthio group, ethylthio group, propylthio group, isopropylthio group, butylthio group, isobutylthio group, pentylthio group, and cyclopentylthio group.
The lower alkoxycarbonyl group (8) means an alkoxycarbonyl group containing 2 to 7 carbon atoms in total, and examples include methoxycarbonyl group, ethoxycarbonyl group, propoxycarbonyl group, and butoxycarbonyl group.
The lower alkylsulfonyl group (10) means a straight, branched or cyclic C₁₋₆ alkylsulfonyl group, and examples thereof include methanesulfonyl group, ethanesulfonyl group, and trifluoromethanesulfonyl group.

The amino group optionally substituted with 1 or 2 substituents (11) means, in addition to unsubstituted amino group, amino groups substituted with 1 or 2 of the lower alkyl groups as described above, lower alkanoylamino groups, lower alkoxycarbonylamino groups, or ureido group optionally substituted with 1 or 2 of the lower alkyl groups as described above. Examples of the amino group substituted with 1 or 2 of the lower alkyl groups as described above include methylamino group, ethylamino group, propylamino group, isopropylamino group, cyclopropylamino group, butylamino group, isobutylamino group, cyclopentylmethylamino group, dimethylamino group, diethylamino group, dipropylamino group, dibutylamino group, N-methyl-N-ethylamino group, N-ethyl-N-propylamino group, and N-methyl-N-cyclopentylmethylamino group. The lower alkanoylamino group means an amino group substituted with a straight or branched C₂₋₆ alkanoyl group, and examples thereof include acetylamino group and propionylamino group. The lower alkoxycarbonylamino group means an amino group substituted a straight or branched C₂₋₆ lower alkoxycarbonyl group, and examples thereof include methoxycarbonylamino group and ethoxycarbonylamino group. Examples of the ureido group optionally substituted with 1 or 2 of the lower alkyl groups include aminocarbonylamino group, N1-methylaminocarbonylamino group, N1-ethylaminocarbonylamino group, N3-methylaminocarbonylamino group, N1,N1-dimethylaminocarbonylamino group, N1,N3-dimethylaminocarbonylamino group, and N1-methyl-N3-ethylaminocarbonylamino group.

Examples of the carbamoyl group optionally substituted with 1 or 2 substituents (12) include, in addition to unsubstituted carbamoyl group, carbamoyl groups substituted with 1 or 2 of the lower alkyl groups as described above, and examples thereof include methylcarbamoyl group, ethylcarbamoyl group, dimethylcarbamoyl group, and methylethylcarbamoyl group.

The aminosulfonyl group optionally substituted with 1 or 2 substituents (13) means, in addition to unsubstituted aminosulfonyl group, aminosulfonyl group substituted with 1 or 2 of the lower alkyl groups as described above, and examples thereof include methylaminosulfonyl group, ethylaminosulfonyl group, propylaminosulfonyl group, isopropylaminosulfonyl group, primary, secondary, or tertiary butylaminosulfonyl group, cyclopropylaminosulfonyl group, cyclobutylaminosulfonyl group, cyclopentylaminosulfonyl group, cyclohexylaminosulfonyl group, cyclopentylmethylaminosulfonyl group, dimethylaminosulfonyl group, and diethylaminosulfonyl group.

Examples of the 4- to 7-membered aliphatic heterocyclic group in the 4- to 7-membered alicyclic heterocyclic group optionally substituted with 1 or 2 substituents (14) include azetidinyl group, pyrrolidinyl group, piperidinyl group, piperadinyl group, hexahydropyridazinyl group, tetrahydropyrimidinyl group, pyrazolidinyl group, imidazolidinyl group, homopiperadinyl group, morpholinyl group, and thiomorpholinyl group.
These groups maybe substituted with a substituent such as hydroxyl group, oxo group, carboxyl group, sulfo group, cyano group, and nitro group, as well as the halogeno group, the lower alkoxy group, the alkylsulfonyl group, the alkyl group optionally substituted with 1 or 2 substituents, the amino group optionally substituted with 1 or 2 substituents, the carbamoyl group optionally substituted with 1 or 2 substituents, the lower acyl group, or the aminosulfonyl group optionally substituted with 1 or 2 substituents as described above.

The optionally substituted lower alkenyl group (15) includes a straight, branched or cyclic C₂₋₆ alkenyl group optionally substituted with 1 or 2 substituents selected from carbamoyl group, lower alkoxycarbonyl groups, carboxyl group, and alicyclic heterocyclic carbonyl groups. Examples of the lower alkoxycarbonyl groups include those mentioned above in (v). Exemplary alicyclic heterocyclic carbonyl groups are as mentioned above for (iv). Examples of the group (15) include vinyl group, 2-propenyl group, 2-butenyl group, 3-butenyl group, pentenyl group, cyclopentenyl group, carbamoylvinyl group, carbamoylpropenyl group, methoxycarbonylvinyl group, methoxycarbonylpropenyl group, ethoxycarbonylvinyl group, ethoxycarbonylpropenyl group, carboxyvinyl group, carboxypropenyl group, pyrrolidinecarbonylvinyl group, and pyrrolidinecarbonylpropenyl group.

The optionally substituted lower alkynyl group (16) includes a straight, branched or cyclic C₂₋₆ alkynyl group optionally substituted with 1 or 2 substituents selected from carbamoyl group, lower alkoxycarbonyl groups, carboxyl group, and alicyclic heterocyclic carbonyl groups. Examples of the lower alkoxycarbonyl groups include those mentioned above in (v). Examples of the alicyclic heterocyclic carbonyl groups include those mentioned above in (iv). Examples of the group (16) include ethynyl group, 2-propynyl group, carbamoylethynyl group, carbamoylpropynyl group, methoxycarbonylethynyl group, methoxycarbonylpropynyl group, ethoxycarbonylethynyl group, ethoxycarbonylpropynyl group, carboxyethynyl group, carboxypropynyl group, pyrrolidinecarbonylethynyl group, and pyrrolidinecarbonylpropynyl group.

The optionally substituted lower alkanoyl group (17) includes a straight, branched or cyclic C₂₋₇ alkanoyl group optionally substituted with 1 or 2 substituents selected from carbamoyl group, lower alkoxycarbonyl groups, carboxyl group, and alicyclic heterocyclic carbonyl groups. Examples of the lower alkoxycarbonyl groups include those mentioned above in (v). Examples of the alicyclic heterocyclic carbonyl groups include those mentioned above in (iv). Examples of the group (17) include acetyl group, propionyl group, butylyl group, pentanoylgroup, cyclopropylcarbonylgroup, cyclobutylcarbonylgroup, cyclopentylcarbonyl group, carbamoylacetyl group, carbamoylpropionyl group, methoxycarbonylacetyl group, methoxycarbonylpropanoyl group, ethoxycarbonylacetyl group, ethoxycarbonylpropanoyl group, carboxyacetyl group, carboxypropanoyl group, pyrrolidinecarbonylacetyl group, and pyrrolidinecarbonylpropanoyl group.

The substituent of the aromatic heterocyclic group or the phenyl group, Ar₁, is preferably located at para position to the pyrazole ring.

In the compound (I) of the present invention, when Ar₁ is a 5- or 6-membered aromatic heterocyclic group having 1 to 3 substituents, Ar₂ is a 5- or 6-membered aromatic heterocyclic group optionally substituted with 1 to 3 substituents; and when Ar₁ is an unsubstituted 5- or 6-membered aromatic heterocyclic group, Ar₂ is a 5- or 6-membered aromatic heterocyclic group substituted with 1 to 3 substituents, or phenyl group having carbamoyl group substituted with 1 or 2 substituents or having a lower alkyl group substituted with 1 or 2 substituents; and when Ar₁ is phenyl group optionally substituted with 1 to 3 substituents, Ar₂ is a 5- or 6-membered aromatic heterocyclic group substituted with 1 to 3 substituents.

Next, substituents R1 and R2 are described.

The lower acyl group means an acyl group having a straight, branched or cyclic C₁₋₆ alkyl group, and examples thereof include formyl group, acetyl group, propionyl group, primary and secondary butylyl group, pivaloyl group, cyclopropylcarbonyl group, cyclobutylcarbonyl group, cyclopentylcarbonyl group, cyclohexylcarbonyl group, cyclopropylmethylcarbonyl group, cyclobutylmethylcarbonyl group, and cyclopentylmethylcarbonyl group.

The lower alkoxycarbonyl group means an alkoxycarbonyl group having a straight, branched or cyclic C₁₋₆ alkyl group, and examples thereof include methoxycarbonyl group, ethoxycarbonyl group, propoxycarbonyl group, isopropoxycarbonyl group, primary, secondary, or tertiary butoxycarbonyl group, cyclobutyloxycarbonyl group, cyclopentyloxycarbonyl group, cyclohexyloxycarbonyl group, and cyclopentylmethyloxycarbonyl group.

The lower alkoxy group means a straight, branched or cyclic C₁₋₆ alkoxy group, examples of which include methoxy group, ethoxy group, propoxy group, isopropoxy group, butoxy group, isobutoxy group, pentoxy group, and cyclopentyloxy group.

The lower alkyl group optionally substituted with 1 or 2 substituents means a straight, branched or cyclic C₁₋₆ alkyl group which is optionally substituted with one group or the same or different two groups selected from hydroxyl group, halogeno groups, carboxyl group, sulfo group, straight, branched or cyclic C₁₋₃ alkoxy groups, alkoxycarbonyl groups having a straight, branched or cyclic C₁₋₃ alkyl group, amino groups optionally substitutedwith 1 or 2 straight, branched or cyclic C₁₋₃ alkyl groups, 4- to 7-membered aliphatic heterocyclic groups optionally substitutedwith 1 or 2 substituents, carbamoyl groups optionally substituted with 1 or 2 straight, branched or cyclic C₁₋₃ alkyl groups, ureido groups optionally substitutedwith 1 or 2 straight, branched or cyclic C₁₋₃ alkyl groups, phenyl groups optionally substituted with 1 to 3 substituents, 5- or 6-membered aromatic heterocyclic groups optionally substituted with 1 to 3 substituents, and C₃₋₆ cyclic alkyl groups. The cyclic alkyl group contains 3 to 6 carbon atoms.

Examples of such groups include methyl group, ethyl group, propyl group, isopropyl group, primary, secondary, or tertiary butyl group, pentyl group, neopentyl group, hexyl group, cyclopropyl group, cyclobutyl group, cyclopentyl group, cyclohexyl group, bicyclo[2.2.1]pentyl group, cyclopropylmethyl group, cyclopentylmethyl group, hydroxymethyl group, 2-hydroxyethyl group, 3-hydroxypropyl group, 2-hydroxypropyl group, 1-hydroxymethyl cyclopentyl group, 1-hydroxymethyl cyclohexyl group, 2-fluoroethyl group, 3-fluoropropyl group, 2-fluoropropyl group, 2-fluorocyclopropyl group, 3-fluorocyclopentyl group, 4-fluorocyclohexyl group, 4,4-difluorocyclohexyl group, 2-chloroethyl group, 3-chloropropyl group, 2-chloropropyl group, trifluoromethyl group, carboxymethyl group, 2-carboxyethyl group, 3-carboxypropyl group, 2-carboxypropyl group, sulfomethyl group, 2-sulfoethyl group, 1-sulfoethyl group, 3-sulfopropyl group, 2-sulfopropyl group, methoxymethyl group, ethoxymethyl group, propoxymethyl group, isopropoxymethyl group, 2-methoxyethyl group, 3-methoxypropyl group, 3-methoxycyclopropyl group, 3-methoxy cyclopentyl group, 4-methoxycyclohexyl group, methoxycarbonylmethyl group, ethoxycarbonylmethyl group, propoxycarbonylmethyl group, 2-methoxycarbonylethyl group, 2-ethoxycarbonylethyl group, 2-propoxycarbonylethyl group, aminomethyl group, 2-aminoethyl group, 1-aminoethyl group, 3-aminopropyl group, 2-aminopropyl group, methylaminomethyl group, 2-(methylamino)ethyl group, 1-(methylamino)ethyl group, 3-(methylamino)propyl group, 2-(methylamino)propyl group, dimethylaminomethyl group, 2-(dimethylamino)ethyl group, 1-(dimethylamino)ethyl group, 3-(dimethylamino)propyl group, 2-(dimethylamino)propyl group, 2-(methylethylamino)ethyl group, 1-(methylethylamino)ethyl group, tetrahydrofuryl group, tetrahydropyranyl group, pyrrolidinyl group, 1-methylpyrrolidinyl group, 1-ethylpyrrolidinyl group, piperidino group, piperadino group, N-methylpiperazino group, carbamoylmethyl group, methylcarbamoylmethyl group, ethylcarbamoylmethyl group, dimethylcarbamoylmethyl group, methylethylcarbamoylmethyl group, carbamoylethyl group, methylcarbamoylethyl group, ethylcarbamoylethyl group, dimethylcarbamoylethyl group, methylethylcarbamoylethyl group, carbamoylpropyl group, 2-carbamoyl cyclopropyl group, ureidomethyl group, N3-methylureidomethyl group, N3-ethylureidomethyl group, N3,N3-dimethylureidomethyl group, N3-methyl-N3-ethylureidomethyl group, 2-(ureido)ethyl group, 2-(N3-methylureido)ethyl group, 2-(N3-ethylureido)ethyl group, 2-(N3,N3-dimethylureido)ethyl group, 2-(N3-methyl-N3-ethylureido)ethyl group, 3-(ureido)propyl group, 2-(ureido)cyclopropyl group, N1-methylureidomethyl group, N1-ethylureidomethyl group, N1,N1-dimethylureidomethyl group, N1-methyl-N1-ethylureidomethyl group, 2-(ureido)ethyl group, 2-(N1-methylureido)ethyl group, 2-(N1-ethylureido)ethyl group, 2-(N1,N1-dimethylureido)ethyl group, 2-(N1-methyl-N1-ethylureido)ethylgroup,N1,N3-dimethylureidomethyl group, N1-methyl-N3-ethylureidomethyl group, 2-(N3-methyl-N1-ethyl)ureidoethyl group, 2-(N1,N3-diethylureido)ethyl group, 1-carbamoyl-2-hydroxyethyl group, 1,2-dicarbamoylethyl group, 1-carbamoyl-2-cyclopentyl group, 1-carbamoyl-1-cyclopentyl group, 1-carbamoyl-1-cyclohexyl group, 1-carbamoyl-1-methylethyl group, 1-carbamoyl-2,2-dimethylpropyl group, 1-aminomethyl-cyclopentyl group, 1-methylaminomethyl-cyclopentyl group, 1-dimethylaminomethyl-cyclopentyl group, 1-aminomethyl-cyclohexyl group, 1-methylaminomethyl-cyclohexyl group, and 1-dimethylaminomethyl-cyclohexyl group.

The carbamoyl group optionally substituted with 1 or 2 substituents means a carbamoyl group optionally substituted with 1 or 2 straight, branched, or cyclic C₁₋₆ alkyl groups, and examples thereof include, in addition to unsubstituted carbamoyl group, methylcarbamoyl group, ethylcarbamoyl group, propylcarbamoyl group, isopropylcarbamoyl group, primary, secondary, or tertiary butylcarbamoyl group, pentylcarbamoyl group, hexylcarbamoyl group, cyclopropylcarbamoyl group, cyclobutylcarbamoyl group, cyclopentylcarbamoyl group, cyclohexylcarbamoyl group, cyclopropylmethylcarbamoyl group, cyclopentylmethylcarbamoyl group, dimethylcarbamoyl group, methylethylcarbamoyl group, diethylcarbamoyl group, methylpropylcarbamoyl group, methylisopropylcarbamoyl group, methylcyclopropylcarbamoyl group, and methylcyclopropylmethylcarbamoyl group.

The oxamoyl group optionally substituted with 1 or 2 substituents means an oxamoyl group optionally substituted with 1 or 2 straight, branched or cyclic C₁₋₆ alkyl groups, and examples thereof include, in addition to unsubstituted oxamoyl group, methyloxamoyl group, ethyloxamoyl group, propyloxamoyl group, isopropyloxamoyl group, primary, secondary, or tertiary butyloxamoyl group, pentyloxamoyl group, hexyloxamoyl group, cyclopropyloxamoyl group, cyclobutyloxamoyl group, cyclopentyloxamoyl group, cyclohexyloxamoyl group, cyclopropylmethyloxamoyl group, cyclopentylmethyloxamoyl group, dimethyloxamoyl group, methylethyloxamoyl group, diethyloxamoyl group, methylpropyloxamoyl group, methylisopropyloxamoyl group, methyl cyclopropyloxamoyl group, and methyl cyclopropylmethyloxamoyl group.

The amino group optionally substituted with 1 or 2 substituents means an amino group optionally substituted with 1 or 2 straight, branched or cyclic C₁₋₆ alkyl groups, and examples thereof include, in addition to unsubstituted amino group, methylamino group, ethylamino group, propylamino group, isopropylamino group, primary, secondary, or tertiary butylamino group, pentylamino group, hexylamino group, cyclopropylamino group, cyclobutylamino group, cyclopentylamino group, cyclohexylamino group, cyclopropylmethylamino group, cyclopentylmethylamino group, dimethylamino group, methylethylamino group, diethylamino group, methylpropylamino group, methylisopropylamino group, methyl cyclopropylamino group, methyl cyclopropylmethylamino group, and methyl tertiary butoxycarbonylamino group.

Examples of the 4- to 7-membered aliphatic hyterocyclic group which is optionally substituted with 1 or 2 substituents include azetidinyl group, pyrrolidinyl group, piperidinyl group, piperadinyl group, hexahydropyridazinyl group, hexahydropyrimidinyl group, pyrazolidinyl group, imidazolidinyl group, homopiperadinyl group, morpholinyl group, and thiomorpholinyl group.
These groups maybe substitutedwith a substituent such as hydroxyl group, oxo group, carboxyl group, sulfo group, cyano group, nitrogroup, or with the halogeno group, the lower alkoxy group, the alkylsulfonyl group, the alkyl group optionally substituted with 1 or 2 substituents, the amino group optionally substituted with 1 or 2 substituents, the carbamoyl group optionally substituted with 1 or 2 substituents, the lower acyl group, or the aminosulfonyl group optionally substituted with 1 or 2 substituents as described above.

Examples of the phenyl group optionally substituted with 1 to 3 substituents are the groups as mentioned above for Ar₁ and Ar₂, and examples of the 5- or 6-membered aromatic heterocyclic group optionally substituted with 1 to 3 substituent are the groups as mentioned above for Ar₁ and Ar₂.

Next, compounds (I) of the present invention are described in further detail.

Examples of the preferable aromatic heterocyclic group optionally substituted with 1 to 3 substituents represented by Ar₁ and Ar₂ in general formula (I) include pyridyl, pyridazinyl, pyrimidinyl, pyrazinyl, thiadiazolyl, 1H-pyrrolyl group, 1H-imidazolyl group and 1H-pyrazolyl group optionally substituted with 1 to 3 substituents selected from C₁₋₆ alkyl groups, hydroxy C₁₋₆ alkyl groups, halogeno C₁₋₆ alkyl groups, amino C₁₋₆ alkyl groups, mono- or di (C₁₋₆ alkyl) amino C₁₋₆ alkyl groups, C₂₋₇ alkanoylamino C₁₋₆ alkyl groups, C₆₋₁₄ arylcarbonylamino C₁₋₆ alkyl groups, alicyclic heterocyclic carbonylamino C₁₋₆ alkyl groups, C₁₋₆ alkoxycarbonylamino C₁₋₆ alkyl groups, C₆₋₁₄ aryloxycarbonylamino C₁₋₆ alkyl groups, carbamoylamino C₁₋₆ alkyl groups, C₁₋₆ alkylcarbamoylamino C₁₋₆ alkyl groups, alicyclic heterocyclic carbamoylamino C₁₋₆ alkyl groups, hydroxyamino C₁₋₆ alkyl groups, C₁₋₆ alkoxyamino C₁₋₆ alkyl groups, C₁₋₆ alkylsulfonylamino C₁₋₆ alkyl groups, C₆₋₁₄ arylsulfonylamino C₁₋₆ alkyl groups, carbamoyl C₁₋₆ alkyl groups, C₁₋₆ alkylcarbamoyl C₁₋₆ alkyl groups, C₁₋₆ alkoxycarbonyl C₁₋₆ alkyl groups, carboxy C₁₋₆ alkyl groups, alicyclic heterocyclic carbonyl C₁₋₆ alkyl groups, halogeno groups, hydroxy group, cyano group, C₁₋₆ alkoxy groups, carbamoyl C₁₋₆ alkoxy groups, C₁₋₆ alkoxycarbonyl C₁₋₆ alkoxy groups, carboxy C₁₋₆ alkoxy groups, alicyclic heterocyclic carbonyl C₁₋₆ alkoxy groups, C₆₋₁₄ aralkyloxy groups, C₁₋₆ alkylthio groups, C₁₋₆ alkoxycarbonyl groups, carboxyl group, C₁₋₆ alkylsulfonyl groups, amino group, mono- or di (C₁₋₆, alkyl)amino groups, C₂₋₇ alkanoylamino groups, C₁₋₆ alkoxycarbonylamino groups, C₁₋₆ alkylureido groups, carbamoyl group, C₁₋₆ alkylcarbamoyl groups, aminosulfonyl group, C₁₋₆ alkylaminosulfonyl groups, aliphatic heterocyclic groups, C₂₋₆ alkenyl groups, C₂₋₆ alkynyl groups, and C₂₋₇ alkanoyl groups.

More preferable examples of the aromatic heterocyclic group include 2-pyridyl group, 3-pyridyl group, 6-methoxy-3-pyridyl group, 5-methoxy-2-pyridyl group, 4-methyl-2-pyridyl group, 5-methyl-2-pyridyl group, 6-methyl-3-pyridyl group, 6-hydroxy-3-pyridyl group, 6-cyano-3-pyridyl group, 6-carbamoyl-3-pyridyl group, 4-carbamoyl-2-pyridyl group, 5-carbamoyl-2-pyridyl group, 4-hydroxymethyl-2-pyridyl group, 5-hydroxy-2-pyridyl group, 5-cyano-2-pyridyl group, 5-carboxyl-2-pyridyl group, 5-hydroxymethyl-2-pyridyl group, 5-aminomethyl-2-pyridyl group, 5-chloro-2-pyridyl group, 5-amino-2-pyridyl group, 5-dimethylamino-2-pyridyl group, 5-fluoro-2-pyridyl group, 4-methoxy-2-pyridyl group, 5-formyl-2-pyridyl group, 5-acetyl-2-pyridyl group, 5-ethynyl-2-pyridyl group, 5-(1-hydroxyethyl)-2-pyridyl group, 5-(2-methoxycarbonyl vinylene)-2-pyridyl group, 5-(2-methoxycarbonylethyl)-2-pyridyl group, 5-(2-carbamoylethyl)-2-pyridyl group, 5-(methylamino)methyl-2-pyridyl group, 5-(methanesulfonylamino) methyl-2-pyridyl group, 5- (acetylamino) methyl-2-pyridyl group, 5-(methoxycarbonylamino)methyl-2-pyridyl group, 5-(phenoxycarbonylamino)methyl-2-pyridyl group, 5-(cyclopentanecarbonylamino)methyl-2-pyridyl group, 5-([3-(tetrahydro-2H-pyran-4-yl)ureido]methyl)-2-pyridyl group, 5-(morpholinocarbonylamino)methyl-2-pyridyl group, 5-(carbamoylmethyloxy)-2-pyridyl group, 3-pyridazinyl group, 6-methoxy-3-pyridazinyl group, 6-methyl-3-pyridazinyl group, 2-pyrazinyl group, 5-methoxy-2-pyrazinyl group, 5-methyl-2-pyrazinyl group, 5-amino-2-pyrazinyl group, 2-pyrimidyl group, 4-pyrimidyl group, 5-methoxy-1,3,4-thiadiazole-2-yl group, 1H-pyrrole-2-yl group, 1-methyl-1H-pyrrole-2-yl group, 1H-pyrrole-3-yl group, 1-methyl-1H-pyrrole-3-yl group, 1H-pyrrole-1-yl group, 1-methyl-1H-imidazole-4-yl group, and 1H-pyrazole-3-yl group.

Preferable examples of the phenyl group optionally substituted with 1 to 3 substituents represented by Ar₁ and Ar₂ in general formula (I) include phenyl groups optionally substitutedwith 1 to 3 substituents selected from C₁₋₆ alkyl groups, hydroxy C₁₋₆ alkyl groups, halogeno C₁₋₆ alkyl groups, amino C₁₋₆ alkyl groups, mono- or di(C₁₋₆ alkyl) amino C₁₋₆ alkyl groups, C₂₋₇ alkanoylamino C₁₋₆ alkyl groups, C₆₋₁₄ arylcarbonylamino C₁₋₆ alkyl groups, alicyclic heterocyclic carbonylamino C₁₋₆ alkyl groups, C₁₋₆ alkoxycarbonylamino C₁₋₆ alkyl groups, C₆₋₁₄ aryloxycarbonylamino C₁₋₆ alkyl groups, carbamoylamino C₁₋₆ alkyl groups, C₁₋₆ alkylcarbamoylamino C₁₋₆ alkyl groups, alicyclic heterocyclic carbamoylamino C₁₋₆ alkyl groups, hydroxyamino C₁₋₆ alkyl groups, C₁₋₆ alkoxyamino C₁₋₆ alkyl groups, C₁₋₆ alkylsulfonylamino C₁₋₆ alkyl groups, C₆₋₁₄ arylsulfonylamino C₁₋₆ alkyl groups, carbamoyl C₁₋₆ alkyl groups, C₁₋₆ alkylcarbamoyl C₁₋₆ alkyl groups, C₁₋₆ alkoxycarbonyl C₁₋₆ alkyl groups, carboxy C₁₋₆ alkyl groups, alicyclic heterocyclic carbonyl C₁₋₆ alkyl groups, halogeno groups, hydroxy group, cyano group, C₁₋₆ alkoxy groups, carbamoyl C₁₋₆ alkoxy groups, C₁₋₆ alkoxycarbonyl C₁₋₆ alkoxy groups, carboxy C₁₋₆ alkoxy groups, alicyclic heterocyclic carbonyl C₁₋₆ alkoxy groups, C₆₋₁₄ aralkyloxy groups, C₁₋₆ alkylthio groups, C₁₋₆ alkoxycarbonyl groups, carboxyl group, C₁₋₆ alkylsulfonyl groups, amino group, mono- or di(C₁₋₆ alkyl) amino groups, C₂₋₇ alkanoylamino groups, C₁₋₆ alkoxycarbonylamino groups, C₁₋₆ alkylureido groups, carbamoyl group, C₁₋₆ alkylcarbamoyl groups, aminosulfonyl group, C₁₋₆ alkylaminosulfonyl groups, aliphatic heterocyclic groups, C₂₋₆ alkenyl groups, C₂₋₆ alkynyl groups, and C₂₋₇ alkanoyl groups. More preferable examples of the phenyl group include phenyl group, 4-methylphenyl group, 4-ethylphenyl group, 4-N,N-dimethylaminophenyl group, 3-N,N-dimethylaminophenyl group, 4-cyanophenyl group, and 4-carbamoylphenyl group.

The groups represented by R1 in general formula (I) include C₁₋₆ alkyl groups, C₁₋₆ alkoxy-C₁₋₆ alkyl groups, carbamoyl-C₁₋₆ alkyl groups, hydroxy-C₁₋₆ alkyl groups, halogeno-C₃₋₆ cycloalkyl groups, carboxy-C₁₋₆ alkyl groups, amino-C₃₋₆ cycloalkyl groups, N-C₁₋₆ alkylamino-C₃₋₆ cycloalkyl groups, N,N-di(C₁₋₆ alkyl)amino-C₃₋₆ cycloalkyl groups, N,N-di (C₁₋₆ alkyl) amino-C₁₋₆ alkyl-C₃₋₆ cycloalkyl groups, hydroxy C₁₋₆ alkyl-C₃₋₆ cycloalkyl groups, propargyl-C₃₋₆ cycloalkyl groups, carbamoyl-C₃₋₆ cycloalkyl groups, ε-amino caprolactam group, C₃₋₆ cycloalkyl-C₁₋₆ alkyl groups, azetidinyl group, N-C₁₋₆ alkyl azetidinyl groups, pyrrolidinyl group, N-C₁₋₆ alkylpyrrolidinyl groups, pyrrolidinyl-C₁₋₆ alkyl groups, N-C₁₋₆ alkyl-pyrrolidinyl-C₁₋₆ alkyl groups, tetrahydrofuranyl group, tetrahydropyranyl group, tetrahydrofuran-C₁₋₆ alkyl groups, phenyl group, C₁₋₆ alkoxy-phenyl groups, halogenophenyl groups, cyanophenyl group, phenyl C₁₋₆ alkyl groups, pyridyl group, and pyridyl-C₁₋₆ alkyl group.

Examples of more preferable groups for R1 include methyl group, ethyl group, n-propyl group, isopropyl group, isobutyl group, t-butyl group, (2-hydroxy-1,1-dimethyl)ethyl group, aminocyclopropyl group, N,N-dimethylaminocyclopropyl group, N-methylazetidinyl group, pyrrolidinyl group, N-methylpyrrolidinyl group, N,N-dimethylamino-t-butyl group, 1-(N,N-dimethylaminomethyl)-1-cyclopentyl group, 1-methyl-1-(N-methylpyrrolidinyl) group, pyrrolidinylethyl group, methoxy-t-butyl group, tetrahydrofuryl group, tetrahydropyranyl group, 1-hydroxymethyl-1-cyclopentyl group, 1-hydroxymethyl-1-cyclohexyl group, tetrahydrofurylmethyl group, fluorocyclopropyl group, 1-carbamoyl-1-methylethyl group, 1-carbamoyl-1-cyclopentyl group, 2-carbamoyl-1-cyclopentyl group, 2-carbamoyl-1-cyclohexyl group, 1-carbamoyl-2,2-dimethylpropyl group, ε-caprolactam-2-yl group, cyclopropyl group, cyclobutyl group, cyclohexyl group, bicycloheptyl group, cyclohexylmethyl group, neopentyl group, 1-propargyl-1-cyclohexyl group, phenyl group, pyridyl group, methoxypyridyl group, fluoropyridyl group, methoxyphenyl group, fluorophenyl group, benzyl group, 1-cyanobenzyl group, 1,1-dimethylbenzyl group, pyridylmethyl group, methoxy group, cyanomethyl group, piperidino group, (2-fluoro-1,1-dimethyl)ethyl group, and (2-fluoro-1-fluoromethyl-1-methyl)ethyl group.

The groups represented by R2 include a hydrogen atom, a C₁₋₆ alkyl group, a carboxy C₁₋₆ alkyl group, a C₁₋₆ alkoxycarbonyl-C₁₋₆ alkyl group, and a carbamoyl C₁₋₆ alkyl group. More preferable examples of R2 include a hydrogen atom, carbamoylmethyl group, carboxymethyl group, methyl group, ethyl group, n-propyl group, isopropyl group, and isobutyl group.

With regard to the salt of the compound (I) of the present invention, not all the compounds of the present invention form a salt. However, when the compound (I) has carboxyl group, amino group, or the like, and/or when Ar₁ or Ar₂ is pyridine ring or the like, the compound can form a salt, and in some case, the salt may also form a solvate. The term "salt" as used herein may be exemplified by a salt with an inorganic acid such as hydrochloric acid, hydrobromic acid, sulfuric acid, or nitric acid, a salt with an organic acid such as methanesulfonic acid, p-toluenesulfonic acid, fumaric acid, or trifluoroacetic acid, and a salt with an alkaline metal or an alkaline earth metal ion such as sodium, potassium, or calcium ion.

The solvate of the present compound (I) and the solvate of a salt of the present compound (I) include those formed by addition of the solvent used in the precipitation of the crystal, and also, those formed by absorbingmoisture in the air. Examples of the solvent include lower alcohols such as methanol and ethanol, other organic solvents such as acetone and acetonitrile, and water.

Typical process for producing the compounds (I) of the present invention is described below.

In producing a compound (I) of the present invention, a pyrazolecarboxylic acid (7) produced by the procedure as described below may be used as an intermediate.

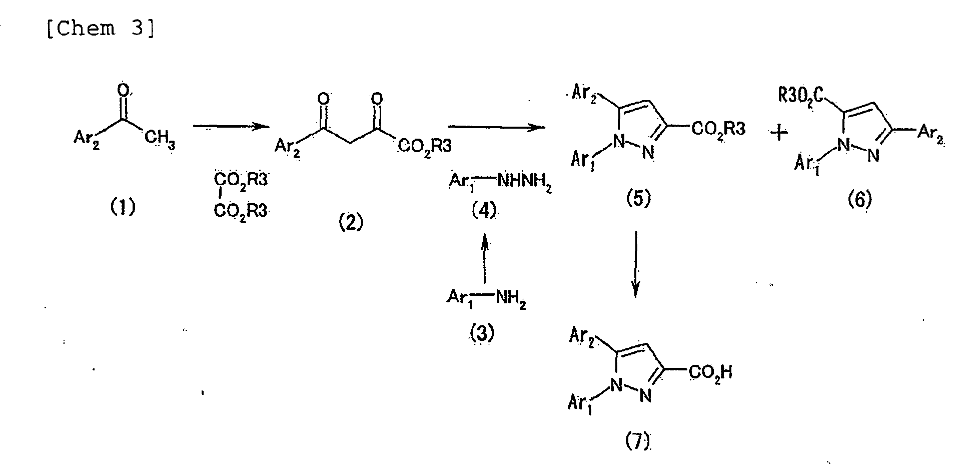

(In the reaction scheme, Ar₁ and Ar₂ are as defined above, andR3 represents methyl group or ethyl group).

A compound (2) may be produced by dissolving or suspending a compound (1) and a dialkyl oxalate in an adequate solvent such as N,N-dimethylformamide, adding sodium hydride under argon stream at a temperature of -20 to 20°C, and stirring the mixture.

A compound (2) may also be produced by treating a compound (1) and a dialkyl oxalate in the presence of a sodium alkoxide (methoxide or ethoxide) in an alcohol (methanol or ethanol) solution. The reaction temperature is preferably in the range of from -10 to 100°C.

A compound (2) can also be produced by dissolving a compound (1) in an inert solvent such as tetrahydrofuran, cooling the solution to -78°C, treating the solution with a base such as lithium bis (trimethylsilyl) amide, adding a dialkyl oxalate, and stirring the solution. The reaction temperature is preferably in the range of from -78 to 20°C.

The compound (1) used may be either a commercially available product, or the one produced by the method described in the Referential Example or a similar method.

A compound (5) can be produced by dissolving a compound (2) in an alcohol (methanol or ethanol), adding a hydrazine derivative (4) or its salt at room temperature, adding an adequate amount of acetic acid, and heating the mixture under reflux. The byproduct, position isomer (6), produced in the course of this step may be readily separated and removed from the compond (5) by column chromatography on silica gel.

Alternatively, the reaction for producing the pyrazole ring may be accomplished by adding an adequate amount of triethylamine or conc. hydrochloric acid instead of adding acetic acid and heating the mixture under reflux, and in some cases, compound (5) can be produced even without adding acetic acid, triethylamine, or conc. hydrochloric acid.

In the reaction for producing the pyrazole ring, a 4,5-dihydro-5-hydroxy-1H-pyrazole derivative is sometimes produced as an intermediate together with a compound (5). In this case, the compound (5) may be efficiently recovered by dissolving the resulting mixture in a solvent such as methylene chloride or N, N-dimethylformamide, adding methanesulfonyl chloride, triethylamine, and 4-dimethylaminopyridine, and stirring the mixture.

A pyrazolecarboxylic acid (7) may be produced by hydrolyzing a compound (5) by a method commonly used in the art.

The hydrolysis may be conducted in the presence of a base or a Lewis acid. The base used may be a hydroxide of an alkaline metal (such as lithium, sodium, or potassium), and the reaction temperature in such a case is preferably in the range of from room temperature to 100°C. The Lewis acid use may be boron tribromide, and the reaction temperature in such a case is preferably in the range of from -20 to 100°C, and more preferably in the range of from -5 to 50°C.

A compound (5) produced by the procedure as described above may be changed to another compound (5), for example, by modifying a substituent on Ar₁ using the knowledge common in the art of organic chemistry. In an embodiment, a compound (5) wherein the substituent on Ar₁ is a halogeno group such as chloro or bromo group may be dissolved in methanol, and after adding sodium methoxide, the solution is heated under reflux to produce a compound (5) wherein the substituent on Ar₁ is methoxy group. In another embodiment, a compound (5) wherein the substituent on Ar₁ is a halogeno group such as chloro or bromo group may be dissolved in a mixed solvent of methanol and toluene, and after adding sodium methoxide and a catalyst such as copper (I) bromide, the solution is heated under reflux to produce a compound wherein the substituent on Ar₁ is methoxy group.

The intermediate (5) of the compound (I) of the present invention produced by the procedure as described above may be further modified using the knowledge common in the art of organic chemistry to produce another compound (5). For example, a hydroxy derivative (5b), a trifluoromethanesulfonyloxy derivative (5c), a cyano derivative (5d), and the like may be produced from a benzyloxy derivative (5a).

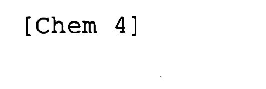

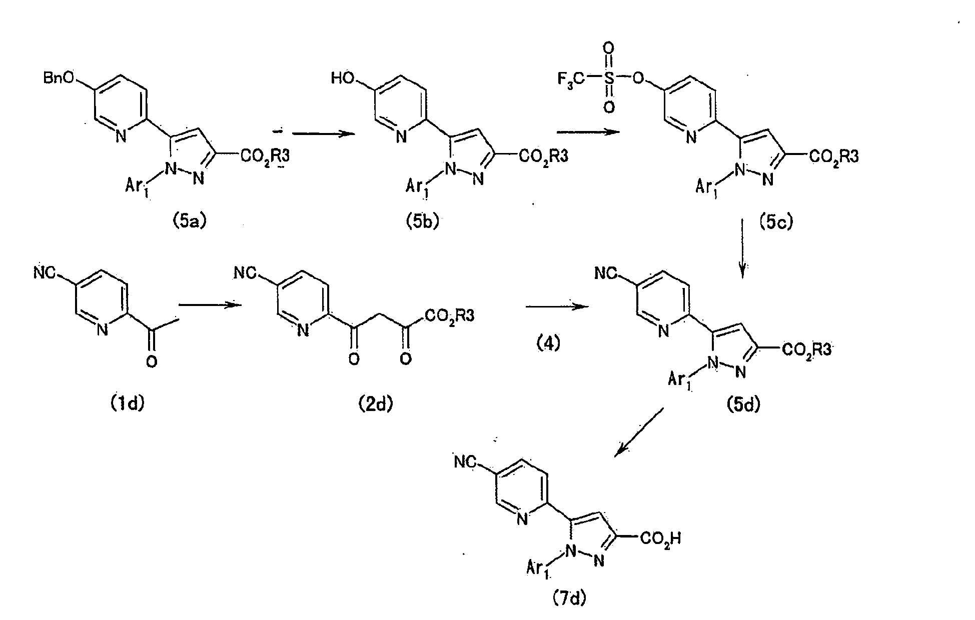

(In the reaction scheme, Ar₁ is as defined above, Bn represents benzyl group, and R3 represents methyl group or ethyl group).

More specifically, hydroxy derivative (5b) may be produced by dissolving benzyloxy derivative (5a) in a solvent such as ethanol, and catalytically reducing the benzyloxy derivative (5a) by using 10% palladium on carbon as a catalyst.

Trifluoromethanesulfonyloxy derivative (5c) may be produced by dissolving hydroxy derivative (5b) in a solvent such as methylene chloride for reaction with trifluoromethanesulfonic anhydride in the presence of a base such as pyridine at a temperature of from -50 to 50°C.

A cyano derivative (5d) may be produced by dissolving a trifluoromethanesulfonyloxy derivative (5c) in a solvent such as dichloroethane, adding tri-n-butyltin cyanide and tetrakis (triphenylphosphine)palladium (0), and stirring the mixture. The reaction temperature is preferably in the range of from 10 to 100°C. The reaction conditions and the reagents usedmaybe adequately selected based on the knowledge common in the art of organic chemistry.

Alternatively, a cyano derivative (5d) may be produced by dissolving an acetylpyridine derivative (1d) in an inert solvent such as tetrahydrofuran, cooling the solution to -78°C, treating the solution with lithium bis (trimethylsilyl) amide, then adding a dialkyl oxalate, stirring the mixture to produce a compound (2d), dissolving the thus produced compound (2d) in 1N hydrochloric acid solution in ethanol, adding thereto a hydrazine derivative-(4) at room temperature, and heating the mixture under reflux.

The acetylpyridine derivative (1d) used may be produced by the method described in the Referential Example or a similar method.

A pyrazolecarboxylic acid (7d) may be produced by dissolving a cyano derivative (5d) in tetrahydrofuran and water, adding thereto an equivalent amount of lithium hydroxide monohydrate, and stirring the mixture. The reaction temperature is preferably in the range of from 0 to 50°C.

A carboxylic acid derivative (5h), an amino derivative (5i), and the like may be produced from a methylpyrazine derivative (5g) as shown below.

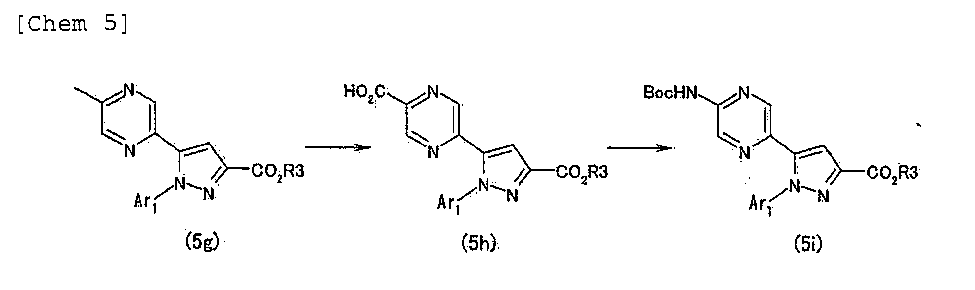

(In the reaction scheme, Ar₁ and R3 are as defined above, and Boc represents tert-butoxycarbonyl group.)

More specifically, a carboxylic acid derivative (5h) may be produced by dissolving a methylpyrazine derivative (5g) in a solvent such as pyridine, adding thereto selenium dioxide at room temperature, and heating the mixture under reflux.

An amino derivative (5i) maybe produced by dissolving a carboxylic acid derivative (5h) in a solvent such as 1,4-dioxane, adding thereto tert-butanol, triethylamine, and diphenylphosphorylazide at room temperature, and heating the mixture under reflux. The reaction conditions and the reagents used may be adequately selected based on the knowledge common in the art of organic chemistry

Furthermore, an ethynyl derivative (5j) or the like maybe produced from a trifluoromethanesulfonyloxy derivative (5c) as shown below.

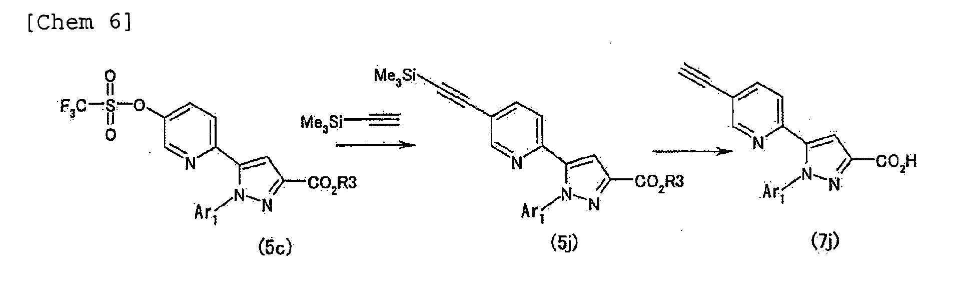

(In the reaction scheme, Ar₁ and R3 are as defined above.)

More specifically, an ethynyl derivative (5j) may be produced by dissolving a trifluoromethanesulfonyloxy derivative (5c) in N,N-dimethylformamide and triethylamine, adding thereto trimethylsilyl acetylene and bis(triphenylphosphine)palladium (II) dichloride, and stirring the mixture. The reaction temperature is preferably in the range of from 10 to 100°C. The reaction conditions and the reagents used may be adequately selected based on the knowledge common in the art of organic chemistry.

A pyrazolecarboxylic acid derivative (7j) may be produced by dissolving an ethynyl derivative (5j) in ethanol and tetrahydrofuran, and hydrolyzing the ethynyl derivative (5j) with sodium hydroxide. The reaction temperature is preferably in the range of from 0 to 100°C.

The hydrazine derivative (4) or its salt used in the reaction for forming the pyrazole ring may be either a commercially available product, or the one produced using the technique described in the Referential Example by reacting a halogenated Ar₁ with hydrazine, or a similar method. More specifically, a hydrazine derivative (4) or its salt may be produced by dissolving an amine (3) in conc. hydrochloric acid, adding thereto sodium nitrite in an ice bath to produce a diazo derivative, and treating the diazo derivative with tin (II) chloride. The reaction temperature is preferably in the range of from -10 to 20°C.

The amine (3) usedmay be either a commercially available compound or the one produced by the method described in the Referential Example or a similar method.

A compound (I) of the present invention may be produced by condensing the pyrazolecarboxylic acid derivative (7) produced by the procedure as described above with an amine (8).

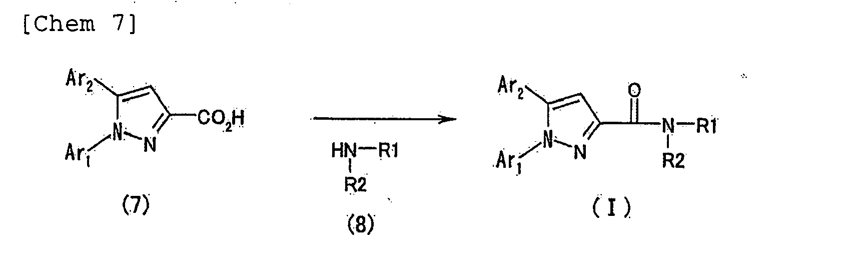

(In the reaction scheme, R1, R2, Ar₁, and Ar₂ are as defined above.)

The condensation as described above may be accomplished by any of the methods generally used for peptide synthesis. The method commonly used for peptide synthesis include azide method, acid chloride method, acid anhydride method, DCC (dicyclohexylcarbodiimide) method, active ester method, carbodiimidazole method, DCC/HOBT(1-hydroxybenzotriazole) method, amethodusingwater-soluble carbodiimide, and a method using diethyl cyanophosphate, and these methods are described, for example, in M. Bodanszky, Y.S. Klausner, and M.A. Ondetti, "Peptide Synthesis", A Wiley-interscience publication, NewYork, 1976; Gv. Pettit, "SyntheticPeptides", Elsevier Scientific Publication Company, New York, 1976; and Japanese Society of Chemistry ed. "Lectures on Experimental Chemistry 4th ed., vol. 22, Organic Synthesis IV", Maruzen Publishing, 1992. The solvents which may be used in such condensation include N,N-dimethylformamide, pyridine, chloroform, methylene chloride, tetrahydrofuran, 1,4-dioxane, acetonitrile, and a mixture thereof. The reaction temperature is preferably in the range of from -20 to 50°C, and more preferably from -10 to 30°C. The amine (8) used may be either a commercially available product or the one produced by the method described in the Referential Example or a similar method.

When the amine (8) used in the condensation as described above has a functional group such as hydroxyl group, amino group, or carboxyl group, such functional group may require preliminary protection by using an adequate protective group. Typical protective groups which may be used for hydroxyl group include tert-butyl group and benzyl group, and typical protective group for amino group include trifluoroacetyl group, tert-butoxycarbonyl group, and benzyloxycarbonyl group. When the functional group is carboxyl group, the amine may be used in the condensation after deriving the carboxyl group into methyl ester or tert-butyl ester. These protective groups may be cleaved off under conditions appropriate for each protective group.

The compound (I) of the present invention produced by the procedure as described above may be further modified by using the knowledge common in the art of organic chemistry to produce another compound (I) of the present invention. For example, a hydroxy derivative (Ib), a trifluoromethanesulfonyloxy derivative (Ic), a cyano derivative (Id), a carbamoyl derivative (Ie), an aminomethyl derivative (If), and a hydroxymethyl derivative (Ig) may be produced from a compound (Ia).

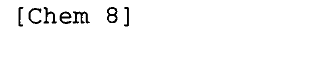

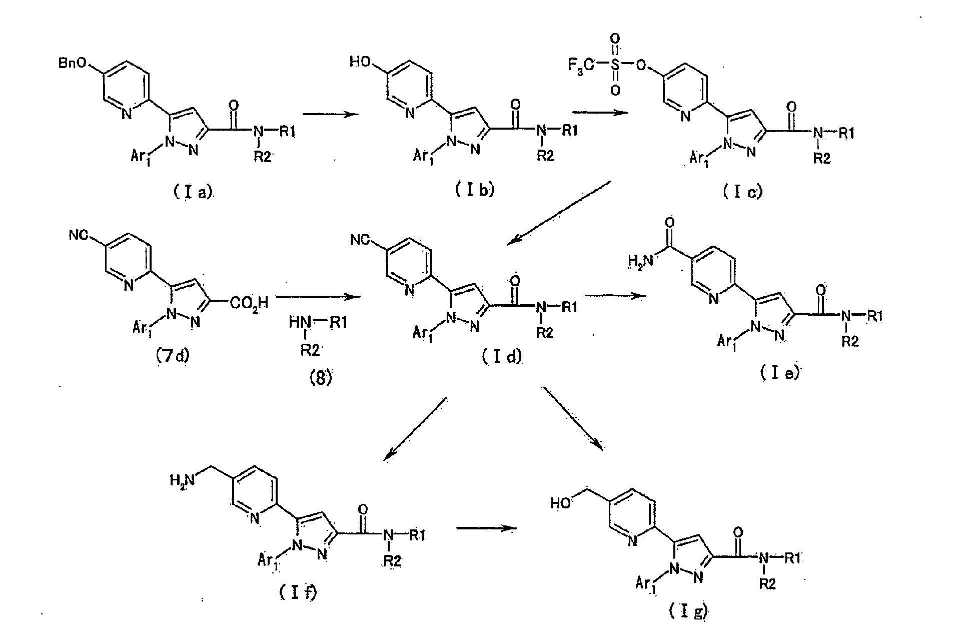

(In the reaction scheme, Ar₁, R1, and R2 are as defined above, Bn represents benzyl group, and Boc represents tert-butoxycarbonyl group.)

More specifically, a hydroxy derivative (Ib) may be produced by dissolving a benzyloxy derivative (Ia) in a solvent such as ethanol, and catalytically reducing the benzyloxy derivative (Ia) by using 10% palladium on carbon as a catalyst.

A compound (Ic) may be produced by dissolving a hydroxy derivative (Ib) in a solvent such as methylene chloride, and reacting it with trifluoromethanesulfonic anhydride in the presence of a base such as pyridine at a temperature in the range of from -50 to 50°C.

A cyano derivative (Id) may be produced by dissolving a compound (Ic) in a solvent such as dichloroethane, and reacting it with tri-n-butyl tin cyanide and tetrakis(triphenylphosphine)palladium (0). The reaction temperature is preferably in the range of from 10 to 100°C. The reaction conditions and the reagents used may be adequately selected based on the knowledge common in the art of organic chemistry.

Alternatively, a cyano derivative (Id) may be produced by condensing a cyano carboxylic acid derivative (7d) with an amine (8) by using a method commonly used in the art of peptide synthesis.

A carbamoyl derivative (Ie) may be produced by dissolving a cyano derivative (Id) in adequate solvents such as methanol and tetrahydrofuran, and hydrolyzing the cyano derivative (Id) with sodium hydroxide. The reaction temperature is preferably in the range of from 0 to 100°C.

Alternatively, a carbamoyl derivative (Ie) may be produced by converting a cyano derivative (Id) to a carboxylic acid derivative, and then reacting it with aqueous ammonia or ammonium chloride using an adequate condensing agent.

An aminomethyl derivative (If) may be produced by dissolving a cyano derivative (Id) in 2.0M ammonia solution in ethanol, and catalytically reducing the cyano derivative (Id) using a nickel-silica alumina as a catalyst (at 8 atm).

A hydroxymethyl derivative (Ig) may be produced by dissolving a cyano derivative (Id) in an inert solvent such as tetrahydrofuran, adding thereto diisobutylaluminum hydride, stirring the resulting mixture, treating the mixture under an acidic condition for conversion into an aldehyde derivative, and further reducing the aldehyde derivative with sodium borohydride. The reaction temperature is preferably in the range of from -10 to 50°C.

A compound (I) of the present invention produced by the procedure as described above may also be further modified by using the knowledge common in the art of organic chemistry to produce another compound (I) of the present invention. For example, derivatives such as aldehyde, 1-hydroxy-1-ethyl, methylaminomethyl, acrylate, propionate, and propionamide derivatives (I1 to Iq) may be produced from a compound (Ig).

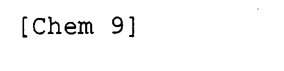

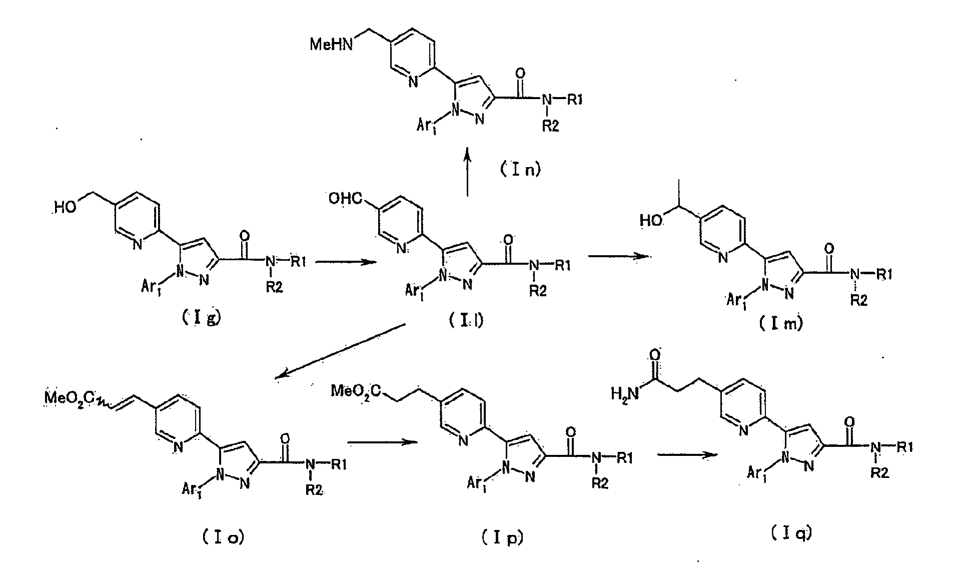

(In the reaction scheme, Ar₁, R1, and R2 are as defined above.)

More specifically, an aldehyde derivative (Il) may be produced by dissolving a hydroxymethyl derivative (Ig) in a solvent such as methylene chloride, adding Dess-Martin reagent (1,1,1-tris(acetoxy)-1,1-dihydro-1,2-benziodoxol-3-(1H)-one), and stirring the mixture. The reaction temperature is preferably in the range of from 0 to 50°C. The reaction conditions and the reagents used may be adequately selected based on the knowledge common in the art of organic chemistry.

A1-hydroxy-1-ethyl derivative (Im) maybe produced by dissolving an aldehyde derivative (Il) in an inert solvent such as tetrahydrofuran, cooling the solution to -78°C, and treating the solution with methyl magnesium bromide.

A methylaminomethyl derivative (In) may be produced by dissolving an aldehyde derivative (Il), acetic acid, and methylamine in a solvent such as methanol, adding thereto cyano sodiumborohydride, and stirring the mixture. The reaction temperature is preferably in the range of from 0 to 50°C.

An acrylate derivative (Io) may be produced by adding an aldehyde derivative (Il) to a reaction liquid prepared by dissolving trimethyl phosphonoacetate in an inert solvent such as tetrahydrofuran, adding thereto sodium hydride, and stirring the mixture. The reaction temperature is preferably in the range of from 0 to 50°C.

A propionate derivative (Ip) may be produced by dissolving an acrylate derivative (Io) inasolventsuchasmethanol, and catalytically reducing the acrylate derivative (Io) by using 10% palladium on carbon as a catalyst.

A propionamide derivative (Iq) may be produced by dissolving a propionate derivative (Ip) in tetrahydrofuran and water, adding thereto an equivalent amount oflithium hydroxide monohydrate, stirring the mixture for conversion into a propionic acid derivative, dissolving the resulting propionic acid derivative in a solvent such as N,N-dimethylformamide, adding thereto ammonium chloride or aqueous ammonia and an adequate condensing agent, and stirring the mixture.

Thecompound (I) of the present invention produced by the procedure as described above may also be further modified by using the knowledge common in the art of organic chemistry to produce another compound (I) of the present invention. For example, an acetyl derivative (Is) may be produced from a compound (Ir).

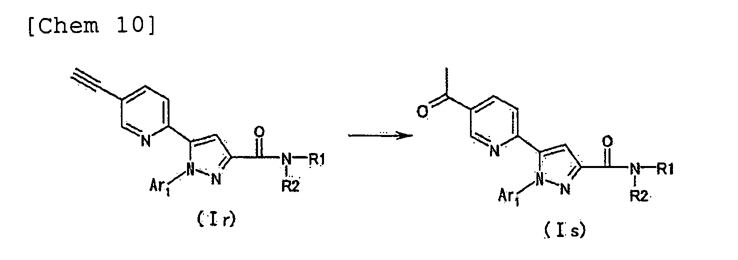

(In the reaction scheme, Ar₁, R1, and R2 are as defined above.)

More specifically, an acetyl derivative (Is) may be produced by dissolving an acetylene derivative (Ir) in acetone and water (3:1 v/v), adding thereto mercuric sulfate and sulfuric acid at room temperature, and heating the mixture under reflux.

The compound (I) of the present invention produced by the procedure as described above may also be further modified by using the knowledge common in the art of organic chemistry to produce another compound (I) of the present invention. For example, derivatives such as sulfoneamide, amide, urethane, urea derivatives may be produced from a compound (If).

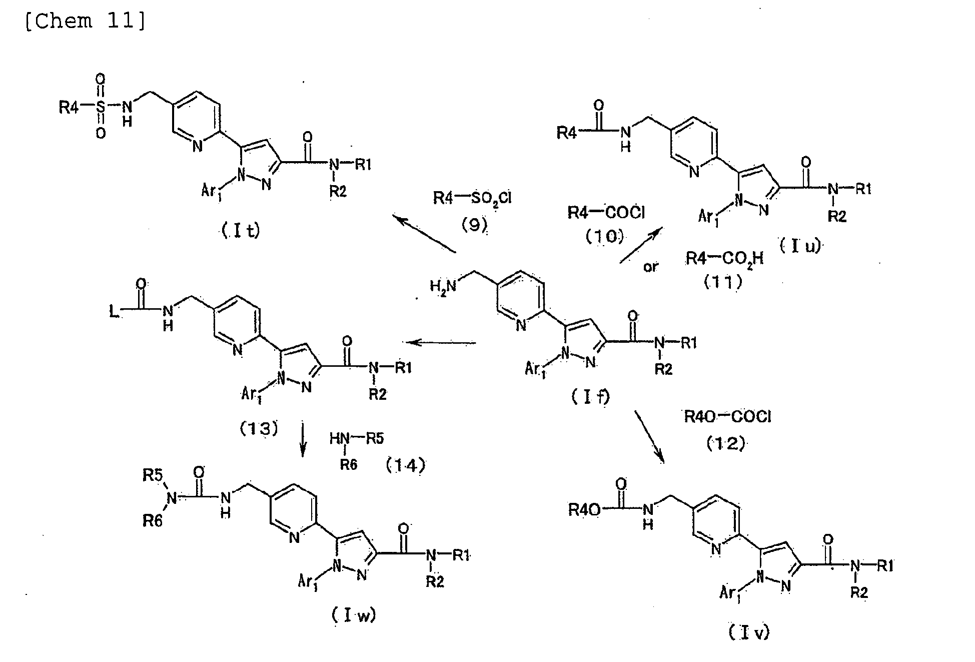

(In the reaction scheme, Ar₁, R1, and R2 are as defined above; R4 represents a lower alkyl group or an aryl group; R5 and R6 may independently represent hydrogen, a lower alkyl group, or an alicyclic heterocyclic group, or R5, R6, and N may together represent a 4- to 7-membered alicyclic heterocyclic group; and L represents a leaving group.)

More specifically, a sulfoneamide derivative (It) maybeproduced by dissolving an aminomethyl derivative (If) in a solvent such as methylene chloride, adding thereto sulfonyl chloride derivative (9) in the presence of an organic base such as triethylamine, and stirring the mixture. The reaction temperature is preferably in the range of from -10 to 50°C.

An amide derivative (Iu) may be produced by condensing an aminomethyl derivative (If) with an acid chloride (10) or a carboxylic acid (11) by using a method commonly used for peptide synthesis. For example, an amide derivative (Iu) may be produced by dissolving an aminomethyl derivative (If) in a solvent such as methylene chloride, adding thereto an acid chloride (10) in the presence of an organic base such as triethylamine, and stirring the mixture. The reaction temperature is preferably in the range of from -10 to 50°C.

An urethane derivative (Iv) may be produced by dissolving an aminomethyl derivative (If) in a solvent such as methylene chloride, and treating the solution with a chloroformate (12) in the presence of an organic base such as triethylamine. The reaction temperature is preferably in the range of from -10 to 50°C.

An urea derivative (Iw) may be produced by dissolving an aminomethyl derivative (If) in a solvent such as methylene chloride, adding thereto 4-nitrophenyl chloroformate in the presence of an organic base such as triethylamine, stirring the mixture to produce a compound (13) wherein the leaving group L is 4-nitrophenyloxy group, and reacting the compound (13) with an amine (14). The reaction temperature is preferably in the range of from -10 to 50°C.

Alternatively, an urea derivative (Iw) may be produced by treating an aminomethyl derivative (If) with an alkyl isocyanate in a solvent such as methylene chloride.
The sulfonyl chloride derivative (9), acid chloride (10), carboxylic acid (11), chloroformate (12), and amine (14) used in the procedure as described above may be either commercially available products or those produced by the methods described in the Referential Examples or similar methods.

The compounds (I) of the present invention, the salts or the solvates thereof, or the solvates of the salts have a potent anti-platelet coagulation activity, and they exhibit effectiveness in a high shear stress-induced thrombosis model. Therefore, the compounds (I) of the present invention, the salts or the solvates thereof, or the solvates of the salts are useful in human and other mammals as a prophylactic and/or therapeutic agent for ischemic diseases caused by thrombus or embolus such as myocardial infarction, angina pectoris (chronic stable angina, unstable angina, etc.), ischemic cerebrovascular disorder (transient ischemic attack (TIA), cerebral infarction, etc.), peripheral vascular disease, embolism after replacement with an artificial vessel, thrombotic embolism after coronary artery intervention (coronary artery bypass grafting (CAGB), percutaneous transluminal coronary angioplasty (PTCA), stentplacement, etc.), diabetic retinopathy and nephropathy, and embolism after replacement with an artificial heart valve, and also, as a prophylactic and/or therapeutic agent for thrombus and embolus associated with vascular operation, blood extracorporeal circulation, and the like.

When a compound (I) of the present invention, its salt or solvate, or the solvate of its salt is used as a drug, it may be administered preferably at a dose of 0.1 mg to 1 g per day per adult, and more preferably at 0.5 mg to 500 mg per day per adult either as a single dose or several divided doses, although the dose may vary depending on the age, sex, symptoms of the patient and the like. If necessary, the compound/salt/solvate may also be administered at a dose exceeding such daily dose.
A drug containing a compound (I) of the present invention, its salt or solvate, or a solvate of such salt is not limited with regard to its administration route or dosage form, and it may be administered via any route and in any dosage form as desired. More specifically, it may be prepared by any preparation method commonly used in the pharmaceutical preparation by incorporating a pharmaceutically acceptable vehicle as desired, and it may be prepared in any dosage form suitable for the selected administration route.
Oral preparations include solid preparations such as tablet, powder, granules, pill, and capsule, as well as liquid preparations such as solution, syrup, elixir, suspension, and emulsion.
An injectionmaybe prepared by filling a container with a solution of a compound (I), its salt or solvate, or a solvate of such salt. Alternatively, a solid prepared, for example, by freeze drying the solution is also usable as a preparation which is rehydrated before its use.
In the production of such preparation, pharmaceutically acceptable additives such as a binder, a disintegrant, a dissolution promoter, a lubricant, a filler, and an excipient may be selected as desired for incorporation in the preparation.

### [EXAMPLES]

Next, production of typical compounds the present invention is described. Also demonstrated by conducing tests is that the compounds produced exhibits strong platelet coagulation inhibitory action without inhibiting COX-1 or COX-2.

[Referential Example 1] 5-hydrazino-2-methoxypyridine hydrochloride

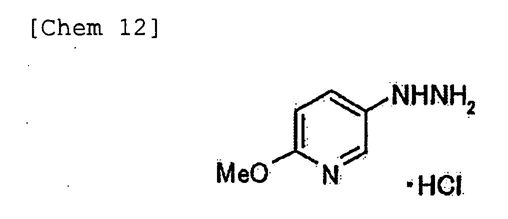

A solution of sodium nitrite (3.795 g) in water (20 ml) was added dropwise to a solution of 5-amino-2-methoxypyridine (6.21 g) in conc. hydrochloric acid (50 ml) over 60 minutes under ice cooling, and the mixture was stirred at the same temperature for 30 minutes. A solution of tin (II) chloride dihydrate (39.5 g) in conc. hydrochloric acid (30 ml) was added dropwise to the reaction liquid at an inner temperature of about 10°C over 30 minutes, and the mixture was stirred at room temperature for 2 hours. To the reaction liquid were added a solution of sodium hydroxide (75 g) in water (300 ml) and diethylether under ice cooling, and the phases were separated. The aqueous layer was extracted twice with diethylether, and after saturating the aqueous layer with sodium chloride, the aqueous layer was again extracted with diethylether. The organic layers were combined, and dried over anhydrous sodium sulfate. After filtration, 1M solution of hydrochloric acid in ethanol (50 ml) was added to the filtrate, and the mixture was stirred. The resulting solid precipitate was collected by filtration, washed with diethylether, and dried to give the title compound (5.02 g, 57%).

¹H-NMR (400 MHz, DMSO-d₆)δ: 3.81 (3H, s), 6.82 (1H, d, J = 8.8 Hz), 7.57 (1H, dd, J = 8.8, 2.9 Hz), 7.97 (1H, d, J = 2.9 Hz), 8.55-9.20 (1H, br), 10.13-10.50 (3H, br).
MS (ESI)m/z: 140 (M+H)⁺.

[Referential Example 2] 5-hydrazino-2-methoxypyridine

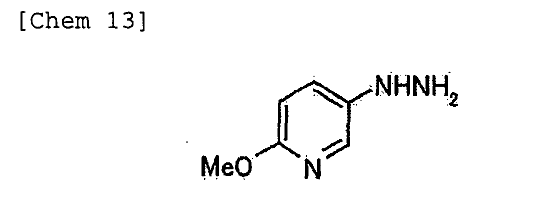

A solution of sodium nitrite (3.795 g) in water (20 ml) was added to a solution of 5-amino-2-methoxypyridine (6.207 g) in conc. hydrochloric acid (50 ml) over 80 minutes under ice cooling, and the mixture was stirred at the same temperature for 30 minutes. A solution of tin (II) chloride dihydrate (39.5 g) in conc. hydrochloric acid (30 ml) was added dropwise to the reaction liquid at an inner temperature of about 10°C over 60 minutes, and the mixture was stirred at room temperature for 12.5 hours. To the reaction liquidwere added a solution of sodium hydroxide (54 g) in water (200 ml) and chloroform under ice cooling. After removing the insoluble content by filtration, the aqueous layer was separated, then extracted twice with chloroform. The organic layers were combined and dried over anhydrous sodium sulfate. After filtration, the solvent was evaporated under reduced pressure to give the title compound (4.23 g, 60%) as a solid.

¹H-NMR (400 MHz, CDCl₃)δ: 3.50-3.68 (2H, br), 3.88 (3H, s), 4.86-5.03 (1H, br), 6.66 (1H, d, J = 8.8 Hz), 7.20 (1H, dd, J = 8.8, 2.9 Hz), 7.77 (1H, d, J = 2.9 Hz).
MS (ESI)m/z: 140 (M+H)⁺.

[Referential Example 3] Ethyl 4-(2-pyridyl)-2,4-dioxobutanoate

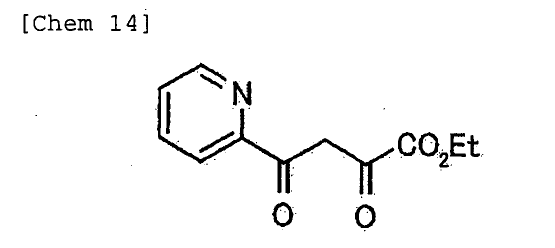

Under argon atmosphere, 2-acetylpyridine (1.39 ml) was added dropwise to a suspension of 60% sodium hydride (0.991 g) in N, N-dimethylformamide (30 ml), and the mixture was stirred for 5 minutes at 0°C and for another 30 minutes at room temperature. To the reaction liquid was added dropwise diethyl oxalate (3.36 ml) at 0°C, and the mixture was stirred for 10 minutes and for another 18 hours at room temperature. To the reaction liquid were added water and diethylether, and the aqueous layer was separated. The aqueous layer was neutralized with 1N aqueous hydrochloric acid (24.8 ml), and the solution was extracted with ethyl acetate. The organic layer was washed twice with water, and dried over anhydrous sodium sulfate. After filtration, the solvent was evaporated under reduced pressure, and the residue was purified by column chromatography on silica gel (methanol-chloroform) to give the title compound (1.12 g, 41%) as a solid.

¹H-NMR (400MHz, CDCl₃)δ: 1.40-1.43 (3H, m), 4.38-4.43 (2H, m), 7.51-7.54 (1H, m), 7.62 (1H, s), 7.89-7.93 (1H, m), 8.18 (1H, d, J = 8.0 Hz), 8.73 (1H, d, J = 4.4 Hz).
MS (EI)m/z: 221 (M⁺).

[Referential Example 4] 1-(6-Methoxy-3-pyridyl)-5-(2-pyridyl)-1H-pyrazole-3-carboxylic acid

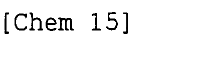

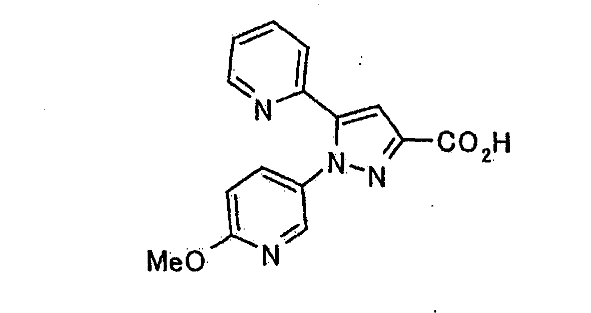

### 1) Ethyl 5-hydroxy-1-(6-methoxy-3-pyridyl)-5-(2-pyridyl)-4,5-dihydro-1H-pyrazole-3-carboxylate

A solution of the ethyl 4- (2-pyridyl) -2, 4-dioxobutanoate (1.10 g) of Referential Example 3 and the 5-hydrazino-2-methoxypyridine (0.692 g) of Referential Example 2 in ethanol (22 ml) was heated under reflux for 14 hours. After cooling with air, the solvent was evaporated, and the residue was purified by column chromatography on silica gel (hexane-ethyl acetate), and again by column chromatography on silica gel (toluene-acetone) to give ethyl 5-hydroxy-1-(6-methoxy-3-pyridyl)-5-(2-pyridyl)-4,5-dihydro-1H-py razole-3-carboxylate (0.575 g, 34%) as a solid.

¹H-NMR (400 MHz, CDCl₃)δ: 1.37-1.40 (3H, m), 3.47-3.64 (2H, m), 3.81 (3H, s), 4.35-4.40 (2H, m), 6.57-6.59 (1H, m), 6.85 (1H, m), 7.34-7.38 (1H, m), 7.45-7.48 (1H, m), 7.52-7.59 (2H, m), 7.79-7.83 (1H, m), 8.55-8.57 (1H, m).

### 2) Ethyl 1-(6-methoxy-3-pyridyl)-5-(2-pyridyl)-1H-pyrazole-3-carboxylate

Acetic acid (0.456 ml) was added to a solution of the ethyl 5-hydroxy-1-(6-methoxy-3-pyridyl)-5-(2-pyridyl)-4,5-dihydro-1H-py razole-3-carboxylate (0.546 g) in ethanol (11 ml), and the mixture was heated under reflux for 4 hours. After cooling with air, saturated aqueous sodium bicarbonate, water, and ethyl acetate were added to the reaction liquid, and the phases were separated. The organic layer was dried over anhydrous sodium sulfate. After filtration, the solvent was evaporated under reduced pressure, and the residue was purified by column chromatography on silica gel (hexane-ethyl acetate) to give ethyl 1-(6-methoxy-3-pyridyl)-5-(2-pyridyl)-1H-pyrazole-3-carboxylate (0.516 g, 100%) as a solid.

¹H-NMR (400 MHz, CDCl₃)δ: 1.43 (3H, t, J = 7.2 Hz), 3.95 (3H, s), 4.46 (2H, q, J = 7.2 Hz), 6.76-6.78 (1H, m), 7.22-7.28 (2H, m), 7.35-7.37 (1H, m), 7.66-7.71 (2H, m), 8.11 (1H, m), 8.52-8.54 (1H, m).
MS (FAB)m/z: 325 (M+H)⁺.

### 3) The title compound

1N aqueous sodium hydroxide (3.38 ml) was added to a solution of the ethyl 1-(6-methoxy-3-pyridyl)-5-(2-pyridyl)-1H-pyrazole-3-carboxylate (0.438 g) in methanol (8.8 ml) at room temperature, and the mixture was stirred for 4 hours. The reaction solvent was evaporated under reduced pressure. After adding 1N aqueous hydrochloric acid (3.38 ml) to the residue for neutralization, water and ethyl acetate were added to the solution, then the phases were separated. The organic layer was dried over anhydrous sodium sulfate, and after filtration, the solvent was evaporated under reduced pressure to give the title compound (0.344 g, 86%) as a solid.

¹H-NMR (400 MHz, DMSO-d₆)δ: 3.89 (3H, s), 6.89 (1H, d, J = 8.8 Hz), 7.33-7.37 (2H, m), 7.67-7.73 (2H, m), 7.85-7.89 (1H, m), 8.14 (1H, d, J = 2.4 Hz), 8.44-8.46 (1H, m), 13.06 (1H, br).
MS (FAB)m/z: 297 (M+H)⁺.

[Referential Example 5] 1-(6-Methoxy-3-pyridyl)-5-phenyl-1H-pyrazole-3-carboxylic acid

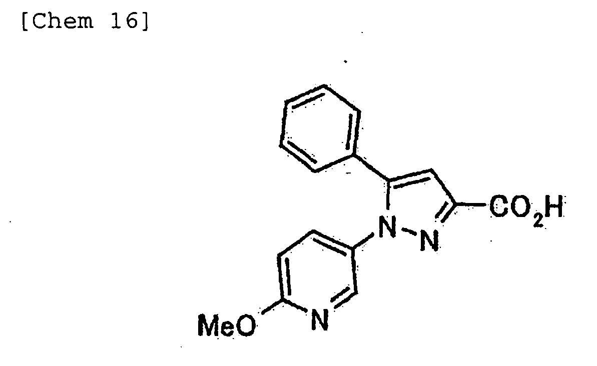

### 1) Ethyl 1-(6-methoxy-3-pyridyl)-5-phenyl-1H-pyrazole-3-carboxylate

To a solution of acetophenone (9.85 g) in N, N-dimethylformamide (80 ml) was added 60% sodium hydride (6.56 g) at 0°C, and the mixture was stirred for 30 minutes. A solution of diethyl oxalate (23.97 g) in N,N-dimethylformamide (80 ml) was added dropwise to the reaction liquid over 10 minutes, and the mixture was stirred at room temperature for 13 hours. The reaction liquid was acidified by adding 1N hydrochloric acid (180 ml), and water and ethyl acetate were added and the phases were separated. The organic layer was washed with water and brine, and it was dried over anhydrous magnesium sulfate. After filtration, the solvent was evaporated under reduced pressure to give ethyl 4-phenyl-2,4-dioxobutanoate (22.96 g, measured) as an oily product. This product was used in the subsequent reaction without further purification. The thus obtained ethyl 4-phenyl-2,4-dioxobutanoate was dissolved in ethanol (200 ml), and to this was added 5-hydrazino-2-methoxypyridine (11.39 g) of Referential Example 2, and the mixture was heated under reflux for 4 hours. After cooling with air, the reaction solvent was evaporated under reduced pressure, and the residue was purified by column chromatography on silica gel (ethyl acetate-hexane) to give ethyl 1-(6-methoxy-3-pyridyl)-5-phenyl-1H-pyrazole-3-carboxylate (16.37g, 61%) as an oily product.

¹H-NMR (400 MHz, CDCl₃)δ: 1.42 (3H, t, J = 7.0 Hz), 3.93 (3H, s), 4.45 (2H, q, J=7.0 Hz), 6.73 (1H, d, J= 8.8 Hz), 7.04 (1H, s), 7.19-7.26 (2H, m), 7.30-7.37 (3H, m), 7.57 (1H, dd, J = 8.8, 2.6 Hz), 8.11 (1H, d, J = 2.6 Hz).
MS (ESI)m/z: 324 (M+H)⁺.

### 2) The title compound

To a solution of the ethyl 1-(6-methoxy-3-pyridyl)-5-phenyl-1H-pyrazole-3-carboxylate (16.37 g) in methanol (250 ml) was added 1N aqueous sodium hydroxide (126 ml), and the mixture was stirred for 30 minutes. The reaction solvent was evaporated under reduced pressure. Water and diethylether were added to the residue, and the aqueous layer was separated. After acidifying the aqueous layer by adding 1N aqueous hydrochloric acid (140ml), and the solution was extracted with ethyl acetate. The organic layer was washed with water and brine, and dried over anhydrous sodium sulfate. After filtration, the solvent was evaporated under reduced pressure to give the title compound (13.88 g, 92%) as a solid.

¹H-NMR (400 MHz, CDCl₃)δ: 3.94 (3H, s), 6.75 (1H, d, J = 8.8 Hz), 7.10 (1H, s), 7.21-7.27 (2H, m), 7.32-7.39 (3H, m), 7.58 (1H, dd, J= 8.8, 2.6 Hz), 8.12 (1H, d, J = 2.6 Hz).
MS (ESI)m/z: 296 (M+H)⁺.

[Referential Example 6] 3-Hydrazinopyridine hydrochloride

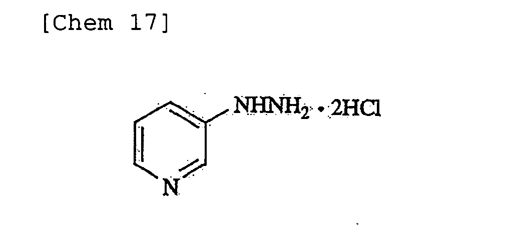

A solution of sodium nitrite (4.28 g) in water (20 ml) was added dropwise to a solution of 3-aminopyridine (5.15 g) in conc. hydrochloric acid (54 ml) at an inner temperature of 0 to 5°C over 30 minutes, and the mixture was stirred for another 5 minutes. The reaction liquid was added dropwise to a solution of tin (II) chloride dihydrate (43.68 g) in conc. hydrochloric acid (30 ml) at an inner temperature of 0 to 10°C over 1 hour, and the mixture was stirred for another 0.5 hour. The solid precipitate was collected by filtration, and this solid was washed with diethylether and dried under reduced pressure to give the title compound (16.38 g, measured).

¹H-NMR (400 MHz, DMSO-d₆)δ: 7.93 (1H, dd, J = 8.8, 5.6 Hz), 8.09 (1H, dd, J = 8.8, 2.7 Hz), 8.43 (1H, d, J = 5.6 Hz), 8.51 (1H, d-like, J = 2.7 Hz).
MS (ESI)m/z: 109 (M)⁺.

[Referential Example 7] 1-(5-Methoxy-2-pyridyl)-5-phenyl-1H-pyrazole-3-carboxylic acid

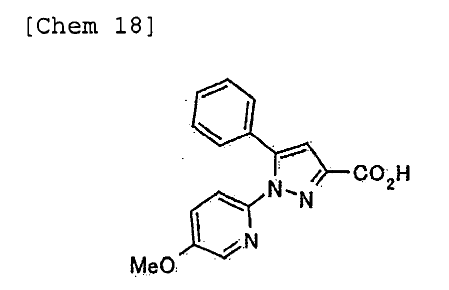

### 1) 5-Amino-2-chloropyridine

Conc. hydrochloric acid (1 ml) was added to a solution of 2-chloro-5-nitropyridine (20 g) in a mixture of ethanol (160 ml) and water (40 ml). Iron which was reduced by hydrogen (70.5 g) was added in several potions to this mixture at room temperature and the mixture was stirred at 90°C for 1 hour. After cooling with air, the reaction liquid was filtered through celite, and the solvent was evaporated under reduced pressure. The residue was purified by chromatography on silica gel (ethyl acetate-hexane) to give the amine derivative (15.2 g, 94%) as a solid.

¹H-NMR (400 MHz, CDCl₃)δ: 3.71 (2H, br s), 6.96 (1H, dd, J = 8.3, 2.9 Hz), 7.08 (1H, d, J = 8.3 Hz), 7.85 (1H, d, J = 2.9 Hz).
LC-MSm/z: 129 (M+H)⁺.

### 2) 5-Acetoxy-2-chloropyridine

To a solution of the 5-amino-2-chloropyridine (18 g) in ethanol (360 ml) was added 48% aqueous tetrafluoroboric acid (40.5 ml), and tert-butyl nitrite (23.5 ml) was added dropwise at-5°C, and the mixture was stirred for 20 minutes. Diethylether was added to the reaction liquid, and the precipitate was collected by filtration and dried to give6-chloropyridine-3-diazoniumtetrafluoroborate (32 g, measured). A solution of this diazonium salt (32 g) in anhydrous acetic acid (160 ml) was gradually heated to 90°C, and the solution was stirred for 45 minutes. After cooling with air, the reaction solvent was evaporated under reduced pressure, and to the residue was added ethyl acetate and water, then the phases were separated. The organic layer was washed with water and brine in this order, and dried over anhydrous magnesium sulfate. After filtration, the solvent was evaporated under reduced pressure, and the residue was purified by chromatography on silica gel (hexane-ethyl acetate) to give 5-acetoxy-2-chloropyridine (10 g, 42%) as a solid.

¹H-NMR (400 MHz, CDCl₃)δ: 2.33 (3H, s), 7.34 (1H, d, J = 8.8 Hz), 7.47 (1H, dd, J = 8.8, 2.9 Hz), 8.21 (1H, d, J = 2.9 Hz).
LC-MSm/z: 172 (M+H)⁺.

### 3) 2-Chloro-5-hydroxypyridine

Potassium carbonate (400 mg) was added to a solution of the 5-acetoxy-2-chloropyridine (10 g) in methanol (200 ml), and the mixture was stirred at room temperature for 20 hours. The reaction solvent was evaporated under reduced pressure, and the residue was purified by chromatography on silica gel (ethyl acetate) to give 2-chloro-5-hydroxypyridine (6.86 g, 91%) as a solid.

¹H-NMR (400 MHz, DMSO-d₆)δ: 7.24 (1H, dd, J = 8.8, 2.9 Hz), 7.29 (1H, d, J = 8.8 Hz), 7.91 (1H, d, J = 2.9 Hz), 10.22 (1H, br).
LC-MSm/z: 130 (M+H)⁺.

### 4) 2-Chloro-5-methoxypyridine

To a solution of the 2-chloro-5-hydroxypyridine (1.30 g) and methyl iodide (1.25 ml) in N,N-dimethylformamide (26 ml) was added dropwise 28% solution of sodium methoxide in methanol (2.0 ml), and the mixture was stirred at room temperature for 1.5 hours. Saturated aqueous ammonium chloride and ethyl acetate were added to the reaction liquid, then the phases were separated. The organic layer was washed with brine and dried over anhydrous magnesium sulfate. After filtration, the solvent was evaporated under reduced pressure, and the residue was purified by chromatography on silica gel (hexane-ethyl acetate) to give 2-chloro-5-methoxypyridine (1.40 g, 98%) as a solid.

¹H-NMR (400 MHz, CDCl₃)δ: 3.85 (3H, s), 7.17-7.25 (2H, m), 8.05 (1H, d, J = 2.9 Hz).
LC-MSm/z: 144 (M+H)⁺.

### 5) 2-Hydrazino-5-methoxypyridine

A solution of the2-chloro-5-methoxypyridine (4.0 g) in hydrazine monohydrate (30 ml) was stirred at 100°C for 24 hours. After cooling with air, the reaction solvent was evaporated under reduced pressure. To the residue was added chloroform and 1N aqueous sodium hydroxide and the phases were separated. The organic layer was dried over anhydrous magnesium sulfate. After filtration,__the solvent was evaporated under reduced pressure to give 2-hydrazino-5-methoxypyridine (705 mg, 18%) as an oily product.
LC-MSm/z: 140 (M+H)⁺.

### 6)Ethyll-(5-methoxy-2-pyridyl)-5-phenyl-1H-pyrazole-3-carboxylate

A solution of the 2-hydrazino-5-methoxypyridine (705 mg) and the ethyl 2, 4-dioxo-4-phenyl butanoate Referential Example 5(1) (1.12 g) in ethanol (25 ml) was heated under reflux for 19 hours. After cooling with air, the reaction solvent was evaporated under reduced pressure. Ethyl acetate and saturated aqueous sodium bicarbonate were added to the residue and the phases were separated. The organic layer was washed with brine, and dried over anhydrous magnesium sulfate. After filtration, the solvent was evaporated under reduced pressure, and the residue was purified by chromatography on silica gel (hexane-ethyl acetate) to give ethyl 1-(5-methoxy-2-pyridyl)-5-phenyl-1H-pyrazole-3-carboxylate(705mg, 43%) as an amorphous product.

¹H-NMR (400 MHz, CDCl₃)δ: 1.42 (3H, t, J = 7.1 Hz), 3.88 (3H, s), 4.45 (2H, q, J = 7.1 Hz), 7.03 (1H, s), 7.22-7.32 (6H, m), 7.45 (1H, d, J = 6.8 Hz), 8.05 (1H, d, J = 3.1 Hz).
LC-MSm/z: 324 (M+H)⁺.

### 7) The title compound

To a solution of the ethyl 1-(5-methoxy-2-pyridyl)-5-phenyl-1H-pyrazole-3-carboxylate(700mg) in methanol (7 ml) and tetrahydrofuran (7 ml) was added 1N aqueous sodium hydroxide (3.5 ml), and the mixture was stirred at room temperature for 2 hours. To the reaction liquid was added 1N aqueous hydrochloric acid (3.6ml) under ice cooling, and water and ethyl acetate were added and phases were separated. The organic layer was washed with brine, and dried over anhydrous magnesium sulfate. After filtration, the solvent was evaporated under reduced pressure to give the title compound (602 mg, 94%) as a solid.

¹H-NMR (400 MHz, CDCl₃)δ: 3.89 (3H, s), 7.09 (1H, s), 7.23-7.35 (6H, m), 7.46 (1H, d, J = 6.9 Hz), 8.08 (1H, d, J = 3.1 Hz).
LC-MSm/z: 296 (M+H)⁺.

[Referential Example 8] 1-(5-Methoxy-2-pyridyl)-5-(2-pyridyl)-1H-pyrazole-3-carboxylic acid

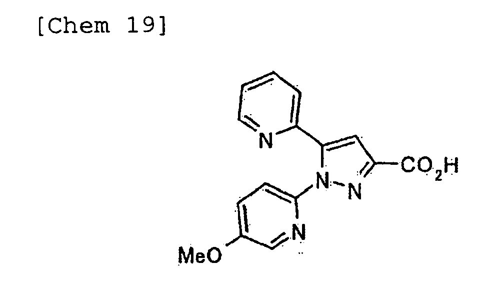

### 1) 5-bromo-2-hydrazinopyridine

Hydrazine monohydrate (10 ml) was added to a solution of 2,5-dibromopyridine (10.0g) in pyridine (100ml) at room temperature, and the mixture was heated under reflux for 13 hours. After cooling with air, the reaction solvent was evaporated under reduced pressure. To the residue were added 0.5N aqueous sodium hydroxide and chloroform and phases were separated. The organic layer was dried over anhydrous sodium sulfate. After filtration, the solvent was evaporated under reduced pressure to give 5-bromo-2-hydrazinopyridine (7.61 g, 96%) as a solid.

¹H-NMR (400 MHz, DMSO-d₆)δ: 6.67 (1H, d, J = 9.0 Hz), 7.55 (1H, dd, J = 9.0, 2.4 Hz), 7.64 (1H, s), 8.00 (1H, d, J = 2.4 Hz).
EI-MSm/z: 188 (M⁺).

### 2) Ethyl 1-(5-bromo-2-pyridyl)-5-(2-pyridyl)-1H-pyrazole-3-carboxylate

To a suspension of the 5-bromo-2-hydrazinopyridine (7.12 g) and the ethyl 4-(2-pyridyl)-2,4-dioxobutanoate of Referential Example 4 (8.38 g) in ethanol (126 ml) was added acetic acid (8.67 ml) at room temperature, and the mixture was heated under reflux for 12 hours. After cooling with air, saturated aqueous sodium bicarbonate and ethyl acetate were added to the reaction liquid. The phases were separated, and the organic layer was dried over anhydrous sodium sulfate. After filtration, the solvent was evaporated under reduced pressure, and the residue was purified by column chromatography on silica gel (hexane-ethyl acetate) to give the dihydropyrazole derivative. To a solution of this dihydropyrazole derivative in ethanol (146 ml) was added conc. hydrochloric acid (4.9 ml) at room temperature, and the mixture was heated under reflux for 3 hours. After cooling with air, saturated aqueous sodium bicarbonate and ethyl acetate were added to the reaction liquid. The phases were separated, and the organic layer was dried over anhydrous sodium sulfate. After filtration, the solvent was evaporated under reduced pressure, and the residue was purified by column chromatography on silica gel (hexane-ethyl acetate) to give ethyl 1-(5-bromo-2-pyridyl)-5-(2-pyridyl)-1H-pyrazole-3-carboxylate (11.6 g, 82%) as a solid.

¹H-NMR (400 MHz, CDCl₃)δ: 1.42 (3H, t, J = 7.2 Hz), 4.45 (2H, q, J = 7.2 Hz), 7.20 (1H, s), 7.23-7.25 (1H, m), 7.49 (1H, dd, J = 7.8, 0.7 Hz), 7.72-7.75 (2H, m), 7.95-7.97 (1H, m), 8.26 (1H, d, J = 2.2 Hz), 8.45-8.46 (1H, m).
EI-MSm/z: 373 (M⁺).

### 3) The title compound

Sodium methoxide (1.74 g) and copper (I) bromide (0.231 g) were added to a solution of the ethyl 1-(5-bromo-2-pyridyl)-5-(2-pyridyl)-1H-pyrazole-3-carboxylate (3.00 g) in a mixture of methanol (30 ml) and toluene (30 ml) at room temperature under argon atmosphere, and the mixture was heated under reflux for 47 hours. After cooling with air, water (50 ml) was added to the reaction liquid at room temperature, and the mixture was stirred for 1 hour and 30 minutes. To the reaction liquid were added water, acetic acid (10 ml), and a mixed solvent of methanol and chloroform (1:10), then the phases were separated. The organic layer was dried over anhydrous sodium sulfate. After filtration, the solvent was evaporated under reduced pressure to give the title compound (1.68 g, 71%) as a solid.

¹H-NMR (400MHz, DMSO-d₆)δ: 4.17 (3H, s), 7.56-8.71 (8H, m), 13.35 (1H, s).
FAB-MSm/z: 297 (M+H)⁺.

[Referential Example 9] 1-(6-Methoxy-3-pyridazinyl)-5-(2-pyridyl)-1H-pyrazole-3-carboxylic acid

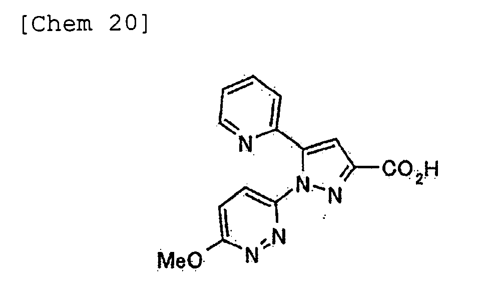

### Method A

### 1) Ethyl 1-(6-chloro-3-pyridazinyl)-5-(2-pyridyl)-1H-pyrazole-3-carboxylate

A solution of 3-chloro-6-hydrazinopyridazine (1.59 g) and the ethyl 4-(2-pyridyl)-2,4-dioxobutanoate (2.45g) of Referential Example 4 in ethanol (60 ml) was heated under reflux for 6 hours, and after adding conc. hydrochloric acid (1 ml) to the reaction liquid, the mixture was heated under reflux for another 1 hour. After cooling with air, the reaction solvent was evaporated under reduced pressure. Ethyl acetate and saturated aqueous sodium bicarbonate were added to the residue and the phases were separated. The organic layer was washed with brine, and dried over anhydrous magnesium sulfate. After filtration, the solvent was evaporated under reduced pressure, and the residue was purified by chromatography on silica gel (ethyl acetate-hexane) to give ethyl 1-(6-chloro-3-pyridazinyl)-5-(2-pyridyl)-1H-pyrazole-3-carboxylat e (1.50 g, 41%) as a solid.

¹H-NMR (400 MHz, CDCl₃)δ: 1.44 (3H, t, J = 7.0 Hz), 4.46 (2H, q, J = 7.0 Hz), 7.23 (1H, s), 7.24-7.27 (1H, m), 7.62-7.65 (1H, m), 7.69 (1H, d, J = 9.0 Hz), 7.76-7.81 (1H, m), 8. 10 (1H, d, J = 9.0 Hz), 8.40 (1H, d, J = 4.6 Hz).
LC-MSm/z: 330 (M+H)⁺.

### 2) Methyl 1-(6-methoxy-3-pyridazinyl)-5-(2-pyridyl)-1H-pyrazole-3-carboxylate

To a solution of the ethyl 1-(6-chloro-3-pyridazinyl)-5-(2-pyridyl)-1H-pyrazole-3-carboxylat e (1.50 g) inmethanol (45ml) was added 28% solution of sodiummethoxide in methanol (3 ml), and the mixture was heated under reflux for 2 hours. After cooling with air, the reaction liquid was evaporated under reduced pressure. Ethyl acetate and saturated aqueous sodium bicarbonate was added to the residue, then the phases were separated. The organic layer was dried over anhydrous magnesium sulfate. After filtration, the solvent was evaporated under reduced pressure, and the residue was purified by chromatography on silica gel (ethyl acetate-hexane) to give methyl 1-(6-methoxy-3-pyridazinyl)-5-(2-pyridyl)-1H-pyrazole-3-carboxyla te (480 mg, 34%) as a solid.

¹H-NMR (400 MHz, CDCl₃)δ: 3.99 (3H, s), 4.10 (3H, s), 7.15 (1H, d, J =9.3 Hz), 7.21-7.23 (1H, m), 7.24 (1H, s), 7.58-7.61 (1H, m), 7.73-7.78 (1H, m), 7.93 (1H, d, J = 9.3 Hz), 8.40-8.41 (1H, m).
LC-MSm/z: 312 (M+H)⁺.

### 3) The title compound

To a solution of the methyl 1-(6-methoxy-3-pyridazinyl)-5-(2-pyridyl)-1H-pyrazole-3-carboxyla te (475 mg) in ethanol (10 ml) and tetrahydrofuran (10 ml) was added 1N aqueous sodium hydroxide (3 ml), and the mixture was stirred at room temperature for 20 hours. To the reaction liquid was added 1N aqueous hydrochloric acid (3 ml) for neutralization under ice cooling, and a mixed solvent of chloroform and methanol (10:1) was added to the reaction liquid. And then the phases were separated. The organic layer was dried over anhydrous magnesium sulfate. After filtration, the solvent was evaporated under reduced pressure to give the title compound (300 mg, 66%) as a solid.

¹H-NMR (400 MHz, DMSO-d₆)δ: 4.04 (3H, s), 7.32-7.35 (1H, m), 7.41 (1H, s), 7.49 (1H, d, J = 9.3 Hz), 7.80-7.82 (1H, m), 7.87-7.91 (1H, m), 7.99 (1H, d, J = 9.3 Hz), 8.35-8.36 (1H, m).
LC-MSm/z: 298 (M+H)⁺.

### Method B

### 1) Methyl 4-(2-pyridyl)-2,4-dioxobutanoate

Under argon atmosphere, a solution of 2-acetylpyridine (2.56 g) in methanol (26 ml) was added to a solution of dimethyl oxalate (5.00 g) and sodium methoxide (2.29 g) in methanol (26 ml) at room temperature, and the mixture was stirred for 15 minutes. The mixture was stirred for another 45 minutes at 60°C. After cooling with air, water and diethylether were added to the reaction solution, then the aqueous layer was separated. To the aqueous layer were added saturated aqueous ammonium chloride and chloroform, then the phases were separated. The organic layer was dried over anhydrous sodium sulfate. After filtration, the solvent was evaporated under reduced pressure to give methyl 4-(2-pyridyl)-2,4-dioxobutanoate (3.44 g, 79%) as a solid.

¹H-NMR (400 MHz, CDCl₃)δ: 3.94 (3H, s), 7.54-7.50 (1H, m), 7.64 (1H, s), 7.93-7.89 (1H, m), 8.19-8.16 (1H, m), 8.74-8.72 (1H, m).
EI-MSm/z: 207 (M⁺).

### 2) Methyl 1-(6-chloro-3-pyridazinyl)-5-(2-pyridyl)-1H-pyrazole-3-carboxylate

A solution of the methyl 4-(2-pyridyl) -2, 4-dioxobutanoate (4.143 g) and 3-chloro-6-hydrazinopyridazine (2.891 g) in methanol (100 ml) was heated under reflux for 109 hours. To the reaction liquid was added conc. hydrochloric acid (2 ml), and the mixture was heated under reflux for another 6 hours. After cooling with air, saturated aqueous sodium bicarbonate and ethyl acetate were added to the reaction liquid, then the phases were separated. The organic layer was washed with water and brine and dried over anhydrous sodium sulfate. After filtration, the solvent was evaporated under reduced pressure to give methyl
1-(6-chloro-3-pyridazinyl)-5-(2-pyridyl)-1H-pyrazole-3-carboxylat e (3.169 g, 50%) as a solid.

¹H-NMR (400 MHz, CDCl₃)δ: 4.00 (3H, s), 7.24-7.28 (1H, m), 7.24 (1H, s), 7.64 (1H, dt, J = 7.8, 1.2 Hz), 7.70 (1H, d, J = 9.0 Hz), 7.79 (1H, td, J = 7.8, 1.7 Hz), 8.09 (1H, d, J = 9.0 Hz), 8.38-8.41 (1H, m).
ESI-MSm/z: 316 (M+H)⁺.

### 3) Methyl 1-(6-methoxy-3-pyridazinyl)-5-(2-pyridyl)-1H-pyrazole-3-carboxylate

Sodium methoxide (1.530 g) was added to a solution of the methyl 1-(6-chloro-3-pyridazinyl)-5-(2-pyridyl)-1H-pyrazole-3-carboxylat e (2.981 g) in methanol (190 ml) at room temperature, and the mixture was stirred for 19 hours. To the reaction liquid was added 1N aqueous hydrochloric acid (19 ml), and methanol was evaporated under reduced pressure. Water was added to the residue, and the insoluble content was collected by filtration and dried to give methyl 1-(6-methoxy-3-pyridazinyl)-5-(2-pyridyl)-1H-pyrazole-3-carboxyla te (2.571 g, 87%) as a solid.

### 4) The title compound

1N aqueous sodium hydroxide (15 ml) was added to a solution of the methyl 1-(6-methoxy-3-pyridazinyl)-5-(2-pyridyl)-1H-pyrazole-3-carboxylate (2.20g) in a mixture of methanol (30 ml) and tetrahydrofuran (30 ml) at room temperature, and the mixture was stirred for 2.5 hours. To the reaction liquid was added a mixed solvent of 1N aqueous hydrochloric acid (15 ml) for neutralization under ice cooling, and a mixed solvent of chloroform-methanol (10:1) was added to the reaction solution. The phases were separated. The organic layer was dried over anhydroussodiumsulfate. After filtration, the solvent was evaporated under reduced pressure, and isopropylether was added to the residue. The precipitated solid was collected by filtration to give the title compound (1.42 g, 47.6%).

[Referential Example 10] 5-(4-Dimethylaminophenyl)-1-(6-methoxy-3-pyridazinyl)-1H-pyrazole -3-carboxylic acid

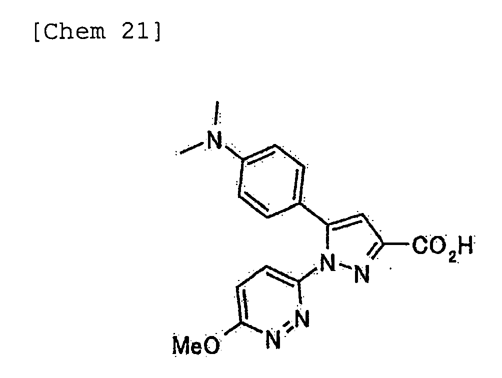

### 1) Methyl 4-(4-dimethylaminophenyl)-2,4-dioxobutanoate

The procedure of Referential Example 9, Method B (1) was repeated by using 4'-dimethylaminoacetophenone (1.224 g), dimethyl oxalate (1.771 g) and sodium methoxide (180 mg) to give methyl 4-(4-dimethylaminophenyl)-2,4-dioxobutanoate (742mg, 39%) as a solid.

¹H-NMR (400 MHz, CDCl₃)δ: 3.10 (6H, s), 3.93 (3H, s), 6.69 (2H, d, J = 9.0 Hz), 7.01 (1H, s), 7.92 (2H, d, J = 9.0 Hz).
ESI-MSm/z: 250 (M+H)⁺.

### 2) Methyl 1-(6-chloro-3-pyridazinyl)-5-(4-dimethylaminophenyl)-1H-pyrazole-3-carboxylate

A solution of the methyl 4-(4-dimethylaminophenyl)-2,4-dioxobutanoate (742 mg) and 3-chloro-6-hydrazinopyridazine (473 mg) inmethanol (30ml) was heated under reflux for 18 hours. After cooling with air, the reaction solvent was evaporated under reduced pressure, and to the residue was added saturated aqueous sodium bicarbonate and chloroform, then the phases were separated. The organic layer was dried over anhydrous sodium sulfate. After filtration, the solvent was evaporated under reduced pressure, and the residue was purified by column chromatography on silica gel (chloroform-methanol) to give methyl 1-(6-chloro-3-pyridazinyl)-5-(4-dimethylaminophenyl)-1H-pyrazole-3-carboxylate (679 mg, 63%) as a solid.

¹H-NMR (400 MHz, CDCl₃)δ: 2.98 (6H, s), 3.98 (3H, s), 6.65 (2H, d, J = 8.8 Hz), 6.97 (1H, s), 7.16 (2H, d, J = 8.8 Hz), 7.62 (1H, d, J = 9.0 Hz), 7.90 (1H, d, J = 9.0 Hz).
ESI-MSm/z: 358 (M+H)⁺.

### 3) The title compound

Sodium methoxide (307 mg) was added to a solution of the methyl 1-(6-chloro-3-pyridazinyl)-5-(4-dimethylaminophenyl)-1H-pyrazole-3-carboxylate (679 mg) in methanol (50 ml), and the mixture was heated under reflux for 13 hours. Water (205 µl) was added to the reaction liquid, and the mixture was heated under reflux for another 6 hours. After cooling with air, the reaction solvent was evaporated under reduced pressure, and water and ethyl acetate were added to the residue, then the aqueous layer was separated. To the aqueous layer was added 1N aqueous hydrochloric acid (3.8 ml) for neutralization, and chloroform was added to the reaction solution. The phases were separated. The organic layer was dried over anhydrous sodium sulfate. After filtration, the solvent was evaporated under reduced pressure to give the title compound (592 mg, 91%) as a solid.

¹H-NMR (400 MHz, CDCl₃)δ: 2.97 (6H, s), 4.16 (3H, s), 6.64 (2H, d, J = 8.8 Hz), 7.01 (1H, s), 7.07 (1H, d, J = 9.0 Hz), 7.15 (2H, d, J = 8.8 Hz), 7.60 (1H, d, J = 9.0 Hz).
ESI-MSm/z: 340 (M+H)⁺.

[Referential Example 11] 1-(5-Methoxy-2-pyrazinyl)-5-(2-pyridyl)-1H-pyrazole-3-carboxylic acid

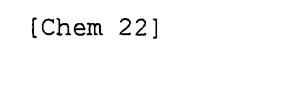

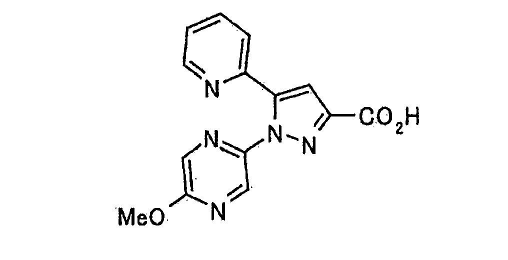

### 1) 5-Chloro-2-hydrazinopyrazine

A solution of 5-chloro-2-hydroxpyrazine (1.84 g) synthesized from aminopyrazine by the method of Palamidessi et al. (J.Org.Chem., vol.29, pp 2491-2492, 1964) in phosphorus oxychloride (28ml) was placed in a sealed tube, and the solution was stirred at an outer temperature of 130°C for 6 hours. After cooling with air, ice cold water and dichloromethane were added to the reaction liquid, then the phases were separated. The organic layer was dried over anhydrous sodium sulfate. After filtration, the solvent was evaporated under reduced pressure. Hydrazine monohydrate (1.39 ml) was added to a solution of the residue in ethanol (14 ml), and the mixture was stirred at room temperature for 150 minutes and for another 15 minutes at 80°C. After cooling with air, the reaction liquid was evaporated under reduced pressure, and to the residue was added water and a mixed solvent of chloroform and methanol (1:10), then the phases were separated. The organic layer was dried over anhydrous sodium sulfate. After filtration, the solvent was evaporated under reduced pressure to give 5-chloro-2-hydrazinopyrazine(0.325 g, 16%) as a solid.

¹H-NMR (400 MHz, DM_{S}O-d₆)δ: 4.32 (2H, br s), 7.92 (1H, s), 7.99 (1H, s), 8.13 (1H, s).
EI-MSm/z: 144 (M⁺).

### 2) Methyl 1-(5-chloro-2-pyrazinyl)-5-(2-pyridyl)-1H-pyrazole-3-carboxylate

The procedure of Referential Example 9, Method B (2) was repeated by using the methyl 4-(2-pyridyl)-2,4-dioxobutanoate (0.414 g) of Referential Example 9, Method B(1) and the 5-chloro-2-hydrazinopyrazine (0.289 g) to give methyl 1-(5-chloro-2-pyrazinyl)-5-(2-pyridyl)-1H-pyrazole-3-carboxylate (0.260 g, 41%) as a solid.

¹H-NMR (400 MHz, CDCl₃)δ: 4.00 (3H, s), 7.25-7.28 (2H, m), 7.59-7.61 (1H, m), 7.77-7.81 (1H, m), 8.25 (1H, m), 8.39-8.41 (1H, m), 8.84-8.85 (1H, m).
FAB-MSm/z: 316 (M+H)⁺.

### 3) The title compound

Sodium methoxide (0.13 g) was added to a solution of the methyl 1-(5-chloro-2-pyrazinyl)-5-(2-pyridyl)-1H-pyrazole-3-carboxylate (0.254g) in methanol (5.1ml), and the mixture was heated under reflux for 70 minutes. After cooling with air, water (5.1 ml) was added, and the mixture was stirred at room temperature for 10 minutes. 1N aqueous hydrochloric acid (2. 41 ml), water, and chloroform were added to the reaction liquid, then the phases were separated. The organic layer was evaporated under reduced pressure to give the title compound (0.237 g, 99%) as a solid.

¹H-NMR (400 MHz, DMSO-d₆)δ: 3.98 (3H, s), 7.29-7.32 (1H, m), 7.37 (1H, s), 7.74-7.87 (2H, m), 8.11 (1H, s), 8.33-8.34 (1H, m), 8.52 (1H, s), 13.15 (1H, br s).
FAB-MSm/z: 298 (M+H)⁺.

[Referential Example 12] Ethyl 1-(6-Methyl-3-pyridyl)-5-(2-pyridyl)-1H-pyrazole-3-carboxylate

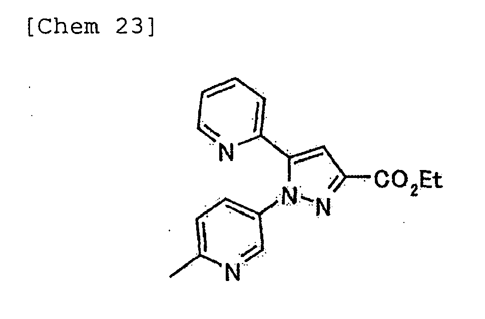

### 1) 5-Hydrazino-2-methylpyridine

To a solution of 6-methylnicotinic acid (5.13 g) in dioxane (75 ml) were added triethylamine (5.7 ml), diphenylphosphorylazide (8.8 ml), and tert-butanol (7.1 ml) at room temperature, and the mixture was stirred at 100°C for 19 hours. After cooling with air, the reaction solvent was evaporated under reduced pressure, and chloroform and water were added to the residue and the phases were separated. The organic layer was washed with brine, and dried over anhydrous sodium sulfate. After filtration, the solvent was evaporated under reduced pressure, and the residue was purified by column chromatography on silica gel (chloroform-methanol) to give 5-(tert-butoxycarbonyl)amino-2-methylpyridine (6.79g, 87%) as a solid. The procedure of Referential Example 2 was repeated by using this compound (5.179g) to give 5-hydrazino-2-methylpyridine (0.84 g, 32%) as a solid.

¹H-NMR (400 MHz, CD₃OD)δ: 2.38 (3H, s), 7.06 (1H, d, J = 8.3 Hz), 7.21 (1H, dd, J = 8.3, 2.5 Hz), 7.99 (1H, d, J = 2.5 Hz).
LC-MSm/z: 124 (M+H)⁺.

### 2) The title compound

The procedure of Referential Example 5 (2) was repeated by using the 5-hydrazino-2-methylpyridine (1.20 g) and the ethyl 4-(2-pyridyl)-2,4-dioxobutanoate (3.48 g) of Referential Example 4 to give the title compound (0.459 g, 15%) as an oily product.

¹H-NMR (400 MHz, CDCl₃)δ: 1.43 (3H, t like, J = 7.3 Hz), 2.60 (3H, s), 4.46 (2H, q, J= 7.3 Hz), 7.20-7.50 (4H, m), 7.67-7.80 (2H, m), 8.39 (1H, br), 8.51 (1H, br).
FAB-MSm/z: 309 (M+H)⁺.

[Referential Example 13] Lithium 1-(6-methoxy-3-pyridyl)-5-(3-pyridazinyl)-1H-pyrazole-3-carboxylate

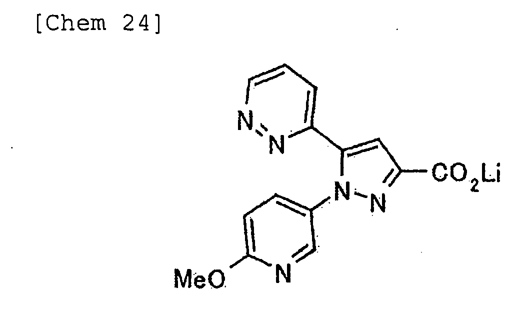

### 1) Methyl 4-(3-pyridazinyl)-2,4-dioxobutanoate

A 1.0M solution of lithium bis(trimethylsilyl)amide in tetrahydrofuran (19 ml) was added dropwise to a solution of 3-acetylpyridazine (2.097 g) in tetrahydrofuran (50 ml) under argon atmosphere at -78°C, and the mixture was stirred for 1 hour. A solution of dimethyl oxalate (4.055 g) in tetrahydrofuran (35 ml) was added dropwise to the reaction liquid, and the mixture was stirred at 0°C for 2 hours. The reaction solvent was evaporated under reduced pressure, and water and diethylether were added to the residue, then the aqueous layer was separated. The aqueous layer was then acidified with 1N aqueous hydrochloric acid, and the solution was extracted with ethyl acetate. The organic layer was dried over anhydrous sodium sulfate.
After filtration, the solvent was evaporated under reduced pressure to give methyl 4-(3-pyridazinyl)-2,4-dioxobutanoate (2.63 g, 73%) as a solid.

¹H-NMR (400 MHz, CDCl₃)δ: 3.97 (3H, s), 7.73 (1H, dd, J = 8.5, 5.1 Hz), 7.96 (1H, s), 8.28 (1H, dd, J = 8.5, 1.8 Hz), 9.38 (1H, dd, J = 5.1, 1.8 Hz).
ESI-MSm/z: 209 (M+H)⁺.

### 2) Methyl 1-(6-methoxy-3-pyridyl)-5-(3-pyridazinyl)-1H-pyrazole-3-carboxylate

The 5-hydrazino-2-methoxypyridine (726 mg) of Referential Example 2 was added to a solution of the methyl 4-(3-pyridazinyl)-2,4-dioxobutanoate (1.086 g) in methanol (10 ml), and the mixture was heated under reflux for 4 hours. After cooling with air, the reaction solvent was evaporated under reduced pressure, and the residue was purified by thin layer chromatography on silica gel (ethyl acetate) to give methyl 1-(6-methoxy-3-pyridyl)-5-(3-pyridazinyl)-1H-pyrazole-3-carboxyla te (309 mg, 19%) as a solid.

¹H-NMR (400 MHz, CDCl₃)δ: 3.95 (3H, s), 4.00 (3H, s), 6.80 (1H, d, J = 8.8 Hz), 7.43 (1H, s), 7.51 (2H, d, J = 3.4 Hz), 7.70 (1H, dd, J = 8.8, 2.7 Hz), 8.11 (1H, d, J = 2.7 Hz), 9.15 (1H, t, J = 3.4 Hz). ESI-MSm/z: 312 (M+H)⁺.

### 3) The title compound

Lithium hydroxide monohydrate (42 mg) was added to a solution of the methyl 1-(6-methoxy-3-pyridyl)-5-(3-pyridazinyl)-1H-pyrazole-3-carboxyla te (309 mg) in methanol (20 ml), and the mixture was heated under reflux for 18 hours. After cooling with air, the reaction solvent was evaporated under reduced pressure to give the title compound (322 mg, measured) as an amorphous product.
ESI-MSm/z: 298 (M+H)⁺.

[Referential Example 14] 5-(5-Benzyloxy-2-pyridyl)-1-(6-methoxy-3-pyridyl)-1H-pyrazole-3-c arboxylic acid

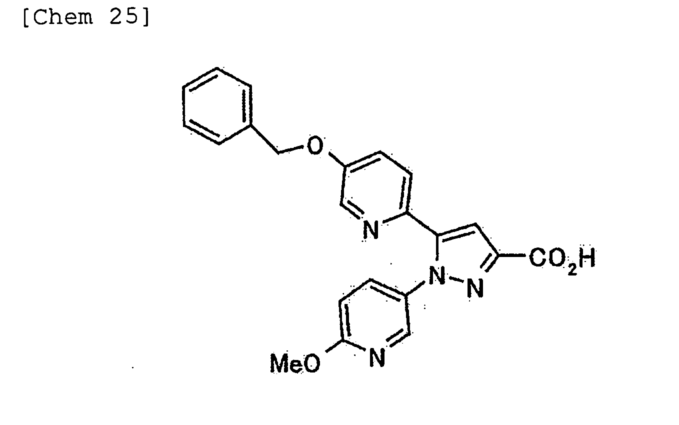

### 1) 5-Benzyloxy-2-methylpyridine

Benzyl bromide (10.9 ml) was added to a solution of 3-hydroxy-6-methylpyridine (10.0 g) and potassium carbonate (38.0) inacetonitrile (200ml) at room temperature, and the mixture was stirred for 12 hours. Water and ethyl acetate were added to the reaction liquid, then the phases were separated. The organic layer was dried over anhydrous sodium sulfate. After filtration, the solvent was evaporated under reduced pressure, and the residue was purified by column chromatography on silica gel (ethyl acetate-hexane) to give 5-benzyloxy-2-methylpyridine (4.14 g, 23%) as an oily product.

¹H-NMR (400 MHz, CDCl₃)δ: 2.48 (3H, s), 5.08 (2H, s), 7.05 (1H, d, J = 8.5 Hz), 7.16 (1H, dd, J = 8.5, 2.9 Hz), 7.31-7.43 (5H, m), 8.26 (1H, d, J = 2.9 Hz).
EI-MS m/z: 199 (M⁺).

### 2) 1-(5-Benzyloxy-2-pyridyl)ethanone

Selenium dioxide (9.20 g) was added to a solution of the 5-benzyloxy-2-methylpyridine (4.13 g) in pyridine (83 ml) at room temperature, and the mixture was heated under reflux for 61 hours. After cooling with air, water and chloroform were added to the reaction liquid, then the phases were separated. The organic layer was dried over anhydrous magnesium sulfate. After filtration, the solvent was evaporated under reduced pressure, and triethylamine (6.35 ml) was added to a solution of the residue, N, O-dimethyl hydroxylamine hydrochloride (2.22 g), 1-ethyl-3-(3-dimethylaminopropyl)carbodiimide hydrochloride (4.37 g), and 1-hydroxybenzotriazole (3.08 g) in N,N-dimethylformamide (95 ml) at room temperature, and the mixture was stirred for 61 hours. Water and ethyl acetate were added to the reaction liquid and the phases were separated, and the organic layer was dried over anhydrous sodium sulfate. After filtration, the solvent was evaporated under reduced pressure, and the residue was purified by column chromatography on silica gel (ethyl acetate-hexane) to give 5-benzyloxypyridine-2-carboxylic acid methoxymethylamide (3.75 g, 66%) as an oily product. (FAB-MSm/z: 273(M+H)⁺.)
Under argon atmosphere, a 1.10M solution of methyl lithium in diethylether (13.7 ml) was added dropwise to a solution of the 5-benzyloxypyridine-2-carboxylic acid methoxymethylamide (3.74 g) in tetrahydrofuran (75 ml) at 0°C, and the mixture was stirred for 40 minutes. Water and ethyl acetate were added to the reaction liquid and the phases were separated, and the organic layer was dried over anhydroussodiumsulfate. After filtration, the solvent was evaporated under reduced pressure, and the residue was purified by column chromatography on silica gel (ethyl acetate-hexane) to give 1-(5-benzyloxy-2-pyridyl)ethanone (1.47 g, 47%) as an oily product.

¹H-NMR (400 MHz, CDCl₃)δ: 2.67 (3H, s), 5.18 (2H, s), 7.30-7.45 (6H, m), 8.03 (1H, d, J = 8.8 Hz), 8.39 (1H, d, J = 2.7 Hz).
EI-MS m/z: 227 (M⁺).

### 3) Ethyl 4-(5-benzyloxy-2-pyridyl)-2,4-dioxobutanoate

A solution of diethyl oxalate (1.75 ml) and the 1-(5-benzyloxy-2-pyridyl)ethanone (1.46 g) in ethanol (15 ml) were added to a solution of sodium ethoxide (0.874 g) in ethanol (15 ml) under argon atmosphere, and the mixture was stirred at room temperature for 7 hours and for another 1 hour at 60°C. After cooling with air, sodium ethoxide (0.874 g) and diethyl oxalate (1.75 ml) were added to the reaction liquid, and the mixture was stirred at 60°C for 1 hour. After cooling with air, water and diehylether were added to the reaction liquid, and the aqueous layer was separated. To the aqueous layer were added saturated aqueous ammonium chloride and chloroform, and the phases were separated. The organic layer was dried over anhydrous sodium sulfate. After filtration, the solvent was evaporated under reduced pressure to give ethyl 4- (5-benzyloxy-2-pyridyl) -2, 4-dioxobutanoate (1.38 g, 66%) as a solid.

¹H-NMR (400 MHz, CDCl₃)δ: 1.38-1.42 (3H, m), 4.35-4.42 (2H, m), 5.20 (2H, s), 7.35-7.44 (6H, m), 7.59 (1H, s), 8.14 (1H, d, J = 8.8 Hz), 8.44 (1H, d, J = 2.7 Hz).
EI-MSm/z: 327 (M⁺).

### 4) Ethyl 5-(5-benzyloxy-2-pyridyl)-1-(6-methoxy-3-pyridyl)-1H-pyrazole-3-carboxylate

Acetic acid (0.958 ml) was added to a solution of the ethyl 4-(5-benzyloxy-2-pyridyl)-2,4-dioxobutanoate (1.37 g) and the 5-hydrazino-2-methoxypyridine (0.699 g) of Referential Example 2 in ethanol (27 ml), and the mixture was heated under reflux for 12 hours. After cooling with air, saturated aqueous sodium bicarbonate and ethyl acetate were added to the reaction liquid and the phases were separated, and the organic layer was dried over anhydrous sodium sulfate. After filtration, the solvent was evaporated under reduced pressure, and the residue was purified by column chromatography on silica gel (ethyl acetate-hexane) to give ethyl 5-(5-benzyloxy-2-pyridyl)-1-(6-methoxy-3-pyridyl)-1H-pyrazole-3-c arboxylate (1.50 g, 83%) as a solid.

¹H-NMR (400 MHz, CDCl₃)δ: 1.42 (3H, t, J = 7.1 Hz), 3.95 (3H, s), 4.45 (2H, q, J = 7.1 Hz), 5.10 (2H, s), 6.76 (1H, d, J = 8.8 Hz), 7.18-7.42 (8H, m), 7.66 (1H, dd, J = 8.8, 2.7 Hz), 8.10 (1H, d, J = 2.7 Hz), 8.28 (1H, d, J = 2.7 Hz).
FAB-MSm/z: 431 (M+H)⁺.

### 5) The title compound

1N sodium hydroxide (8.65 ml) was added to a solution of the ethyl 5-(5-benzyloxy-2-pyridyl)-1-(6-methoxy-3-pyridyl)-1H-pyrazole-3-c arboxylate (1.49g) in a mixture of methanol (30 ml) and tetrahydrofuran (30 ml) at room temperature, and the mixture was stirred for 90 minutes. After evaporation of the reaction solvent under reduced pressure, the residue was dissolved in water and chloroform, and 1N aqueous hydrochloric acid (8.65 ml) was added to the solution, and the phases were separated. The organic layer was dried over anhydrous sodium sulfate. After filtration, the solvent was evaporated under reduced pressure to give the title compound (1.27 g, 91%) as a solid.

¹H-NMR (400 MHz, CDCl₃)δ: 3.95 (3H, s), 5.11 (2H, s), 6.75-6.78 (1H, m), 7.22-7.41 (8H, m), 7.66 (1H, dd, J = 8.8, 2.7 Hz), 8.11 (1H, dd, J = 2.7, 0.7 Hz), 8.30 (1H, dd, J = 2.7, 0.7 Hz).
EI-MSm/z: 402 (M⁺).

[Referential Example 15] 5-(3-Dimethylaminophenyl)-1-(6-methoxy-3-pyridyl)-1H-pyrazole-3-c arboxylic acid

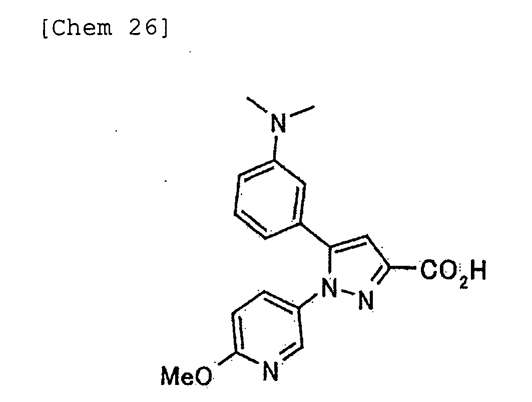

### 1) Ethyl 5-(3-dimethylaminophenyl)-1-(6-methoxy-3-pyridyl)-1H-pyrazole-3-carboxylate

A solution of 1-(3-dimethylaminophenyl)-1-ethanone (1.63 g) in ethanol (20 ml) and diethyl oxalate (3.10 ml) were added to a solution of sodium ethoxide (1.63 g) in ethanol (20 ml), and the mixture was stirred at room temperature for 1 hour. To the reaction liquid was added the 5-hydrazino-2-methoxypyridine hydrochloride (2.52 g) of Referential Example 1, and the mixture was heated under reflux for 14.5hours. After cooling with air, the reaction solvent was evaporated under reduced pressure. To the residue were added ethyl acetate and saturated aqueous sodium bicarbonate, and the phases were separated, and the organic layer was dried over anhydrous sodium sulfate. After filtration, the solvent was evaporated under reduced pressure, and the residue was purified by column chromatography on silica gel (ethyl acetate-hexane) to give ethyl 5-(3-dimethylaminophenyl)-1-(6-methoxy-3-pyridyl)-1H-pyrazole-3-c arboxylate (3.30 g, 90%) as an oily product.

¹H-NMR (400 MHz, CDCl₃)δ: 1.43 (3H, t, J = 7.1 Hz), 2.87 (6H, s), 3.93 (3H, s), 4.46 (2H, q, J = 7.1 Hz), 6.50 (1H, d, J = 7.6 Hz), 6.54-6.55 (1H, m), 6.69 (1H, dd, J = 8.3, 2.4 Hz), 6.73 (1H, d, J = 8.8 Hz), 7.03 (1H, s), 7.16 (1H, dd, J = 8.1, 7.8 Hz), 7.59 (1H, dd, J = 8.8, 2.7 Hz), 8.15 (1H, d, J = 2.7 Hz).
ESI-MSm/z: 367 (M+H)⁺.

### 2) The title compound

1N aqueous sodium hydroxide (22.5 ml) was added to a solution of the ethyl 5-(3-dimethylaminophenyl)-1-(6-methoxy-3-pyridyl)-1H-pyrazole-3-c arboxylate (3.30 g) in methanol (70 ml), and the mixture was stirred at room temperature for 3.5 hours. The solid precipitate was collected by filtration to give sodium salt of the title compound (1.55 g, 47%). After evaporation of the methanol under reduced pressure, water was added to the residue. 1N aqueous hydrochloric acid (22.5ml) and ethyl acetate were added to the solution, and the phases were separated, and the organic layer was dried over anhydrous magnesium sulfate. After filtration, the solvent was evaporated under reduced pressure to give the title compound (1.56 g, 51%) as an amorphous product.

¹H-NMR (400 MHz, CDCl₃)δ: 2.88 (6H, s), 3.94 (3H, s), 6.50-6.52 (1H, m), 6.55-6.56 (1H, m), 6.71 (1H, dd, J = 8.3, 2.7 Hz), 6.74 (1H, d, J = 8.8 Hz), 7.09 (1H, s), 7.18 (1H, dd, J = 8.3, 7.8 Hz), 7.59 (1H, dd, J = 8.8, 2.7 Hz), 8.16 (1H, d, J = 2.7 Hz).
ESI-MSm/z: 339 (M+H)⁺.

[Referential Example 16] 1-(6-Methoxy-3-pyridazinyl)-5-(4-methyl-2-pyridyl)-1H-pyrazole-3-carboxylic acid

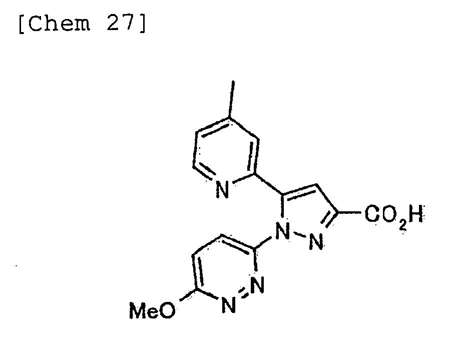

### 1) 1-(4-methyl-2-pyridyl)ethanone

A 1.58M solution of n-butyllithium in hexane (17 ml) was added dropwise over 10 minutes to a solution of 2-bromo-4-picoline (3.0 g) in diethylether (45 ml) at -78°C, and the solution was stirred for 20 minutes. N,N-dimethyl acetamide (2.5 ml) was added dropwise to the reaction liquid, and the temperature of the reaction liquid was gradually elevated to room temperature, and the mixture was stirred for 2 hours. Water and ethyl acetate were added to the reaction liquid and the phases were separated, and the organic layer was dried over anhydrous magnesium sulfate. After filtration, the solvent was evaporated under reduced pressure, and the residue was purified by column chromatography on silica gel (hexane-ethyl acetate) to give 1-(4-methyl-2-pyridyl)ethanone (1.64 g, 70%) as an oily product.

¹H-NMR (400 MHz, CDCl₃)δ: 2.38 (3H, s), 2.66 (3H, s), 7.23 (1H, dd, J = 4.88, 0.86 Hz), 7.81 (1H, d, J = 0.86 Hz), 8.48 (1H, d, J = 4.88 Hz).

### 2) Ethyl 4-(4-methyl-2-pyridyl)-2,4-dioxobutanoate

Diethyl oxalate (3.3ml) was added to a solution of sodium ethoxide (1.66 g) in ethanol (50 ml) at room temperature, and the mixture was stirred for 10 minutes. The 1-(4-methyl-2-pyridyl)ethanone (1. 64 g) was then added and the mixture was stirred for 30 minutes. After water and diethylether were added to the reaction liquid, the aqueous layer was separated. The aqueous layer was adjusted to pH 2 with 1N aqueous hydrochloric acid, and chloroform was added to the solution, and the phases were separated. The organic layer was dried over anhydrous magnesium sulfate. After filtration, the solvent was evaporated under reduced pressure to give ethyl 4-(4-methyl-2-pyridyl)-2,4-dioxobutanoate (2.35 g, 82%) as a solid.

¹H-NMR (400 MHz, CDCl₃)δ: 1.40 (3H, t, J=7.08Hz), 2.49 (3H, s), 4.39 (2H, q, J = 7.08 Hz), 7.38 (1H, d, J = 4.88 Hz), 7.47 (1H, br), 8.01 (1H, s), 8.60 (1H, d, J = 4.88 Hz).
ESI-MSm/z: 236 (M+H)⁺.

### 3) Ethyl 1-(6-chloro-3-pyridazinyl)-5-(9-methyl-2-pyridyl)-1H-pyrazole-3-carboxylate

The solution of the ethyl 4-(4-methyl-2-pyridyl)-2,4-dioxobutanoate (2.35 g) and 3-chloro-6-hydrazinopyridazine (1.9 g) in ethanol (100 ml) was heated under reflux for 30 minutes. After cooling with air, conc. hydrochloric acid (5 ml) was added to the solution, and the mixture was again heated under reflux for 1 hour. After cooling with air, saturated aqueous sodium bicarbonate and ethyl acetate were added to the reaction liquid and the phases were separated, and the organic layer was dried over anhydrous magnesium sulfate. After filtration, the solvent was evaporated under reduced pressure, and purified by column chromatography on silica gel (hexane-ethyl acetate) to give ethyl 1-(6-chloro-3-pyridazinyl)-5-(4-methyl-2-pyridyl)-1H-pyrazole-3-carboxylate (1.19 g, 35%) as an amorphous product.

¹H-NMR (400 MHz, CDCl₃)δ: 1.43 (3H, t, J = 7.08 Hz), 2.41 (3H, s), 4.48 (2H, q, J = 7.08 Hz), 7.07 (1H, m), 7.21 (1H, s), 7.47 (1H, s), 7.70 (1H, d, J = 9.03 Hz), 8.09 (1H, d, J = 9.03 Hz), 8.23 (1H, d, J = 5.13 Hz).
ESI-MSm/z: 344 (M+H)⁺.

### 4) The title compound

Sodium methoxide (380 mg) was added to a solution of the ethyl 1-(6-chloro-3-pyridazinyl)-5-(4-methyl-2-pyridyl)-1H-pyrazole-3-c arboxylate (1.19 g) in methanol (30 ml) at room temperature, and the mixture was stirred for 19.5 hours. 1N aqueous hydrochloric acid and a mixed solvent of chloroform and methanol were added to the reaction liquid and the phases were separated, and the organic layer was dried over anhydrous magnesium sulfate. After filtration, the solvent was evaporated under reduced pressure to give the title compound (560 mg, 52%) as a solid.

¹H-NMR (400 MHz, DMSO-d₆)δ: 2.37 (3H, s), 4.02 (3H, s), 7.20 (1H, d, J = 4.88 Hz), 7.37 (1H, s), 7.46 (1H, d, J = 9.28 Hz)., 7.72 (1H, s), 7.96 (1H, d, J = 9.28 Hz), 8.21 (1H, d,4.88 Hz).
ESI-MSm/z: 312 (M+H)⁺.

[Referential Example 17] 5-(4-Dimethylaminophenyl)-1-(6-methoxy-3-pyridyl)-1H-pyrazole-3-carboxylic acid

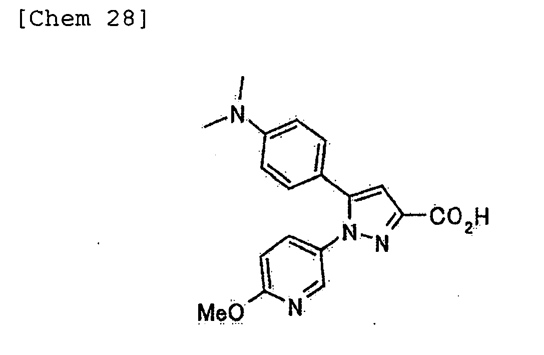

### 1) Ethyl 5-(4-dimethylaminophenyl)-1-(6-methoxy-3-pyridyl)-1H-pyrazole-3-carboxylate

To a solution of sodium ethoxide (1.36 g) in ethanol (50 ml) was added a solution of diethyl oxalate (2.72 ml) and 4'-dimethylaminoacetophenone (1.632 g) in ethanol (50 ml), and the mixture was heated under reflux for 16 hours. After cooling with air, 5-hydrazino-2-methoxypyridine hydrochloride (2.102 g) of Referential Example 1 was added to the solution and the mixture was heated under reflux for 3 hours. After cooling with air, the reaction solvent was evaporated under reduced pressure. Water and ethyl acetate were added to the residue and the phases were separated, and the organic layer was washed with brine and dried over anhydrous sodium sulfate. After filtration, the solvent was evaporated under reduced pressure, and the residue was purified by column chromatography on silica gel (ethyl acetate-hexane) to give ethyl 5-(4-dimethylaminophenyl)-1-(6-methoxy-3-pyridyl)-1H-pyrazole-3-c arboxylate (2.134 g, 58%) as an oily product.

¹H-NMR (400 MHz, CDCl₃)δ: 1.42 (3H, t, J = 7.1 Hz), 2.97 (6H, s), 3.94 (3H, s), 4.45 (2H, q, J = 7.1 Hz), 6.62 (2H, d, J= 8.8 Hz), 6.73 (1H, d, J = 8.8 Hz), 6.94 (1H, s), 7.06 (2H, d, J = 8.8 Hz), 7.58 (1H, dd, J = 8.8, 2.7 Hz), 8.16 (1H, d, J = 2.7 Hz).
ESI-MSm/z: 367 (M+H)⁺.

### 2) The title compound

The procedure of Referential Example 15 (2) was repeated by using the ethyl 5-(4-dimethylaminophenyl)-1-(6-methoxy-3-pyridyl)-1H-pyrazole-3-c arboxylate (2.134 g) to give the title compound (1.777 g, 90%) as a solid.

¹H-NMR (400 MHz, CDCl₃)δ: 2.98 (6H, s), 3.96 (3H, s), 6.63 (2H, d, J = 8.8 Hz), 6.75 (1H, d, J = 8.8 Hz), 6.99 (1H, s), 7.07 (2H, d, J = 8.8 Hz), 7.58 (1H, dd, J = 8.8, 2.7 Hz), 8.18 (1H, d, J = 2.7 Hz). ESI-MSm/z: 339 (M+H)⁺.

[Referential Example 18] 5-(5-Benzyloxy-2-pyridyl)-1-(3-pyridyl)-1H-pyrazole-3-carboxylic acid

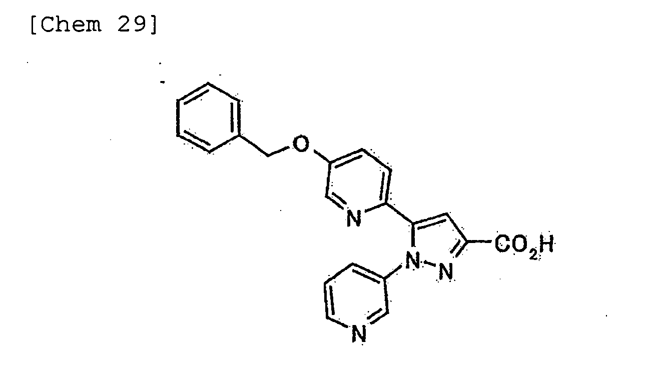

### 1) 3-Hydrazinopyridine

The procedure of Referential Example 2 was repeated by using 3-aminopyridine (13.0 g) to give 3-hydrazinopyridine (12.5 g, 83%) as a solid.

¹H-NMR (400 MHz, DMSO-d₆)δ: 4.02 (2H, br s), 6.89 (1H, br s), 7.04-7.12 (2H, m), 7.76-7.78 (1H, m), 8.08 (1H, m).
EI-MSm/z: 109 (M⁺).

### 2) Ethyl 5-(5-benzyloxy-2-pyridyl)-1-(3-pyridyl)-1H-pyrazole-3-carboxylate

The procedure of Referential Example 14 (4) was repeated by using the ethyl 4-(5-benzyloxy-2-pyridyl)-2,4-dioxobutanoate (7.61 g) of Referential Example 14 (3) and the 3-hydrazinopyridine (3.04 g) to give ethyl 5-(5-benzyloxy-2-pyridyl)-1-(3-pyridyl)-1H-pyrazole-3-carboxylate (7.38 g, 79%) as a solid.

¹H-NMR (400 MHz, CDCl₃)δ: 1.41-1.44 (3H, m), 4.43-4.49 (2H, m), 5.10 (2H, s), 7.20 (1H, s), 7.25 (1H, dd, J = 8.8, 2.9 Hz), 7.35-7.41 (7H, m), 7.82-7.85 (1H, m), 8.23 (1H, d, J = 2.9 Hz), 8.52 (1H, m), 8.59 (1H, dd, J = 4.9, 1.5 Hz).
FAB-MSm/z: 401 (M+H)⁺.

### 3) The title compound

The procedure of Referential Example 14 (5) was repeated by using the ethyl 5-(5-benzyloxy-2-pyridyl)-1-(3-pyridyl)-1H-pyrazole-3-carboxylate (5.16 g) to give the title compound (4.61 g, 96%) as a solid.

¹H-NMR (400 MHz, DMSO-d₆)δ: 5.19 (2H, s), 7.27-7.28 (1H, m), 7.35-7.54 (7H, m), 7.70 (1H, d, J = 8.8 Hz), 7.78-7.81 (1H, m), 8.19 (1H, d, J = 2.9 Hz), 8.51 (1H, d, J = 2.4 Hz), 8.60-8.61 (1H, m), 13.10 (1H, br s).
FAB-MSm/z: 373 (M+H)⁺.

[Referential Example 19] 1-(5-Ethylthio-1,3,4-thiadiazol-2-yl)-5-(2-pyridyl)-1H-pyrazole-3 -carboxylic acid

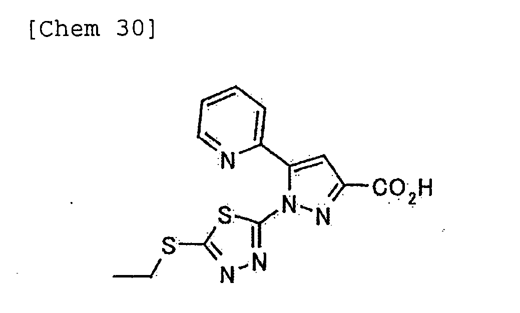

### 1) 2-Chloro-5-ethylthio-1,3,4-thiadiazole

To a solution of copper (0.40 g) in conc. hydrochloric acid (60 ml) was added 2-amino-5-ethylthio-1,3,4-thiadiazole (4.0 g), and a solution of sodium nitrite (1.88 g) in water (17 ml) was gradually added dropwise to the reaction liquid. The mixture was stirred for 10 minutes at 0°C, and for another 4 hours at room temperature. Water and chloroform were added to the reaction liquid and the phases were separated, and the organic layer was dried over anhydrous sodium sulfate. After filtration, the solvent was evaporated under reduced pressure, and the residue was purified by column chromatography on silica gel (chloroform) to give 2-chloro-5-ethylthio-1,3,4-thiadiazole (2.99 g, 67%) as an oily product.

¹H-NMR (400 MHz, CDCl₃)δ: 1.44-1.48 (3H, m), 3.30-3.36 (2H, m).
FAB-MSm/z: 181 (M+H)⁺.

### 2) 5-Ethylthio-2-hydrazino-1,3,4-thiadiazole

Hydrazine monohydrate (10 ml) was added to a solution of the 2-chloro-5-ethylthio-1,3,4-thiadiazole (2.98 g) in ethanol (30 ml) at room temperature, and the mixture was heated under reflux for 1 hour. After cooling with air, the reaction solvent was evaporated under reduced pressure. Water and a mixed solvent of chloroform and methanol were added to the residue, and the phases were separated. The solvent of the organic layer was evaporated under reduced pressure to give 5-ethylthio-2-hydrazino-1,3,4-thiadiazole (2.60 g, 89%) as a solid.

¹H-NMR (400 MHz, CDCl₃)δ: 1.39 (3H, t, J = 7.3 Hz), 3.11 (2H, q, J = 7.3 Hz), 4.39 (2H, br s).
EI-MSm/z: 176 (M⁺).

### 3) Methyl 1-(5-ethylthio-1,3,4-thiadiazol-2-yl)-5-(2-pyridyl)-1H-pyrazole-3 -carboxylate

The procedure of Referential Example 9, Method B (2) was repeated by using the methyl 4-(2-pyridyl)-2,4-dioxobutanoate (3.04 g) of Referential Example 9, Method B(1) and the 5-ethylthio-2-hydrazino-1,3,4-thiadiazole (2.59 g) to give methyl 1-(5-ethylthio-1,3,4-thiadiazol-2-yl)-5-(2-pyridyl)-1H-pyrazole-3 -carboxylate (1.41 g, 28%) as a solid.

¹H-NMR (400 MHz, CDCl₃)δ: 1.43-1.47 (3H, m), 3.29-3.34 (2H, m), 3.98 (3H, m), 7.20 (1H, m), 7.30-7.33 (1H, m), 7.63-7.65 (1H, m), 7.76-7.81 (1H, m), 8.56-8.58 (1H, m).
EI-MSm/z: 347 (M⁺) .

### 4) The title compound

1N sodium hydroxide (6.98 ml) was added to a solution of the methyl 1-(5-ethylthio-1,3,4-thiadiazol-2-yl)-5-(2-pyridyl)-1H-pyrazole-3 -carboxylate (0.970 g) in a mixture of methanol (9.7 ml) and tetrahydrofuran (9.7ml) at room temperature, and the mixture was stirred for 30 minutes. To the reaction liquidwas added 1N aqueous hydrochloric acid (6.98 ml) and a mixed solvent of chloroform and methanol, and the phases were separated, and the solvent of the organic layer was evaporated under reduced pressure to give the title compound (0.929 g, 100%) as a solid.

¹H-NMR (400 MHz, DMSO-d₆)δ: 1.38 (3H, t, J = 7.3 Hz), 3.32 (2H, q, J =7.2Hz), 7.37 (1H, s), 7.40-7.43 (1H, m), 7.83-7.85 (1H, m), 7.90-7.94 (1H, m), 8.50-8.51 (1H, m), 13.48 (1H, br s).
EI-MSm/z: 333 (M⁺).

[Referential Example 20] Ethyl 5-(4-carbamoyl-2-pyridyl)-1-(6-methoxy-3-pyridyl)-1H-pyrazole-3-c arboxylate

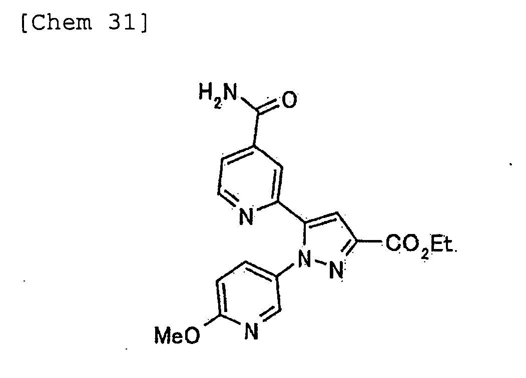

### 1) Ethyl 1-(6-methoxy-3-pyridyl)-5-(4-methyl-2-pyridyl)-1H-pyrazole-3-carboxylate

The procedure of Referential Example 8 (2) was repeated by using the ethyl 4-(4-methyl-2-pyridyl)-2,4-dioxobutanoate (2.83 g) of Referential Example 16 (2) and the 5-hydrazino-2-methoxypyridine (1.67 g) of Referential Example 2 to give ethyl 1-(6-methoxy-3-pyridyl)-5-(4-methyl-2-pyridyl)-1H-pyrazole-3-carboxylate (1.66 g, 41%) as a solid.

¹H-NMR (400 MHz, CDCl₃)δ: 1.43 (3H, t, J = 7.2 Hz), 2.34 (3H, s), 3.94 (3H, s), 4.46 (2H, q, J = 7.2 Hz), 6.76 (1H, d, J = 8. 8 Hz), 7.05-7.06 (1H, m), 7.23-7.24 (2H, m), 7.66-7.69 (1H, m), 8.10 (1H, d, J = 2.8 Hz), 8.36 (1H, d, J = 4.8 Hz).
EI-MSm/z: 338 (M⁺).

### 2) The title compound

Selenium dioxide (0.390 g) was added to a solution of the ethyl 1-(6-methoxy-3-pyridyl)-5-(4-methyl-2-pyridyl)-1H-pyrazole-3-carb oxylate (0.595 g) in pyridine (12 ml), and the mixture was heated under reflux for 24 hours. After coolingwith air, another portion of selenium dioxide (0.390 g) was added, and the mixture was heated under reflux for 6 hours. After cooling with air, water and chloroform were added to the reaction liquid and the phases were separated, and the organic layer was dried over anhydrous sodium sulfate. After filtration, the solvent was evaporated under reduced pressure, and triethylamine (1.50 ml) was added to a solution of the resulting residue, 1-ethyl-3-(3-dimethylaminopropyl)carbodiimide hydrochloride (0.371 g), 1-hydroxybenzotriazole (0.262 g), and ammonium chloride (0.471 g) inN,N-dimethylformamide (12ml) at room temperature, and the mixture was stirred for 14 hours. Water and ethyl acetate were added to the reaction liquid and the phases were separated, and the organic layer was dried over anhydrous sodium sulfate. After filtration, the solvent was evaporated under reduced pressure, and the residue was purified by column chromatography on silica gel (chloroform-methanol) to give the title compound (0.247 g, 38%) as a solid.

¹H-NMR (400 MHz, CDCl₃)δ: 1.43 (3H, t, J = 7.2 Hz), 3.95 (3H, s), 4.46 (2H, q, J = 7.2 Hz), 6.76-6.78 (1H, m), 7.33 (1H, s), 7.54-7.56 (1H, m), 7.65-7.68 (1H, m), 7.85-7.86 (1H, m), 8.08-8.09 (1H, m), 8.61-8.62 (1H, m).
FAB-MSm/z: 368 (M+H)⁺.

[Referential Example 21] Methyl 3-(N-tert-butyl)aminopropionate

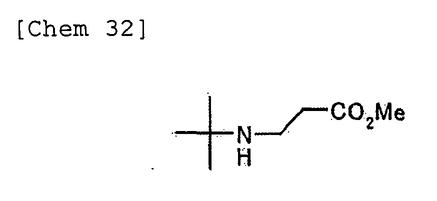

A mixture of tert-butylamine (1.4 ml) and methyl acrylate (1.5 ml) was stirred at 70°C for 17 hours. After cooling with air, the product was used for the subsequent reaction without purification.

¹H-NMR (400 MHz, CDCl₃)δ: 1.10 (9H, s), 2.49 (2H, t, J = 6.6 Hz), 2.82 (2H, t, J = 6.6 Hz), 3.67 (3H, s).
ESI-MSm/z: 160 (M+H)⁺.

[Referential Example 22] 1-(6-Methoxy-3-pyridyl)-5-(2-pyrazinyl)-1H-pyrazole-3-carboxylic acid

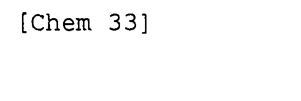

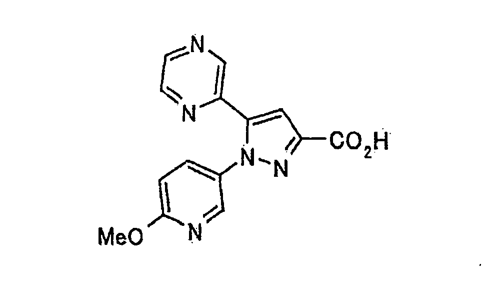

A 1.0M solution of lithium bis(trimethylsilyl)amide in tetrahydrofuran (11.0 ml) was added to a solution of 1-(2-pyrazinyl)-1-ethanone (1.22g) intetrahydrofuran (10ml) at-78°C, and the mixture was stirred for 55 minutes. Diethyl oxalate (2.05 ml) was then added to the solution and the temperature of the mixture was gradually elevated to room temperature. After stirring the mixture for 6.5 hours, 1N aqueous hydrochloric acid (11 ml), water, and diethylether were added to the reaction liquid and the phases were separated. The aqueous layer was then saturated with sodium chloride and extracted with ethyl acetate, and the combined organic layers were evaporated under reduced pressure to give crude ethyl 4-(2-pyrazinyl)-2,4-dioxobutanoate (1.83 g, 82%) as a solid. To a suspension of this crude product (1.58 g) in ethanol (20 ml) was added a solution prepared by adding triethylamine (1.9 ml) to a suspension of the 5-hydrazino-2-methoxypyridine hydrochloride (1.50 g) of Referential Example 1 in ethanol (80 ml), and the mixture was heated under reflux for 19 hours. Acetic acid (5 ml) was added to the reaction liquid, and the mixture was heated under reflux for 1.5 days. After cooling with air, ethyl acetate and saturated aqueous sodium bicarbonate was added to the reaction liquid and the phases were separated, and the organic layer was dried over anhydrous sodium sulfate. After filtration, the solvent was evaporated under reduced pressure, and the residue was purified by column chromatography on silica gel (ethyl acetate-hexane) to give ethyl 1-(6-methoxy-3-pyridyl)-5-(2-pyrazinyl)-1H-pyrazole-3-carboxylate (1.05 g, 45%) as a solid. 1N aqueous sodium hydroxide (10.0 ml) was added to a solution of this ethyl 1H-pyrazole-3-carboxylate derivative (1.05g) in ethanol (30 ml) at room temperature for 16 hours. 1N aqueous hydrochloric acid (15 ml), water, and ethyl acetate were added to the reaction liquid and the phases were separated, and the organic layer was dried over anhydrous magnesium sulfate. After filtration, the solvent was evaporated under reduced pressure to give the crude product of the title compound (0.883 g, 92%) as a solid. This crude product was used in the subsequent reaction without further purification.

[Referential Example 23] 1-(6-Methoxy-3-pyridyl)-5-(1-methyl-1H-2-pyrrolyl)-1H-pyrazole-3-carboxylic acid

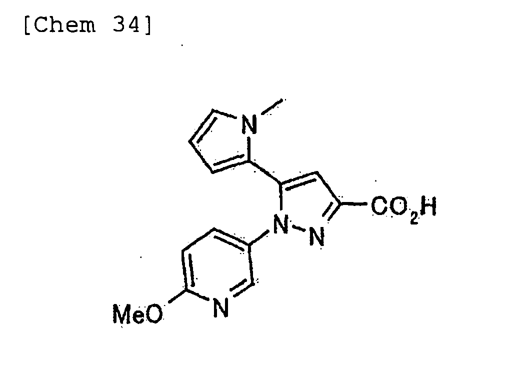

The procedure of Referential Example 22 was repeated by using 1-(1-methyl-1H-2-pyrrolyl)-1-ethanone (1.19 ml) to give crude product of the title compound (2.57 g, quantative) as an amorphous product.
[Referential Example 24] 5-(4-Cyanophenyl)-1-(3-pyridyl)-1H-pyrazole-3-carboxylic acid

### 1) Ethyl 4-(4-bromophenyl)-2,4-dioxobutanoate

The procedure of Referential Example 16 (2) was repeated by using 4-bromoacetophenone (8.50 g), diethyl oxalate (11.6 ml), and sodium ethoxide to give ethyl 4- (4-bromophenyl) -2, 4-dioxobutanoate (10.4 g, 81%) as a solid.

¹H-NMR (400 MHz, CDCl₃)δ: 1.40-1.43 (3H, m), 4.38-4.43 (2H, m), 7.03 (1H, s), 7.63-7.67 (2H, m), 7.84-7.87 (2H, m).
FAB-MSm/z: 299, 301 (M+H)⁺.

### 2) Ethyl 5-(4-bromophenyl)-1-(3-pyridyl)-1H-pyrazole-3-carboxylate

The procedure of Referential Example 8 (2) was repeated by using the ethyl 4-(4-bromophenyl)-2,4-dioxobutanoate (7.00 g) and the 3-hydrazinopyridine (2.55 g) of Referential Example 18 (1) to give ethyl 5-(4-bromophenyl)-1-(3-pyridyl)-1H-pyrazole-3-carboxylate (3.83 g, 44%) as a solid.

¹H-NMR (400 MHz, CDCl₃)δ: 1.44 (3H, t, J = 7.1 Hz), 4.47 (2H, q, J = 7.1 Hz), 7.07-7.10 (3H, m), 7.34-7.38 (1H, m), 7.47-7.51 (2H, m), 7.71-7.74 (1H, m), 8.57-8.58 (1H, m), 8. 62 (1H, dd, J = 4.8, 1. 6 Hz). EI-MSm/z: 371, 373 (M⁺).

### 3) Ethyl 5-(4-cyanophenyl)-1-(3-pyridyl)-1H-pyrazole-3-carboxylate

A solution of the ethyl 5-(4-bromophenyl)-1-(3-pyridyl)-1H-pyrazole-3-carboxylate (1.50g), zinc cyanide (0.284g), and tetrakis (triphenylphosphine) palladium (0) (0.233 g) in N,N-dimethylformamide (30 ml) was stirred at 100°C for 18 hours under argon atmosphere. After cooling with air, zinc cyanate (2.35 g) and tetrakis (triphenylphosphine) palladium (0) (1. 864 g) were added and the mixture was stirred at 100°C for 30 hours. After cooling with air, 2N aqueous ammonia solution, ethyl acetate and chloroform were added to the reaction liquid and the phases were separated, and the organic layer was washed with water and dried over anhydrous sodium sulfate. After filtration, the solvent was evaporated under reduced pressure, and the residue was purified by column chromatography on silica gel (ethyl acetate-chloroform) to give ethyl 5-(4-cyanophenyl)-1-(3-pyridyl)-1H-pyrazole-3-carboxylate(0.460g, 36%) as an amorphous product.

¹H-NMR (400 MHz, CDCl₃)δ: 1.44 (3H, t, J = 7.2 Hz), 4.48 (2H, q, J = 7.2 Hz), 7.16 (1H, s), 7.33-7.36 (2H, m), 7.39 (1H, dd, J = 8.2, 4.8 Hz), 7.64-7.67 (2H, m), 7.73-7.76 (1H, m), 8.56 (1H, d, J = 2.4 Hz), 8.66 (1H, dd, J = 4.8, 1. 6 Hz).
EI-MSm/z: 318 (M⁺).

### 4) The title compound

A solution of lithium hydroxide monohydrate (66.0 mg) in water (4.5 ml) was added to a suspension of the ethyl 5-(4-cyanophenyl)-1-(3-pyridyl)-1H-pyrazole-3-carboxylate (0.455 g) in tetrahydrofuran (9 ml) at room temperature, and the mixture was stirred for 80 minutes. To the reaction liquid were added 1N aqueous hydrochloric acid (1.65 ml) and a mixed solvent of chloroform and methanol (10:1), and the phases were separated, and the solvent of the organic layer was evaporated under reduced pressure to give the title compound (0.384 g, 93%) as a solid.

¹H-NMR (400 MHz, DMSO-d₆)δ: 7.27 (1H, m), 7.48-7.55 (3H, m), 7.82-7.87 (3H, m), 8.56 (1H, d, J = 2.4 Hz), 8.65-8.66 (1H, m), 13.18 (1H, br s).
EI-MSm/z: 290 (M⁺).

[Referential Example 25] 5-(5-Chloro-2-pyridyl)-1-(3-pyridyl)-1H-pyrazole-3-carboxylic acid

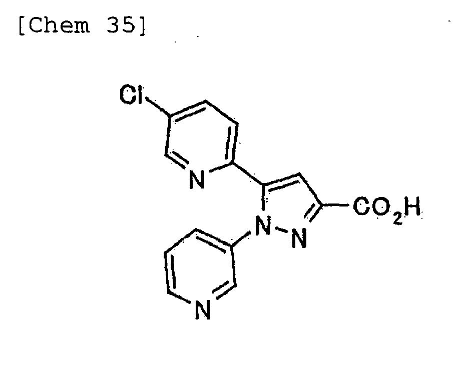

### 1) 2-Bromo-5-chloropyridine

Bromine (12 ml) was added to a solution of 2-amino-5-chloropyridine (5g) in 47% hydrobromic acid (50 ml) at 0°C, and a solution of sodium nitrite (15 g) in water (20 ml) was added dropwise to this reaction liquid. After stirring the mixture for 1 hour, a solution of sodium hydroxide (32 g) in water (80 ml) and ethyl acetate to the reaction liquid, and the phases were separated. The organic layer was dried over anhydrous sodium sulfate. After filtration, the solvent was evaporated under reduced pressure to give 2-bromo-5-chloropyridine (6.8 g, 91%) as a solid.

¹H-NMR (400 MHz, CDCl₃)δ: 7.44 (1H, d, J = 8.42 Hz), 7.54 (1H, m), 8.36 (1H, s).

### 2) 1-(5-chloro-2-pyridyl)ethanone

A 1.56M solution of n-butyllithium in hexane (27 ml) was added dropwise to a solution of the 2-bromo-5-chloropyridine (6.8 g) in diethylether (45 ml) at-78°C, and thereafter, N,N-dimethyl acetamide (5 ml) was added dropwise to the solution. The mixture was stirred for 30 minutes. To the reaction liquid was added saturated aqueous ammonium chloride and ethyl acetate, and the phases were separated. The organic layer was dried over anhydrous sodium sulfate. After filtration, the solvent was evaporated under reduced pressure, and the residue was purified by column chromatography on silica gel (hexane-ethyl acetate) to give 1-(5-chloro-2-pyridyl)ethanone (3.26 g, 59%) as a solid.

¹H-NMR (400 MHz, CDCl₃)δ: 2.70 (3H, s), 7.80 (1H, dd, J = 8.42, 2.32 Hz), 8.00 (1H, d, J = 8.42 Hz), 8.62 (1H, d, J = 2.32 Hz).

### 3) Ethyl 4-(5-chloro-2-pyridyl)-2,4-dioxobutanoate

Dimethyl oxalate (5 g) was added to a solution of sodium methoxide (2.26 g) in ethanol (100 ml), and the mixture was stirred for 5 minutes. 1- (5-Chloro-2-pyridyl) ethanone (3.26 g) was then added to the solution and the mixture was stirred at room temperature for 45 minutes. After water and diethylether were added to the reaction liquid, the aqueous layer was separated. The aqueous layer was acidified with 1N aqueous hydrochloric acid, and chloroform was added to the solution and the phases were separated. The organic layer was dried over anhydrous sodium sulfate. After filtration, the solvent was evaporated under reduced pressure to give ethyl 4-(5-chloro-2-pyridyl)-2,4-dioxobutanoate (4.12 g, 77%) as a solid.

¹H-NMR (400 MHz, CDCl₃)δ: 1.42 (3H, t, J = 7.08 Hz), 4.41 (2H, q, J = 7.08 Hz), 7.64 (1H, s), 7.87 (1H, dd, J = 8.42, 2.44 Hz), 8.11 (1H, d, J = 8.42 Hz), 8.67 (1H, d, J = 2.44 Hz).
ESI-MSm/z: 256 (M+H)⁺.

### 4) Ethyl 5-(5-chloro-2-pyridyl)-1-(3-pyridyl)-1H-pyrazole-3-carboxylate

Acetic acid (5 ml) was added a solution of the ethyl 4-(5-chloro-2-pyridyl)-2,4-dioxobutanoate (2.59 g) and the 3-hydrazinopyridine (1.2 g) of Referential Example 18(1) in ethanol (100 ml), and the mixture was heated under reflux for 16.5 hours. After cooling with air, water and ethyl acetate were added to the reaction liquid and the phases were separated. The organic layer was washed with brine, and dried over anhydrous sodium sulfate. After filtration, the solvent was evaporated under reduced pressure, and the residue was purified by column chromatography on silica gel (methanol-chloroform) to give ethyl 5-(5-chloro-2-pyridyl)-1-(3-pyridyl)-1H-pyrazole-3-carboxylate (1.5 g).

¹H-NMR (400 MHz, CDCl₃)δ: 1.43 (3H, t, J = 7.08 Hz), 4.46 (2H, q, J = 7. 08 Hz), 7.29 (1H, s), 7.39 (1H, dd, J = 8.30, 4. 88 Hz), 7. 42 (1H, d, J = 8.30 Hz), 7.71 (1H, dd, J = 8.42, 2.44 Hz), 7.83 (1H, ddd, J = 8.42, 2.44, 1.59 Hz), 8.41 (1H, d, J = 1.59 Hz), 8.54 (1H, d, J = 2.44 Hz), 8.62 (1H, dd, J = 4.88, 1.59 Hz).
ESI-MSm/z: 329 (M+H)⁺.

### 5) The title compound

Sodium methoxide (573 mg) was added to a solution of the ethyl 5-(5-chloro-2-pyridyl)-1-(3-pyridyl)-1H-pyrazole-3-carboxylate (500 mg) in methanol (10 ml), and the mixture was stirred at room temperature for 17.5 hours. To the reaction liquidwere added 1N aqueous hydrochloric acid and chloroform, and the phases were separated, and the organic layer was dried over anhydrous sodium sulfate. After filtration, the solvent was evaporated under reduced pressure to give the title compound (348 mg, 76%) as a solid.

¹H-NMR (400 MHz, CD₃OD/CDCl₃)δ: 7.30 (1H, s), 7.33 (1H, s), 7.56 (1H, m), 7.84 (2H, m), 8.38 (1H, m), 8.57 (2H, m).
ESI-MSm/z: 301 (M+H)⁺.

[Referential Example 26] 1-Amino-1-cyclopentanecarboxamide trifluoroacetate salt

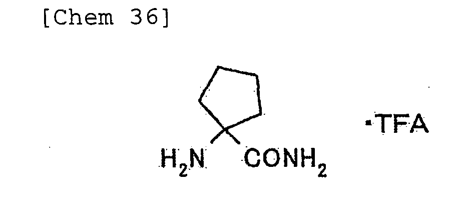

Di-tert-butyl dicarbonate (12.0 g) and 6N aqueous sodium hydroxide (9 ml) were added to a solution of 1-amino-1-cyclopentanecarboxylic acid (6.45 g) in tetrahydrofuran (60 ml), and the mixture was stirred at room temperature for 20 hours. The reaction solution was acidified (weak acidic) with conc. hydrochloric acid, and dichloromethane was added to the solution, and the phases were separated. The organic layer was dried over anhydrous magnesium sulfate. After filtration, the solvent was evaporated under reduced pressure to give 1-tert-butoxycarbonylamino-1-cyclopentanecarboxylic acid. (FAB-MSm/z: 230(M+H)⁺.)
To a solution of the resulting carboxylic acid derivative in dichloromethane (100 ml) was added 1-hydroxybenzotriazole (10.2 g), 1-ethyl-3-(3-dimethylaminopropyl)carbodiimide hydrochloride (14.4 g), 28% aqueous ammonia (5 ml), and triethylamine (11 ml), and the mixture was stirred at room temperature for 16 hours. Water and dichloromethane were added to the reaction liquid and the phases were separated, and the organic layer was dried over anhydrous magnesium sulfate. After filtration, the solvent was evaporated under reduced pressure to give 1-tert-butoxycarbonylamino-1-cyclopentanecarboxamide (2.85 g, 25%). (FAB-MSm/z:229(M+H)⁺.)
Trifluoroacetic acid (4 ml) was added to a solution of the resulting carboxamide derivative (2.85 g) in dichloromethane (20 ml), and the mixture was stirred for 4 hours. The reaction solvent was evaporated under reduced pressure to give the title compound (2.16 g, 71%).
FAB-MSm/z: 129 (M+H)⁺.

[Referential Example 27] 3-Aminopyrrolidine-1-carboxylic acid benzyl ester trifluoroacetate

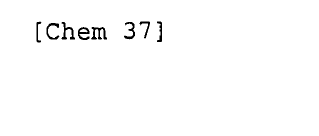

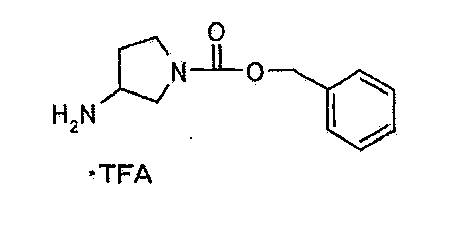

### 1) 3-(N-tert-butoxycarbonyl)aminopyrrolidine-1-carboxylic acid benzyl ester

Benzyl chloroformate (1.43 ml) was added to a solution of pyrrolidine-3-carbamic acid tert-butyl ester (1.862 g) and triethylamine (1.39 ml) in dichloromethane (20 ml) under ice cooling, and the mixture was stirred at room temperature for 2 hours. The reaction solvent was evaporated under reduced pressure, and water and ethyl acetate were added to the residue, and the phases were separated. The organic layer was washed with 5% aqueous citric acid, water, and brine in this order, and dried over anhydrous sodium sulfate. After filtration, the solvent was evaporated under reduced pressure to give 3-(N-tert-butoxycarbonyl)aminopyrrolidine-1-carboxylic acid benzyl ester (2.676 g, 83%) as a solid.

¹H-NMR (400 MHz, CDCl₃) δ: 1.44 (9H, s), 1.74-1.89 (1H, br m), 2.07-2.19 (1H, br m), 3.19-3.31 (1H, br m), 3.42-3.53 (2H, br m), 3.62-3.70 (1H, m), 4.13-4.27 (1H, br), 4.52-4.66 (1H, br), 5.12 (2H, s), 7.25-7.41 (5H, m).

### 2) The title compound

Anisole (2 ml) and trifluoroacetic acid (8 ml) were added to a solution of the 3-(N-tert-butoxycarbonyl)aminopyrrolidine-1-carboxylic acid benzyl ester (2. 67 6 g) in dichloromethane (10 ml), and the mixture was stirred at room temperature for 1 hour. The reaction solvent was evaporated under reduced pressure to give the title compound (2.691 g, 96%) as a solid.

¹H-NMR (400 MHz, DMSO-d₆)δ: 1.89-2.03 (1H, br m), 2.12-2.27 (1H, br m), 3.29-3.75 (5H, m), 3.78-3.87 (1H, br), 5.08 (2H, s), 7.28-7.43 (5H, m), 8.05-8.25 (3H, br s).
ESI-MSm/z: 221 (M+H)⁺.

[Referential Example 28] 2-(N-tert-Butylamino)acetamide

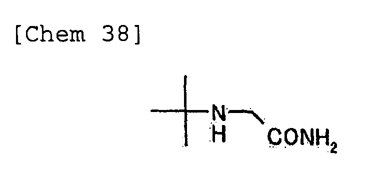

To a solution of tert-butylamine (0.946 ml) in N,N-dimethylformamide (8 ml) was added potassium carbonate (993 mg) and a solution of bromoacetamide (810 mg) in N,N-dimethylformamide (2 ml) under ice cooling, and the mixture was stirred at room temperature for 20 hours. After filtration of the reaction liquid, the solvent was evaporated under reduced pressure, and toluene was added to the residue and the mixture was evaporated together with toluene under reduced pressure to give the title compound (610 mg, 79%) as a solid.

¹H-NMR (400 MHz, CDCl₃)δ: 1.19 (9H, s), 3.24 (2H, s).
LC-MSm/z: 129 (M-H)⁺.

[Referential Example 29] 5-(4-Hydroxymethyl-2-pyridyl)-1-(6-methoxy -3-pyridyl)-1H-pyrazole-3-carboxylic acid

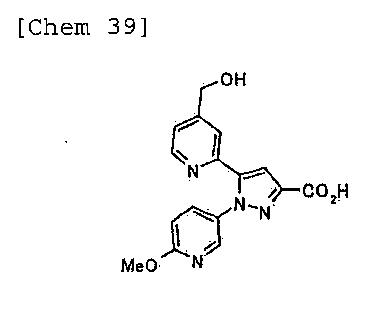

### 1) 2-acetyl-4-methylpyridine

A 1.58M solution of n-butyllithium in hexane (22 ml) was added dropwise to a solution of 2-bromo-4-picoline (4 g) in diethylether (60 ml) over 5 minutes at -78°C, and the mixture was stirred for 5 minutes. N,N-dimethyl acetamide (3.3 ml) was added dropwise to the reaction liquid, and the mixture was stirred at room temperature for 14.5 hours. Water and ethyl acetate were added to the reaction liquid and the phases were separated, and the organic layer was dried over anhydrous magnesium sulfate. After filtration, the solvent was evaporated under reduced pressure, and the residue was purified by column chromatography on silica gel (hexane-ethyl acetate) to give 2-acetyl-4-methylpyridine (1.86 g, 59%) as an oily product.

¹H-NMR (400 MHz, CDCl₃)δ: 2.42 (3H, s), 2.72 (3H, s), 7.29 (1H, dd, J = 4.94, 0.67 Hz), 7.86 (1H, d, J = 0.67 Hz), 8.54 (1H, d, J = 4.94 Hz).

### 2) Ethyl 4-(4-methyl-2-pyridyl)-2,4-dioxobutanoate

The procedure of Referential Example 14 (3) was repeated by using the 2-acetyl-4-methylpyridine (1.86 g) and diethyl oxalate (3.74 ml) to give ethyl 4-(4-methyl-2-pyridyl)-2,4-dioxobutanoate (3.82 g) as a solid.

¹H-NMR (400 MHz, CDCl₃)δ: 1.38 (3H, t, J=7.08Hz), 2.51 (3H, s), 4.36 (2H, q, J = 7.08 Hz), 7.43 (2H, br), 8.03 (1H, s), 8.65 (1H, d, J = 5.01 Hz).
ESI-MSm/z: 236 (M+H)⁺.

### 3) Ethyl 5-(4-methyl-2-pyridyl)-1-(6-methoxy-3-pyridyl)-1H-pyrazole-3-carboxylate

Triethylamine (3.4 ml) was added to a suspension of the ethyl 4-(4-methyl-2-pyridyl)-2,4-dioxobutanoate (3.82 g) and the 5-hydrazino-2-methoxypyridine hydrochloride (5.1 g) of Referential Example 1 in ethanol (70 ml), and the mixture was heated under reflux for 2 hours. After cooling with air, water and chloroform were added to the reaction liquid and the phases were separated, and the organic layer was dried over anhydrous magnesium sulfate. After filtration, the solvent was evaporated under reduced pressure, and the solid precipitate was washed with a mixed solvent of hexane and ethyl acetate (3:1) to give ethyl 5-(4-methyl-2-pyridyl)-1-(6-methoxy-3-pyridyl)-1H-pyrazole-3-carb oxylate. The solvent of the washing solution was evaporated under reduced pressure, and the residue was purified by column chromatography on silica gel (hexane-ethyl acetate) to give ethyl 5-(4-methyl-2-pyridyl)-1-(6-methoxy-3-pyridyl)-1H-pyrazole-3-carboxylate. This product combined with the solid as described above yielded 1.20 g (26%).

¹H-NMR (400 MHz, CDCl₃)δ: 1.43 (3H, t, J = 7.08 Hz), 2.35 (3H, s), 3.94 (3H, s), 4.45 (2H, q, J = 7.08 Hz), 6.75 (1H, dd, J = 8.79, 0.61 Hz), 7.05 (1H, ddd, J = 5.13, 1.59, 0.73 Hz), 7.23 (1H, t, J = 0.73 Hz), 7.24 (1H, s), 7.67 (1H, dd, J = 2.81, 8.79Hz), 8.09 (1H, dd, J=2.81, 0.49 Hz), 8.35 (1H, d, J = 0.49, 5.13 Hz).
ESI-MSm/z: 339 (M+H)⁺.

### 4) Ethyl 5-(4-carboxy-2-pyridyl)-1-(6-methoxy-3-pyridyl)-1H-pyrazole-3-carboxylate

Selenium dioxide (1.77 g) was added to a solution of the ethyl 5-(4-methyl-2-pyridyl)-1-(6-methoxy-3-pyridyl)-1H-pyrazole-3-carb oxylate (774 mg) in pyridine (6 ml) at room temperature, and the mixture was stirred at 120°C for 7 days. After cooling with air, water and chloroform were added to the reaction liquid and the phases were separated, and the organic layer was dried over anhydrous magnesium sulfate. After filtration, the solvent was evaporated under reduced pressure to give ethyl 5-(4-carboxy-2-pyridyl)-1-(6-methoxy-3-pyridyl)-1H-pyrazole-3-carboxylate (1.14 g, measured) as an amorphous product.

¹H-NMR (400 MHz, CDCl₃)δ: 1.43 (3H, t, J = 7.08 Hz), 3.94 (3H, s), 4.46 (2H, q, J = 7.08 Hz), 6.78 (1H, d, J = 8.79 Hz), 7.36 (1H, s), 7.70 (1H, dd, J = 2.69, 8.79 Hz), 7.84 (1H, dd, J = 4.88, 1.47 Hz), 8.07 (1H, s), 8.12 (1H, d, J = 2.69 Hz), 8.66 (1H, d, J = 4.88 Hz).
ESI-MSm/z: 369 (M+H)⁺.

### 5) Ethyl 5-(4-hydroxymethyl-2-pyridyl)-1-(6-methoxy-3-pyridyl)-1H-pyrazole -3-carboxylate

Borane-dimethyl sulfate complex (0.95 ml) was added dropwise to a solution of the ethyl 5-(4-carboxy-2-pyridyl)-1-(6-methoxy-3-pyridyl)-1H-pyrazole-3-carboxylate (1.14 g) in tetrahydrofuran (30 ml) at 0°C, and the mixture was stirred at room temperature for 18.5 hours. A mixed solvent of water and glacial acetic acid (1:2) and ethyl acetate were added to the reaction liquid and the phases were separated, and the organic layer was washed with saturated aqueous sodium bicarbonate and brine, and dried over anhydrous magnesium sulfate. After filtration, the solvent was evaporated under reduced pressure, and the residue was purified by column chromatography on silica gel (chloroform-methanol) to give ethyl 5-(4-hydroxymethyl-2-pyridyl)-1-(6-methoxy-3-pyridyl)-1H-pyrazole -3-carboxylate (250 mg, 23%) as an oily product.

¹H-NMR (400 MHz, CDCl₃)δ: 1. 42 (3H, t, J = 7.08 Hz), 3. 94 (3H, s), 4.45 (2H, q, J = 7.08 Hz), 4.72 (2H, s), 6.75 (1H, d, J = 8.79 Hz), 7.22 (1H, ddd, J = 5.01, 1.59, 0.85 Hz), 7.25 (1H, s), 7.43 (1H, dd, J = 1.59, 0.85 Hz), 7.67 (1H, dd, J = 2.69, 8.79 Hz), 8.08 (1H, dd, J = 2.69, 0.61 Hz), 8.45 (1H, dd, J = 0.61, 5.01 Hz). ESI-MSm/z: 355 (M+H)⁺.

### 6) The title compound

The procedure of Referential Example 16(4) was repeated by using the ethyl 5-(4-hydroxymethyl-2-pyridyl)-1-(6-methoxy-3-pyridyl)-1H-pyrazole -3-carboxylate (250mg) togivethetitlecompound (232mg, quantitative).
EI-MSm/z:327(M+H)⁺.

[Referential Example 30] Methyl 5-(4-carbamoyl-2-pyridyl)-1-(6-methoxy-3-pyridazinyl)-1H-pyrazole -3-carboxylate

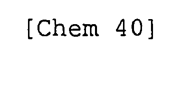

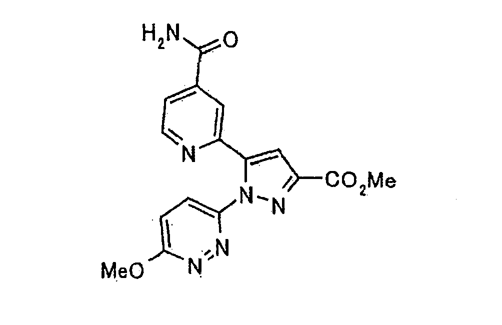

### 1) Ethyl 1-(6-chloro-3-pyridazinyl)-5-(4-methyl-2-pyridyl)-1H-pyrazole-3-carboxylate

The procedure of Referential Example 9(1) was repeated by using the ethyl 4-(4-methyl-2-pyridyl)-2,4-dioxobutanoate (4.46 g) of Referential Example 29 (2) and 3-chloro-6-hydrazinopyridazine (3.6g) to give ethyl 1-(6-chloro-3-pyridazinyl)-5-(4-methyl-2-pyridyl)-1H-pyrazole-3-c arboxylate (1.79 g, 27%) as an amorphous product.

¹H-NMR (400 MHz, CDCl₃)δ: 1.43 (3H, t, J = 7.08 Hz), 2.41 (3H, s), 4.48 (2H, q, J = 7.08 Hz), 7.07 (1H, m), 7.20 (1H, s), 7.47 (1H, s), 7.69 (1H, d, J = 9.03 Hz), 8.08 (1H, d, J = 9.03 Hz), 8.23 (1H, d, J = 4.88 Hz).
ESI-MSm/z: 344 (M+H)⁺.

### 2) Methyl 1-(6-methoxy-3-pyridazinyl)-5-(4-methyl-2-pyridyl)-1H-pyrazole-3-carboxylate

The procedure of Referential Example 9, Method A (2) was repeated' by using the ethyl 1-(6-chloro-3-pyridazinyl)-5-(4-methyl-2-pyridyl)-1H-pyrazole-3-carboxylate (600 mg) to give methyl 1-(6-methoxy-3-pyridazinyl)-5-(4-methyl-2-pyridyl)-1H-pyrazole-3-carboxylate (455 mg, 84%) as a solid.

¹H-NMR (400 MHz, CDCl₃)δ: 2.40 (3H, s), 3.99 (3H, s), 4.10 (3H, s), 7.04 (1H, m), 7.15 (1H, d, J = 9.28 Hz), 7.21 (1H, s), 7.44 (1H, s), 7.93 (1H, d, J = 9.28 Hz), 8.24 (1H, d,4.88 Hz). ESI-MSm/z: 326 (M+H)⁺.

### 3) Methyl 5-(4-carboxy-2-pyridyl)-1-(6-methoxy-3-pyridazinyl)-1H-pyrazole-3 -carboxylate

The procedure of Referential Example 29 (4) was repeated by using the methyl 1-(6-methoxy-3-pyridazinyl)-5-(4-methyl-2-pyridyl)-1H-pyrazole-3-carboxylate (147 mg) to give methyl 5-(4-carboxy-2-pyridyl)1-(6-methoxy-3-pyridazinyl)-1H-pyrazole-3-carboxylate (64 mg, 40%).

¹H-NMR (400 MHz, DMSO-d₆)δ: 3.88 (3H, s), 4.02 (3H, s), 7.49 (1H, d, J = 9.28 Hz), 7.59 (1H, s), 7.75 (1H, d, J = 5.01 Hz), 8.01 (1H, d, J = 9.28 Hz), 8.15 (1H, s), 8.54 (1H, d, J = 5.01 Hz). EI-MSm/z: 356 (M+H)⁺.

### 4) The title compound

Ammonium chloride (14 mg), 1-hydroxybenzotriazole (30 mg), 1-ethyl-3- (3-dimethylaminopropyl) carbodiimide hydrochloride (41 mg), and triethylamine (75 µl) were added to a solution of the methyl 5-(4-carboxy-2-pyridyl)-1-(6-methoxy-3-pyridazinyl)-1H-pyrazole-3 -carboxylate (64mg) in N,N-dimethylformamide (3ml) at room temperature, and the mixture was stirred at 19 hours. Water and chloroform were added to the reaction liquid and the phases were separated, and the organic layer was dried over anhydrous magnesium sulfate. After filtration, the solvent was evaporated under reduced pressure, and the residue was purified by thin layer chromatography on silica gel (chloroform-methanol) to give the title compound (29 mg, 45%) as a solid.

¹H-NMR (400 MHz, CDCl₃ and CD₃OD)δ: 3.98 (3H, s), 4. 09 (3H, s), 7.38 (1H, d, J = 9.28 Hz), 7.74 (1H, dd, J = 1.58, 5.13 Hz), 7.81 (1H, s), 7.99 (1H, d, J = 9.28 Hz), 8.13 (1H, s), 8.50 (1H, d, J = 5.13 Hz).

[Referential Example 31] 5-Hydrazino-2-methylpyridine

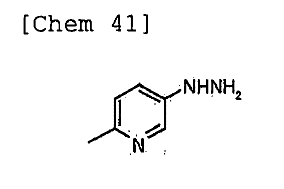

### 1) (6-Methylpyridin-3-yl)carbamic acid tert-butyl ester

Triethylamine (23.0 ml), diphenylphosphorylazide (35. 6 ml), and tert-butanol (30. 0 ml) were added to a suspension of 6-methylnicotinic acid (21.58 g) in 1,4-dioxane (300 ml) at room temperature, and the mixture was heated under reflux for 18 hours. After cooling with air, the reaction solvent was evaporated under reduced pressure, and chloroform and water were added to the residue and the phases were separated. The organic layer was washed with brine, and dried over anhydroussodiumsulfate. After filtration, the solvent was evaporated under reduced pressure, and the residue was purified by column chromatography on silica gel (chloroform-ethyl acetate) to give (6-methylpyridin-3-yl)carbamic acid tert-butyl ester (28.7 g, 88%) as a solid.

¹H-NMR (400 MHz, CDCl₃)δ: 1.54 (9H, s), 2.49 (3H, s), 6.52 (1H, br), 7.09 (1H, d, J = 8.6 Hz), 7.86 (1H, br), 8.29 (1H, d, J = 2.7 Hz). EI-MSm/z: 208 (M)⁺.

### 2) The title compound

Conc. hydrochloric acid (100 ml) was gradually added to (6-methylpyridin-3-yl) carbamic acid tert-butyl ester (28.7 g) at 0°C, and the mixture was stirred for 30 minutes. Under the inner temperature of the reaction liquid maintained at 0 to 5°C, a solution of sodium nitrite (10.51 g) in water (38 ml) was added over 30 minutes, and the mixture was stirred for 15 minutes. This reaction liquid was added dropwise to a solution of tin (II) chloride dihydrate (108.7 g) in conc. hydrochloric acid (54 ml) over 50 minutes under the inner temperature of the reaction liquid maintained at 0 to 5°C, and the mixture was stirred for 1 hour. Under the inner temperature of the reaction liquid maintained at 0 to 10°C, 6N aqueous sodium hydroxide (700 ml) and a mixed solvent of chloroform and methanol (10:1) were added to the reaction liquid, and the phases were separated. The organic layer was filtered through celite to remove the insoluble content, and the filtrate was washed with water. The aqueous layer was extracted with a mixed solvent of chloroform and methanol (10:1), and the combined organic phases were washed with brine and dried over anhydrous sodium sulfate. After filtration, the solvent was evaporated under reduced pressure to give the title compound (7.66 g, 45%) as a solid.

¹H-NMR (400 MHz, DMSO-d₆)δ: 2.29 (3H, s), 3.97 (2H, br), 6.66 (1H, br), 6.94 (1H, d, J = 8.3 Hz), 7.05 (1H, dd, J = 3.0, 8.3 Hz), 7.98 (1H, d, J = 3.0 Hz). ESI-MSm/z: 124 (M+H)⁺.

[Referential Example 32] 3-Hydrazinopyridine

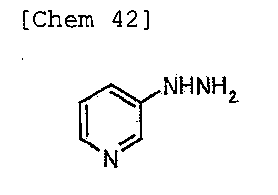

A solution of sodium nitrite (10.5 g) in water (39 ml) was added dropwise to a solution of 3-aminopyridine (13.0 g) in conc. hydrochloric acid (104 ml) at a temperature below 5°C over 30 minutes, and the mixture was stirred for 15 minutes. The reaction liquid was added dropwise to a solution of tin (II) chloride dihydrate (109 g) in conc. hydrochloric acid (59 ml) under cooling to the inner temperature of 0 to 10°C over 30 minutes, and the solution was stirred for 1 hour. 6N aqueous sodium hydroxide (796 ml) was added dropwise to the reaction liquid at the same temperature, and a mixed solvent of methanol and chloroform (1:10) was added to the reaction liquid and the phases were separated, and the solvent in the organic layer was evaporated under reduced pressure to give the title compound (12.5 g, 83%) as a solid.

¹H-NMR (400MHz, DMSO-d₆)δ: 4.02 (2H, br s), 6.89 (1H, br s), 7.04-7.12 (2H, m), 7.76-7.78 (1H, m), 8.08 (1H, m). EI-MSm/z: 109 (M⁺).

### [Referential Example 33] 2-Hydrazinopyrazine

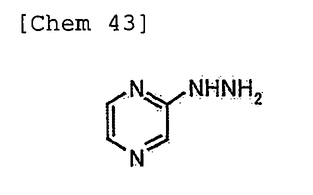

Hydrazine monohydrate (21.80 g) was added to a solution of 2-chloropyrazine (10.44 g) in ethanol (65 ml) at room temperature, and the mixture was heated under reflux for 17 hours. After cooling with air, the reaction solvent was evaporated under reduced pressure, and benzene was added to the residue, then the insoluble content was' removed by decantation. After evaporation of the benzene solution under reduced pressure, hexane was added to the resulting solid, and the product was collected by filtration to give the title compound (4.67 g, 47%).

¹H-NMR (400 MHz, CDCl₃)δ: 7.89 (1H, d, J = 2.7 Hz), 7.99-8.05 (1H, m), 8.20 (1H, d, J = 1.5 Hz). ESI-MSm/z: 111 (M+H)⁺.

[Referential Example 34] 2-Hydrazinopyrimidine

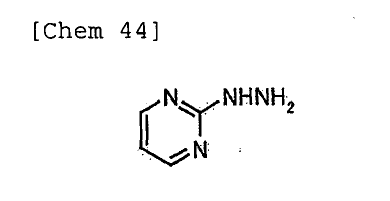

Hydrazine monohydrate (20 ml) was added to a suspension of 2-chloropyrimidine (6.00 g) in ethanol (60 ml) at room temperature, and the mixture was stirred for 80 minutes. The solvent was evaporated under reduced pressure, and water (34 ml) was added to the residue.
The solid precipitate was collected by filtration to give the title compound (2.30 g, 40%).

¹H-NMR (400 MHz, DMSO-d₆)δ: 4.12 (2H, s), 6.57-6.60 (1H, m), 8.12 (1H, s), 8.30 (2H, d, J = 4.9 Hz). EI-MSm/z: 110 (M⁺).

[Referential Example 35] 5-(5-Methyl-2-pyridyl)-1-(2-pyridyl)-1H-pyrazole-3-carboxylic acid

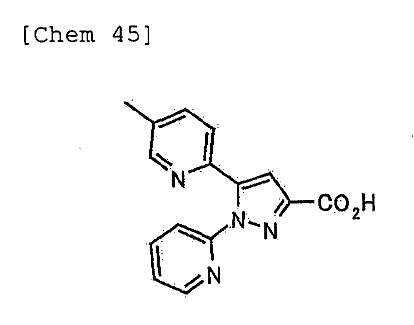

### 1) 1-(5-Methyl-2-pyridyl)ethanone

n-Butyllithium (1.58M solution in hexane, 24 ml) was added to a solution of 2-bromo-5-picoline (5.0 g) in diethylether (100 ml) in 5 minutes at -78°C, and the mixture was stirred for 5 minutes. N,N-Dimethyl acetamide (3.5 ml) was added to the reaction liquid at the same temperature, and the mixture was stirred for 2 hours. Water and ethyl acetate were added to the reaction liquid and the phases were separated, and the organic layer was dried over anhydrous magnesium sulfate. After filtration, the solvent was evaporated under reduced pressure, and the residue was purified by column chromatography on silica gel (hexane-ethyl acetate) to give 1-(5-methyl-2-pyridyl)ethanone (3.43 g, 87%) as an oily product.

¹H-NMR (400 MHz, CDCl₃)δ: 2.42 (3H, s), 2.71 (3H, s), 7.62 (1H, dd, J = 1.59, 7.93 Hz), 7.94 (1H, d, J = 7.93 Hz), 8.50 (1H, s).

### 2) Ethyl 4-(5-methyl-2-pyridyl)-2,4-dioxobutanoate

Diethyl oxalate (7 ml) was added dropwise to a solution of sodium ethoxide (3.5 g) in ethanol (60 ml) at room temperature, and a solution of the 1- (5-methyl-2-pyridyl) ethanone (3.43 g) in ethanol (40 ml) was added to the reaction liquid. The mixture was stirred for 2 hours, and water and diethylether were added to the reaction liquid and the phases were separated. The aqueous layer was acidified by adding 1N aqueous hydrochloric acid, and chloroform was added and the phases were separated. The organic layer was dried over anhydrous magnesium sulfate. After filtration, the solvent was evaporated under reduced pressure to give ethyl 4- (5-methyl-2-pyridyl)-2, 4-dioxobutanoate (6.8 g) as a solid.

¹H-NMR (400 MHz, CDCl₃)δ: 1.40 (3H, t, J = 7.08 Hz), 2.47 (3H, s), 4.39 (2H, q, J = 7.08 Hz), 7.49 (1H, br), 7.74 (1H, dd, J = 1.47, 8.06 Hz), 8.08 (1H, d, J = 8.06 Hz), 8.58 (1H, d, J = 0.73 Hz). EI-MSm/z: 236 (M+H)⁺.

### 3) Ethyl 5-(5-methyl-2-pyridyl)-1-(2-pyridyl)-1H-pyrazole-3-carboxylate

To a solution of the ethyl 4-(5-methyl-2-pyridyl)-2,4-dioxobutanoate (3.30 g) in ethanol (30 ml) were added 2-hydrazinopyridine (2.0 g) and conc. hydrochloric acid (2 ml), and the mixture was heated under reflux for 18 hours. After cooling with air, chloroform and saturated sodium hydrogencarbonate solution were added to the reaction liquid and the phases were separated. The organic layer was dried over anhydrous magnesium sulfate. After filtration, the solvent was evaporated under reduced pressure, and the residue was purified by column chromatography on silica gel (methanol-chloroform) to give ethyl 5-(5-methyl-2-pyridyl)-1-(2-pyridyl)-1H-pyrazole-3-carboxylate (1.87 g).

¹H-NMR (400 MHz, CDCl₃)δ: 1.42 (3H, t, J = 7.08 Hz), 2.33 (3H, s), 4.45 (2H, q, J = 7.08 Hz), 7.20 (1H, s), 7.25 (1H, m), 7.33 (1H, d, J = 7.93 Hz), 7.52 (1H, d, J = 7.93 Hz), 7.76 (1H, d, J = 8.05 Hz), 7.85 (1H, dd, J = 8.05, 1.84 Hz), 8.28 (2H, s). EI-MSm/z: 309 (M+H)⁺.

### 4) The title compound

Sodium ethoxide (830 mg) was added to a solution of the ethyl 5-(5-methyl-2-pyridyl)-1-(2-pyridyl)-1H-pyrazole-3-carboxylate (1.86 g) in ethanol (30 ml) at room temperature, and the mixture was stirred for 2 hours. Sodium ethoxide (700 mg) was then added to the solution, and the mixture was stirred for 30 minutes. Water and diethylether were added to the reaction liquid and the phases were separated, and the aqueous layer was acidified (to pH 4) with 1N aqueous hydrochloric acid, and the solution was extracted with chloroform. The organic layer was dried over anhydrous magnesium sulfate. After filtration, the solvent was evaporated under reduced pressure to give the title compound (1.17 g, 30% in two steps).

¹H-NMR (400 MHz, CDCl₃)δ: 2.36 (3H, s), 7.23 (1H, s), 7.29 (1H, m), 7.37 (1H, d, J = 7.93 Hz), 7.55 (1H, d, J = 7.93 Hz), 7.79 (1H, d, J = 8.06 Hz), 7.86 (1H, dt, J = 8.06, 1.83 Hz), 8.27 (1H, ddd, J = 4.88, 1.83, 0.86 Hz), 8.35 (1H, s). EI-MSm/z: 281 (M+H)⁺.

[Referential Example 36] 5-(5-Methyl-2-pyrazinyl)-1-(3-pyridyl)-1H-pyrazole-3-carboxylic acid

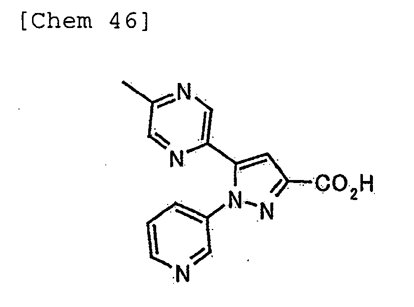

### 1) 5-Methylpyrazine-2-carboxylic acid N-methoxy-N-methylamide

Triethylamine (28.9 ml) was added to a solution of 5-methylpyrazine-2-carboxylic acid (13.0 g), N,O-dimethyl hydroxylamine hydrochloride (10.1 g), 1-(3-dimethylaminopropyl)-3-ethylcarbodiimide hydrochloride (19.8 g), and 1-hydroxybenzotriazole (14.0 g) in N, N-dimethylformamide (130 ml) at room temperature, and the mixture was stirred for 63 hours. Water and ethyl acetate were added to the reaction liquid and the phases were separated, and the organic layer was dried over anhydrous sodium sulfate. After filtration, the solvent was evaporated under reduced pressure, and the residue was purified by column chromatography on silica gel (hexane-ethyl acetate) to give 5-methylpyrazine-2-carboxylic acid N-methoxy-N-methylamide (12.3 g, 72%) as an oily product.

¹H-NMR (400 MHz, CDCl₃)δ: 2.63 (3H, s), 3.41 (3H, s), 3.74 (3H, s), 8.46 (1H, s), 8.82 (1H, s). FAB-MSm/z: 182 (M+H)⁺.

### 2) 1-(5-Methyl-2-pyrazinyl)ethanone

Methyl lithium (1.02M solution in diethylether, 72.6 ml) was added dropwise to a solution of the 5-methylpyrazine-2-carboxylic acid N-methoxy-N-methylamide (12.2 g) in tetrahydrofuran (183ml) over 20 minutes at -78°C under argon atmosphere, and the mixture was stirred for another 130 minutes. Water and ethyl acetate were added to the reaction liquid at 0°C and the phases were separated, and the organic layer was dried over anhydrous sodium sulfate. After filtration, the solvent was evaporated under reduced pressure, and the residue was purified by column chromatography on silica gel (ethyl acetate-hexane) to give 1-(5-methyl-2-pyrazinyl)ethanone (7.9 g, 86%) as a solid.

¹H-NMR (400 MHz, CDCl₃)δ: 2.66 (3H, s), 2.70 (3H, s), 8.50 (1H, m), 9.11 (1H, d, J = 1.5 Hz).
ESI-MSm/z: 137 (M+H)⁺.

### 3) Ethyl 4-(5-methyl-2-pyrazinyl)-2,4-dioxobutanoate

Lithium bis(trimethylsilyl)amide (1.0M solution in tetrahydrofuran, 63.7 ml) was added dropwise to a solution of the 1- (5-methyl-2-pyrazinyl) ethanone (7.89 g) in tetrahydrofuran (118 ml) over 20 minutes at -78°C under argon atmosphere, and the mixture was stirred for another 30 minutes. Diethyl oxalate (11.8 ml) was added dropwise to the reaction liquid, and the mixture was stirred for 10 minutes. After stirring at 0°C for 30 minutes and at room temperature for 1.5 hours, water and diethylether were added to the reaction liquid and the phases were separated, and saturated aqueous ammonium chloride and chloroform were added to the aqueous layer. The phases were separated. The organic layer was dried over anhydrous sodium sulfate. After filtration, the solvent was evaporated under reduced pressure to give ethyl 4-(5-methyl-2-pyrazinyl)-2,4-dioxobutanoate (4.92 g, 36%) as a solid.

¹H-NMR (400 MHz, CDCl₃)δ: 1.39-1.43 (3H, m), 2.69 (3H, s), 4.38-4.43 (2H, m), 7.60 (1H, s), 8.55 (1H, m), 9.21 (1H, d, J = 1.2 Hz).
FAB-MSm/z: 237 (M+H)⁺.

### 4) Ethyl 5-(5-methyl-2-pyrazinyl)-1-(3-pyridyl)-1H-pyrazole-3-carboxylate

A solution of the ethyl 4-(5-methyl-2-pyrazinyl)-2,4-dioxobutanoate (4.91 g) and the 3-hydrazinopyridine (2.27 g) of Referential Example 32 in ethanol (98 ml) was heated under reflux for 40 minutes. Acetic acid (5.95 ml) was added to the reaction liquid, and the mixture was heated under reflux for another 14 hours. After cooling with air, saturated aqueous sodium bicarbonate and chloroform were added to the reaction liquid and the phases were separated, and the organic layer was dried over anhydrous sodium sulfate. After filtration, the solvent was evaporated under reduced pressure, and the residue was dissolved in ethanol (99 ml). Conc. hydrochloric acid (3.3 ml) was added to the reaction liquid, and the mixture was heated under reflux for 1 hour. After cooling with air, saturated aqueous sodium bicarbonate and chloroform were added to the reaction liquid and the phases were separated, and the organic layer was dried over anhydrous sodium sulfate. After filtration, the solvent was evaporated under reduced pressure, and the residue was purified by column chromatography on silica gel (acetone-toluene) to give ethyl 5-(5-methyl-2-pyrazinyl)-1-(3-pyridyl)-1H-pyrazole-3-carboxylate (3.16 g, 49%) as a solid.

¹H-NMR (400 MHz, CDCl₃)δ: 1.42-1.46 (3H, m), 2.58 (3H, s), 4.45-4.51 (2H, m), 7.34 (1H, s), 7.38-7.41 (1H, m), 7.83-7.86 (1H, m), 8.30-8.31 (1H, m), 8.54 (1H, d, J = 2.4 Hz), 8.63 (1H, d, J = 1.5 Hz), 8.64 (1H, d, J = 1.5 Hz). FAB-MSm/z: 310 (M+H)⁺.

### 5) The title compound

The procedure of Referential Example 14 (5) was repeated by using the ethyl 5-(5-methyl-2-pyrazinyl)-1-(3-pyridyl)-1H-pyrazole-3-carboxylate (0.60 g) and 1N aqueous sodium hydroxide (4.85 ml) to give the title compound (0.525 g, 96%) as a solid.

¹H-NMR (400 MHz, DMSO-d₆)δ: 2.50 (3H, s), 7.50-7.53 (2H, m), 7.84-7.87 (1H, m), 8.36 (1H, s), 8.58 (1H, d, J = 2.4 Hz), 8.62-8.64 (1H, m), 8.92 (1H, d, J = 1.2 Hz), 13.17 (1H, br s). FAB-MSm/z: 282 (M+H)⁺.

[Referential Example 37] 5-(5-Methyl-2-pyridyl)-1-(3-pyridyl)-1H-pyrazole-3-carboxylic acid

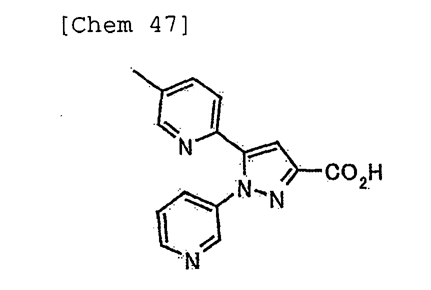

### [Method A]

### 1) Ethyl 5-(5-methyl-2-pyridyl)-1-(3-pyridyl)-1H-pyrazole-3-carboxylate

The 3-hydrazinopyridine (2.0 g) of Referential Example 32 and conc. hydrochloric acid (2 ml) were added to a solution of the ethyl 4-(5-methyl-2-pyridyl)-2,4-dioxobutanoate (3.50 g) of Referential Example 35(2) in ethanol (30 ml), and the mixture was heated under reflux for 17.5 hours. After cooling with air, saturated aqueous sodium bicarbonate and chloroform were added to the reaction liquid and the phases were separated, and the organic layer was dried over anhydrous magnesium sulfate. After filtration, the solvent was evaporated under reduced pressure, and the residue was purified by column chromatography on silica gel (chloroform-methanol) to give ethyl 5-(5-methyl-2-pyridyl)-1-(3-pyridyl)-1H-pyrazole-3-carboxylate (2.51 g, 55%).

¹H-NMR (400 MHz, CDCl₃)δ: 1.43 (3H, t, J = 7.14 Hz), 2.34 (3H, s), 4.46 (2H, q, J = 7.14 Hz), 7.24 (1H, s), 7.32 (1H, d, J = 8.12 Hz), 7.37 (1H, dd, J = 8.12, 4.82 Hz), 7.51 (1H, dd, J = 8.12, 2.08 Hz), 7.84 (1H, dd, J = 8.012, 1.44 Hz), 8.30 (1H, s), 8.51 (1H, d, J = 2.56 Hz), 8.59 (1H, dd, J = 4.83, 1.34 Hz).
ESI-MSm/z: 309 (M+H)⁺.

### 2) The title compound

Sodium ethoxide (1.11 g) was added to a solution of the ethyl 5-(5-methyl-2-pyridyl)-1-(3-pyridyl)-1H-pyrazole-3-carboxylate (2.51 g) in ethanol (80 ml) at room temperature, and the mixture was stirred for 19.5 hours. Water and diethylether were added to the reaction liquid and the layer was separated, and the aqueous layer was acidified by adding 1N aqueous hydrochloric acid. Chloroform was added to the solution, and the phases were separated, and the organic layer was dried over anhydrous magnesium sulfate. After filtration, the solvent was evaporated under reduced pressure to give the title compound (1.33 g, 58%).

¹H-NMR (400 MHz, CDCl₃/CD₃OD)δ: 2.38 (3H, s), 7.26 (1H, s), 7.45 (2H, m), 7.68 (1H, br), 7.85 (1H, ddd, J = 8.30, 2.44, 1.47 Hz), 8.30 (1H, s), 8.52 (1H, d, J = 2.56 Hz), 8.56 (1H, dd, J = 4.76, 1.47 Hz).
ESI-MSm/z: 281 (M+H)⁺.

### [Method B]

### 1) Methyl 4-(5-methyl-2-pyridyl)-2,4-dioxobutanoate

Dimethyl oxalate (10.4 g) was added to a solution of sodium methoxide (4.74 g) in methanol (200 ml) at room temperature, and the mixture was stirred for 5 minutes. The 1- (5-methyl-2-pyridyl) ethanone (5.93 g) of Referential Example 35 (1) was added to the reaction liquid at room temperature, and the mixture was stirred for 5 hours. Water and diethylether were added to the reaction liquid and the phases were separated, and the aqueous layer was acidified with 1N aqueous hydrochloric acid and extracted with chloroform. The organic layer was dried over anhydrous magnesium sulfate. After filtration, the solvent was evaporated under reduced pressure to give methyl 4-(5-methyl-2-pyridyl)-2,4-dioxobutanoate (7.31 g, 75%) as a solid.

¹H-NMR (400 MHz, CDCl₃)δ: 2.46 (3H, s), 3.92 (3H, s), 7.58 (1H, br), 7.70 (1H, dd, J= 8.06, 1.83 Hz), 8.08 (1H, d, J= 8.06 Hz), 8.54 (1H, d, J = 1.22 Hz).

### 2) Methyl 5-(5-methyl-2-pyridyl)-1-(3-pyridyl)-1H-pyrazole-3-carboxylate

The 3-hydrazinopyridine (3.0 g) of Referential Example 32 and conc. hydrochloric acid (4 ml) were added to a solution of the methyl 4-(5-methyl-2-pyridyl)-2,4-dioxobutanoate (4.0 g) in methanol (250 ml), and the mixture was heated under reflux for 3.5 hours. After cooling with air, saturated aqueous sodium bicarbonate and chloroform were added to the reaction liquid and the phases were separated, and the organic layer was dried over anhydrous magnesium sulfate. After filtration, the solvent was evaporated under reduced pressure, and the residue was purified by column chromatography on silica gel (methanol-chloroform) to give methyl 5-(5-methyl-2-pyridyl)-1-(3-pyridyl)-1H-pyrazole-3-carboxylate (3.24 g, 61%) as a solid.

¹H-NMR (400 MHz, CDCl₃)δ: 2.34 (3H, s), 3.99 (3H, s), 7.27 (1H, s), 7.33 (1H, d, J = 8.06 Hz), 7.37 (1H, ddd, J = 8.18, 4.76, 0.73 Hz), 7.53 (1H, ddd, J = 8.06, 2.20, 0.73 Hz), 7.84 (1H, ddd, J = 8.18, 2.56, 1.47 Hz), 8.30 (1H, d, J = 1.47 Hz), 8.50 (1H, d, J = 2.32 Hz), 8.59 (1H, dd, J = 4.76, 1.47 Hz).
ESI-MSm/z: 295 (M+H)⁺.

### 3) The title compound

1N aqueous sodium hydroxide (16 ml) was added to a solution of the methyl 5-(5-methyl-2-pyridyl)-1-(3-pyridyl)-1H-pyrazole-3-carboxylate (3.24 g) in tetrahydrofuran (100ml) at room temperature, and the mixture was stirred for 2 hours. 1N aqueous hydrochloric acid and chloroform were added to reaction liquid and the phases were separated, and the organic layer was dried over anhydrous magnesium sulfate. After filtration, the solvent was evaporated under reduced pressure to give' the title compound (2.0 g, 65%) as a solid.

¹H-NMR (400 MHz, DMSO-d₆)δ: 2.28 (3H, s), 7.30 (1H, s), 7.49 (1H, dd, J = 8.18, 4.76 Hz), 7.63 (1H, d, J = 8.18 Hz), 7.70 (1H, dd, J = 8.18, 1.58 Hz), 7.79 (1H, d, J = 8.18 Hz), 8.24 (1H, s), 8.49 (1H, d, J = 1.56 Hz), 8.59 (1H, dd, J = 4.76, 1.59 Hz).
ESI-MSm/z: 281 (M+H)⁺.

[Referential Example 38] 5-(5-Chloro-2-pyridyl)-1-(2-pyridyl)-1H-pyrazole-3-carboxylic acid

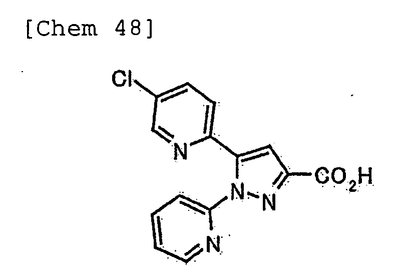

### 1) Ethyl 5-(5-chloro-2-pyridyl)-1-(2-pyridyl)-1H-pyrazole-3-carboxylate

The procedure of Referential Example 37, MethodA (1) was repeated by using the ethyl 4-(5-chloro-2-pyridyl)-2,4-dioxobutanoate (2.23 g) of Referential Example 25(3) and 2-hydrazinopyridine (1.05 g) to give ethyl 5-(5-chloro-2-pyridyl)-1-(2-pyridyl)-1H-pyrazole-3-carboxylate (1.11 g, 53%).

¹H-NMR (400 MHz, CDCl₃)δ: 1.43 (3H, t, J = 7.08 Hz), 4.46 (2H, q, J = 7.08 Hz), 7.22 (1H, s), 7.29 (1H, ddd, J = 7.20, 4.88, 1.22 Hz), 7.41 (1H, dd, J = 8.42, 0.73 Hz), 7.69 (1H, dd, J = 8.42, 2.44 Hz), 7.81 (1H, ddd, J = 8.18, 1.22, 0. 98 Hz), 7.88 (1H, ddt, J = 7.20, 1.83, 0.86 Hz), 8.25 (1H, ddd, J = 4.88, 1.83, 0.86 Hz), 8.40 (1H, dd, J = 2.44, 0.73 Hz).
ESI-MSm/z: 329 (M+H)⁺.

### 2) The title compound

The procedure of Referential Example 37, MethodA (2) was repeated by using the ethyl 5-(5-chloro-2-pyridyl)-1-(2-pyridyl)-1H-pyrazole-3-carboxylate (1.11 g) to give the title compound (939 mg, 92%) as a solid.

¹H-NMR (400 MHz, DMSO-d₆)δ: 7.31 (1H, s), 7.46 (1H, s), 7.71 (2H, m), 7.99 (2H, m), 8.29 (1H, s), 8.44 (1H, s).
ESI-MSm/z: 301 (M+H)⁺.

[Referential Example 39] 5-(5-Cyano-2-pyridyl)-1-(2-pyridyl)-1H-pyrazole-3-carboxylic acid

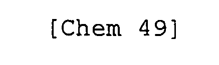

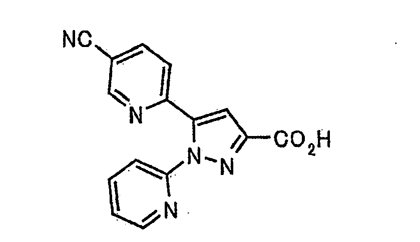

### 1) Ethyl 5-(5-benzyloxy-2-pyridyl)-1-(2-pyridyl)-1H-pyrazole-3-carboxylate

The procedure of Referential Example 8 (2) was repeated by using the ethyl 4-(5-benzyloxy-2-pyridyl)-2,4-dioxobutanoate (3.11 g) of Referential Example 14(3) and 2-hydrazinopyridine (1.14 g) to give ethyl 5-(5-benzyloxy-2-pyridyl)-1-(2-pyridyl)-1H-pyrazole-3-carboxylate (2.57 g, 68%) as an oily product.

¹H-NMR (400 MHz, CDCl₃)δ: 1.42 (3H, t, J = 7.2 Hz), 4.45 (2H, q, J = 7.1 Hz), 5.12 (1H, s), 7.16 (1H, s), 7.24-7.28 (2H, m), 7.35-7.41 (6H, m), 7.75 (1H, d, J = 8.1 Hz), 7.82-7.86 (1H, m), 8.21 (1H, d, J = 2.9 Hz), 8.28 (1H, d, J = 3.7 Hz).
ESI-MSm/z: 401 (M+H)⁺.

### 2) Ethyl 5-(5-hydroxy-2-pyridyl)-1-(2-pyridyl)-1H-pyrazole-3-carboxylate

To a solution of the ethyl 5-(5-benzyloxy-2-pyridyl)-1-(2-pyridyl)-1H-pyrazole-3-carboxylate (2.57 g) in a mixture of ethanol (30 ml) and ethyl acetate (30 ml) was added 10% palladium on carbon (1.50 g) at room temperature, and the mixture was stirred overnight under hydrogen atmosphere. After filtering the reaction liquid, the solvent was evaporated under reduced pressure to give ethyl 5-(5-hydroxy-2-pyridyl)-1-(2-pyridyl)-1H-pyrazole-3-carboxylate (2.06 g, measured) as an amorphous product.

¹H-NMR (400 MHz, CDCl₃)δ: 1.41 (3H, t, J = 7.2 Hz), 4.44 (2H, q, J = 7.1 Hz), 7.05 (1H, dd, J = 8.5, 2.7 Hz), 7.11 (1H, s), 7.21 (1H, d, J = 8.8 Hz), 7.26-7.28 (2H, m), 7.71 (1H, d, J = 8.1 Hz), 7.80-7.83 (1H, m), 7.89 (1H, s), 8.26 (1H, d, J = 3.9 Hz).
ESI-MSm/z: 311 (M+H)⁺.

### 3) Ethyl 1-(2-pyridyl)-5-(5-trifluoromethanesulfonyloxy-2-pyridyl)-1H-pyra zole-3-carboxylate

Trifluoromethanesulfonic anhydride (1.30 ml) was added to a solution of the ethyl 5-(5-hydroxy-2-pyridyl)-1-(2-pyridyl)-1H-pyrazole-3-carboxylate (2.00 g) in dichloromethane (50 ml) and pyridine (13 ml) at room temperature under nitrogen atmosphere, and the mixture was stirred overnight. Water was added to the reaction liquid, and the solution was extracted with ethyl acetate. The organic layer was washed with water and brine, and dried over anhydrous sodium sulfate. After filtration, the solvent was evaporated under reduced pressure, and the residue was purified by column chromatography on silica gel (ethyl acetate-chloroform) to give ethyl
1-(2-pyridyl)-5-(5-trifluoromethanesulfonyloxy-2-pyridyl)-1H-pyra zole-3-carboxylate (2.00 g, 70%) as an oily product.

¹H-NMR (400 MHz, CDCl₃)δ: 1.43 (3H, t, J = 7.1 Hz), 4.47 (2H, q, J = 7.1 Hz), 7.26-7.31 (2H, m), 7.59 (1H, d, J = 8.5 Hz), 7.67 (1H, dd, J = 8.7, 2.8 Hz), 7.86-7.91 (2H, m), 8.20 (1H, d, J = 5.1 Hz), 8.42 (1H, d, J = 2.7 Hz).
ESI-MSm/z: 443 (M+H)⁺.

### 4) The title compound

A suspension of tri-n-butyl tin cyamide (8.15 g) and tetrakis (triphenylphosphine)palladium(0) (11.2 g) in 1,2-dichloroethane (70 ml) was heated under reflux for 2 hours. After cooling with air, ethyl 1-(2-pyridyl)-5-(5-trifluoromethanesulfonyloxy-2-pyridyl)-1H-pyra zole-3-carboxylate (2.00 g) was added, and the mixture was heated under reflux overnight. After cooling with air, saturated aqueous sodium bicarbonate was added to the reaction liquid, and the mixture was filtered through celite. Chloroform and water were added to the filtrate and the phases were separated. The organic layer was dried over anhydrous sodium sulfate. After filtration, the solvent was evaporated under reduced pressure, and the residue was purified by column chromatography on silica gel (ethyl acetate-chloroform) to give ethyl 5-(5-cyano-2-pyridyl)-1-(2-pyridyl)-1H-pyrazole-3-carboxylate (950 mg, 46%) as an oily product. Lithium hydroxide monohydrate (124 mg) was added to a solution of this ethyl ester derivative (940 mg) in tetrahydrofuran (20 ml) and water (5 ml) at room temperature, and the mixture was stirred overnight. The reaction liquid was acidified by adding 1N aqueous hydrochloric acid, and chloroform-methanol (10:1) was added to the solution and the phases were separated. The organic layer was dried over anhydrous sodium sulfate. After filtration, the solvent was evaporated under reducedpressure to give the title compound (375 mg, 44%) as an amorphous product.

¹H-NMR (400 MHz, DMSO-d₆)δ: 7.43-7.48 (2H, m), 7.78 (1H, d, J = 8.1 Hz), 7.89 (1H, d, J = 8.3 Hz), 8.04-8.08 (1H, m), 8.26-8.30 (1H, m), 8.36-8.39 (1H, m), 8.82 (1H, s).

[Referential Example 40] 5-(5-Cyano-2-pyridyl)-1-phenyl-1H-pyrazole-3-carboxylic acid

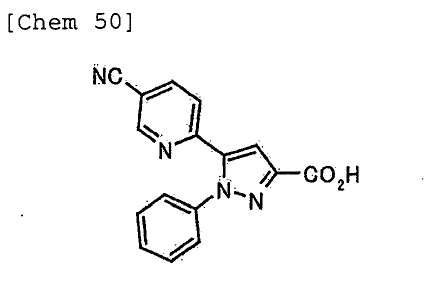

### 1) Ethyl 5-(5-benzyloxy-2-pyridyl)-1-phenyl-1H-pyrazole-3-carboxylate

The procedure of Referential Example 8 (2) was repeated by using the ethyl 4-(5-benzyloxy-2-pyridyl)-2,4-dioxobutanoate (3.14 g) of Referential Example 14 (3) and phenylhydrazine (1.16 ml) to give ethyl
5-(5-benzyloxy-2-pyridyl)-1-phenyl-1H-pyrazole-3-carboxylate (2.95 g, 77%) as a solid.

¹H-NMR (400 MHz, CDCl₃)δ: 1.41 (3H, t, J = 7.1 Hz), 4.44 (2H, q, J = 7.2 Hz), 5.10 (2H, s), 7.09-7.41 (13H, m), 8.32 (1H, d, J = 2.7 Hz).

### 2) Ethyl5-(5-hydroxy-2-pyridyl)-1-phenyl-1H-pyrazole-3-carboxylate

To a solution of the ethyl 5-(5-benzyloxy-2-pyridyl)-1-phenyl-1H-pyrazole-3-carboxylate (2.83 g) in ethanol (30 ml) and ethyl acetate (30 ml) was added 10% palladium on carbon (1.50 g), and the mixture was stirred overnight under hydrogen atmosphere. The reaction liquid was filtered, and the solvent was evaporated under reduced pressure to give ethyl 5-(5-hydroxy-2-pyridyl)-1-phenyl-1H-pyrazole-3-carboxylate(1.98g, 90%) as a solid.

¹H-NMR (400 MHz, CDCl₃)δ: 1.39 (3H, t, J = 7.2 Hz), 4.43 (2H, q, J = 7.1 Hz), 7.05 (2H, d, J = 3.7 Hz), 7.17 (1H, s), 7.27-7.30 (6H, m), 8.05 (1H, s).
ESI-MSm/z: 310 (M+H)⁺.

### 3) Ethyl 1-phenyl-5-(5-trifluoromethanesulfonyloxy-2-pyridyl)-1H-pyrazole-3-carboxylate

The procedure of Referential Example 39 (3) was repeated by using the ethyl 5-(5-hydroxy-2-pyridyl)-1-phenyl-1H-pyrazole-3-carboxylate(1.98g) and trifluoromethanesulfonic anhydride (1.29 ml) to give ethyl 1-phenyl-5-(5-trifluoromethanesulfonyloxy-2-pyridyl)-1H-pyrazole-3-carboxylate (2.60 g, 92%) as an oily product.

¹H-NMR (400 MHz, CDCl₃)δ: 1.43 (3H, t, J = 7.1 Hz), 4.46 (2H, q, J = 7.2 Hz), 7.29-7.43 (7H, m), 7.57 (1H, dd, J = 8.8, 2.7 Hz), 8.51 (1H, d, J = 2.7 Hz).

### 4) The title compound

The procedure of Referential Example 39 (4) was repeated by using tri-n-butyl tin cyamide (7.42 g), tetrakis (triphenylphosphine)palladium(0) (10.17 g), and the ethyl 1-phenyl-5-(5-trifluoromethanesulfonyloxy-2-pyridyl)-1H-pyrazole-3-carboxylate (2.59 g) to give ethyl 5-(5-cyano-2-pyridyl)-1-phenyl-1H-pyrazole-3-carboxylate (2.708 g) as a solid. The procedure of Referential Example 39(4) was repeated byusingthiscyanoderivative (2.68g) and lithium hydroxide monohydrate (369 mg) to give the title compound (951 mg, 56%) as a solid.
ESI-MSm/z: 291 (M+H)⁺.

[Referential Example 41] 5-[5-(tert-Butoxycarbonylamino)-2-pyridyl]-1-(3-pyridyl)-1H-pyrazole-3-carboxylic acid

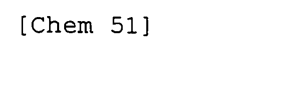

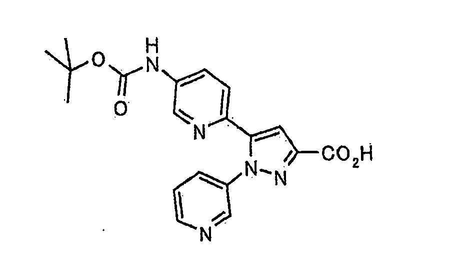

### 1) 2-Acetyl-5-aminopyridine

Methyl magnesium bromide (0.93M solution in tetrahydrofuran, 200 ml) was added dropwise to a solution of 5-amino-2-cyanopyridine (10.13 g) in tetrahydrofuran (200 ml) under ice cooling and nitrogen atmosphere over 25 minutes, and the mixture was stirred at room temperature for 5 hours. Saturated aqueous ammonium chloride was added to the reaction liquid under ice cooling, and sulfuric acid (20 ml) was then added dropwise, and the mixture was stirred at room temperature for 80 minutes. A solution of sodium hydroxide (20 g) in water (100 ml) was added dropwise to the reaction liquid under ice cooling, and ethyl acetate was added to the solution and the phases were separated. The organic layer was washed with brine and dried over anhydrous sodium sulfate. After filtration, the solvent was evaporated under reduced pressure, and the residue was purified by column chromatography on silica gel (methanol-chloroform) to give 2-acetyl-5-aminopyridine (7.68 g, 66%) as a solid.

¹H-NMR (400 MHz, CDCl₃)δ: 2.64 (3H, s), 4.00-4.30 (2H, br), 6.98 (1H, dd, J = 2.7, 8.5 Hz), 7.91 (1H, dd, J = 0.5, 8.5 Hz), 8.06 (1H, dd, J = 0.5, 2.7 Hz).
ESI-MSm/z: 137 (M+H)⁺.

### 2) 2-Acetyl-5-(tert-butoxycarbonylamino)pyridine

A solution of di-tert-butoxy dicarbonate (11.10 g) in dichloromethane (30 ml) was added to a solution of 2-acetyl-5-aminopyridine (6.30g) and4-(dimethylamino)pyridine (5.65 g) in dichloromethane (150 ml) under ice cooling, and the mixture was stirred at room temperature for 1 hour. The solid precipitate was removed by filtration, and the solvent was evaporated under reduced pressure. The residue was purified by column chromatography on silica gel (methanol-chloroform) to give 2-acetyl-5-(tert-butoxycarbonylamino)pyridine (8.04 g, 73%) as a solid.

¹H-NMR (400 MHz, CDCl₃)δ: 1.54 (9H, s), 2.68 (3H, s), 6.74 (1H, br s), 8.03 (1H, d, J = 8.5 Hz), 8.11 (1H, dd, J = 8.5, 2.4 Hz), 8.46 (1H, dd, J = 2.4, 0.5 Hz).
ESI-MSm/z: 237 (M+H)⁺.

### 3) Ethyl 4-[5-(tert-butoxycarbonylamino)-2-pyridyl]-2,4-dioxobutanoate

Diethyl oxalate (9.2 ml) was added to a solution of sodium ethoxide (4.63g) in ethanol (340 ml). To the reaction liquid was added a solution of the 2-acetyl-5-(tert-butoxycarbonylamino)pyridine (8.04 g) in ethanol (60 ml) at room temperature, and the mixture was stirred for' 45 minutes and heated under reflux for 30 minutes. After cooling with air, the reaction solvent was evaporated under reduced pressure, and 5% aqueous citric acid and ethyl acetate were added to the residue and the phases were separated. The organic layer was washed with water and brine, and dried over anhydrous sodium sulfate. After filtration, the solvent was evaporated under reduced pressure, and the residue was purified by column chromatography on silica gel (methanol-chloroform) to give ethyl 4-[5-(tert-butoxycarbonylamino)-2-pyridyl]-2,4-dioxobutanoate (1.70 g, 14.8%) as a solid.

¹H-NMR (400 MHz, CDCl₃)δ: 1.41 (3H, t, J = 7.1 Hz), 1.55 (9H, s), 4.40 (2H, q, J = 7.1 Hz), 6.78 (1H, br s), 7.59 (1H, s), 8.14 (1H, d, J = 8.8 Hz), 8.19 (1H, dd, J = 8.8, 2.4 Hz), 8.51 (1H, d, J = 2.4 Hz). ESI-MSm/z: 337 (M+H)⁺.

### 4) Ethyl 5-[5-(tert-butoxycarbonylamino)-2-pyridyl]-4,5-dihydro-5-hydroxy-1-(3-pyridyl)-1H-pyrazole-3-carboxylate

A solution of the ethyl 4-[5-(tert-butoxycarbonylamino)-2-pyridyl]-2,4-dioxobutanoate (1.59 g) and the 3-hydrazinopyridine (0.52 g) of Referential Example 32 in ethanol (100 ml) was heated under reflux for 17 hours. After cooling with air, the reaction solvent was evaporated under reduced pressure, and the residue was purified by column chromatography on silica gel (methanol-chloroform) to give ethyl 5-[5-(tert-butoxycarbonylamino)-2-pyridyl]-4,5-dihydro-5-hydroxy-1-(3-pyridyl)-1H-pyrazole-3-carboxylate (1.382g, 68%) as an amorphous product.

¹H-NMR (400 MHz, CDCl₃)δ: 1.40 (3H, t, J = 7.1 Hz), 1.53 (9H, s), 3.47 (1H, d, J= 19.0 Hz), 3.63 (1H, d, J = 19.0 Hz), 4.39 (2H, q, J= 7.1 Hz), 6.71 (1H, br s), 6.78 (1H, s), 7.06 (1H, ddd, J = 8.3, 4.6, 0.7 Hz), 7.35 (1H, d, J = 8.8 Hz), 7.46 (1H, ddd, J = 8.3, 2.7, 1.5 Hz), 7.98-8.03 (1H, m), 8.14 (1H, dd, J = 4.6, 1.5 Hz), 8.19 (1H, dd, J = 2.7, 0.7 Hz), 8.49 (1H, dd, J = 2.7, 0.5 Hz).
ESI-MSm/z: 428 (M+H)⁺.

### 5) Ethyl 5-[5-(tert-butoxycarbonylamino)-2-pyridyl]-1-(3-pyridyl)-1H-pyrazole-3-carboxylate

Triethylamine (1.96 ml) and methanesulfonyl chloride (327 µl) were added to a solution of the ethyl 5-[5-(tert-butoxycarbonylamino)-2-pyridyl]-4,5-dihydro-5-hydroxy-1-(3-pyridyl)-1H-pyrazole-3-carboxylate (1.205 g) and 4-(dimethylamino)pyridine (344 mg) in N,N-dimethylformamide (30 ml) at room temperature, and the mixture was stirred for 4 hours. The reaction solvent was evaporated under reduced pressure, and water and ethyl acetate were added to the residue and the phases were separated. The organic layer was washed with water and brine, and dried over anhydrous sodium sulfate. After filtration, the solvent was evaporated under reduced pressure, and the residue was purified by column chromatography on silica gel (hexane-ethyl acetate), and then by thin layer chromatography on silica gel (hexane-ethyl acetate) to give ethyl 5-[5-(tert-butoxycarbonylamino)-2-pyridyl]-1-(3-pyridyl)-1H-pyrazole-3-carboxylate (506 mg, 43%) as a solid.

¹H-NMR (400 MHz, CDCl₃)δ: 1.43 (3H, t, J = 7.1 Hz), 1.52 (9H, s), 4.46 (2H, q, J = 7.1 Hz), 6.59 (1H, br s), 7.23 (1H, s), 7.36 (2H, ddd, J = 8.1, 4.6, 0.7 Hz), 7.38 (2H, d, J = 8.1 Hz), 7.81 (1H, ddd, J = 8.1, 2.4, 1.5 Hz), 8.02-8.08 (1H, br m), 8.26 (1H, d, J = 2.7 Hz), 8.53 (1H, d, J = 2.4 Hz), 8.59 (1H, dd, J = 4.9, 1.5 Hz).
ESI-MSm/z: 410 (M+H)⁺.

### 6) The title compound

1N aqueous sodium hydroxide (3.7 ml) was added to a suspension of the ethyl 5-[5-(tert-butoxycarbonylamino)-2-pyridyl]-1-(3-pyridyl)-1H-pyraz ole-3-carboxylate (505 mg) in ethanol (20 ml) at room temperature, and the mixture was heated under reflux for 10 minutes. After cooling with air, the solvent was evaporated under reduced pressure, and water and ethyl acetate were added to the residue and the aqueous layer was separated. The aqueous layer was acidified with 5% aqueous citric acid, and the solid precipitate was collected by filtration to give the title compound (357 mg, 75.8%) as a solid.

¹H-NMR (400 MHz, DMSO-d₆)δ: 1.48 (9H, s), 7.27 (1H, s), 7.50 (1H, dd, J = 8.1, 4.9 Hz), 7.65 (1H, d, J = 8.5 Hz), 7.79 (1H, ddd, J = 8.1, 2.4, 1.5 Hz), 7.96 (1H, dd, J = 8.8, 2.4 Hz), 8.41 (1H, d, J = 2.4 Hz), 8.50 (1H, d, J = 2.4 Hz), 8.60 (1H, dd, J = 4.9, 1.2 Hz), 9.71 (1H, s).
ESI-MSm/z: 382 (M+H)⁺.

[Referential Example 42] 5-(5-Methyl-2-pyrazinyl)-1-(6-methyl-3-pyridyl)-1H-pyrazole-3-carboxylic acid

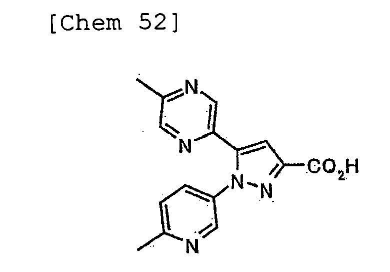

### 1) Ethyl 5-(5-methyl-2-pyrazinyl)-1-(6-methyl-3-pyridyl)-1H-pyrazole-3-carboxylate

A solution of the ethyl 4-(5-methylpyrazin-2-yl)-2,4-dioxobutanoate (4.51 g) of Referential Example 36(3) and the 5-hydrazino-2-methylpyridine (2.35 g) of Referential Example 31 in ethanol (90 ml) was heated under reflux for 80 minutes. Acetic acid (5.46 ml) was added to the reaction liquid, and the mixture was heated under reflux for another 15 hours. Conc. hydrochloric acid (3 ml) was added to the reaction liquid and the mixture was heated under reflux for 1 hour. After cooling with air, aqueous sodium bicarbonate and chloroform were added to the reaction liquid and the phases were separated, and the organic layer was dried over anhydroussodiumsulfate. After filtration, the solvent was evaporated under reduced pressure, and the residue was purified by column chromatography on silica gel (acetone-toluene) to give ethyl 5-(5-methyl-2-pyrazinyl)-1-(6-methyl-3-pyridyl)-1H-pyrazole-3-carboxylate (1.72 g, 28%) as a solid.

¹H-NMR (400 MHz, CDCl₃)δ: 1.44 (3H, t, J = 7.1 Hz), 2.58 (3H, s), 2.62 (3H, s), 4.48 (2H, q, J = 7.1 Hz), 7.25 (1H, d, J = 8.3 Hz), 7.34 (1H, s), 7.73 (1H, dd, J = 8.3, 2.7 Hz), 8.34 (1H, m), 8.40-8.41 (1H, m), 8.59 (1H, d, J = 1.5 Hz).
FAB-MSm/z: 324 (M+H)⁺.

### 2) The title compound

A solution of lithium hydroxide monohydrate (0.244 g) in water (17 ml) was added to a suspension of the ethyl 5-(5-methyl-2-pyrazinyl)-1-(6-methyl-3-pyridyl)-1H-pyrazole-3-carboxylate (1.71 g) in tetrahydrofuran (34 ml) at room temperature, and the mixture was stirred for 100 minutes. The reaction liquid was neutralized by adding 1N aqueous hydrochloric acid (5.82 ml), and water (250 ml) was added. The solid precipitate was collected by filtration to give the title compound (1.15 g, 74%) as a solid.

¹H-NMR (400 MHz, DMSO-d₆)δ: 2.50-2.53 (6H, m), 7.35 (1H, d, J = 8.3 Hz), 7.48 (1H, m), 7.72 (1H, dd, J = 8.3, 2.4 Hz), 8.39 (1H, m), 8.42 (1H, d, J = 2.4 Hz), 8.87 (1H, s), 13.14 (1H, br s). FAB-MSm/z: 296 (M+H)⁺.

[Referential Example 43] 1-(6-Methyl-3-pyridyl)-5-(1-methyl-1H-pyrrol-3-yl)-1H-pyrazole-3-carboxylic acid

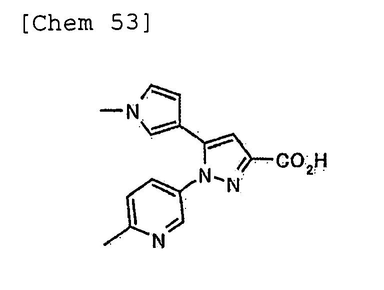

### 1) Methyl 4-(1-methyl-1H-pyrrol-3-yl)-2,4-dioxobutanoate

The procedure of Referential Example 36 (3) was repeated by using 3-acetyl-1-methyl-1H-pyrrole (5.03 g), lithium bis(trimethylsilyl)amide (1.0M solution in tetrahydrofuran, 45 ml), and dimethyl oxalate (9.53 g) to give methyl 4-(1-methyl-1H-pyrrol-3-yl)-2,4-dioxobutanoate (6.52 g, 76%) as a solid.

¹H-NMR (400 MHz, CDCl₃)δ: 3.72 (3H, s), 3.91 (3H, s), 6.60-6.66 (2H, m), 6.70 (1H, s), 7.37 (1H, s like).
FAB-MSm/z: 210 (M+H)⁺.

### 2) Methyl 1-(6-methyl-3-pyridyl)-5-(1-methyl-1H-pyrrol-3-yl)-1H-pyrazole-3-carboxylate

A solution of the methyl 4-(1-methyl-1H-pyrrol-3-yl)-2,4-dioxobutanoate (3.00 g) and the 5-hydrazino-2-methylpyridine (2.00 g) of Referential Example 31 in methanol (80 ml) was heated under reflux for 20 minutes. After cooling with air, acetic acid (3.3 ml) was added to the reaction liquid, and the mixture was heated under reflux for 14 hours. After cooling with air, the reaction solvent was evaporated under reduced pressure, and chloroform and saturated aqueous sodium bicarbonate were added to the residue and the phases were separated. The organic layer was washed with brine, and dried over anhydrous sodium sulfate. After filtration, the solvent was evaporated under reduced pressure, and the residue was purified by column chromatography on silica gel (acetone-chloroform) to give methyl 1-(6-methyl-3-pyridyl)-5-(1-methyl-1H-pyrrol-3-yl)-1H-pyrazole-3-carboxylate (2.96 g, 70%) as a solid.

¹H-NMR (400 MHz, CDCl₃)δ: 2.62 (3H, s), 3.58 (3H, s), 3.94 (3H, s), 5.85-5.92 (1H, m), 6.41-6.46 (1H, m), 6.48-6.53 (1H, m), 6.91 (1H, s like), 7.23 (1H, d, J = 8.1 Hz), 7.66-7.74 (1H, m), 8.53-8.60 (1H, m).
FAB-MSm/z: 297 (M+H)⁺.

### 3) The title compound

Lithium hydroxide monohydrate (0.475g) was added to a suspension of the methyl 1-(6-methyl-3-pyridyl)-5-(1-methyl-1H-pyrrol-3-yl)-1H-pyrazole-3-carboxylate (2.96 g) in methanol (25 ml) and water (15 ml) at room temperature, and the mixture was stirred for 1.5 hours. The reaction solvent was evaporated under reduced pressure, and the residue was neutralized by adding 1N aqueous hydrochloric acid, and the solid precipitate was collected by filtration to give the title compound (1.32 g, 47%).

¹H-NMR (400 MHz, DMSO-d₆)δ: 2.56 (3H, s), 3.55 (3H, s), 5.72-5.76 (1H, m), 6.65-6.76 (2H, m), 6.87-6.90 (1H, m), 7.37-7.44 (1H, m), 7.76-7.81 (1H, m), 8.44-8.50 (1H, m).
ESI-MSm/z: 283 (M+H)⁺.

[Referential Example 44] 1-(6-Methoxy-3-pyridyl)-5-(4-pyrimidinyl)-1H-pyrazole-3-carboxylic acid

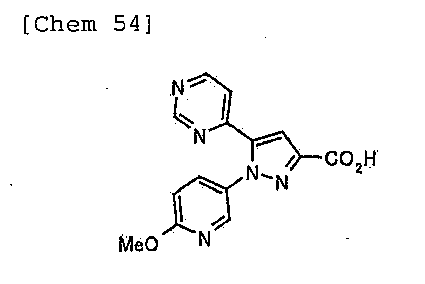

### 1) 4-Acetyl-2-methylthiopyrimidine

A mixture of 3,3-dimethylbutane-2-one (25.15 g) and N,N-dimethylformamide dimethylacetal (126 ml) was stirred at 100°C for 48 hours. After cooling with air, the low boiling components produced in the reaction were evaporated under reduced pressure, and methanol (400 ml), thiourea (28.92 g), and sodium methoxide (15.39 g) were added to the residue, and the mixture was heated under reflux for 118 hours. After cooling with air, sodium methoxide (10.26 g) was added to the reaction liquid, and methyl iodide (17.8 ml) was added dropwise to the reaction mixture under ice cooling, and the mixture was stirred at room temperature for 5 hours. The reaction solvent was evaporated under reduced pressure, and water and ethyl acetate were added to the residue and the phases were separated. The organic layer was washed with water and brine, and dried over anhydrous sodium sulfate. After filtration, the solvent was evaporated under reduced pressure, and 3N aqueous hydrochloric acid (400 ml) was added to the residue, and the mixture was stirred at room temperature for 15 hours. Ethyl acetate was added to the reaction liquid and the phases were separated, and the organic layer was dried over anhydrous sodium sulfate. After filtration, the solvent was evaporated under reduced pressure, and the residue was purified by column chromatography on silica gel (ethylacetate-hexane) to give 4-acetyl-2-methylthiopyrimidine (26.34 g, 82%) as a solid.

¹H-NMR (400 MHz, CDCl₃)δ: 2.63 (3H, s), 2.70 (3H, s), 7.51 (1H, d, J = 4.9 Hz), 8.74 (1H, d, J = 4.9 Hz).
ESI-MSm/z: 169 (M+H)⁺.

### 2) Methyl 4-(2-methylthio-4-pyrimidinyl)-2,4-dioxobutanoate

Under argon atmosphere, the procedure of Referential Example 36 (3) was repeated by using the 4-acetyl-2-methylthiopyrimidine (197 mg), lithium bis(trimethylsilyl)amide (1.0M solution in tetrahydrofuran, 1.40ml), and dimethyl oxalate (276 mg) to give methyl 4-(2-methylthio-4-pyrimidinyl)-2,4-dioxobutanoate (294 mg, 98%) as a solid.
ESI-MSm/z: 255 (M+H)⁺.

### 3) Methyl 1-(6-methoxy-3-pyridyl)-5-(2-methylthio-4-pyrimidinyl)-1H-pyrazole-3-carboxylate

The procedure of Referential Example 43 (2) was repeated by using the methyl 4-(2-methylthio-4-pyrimidinyl)-2,4-dioxobutanoate (294 mg) and the 5-hydrazino-2-methoxypyridine (161 mg) of Referential Example 2 to give methyl 1-(6-methoxy-3-pyridyl)-5-(2-methylthio-4-pyrimidinyl)-1H-pyrazol e-3-carboxylate (204 mg, 49%) as a solid.

¹H-NMR (400 MHz, CDCl₃)δ: 2.14 (3H, s), 3.98 (3H, s), 3.99 (3H, s), 6.83 (1H, d, J= 8.8 Hz), 7.03 (1H, d, J = 5.1 Hz), 7.45 (1H, s), 7.63 (1H, dd, J = 8.8, 2.7 Hz), 8.16 (1H, d, J = 2.7 Hz), 8.52 (1H, d, J = 5.1 Hz).
ESI-MSm/z: 358 (M+H)⁺.

### 4) Methyl 1-(6-methoxy-3-pyridyl)-5-(4-pyrimidinyl)-1H-pyrazole-3-carboxylate

Raneynickel (used in an excessive amount, and an activatedproduct is used after washing with water and methanol) was added to a solution of the methyl 1-(6-methoxy-3-pyridyl)-5-(2-methylthio-4-pyrimidinyl)-1H-pyrazol e-3-carboxylate (198mg) inmethanol (25ml), and the mixture was stirred in a sealed tube at an outer temperature of 120°C for 16 hours. After cooling with air, chloroform was added to the reaction liquid and the insoluble content was removed by filtration. The filtrate solvent was evaporated under reduced pressure, and the residue was purified by column chromatography on silica gel (methanol-chloroform) to give methyl 1-(6-methoxy-3-pyridyl)-5-(4-pyrimidinyl)-1H-pyrazole-3-carboxylate (123 mg, 71%) as a solid.

¹H-NMR (400 MHz, CDCl₃)δ: 3.98 (3H, s), 3.99 (3H, s), 6.82 (1H, d, J = 8.8 Hz), 7.34 (1H, d, J = 5.1 Hz), 7.48 (1H, s), 7.67 (1H, dd, J = 8.8, 2.7 Hz), 8.14 (1H, d, J = 2.7 Hz), 8.75 (1H, d, J = 5.1 Hz), 9.11 (1H, s).
ESI-MSm/z: 312 (M+H)⁺.

### 5) The title compound

The procedure of Referential Example 9, Method B (4) was repeated by using the methyl 1-(6-methoxy-3-pyridyl)-5-(4-pyrimidinyl)-1H-pyrazole-3-carboxylate (122 mg) to give the title compound (100 mg, 86%) as a solid.

¹H-NMR (400 MHz, CDCl₃)δ: 4.00 (3H, s), 6.84 (1H, d, J = 8.8 Hz), 7.37 (1H, dd, J = 5.4, 1.2 Hz), 7.51 (1H, s), 7.67 (1H, dd, J = 8.8, 2.7 Hz), 8.16 (1H, d, J = 2.7 Hz), 8.78 (1H, d, J = 5.4 Hz), 9.12 (1H, d, J = 1.2 Hz).
ESI-MSm/z: 298 (M+H)⁺.

[Referential Example 45] 5-(5-Methyl-2-pyridyl)-1-(2-pyrazinyl)-1H-pyrazole-3-carboxylic acid

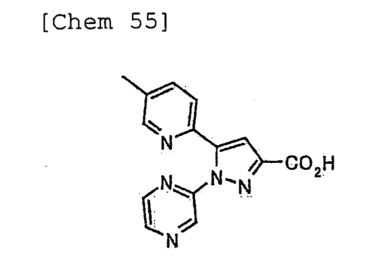

### 1) Ethyl 5-(5-methyl-2-pyridyl)-1-(2-pyrazinyl)-1H-pyrazole-3-carboxylate

A solution of the 2-hydrazinopyrazine(2.363 g) of Referential Example 33 and the ethyl 4-(5-methyl-2-pyridyl)-2,4-dioxobutanoate (5.04 g) of Referential Example 35(2) in ethanol (100 ml) was heated under reflux for 2 hours. After cooling with air, conc. hydrochloric acid (2.65 ml) was added to the reaction liquid, and the mixture was heated under reflux for 1 hour. After cooling with air, the reaction liquid was neutralized with 1N aqueous sodiumhydroxide, and the solution was extracted with chloroform. The aqueous layer was then extracted' with chloroform, and the combined layers were washed with brine and dried over anhydrous sodium sulfate. After filtration, the solvent was evaporated under reduced pressure, and the residue was purified by column chromatography on silica gel (acetone-chloroform) to give ethyl 5-(5-methyl-2-pyridyl)-1-(2-pyrazinyl)-1H-pyrazole-3-carboxylate (1.336 g, 22%) as an amorphous product.

¹H-NMR (400 MHz, CDCl₃)δ: 1.43 (3H, t, J = 6.9 Hz), 2.34 (3H, s), 4.47 (2H, q, J = 6.9 Hz), 7.23 (1H, s), 7.24-7.30 (1H, m), 7.46 (1H, d, J = 7.9 Hz), 7. 56 (1H, dd, J = 7.9, 1. 5 Hz), 8.21 (1H, br s), 8.28-8.32 (1H, m), 8.56 (1H, d, J = 2.4 Hz), 9.02 (1H, d like, J = 1.5 Hz).
FAB-MSm/z: 310 (M+H)⁺.

### 2) The title compound

Lithium hydroxide monohydrate (0.222 g) was added to a solution of the ethyl 5-(5-methyl-2-pyridyl)-1-(2-pyrazinyl)-1H-pyrazole-3-carboxylate (1.470 g) in a mixture of tetrahydrofuran (20 ml), ethanol (10 ml), and water (5 ml) at room temperature, and the mixture was stirred for 2.5 hours. The reaction liquid was acidified (to pH 5 to 6) by adding 1N aqueous hydrochloric acid, and a mixed solvent of chloroform and methanol (15:1) was added and the phases were separated. The aqueous layer was extracted with a mixed solvent of chloroform and methanol (15:1), and the combined organic layers were dried over anhydrous sodium sulfate. After filtration, the solvent was evaporated under reduced pressure to give the title compound (1.315 g, 98%) as a solid.

¹H-NMR (400 MHz, CDCl₃)δ: 2.35 (3H, s), 7.26 (1H, s), 7.48 (1H, d, J = 8.1 Hz), 7.58 (1H, d, J = 8.1, 1.0 Hz), 8.24 (1H, br s), 8.29 (1H, t like, J = 2.4 Hz), 8.58 (1H, d, J = 2.4 Hz), 9.04 (1H, s like). FAB-MSm/z: 282 (M+H)⁺.

[Referential Example 46] 1-(6-Methoxy-3-pyridyl)-5-(1H-pyrrol-2-yl)-1H-pyrazole-3-carboxylic acid

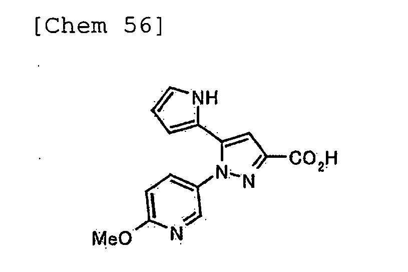

Diethyl oxalate (3.10 ml) and 1-[1-(phenylsulfonyl)-1H-pyrrol-2-yl]-1-ethanone (2.49 g) were added to a solution of sodium ethoxide (1.63 g) in ethanol (20 ml) under ice cooling, and the mixture was stirred at room temperature for 5 hours. To the reaction liquid were added the 5-hydrazino-2-methoxypyridine hydrochloride (2.52 g) of Referential Example 1 and ethanol (20 ml), and the mixture was heated under reflux for 14.5 hours. After cooling with air, the reaction solvent was evaporated under reduced pressure, and ethyl acetate and saturated aqueous sodium bicarbonate were added to the residue and the phases were separated. The aqueous layer was extracted with ethyl acetate, and the combined layers were dried over anhydrous sodium sulfate. After filtration, the solvent was evaporated under reduced pressure, and the residue was purified by column chromatography on silica gel (ethyl acetate-hexane) to give ethyl 1-(6-methoxy-3-pyridyl)-5-[1-(phenylsulfonyl)-1H-pyrrol-2-yl]-1H-pyrazole-3-carboxylate (3.28 g, 72%) as an oily product. To a solution of this ethyl ester derivative (3.28 g) in ethanol (22 ml) was added 1N aqueous sodium hydroxide (22 ml), and the mixture was stirred at room temperature for 2 days. 1N aqueous hydrochloric acid was added to the reaction liquid, and the solid content was collected by filtration to give the title compound (1.40 g, 68%) as a solid.

¹H-NMR (400 MHz, DMSO-d₆)δ: 3.94 (3H, s), 5.49-5.51 (1H, m), 5.98-6.00 (1H, m), 6.87-6.89 (1H, m), 6.98 (1H, dd, J = 8.8, 0.5 Hz), 7.08 (1H, s), 7.80 (1H, dd, J = 8.8, 2.7 Hz), 8.25 (1H, dd, J = 2.7, 0.5 Hz), 11.39 (1H, br s).
ESI-MSm/z: 285 (M+H)⁺.

[Referential Example 47] 5-(5-Methyl-2-pyridyl)-1-(3-pyridazinyl)-1H-pyrazole-3-carboxylic acid

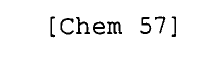

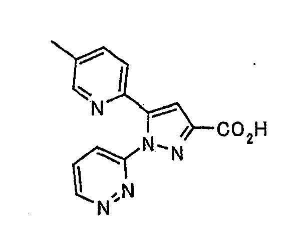

### 1) Ethyl 1-(6-chloro-3-pyridazinyl)-5-(5-methyl-2-pyridyl)-1H-pyrazole-3-carboxylate

Conc. hydrochloric acid (1.2 ml) was added to a solution of 3-chloro-6-hydrazinopyridazine (3.44 g) and the ethyl 4-(5-methyl-2-pyridyl)-2,4-dioxobutanoate (5.56 g) of Referential Example 35 (2) in ethanol (60 ml) at room temperature, and the mixture was heated under reflux for 16 hours. After cooling with air, the reaction liquid was neutralized with 1N aqueous sodium hydroxide, and the solid precipitate was collected by filtration to give ethyl 1-(6-chloro-3-pyridazinyl)-5-(5-methyl-2-pyridyl)-1H-pyrazole-3-carboxylate (4.48 g, 55%).

¹H-NMR (400 MHz, CDCl₃)δ: 1.43 (3H, t, J = 7.0 Hz), 2.34 (3H, s), 4.46 (2H, q, J = 7.0 Hz), 7.19 (1H, s), 7.51 (1H, d, J = 7.8 Hz), 7.57 (1H, dd, J = 1.0, 7.8 Hz), 7.68 (1H, d, J = 9.0 Hz), 8.08 (1H, d, J = 9.0 Hz), 8.21 (1H, d, J = 1.0 Hz).
FAB-MSm/z: 344 (M+H)⁺.

### 2) Ethyl 5-(5-methyl-2-pyridyl)-1-(3-pyridazinyl)-1H-pyrazole-3-carboxylate

To a solution of the ethyl 1-(6-chloro-3-pyridazinyl)-5-(5-methyl-2-pyridyl)-1H-pyrazole-3-c arboxylate (2.58 g) in ethanol (70 ml) were added 10% palladium on carbon (1.27 g) and ammonium formate (2.615 g) at room temperature, and the mixture was stirred at 75°C for 1 hour. After cooling with air, the insoluble content was removed by filtration, and the solvent was evaporated under reduced pressure. Water and chloroform were added to the residue and the phases were separated, and the aqueous layer was extracted with chloroform. The extract layers were washed with brine, and dried over anhydrous sodium sulfate. After filtration, the solvent was evaporated under reduced pressure, and the residue was purified by column chromatography on silica gel (methanol-chloroform) to give ethyl 5-(5-methyl-2-pyridyl)-1-(3-pyridazinyl)-1H-pyrazole-3-carboxylate(1.126 g, 49%) as a solid.

¹H-NMR (400 MHz, CDCl₃)δ: 1.43 (3H, t, J = 7.1 Hz), 2.32 (3H, s), 4.47 (2H, q, J = 7.1 Hz), 7.21 (1H, s), 7.51 (1H, d like, J = 8.0 Hz), 7.55-7.61 (1H, m), 7.66 (1H, dd, J = 4.9, 8.5 Hz), 8.07-8.13 (1H, m), 8.19 (1H, br), 9.12 (1H, dd, J = 2.5, 4.9 Hz).
ESI-MSm/z: 310 (M+H)⁺.

### 3) The title compound

Lithium hydroxide monohydrate (0.173 g) was added to a solution of the ethyl 5-(5-methyl-2-pyridyl)-1-(3-pyridazinyl)-1H-pyrazole-3-carboxylate (1.126 g) in tetrahydrofuran (20 ml), ethanol (10 ml), and water (20 ml) at room temperature, and the mixture was stirred for 2.5 hours. The reaction liquid was acidified (to pH 5) with 1N aqueous hydrochloric acid, and chloroform-methanol (15:1) was added to the solution and the phases were separated. The aqueous layer was extracted with chloroform-methanol (15:1), and the combined layers were dried over anhydrous sodium sulfate. After filtration, the solvent was evaporated under reducedpressure to give the title compound (0.688 g, 69%) as a solid.

¹H-NMR (400 MHz, DMSO-d₆)δ: 2.27 (3H, s), 7.37 (1H, s like), 7.67-7.79 (2H, m), 7.97 (1H, dd, J = 4.6, 8.3 Hz), 8.08 (1H, d, J = 8.3 Hz), 8.14 (1H, br s), 9.29 (1H, d, J = 4.6 Hz).
ESI-MSm/z: 282 (M+H)⁺.

[Referential Example 48] 1-(6-Methoxy-3-pyridyl)-5-(1H-pyrrol-1-yl)-1H-pyrazole-3-carboxylic acid

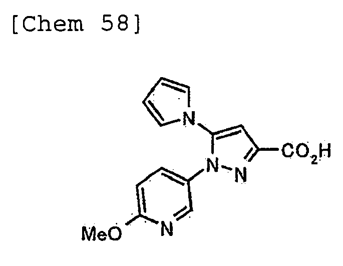

### 1) Ethyl 1-(6-methoxy-3-pyridyl)-5-(1H-pyrrol-1-yl)-1H-pyrazole-3-carboxylate

Diethyl oxalate (6.79 ml) was gradually added to a suspension of sodium ethoxide (3.40 g) in tert-butylmethyl ether (30 ml) at room temperature, and the mixture was stirred at 60°C for 10 minutes. Acetonitrile (2.61 ml) was gradually added to the reaction liquid, and the mixture was heated under reflux for 4 hours. After cooling with air, the resulting solid was collected by filtration to give 1-cyano-3-ethoxy-3-oxo-1-propen-2-ol sodium salt (6.17 g, 75%) as a solid. To a solution of the 5-hydrazino-2-methoxypyridine (5.00 g) of Referential Example 2 in ethanol (100 ml) were added a 1M hydrochloric acid in ethanol (36.0 ml) and the 1-cyano-3-ethoxy-3-oxo-1-propen-2-ol sodium salt (5.86 g), and the mixture was heated under reflux for 16 hours. After cooling with air, the reaction solvent was evaporated under reduced pressure, and ethyl acetate and saturated aqueous sodium bicarbonate were added to the residue and the phases were separated. The organic layer was dried over anhydrous sodium sulfate. After filtration, the solvent was evaporated under reduced pressure, and the residue was purified by column chromatography on silica gel (ethyl acetate-hexane) to give ethyl 5-amino-1-(6-methoxy-3-pyridyl)-1H-pyrazole-3-carboxylate (5.09 g, 54%) as a solid. To a solution of this 5-aminopyrazole derivative (2. 62 g) in acetic acid (50 ml) was added 2, 5-dimethoxy tetrahydrofuran (1.94 ml), and the mixture was heated under reflux for 3 hours. After cooling with air, the reaction solvent was evaporated under reduced pressure, and ethyl acetate and saturated aqueous sodium bicarbonate were added to the residue and the phases were separated, and the organic layer was dried over anhydrous sodium sulfate. After filtration, the solvent was evaporated under reduced pressure, and the residue was purified by column chromatography on silica gel (ethyl acetate-hexane) to give ethyl 1-(6-methoxy-3-pyridyl)-5-(1H-pyrrol-1-yl)-1H-pyrazole-3-carboxylate (2.92 g, 93%) as a solid.

¹H-NMR (400 MHz, CDCl₃)δ: 1.43 (3H, t, J = 7.1 Hz), 3.94 (3H, s), 4.46 (2H, q, J = 7.2 Hz), 6.28-6.30 (2H, m), 6.64-6.65 (2H, m), 6.72 (1H, d, J= 9.3 Hz), 6.92 (1H, s), 7.42 (1H, dd, J=8.8, 2.7 Hz), 8.00 (1H, d, J = 2.4 Hz).
ESI-MSm/z: 313 (M+H)⁺.

### 2) The title compound

To a solution of the ethyl 1-(6-methoxy-3-pyridyl)-5-(1H-pyrrol-1-yl)-1H-pyrazole-3-carboxyl ate (2.91 g) in a mixture of ethanol (30 ml) and tetrahydrofuran (15 ml) was added 1N aqueous sodium hydroxide (15.0 ml), and the mixture was stirred at room temperature for 3 hours. The reaction liquid was acidified with 1N aqueous hydrochloric acid, and ethyl acetate was added to the solution and the phases were separated. The organic layer was dried over anhydrous sodium sulfate. After filtration, the solvent was evaporated under reduced pressure to give the title compound (2.96 g, measured) as a solid.

¹H-NMR (400 MHz, DMSO-d₆)δ: 3.87 (3H, s), 6.21-6.22 (2H, m), 6.88-6.90 (3H, m), 7.02 (1H, s), 7.61 (1H, dd, J = 8.9, 2.8 Hz), 8.00 (1H, d, J = 2.7 Hz).
ESI-MSm/z: 285 (M+H)⁺.

[Referential Example 49] 5-(5-Methoxy-2-pyridyl)-1-(6-methyl-3-pyridyl)-1H-pyrazole-3-carboxylic acid

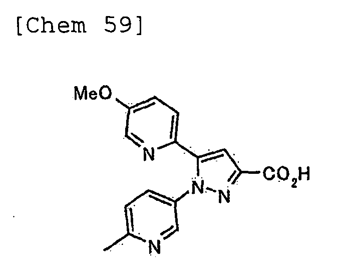

### 1) 5-methoxypyridine-2-carboxylic acid N-methoxy-N-methylamide

Under argon atmosphere, a solution of 5-hydroxy-2-methylpyridine (30.0g) in dimethyl sulfoxide (200ml) was added dropwise to a suspension of sodium hydride (55% in oil, 12.6 g) in dimethyl sulfoxide (100 ml) over 20 minutes at 0°C, and the mixture was stirred for 35 minutes. At the same temperature, methyl iodide (18.0 ml) was added to the reaction liquid over 15 minutes, and the mixture was stirred at room temperature for 2 hours. Water and diethylether were added to the reaction liquid and the phases were separated, and the organic layer was dried over anhydrous sodium sulfate. After filtration, the solvent was evaporated under reduced pressure to give 5-methoxy-2-methylpyridine (18.7 g).' To a solution of this crude product (18.7 g) in pyridine (187 ml) was added selenium dioxide (33.7g), and the mixture was heated under reflux for 62 hours. After cooling with air, water and chloroform were added to the reaction liquid and the phases were separated. The organic layer was dried over anhydrous sodium sulfate. After filtration, the solvent was evaporated under reduced pressure to give 5-methoxypyridine-2-carboxylic acid (19.1 g) . To a suspension of the thus obtained crude product (19.1 g), N,O-dimethylhydroxyamine hydrochloride (16.3 g), and 3-(3-dimethylaminopropyl)-1-ethyl-carbodiimide hydrochloride (32.1 g), and 1-hydroxybenzotriazole (22.6 g) in dichloromethane (250 ml) was added triethylamine (46.6 ml) at 0°C, and the mixture was stirred for 30 minutes, and the mixture was further stirred at room temperature for 14 hours. Water and chloroform were added to the reaction liquid and the phases were separated, and the organic layer was dried over anhydroussodiumsulfate. After filtration, the solvent was evaporated under reduced pressure, and the residue was purified by column chromatography on silica gel (ethyl acetate-chloroform) to give 5-methoxypyridine-2-carboxylic acid N-methoxy-N-methylamide (15.3 g, 51%) as an oily product.

¹H-NMR (400 MHz, CDCl₃)δ: 3.44 (3H, s), 3.79-3.84 (3H, m), 3.91 (3H, s), 7.24-7.28 (1H, m), 7.75 (1H, d, J = 8.5 Hz), 8.30 (1H, d, J = 2.9 Hz).
FAB-MSm/z: 197 (M+H)⁺.

### 2) 1-(5-methoxypyridin-2-yl)ethanone

The procedure of Referential Example 36 (2) was repeated by using the 5-methoxypyridine-2-carboxylic acid N-methoxy-N-methylamide (15.3 g) and methyl lithium (0.98M diethylether solution, 87.5 ml) under argon atmosphere to give 1-(5-methoxypyridine-2-yl)ethanone (5.41 g, 46%) as an oily product.

¹H-NMR (400 MHz, CDCl₃)δ: 2.69 (3H, s), 3.94 (3H, s), 7.27 (1H, dd, J = 8.5, 2.9 Hz), 8.06 (1H, d, J = 8.5 Hz), 8.34 (1H, d, J = 2.9 Hz). FAB-MSm/z: 152 (M+H)⁺.

### 3) Ethyl 4-(5-methoxy-2-pyridyl)-2,4-dioxobutanoate

Under argon atmosphere, diethyl oxalate (9.70 ml) was added to a solution of sodium ethoxide (4.86 g) in ethanol (54 ml) at room temperature, and a solution of the 1-(5-methoxypyridin-2-yl)ethanone (5.40 g) in ethanol (54 ml) was added dropwise to the reaction liquid at room temperature, and the mixture was stirred for 140 minutes. Water and diethylether were added to the reaction liquid and the phases were separated, and saturated aqueous ammonium chloride was added to the aqueous layer, and the solution was extracted with chloroform. The organic layer was dried over anhydrous sodium sulfate, and after filtration, the solvent was evaporated under reduced pressure to give ethyl 4-(5-methoxy-2-pyridyl)-2,4-dioxobutanoate (5.10 g, 57%) as a solid.

¹H-NMR (400 MHz, CDCl₃)δ: 1.39-1.43 (3H, m), 3.96 (3H, s), 4.37-4.42 (2H, m), 7.31 (1H, dd, J = 8.8, 2.9 Hz), 7.60 (1H, s), 8.16 (1H, d, J = 8.8 Hz), 8.38 (1H, d, J = 2.9 Hz) .
EI-MSm/z: 251 (M⁺).

### 4) Ethyl 5-(5-methoxy-2-pyridyl)-1-(6-methyl-3-pyridyl)-1H-pyrazole-3-carboxylate

A solution of the ethyl 4-(5-methoxy-2-pyridyl)-2,4-dioxobutanoate (4.79 g) and the 5-hydrazino-2-methylpyridine (2.35 g) of Referential Example 31 in ethanol (96 ml) was heated under reflux for 1 hour, and acetic acid (5.47 ml) was added to the reaction liquid. The mixture was heated under reflux for 63 hours. After cooling with air, saturated aqueous sodium bicarbonate and chloroform were added to the reaction liquid and the phases were separated. The organic layer was dried over anhydrous sodium sulfate, and after filtration, the solvent was evaporated under reduced pressure. The residue was purified by column chromatography on silica gel (acetone-toluene) to give ethyl 5-(5-methoxy-2-pyridyl)-1-(6-methyl-3-pyridyl)-1H-pyrazole-3-carb oxylate (1.49 g, 23%) as a solid.

¹H-NMR (400 MHz, CDCl₃)δ: 1.41-1.44 (3H, m), 2.59 (3H, s), 3.86 (3H, s), 4.43-4.48 (2H, m), 7.16-7.34 (4H, m), 7.71-7.73 (1H, m), 8.19 (1H, d, J = 2.8 Hz), 8.38 (1H, d, J = 2.4 Hz).
FAB-MSm/z: 339 (M+H)⁺.

### 5) The title compound

The procedure of Referential Example 9, Method B (4) was repeated by using the ethyl 5-(5-methoxy-2-pyridyl)-1-(6-methyl-3-pyridyl)-1H-pyrazole-3-carb oxylate (1.48 g) to give the title compound (0.970 g, 71%) as a solid.

¹H-NMR (400 MHz, DMSO-d₆)δ: 2.53 (3H, s), 3.84 (3H, m), 7.23 (1H, m), 7.34 (1H, d, J = 8.5 Hz), 7.45-7.48 (1H, m), 7.63-7.67 (2H, m), 8.16 (1H, d, J = 3.2 Hz), 8.37 (1H, d, J = 2.7 Hz), 13.0.3 (1H, br s).
FAB-MSm/z: 311 (M+H)⁺.

[Referential Example 50] 5-(5-Methyl-2-pyridyl)-1-(2-pyrimidinyl)-1H-pyrazole-3-carboxylic acid

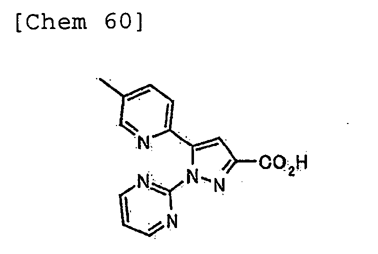

### 1) Ethyl 5-(5-methyl-2-pyridyl)-1-(2-pyrimidinyl)-1H-pyrazole-3-carboxylate

Acetic acid (2.60 ml) was added to a suspension of the ethyl 4-(5-methyl-2-pyridyl)-2,4-dioxobutanoate (2.14 g) of Referential Example 352) and the 2-hydrazinopyrimidine (1.00 g) of Referential Example 34 in ethanol (43 ml) at room temperature, and the mixture was heated under reflux for 16 hours. After cooling with air, conc. hydrochloric acid (2.9 ml) was added, and the mixture was heated under reflux for 110 minutes. After cooling with air, saturated aqueous sodium bicarbonate and chloroform were added to the reaction liquid and the phases were separated, and the organic layer was dried over anhydroussodiumsulfate. After filtration, the solvent was evaporated under reduced pressure, and the residue was purified by column chromatography on silica gel (acetone-toluene) to give ethyl 5-(5-methyl-2-pyridyl)-1-(2-pyrimidinyl)-1H-pyrazole-3-carboxylate (1.22 g, 43%) as a solid.

¹H-NMR (400 MHz, CDCl₃)δ: 1.42 (3H, t, J = 7.2 Hz), 2.34 (3H, s), 4.45 (2H, q, J = 7.2 Hz), 7.22 (1H, s), 7.29-7.31 (1H, m), 7.44 (1H, d, J = 8.1 Hz), 7.54 (1H, dd, J = 8.1, 2.2 Hz), 8.21-8.22 (1H, m), 8.72 (2H, d, J = 4.9 Hz).
ESI-MSm/z: 309 (M⁺).

### 2) The title compound

A solution of lithium hydroxide monohydrate (0.181 g) in water (12 ml) was added dropwise to a solution of the ethyl 5-(5-methyl-2-pyridyl)-1-(2-pyrimidinyl)-1H-pyrazole-3-carboxylate (1.21 g) in tetrahydrofuran (24 ml) at room temperature, and the mixture was stirred for 3 hours. The reaction liquid was neutralized by adding 1N aqueous hydrochloric acid (4.30 ml), and water and a mixed solvent of methanol and chloroform (1:10) were added and the phases were separated. The solvent of the organic layer was evaporatedunder reducedpressure, and the resulting solid was collected by filtration to give the title compound (1.01 g, 92%) as a solid.

¹H-NMR (400 MHz, DMSO-d₆)δ: 2.27 (3H, s), 7.31 (1H, s), 7.61-7.72 (3H, m), 8.11 (1H, s), 8.86 (2H, d, J = 4.9 Hz), 13.11 (1H, br s).
ESI-MSm/z: 281 (M⁺).

[Referential Example 51] 1-(6-Methoxy-3-pyridazinyl)-5-(2-pyrazinyl)-1H-pyrazole-3-carboxylic acid

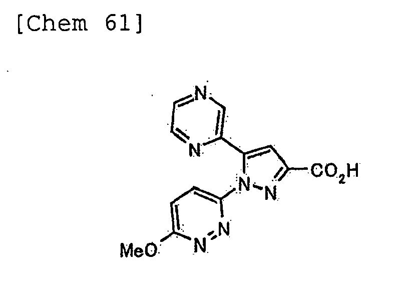

### 1) Methyl 1-(6-chloro-3-pyridazinyl)-5-(2-pyrazinyl)-1H-pyrazole-3-carboxylate

Lithium bis(trimethylsilyl)amide (1.0M solution in tetrahydrofuran, 55.0 ml) was added to a solution of 1- (2-pyrazinyl) -1-ethanone (6.10 g) in tetrahydrofuran (50 ml) at -78°C, and the solution was stirred for 45 minutes. Dimethyl oxalate (8.85 g) was added to the reaction liquid, and the solution was stirred for 10 minutes, and the stirring was continued for another 2.5 hours while resuming room temperature. Diethylether and water were added to the reaction liquid and the aqueous layer was separated. To the aqueous layer was added 1N aqueous hydrochloric acid (55ml), and then, sodium chloride until saturation. The aqueous layer was extracted with diethylether, and it was further extracted with ethyl acetate. The combined organic layers were dried over anhydrous sodium sulfate. After filtration, the solvent was evaporated under reduced pressure to give methyl 4-(2-pyrazinyl)-2,4-dioxobutanoate (10.0 g, 96%) as a solid. To a solution of this crude methyl butanoate derivative (6.27 g) in methanol (150 ml) was added 3-chloro-6-hydrazinopyridazine (4.35 g), and the mixture was heated under reflux for 18.5 hours. Conc. hydrochloric acid (0.750 ml) was added to the reaction liquid, and the mixture was heated under reflux for 2 hours. After cooling with air, ethyl acetate and saturated aqueous sodium bicarbonate were added to the reaction liquid and the phases were separated, and the organic layer was dried over anhydrous sodium sulfate. After filtration, the solvent was evaporated under reduced pressure. Methanol was added to the residue, and the resulting solid was collected by filtration to give methyl 1-(6-chloro-3-pyridazinyl)-5-(2-pyrazinyl)-1H-pyrazole-3-carboxylate (5.74 g, 60%) as a solid.

¹H-NMR (400 MHz, CDCl₃)δ: 4.02 (3H, s), 7.33 (1H, s), 7.72 (1H, d, J = 9.3 Hz), 8.16 (1H, d, J = 9.0 Hz), 8.42 (1H, dd, J = 2.4, 1.5 Hz), 8.57 (1H, d, J = 2.7 Hz), 8.90 (1H, d, J = 1.5 Hz).
ESI-MSm/z: 317[(M+H)⁺, ³⁵Cl], 319[(M+H)⁺, ³⁷Cl].

### 2) The title compound

Sodium methoxide (1.60 g) was added to a suspension of the methyl 1-(6-chloro-3-pyridazinyl)-5-(2-pyrazinyl)-1H-pyrazole-3-carboxylate (6.25 g) in methanol (50 ml) and tetrahydrofuran (100 ml) at room temperature, and the mixture was stirred for 8 hours. 1N aqueous sodium hydroxide (40.0 ml) was added to the reaction liquid, and the mixture was stirred for 19 hours. Diethylether and water were added to the reaction liquid and the aqueous layer was separated, and the aqueous layer was acidified with hydrochloric acid, and the solution was extracted with diethylether. The aqueous layer was extracted with ethyl acetate, and the combined organic layers were dried over anhydrous sodium sulfate. After filtration, the solvent was evaporated under reduced pressure. Methanol and diethylether were added to the residue, and the resulting solid was collected to give the title compound (4.37 g, 74%) as a solid.

¹H-NMR (400 MHz, DMSO-d₆)δ: 4.04 (3H, s), 7.51 (1H, d, J = 9.3 Hz), 7.55 (1H, s), 8.06 (1H, d, J = 9.3 Hz), 8.49 (1H, dd, J = 2.4, 1.5 Hz), 8.62 (1H, d, J = 2.4 Hz), 9.07 (1H, d, J = 1.5 Hz).
ESI-MSm/z: 299 (M+H)⁺.

[Referential Example 52] 5-(5-Fluoro-2-pyridyl)-1-(3-pyridyl)-1H-pyrazole-3-carboxylic acid

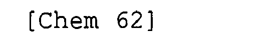

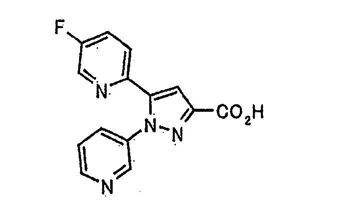

### 1) Ethyl 5-(5-amino-2-pyridyl)-1-(3-pyridyl)-1H-pyrazole-3-carboxylate

Conc. hydrochloric acid (0.59 ml) was added to a solution of the ethyl 5-[5-(tert-butoxycarbonylamino)-2-pyridyl]-4,5-dihydro-5-hydroxy-1-(3-pyridyl)-1H-pyrazole-3-carboxylate (1.205 g) of Referential Example 41(4) in ethanol (30 ml), and the mixture was heated under reflux for 14 hours. The reaction solvent was evaporated under reduced pressure, and saturated aqueous sodium bicarbonate and ethyl acetate were added to the residue and the phases were separated. The organic layer was dried over anhydrous sodium sulfate. After filtration, the solvent was evaporated under reduced pressure, and the residue was purified by column chromatography on silica gel (methanol-chloroform) to give ethyl 5-(5-amino-2-pyridyl)-1-(3-pyridyl)-1H-pyrazole-3-carboxylate (609 mg, 69%) as an oily product.

¹H-NMR (400 MHz, CDCl₃)δ: 1.42 (3H, t, J = 7.1 Hz), 3.85 (2H, br s), 4.45 (2H, q, J = 7.1 Hz), 6.95 (1H, dd, J = 8.5, 2.9 Hz), 7.15 (1H, s), 7.20 (1H, d, J = 8.5 Hz), 7.35 (1H, ddd, J = 8.3, 4.9, 0.7 Hz), 7.84 (1H, ddd, J = 8.3, 2.7, 1.5 Hz), 7.93 (1H, dd, J = 2.9, 0.5 Hz), 8.53 (1H, dd, J = 2.4, 0.5 Hz), 8.57 (1H, dd, J = 4.9, 1.5 Hz).
ESI-MSm/z: 310 (M+H)⁺.

### 2) Ethyl 5-(5-fluoro-2-pyridyl)-1-(3-pyridyl)-1H-pyrazole-3-carboxylate

To a solution of the ethyl 5-(5-amino-2-pyridyl)-1-(3-pyridyl)-1H-pyrazole-3-carboxylate (604 mg) in ethanol (30 ml) was added 42% tetrafluoroboric acid (15 ml), and ethyl nitrite (15% ethanol solution, 3.9 ml) was added to the reaction liquid at 0°C, and the mixture was stirred for 20 minutes. Diethylether (200 ml) was added to the reaction liquid, and diethylether was removed by decantation. To the resulting viscous oilyproduct was added toluene (50 ml), and the mixture was heated under reflux for 1 hour. After cooling with air, the reaction solvent was evaporated under reduced pressure, and saturated aqueous sodium bicarbonate and ethyl acetate were added to the residue and the phases were separated. The organic layer was dried over anhydrous sodium sulfate. After filtration, the solvent was evaporated under reduced pressure, and the residue was purified by thin layer chromatography on silica gel (hexane-ethyl acetate) to give ethyl 5-(5-fluoro-2-pyridyl)-1-(3-pyridyl)-1H-pyrazole-3-carboxylate (282 mg, 46%) as a solid.

¹H-NMR (400 MHz, CDCl₃)δ: 1.42 (3H, t, J = 7.1 Hz), 3.85 (2H, br s), 4.45 (2H, q, J = 7.1 Hz), 6.95 (1H, dd, J = 8.5, 2.9 Hz), 7.15 (1H, s), 7.20 (1H, d, J = 8.5 Hz), 7.35 (1H, ddd, J = 8.3, 4.9, 0.7 Hz), 7.84 (1H, ddd, J = 8.3, 2.7, 1.5 Hz), 7.93 (1H, dd, J = 2.9, 0.5 Hz), 8.53 (1H, dd, J = 2.4, 0.5 Hz), 8.57 (1H, dd, J = 4.9, 1.5 Hz).
ESI-MSm/z: 313 (M+H)⁺.

### 3) The title compound

To a solution of the ethyl 5-(5-fluoro-2-pyridyl)-1-(3-pyridyl)-1H-pyrazole-3-carboxylate (282 mg) in ethanol (10 ml) was added 1N aqueous sodium hydroxide (2.7 ml) at room temperature, and the mixture was stirred for 2 hours. The reaction solvent was evaporated under reduced pressure, and water and 1N aqueous hydrochloric acid (2.7 ml) were added to the residue. The solidprecipitate was collected by filtration to give the title compound (249 mg) as a solid.

¹H-NMR (400 MHz, DMSO-d₆)δ: 7.38 (1H, s), 7.50 (1H, dd, J = 8.3, 4.9 Hz), 7.78-7.90 (3H, m), 8.41 (1H, d, J = 2.2 Hz), 8.53 (1H, d, J = 2.4 Hz), 8.62 (1H, dd, J = 4.9, 1.2 Hz).
ESI-MSm/z: 285 (M+H)⁺.

[Referential Example 53] 1-(6-Methoxy-3-pyridyl)-5-(4-methoxy-2-pyridyl)-1H-pyrazole-3-carboxylic acid

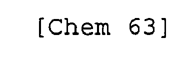

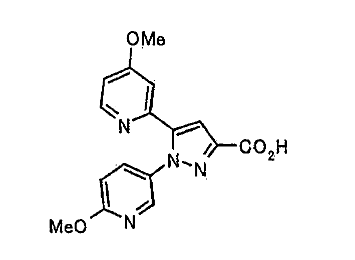

### 1) 4-Methoxypyridine-2-carbonitrile

Under argon atmosphere, triethylamine (17.8 ml) was added to a solution of 4-methoxypyridine-N-oxide (8.0 g) in acetonitrile (160 ml) at room temperature, and trimethylsilylcyanide (24.1 ml) was added dropwise to the solution. The mixture was stirred for 20 minutes, and heated under reflux for another 14 hours. After cooling with air, the reaction solvent was evaporated under reduced pressure. Saturated aqueous sodium bicarbonate and ethyl acetate were added to the residue, and the phases were separated. The organic layer was dried over anhydrous sodium sulfate. After filtration, the solventwas evaporated under reduced pressure, and the residue was purified by column chromatography on silica gel (hexane-ethyl acetate) to give 4-methoxypyridine-2-carbonitrile (1.57 g, 18%) as a solid.

¹H-NMR (400 MHz, CDCl₃)δ: 3.91 (3H, s), 7.00-7.02 (1H, m), 7.22 (1H, d, J = 2.4 Hz), 8.51 (1H, d, J = 6.0 Hz).
EI-MSm/z: 134 (M⁺).

### 2) 1-(4-Methoxy-2-pyridyl)ethanone

Under argon atmosphere, methyl magnesium bromide (0.93M solution in tetrahydrofuran , 13.8 ml) was added dropwise to a solution of the 4-methoxypyridine-2-carbonitrile (1.56 g) in tetrahydrofuran (31 ml) at -78°C, and the mixture was stirred for 15 minutes. The mixture was stirred at 0°C for another 15 minutes, and at room temperature for another 5 hours. Water and ethyl acetate were added to the reaction liquid and the phases were separated, and the organic layer was dried over anhydrous sodium sulfate. After filtration, the solvent was evaporated under reduced pressure, and the residue was purified by column chromatography on silica gel (hexane-ethyl acetate) to give 1-(4-methoxy-2-pyridyl)ethanone (1.30 g, 73%) as a solid.

¹H-NMR (400 MHz, CDCl₃)δ: 2.72 (3H, s), 3.91 (3H, s), 6.97-6.99 (1H, m), 7.57-7.58 (1H, m), 8.48-8.50 (1H, m).
ESI-MSm/z: 152 (M+H)⁺.

### 3) Ethyl 4-(4-methoxy-2-pyridyl)-2,4-dioxobutanoate

The procedure of Referential Example 3 was repeated by using the 1- (4-methoxy-2-pyridyl) ethanone (1.28 g) and diethyl oxalate (2.30 ml) to give ethyl 4-(4-methoxy-2-pyridyl)-2,4-dioxobutanoate (0.713 g, 33%) as a solid.

¹H-NMR (400 MHz, CDCl₃)δ: 1.39-1.43 (3H, m), 3.96 (3H, s), 4.37-4.42 (2H, m), 7.03-7.05 (1H, m), 7.72 (1H, d, J = 2.8 Hz), 8.02 (1H, s), 8.50 (1H, d, J = 5.6 Hz).
ESI-MSm/z: 251 (M⁺).

### 4) Ethyl 1-(6-methoxy-3-pyridyl)-5-(4-methoxy-2-pyridyl)-1H-pyrazole-3-car boxylate

The procedure of Referential Example 4 was repeated by using the ethyl 4-(4-methoxy-2-pyridyl)-2,4-dioxobutanoate (0.691 g) and the 5-hydrazino-2-methoxypyridine (0.383 g) of Referential Example 2 to give ethyl 1-(6-methoxy-3-pyridyl)-5-(4-methoxy-2-pyridyl)-1H-pyrazole-3-car boxylate (0.473 g, 49%) as a solid.

¹H-NMR (400 MHz, CDCl₃)δ: 1.41-1.44 (3H, m), 3.82 (3H, s), 3.95 (3H, s), 4.43-4.48 (2H, m), 6.75-6.78 (2H, m), 6.89 (1H, d, J = 2.4 Hz), 7.25 (1H, s), 7.68 (1H, dd, J = 8.8, 2.4 Hz), 8.11 (1H, d, J = 2.4 Hz), 8.33 (1H, d, J = 5.6 Hz).
FAB-MSm/z: 355 (M+H)⁺.

### 5) The title compound

To a solution of the ethyl 1-(6-methoxy-3-pyridyl)-5-(4-methoxy-2-pyridyl)-1H-pyrazole-3-car boxylate (0.416 g) in a mixture of methanol (6.3 ml) and tetrahydrofuran (6.3 ml) was added 1N aqueous sodium hydroxide (2.23 ml) at room temperature, and the mixture was stirred for 5 hours. The reaction liquid was neutralized by adding 1N aqueous hydrochloric acid (2.23 ml), and water and chloroform were added to the solution, and the phases were separated. The aqueous layer was extracted twice with chloroform, and the combined organic layers were dried over anhydrous sodium sul fate. After filtration, the solvent was evaporated under reduced pressure to give the title compound (0.353 g, 92%) as a solid.

¹H-NMR (400 MHz, DMSO-d₆)δ: 3.86 (3H, s), 3.89 (3H, s), 6.88 (1H, d, J = 8.8 Hz), 6.93 (1H, dd, J = 5.6, 2.4 Hz), 7.29 (1H, d, J = 2.4 Hz), 7.37 (1H, s), 7.69-7.72 (1H, m), 8.14 (1H, d, J = 2.8 Hz), 8.24 (1H, d, J = 5.6 Hz), 13.05 (1H, br).
FAB-MSm/z: 327 (M+H)⁺.

[Referential Example 54] 1-(6-Methoxy-3-pyridyl)-5-(4-methyl-2-pyridyl)-1H-pyrazole-3-carboxylic acid

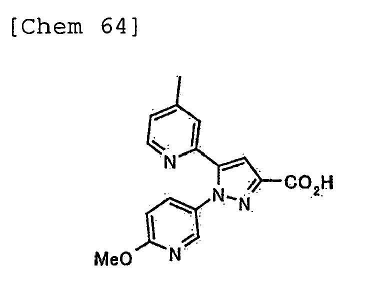

### 1) 4-Methylpyridine-2-carbonitrile

The procedure of Referential Example 53 (1) was repeated by using 4-methylpyridine-N-oxide (6.00 g) to give 4-methylpyridine-2-carbonitrile (4.65 g, 72%) as a solid.

¹H-NMR (400 MHz, CDCl₃)δ: 2.44 (3H, s), 7.33-7.35 (1H, m), 7.53 (1H, s), 8.57 (1H, d, J = 4.8 Hz).
EI-MSm/z: 118 (M⁺).

### 2) 1-(4-Methyl-2-pyridyl)ethanone

The procedure of Referential Example 53 (2) was repeated by using the 4-methylpyridine-2-carbonitrile (4.46 g) to give 1-(4-methyl-2-pyridyl)ethanone (4.38 g, 86%) as an oily product.

¹H-NMR (400 MHz, CDCl₃)δ: 2.43 (3H, s), 2.72 (3H, s), 7.28-7.29 (1H, m), 7.87 (1H, m), 8.54 (1H, d, J = 5.2 Hz).
EI-MSm/z: 135 (M⁺).

### 3) Ethyl 4-(4-methyl-2-pyridyl)-2,4-dioxobutanoate

Diethyl oxalate (4.42 ml) was added to a solution of sodium ethoxide (2.22 g) in ethanol (22 ml), and the mixture was stirred for 10 minutes. To this mixture was added a solution of the 1-(4-methyl-2-pyridyl)ethanone (2.20 g) in ethanol (22 ml), and the mixture was stirred at room temperature for 20 minutes. After adding water, the reaction liquid was washed with diethylether. Saturated aqueous ammonium chloride and chloroform were added to the aqueous layer and the phases were separated, and the organic layer was dried over anhydrous sodium sulfate. After filtration, the solvent was evaporated under reduced pressure to give ethyl 4-(4-methyl-2-pyridyl)-2,4-dioxobutanoate (2.84 g, 74%) as an oily product.

¹H-NMR (400 MHz, CDCl₃)δ: 1.41 (3H, t, J = 7.2 Hz), 2.47 (3H, s), 4.40' (2H, q, J = 7.2 Hz), 7.34-7.35 (1H, m), 7.52 (1H, br), 8.01 (1H, s), 8.57 (1H, d, J = 5.2 Hz).
EI-MSm/z: 235 (M⁺).

### 4) Ethyl 1-(6-methoxy-3-pyridyl)-5-(4-methyl-2-pyridyl)-1H-pyrazole-3-carb oxylate

The procedure of Referential Example 8 (2) was repeated by using the ethyl 4- (4-methyl-2-pyridyl) -2,4-dioxobutanoate (2.83 g) and the 5-hydrazino-2-methoxypyridine (1.67 g) of Referential Example 2 to give ethyl 1-(6-methoxy-3-pyridyl)-5-(4-methyl-2-pyridyl)-1H-pyrazole-3-carb oxylate (1.66 g, 41%) as a solid.

¹H-NMR (400 MHz, CDCl₃)δ: 1.43 (3H, t, J = 7.2 Hz), 2.34 (3H, s), 3.94 (3H, s), 4.46 (2H, q, J = 7.2 Hz), 6.76 (1H, d, J = 8.8 Hz), 7.05-7.06 (1H, m), 7.23-7.24 (2H, m), 7.66-7.69 (1H, m), 8.10 (1H, d, J = 2.8 Hz), 8.36 (1H, d, J = 4.8 Hz).
EI-MSm/z: 338 (M⁺).

### 5) The title compound

The procedure of Referential Example 9, Method B (4) was repeated by using the ethyl 1-(6-methoxy-3-pyridyl)-5-(4-methyl-2-pyridyl)pyrazole-3-carboxyl ate (1.04 g) to give the title compound (0.944 g, 99%) as a solid.

¹H-NMR (400 MHz, DMSO-d₆)δ: 2.43 (3H, s), 3.89 (3H, s), 6.87 (1H, d, J = 8. 8 Hz), 7.17-7.19 (1H, m), 7. 30 (1H, s), 7. 59 (1H, s), 7.68-7.71 (1H, m), 8.13 (1H, d, J = 2.8 Hz), 8.27-8.30 (1H, m), 13.04 (1H, br). EI-MSm/z: 310 (M⁺).

[Referential Example 55] 5-(6-Methoxy-3-pyridyl)-1-(3-pyridyl)-1H-pyrazole-3-carboxylic acid

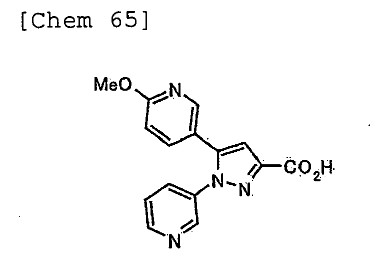

### 1) 3-Acetyl-6-methoxypyridine

To a solution of methyl 6-methoxynicotinate (20.07 g) in methanol (200 ml) was added 1N aqueous sodium hydroxide (140 ml) at room temperature, and the mixture was stirred for 16 hours. The solvent was evaporated under reduced pressure, and the residue was acidified (to pH 4) by adding 1N aqueous hydrochloric acid. The solid precipitate was collected by filtration to give 6-methoxynicotinic acid (15.12 g, 82%) (ESI-MSm/z: 154(M+H)⁺). To a solution of the thus obtained 6-methoxynicotinic acid (15.0g) in dichloromethane (600 ml) were added N,O-dimethyl hydroxylamine hydrochloride (11.5 g), 1-(3-dimethylaminopropyl)-3-ethylcarbodiimide hydrochloride (41.0 g), 1-hydroxybenzotriazole (14.5 g), and triethylamine (54 ml) at room temperature, and the mixture was stirred for 16 hours. Water and dichloromethane were added to the reaction liquid and the phases were separated, and the aqueous layer was extracted with dichloromethane. The combined organic layers were washed with brine, and dried over anhydroussodiumsulfate. After filtration, the solvent was evaporated under reduced pressure, and the residue was purified by column chromatography on silica gel (acetone-chloroform) to give 6-methoxynicotinic acid N-methoxy-N-methylamide (19.2 g, measured) as an oily product (ESI-MSm/z: 197(M+H)⁺). Methyl lithium (0.98M solution in diethylether, 135 ml) was added dropwise to a solution of this 6-methoxynicotinic acid N-methoxy-N-methylamide (19.21 g) in tetrahydrofuran (400 ml) over 30 minutes at -78°C, the mixture was stirred for another 30 minutes. Saturated aqueous ammonium chloride, water, and ethyl acetate were added to the reaction liquid and the phases were separated, and the aqueous layer was further extracted with ethyl acetate. The combined organic layers were washed with brine, and dried over anhydrous sodium sulfate. After filtration, the solvent was evaporated under reduced pressure, and diethylether was added to the residue. The solid precipitate was collected by filtration to give the title compound (10.86 g, 73%).

¹H-NMR (400 MHz, CDCl₃)δ: 2.57 (3H, s), 4.01 (3H, s), 6.79 (1H, d, J = 8.8 Hz), 8.14 (1H, dd, J = 2.5, 8.8 Hz), 8.78 (1H, d, J = 2.5 Hz). ESI-MSm/z: 152 (M+H)⁺.

### 2) Methyl 4-(6-methoxy-3-pyridyl)-2,4-dioxobutanoate

### [Method A]

Sodium methoxide (0.229 g) was added to a solution of the 3-acetyl-6-methoxypyridine (0.309 g) and dimethyl oxalate (0.484 g) in methanol (15 ml) at room temperature, and the mixture was stirred for 1.5 hours. The mixture was stirred at 45°C for another 20 hours. After cooling with air, the solidprecipitate was collected by filtration, washed with diethylether, and after the solidwas dissolved in chloroform and water, the solution was acidified by 1N aqueous hydrochloric acid and the phases were separated. The aqueous layer was extracted with chloroform, and the combined organic layers were washed with brine, and dried over anhydrous sodium sulfate. After filtration, the solvent was evaporated under reduced pressure to give methyl 4-(6-methoxy-3-pyridyl)-2,4-dioxobutanoate (0.294 g, 61%) as a solid.

¹H-NMR (400 MHz, CDCl₃)δ: 3.94 (3H, s), 4.03 (3H, s), 6.83 (1H, d like, J = 8.8 Hz), 7.00 (1H, s), 8.15 (1H, dd, J = 8.8, 2.5 Hz), 8.84 (1H, d, J = 2.5 Hz).
ESI-MSm/z: 238 (M+H)⁺.

### [Method B]

Sodium hydride (55%, 0.185 g) was added to a solution of the 3-acetyl-6-methoxypyridine (0.321 g) in N,N-dimethylformamide (6.0 ml) at 0°C, and the mixture was stirred for 25 minutes. Dimethyl oxalate (0.498 g) was added to the reaction liquid at 0°C, and the mixture was stirred at room temperature for 1 hour. Water and diethylether were added to the reaction liquid and the aqueous layer was separated, and the aqueous layer was acidified (to pH 4) with 1N aqueous hydrochloric acid, and the solution was extracted with ethyl acetate. The aqueous layer was further extracted with ethyl acetate, and the combined organic layers were washed with brine, and dried over anhydrous sodium sulfate.
After filtration, the solvent was evaporated under reduced pressure to give methyl 4-(6-methoxy-3-pyridyl)-2,4-dioxobutanoate (0.504 g, measured) as a solid.

### 3) Methyl 5-(6-methoxy-3-pyridyl)-1-(3-pyridyl)-1H-pyrazole-3-carboxylate

The 3-hydrazinopyridine (3.45 g) of Referential Example 32 was added to a solution of the methyl 4-(6-methoxy-3-pyridyl)-2,4-dioxobutanoate (6.80 g) in methanol (120 ml) at room temperature, and the mixture was heated under reflux for 30 minutes. After cooling with air, acetic acid (6.5 ml) was added and the mixture was heated under reflux for 14 hours. After cooling with air, the solvent of the reaction liquidwas evaporated under reduced pressure. Water and chloroform were added to the residue, and the mixture was neutralized with 1N aqueous sodium hydroxide and the phases were separated. The aqueous layer was extracted with chloroform, and the combined organic layers were washed with brine, and dried over anhydrous sodium sulfate. After filtration, the solvent was evaporated under reduced pressure, and the residue was purified by column chromatography on silica gel (acetone-chloroform) to give methyl 5-(6-methoxy-3-pyridyl)-1-(3-pyridyl)-1H-pyrazole-3-carboxylate (4.53 g, 51%) as an oily product.

¹H-NMR (400 MHz, CDCl₃)δ: 3.99 (3H, s), 4.03 (3H, s), 6.71 (1H, d, J = 8.5 Hz), 7.06 (1H, s), 7.32-7.35 (2H, m), 7.72-7.80 (1H, m), 8.09 (1H, d, J = 8.5 Hz), 8.58 (1H, d, J = 2.4 Hz), 8.62 (1H, dd, J = 2.4, 4.8 Hz).
FAB-MSm/z: 311 (M+H)⁺.

### 4) The title compound

Lithium hydroxide monohydrate (0.730 g) was added to a solution of the methyl 5-(6-methoxy-3-pyridyl)-1-(3-pyridyl)-1H-pyrazole-3-carboxylate (4.89 g) in tetrahydrofuran (30 ml), methanol (15 ml), and water (30 ml) at room temperature, and the mixture was stirred for 1.5 hours. The reaction solvent was evaporated under reduced pressure, and the residue was acidified (to pH 6 to 5) with 1N aqueous hydrochloric acid. The solid precipitate was collected by filtration to give the title compound (3.278 g, 70%).

¹H-NMR (400 MHz, DMSO-d₆)δ: 3.86 (3H, s), 6.83 (1H, d, J = 8.8 Hz), 7.16 (1H, s), 7.52-7.60 (2H, m), 7.83-7.91 (1H, m), 8.17 (1H, d, J = 2.4 Hz), 8.58 (1H, d, J = 2.4 Hz), 8.64 (1H, dd, J = 4.9, 1.5 Hz).
ESI-MSm/z: 297 (M+H)⁺.

[Referential Example 56] 5-(6-Methoxy-3-pyridyl)-1-(2-pyridyl)-1H-pyrazole-3-carboxylic acid

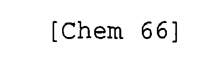

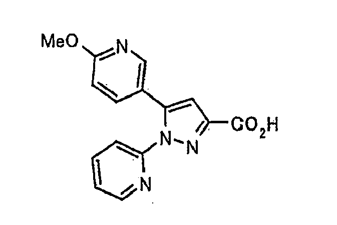

### 1) Methyl 5-(6-methoxy-3-pyridyl)-1-(2-pyridyl)-1H-pyrazole-3-carboxylate

The procedure of Referential Example 55 (3) was repeated by using the methyl 4-(6-methoxy-3-pyridyl)-2,4-dioxobutanoate (0.869 g) of Referential Example 55(2) and 2-hydrazinopyridine (0.628 g) to give methyl 5-(6-methoxy-3-pyridyl)-1-(2-pyridyl)-1H-pyrazole-3-carboxylate (0.464 g, 41%) as an oily product.

¹H-NMR (400 MHz, CDCl₃)δ: 3.95 (3H, s), 3.98 (3H, s), 6.70 (1H, d like, J = 8.6 Hz), 7.01 (1H, s), 7.25-7.35 (1H, m), 7.46 (1H, dd, J = 8.6, 2.4 Hz), 7.73 (1H, d like, J = 8.0 Hz), 7.81-7.88 (1H, m), 8.12 (1H, d like, J = 2.0 Hz), 8.32-8.36 (1H, m).
ESI-MSm/z: 311 (M+H)⁺.

### 2) The title compound

The procedure of Referential Example 55 (4) was repeated by using the methyl 5-(6-methoxy-3-pyridyl)-1-(2-pyridyl)-1H-pyrazole-3-carboxylate (0.464 g) and lithium hydroxide monohydrate (70.2 mg) to give the title compound (0.133 g, 29%) as a solid.

¹H-NMR (400 MHz, DMSO-d₆)δ: 3.85 (3H, s), 6.79 (1H, d, J = 8.5 Hz), 7.11 (1H, s), 7.46-7.57 (2H, m), 7.77 (1H, d, J = 8.1 Hz), 8.07 (1H, dt, J = 8.1, 2.0 Hz), 8.14 (1H, d, J = 2.4 Hz), 8.34-8.37 (1H, m). ESI-MSm/z: 297 (M+H)⁺.

[Referential Example 57] 5-[5-(tert-Butoxycarbonylamino)-2-pyrazinyl]-1-(3-pyridyl)-1H-pyrazole-3-carboxylic acid

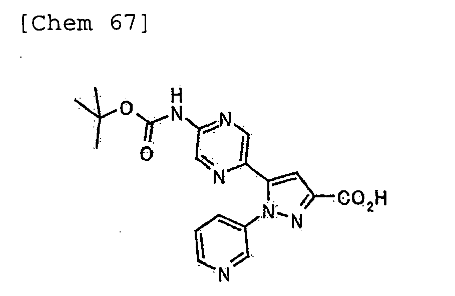

### 1) Ethyl 5-(5-carboxy-2-pyrazinyl)-1-(3-pyridyl)-1H-pyrazole-3-carboxylate

Selenium dioxide (3.66 g) was added to the ethyl 5-(5-methyl-2-pyrazinyl)-1-(3-pyridyl)-1H-pyrazole-3-carboxylate (2.55 g) of Referential Example 36(4) in pyridine (51 ml) at room temperature, and the mixture was heated under reflux for 67 hours. After cooling with air, water and chloroform were added to the reaction liquid and the phases were separated, and the organic layer was dried over anhydrous sodium sulfate. After filtration, the solvent was evaporated under reduced pressure, and the resulting solid was washed with diethylether to give ethyl 5-(5-carboxy-2-pyrazinyl)-1-(3-pyridyl)-1H-pyrazole-3-carboxylate (2.51 g, 90%).

¹H-NMR (400 MHz, DMSO-d₆)δ: 1.34-1.37 (3H, m), 4.36-4.41 (2H, m), 7.54 (1H, dd, J = 8.2, 4.8 Hz), 7.84 (1H, s), 7.92-7.94 (1H, m), 8.65-8.68 (2H, m), 8.90-8.91 (1H, m), 9.24-9.25 (1H, m).
FAB-MSm/z: 340 (M+H)⁺.

### 2) Ethyl 5-[5-(tert-butoxycarbonylamino)-2-pyrazinyl]-1-(3-pyridyl)-1H-pyr azole-3-carboxylate

Triethylamine (2.41 ml), diphenylphosphorylazide (3.73 ml), and tert-butanol (3.31 ml) were added to a suspension of the ethyl 5-(5-carboxy-2-pyrazinyl)-1-(3-pyridyl)-1H-pyrazole-3-carboxylate (5.34 g) in 1,4-dioxane (107 ml) at room temperature, and the mixture was stirred at 100°C for 20 minutes. After coolingwith air, the reaction solvent was evaporated under reduced pressure, and the residue was purified by column chromatography on silica gel (ethyl acetate-chloroform) to give ethyl 5-[5-(tert-butoxycarbonylamino)-2-pyrazinyl]-1-(3-pyridyl)-1H-pyr azole-3-carboxylate (4.63 g, 72%) as a solid.

¹H-NMR (400 MHz, CDCl₃)δ: 1.42-1.46 (3H, m), 1.53 (9H, s), 4.45-4.50 (2H, m), 7.29 (1H, d, J = 0.5 Hz), 7.37-7.44 (2H, m), 7.82-7.85 (1H, m), 8.35-8.36 (1H, m), 8.56 (1H, d, J = 2.4 Hz), 8.63 (1H, dd, J = 4.9, 1.2 Hz), 9.13 (1H, d, J = 1.2 Hz).
ESI-MSm/z: 411 (M+H)⁺.

### 3) The title compound

To a suspension of the ethyl 5-[5-(tert-butoxycarbonylamino)-2-pyrazinyl]-1-(3-pyridyl)-1H-pyr azole-3-carboxylate (0.336 g) in a mixture of methanol (6.7 ml) and tetrahydrofuran (6.7 ml) was added 1N aqueous sodium hydroxide (2.05 ml) at room temperature, and the mixture was stirred for 3 hours. The reaction liquid was neutralized by adding 1N aqueous hydrochloric acid (2.05ml), and water and methanol-chloroform (1:10) were added to the solution and the phases were separated. The solvent was evaporated under reduced pressure to give the title compound (0.286 g, 91%) as a solid.

¹H-NMR (400 MHz, DMSO-d₆)δ: 1.47 (9H, s), 7.45 (1H, s), 7.51 (1H, dd, J = 8.2, 4.8 Hz), 7.83-7.86 (1H, m), 8.57 (1H, d, J=2.4 Hz), 8.61-8.62 (1H, m), 8.68 (1H, m), 8.81 (1H, m), 10.38 (1H, s), 13.13 (1H, br s). EI-MSm/z: 382 (M⁺) .

[Referential Example 58] 5-(4-Methyl-2-pyridyl)-1-(3-pyridyl)-1H-pyrazole-3-carboxylic acid

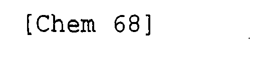

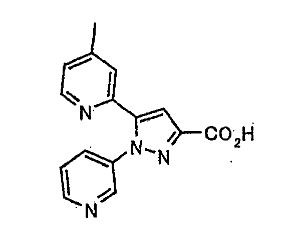

### 1) Ethyl 5-(4-methyl-2-pyridyl)-1-(3-pyridyl)-1H-pyrazole-3-carboxylate

The procedure of Referential Example 35 (3) was repeated by using the ethyl 4-(4-methyl-2-pyridyl)-2,4-dioxobutanoate (1.70 g) of Referential Example 54(3) and the 3-hydrazinopyridine (1.2 g) of Referential Example 32 to give ethyl 5-(4-methyl-2-pyridyl)-1-(3-pyridyl)-1H-pyrazole-3-carboxylate(1. 27 g, 57%).

¹H-NMR (400 MHz, CDCl₃)δ: 1.44 (3H, t, J = 7.08 Hz), 2.37 (3H, s), 4.46 (2H, q, J = 7.08 Hz), 7.07 (1H, s), 7.28 (2H, m), 7.37 (1H, m), 7.86 (1H, m), 8.32 (1H, s), 8.52 (1H, s), 8.60 (1H, m).
ESI-MSm/z: 309 (M+H)⁺.

### 2) The title compound

The procedure of Referential Example 53 (5) was repeated by using the ethyl 5-(4-methyl-2-pyridyl)-1-(3-pyridyl)-1H-pyrazole-3-carboxylate (1.27 g) and 1N aqueous sodium hydroxide (6.18 ml) to give the title compound (375 mg, 32%).

¹H-NMR (400 MHz, DMSO-d₆)δ: 2.34 (3H, s), 7.18 (1H, d, J = 5.01 Hz), 7.35 (1H, s), 7.49 (1H, dd, J = 8.18, 4.76 Hz), 7.66 (1H, s), 7.79 (1H, ddd, J = 8.18, 2.44, 1.34 Hz), 8.23 (1H, d, J = 5.01 Hz), 8.49 (1H, d, J = 2.44 Hz), 8.59 (1H, dd, J = 4.76, 1.34 Hz).
ESI-MSm/z: 281 (M+H)⁺.

[Referential Example 59] 5-[5-(tert-Butoxycarbonylamino)-2-pyridyl]-1-(2-pyrazinyl)-1H-pyrazole-3-carboxylic acid

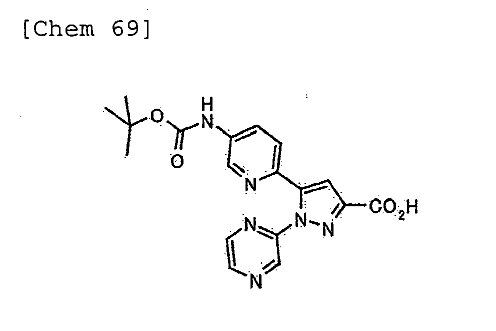

### 1) Ethyl 5-[5-(tert-butoxycarbonylamino)-2-pyridyl]-1-(2-pyrazinyl)-1H-pyr azole-3-carboxylate

A solution of the ethyl 4-[5-(tert-butoxycarbonylamino) -2-pyridyl] -2, 4-dioxobutanoate (1.009g) of Referential Example 41(3) and the 2-hydrazinopyrazine(330 mg) of Referential Example 33 in ethanol (30 ml) was heated under reflux for 88 hours. After cooling with air, the reaction solvent was evaporated under reduced pressure, and the residue was purified by column chromatography on silica gel (methanol-chloroform), and then by thin layer chromatography on silica gel (methanol-chloroform) to give ethyl 5-[5-(tert-butoxycarbonylamino)-2-pyridyl]-1-(2-pyrazinyl)-1H-pyr azole-3-carboxylate (590 mg, 47%) as an amorphous product.

¹H-NMR (400 MHz, CDCl₃)δ: 1.43 (3H, t, J = 7.1 Hz), 1.52 (9H, s), 4.47 (2H, q, J = 7.1 Hz), 6.55 (1H, br s), 7.21 (1H, s), 7.51 (1H, d, J = 8.5 Hz), 8.01-8.08 (1H, br m), 8.20 (1H, dd, J = 2.7, 0.5 Hz), 8.29 (1H, dd, J = 2.7, 1.5 Hz), 8.56 (1H, d, J = 2.7 Hz), 9.02 (1H, dd, J = 1.5, 0.5 Hz).
ESI-MSm/z: 411 (M+H)⁺.

### 2) The title compound

To a solution of the ethyl 5-[5-(tert-butoxycarbonylamino)-2-pyridyl]-1-(2-pyrazinyl)-1H-pyr azole-3-carboxylate (589 mg) in ethanol (10 ml) was added 1N aqueous sodium hydroxide (4.30 ml) at room temperature, and the mixture was stirred for 2 hours. The reaction solvent was evaporated under reduced pressure, and after adding water to the residue, the residue was acidified by adding 5% aqueous citric acid, and the solid precipitate was collected to give the title compound (441 mg, 80%).

¹H-NMR (400 MHz, DMSO-d₆)δ: 1.48 (9H, s), 7.31 (1H, s), 7.72 (1H, d, J = 8.8 Hz), 7.95 (1H, dd, J = 8.8, 2.4 Hz), 8.34 (1H, d, J = 2.4 Hz), 8.49 (1H, dd, J = 2.4, 1.5 Hz), 8.74 (1H, d, J = 2.4 Hz), 8.95 (1H, d, J = 1.5 Hz), 9.68 (1H, br s).
ESI-MSm/z: 383 (M+H)⁺.

[Referential Example 60 1-(6-Methoxy-3-pyridyl)-5-(1H-pyrazole-3-yl)-1H-pyrazole-3-carboxylic acid

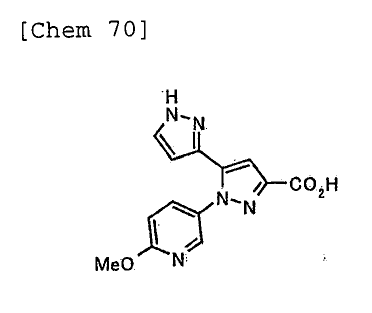

### 1) 1-{1-[(4-Methylphenyl)sulfonyl]-1H-pyrazol-3-yl}-1-ethanone

To a solution of 1-(1H-pyrazol-5-yl)-1-ethanone hydrochloride (2.93g) in pyridine (60 ml) was added 4-methylbenzenesulfonyl chloride (5.72 g), and the mixture was heated under reflux for 3.5 hours. After cooling with air, the reaction solvent was evaporated under reduced pressure, and ethyl acetate and water were added to the residue and the phases were separated. The organic layer was washed with 1N aqueous hydrochloric acid, and dried over anhydrous sodium sulfate. After filtration, the solvent was evaporated under reduced pressure, and the residue was solidified from methanol, diethylether, and hexane to givel-{1-[(4-methylphenyl)sulfonyl]-1H-pyrazol-3-yl}-1-ethanone (1.89 g, 35%). The filtrate solvent was evaporated under reduced pressure, and the residue was solidified from methanol, diethylether, and hexane to give 1-{1-[(4-methylphenyl)sulfonyl]-1H-pyrazol-3-yl}-1-ethanone (2.09g, 39%).

¹H-NMR (400 MHz, CDCl₃)δ: 2.45 (3H, s), 2.57 (3H, s), 6.83 (1H, d, J = 2.7 Hz), 7.36-7.38 (2H, m), 7,92-7.96 (2H, m), 8.10 (1H, d, J = 2.9 Hz).
ESI-MSm/z: 265 (M+H)⁺.

### 2) Ethyl 1-(6-methoxy-3-pyridyl)-5-{1-[(4-methylphenyl)sulfonyl]-1H-pyrazo 1-3-yl}-1H-pyrazole-3-carboxylate

Lithium bis(trimethylsilyl)amide (1.0M solution in tetrahydrofuran, 16.5 ml) was added to a suspension of the 1-{1-[(4-methylphenyl)sulfonyl]-1H-pyrazol-3-yl}-1-ethanone (3.97 g) in tetrahydrofuran (15 ml) over 35 minutes at -78°C, and diethyl oxalate (3.05 ml) was added to the reaction liquid. The mixture was stirred for 15 minutes, and stirring was continued for another 3.5 hours while resuming the room temperature. Diethylether and water were added to the reaction liquid and the aqueous layer was separated, and the aqueous layer was acidified by adding 1N aqueous hydrochloric acid and the solution was extracted with diethylether. The aqueous layer was further extracted with ethyl acetate, and the combined organic layers were dried over anhydrous sodium sulfate. After filtration, the solvent was evaporated under reduced pressure, and dichloromethane was added to the residue. The resulting solid was removed by filtration, and the solvent was evaporated under reduced pressure to give ethyl 4-{1-[(4-methylphenyl)sulfonyl]-1H-pyrazol-3-yl}-2,4-dioxobutanoa te (5.08 g, 92%) as an oily product. A solution of this ethyl butanoate derivative (5.08 g) and the 5-hydrazino-2-methoxypyridine (1.93 g) of Referential Example 2 in ethanol (70 ml) was heated under reflux for 14.5 hours. After cooling with air, ethyl acetate and saturated aqueous sodium bicarbonate were added to the reaction liquid and the phases were separated, and the organic layer was dried over anhydrous sodium sulfate. After filtration, the solvent was evaporated under reduced pressure, and the residue was purified by column chromatography on silica gel (ethyl acetate-hexane) to give ethyl 1-(6-methoxy-3-pyridyl)-5-{1-[(4-methylphenyl)sulfonyl]-1H-pyrazo 1-3-yl}-1H-pyrazole-3-carboxylate (2.58 g, 39%) as an amorphous product.

¹H-NMR (400 MHz, CDCl₃)δ: 1.41 (3H, t, J = 7.1 Hz), 2.45 (3H, s), 4.01 (3H, s), 4.44 (2H, q, J = 7.1 Hz), 6.24 (1H, d, J = 2.7 Hz), 6.73 (1H, d, J = 8.8 Hz), 7.23 (1H, s), 7.31 (2H, d, J = 8.5 Hz), 7.58 (1H, dd, J = 8.8, 2.7 Hz), 7.76 (2H, d, J = 8.3 Hz), 8.04 (1H, d, J = 2.7 Hz), 8.15 (1H, d, J = 2.7 Hz).
ESI-MSm/z: 468 (M+H)⁺.

### 3) The title compound

The procedure of Referential Example 48 (2) was repeated by using the ethyl 1-(6-methoxy-3-pyridyl)-5-{1-[(4-methylphenyl)sulfonyl]-1H-pyrazo 1-3-yl}-1H-pyrazole-3-carboxylate (2.58 g) to give the title compound (1.33 g, 84%) as a solid.

¹H-NMR (400 MHz, DMSO-d₆)δ: 3.92 (3H, s), 6.29 (1H, br s), 6.94 (1H, d, J = 8.8 Hz), 7.14 (1H, s), 7.75 (1H, d, J = 2.2 Hz), 7.80 (1H, dd, J = 8.8, 2.7 Hz), 8.24-8.25 (1H, m), 13.09 (1H, br s).
ESI-MSm/z: 286 (M+H)⁺.

[Referential Example 61] 5-(6-Methyl-3-pyridazinyl)-1-(3-pyridyl)-1H-pyrazole-3-carboxylic acid

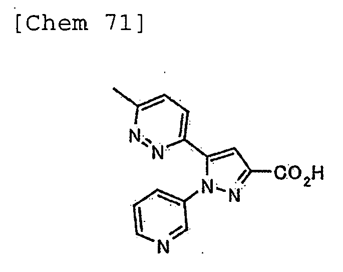

### 1) 3-Acetyl-6-methylpyridazine

Methyl magnesium iodide (2.0M solution in diethylether, 30 ml) was added dropwise to a solution of 6-methyl-3-pyridazinecarbonitrile (6.00 g) in a mixture of diethylether (100 ml) and benzene (20 ml) at -15°C, and the mixture was stirred for 1.5 hours. To the reaction liquid was added 1N aqueous hydrochloric acid (60 ml), and the mixture was stirred for 15 minutes and the aqueous layer was separated. The aqueous layer was made basic by adding saturated aqueous sodium bicarbonate, and the solution was extracted with dichloromethane, and the organic layer was dried over anhydrous magnesium sulfate. After filtration, the solvent was evaporated under reduced pressure, and the residue was purified by chromatography on silica gel (ethyl acetate-hexane) to give 3-acetyl-6-methylpyridazine (4.84 g, 71%) as a solid.

¹H-NMR (400 MHz, CDCl₃)δ: 2.82 (3H, s), 2.88 (3H, s), 7.47 (1H, d, J = 8.5 Hz), 8.03 (1H, d, J = 8.5 Hz).
LC-MSm/z: 137 (M+H)⁺.

### 2) Methyl 4-(6-methyl-3-pyridazinyl)-2,4-dioxobutanoate

Lithium bis(trimethylsilyl)amide (1.0M solution in tetrahydrofuran, 33 ml) was added to a solution of the 3-acetyl-6-methylpyridazine (4.03 g) in tetrahydrofuran (100 ml) at -78°C, and the mixture was stirred for 1 hour. A solution of dimethyl oxalate (7.0 g) in tetrahydrofuran (30 ml) was added to the reaction liquid at -78°C, and the mixture was stirred at 0°C for 2 hours. Water and diethylether were added to the reaction liquid and the aqueous layer was separated, and the aqueous layer was acidified (to pH 4) with 1N aqueous hydrochloric acid and the solution was extracted with chloroform. The aqueous layer was further extracted with chloroform, and the combined organic layers were washed with brine, and dried over anhydrous sodium sulfate. After filtration, the solvent was evaporated under reduced pressure to give methyl 4-(6-methyl-3-pyridazinyl)-2,4-dioxobutanoate(5.42g, 82%) as a solid.

¹H-NMR (400 MHz, CDCl₃)δ: 2.78 (3H, s), 3.89 (3H, s), 7.47 (1H, d, J = 8.5 Hz), 7.84 (1H, s), 8.07 (1H, d, J = 8.5 Hz).
ESI-MSm/z: 223 (M+H)⁺.

### 3) Methyl 5-(6-methyl-3-pyridazinyl)-1-(3-pyridyl)-1H-pyrazole-3-carboxylat e

The 3-hydrazinopyridine (3.00 g) of Referential Example 32 was added to a solution of the methyl 4-(6-methyl-3-pyridazinyl)-2,4-dioxobutanoate (5.42 g) in methanol (140 ml) at room temperature, and the mixture was heated under reflux for 30 minutes. After cooling with air, acetic acid (5.6 ml) was added and the mixture was heated under reflux for 14 hours. Conc. hydrochloric acid (1.2 ml) was then added and the mixture was heated under reflux for 24 hours. After cooling with air, pH of the reaction liquid was adjusted to 4 by adding 1N aqueous sodium hydroxide, and the reaction solvent was evaporated under reduced pressure. Chloroform and water were added to the residue and the phases were separated, and the aqueous layer was further extracted with chloroform. The combined organic layers were washed with brine, and dried over anhydrous sodium sulfate. After filtration, the solvent was evaporated under reduced pressure, and the residue was purified by column chromatography on silica gel (methanol-chloroform) to give methyl 5-(6-methyl-3-pyridazinyl)-1-(3-pyridyl)-1H-pyrazole-3-carboxylat e (2.98 g, 41%) as a solid.

¹H-NMR (400 MHz, CDCl₃)δ: 2.73 (3H, s), 4.00 (3H, s), 7.32-7.43 (3H, m), 7.46 (1H, d, J = 8.7 Hz), 7.86-7.92 (1H, m), 8.53 (1H, d like, J = 2.5 Hz), 8.62 (1H, dd, J = 4.9, 1.5 Hz).
FAB-MSm/z: 296 (M+H)⁺.

### 4) The title compound.

To a suspension of the methyl 5-(6-methyl-3-pyridazinyl)-1-(3-pyridyl)-1H-pyrazole-3-carboxylat e (2.98 g) in a mixture of tetrahydrofuran (20 ml) and methanol (100 ml) was added 1N aqueous sodium hydroxide (25 ml), and the mixture was stirred at 40°C for 6 hours. After cooling with air, the reaction liquid was acidified (to pH 4) with 1N aqueous hydrochloric acid, and the reaction solvent was evaporated under reduced pressure.
Diethylether was added to the residue, and the solid precipitate was collected by filtration to give the title compound (2.84 g, measured) as a solid.

¹H-NMR (400 MHz, DMSO-d₆)δ: 2.61 (3H, s), 7.50-7.57 (2H, m), 7.68 (1H, d, J = 8.6 Hz), 7.85-7.92 (1H, m), 7.96 (1H, d, J = 8.6 Hz), 8.56 (1H, d like, J = 2.4 Hz), 8.62 (1H, dd, J = 3.4, 1.5 Hz).
FAB-MSm/z: 282 (M+H)⁺.

[Referential Example 62] Ethyl 5-(1-methyl-1H-pyrrol-3-yl)-1-(3-pyridyl)-1H-pyrazole-3-carboxylate

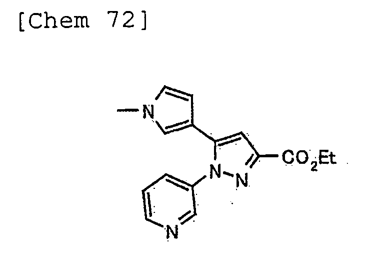

Lithium bis(trimethylsilyl)amide (1.0M solution in tetrahydrofuran, 11.1 ml) was added dropwise to a solution of 3-acetyl-1-methylpyrrole (1.20 ml) in tetrahydrofuran (10 ml) at-78°C. After stirring the reaction liquid for 30 minutes, diethyl oxalate (2.06 ml) was added dropwise, and the temperature was elevated to room temperature, and the mixture was stirred at room temperature for 1 hour. The 3-hydrazinopyridine (1.30 g) of Referential Example 32, acetic acid (635 µl), and ethanol (50 ml) were added to the reaction liquid, and the mixture was heated under reflux for 16 hours. After cooling with air, 3-hydrazinopyridine (650 mg) was also added to the reaction liquid, and the mixture was heated under reflux for 3 hours. Conc. hydrochloric acid (0.60 ml) was added to the reaction liquid and the mixture was heated under reflux for 24 hours. After cooling with air, the reaction solvent was evaporated under reduced pressure. Saturated aqueous sodium bicarbonate (100ml), water (50ml), and ethyl acetate (100 ml) were added to the residue, and the aqueous layer was saturated with sodium chloride and the phases were separated. The aqueous layer was further extracted with ethyl acetate, and the combined organic layers were dried over anhydrous sodium sulfate. After filtration, the solvent was evaporated under reduced pressure, and the residue was purified by column chromatography on silica gel (dichloromethane-ethyl acetate) to give the title compound (747 mg, 25%) as a solid.

¹H-NMR (400 MHz, CDCl₃)δ: 1.42 (3H, t, J = 7.2 Hz), 3.59 (3H, s), 4.44 (2H, q, J = 7.2 Hz), 5.88 (1H, dd, J = 2.7, 2.0 Hz), 6.43 (1H, t, J = 2.0 Hz), 6.52 (1H, t, J = 2.7 Hz), 6.92 (1H, s), 7.39 (1H, dd, J = 8.2, 4.8 Hz), 7.85 (1H, ddd, J = 8.2, 2.4, 1.7 Hz), 8.65 (1H, dd, J = 4.8, 1.7 Hz), 8.72 (1H, d, J = 2.4 Hz).
EI-MSm/z: 296 (M⁺).

[Referential Example 63] 3-Hydrazino-6-methylpyridazine

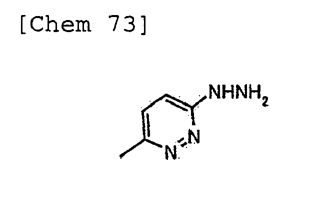

Hydrazine monohydrate (45 ml) was added to a suspension of 3-chloro-6-methylpyridine (3. 00 g) in ethanol (45 ml), and the mixture was heated under reflux for 2.5 hours. After cooling with air, the reaction solvent was evaporated under reduced pressure, and the residue' was purified by chromatography on silica gel (using chloroform-methanol-water (7:3:1) of the lower layer mixed solvent) to give the title compound (2.35 g, 81%) as a solid.

¹H-NMR (400 MHz, DMSO-d₆)δ: 2.39 (3H, s), 4.20 (2H, br), 6.94 (1H, d, J = 9.3 Hz), 7.18 (1H, d, J = 9.3 Hz), 7.64 (1H, br).
ESI-MSm/z: 125 (M+H)⁺.

[Referential Example 64] 1-(6-Methyl-3-pyridazinyl)-5-(5-methyl-2-pyridyl)-1H-pyrazole-3-carboxylic acid

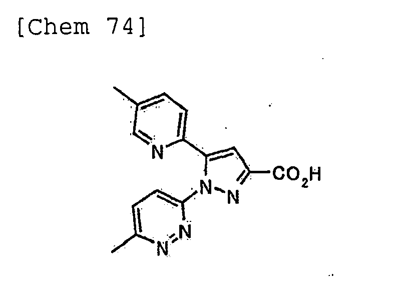

### 1) Ethyl 1-(6-methyl-3-pyridazinyl)-5-(5-methyl-2-pyridyl)-1H-pyrazole-3-c arboxylate

Acetic acid (4.31 ml) was added to a solution of the ethyl 4-(5-methyl-2-pyridyl)-2,4-dioxobutanoate (3.54 g) of Referential Example 35(2) and the 3-hydrazino-6-methylpyridazine (1.87 g) of Referential Example 63 in ethanol (71 ml) at room temperature, and the mixture was heated under reflux for 15 hours. To the reaction liquid was added conc. hydrochloric acid (4.7 ml), and the mixture was heated under reflux for 3 hours. After cooling with air, saturated aqueous sodium bicarbonate and chloroform were added to the reaction liquid and the phases were separated, and the organic layer was dried over anhydrous sodium sulfate. After filtration, the solvent was evaporated under reduced pressure, and the residue was purified by column chromatography on silica gel (chloroform-ethyl acetate) to give ethyl 1-(6-methyl-3-pyridazinyl)-5-(5-methyl-2-pyridyl)-1H-pyrazole-3-c arboxylate (1.13 g, 23%) as a solid.

¹H-NMR (400 MHz, CDCl₃)δ: 1.41-1.44 (3H, m), 2.32 (3H, s), 2.72 (3H, s), 4.43-4.48 (2H, m), 7.18 (1H, s), 7.46-7.56 (3H, m), 7.98 (1H, d, J = 8.8 Hz), 8.21 (1H, m).
EI-MSm/z: 323 (M⁺).

### 2) The title compound

The procedure of Referential Example 9, Method B (4) was repeated by using the ethyl 1-(6-methyl-3-pyridazinyl)-5-(5-methyl-2-pyridyl)-1H-pyrazole-3-c arboxylate (1.12 g) to give the title compound (0.759 g, 74%) as a solid.

¹H-NMR (400 MHz, DMSO-d₆)δ: 2.27 (3H, s), 2.67 (3H, s), 7.32 (1H, s), 7.64-7.69 (2H, m), 7.81 (1H, d, J = 8.8 Hz), 7.94 (1H, d, J = 8.8 Hz), 8.15-8.16 (1H, m), 13.16 (1H, s).
EI-MSm/z: 295 (M⁺).

[Referential Example 65] 5-(6-Methoxy-3-pyridazinyl)-1-(3-pyridyl)-1H-pyrazole-3-carboxylic acid

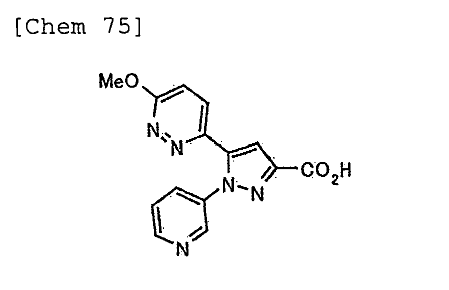

### 1) 3-Methoxypyridazine

In hydrogen atmosphere, 10% palladium on carbon (wet., 3.12 g) was added to a solution of 3-chloro-6-methoxypyridazine (30.0 g) in methanol (200 ml) at room temperature, the mixture was stirred for 17 hours. After filtration of the reaction liquid, the solvent in the filtrate was evaporated under reduced pressure, and the residue was purified by column chromatography on silica gel (ethyl acetate) to give 3-methoxypyridazine (16.3 g, 71%) as an oily product.

¹H-NMR (400 MHz, CDCl₃)δ: 4.14 (3H, s), 6.98 (1H, d, J = 9.0 Hz), 7.37 (1H, dd, J = 4.5, 4.4 Hz), 8.84 (1H, d, J = 4.4 Hz).

### 2) 3-Methoxypyridazine-1-oxide

To a solution of the 3-methoxypyridazine (16.2 g) in dichloromethane (300 ml) was added m-chlorobenzoic acid (44.0 g) at room temperature, and the mixture was stirred for 16 hours. A solution of sodium bicarbonate (9.27 g) in water (100ml) was added to the reaction liquid, and the mixture was stirred for 15 minutes. Saturated aqueous sodium bicarbonate and chloroform were then added to the mixture, and the phases were separated, and the organic layer was dried over anhydrous sodium sulfate. After filtration, the solvent was evaporated under reduced pressure to give 3-methoxypyridazine-1-oxide (15.8 g, 85%) as a solid.

¹H-NMR (400 MHz, CDCl₃)δ: 4.02 (3H, s), 6.66 (1H, d, J = 8.5 Hz), 7.48 (1H, dd, J = 8.5, 5.9 Hz), 7.92 (1H, d, J = 5.9 Hz).

### 3) 6-Cyano-3-methoxypyridazine

A mixture of 3-methoxypyridazine-1-oxide (9.89 g) and dimethyl sulfate (9.45 ml) was stirred at 80°C for 1 hour. After cooling with air, 1,4-dioxane (100ml) was added to the reaction liquid, and a solution of potassium cyanate (8.77 g) in water (30 ml) was added at 0°C, and the mixture was stirred at room temperature for 4.5 hours. The reaction liquid was poured into saturated aqueous sodium bicarbonate, and the mixture was extracted with chloroform, and the organic layer was washed with water, then dried over anhydrous sodium sulfate. After filtration, the solvent was evaporated under reduced pressure, and the residue' was purified by column chromatography on silica gel (hexane-ethyl acetate) to give 6-cyano-3-methoxypyridazine (8.74 g, 72%) as a solid.

¹H-NMR (400 MHz, CDCl₃)δ: 4.24 (3H, s), 7.09 (1H, d, J = 9.0 Hz), 7. 68 (1H, d, J = 9.0 Hz).

### 4) 3-Acetyl-6-methoxypyridazine

Methyl magnesium iodide (0.84M solution in diethylether, 60 ml) was gradually added dropwise to a solution of the 6-cyano-3-methoxypyridazine (5.61 g) in a mixture of diethylether (100 ml) and benzene (20 ml) at -10°C, and the mixture was stirred at the same temperature for 1 hour. The reaction liquid was acidified (to pH 4) by adding 1N aqueous hydrochloric acid at 0°C and the aqueous layer was separated. The aqueous layer was made weakly basic (pH 9) by adding saturated aqueous sodium bicarbonate, and the solution was extracted with dichloromethane. The aqueous layer was extracted with dichloromethane, and the combined organic layers were washed with brine, and dried over anhydrous sodium sulfate. After filtration, the solvent was evaporated under reduced pressure, and the residue was purified by column chromatography on silica gel (ethyl acetate-hexane) to give 3-acetyl-6-methoxypyridazine (3.82 g, 61%) as a solid.

¹H-NMR (400 MHz, CDCl₃)δ: 2.82 (3H, s), 4.23 (3H, s), 7.06 (1H, d, J = 9.3 Hz), 8.04 (1H, d, J = 9.3 Hz).
FAB-MSm/z: 153 (M+H)⁺.

### 5) Methyl 4-(6-methoxy-3-pyridazinyl)-2,4-dioxobutanoate

Lithium bis(trimethylsilyl)amide (1.0M solution in tetrahydrofuran, 27 ml) was added to a solution of the 3-acetyl-6-methoxypyridazine (3.82 g) in tetrahydrofuran (100 ml) at -78°C, and the mixture was stirred for 1 hour. A solution of dimethyl oxalate (5.9 g) in tetrahydrofuran (20 ml) was added to the reaction liquid at -78°C, and the mixture was stirred at 0°C for 2 hours. Water and diethylether were added to the reaction liquid and the aqueous layer was separated, and the aqueous layer was acidified (to pH 4) with 1N aqueous hydrochloric acid, and the solution was extracted with chloroform. The aqueous layer was further extracted with chloroform, and the combined organic layers were washed with brine, and dried over anhydrous sodium sulfate. After filtration, the solvent was evaporated under reduced pressure to give methyl 4-(6-methoxy-3-pyridazinyl)-2,4-dioxobutanoate (5.38 g, 90%) as a solid.

¹H-NMR (400 MHz, CDCl₃)δ: 3.93 (3H, s), 4.26 (3H, s), 7.12 (1H, d, J = 9.3 Hz), 7.86 (1H, s), 8.15 (1H, d, J = 9.3 Hz).
ESI-MSm/z: 239 (M+H)⁺.

### 6) Methyl 5-(6-methoxy-3-pyridazinyl)-1-(3-pyridyl)-1H-pyrazole-3-carboxyla te

The 3-hydrazinopyridine (2.82 g) of Referential Example 32 was added to a solution of the methyl 4-(6-methoxypyridazin-3-yl)-2,4-dioxobutanoate (5.38 g) in methanol (150 ml) at room temperature, and the mixture was heated under reflux' for 45 minutes. Acetic acid (5.2 ml) was added to the reaction liquid, and the mixture was heated under reflux for 14 hours. After cooling with air, the reaction liquid was neutralized with 1N aqueous sodium hydroxide, and the solvent was evaporated under reduced pressure. Chloroform and water were added to the residue and the phases were separated, and the aqueous layer was extracted with chloroform. The combined organic layers were brine, and dried over anhydrous sodium sulfate. After filtration, the solvent was evaporated under reduced pressure, and the residue was purified by column chromatography on silica gel (methanol-chloroform) to give methyl 5-(6-methoxy-3-pyridazinyl)-1-(3-pyridyl)-1H-pyrazole-3-carboxyla te (0.348 g, 5.0%) as a solid. The solvent of the second fraction eluted with the same solvent was evaporated under reduced pressure to give methyl 5-hydroxy-5-(6-methoxy-3-pyridazinyl)-1-(3-pyridyl)-4,5-dihydro-1 H-pyrazole-3-carboxylate (3.11 g, 44%) as an amorphous product. [Methyl 5-(6-methoxy-3-pyridazinyl)-1-(3-pyridyl)-1H-pyrazole-3-carboxyla te:

¹H-NMR (400 MHz, CDCl₃)δ: 3.99 (3H, s), 4.13 (3H, s), 7.00 (1H, d, J = 9.1 Hz), 7.31 (1H, s), 7.37-7.46 (2H, m), 7.86-7.92 (1H, m), 8.54 (1H, d like, J = 2.0 Hz), 8.63 (1H, dd, J = 4.9, 1.5 Hz).
FAB-MSm/z: 312 (M+H)⁺.
Methyl 5-hydroxy-5-(6-methoxy-3-pyridazinyl)-1-(3-pyridyl)-4,5-dihydro-1 H-pyrazole-3-carboxylate: ESI-MSm/z: 330 (M+H)⁺.]
Triethylamine (3.04 ml), methanesulfonyl chloride (1.35 ml), and 4-(dimethylamino)pyridine (0.111 g) were added to a solution of the resulting methyl 5-hydroxy-5-(6-methoxy-3-pyridazinyl)-1-(3-pyridyl)-4,5-dihydro-1 H-pyrazole-3-carboxylate (2.88 g) indichloromethane (80 ml) at room temperature, and the mixture was stirred for 2.5 hours. Methanol was added to the reaction liquid, and chloroform and water were added and the phases were separated. The aqueous layer was further extracted with chloroform, and the combined organic layers were washed with brine, and dried over anhydrous sodium sulf ate. After filtration, the solvent was evaporated under reduced pressure, and the residue was purified by column chromatography on silica gel (acetone-chloroform) to give methyl 5-(6-methoxy-3-pyridazinyl)-1-(3-pyridyl)-1H-pyrazole-3-carboxyla te (1.558 g, 57%) as a solid.

### 7) The title compound

Lithium hydroxide monohydrate (62 mg) was added to a solution of the methyl 5-(6-methoxy-3-pyridazinyl)-1-(3-pyridyl)-1H-pyrazole-3-carboxyla te (0.399 g) in a mixture of tetrahydrofuran (5ml), methanol (10 ml), and water (5 ml), and the mixture was stirred at 40°C for 1 hour. After cooling with air, the reaction liquid was neutralized with 1N aqueous hydrochloric acid, and the solvent was evaporatedunder reducedpressure. The residue was acidified (to pH 4) by adding 1N aqueous hydrochloric acid, and the solid precipitate was collected by filtration to give the title compound (0.303 g, 80%) as a solid.

¹H-NMR (400 MHz, DMSO-d₆)δ: 4.00 (3H, s), 7.34 (1H, d, J = 7.8 Hz), 7.49 (1H, s), 7.53 (1H, dd, J = 4.8, 7.8 Hz), 7.86-7.91 (1H, m), 7.99 (1H, d like, J = 9.3 Hz), 8.60 (1H, d, J = 2.0 Hz), 8.64 (1H, d like, J = 4.8 Hz).
FAB-MSm/z: 298 (M+H)⁺.

[Referential Example 66] 1-(6-Methoxy-3-pyridyl)-5-(1H-pyrrol-3-yl)-1H-pyrazole-3-carboxylic acid

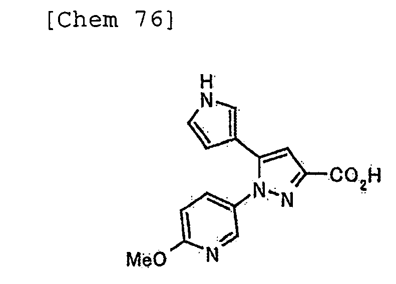

The procedure of Referential Example 36(3) was repeated by using 1-[1-(phenylsulfonyl)-1H-pyrrol-3-yl]-1-ethanone (10.7 g), diethyl oxalate (8.60 ml), and lithium bis(trimethylsilyl)amide (1.0M tetrahydrofuran, 46.3 ml) to give ethyl 4-[1-(phenylsulfonyl)-1H-pyrrol-3-yl]-2,4-dioxobutanoate (12.4 g) as a solid. To a solution of this ethyl butanoate derivative (4.00 g) in ethanol (50 ml) was added the 5-hydrazino-2-methoxypyridine (1.90 g) of Referential Example 2 and acetic acid (3.91ml) at room temperature, and the mixture was heated under reflux overnight. Conc. hydrochloric acid (1.00 ml) was added to the reaction liquid, and the mixture was heated under reflux for 6 days. After cooling with air, the reaction solvent was evaporated under reduced pressure, and saturated aqueous sodium bicarbonate, water, and ethyl acetate were added to the residue and the phases were separated. The organic layer was dried over anhydroussodiumsulfate. After filtration, the solvent was evaporated under reduced pressure, and ethanol (50 ml) and sodium hydroxide (816 mg) were added to the residue at room temperature, and the mixture was stirred for 3 hours. Water (20 ml) was also added to the reaction liquid, and the mixture was stirred overnight. The reaction solvent was evaporated under reduced pressure, and ethyl acetate and water were added to the residue and the phases were separated. The organic layer was dried over anhydrous sodium sulfate. After filtration, the solvent was evaporated under reduced pressure, and the residue was purifiedby column chromatography on silica gel (dichloromethane-ethyl acetate) to give ethyl 1-(6-methoxy-3-pyridinyl)-5-(1H-pyrrole-3-yl)-1H-pyrazole-3-carbo xylate (210 mg, 5%).

¹H-NMR (400 MHz, CDCl₃)δ: 1.41-1.44 (3H, t, J = 7.1 Hz), 3.98 (3H, s), 4.45 (2H, q, J=7.1Hz), 6.04-6.09 (1H, m), 6.57-6.60 (1H, m), 6.73-6.76 (1H, m), 6.80 (1H, d, J = 8.8 Hz), 6.95 (1H, d, J = 0.7 Hz), 7.66 (1H, ddd, J = 8.8, 2.7, 0.7 Hz), 8.25 (1H, d, J = 2.7 Hz), 8.34 (1H, br' s).
FAB-MSm/z: 313 (M+H)⁺.
The solid precipitated from the aqueous layer during the phase separation was also collected by filtration to give the title compound (1.60 g, 41%).

¹H-NMR (400 MHz, DMSO-d₆)δ: 3.89 (3H, d, J = 1.5 Hz), 5.82 (1H, d, J = 1.5 Hz), 6.53 (1H, d, J = 1.5 Hz), 6.61 (1H, s), 6.66-6.73 (1H, m), 6.88 (1H, dd, J = 8.8, 1.0 Hz), 7.70 (1H, ddd, J = 8.7, 1.5, 1.5 Hz), 8.16-8.20 (1H, m), 11.14 (1H, s).
FAB-MSm/z: 285 (M+H)⁺.

[Referential Example 67] 1-(6-Methoxy-3-pyridazinyl)-5-(5-methyl-2-pyridyl)-1H-pyrazole-3-carboxylic acid

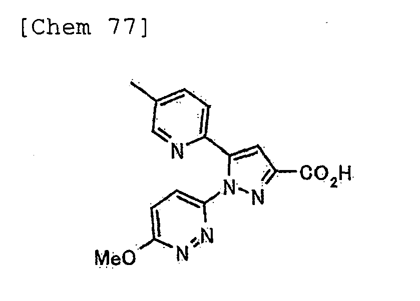

### 1) Methyl 4-(5-methyl-2-pyridyl)-2,4-dioxobutanoate

Dimethyl oxalate (10.4 g) was added to a solution of sodium' methoxide (4.74 g) in methanol (200 ml) at room temperature, and the mixture was stirred for 5 minutes. The 1-(5-methyl-2-pyridyl)-1-ethanone (5.93 g) of Referential Example 35(1) was added to the reaction liquid at room temperature, and the mixture was stirred for 5 hours. Water and diethylether were added to the reaction liquid and the phases were separated, and the aqueous layer was acidified with 1N aqueous hydrochloric acid, and the solution was extracted with chloroform. The organic layer was dried over anhydrous magnesium sulfate. After filtration, the solvent was evaporated under reduced pressure to give methyl 4-(5-methyl-2-pyridyl)-2,4-dioxobutanoate (7.31 g, 75%) as a solid.

¹H-NMR (400 MHz, CDCl₃)δ: 2.46 (3H, s), 3.92 (3H, s), 7.58 (1H, br), 7.70 (1H, dd, J = 8.06, 1.83 Hz), 8.08 (1H, d, J = 8.06 Hz), 8.54 (1H, d, J = 1.22 Hz).

### 2) Methyl 1-(6-chloro-3-pyridazinyl)-5-(5-methyl-2-pyridyl)-1H-pyrazole-3-c arboxylate

To a solution of the methyl 4-(5-methyl-2-pyridyl)-2,4-dioxobutanoate (3.34 g) in methanol (200 ml) was added 3-chloro-6-hydrazinopyridazine (2. 6 g), and the mixture was heated under reflux for 1.5 hours. Conc. hydrochloric acid (3 ml) was added to the reaction liquid, and the mixture was heated under reflux for another 4 hours. After cooling with air, saturated aqueous sodium bicarbonate and ethyl acetate were added to the reaction liquid and the phases were separated, and the organic layer was dried over anhydrous magnesium sulfate. After filtration, the solvent was evaporated under reduced pressure, and the residue was purified by column chromatography on silica gel (hexane-ethyl acetate) to give methyl 1-(6-chloro-3-pyridazinyl)-5-(5-methyl-2-pyridyl)-1H-pyrazole-3-c arboxylate (4.08 g, 82%) as a solid.

¹H-NMR (400 MHz, CDCl₃)δ: 2.33 (3H, s), 4.00 (3H, s), 7.20 (1H, s), 7.58 (2H, m), 7.68 (1H, d, J = 8.91 Hz), 8.08 (1H, d, J = 8.91 Hz), 8.22 (1H, s).
ESI-MSm/z: 330 (M+H)⁺.

### 3) The title compound

The methyl 1-(6-chloro-3-pyridazinyl)-5-(5-methyl-2-pyridyl)-1H-pyrazole-3-c arboxylate (4.08 g) was added to a solution of sodium methoxide (1.3 g) in methanol (100ml) at room temperature, and the mixture was stirred for 69 hours. Diethylether and water were added to the reaction liquid and the aqueous layer was separated, and 1N aqueous hydrochloric acid was added to the solution and the aqueous layer was extracted with chloroform. The organic layer was dried over anhydrous magnesium sulfate. After filtration, the solvent was evaporated under reduced pressure to give the title compound (3.0 g, 78%) as a solid.

¹H-NMR (400 MHz, CDCl₃)δ: 2.35 (3H, s), 4.10 (3H, s), 7.15 (1H, d, J = 9.28 Hz), 7.23 (1H, s), 7.51 (1H, d, J = 7.93 Hz), 7.60 (1H, d, J = 7.93 Hz), 7.96 (1H, d, J = 9.28 Hz), 8.32 (1H, s).

[Referential Example 68] 1-(6-Methoxy-3-pyridyl)-5-(1-methyl-1H-pyrrol-3-yl)-1H-pyrazole-3 -carboxylic acid

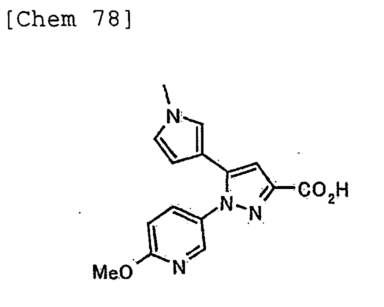

### 1) Ethyl 1-(6-methoxy-3-pyridyl)-5-(1-methyl-1H-pyrrol-3-yl)-1H-pyrazole-3 -carboxylate

Lithium bis(trimethylsilyl)amide (1.0M solution in tetrahydrofuran, 11.1 ml) was added dropwise to a solution of the 3-acetyl-1-methylpyrrole (1.2 ml) in tetrahydrofuran (10 ml) at-78°C, and the mixture was stirred for 30 minutes. Diethyl oxalate (2.06 ml) was added dropwise to the reaction liquid at room temperature for 1 hour, and to the reaction liquid were added the 5-hydrazino-2-methoxypyridine (2.50 g) of Referential Example 2 together with acetic acid (0.6 ml) and ethanol (50 ml), and the mixture was heated under reflux for 18 hours. After cooling with air, saturated aqueous sodium bicarbonate, water and ethyl acetate were added to the reaction liquid and the phases were separated, and the organic layer was dried over anhydrous magnesium sulfate. After filtration, the solvent was evaporated under reduced pressure, and the residue was purified by column chromatography on silica gel (hexane-ethyl acetate) to give ethyl 1-(6-methoxy-3-pyridyl)-5-(1-methyl-1H-pyrrol-3-yl)-1H-pyrazole-3 -carboxylate (2.45 g, 73%) as an oily product.

¹H-NMR (400 MHz, CDCl₃)δ: 1.41 (3H, t, J = 7.20 Hz), 3.59 (3H, s), 3.98 (3H, s), 4.43 (2H, q, J = 7.20 Hz), 5.91 (1H, dd, J= 2.81, 1.83 Hz), 6.41 (1H, t, J = 1.95 Hz), 6.51 (1H, t, J = 2.32 Hz), 6.79 (1H, d, J = 8.79 Hz), 6.90 (1H, s), 7.65 (1H, dd, J = 8.79, 2.69 Hz), 8.25 (1H, d, J = 2.32 Hz).

### 2) The title compound

To a suspension of the ethyl 1-(6-methoxy-3-pyridyl)-5-(1-methyl-1H-pyrrol-3-yl)-1H-pyrazole-3 -carboxylate (2.45 g) in tetrahydrofuran (30 ml) was added 1N aqueous sodium hydroxide (9 ml) at room temperature, and the mixture was stirred for 16 hours. To the reaction liquid was added 1N aqueous sodium hydroxide (4 ml), and the mixture was stirred for 3.5 hours. Water and diethylether were added to the reaction liquid and the phases were separated, and the aqueous layer was acidified with hydrochloric acid, and the solution was extracted with chloroform. The organic layer was dried over anhydrous magnesium sulfate. After filtration, the solvent was evaporated under reduced pressure to give the title compound (1.53 g, 68%) as a solid.

¹H-NMR (400 MHz, DMSO-d₆)δ: 3.54 (3H, s), 3.91 (3H, s), 5.78 (1H, dd, J = 2.69, 1.83 Hz), 6.68 (2H, m), 6.84 (1H, s), 6.95 (1H, d, J = 8.79 Hz), 7.77 (1H, dd, J = 8.89, 2.69 Hz), 8.23 (1H, d, J = 2.69 Hz), 12.81 (1H, br).
EI-MSm/z: 299 (M⁺).

[Referential Example 69] 1-(6-Methoxy-3-pyridyl)-5-(5-methyl-2-pyrazinyl)-1H-pyrazole-3-carboxylic acid

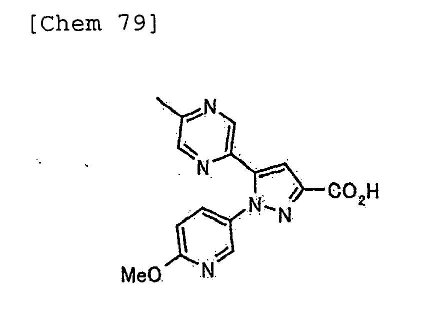

### 1) Ethyl 1-(6-methoxy-3-pyridyl)-5-(5-methyl-2-pyrazinyl)-1H-pyrazole-3-ca rboxylate

The procedure of Referential Example 49 (4) was repeated by using' the ethyl 4-(5-methyl-2-pyrazinyl)-2,4-dioxobutanoate (7.32 g) of Referential Example 36 (3) and the 5-hydrazino-2-methoxypyridine (4.31 g) of Referential Example 2 to give ethyl 1-(6-methoxy-3-pyridyl)-5-(5-methyl-2-pyrazinyl)-1H-pyrazole-3-ca rboxylate (4.80 g, 46%) as a solid.

¹H-NMR (400 MHz, CDCl₃)δ: 1.41-1.45 (3H, m), 2.57 (3H, s), 3.96 (3H, s), 4.44-4.49 (2H, m), 6.79 (1H, dd, J = 8.8, 0.7 Hz), 7.33 (1H, s), 7.68 (1H, dd, J = 8.8, 2.7 Hz), 8.10-8.11 (1H, m), 8.36 (1H, m), 8.54 (1H, d, J = 1.5 Hz).
FAB-MSm/z: 340 (M+H)⁺.

### 2) The title compound

The procedure of Referential Example 9, Method B (4) was repeated by using the ethyl 1-(6-methoxy-3-pyridyl)-5-(5-methyl-2-pyrazinyl)-1H-pyrazole-3-ca rboxylate (1.79 g) to give the title compound (1.43 g, 87%) as a solid.

¹H-NMR (400 MHz, DMSO-d₆)δ: 2.50 (3H, s), 3.90 (3H, s), 6.90 (1H, d, J = 8.8 Hz), 7.47 (1H, s), 7.76 (1H, dd, J = 8.8, 2.7 Hz), 8.19 (1H, d, J = 2.7 Hz), 8.41 (1H, m), 8.85 (1H, d, J = 1.5 Hz), 13.12 (1H, br s).
FAB-MSm/z: 312 (M+H)⁺.

[Referential Example 70] 2-Amino-1-fluoro-2-(fluoromethyl)propane hydrochloride

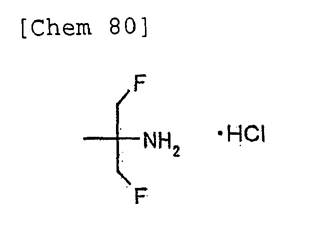

### 1) 3,3'-Dichloropivaloyl chloride

A solution of the 3, 3' -dichloropivaloic acid (49.95 g) in thionyl chloride (70 ml) was heated under reflux for 4 hours. After cooling with air, the reaction solvent was evaporated under reduced pressure, and the residue was purified by distillation under reduced pressure [bp 82-90°C, 15 to 16 mmHg] to give 3, 3-dichloropivaloyl chloride (37.36 g, 68%).

¹H-NMR (400 MHz, CDCl₃)δ: 1.51 (3H, s), 3.80 (2H, d, J = 12.0 Hz), 3.92 (2H, d, J = 11.7 Hz).

### 2) 3,3'-Difluoropivaloyl fluoride

A solution of the 3,3'-dichloropivaloyl chloride (15.0 g) and potassium fluoride (spray dried, 20.0g) in sulforane (50 ml) was stirred at 200°C for 5.5 hours. After cooling with air to 90°C, toluene (30 ml) was added, and the mixture was purified by distillation at atomospheric pressure (bp 105-113°C) to give 3,3'-difluoropivaloyl fluoride (content in toluene, 19%; 19.34 g).

¹H-NMR (400 MHz, CDCl₃)δ: 1.35 (3H, d, J = 1.0 Hz), 4.50 (1H, d, J = 9.2 Hz), 4.58-4.64 (2H, m), 4.70 (1H, d, J = 9.5 Hz).

### 3) The title compound

A solution of the 3,3'-difluoropivaloyl fluoride (toluene solution, 19.3 g) and trimethylsilylazide (2.99 ml) in toluene (30 ml) was stirred overnight at 80°C under argon atmosphere. After cooling with air, conc. hydrochloric acid (5 ml) was added to the reaction liquid, and the mixture was stirred at 60°C for 1 hour. After cooling with air, the reaction solvent was evaporated under reduced pressure, and the residue was solidified with methanol-diethylether to give the title compound (354 mg, 16%).

¹H-NMR (400 MHz, CD₃OD)δ: 1.36 (3H, t, J = 1.8 Hz), 4.51-4.68 (4H, m).

[Referential Example 71] 2-Amino-1-fluoro-2-methylpropane hydrochloride

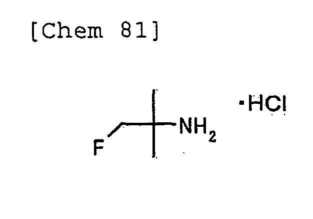

### 1) N-Benzyl-2-amino-2-methyl-1-propanol

A solution of 2-amino-2-methyl-1-propanol (10.0 g), benzaldehyde (11.98 ml), and p-toluenesulfonic acid (10 mg) in benzene (300 ml) was heated under reflux for 4 hours using Dean-Stark apparatus. After cooling with air, the reaction solvent was evaporated under reduced pressure, and the residue was dissolved in methanol (200 ml). Cyano sodium borohydride (8.89 g) was added to the reaction liquid under ice cooling, and the mixture was stirred for 1.5 hours. The reaction solvent was evaporated under reduced pressure, and saturated aqueous sodium bicarbonate and ethyl acetate were added to the residue and the phases were separated. The organic layer was washed with brine, and dried over anhydrous magnesium sulfate. After filtration, the solvent was evaporated under reduced pressure, and the residue was purified by column chromatography on silica gel (dichloromethane-methanol-aqueous ammonia) to give N-benzyl-2-amino-2-methyl-1-propanol (10.36 g, 52%) as a solid.

¹H-NMR (400 MHz, CDCl₃)δ: 1.15 (6H, s), 1.86 (2H, br s), 3.35 (2H, s), 3.68 (2H, s), 7.30 (5H, s).

### 2) 3-Benzyl-4,4-dimethyl-1,2,3-oxathiazole-2-oxide

A solution of thionyl chloride (1.49 ml) in dichloromethane (5 ml) was added dropwise to a solution of the N-benzyl-2-amino-2-methyl-1-propanol (3.32 g) and diisopropylethylamine (12.6 ml) in dichloromethane (50 ml) over 7 minutes at -20°C, and the mixture was stirred for 45 minutes. The reaction solvent was evaporated under reduced pressure, and the residue was purified by column chromatography on silica gel (hexane-ethyl acetate) to give 3-benzyl-4,4-dimethyl-1,2,3-oxathiazole-2-oxide (3.91 g, 94%) as a solid.

¹H-NMR (400 MHz, CDCl₃)δ: 1.24 (3H, s), 1.45 (3H, s), 4.15 (1H, d, J = 14.6 Hz), 4.20 (1H, d, J = 8.1 Hz), 4.27 (1H, d, J = 14.6 Hz), 4.64 (1H, d, J = 8.3 Hz), 7.26-7.42 (5H, m).

### 3) 3-Benzyl-4,4-dimethyl-1,2,3-oxathiazole-2,2-dioxide

Sodium periodate (2.73 g) was added to a solution of the 3-benzyl-4,4-dimethyl-1,2,3-oxathiazole-2-oxide (1.92 g) and ruthenium chloride hydrate (5 mg) in a mixture of acetonitrile (30 ml) and water (30 ml) at room temperature, and the mixture was stirred for 3 days. Water and diethylether were added to the reaction liquid and the phases were separated, and the organic layer was washed with brine, and dried over anhydrous sodium sulfate. After filtration, the solvent was evaporated under reduced pressure, and the residue was purified by column chromatography on silica gel (hexane-ethyl acetate) to give 3-benzyl-4,4-dimethyl-1,2,3-oxathiazole-2,2-dioxide (1.934 g, 94%) as an oily product.

¹H-NMR (400MHz, CDCl₃)δ: 1.30 (6H, s), 4.26 (4H, d, J= 1.2 Hz), 7.26-7.44 (5H, m).

### 4) N-Benzyl-2-amino-1-fluoro-2-methylpropane

Tetrabutylammonium fluoride (1.0M solution in tetrahydrofuran, 15.8 ml) was added to a solution of the 3-benzyl-4,4-dimethyl-1,2,3-oxathiazole-2,2-dioxide (1.91 g) in tetrahydrofuran (10ml) at room temperature, and the mixture was stirred for 3 hours. The reaction liquid was evaporated under reducedpressure, and the residue was dissolved in diethylether (30 ml), and to this reaction liquid was added 20% aqueous sulfuric acid (10 ml) at room temperature,and the mixture was stirred over night. Sodiumbicarbonate was added in several potions to the reaction liquid for neutralization, and the mixture was extracted with diethylether. The organic layer was washed with brine, and dried over anhydrous sodium sulfate. After filtration, the solvent was evaporated under reduced pressure, and the residue was purified by column chromatography on silica gel (hexane-ethyl acetate) to give N-benzyl-2-amino-1-fluoro-2-methylpropane (700 mg, 49%) as an oily product.

¹H-NMR (400 MHz, CDCl₃)δ: 1.16 (6H, d, J = 2.0 Hz), 1.39 (1H, s), 3.73 (2H, s), 4.26 (2H, d, J = 47.9 Hz), 7.21-7.37 (5H, m).

### 5) The title compound

To a solution of the N-benzyl-2-amino-1-fluoro-2-methylpropane (690 mg) in methanol (20 ml) were added 10% palladium on carbon (50 mg) and conc. hydrochloric acid (1 ml), and the mixture was stirred overnight at room temperature under hydrogen atmosphere (3.5 atm). Another portion of 10% palladium on carbon (100 mg) was added to the reaction liquid, and the mixture was stirred at 50°C for 6.5 hours under hydrogen atmosphere (4.0 atm). After cooling with air, the reaction liquid was filtered through celite, and the solvent was evaporated under reduced pressure to give the title compound (506 mg, measured) as a solid.

¹H-NMR (400 MHz, CD₃OD)δ: 1.37 (6H, s), 4.42 (2H, d, J = 37.2 Hz).

[Referential Example 72] 5-(1-Methyl-1H-imidazol-4-yl)-1-(6-methyl-3-pyridyl)-1H-pyrazole-3-carboxylic acid

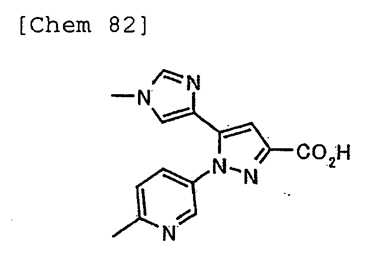

### 1) Ethyl 4-(1-methyl-1H-imidazol-4-yl)-2,4-dioxobutanoate

The procedure of Referential Example 36(3) was repeated by using 4-acetyl-1-methyl-1H-imidazole (0.75 g) and diethyl oxalate (1.64 ml, 12.08 mmol) to give ethyl 4-(1-methyl-1H-imidazol-4-yl)-2,4-dioxobutanoate (1.122 g, 83%) as a solid.

¹H-NMR (400 MHz, CDCl₃)δ: 1.39 (3H, t, J = 7.2 Hz), 3.79 (3H, s), 4.37 (3H, q, J = 7.1 Hz), 7.15 (1H, s), 7.52 (1H, s), 7.70 (1H, s).
ESI-MSm/z: 225 (M+H)⁺.

### 2) Ethyl 5-(1-methyl-1H-imidazol-4-yl)-1-(6-methyl-3-pyridyl)-1H-pyrazole-3-carboxylate

A solution of the ethyl 4-(1-methyl-1H-imidazole-4-yl)-2,4-dioxobutanoate (1.12 g) and the 5-hydrazino-2-methylpyridine (676 mg) of Referential Example 31 in 1N hydrochloric acid-ethanol (40 ml) was heated under reflux for 45 minutes. After cooling with air, chloroform and saturated aqueous sodium bicarbonate were added to the mixture, and the phases were separated, and the organic layer was dried over anhydrous sodium sulfate. After filtration, the solvent was evaporated under reduced pressure, and the residue was purified by thin layer chromatography on silica gel (methanol-chloroform) to give ethyl 5-(1-methyl-1H-imidazol-4-yl)-1-(6-methyl-3-pyridyl)-1H-pyrazole-3-carboxylate (705 mg, 45%) as a solid.

¹H-NMR (400 MHz, CDCl₃)δ: 1.41 (3H, t, J = 7.2 Hz), 2.63 (3H, s), 3.64 (3H, s), 4.44 (2H, q, J = 7.1 Hz), 6.64 (1H, s), 7.15 (1H, s), 7.27 (1H, s), 7.40 (1H, s), 7.78 (1H, dd, J = 8.3, 2.7 Hz), 8.54 (1H, d, J = 2.7 Hz).

### 3) The title compound

The procedure of Referential Example 42 (5) was repeated by using ethyl 5-(1-methyl-1H-imidazol-4-yl)-1-(6-methyl-3-pyridyl)-1H-pyrazole-3-carboxylate (694 mg) and lithium hydroxide monohydrate (112 mg) to give the title compound (444 mg, 70%) as a solid.

¹H-NMR (400 MHz, DMSO-d₆)δ: 2.54 (3H, s), 3.61 (3H, s), 6.96 (1H, s), 7.17 (1H, s), 7.37 (1H, d, J = 8.3 Hz), 7.59 (1H, s), 7.77 (1H, dd, J = 8.3, 2.7 Hz), 8.47 (1H, d, J = 2.4 Hz).
ESI-MSm/z: 284 (M+H)⁺.

[Referential Example 73] 5-(5-Cyano-2-pyridyl)-1-(3-pyridyl)-1H-pyrazole-3-carboxylic acid

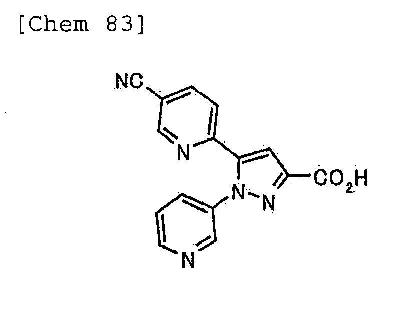

### Method A

### 1) Ethyl 5-(5-hydroxy-2-pyridyl)-1-(3-pyridyl)-1H-pyrazole-3-carboxylate

To a solution of the ethyl 5-(5-benzyloxy-2-pyridyl)-1-(3-pyridyl)-1H-pyrazole-3-carboxylate (4.63 g) of Referential Example 18(2) in ethanol (46 ml) and ethyl acetate (46 ml) was added 10% palladium on carbon (4.63 g), and the mixture was stirred at room temperature for 6 hours in the presence of hydrogen. The catalyst was removed from the reaction liquid by filtration, and the solvent was evaporated under reduced pressure to give ethyl 5-(5-hydroxy-2-pyridyl)-1-(3-pyridyl)-1H-pyrazole-3-carboxylate (3.48 g, 97%) as a solid.

¹H-NMR (400 MHz, DMSO-d₆)δ: 1.32 (3H, t, J = 7.1 Hz), 4.33 (2H, q, J = 7.1 Hz), 7.20-7.25 (2H, m), 7.49 (1H, dd, J = 8.2, 4.8 Hz), 7.55 (1H, d, J = 8.5 Hz), 7.76-7.79 (1H, m), 7.93 (1H, d, J = 2.9 Hz), 8.49 (1H, d, J = 2.7 Hz), 8.58-8.60 (1H, m), 10.31 (1H, br s).
FAB-MSm/z: 311 (M+H)⁺.

### 2) Ethyl 1-(3-pyridyl)-5-(5-trifluoromethanesulfonyloxy-2-pyridyl)-1H-pyrazole-3-carboxylate

Trifluoromethanesulfonic anhydride (2.26 ml) was added dropwise to a solution of ethyl 5-(5-hydroxy-2-pyridyl)-1-(3-pyridyl)-1H-pyrazole-3-carboxylate (3.47 g) in a mixture of dichloromethane (69 ml) and pyridine (23 ml) at room temperature under argon atmosphere, and the mixture was stirred for 85 minutes. Water and chloroform were added to the reaction liquid and the phases were separated, and the organic layer was dried over anhydrous sodium sulfate. After filtration, the solvent was evaporated under reduced pressure, and the residue was purified by column chromatography on silica gel (ethyl acetate-chloroform) to give ethyl 1-(3-pyridyl)-5-(5-trifluoromethanesulfonyloxy-2-pyridyl)-1H-pyra zole-3-carboxylate (4.95 g, quantitative) as a solid.

¹H-NMR (400 MHz, CDCl₃)δ: 1.42-1.45 (3H, m), 4.45-4.50 (2H, m), 7.35 (1H, s), 7.38-7.42 (1H, m), 7.59-7.61 (1H, m), 7.67-7.70 (1H, m), 7.79-7.82 (1H, m), 8.41 (1H, d, J = 2.7 Hz), 8.56 (1H, d, J = 2.4 Hz), 8.65 (1H, dd, J = 4.6, 1.5 Hz).
FAB-MSm/z: 443 (M+H)⁺.

### 3) The title compound

Under argon atmosphere, a suspension of tri-n-butyl tin cyanide (14.1 g) and tetrakis(triphenylphosphine)palladium(0) (19.4 g) in 1,2-dichloroethane (133 ml) was heated under reflux for 2 hours, and a solution of ethyl 1-(3-pyridyl)-5-(5-trifluoromethanesulfonyloxy-2-pyridyl)-1H-pyra zole-3-carboxylate (4.94 g) in 1,2-dichloroethane (109 ml) was added dropwise to the reaction liquid, and the mixture was heated under reflux for 13 hours. After cooling with air, saturated aqueous sodium bicarbonate was added to the reaction liquid, and the mixture was filtered through celite. Water and chloroform were added to the filtrate and the phases were separated, and the organic layer was dried over anhydrous sodium sulfate. After filtration, the solvent was evaporated under reduced pressure, and the residue was purified by column chromatography on silica gel (ethyl acetate-chloroform) to give ethyl 5-(5-cyano-2-pyridyl)-1-(3-pyridyl)-1H-pyrazole-3-carboxylate. A solution of lithium hydroxide monohydrate (0.470 g) in water (43 ml) was added dropwise to a suspension of the resulting ethyl ester derivative in tetrahydrofuran (87 ml) at room temperature, and the mixture was stirred for 40 minutes. Water and chloroform were added to the reaction liquid and the aqueous layer was separated, and the aqueous layer was neutralized by adding 1N aqueous hydrochloric acid (11.2 ml), and a mixed solvent of methanol and chloroform (1:10) was added to the solution, and the phases were separated. The solvent was evaporated under reduced pressure to give the title compound (2.52 g, 77%) as a solid.

¹H-NMR (400 MHz, DMSO-d₆)δ: 7.50-7.54 (1H, m), 7.62 (1H, s), 7.85-7.87 (1H, m), 8.04 (1H, d, J = 8.3 Hz), 8.41-8.44 (1H, m), 8.58 (1H, d, J = 2.4 Hz), 8.65 (1H, d, J = 4.6 Hz), 8.80-8.81 (1H, m), 13.23 (1H, s).
FAB-MSm/z: 292 (M+H)⁺.

### Method B

### 1) 2-Acetyl-5-cyanopyridine

Ammonium peroxodisulfate (10.3 g) was gradually added to a solution of 3-cyanopyridine (3.12 g), pyruvic acid (6.23 ml), and silver nitrate (1.27 g) in a mixture of dichloromethane (150 ml) and water (150 ml) at room temperature. Sulfuric acid (3.2 ml) was gradually added to the reaction liquid under ice cooling, and the mixture was stirred at 40°C for 1.5 hours. To the reaction liquid was added 1N aqueous sodium hydroxide to make the solution basic under ice cooling, and the solution was extracted with dichloromethane. The organic layer was dried over anhydrous sodium sulfate. After filtration, the solvent was evaporated under reduced pressure, and the residue was purified by column chromatography on silica gel (ethyl acetate-hexane) to give 2-acetyl-5-cyanopyridine (903 mg, 21%) as a solid.

¹H-NMR (400 MHz, CDCl₃)δ: 2.75 (3H, s), 8.13-8.16 (2H, m), 8.95 (1H, d, J = 1.2 Hz).

### 2) Ethyl 4-(5-cyano-2-pyridyl)-2,4-dioxobutanoate

The procedure of Referential Example 36 (3) was repeated by using 2-acetyl-5-cyanopyridine (900 mg) and diethyl oxalate (1.67 ml) to give ethyl 4-(5-cyano-2-pyridyl)-2,4-dioxobutanoate (1.188 g, 78%) as a solid.

¹H-NMR (400 MHz, CDCl₃)δ: 1.43 (3H, t, J = 7.1 Hz), 4.42 (2H, q, J = 7.1 Hz), 7.69 (1H, s), 8.18 (1H, dd, J = 8.2, 2.1 Hz), 8.26 (1H, d, J = 8.1 Hz), 8.98 (1H, s).

### 3) Ethyl 5-(5-cyano-2-pyridyl)-1-(3-pyridyl)-1H-pyrazole-3-carboxylate

The procedure of Referential Example 72 (2) was repeated by using the ethyl 4-(5-cyano-2-pyridyl)-2,4-dioxobutanoate (1.72 g) and the 3-hydrazinopyridine (0.92 g) of Referential Example 32 to give ethyl 5-(5-cyano-2-pyridyl)-1-(3-pyridyl)-1H-pyrazole-3-carboxylate (1.01 g, 45%) as a solid.

¹H-NMR (400 MHz, CDCl₃)δ: 1.44 (3H, t, J = 7.1 Hz), 4.48 (2H, q, J = 7.2 Hz), 7.41-7.43 (1H, m), 7.42 (1H, s), 7.61 (1H, d, J = 8.1 Hz), 7.82 (1H, d, J = 8.1 Hz), 8.00 (1H, dd, J = 8.2, 2.1 Hz), 8.54 (1H, d, J = 2.7 Hz), 8.67-8.68 (2H, m).

### 4) The title compound

The procedure of Referential Example 72 (3) was repeated by using ethyl 5-(5-cyano-2-pyridyl)-1-(3-pyridyl)-1H-pyrazole-3-carboxylate (5.69 g) and lithium hydroxide monohydrate (823 mg) to give the title compound (5.19 g, measured) as a solid.

¹H-NMR (400 MHz, DMSO-d₆)δ: 7.53 (1H, dd, J = 8.1, 4.9 Hz), 7.63 (1H, s), 7.87 (1H, d, J = 8.3 Hz), 8.05 (1H, d, J = 8.3 Hz), 8.44 (1H, dd, J = 8.3, 2.2 Hz), 8.59 (1H, d, J = 2.4 Hz), 8.65 (1H, d, J = 4.6 Hz), 8.82 (1H, d, J = 2.2 Hz).
[Referential Example 74] Ethyl 5-(4-cyanophenyl)-1-(3-pyridyl)-1H-pyrazole-3-carboxylate (An alternative for the synthesis of Referential Example 24(3))

### 1) Ethyl 4-(4-cyanophenyl)-2,4-dioxobutanoate

Lithium bis(trimethylsilyl)amide (1.0M solution in tetrahydrofuran, 14.1 ml) was added dropwise to a solution of 4-acetyl benzonitrile (1.0 g) in tetrahydrofuran (50 ml) at -78°C over 10 minutes under argon atmosphere, and the mixture was stirred for another 30 minutes. Diethyl oxalate (1.41 ml) was added dropwise to the reaction liquid, and the mixture was stirred for 10 minutes, and at room temperature for another 0.5 hours. 1N aqueous hydrochloric acid and ethyl acetate were added to the reaction liquid and the phases were separated, and the organic layer was dried over anhydrous magnesium sulfate. After filtration, the solvent was evaporated under reduced pressure to give ethyl 4-(4-cyanophenyl)-2,4-dioxobutanoate (1.7 g, measured) as a solid.

¹H-NMR (400 MHz, CDCl₃)δ: 1.42 (3H, t, J = 7.08 Hz), 4.42 (2H, q, J = 7.08 Hz), 7.07 (1H, s), 7.81 (2H, d, J = 8.30 Hz), 8.08 (2H, d, J = 8.30 Hz).
EI-MSm/z: 245 (M⁺).

### 2) The title compound

Acetic acid (2.16 ml) was added to a solution of the ethyl 4-(4-cyanophenyl)-2,4-dioxobutanoate (3.0 g) and the 3-hydrazinopyridine (2.46 g) of Referential Example 32 in ethanol (300 ml), and the mixture was heated under reflux for 16 hours. After cooling with air, conc. hydrochloric acid (1.5 ml) was added to the reaction liquid, and the mixture was heated under reflux for 0.5 hours. After cooling with air, saturated aqueous sodium bicarbonate and chloroform were added to the reaction liquid and the phases were separated, and the organic layer was dried over anhydrous magnesium sulfate. After filtration, the solvent was evaporated under reduced pressure, and the residue was purified by column chromatography on silica gel (2% methanol-dichloromethane) to give the title compound (2.15 g, 55%) as a solid.
EI-MSm/z: 318 (M⁺).

[Referential Example 75] 5-(5-Cyano-2-pyridyl)-1-(6-methyl-3-pyridyl)-1H-pyrazole-3-carboxylic acid

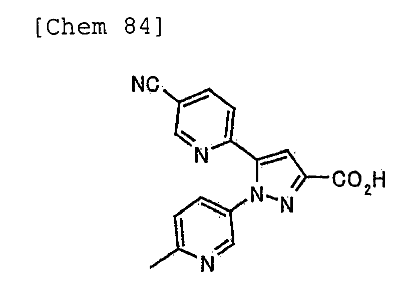

### 1) Ethyl 5-(5-benzyloxy-2-pyridyl)-1-(6-methyl-3-pyridyl)-1H-pyrazole-3-carboxylate

The procedure of Referential Example 9 (4) was repeated by using the ethyl 4-(5-benzyloxy-2-pyridyl)-2,4-dioxobutanoate (2.62 g) of Referential Example 14 (3) and the 5-hydrazino-2-methylpyridine (0.986 g) of Referential Example 31 to give ethyl 5-(5-benzyloxy-2-pyridyl)-1-(6-methyl-3-pyridyl)-1H-pyrazole-3-ca rboxylate (1.74 g, 52%) as a solid.

¹H-NMR (400 MHz, CDCl₃)δ: 1.42 (3H, t, J = 7.1 Hz), 2.60 (3H, s), 4.46 (2H, q, J = 7.1 Hz), 5.10 (2H, s), 7.19-7.42 (9H, m), 7.72 (1H, dd, J = 8.3, 2.4 Hz), 8.26 (1H, d, J = 2.9 Hz), 8.38 (1H, d, J = 2.4 Hz). FAB-MSm/z: 415 (M+H)⁺.

### 2) Ethyl 5-(5-hydroxy-2-pyridyl)-1-(6-methyl-3-pyridyl)-1H-pyrazole-3-carboxylate

To a solution of the ethyl 5-(5-benzyloxy-2-pyridyl)-1-(6-methyl-3-pyridyl)-1H-pyrazole-3-ca rboxylate (1.73 g) in a mixture of ethanol (34 ml) and ethyl acetate (34 ml) was added 10% palladium on carbon (1.73 g), and the mixture was stirred at room temperature in the presence of hydrogen gas for 3.5 hours. The reaction liquid was separated by filtration, and the solvent was evaporated under reduced pressure to give ethyl 5-(5-hydroxy-2-pyridyl)-1-(6-methyl-3-pyridyl)-1H-pyrazole-3-carb oxylate (1.28 g, 95%) as a solid.

¹H-NMR (400 MHz, DMSO-d₆)δ: 1.33 (3H, t, J = 7.1 Hz), 3.33 (3H, s), 4.34 (2H, q, J = 7 .1 Hz), 7.18-7.25 (2H, m), 7.34 (1H, d, J= 8.5Hz), 7.51 (1H, d, J = 8.5 Hz), 7.64-7.67 (1H, m), 7.97 (1H, d, J=2.9Hz), 8.35 (1H, d, J = 2.4 Hz), 10.31 (1H, s).
FAB-MSm/z: 325 (M+H)⁺.

### 3) The title compound

Trifluoromethanesulfonic anhydride (0.791 ml) was added dropwise to a solution of the ethyl 5-(5-hydroxy-2-pyridyl)-1-(6-methyl-3-pyridyl)-1H-pyrazole-3-carb oxylate (1.27 g) in a mixture of dichloromethane (25 ml) and pyridine (8.3 ml) at room temperature under argon atmosphere, and the mixture was stirred for 1 hour. To the reaction liquid was added another portion of the anhydrous trifluoromethanesulfonic anhydride (0.791 ml), and the mixture was stirred for 1 hour. Water and chloroform were added to the reaction liquid and the phases were separated, and the organic layer was dried over anhydrous sodium sulfate. After filtration, the solvent was evaporated under reduced pressure, and the residue was purified by column chromatography on silica gel (ethyl acetate-chloroform) to give ethyl 1-(6-methyl-3-pyridyl)-5-(5-trifluoromethanesulfonyloxy-2-pyridyl )-1H-pyrazole-3-carboxylate (1.77 g, 99%) as a solid.

¹H-NMR (400 MHz, CDCl₃)δ: 1.42-1.45 (3H, m), 2.63 (3H, s), 4.44-4.50 (2H, m), 7.24 (1H, d, J = 8.3 Hz), 7.34 (1H, s), 7.55-7.57 (1H, m), 7.65-7.70 (2H, m), 8.43-8.45 (2H, m).
FAB-MSm/z: 457 (M+H)⁺.
A suspension of tri-n-butyl tin cyanide (4.88 g) and tetrakis (triphenylphosphine)palladium(0) (6.68 g) in 1,2-dichloroethane (48 ml) was heated under reflux for 2 hours under argon atmosphere, and to the reaction liquid was added dropwise a solution of ethyl 1-(6-methyl-3-pyridyl)-5-(5-trifluoromethanesulfonyloxy-2-pyridyl )-1H-pyrazole-3-carboxylate (1.76 g) in 1,2-dichloroethane (39 ml), and the mixture was stirred at 80°C for 23 hours. After cooling with air, saturated aqueous sodium bicarbonate was added to the reaction liquid, and the mixture was filtered through celite, and water and chloroform were added to the filtrate and the phases were separated. The organic layer was dried over anhydrous sodium sulfate. After filtration, the solvent was evaporated under reduced pressure, and the residue was purified by column chromatography on silica gel (ethyl acetate-chloroform) to give ethyl
5-(5-cyano-2-pyridyl)-1-(6-methyl-3-pyridyl)-1H-pyrazole-3-carbox ylate (2.07 g). A solution of lithium hydroxide monohydrate (0.162 g) in water (21 ml) was added dropwise to a suspension of the resulting ethyl ester derivative in tetrahydrofuran (41 ml) at room temperature, and the mixture was stirred for 1.5 hours. Water and chloroform were added to the reaction liquid and the aqueous layer was separated. The aqueous layer was neutralized with 1N aqueous hydrochloric acid (3.86 ml), and the solid precipitate was collected by filtration to give the title compound (0.750 g, 64%) as a solid.

¹H-NMR (400 MHz, DMSO-d₆)δ: 2.54 (3H, s), 7.36 (1H, d, J = 8.3 Hz), 7.57 (1H, s), 7.71-7.73 (1H, m), 7.99 (1H, d, J = 8.3 Hz), 8.40-8.43 (2H, m), 8.84 (1H, d, J = 1.2 Hz), 13.20 (1H, br s).
FAB-MSm/z: 306 (M+H)⁺.

[Referential Example 76] Ethyl 1-(3-pyridyl)-5-{5-[2-(trimethylsilyl)ethynyl]-2-pyridyl}-1H-pyrazole-3-carboxylate

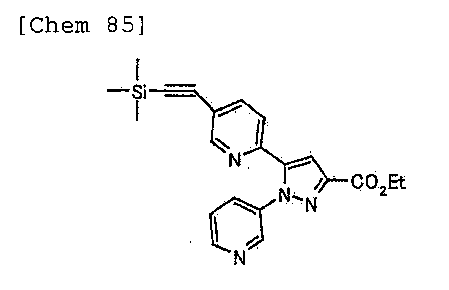

Trimethylsilyl acetylene (0.630 ml) was added to a solution of the ethyl 1-(3-pyridyl)-5-(5-trifluoromethanesulfonyloxy-2-pyridyl)-1H-pyra zole-3-carboxylate (1.85 g) of Referential Example 73 (2) in a mixture of N,N-dimethylformamide (10 ml) and triethylamine (3.6 ml) at room temperature, and the mixture was stirred for 15 minutes. To the reaction liquid was added bis(triphenylphosphine)palladium (II) dichloride (58.7 mg), and the mixture was stirred at 60°C for 1.5 days. Water and ethyl acetate were added to the reaction liquid and the phases were separated, and the organic layer was dried over anhydrous sodium sulfate. After filtration, the solvent was evaporated under reduced pressure, and the residue was purified by column chromatography on silica gel (ethyl acetate-hexane) to give the title compound (0.470 g, 28%) as a solid.

¹H-NMR (400 MHz, CDCl₃)δ: 0.25 (9H, s), 1.43 (3H, t, J = 7.2 Hz), 4.47 (2H, q, J = 7.1 Hz), 7.31 (1H, s), 7.35-7.41 (2H, m), 7.75 (1H, dd, J = 8.1, 2.0 Hz), 7.78-7.81 (1H, m), 8.50 (1H, d, J = 2.0 Hz), 8.54 (1H, d, J = 2.7 Hz), 8.61 (1H, dd, J = 4.9, 1.5 Hz).
ESI-MSm/z: 391 (M+H)⁺.

[Referential Example 77] Ethyl 5-(5-carbamoylmethyloxy-2-pyridyl)-1-(3-pyridyl)-1H-pyrazole-3-carboxylate

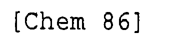

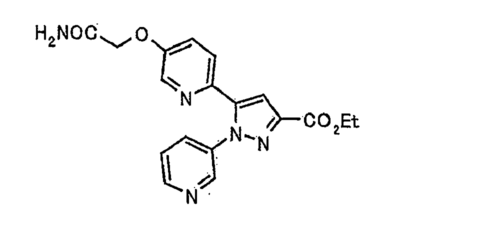

A suspension of the ethyl 5-(5-hydroxy-2-pyridyl)-1-(3-pyridyl)-1H-pyrazole-3-carboxylate (0.310 g) of Referential Example 73(1), 2-chloroacetamide (0.112 g), and potassium carbonate (0.415 g) in acetone (25 ml) was heated under reflux for 3.5 days. After cooling with air, ethyl acetate and saturated aqueous sodium bicarbonate were added to the reaction liquid and the phases were separated, and the aqueous layer was further extracted with ethyl acetate. The combined organic layers were dried over anhydrous sodium sulfate. After filtration, the solvent was evaporated under reduced pressure. And the aqueous layers obtained in the phase separation were combined, and the solvent was evaporated under reduced pressure. Ethanol was added to the residue, and the insoluble content was removed by filtration, and the solvent was evaporated under reduced pressure. The residues were combined and purified by column chromatography on silica gel (methanol-dichloromethane) to give the title compound (0.240 g, 65%) as a solid.

¹H-NMR (400 MHz, CDCl₃)δ: 1.43 (3H, t, J = 7.1 Hz), 4.47 (2H, q, J = 7.2 Hz), 4.54 (2H, s), 5.63 (1H, br s), 6.45 (1H, br s), 7.23 (1H, s), 7.24-7.27 (1H, m), 7.39 (1H, dd, J = 8.3, 5.1 Hz), 7.44 (1H, d, J = 8.8 Hz), 7.85-7.89 (1H, m), 8.21 (1H, d, J = 2.4 Hz), 8.49 (1H, d, J = 2.4 Hz), 8.60 (1H, dd, J = 4.9, 1.5 Hz).
ESI-MSm/z: 368 (M+H)⁺.

[Referential Example 78] 1-(6-Methoxy-3-pyridazinyl)-5-(1-methyl-1H-pyrrol-3-yl)-1H-pyrazole-3-carboxylic acid

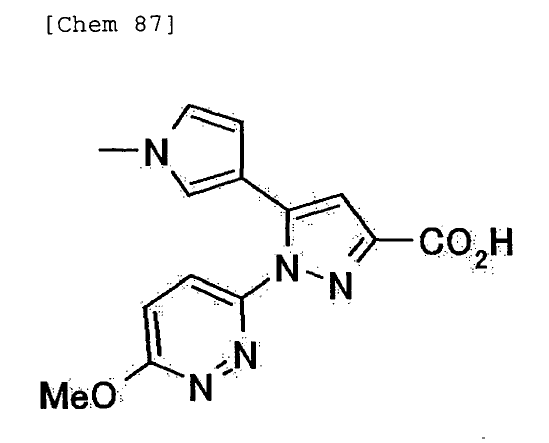

### 1) Methyl 1-(6-chloro-3-pyridazinyl)-5-(1-methyl-1H-pyrrol-3-yl)-1H-pyrazole-3-carboxylate

Lithium (trimethylsilyl) amide (1.0M solution in tetrahydrofuran, 27.8 ml) was added to a solution of 3-acetyl-1-methylpyrrole (3.00 ml) in tetrahydrofuran (30 ml) at -78°C, and the solution was stirred for 45 minutes. A solution of dimethyl oxalate (4.48 g) in tetrahydrofuran (15.0 ml) was added dropwise to the reaction liquid, and the temperature was elevated to room temperature, and the mixture' was stirred at room temperature for 1 hour. To the reaction liquid were added 3-chloro-6-hydrazinopyridazine (4.40g), conc. hydrochloric acid (2.08 ml), and methanol (150 ml), and the mixture was heated under reflux for 16 hours. Another portion of conc. hydrochloric acid (1.04 ml) to the reaction liquid, and the mixture was heated under reflux for 3 hours. After coolingwith air, the reaction solvent was evaporated under reducedpressure, and saturated aqueous sodiumbicarbonate, water, and ethyl acetate were added to the residue, and the resulting solid precipitate was collected by filtration to give methyl 1-(6-chloro-3-pyridazinyl)-5-(1-methyl-1H-pyrrol-3-yl)-1H-pyrazol e-3-carboxylate (2.83 g, 35%).

¹H-NMR (400 MHz, DMSO-d₆)δ: 3.57 (3H, s), 3.85 (3H, s), 5.92-5.95 (1H, m), 6.68-6.72 (1H, m), 6.89 (1H, br s), 7.01 (1H, d, J = 0.7 Hz), 8.10 (1H, d, J = 9.0 Hz), 8.21 (1H, d, J = 9.0 Hz).
EI-MSm/z: 317 (M⁺).

### 2) The title compound

Sodium methoxide (1.43 g) was added to a suspension of the methyl 1-(6-chloro-3-pyridazinyl)-5-(1-methyl-1H-pyrrol-3-yl)-1H-pyrazol e-3-carboxylate (2.80 g) in methanol (50 ml) and tetrahydrofuran (50 ml) at room temperature, and the mixture was heated under reflux for 4 hours. After cooling with air, 1N aqueous sodium hydroxide (18.0 ml) was added to the reaction liquid, and the mixture was stirred overnight. The reaction solventwas evaporatedunder reducedpressure, and water, diethylether, and 1N aqueous hydrochloric acid (50 ml) were added to the residue, and the resulting precipitate was removed by filtration. To the filtrate solvent was further added 1N aqueous hydrochloric acid (50 ml), and the resulting precipitate was collected by filtration to give the title compound (1.02 g, 39%).

¹H-NMR (400 MHz, DMSO-d₆)δ: 3.55 (3H, s), 4.11 (3H, s), 5.82-5.86 (1H, m), 6.65-6.68 (1H, m), 6.77 (1H, d, J = 1.6 Hz), 6.89 (1H, d, J = 1.6 Hz), 7.49 (1H, dd, J = 9.1, 1.6 Hz), 7.85 (1H, d, J = 9.1, 1.6 Hz), 13.00 (1H, br s).
EI-MSm/z: 299 (M⁺).

[Referential Example 79] 1-(6-Methoxy-3-pyridyl)-5-(5-methyl-2-pyridyl)-1H-pyrazole-3-carboxylic acid

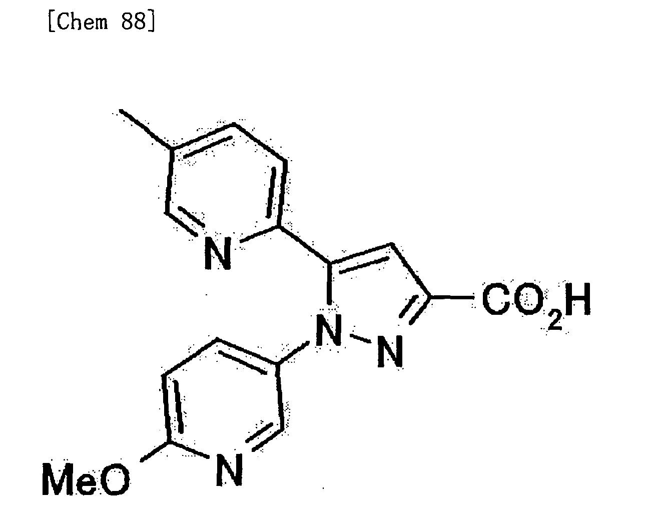

### 1) Ethyl 1-(6-methoxy-3-pyridyl)-5-(5-methyl-2-pyridyl)-1H-pyrazole-3-carboxylate

Acetic acid (8.74 ml) was added to a solution of the ethyl 4-(5-methyl-2-pyridyl)-2,4-dioxobutanoate (8.98 g) of Referential Example 35(2) and the 5-hydrazino-2-methoxypyridine (5.31 g) of Referential Example 2 in ethanol (135 ml) at room temperature, and the mixture was heated under reflux for 21 hours. After cooling with air, saturated aqueous sodium bicarbonate and ethyl acetate were added to the reaction liquid and the phases were separated, and the organic layer was dried over anhydrous sodium sulfate. After filtration, the solvent was evaporated under reduced pressure, and the residue was purified by column chromatography on silica gel (ethyl acetate-hexane) to give ethyl 1-(6-methoxy-3-pyridyl)-5-(5-methyl-2-pyridyl)-1H-pyrazole-3-carb oxylate (7.31 g, 57%) as a solid.

¹H-NMR (400 MHz, CDCl₃)δ: 1.42 (3H, t, J = 7.2 Hz), 2.34 (3H, s), 3.95 (3H, s), 4.45 (2H, q, J= 7.2 Hz), 6.76 (1H, d, J = 8.8 Hz), 7.23-7.30 (2H, m), 7.47-7.50 (1H, m), 7.66-7.69 (1H, m), 8.10 (1H, d, J = 2.4 Hz), 8.36 (1H, m).
FAB-MSm/z: 339 (M+H)⁺.

### 2) The title compound

To a solution of the ethyl 1-(6-methoxy-3-pyridyl)-5-(5-methyl-2-pyridyl)-1H-pyrazole-3-carb oxylate (1.00 g) inmethanol (20 ml) was added 1N aqueous sodiumhydroxide (7.39 ml), and the mixture was stirred at room temperature for 1.5 hours. The solvent was evaporated under reduced pressure, and water, chloroform, and 1N aqueous hydrochloric acid (7.39 ml) were added to the residue and the phases were separated, and the organic layer was dried over anhydrous sodium sulfate. After filtration, the solvent was evaporated under reduced pressure to give the title compound (0.789 g, 86%) as a solid.

¹H-NMR (400 MHz, DMSO-d₆)δ: 2.29 (3H, s), 3.89 (3H, s), 6.87-6.90 (1H, m), 7.26 (1H, s), 7.55-7.57 (1H, m), 7.67-7.72 (2H, m), 8.13 (1H, d, J = 2.8 Hz), 8.30 (1H, m), 13.04 (1H, br).
FAB-MSm/z: 311 (M+H)⁺.

[Referential Example 80] 5-[5-(tert-Butoxycarbonylamino)-2-pyrazinyl]-1-(6-methoxy-3-pyridyl)-1H-pyrazole-3-carboxylic acid

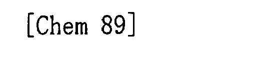

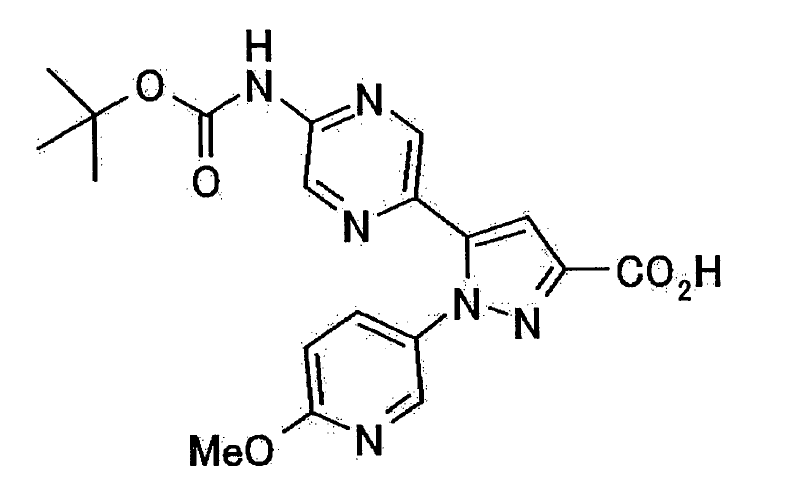

### 1) Ethyl 5-(5-carboxy-2-pyrazinyl)-1-(6-methoxy-3-pyridyl)-1H-pyrazole-3-carboxylate

Selenium dioxide (3.92 g) was added to a solution of the ethyl 1-(6-methoxy-3-pyridyl)-5-(5-methyl-2-pyrazinyl)-1H-pyrazole-3-ca rboxylate (3.00 g) of Referential Example 69 in pyridine (60 ml) under argon atmosphere, and the mixture was heated under reflux for 23 hours. After cooling with air, water and chloroform were added to the reaction liquid and the phases were separated, and the organic layer was dried over anhydrous sodium sulfate. After filtration, the solvent was evaporated under reduced pressure to give ethyl 5-(5-carboxy-2-pyrazinyl)-1-(6-methoxy-3-pyridyl)-1H-pyrazole-3-c arboxylate (2.83 g, 87%) as a solid.

¹H-NMR (400 MHz, DMSO-d₆)δ: 1.35 (3H, t, J = 7.1 Hz), 3.92 (3H, s), 4.37 (2H, q, J= 7.1 Hz), 6.92 (1H, d, J= 8.8 Hz), 7.36-7.40 (1H, m), 7.76-7.84 (1H, m), 8.25 (1H, m), 8.96 (1H, d, J = 1.5 Hz), 9.18 (1H, d, J = 1.5 Hz).
EI-MSm/z: 369 (M⁺).

### 2) Ethyl 5-[5-(tert-butoxycarbonylamino)-2-pyrazinyl]-1-(6-methoxy-3-pyridyl)-1H-pyrazole-3-carboxylate

Under argon atmosphere, triethylamine (1.17 ml), diphenylphosphorylazide (1.80 ml) and tert-butanol (1.60ml) were added to a suspension of the ethyl 5-(5-carboxy-2-pyrazinyl)-1-(6-methoxy-3-pyridyl)-1H-pyrazole-3-c arboxylate (2.81 g) in 1,4-dioxane (56 ml) at room temperature, and the mixture was stirred at 100°C for 25 minutes. After cooling with air, the reaction solvent was evaporated under reduced pressure, and the residue was purified by column chromatography on silica gel (ethyl acetate-hexane) to give ethyl 5-[5-(tert-butoxycarbonylamino)-2-pyrazinyl]-1-(6-methoxy-3-pyrid yl)-1H-pyrazole-3-carboxylate(1.29 g, 39%) as a solid.

¹H-NMR (400 MHz, CDCl₃)δ: 1.42-1.45 (3H, m), 1.54 (9H, s), 3.97 (3H, s), 4.44-4.50 (2H, m), 6.79 (1H, d, J = 8.8 Hz), 7.28 (1H, s), 7.36 (1H, br s), 7.68 (1H, dd, J = 8.8, 2.9 Hz), 8.11-8.12 (1H, m), 8.29 (1H, d, J = 1.7 Hz), 9.18 (1H, d, J = 1.5 Hz).
EI-MSm/z: 440 (M⁺).

### 3) The title compound

To a suspension of the ethyl 5-[5-(tert-butoxycarbonylamino)-2-pyrazinyl]-1-(6-methoxy-3-pyrid yl)-1H-pyrazole-3-carboxylate (1.28 g) in tetrahydrofuran (26 ml) and methanol (26 ml) was added 1N aqueous sodium hydroxide (7.27 ml) at room temperature, and the mixture was stirred for 4.5 hours. The reaction liquid was neutralized by adding 1N aqueous hydrochloric acid (7.27 ml), and water and chloroform were added and the phases were separated, and the solvent was evaporated under reduced pressure to give the title compound (1.19 g, 99%) as a solid.

¹H-NMR (400 MHz, DMSO-d₆)δ: 1.48 (9H, s), 3.91 (3H, m), 6.90 (1H, d, J = 8.8 Hz), 7.41 (1H, m), 7.75-7.78 (1H, m), 8.19 (1H, d, J = 2.7 Hz), 8.62-8.63 (1H, m), 8.86-8.87 (1H, m), 10.39 (1H, s), 13.10 (1H, br s). EI-MSm/z: 412 (M⁺).

[Referential Example 81] 1-(6-Methoxy-3-pyridazinyl)-5-(5-methyl-2-pyrazinyl)-1H-pyrazole-3-carboxylic acid

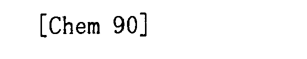

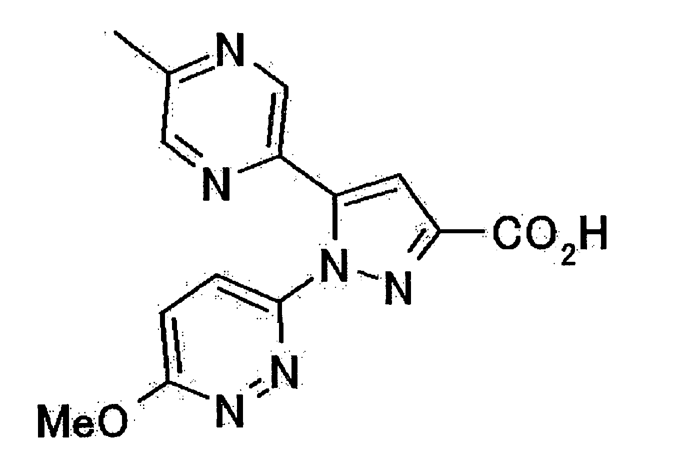

### 1) Methyl 4-(5-methyl-2-pyrazinyl)-2,4-dioxobutanoate

The procedure of Referential Example 36 (3) was repeated by using the 1-(5-methyl-2-pyrazinyl)-1-ethanone (7.24 g) of Referential Example 36(2), dimethyl oxalate (12.6 g), and lithium bis (trimethylsilyl) amide (1.0M tetrahydrofuran solution, 58.5 ml) to give methyl4-(5-methyl-2-pyrazinyl)-2,4-dioxobutanoate (7.84g, 66%) as a solid.

¹H-NMR (400 MHz, DMSO-d₆)δ: 2.63 (3H, s), 3.87 (3H, s), 7.41 (1H, br s), 8.73 (1H, s), 9.13 (1H, s).
EI-MSm/z: 222 (M⁺).

### 2) Methyl 1-(6-chloro-3-pyridazinyl)-5-(5-methyl-2-pyrazinyl)-1H-pyrazole-3-carboxylate

The procedure of Referential Example 9, Method B (2) was repeated by using the methyl 4- (5-methyl-2-pyrazinyl)-2,4-dioxobutanoate (7.83 g) and 3-chloro-6-hydrazinopyridazine (5.09 g) to give methyl 1-(6-chloro-3-pyridazinyl)-5-(5-methyl-2-pyrazinyl)-1H-pyrazole-3 -carboxylate (6.60 g, 56%) as a solid.

¹H-NMR (400 MHz, DMSO-d₆)δ: 2.53 (3H, s), 3.92 (3H, s), 7.63 (1H, s), 8.20-8.29 (2H, m), 8.37 (1H, m), 8.99 (1H, d, J = 1.5 Hz).
EI-MSm/z: 330 (M⁺).

### 3) The title compound

Under argon atmosphere, sodium methoxide (3.22 g) was added to a suspension of the methyl 1-(6-chloro-3-pyridazinyl)-5-(5-methyl-2-pyrazinyl)-1H-pyrazole-3 -carboxylate (6.58 g) in methanol (132 ml) at room temperature, and the mixture was heated under reflux for 2 hours. After cooling with air, water (132 ml) was added, and the mixture was stirred at room temperature for 1 hour. To the reaction liquid was added 1N aqueous hydrochloric acid (50 ml), and the mixture was stirred at room temperature for 10 minutes, and the solid precipitate was collected by filtration to give the title compound (5.50 g, 89%) as a solid.

¹H-NMR (400 MHz, DMSO-d₆)δ: 2.51 (3H, s), 4.05 (3H, s), 7.48-7.50 (2H, m), 8.04 (1H, d, J = 9.3 Hz), 8.37 (1H, m), 8.91 (1H, d, J = 1.5 Hz), 13.28 (1H, br s).
EI-MSm/z: 312 (M⁺).

[Referential Example 82] Methyl 1-(6-chloro-3-pyridazinyl)-5-(5-ethyl-2-pyridyl)-1H-pyrazole-3-carboxylate

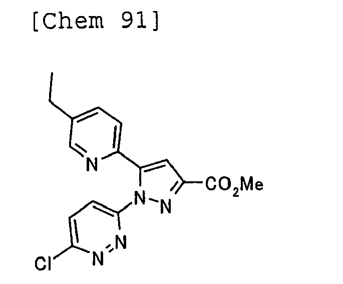

### 1) 5-Ethyl-N-methoxy-N-methyl-2-pyridinecarboxamide

Selenium dioxide (8.32 g) was added to a solution of 5-ethyl-2-methylpyridine (6.06 g) in pyridine (70 ml), and the mixture was stirred at 85°C for 19.5 hours, and then heated under reflux for 28 hours. After cooling with air, the reaction liquid was filtered, and the solvent was evaporated under reduced pressure. Water was added to the residue, and the mixture was filtered through celite, and the solvent was evaporated under reduced pressure to give a crude 5-ethyl-2-pyridinecarboxylic acid (5.79 g, 76%) as a solid. Triethylamine (10.7 ml) and 1-(3-dimethylaminopropyl)-3-ethylcarbodiimide hydrochloride (11.0 g) were added to a suspension of this crude 5-ethyl-2-pyridinecarboxylic acid (5.79 g), N, O-dimethylhydroxylamine hydrochloride (5.61 g), and 1-hydroxybenzotriazole (8.81 g) in N,N-dimethylformamide (100 ml) at room temperature, and the mixture was stirred for 16 hours. The reaction solvent was evaporated under reduced pressure, and ethyl acetate and saturated aqueous sodium bicarbonate were added to the residue and the phases were separated. The aqueous layer was extracted twice with ethyl acetate, and the combined organic layers were dried over anhydrous sodium sulfate. After filtration, the solvent was evaporated under reduced pressure, and the residue was purifiedby column chromatography on silica gel (ethyl acetate-hexane) to give 5-ethyl-N-methoxy-N-methyl-2-pyridinecarboxamide (6.66 g, 89%) as an oily product.

¹H-NMR (400 MHz, CDCl₃)δ: 1.28 (3H, t, J = 7.6 Hz), 2.71 (2H, q, J = 7.6 Hz), 3.42 (3H, s), 3.77 (3H, s), 7.62 (2H, br s), 8.46 (1H, s).
ESI-MSm/z: 195 (M+H)⁺.

### 2) 1-(5-Ethyl-2-pyridyl)-1-ethanone

Methyl lithium (0.98M solution in diethylether, 38.5 ml) was added to a solution of the 5-ethyl-N-methoxy-N-methyl-2-pyridinecarboxamide (6.65 g) in tetrahydrofuran (100 ml) at -78°C, and the mixture was stirred for 15 minutes, and at room temperature for another 3 hours. Saturated aqueous sodium bicarbonate and ethyl acetate were added to the reaction liquid and the phases were separated, and the aqueous layer was further extracted with ethyl acetate. The combined organic layers were dried over anhydrous sodium sulfate. After filtration, the solvent was evaporated under reduced pressure, and the residue was purified by column chromatography on silica gel (ethyl acetate-hexane) to give 1-(5-ethyl-2-pyridyl)-1-ethanone (4.63 g, 90%) as an oily product.

¹H-NMR (400 MHz, CDCl₃)δ: 1.29 (3H, t, J = 7.6 Hz), 2.71 (3H, s), 2.74 (2H, q, J= 7. 6 Hz), 7 . 63-7. 66 (1H, m), 7.98 (1H, d, J = 8.1 Hz), 8.52-8.53 (1H, m).
ESI-MSm/z: 150 (M+H)⁺.

### 3) The title compound

Lithium bis(trimethylsilyl)amide (1.0M solution in tetrahydrofuran, 12.2 ml) was added to a solution of 1- (5-ethyl-2-pyridyl) -1-ethanone (1.52 g) in tetrahydrofuran (10 ml) at -78°C, and the mixture was stirred for 30 minutes. Dimethyl oxalate (1.77 g) was added to the reaction liquid, and the mixture was stirred at the same temperature for 10 minutes, and the stirring was continued for another 2.5 hours while the temperature was gradually resumed to room temperature. Diethylether and water were added to the reaction liquid and the aqueous layer was separated, and 1N aqueous hydrochloric acid (13 ml) was added to the aqueous layer, and the aqueous layer was extracted three times with dichloromethane. The combined organic layers were dried over anhydrous sodium sulfate. After filtration, the solvent was evaporated under reduced pressure to give crude methyl 4-(5-ethyl-2-pyridyl)-2,4-dioxobutanoate (2.12 g, 89%). 3-chloro-6-hydrazinopyridazine (1.30 g) was added to a solution of this crude methyl 4-(5-ethyl-2-pyridyl)-2,4-dioxobutanoate (2.12 g) in methanol, and the mixture was heated under reflux for 15.5 hours. Conc. hydrochloric acid (0.375 ml) was added to the reaction liquid, and the mixture was heated under reflux for 2 hours. Another portion of conc. hydrochloric acid (0.375 ml) was added to the reaction liquid, and the mixture was heated under reflux for 18 hours. After cooling with air, ethyl acetate and saturated aqueous sodium bicarbonate were added to the reaction liquid and the phases were separated, and the aqueous layer was further extracted with ethyl acetate. The combined organic layers were dried over anhydrous sodium sulfate. After filtration, the solvent was evaporated under reduced pressure, and the residue was purified by column chromatography on silica gel (ethyl acetate-hexane) to give the title compound (0.934 g, 30%) as a solid.

¹H-NMR (400 MHz, CDCl₃)δ: 1.27 (3H, t, J = 7.6 Hz), 2.66 (2H, q, J = 7.6 Hz), 4.00 (3H, s), 7.21 (1H, s), 7.55 (1H, dd, J = 8.1, 0.7 Hz), 7.61 (1H, dd, J = 8.1, 2.2 Hz), 7.69 (1H, d, J = 9.0 Hz), 8.07 (1H, d, J = 9.0 Hz), 8.23 (1H, d, J = 2.2 Hz).
ESI-MSm/z: 344[(M+H)⁺, ³⁵Cl], 346[(M+H)⁺, ³⁷Cl].

[Referential Example 83] 1-(6-Methoxy-3-pyridyl)-5-(6-methyl-3-pyridyl)-1H-pyrazole-3-carboxylic acid

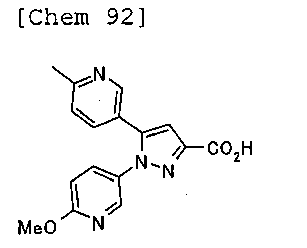

### 1) N-Methoxy-N-methyl-6-methylpyridine-3-carboxamide

Triethylamine (40.7 ml) was added to a suspension of 6-methylnicotinic acid (20.0 g), N,O-dimethylhydroxylamine hydrochloride (14.2 g), 1-ethyl-3-(3-dimethylaminopropyl)-carbodiimide hydrochloride (28.0 g), and 1-hydroxybenzotriazole (7.88 g) in dichloromethane (200 ml) at 0°C under argon atmosphere, and the mixture was stirred for 30 minutes, and at room temperature for another 90 hours. Water and chloroform were added to the reaction liquid and the phases were separated, and the organic layer was dried over anhydrous sodium sulfate. After filtration, the solvent was evaporated under reduced pressure, and the residue was purified by column chromatography on silica gel (ethyl acetate-chloroform) to give N-methoxy-N-methyl-6-methylpyridine-3-carboxamide (17.5 g, 67%) as an oily product.
¹H-NMR (400 MHz, CDCl₃)δ: 2.60 (3H, s), 3.38 (3H, s), 3.56 (3H, s), 7.21 (1H, d, J= 8.1 Hz), 7.93-7.95 (1H, m), 8.86 (1H, d, J = 2.0 Hz). EI-MSm/z: 180 (M⁺).

### 2) 1-(6-Methyl-3-pyridyl)-1-ethanone

The procedure of Referential Example 82 (2) was repeated by using N-methoxy-N-methyl-6-methylpyridine-3-carboxamide (17.5 g) and methyl lithium (0.98M solution in diethylether, 104 ml) to give 1-(6-methyl-3-pyridyl)-1-ethanone (12.3 g, 94%) as an oily product.

¹H-NMR (400 MHz, CDCl₃)δ: 2.61 (3H, s), 2.62 (3H, s), 7.25-7.27 (1H, m), 8.11-8.13 (1H, m), 9.04 (1H, d, J = 2.2 Hz).
ESI-MSm/z: 136 (M+H)⁺.

### 3) Ethyl 4-(6-methyl-3-pyridyl)-2,4-dioxobutanoate

The procedure of Referential Example 36 (3) was repeated by using 1- (6-methyl-3-pyridyl) -1-ethanone (6.29 g), diethyl oxalate (12.6ml), and lithiumbis (trimethylsilyl) amide (1.0M solution in tetrahydrofuran, 51.2ml) to give ethyl 4-(6-methyl-3-pyridyl)-2,4-dioxobutanoate(5.08 g, 47%) as a solid.

¹H-NMR (400 MHz, CDCl₃)δ: 1.39-1.42 (3H, m), 2.65 (3H, s), 4.37-4.42 (2H, m), 7.03 (1H, s), 7.30 (1H, d, J = 8.3 Hz), 8.13-8.16 (1H, m), 9.07 (1H, d, J = 2.2 Hz).
EI-MSm/z: 235 (M⁺).

### 4) Ethyl 1-(6-methoxy-3-pyridyl)-5-(6-methyl-3-pyridyl)-1H-pyrazole-3-carboxylate

The procedure of Referential Example 14 (4) was repeated by using the ethyl 4-(6-methyl-3-pyridyl)-2,4-dioxobutanoate and the 5-hydrazino-2-methoxypyridine (2.99 g) of Referential Example 2 to give ethyl 1-(6-methoxy-3-pyridyl)-5-(6-methyl-3-pyridyl)-1H-pyrazole-3-carb oxylate (4.68 g, 64%) as an oily product.

¹H-NMR (400 MHz, CDCl₃)δ: 1.41-1.44 (3H, m), 2.57 (3H, s), 3.94 (3H, s), 4.43-4.49 (2H, m), 6.77 (1H, d, J = 8.8 Hz), 7.08 (1H, s), 7.13 (1H, d, J = 8.1 Hz), 7.39 (1H, dd, J = 8.1, 2.2 Hz), 7.59 (1H, dd, J = 8.8, 2.7 Hz), 8.08 (1H, d, J = 2.7 Hz), 8.41 (1H, d, J = 2.2 Hz). EI-MSm/z: 338 (M⁺).

### 5) The title compound

The procedure of Referential Example 14 (5) was repeated by using ethyl 1-(6-methoxy-3-pyridyl)-5-(6-methyl-3-pyridyl)-1H-pyrazole-3-carb oxylate (1.10 g) to give the title compound (0.873 g, 86%) as a solid.

¹H-NMR (400 MHz, DMSO-d₆)δ: 2.47 (3H, s), 3.89 (3H, s), 6.92 (1H, d, J = 9.0 Hz), 7.16 (1H, s), 7.26 (1H, d, J = 8.3 Hz), 7.53-7.56 (1H, m), 7.75 (1H, dd, J = 8.8, 2.7 Hz), 8.17 (1H, d, J = 2.7 Hz), 8.40 (1H, d, J = 2.2 Hz), 13.05 (1H, br s).
EI-MSm/z: 310 (M⁺).

[Referential Example 84] 5-[6-(tert-Butoxycarbonylamino)-3-pyridyl]-1-(6-methoxy-3-pyridyl)-1H-pyrazole-3-carboxylic acid

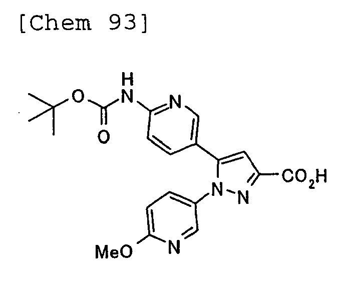

### 1) Ethyl 5-(6-carboxy-3-pyridyl)-1-(6-methoxy-3-pyridyl)-1H-pyrazole-3-carboxylate

The procedure of Referential Example 57 (1) was repeated by using the ethyl 1-(6-methoxy-3-pyridyl)-5-(6-methyl-3-pyridyl)-1H-pyrazole-3-carb oxylate (3.45 g) of Referential Example 83(4) and selenium dioxide (4.53 g) to give ethyl
5-(6-carboxy-3-pyridyl)-1-(6-methoxy-3-pyridyl)-1H-pyrazole-3-car boxylate (3.86 g, quantitative) as an amorphous product.

¹H-NMR (400 MHz, DMSO-d₆)δ: 1.34 (3H, t, J = 7.1 Hz), 3.90 (3H, s), 4.36 (2H, q, J = 7.1 Hz), 6.95 (1H, d, J = 8.8 Hz), 7.36-7.40 (1H, m), 7.79-7.87 (2H, m), 8.03 (1H, d, J = 8.1 Hz), 8.22 (1H, d, J = 2.7 Hz), 8.65 (1H, m).
EI-MSm/z: 368 (M⁺).

### 2) Ethyl 5-[6-(tert-butoxycarbonylamino)-3-pyridyl]-1-(6-methoxy-3-pyridyl)-1H-pyrazole-3-carboxylate

The procedure of Referential Example 57 (2) was repeated by using ethyl 5-(6-carboxy-3-pyridyl)-1-(6-methoxy-3-pyridyl)-1H-pyrazole-3-car boxylate (3.84 g) to give ethyl 5-[6-(tert-butoxycarbonylamino)-3-pyridyl]-1-(6-methoxy-3-pyridyl )-1H-pyrazole-3-carboxylate (2.56 g, 57%) as a solid.

¹H-NMR (400 MHz, CDCl₃)δ: 1.41-1.45 (3H, m), 1.52 (9H, s), 3.95 (3H, s), 4.43-4.49 (2H, m), 6.75-6.78 (1H, m), 7.05 (1H, s), 7.37-7.46 (2H, m), 7.58 (1H, dd, J = 8.8, 2.7 Hz), 7.95 (1H, d, J = 8.8 Hz), 8.10 (1H, d, J = 2.2 Hz), 8.18-8.19 (1H, m).
EI-MSm/z: 439 (M⁺).

### 3) The title compound

The procedure of Referential Example 14 (5) was repeated by using ethyl 5-[6-(tert-butoxycarbonylamino)-3-pyridyl]-1-(6-methoxy-3-pyridyl )-1H-pyrazole-3-carboxylate (2.55 g) to give the title compound (1.75 g, 73%) as a solid.

¹H-NMR (400 MHz, DMSO-d₆)δ: 1.47 (9H, s), 3.89 (3H, s), 6.92 (1H, d, J = 8.8 Hz), 7.12 (1H, s), 7.59 (1H, dd, J = 8.8, 2.4 Hz), 7.73-7.78 (2H, m), 8.16-8.18 (2H, m), 9.95 (1H, s), 13.01 (1H, br s).
EI-MSm/z: 411 (M⁺).

[Referential Example 85] 1-(6-Methoxy-3-pyridazinyl)-5-(6-methyl-3-pyridyl)-1H-pyrazole-3-carboxylic acid

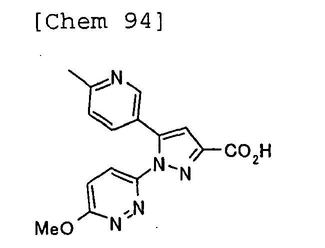

### 1) Methyl 4-(6-methyl-3-pyridyl)-2,4-dioxobutanoate

Under argon atmosphere, lithium bis (trimethylsilyl) amide (1.0M solution in tetrahydrofuran, 48.8 ml) was added dropwise to a solution of the 1-(6-methyl-3-pyridyl)-1-ethanone (6.00 g) of Referential Example 83 (2) in tetrahydrofuran (90 ml) over 20 minutes at -78°C, and the mixture was stirred for 20 minutes. A solution of dimethyl oxalate (10.5 g) in tetrahydrofuran (30 ml) was added dropwise to the reaction liquid over 15 minutes, and the mixture was stirred for 20 minutes, and the stirring was continued at 0°C for 1 hour, and at room temperature for 3 hours. Water and diethylether were added to the reaction liquid and the phases were separated, and saturated aqueous ammonium chloride and chloroform were added to the aqueous layer and the phases were separated, and the organic layer was dried over anhydrous sodium sulfate. After filtration, the solvent was evaporated under reduced pressure to give methyl 4- (6-methyl-3-pyridyl) -2, 4-dioxobutanoate (5. 63 g, 57%) as a solid.

¹H-NMR (400 MHz, CDCl₃)δ: 2.66 (3H, s), 3.96 (3H, s), 7.05 (1H, s), 7.31 (1H, d, J = 8.3 Hz), 8.15-8.17 (1H, m), 9.08 (1H, d, J= 2.2 Hz). EI-MSm/z: 221 (M⁺).

### 2) Methyl 1-(6-chloro-3-pyridazinyl)-5-(6-methyl-3-pyridyl)-1H-pyrazole-3-carboxylate

The procedure of Referential Example 9, Method B (2) was repeated by using methyl 4-(6-methyl-3-pyridyl)-2,4-dioxobutanoate (5.62 g) and 3-chloro-6-hydrazinopyridazine (3.67 g) to give methyl 1-(6-chloro-3-pyridazinyl)-5-(6-methyl-3-pyridyl)-1H-pyrazole-3-c arboxylate (4.82 g, 58%) as a solid.

¹H-NMR (400 MHz, CDCl₃)δ: 2.60 (3H, s), 4.01 (3H, m), 7.07-7.08 (1H, m), 7.21 (1H, d, J = 8.1 Hz), 7.64-7.67 (1H, m), 7.69 (1H, dd, J = 9.0, 1.0 Hz), 8.19 (1H, dd, J = 9.0, 1.0 Hz), 8.44-8.45 (1H, m).
FAB-MSm/z: 330 (M+H)⁺.

### 3) The title compound

Sodium methoxide (2.36 g) was added to a suspension of the methyl 1-(6-chloro-3-pyridazinyl)-5-(6-methyl-3-pyridyl)-1H-pyrazole-3-c arboxylate (4.81 g) in methanol (96 ml) at room temperature under argon atmosphere, and the mixture was heated under reflux for 1 hour and 40 minutes. After cooling with air, water (96 ml) was added to the reaction liquid, and the mixture was stirred for 30 minutes. To the reaction liquid was added 1N aqueous hydrochloric acid (29.2 ml), and the solid precipitate was collected by filtration to give the title compound (4.40 g, 97%).

¹H-NMR (400 MHz, DMSO-d₆)δ: 2.49 (3H, s), 4.03 (3H, s), 7.18 (1H, s), 7.26 (1H, d, J = 7.8 Hz), 7.51 (1H, d, J = 9.3 Hz), 7.60-7.62 (1H, m), 8.05 (1H, d, J = 9.3 Hz), 8.42 (1H, m), 13.22 (1H, br s).
FAB-MSm/z: 312 (M+H)⁺.

[Referential Example 86] 4,4-Difluoropiperidine hydrochloride

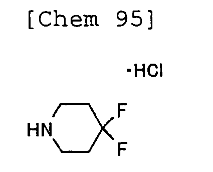

### 1) N-Benzyl-4,4-difluoropiperidine

Under argon atmosphere, diethylaminosulfur trifluoride (8.38 ml) was added dropwise to a solution of 1-benzyl-4-piperidone (5.00 g) in benzene (200 ml) at 0°C, and the mixture was stirred for 30 minutes, and heated under reflux for 18 hours. Saturated aqueous sodium bicarbonate and ethyl acetate were added and the phases were separated at 0°C, and the organic layer was dried over anhydrous sodium sulfate. After filtration, the solvent was evaporated under reduced pressure, and the residue was purified by column chromatography on silica gel (hexane-ethyl acetate) to give N-benzyl-4,4-difluoropiperidine (4.67 g, 84%) as an oily product.

¹H-NMR (400 MHz, CDCl₃)δ: 1.93-2.04 (4H, m), 2.53-2.55 (4H, m), 3.54 (2H, s), 7.24-7.34 (5H, m).
EI-MSm/z: 211 (M⁺).

### 2) The title compound

Under argon atmosphere, 1-chloroethyl chloroformate (2.62 ml) was added dropwise to a solution of N-benzyl-4,4-difluoropiperidine (4. 66 g) in dichloromethane (93 ml) at 0°C, and the mixture was stirred at 55°C for 2 hours. After cooling with air, the reaction solvent was evaporated under reduced pressure, and a solution of the residue in methanol (93 ml) was heated under reflux for 4 hours. After cooling with air, the reaction solvent was evaporated under reduced pressure to give the title compound (3.03 g, 87%) as a solid.
FAB-MSm/z: 122 (M+H)⁺.

[Referential Example 87] 5-(5-Benzyloxy-2-pyridyl)-1-(6-methoxy-3-pyridazinyl)-1H-pyrazole-3-carboxylic acid

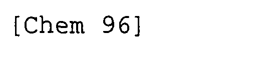

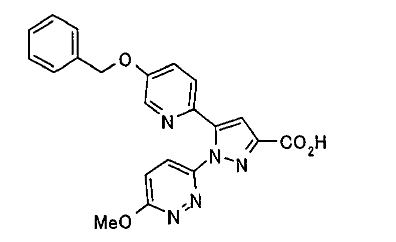

### 1) Methyl 4-(5-benzyloxy-2-pyridyl)-2,4-dioxobutanoate

The procedure of Referential Example 9, Method (B) (1) was repeated by using sodium methoxide (1.5 g), dimethyl oxalate (2.91 g), and the 1-(5-benzyloxy-2-pyridyl)-1-ethanone (3.0 g) of Referential Example 14(2) to give methyl 4-(5-benzyloxy-2-pyridyl)-2,4-dioxobutanoate (4.2 g, measured) as a solid.

¹H-NMR (400 MHz, CDCl₃)δ: 3.92 (3H, s), 5.21 (2H, s), 7.30-7.45 (5H, m), 7.61 (1H, s), 8.15 (1H, d, J = 8.6 Hz), 8.44 (1H, d, J= 2.9 Hz). EI-MSm/z: 313 (M⁺).

### 2) Methyl 5-(5-benzyloxy-2-pyridyl)-1-(6-methoxy-3-pyridazinyl)-1H-pyrazole-3-carboxylate

The procedure of Referential Example 9, Method B (2) was repeated by using methyl 4-(5-benzyloxy-2-pyridyl)-2,4-dioxobutanoate (4.2 g) and 3-chloro-6-hydrazinopyridazine (2.1 g) to give methyl 5-(5-benzyloxy-2-pyridyl)-1-(6-chloro-3-pyridazinyl)-1H-pyrazole-3-carboxylate as a solid. By using this chloro derivative and sodium methoxide (1.5 g), the procedure of Referential Example 9, Method B (3) was repeated to give methyl 5-(5-benzyloxy-2-pyridyl)-1-(6-methoxy-3-pyridazinyl)-1H-pyrazole -3-carboxylate (4.1 g, 73%) as a solid.

¹H-NMR (400 MHz, CDCl₃)δ: 3.98 (3H, s), 4.11 (3H, s), 5.10 (2H, s), 7.13-7.16 (2H, m), 7.26 (1H, s), 7.29 (1H, dd, J = 8.6, 2.9 Hz), 7.32-7.36 (1H, m), 7.36-7.40 (3H, m), 7.51 (1H, d, J = 8.5 Hz), 7.89-7.91 (1H, m), 8.16 (1H, d, J = 2.9 Hz).
EI-MSm/z: 417 (M⁺).

### 3) The title compound

The procedure of Referential Example 14 (5) was repeated by using methyl 5-(5-benzyloxy-2-pyridyl)-1-(6-methoxy-3-pyridazinyl)-1H-pyrazole -3-carboxylate (4.0 g) to give the title compound (3.9 g, measured) as an amorphous product.
EI-MSm/z: 403 (M⁺).

[Referential Example 88] 5-(5-Benzyloxy-2-pyridyl)-1-(6-methyl-3-pyridyl)-1H-pyrazole-3-carboxylic acid

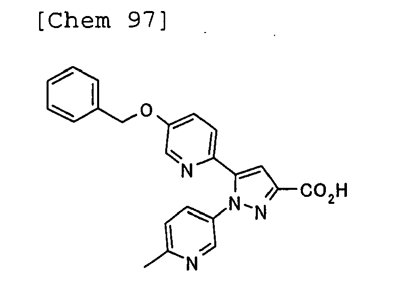

Lithium hydroxide monohydrate (150 mg) was added to a solution of the ethyl 5-(5-benzyloxy-2-pyridyl)-1-(6-methyl-3-pyridyl)-1H-pyrazole-3-ca rboxylate (1.4 g) of Referential Example 75 (1) in tetrahydrofuran (25 ml) and water (10 ml), and the mixture was stirred at room temperature for 2 hours. To the reaction liquid was added 1N aqueous hydrochloric acid (3.8 ml), and the solid precipitated was collected by filtration to give the title compound (1.3 g, measured).

¹H-NMR (400 MHz, DMSO-d₆)δ: 3.23 (3H, s), 5.19 (2H, s), 7.24 (1H, s), 7.35-7.67 (9H, m), 8.22 (1H, s), 8.36 (1H, s), 13.06 (1H, s).
FAB-MSm/z: 387 (M+H)⁺.

[Referential Example 89] 5-(5-Cyano-2-pyridyl)-1-(6-methoxy-3-pyridazinyl)-1H-pyrazole-3-carboxylic acid

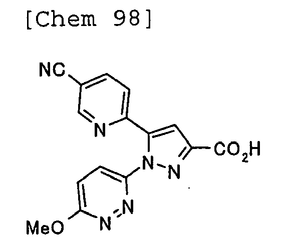

### 1) Methyl 4-(5-cyano-2-pyridyl)-2,4-dioxobutanoate

The procedure of Referential Example 85 (1) was repeated by using the 1-(5-cyano-2-pyridyl)-1-ethanone (5.0 g) of Referential Example 73, Method B(1) and dimethyl oxalate (8.0 g) to give methyl 4-(5-cyano-2-pyridyl)-2,4-dioxobutanoate (8.0 g, quantitative) as a solid.

¹H-NMR (400 MHz, DMSO-d₆)δ: 3.87 (3H, s), 7.47 (1/2 x 1H, br s), 8.13 (1H, dd, J = 8.3, 2.2 Hz), 9.20-9.21 (1H, m).
EI-MSm/z: 232 (M⁺).

### 2) Methyl 5-(5-cyano-2-pyridyl)-1-(6-methoxy-3-pyridazinyl)-1H-pyrazole-3-carboxylate

The procedure of Referential Example 9, Method B (2) was repeated by using methyl 4-(5-cyano-2-pyridyl)-2,4-dioxobutanoate (8.0 g) and 3-chloro-6-hydrazinopyridazine (5.4 g) to give methyl 5-(5-cyano-2-pyridyl)-1-(6-chloro-3-pyridazinyl)-1H-pyrazole-3-ca rboxylate. By using this chloro derivative and sodium methoxide (3.0 g), the procedure of Referential Example 9, Method B (3) was repeated to give methyl 5-(5-cyano-2-pyridyl)-1-(6-methoxy-3-pyridazinyl)-1H-pyrazole-3-c arboxylate (3.3 g, 28%) as a solid.

¹H-NMR (400 MHz, CDCl₃)δ: 4.00 (3H, s), 4.12 (3H, s), 7.19 (1H, d, J = 9.3 Hz), 7.32 (1H, s), 7.97 (1H, dd, J = 9.3 Hz), 8.02-8.05 (1H, m), 8.66 (1H, d, J = 2.2 Hz).
EI-MSm/z: 336 (M⁺).

### 3) The title compound

The procedure of Referential Example 88 was repeated by using methyl 5-(5-cyano-2-pyridyl)-1-(6-methoxy-3-pyridazinyl)-1H-pyrazole-3-c arboxylate (3.3 g) and lithium hydroxide monohydrate (412 mg) to give the title compound (1.97 g, 61%) as a solid.

¹H-NMR (400 MHz, DMSO-d₆)δ: 4.03 (3H, s), 7.49-7.53 (1H, m), 7.58 (1H, s), 8.02-8.07 (2H, m), 8.40-8.43 (1H, m), 8.80-8.82 (1H, m), 13.33 (1H, br s).
EI-MSm/z: 322 (M⁺).

[Referential Example 90] 5-(5-Cyano-2-pyridyl)-1-(6-methoxy-3-pyridyl)-1H-pyrazole-3-carboxylic acid

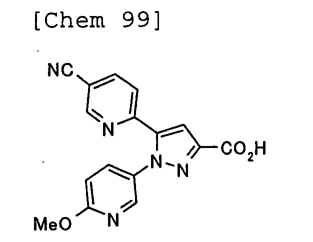

### 1) Ethyl 5-(5-cyano-2-pyridyl)-1-(6-methoxy-3-pyridyl)-1H-pyrazole-3-carboxylate

A solution of the ethyl 4-(5-cyano-2-pyridyl)-2,4-dioxobutanoate (4.0 g) of Referential Example 73, Method B(2) and the 5-hydrazino-2-methoxypyridine (2.6 g) of Referential Example 2 in ethanol (100 ml) was stirred at an outer temperature of 80°C for 20 hours. After cooling with air, the solid precipitate was separated by filtration, and the thus obtained solid was washed with ethanol and purified by column chromatography on silica gel (chloroform) to give ethyl 5-(5-cyano-2-pyridyl)-1-(6-methoxy-3-pyridyl)-1H-pyrazole-3-carbo xylate (2.8 g, 43%) as a solid.

¹H-NMR (400 MHz, CDCl₃) δ: 1.43 (3H, t, J = 7.1 Hz), 3.97 (3H, s), 4.46 (2H, q, J = 7.1 Hz), 6.80 (1H, d, J = 8.8 Hz), 7.40 (1H, s), 7.52 (1H, d, J = 8.1 Hz), 7.65 (1H, dd, J = 8.8, 2.2 Hz), 7.96 (1H, dd, J = 8.3, 1.5 Hz), 8.09 (1H, d, J = 2.7 Hz), 8.74 (1H, d, J = 2.2 Hz).
FAB-MSm/z: 350 (M+H)⁺.

### 2) The title compound

The procedure of Referential Example 88 was repeated by using ethyl 5-(5-cyano-2-pyridyl)-1-(6-methoxy-3-pyridyl)-1H-pyrazole-3-carbo xylate (1.3 g) and lithium hydroxide monohydrate (172 mg) to give the title compound (1.2 g, quantitative) as a solid.

¹H-NMR (400 MHz, DMSO-d₆)δ: 3.91 (3H, s), 6.91 (1H, d, J = 8.8 Hz), 7.56 (1H, s), 7.73-7.78 (1H, m), 7.94-7.97 (1H, m), 8.19-8.20 (1H, m), 8.38-8.41 (1H, m), 8.85-8.87 (1H, m), 13.00-13.40 (1H, br).
FAB-MSm/z: 322 (M+H)⁺.

[Referential Example 91] 5-[5-(tert-Butoxycarbonylamino)-2-pyrazinyl]-1-(6-methoxy-3-pyridazinyl)-1H-pyrazole-3-carboxylic acid

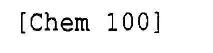

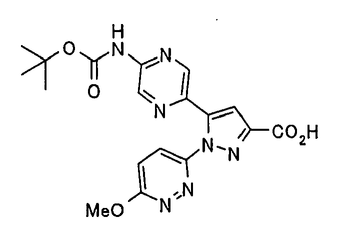

### 1) Methyl 4-(5-methyl-2-pyrazinyl)-2,4-dioxobutanoate

The procedure of Referential Example 85 (1) was repeated by using the 1-(5-methyl-2-pyrazinyl)-1-ethanone (7.24 g) of Referential Example 36(2), dimethyl oxalate (12.6 g), and lithium bis (trimethylsilyl) amide (1.0M solution in tetrahydrofuran, 58.5 ml) to give methyl 4-(5-methyl-2-pyrazinyl)-2,4-dioxobutanoate (7.84 g, 66%) as a solid.

¹H-NMR (400 MHz, DMSO-d₆)δ: 2.63 (3H, s), 3.87 (3H, s), 7.41 (1H, br s), 8.73 (1H, s), 9.13 (1H, s).
EI-MSm/z: 222 (M⁺).

### 2) Methyl 1-(6-chloro-3-pyridazinyl)-5-(5-methyl-2-pyrazinyl)-1H-pyrazole-3-carboxylate

The procedure of Referential Example 9, Method B (2) was repeated by using methyl 4- (5-methyl-2-pyrazinyl)-2,4-dioxobutanoate (7.83 g) and 3-chloro-6-hydrazinopyridazine (5.09 g) to give methyl 1-(6-chloro-3-pyridazinyl)-5-(5-methyl-2-pyrazinyl)-1H-pyrazole-3 -carboxylate (6.60 g, 56%) as a solid.

¹H-NMR (400 MHz, DMSO-d₆)δ: 2.53 (3H, s), 3.92 (3H, s), 7.63 (1H, s), 8.20-8.29 (2H, m), 8.37 (1H, m), 8.99 (1H, d, J = 1.5 Hz).
EI-MSm/z: 330 (M⁺).

### 3) 1-(6-methoxy-3-pyridazinyl)-5-(5-methyl-2-pyrazinyl)-1H-pyrazole-3-carboxylic acid

Under argon atmosphere, sodium methoxide (3.22 g) was added to a suspension of methyl 1-(6-chloro-3-pyridazinyl)-5-(5-methyl-2-pyrazinyl)-1H-pyrazole-3 -carboxylate (6.58 g) in methanol (132 ml) at room temperature, and the mixture was heated under reflux for 2 hours. After cooling with air, water (132 ml) was added, and the mixture was stirred at room temperature for 1 hour. 1N aqueous hydrochloric acid (50 ml) was added to the reaction liquid, and the mixture was stirred at room temperature for 10 minutes, and the solid precipitate was collected by filtration to give 1-(6-methoxy-3-pyridazinyl)-5-(5-methyl-2-pyrazinyl)-1H-pyrazole-3-carboxylic acid (5.50 g, 89%) as a solid.

¹H-NMR (400 MHz, DMSO-d₆)δ: 2.51 (3H, s), 4.05 (3H, s), 7.48-7.50 (2H, m), 8.04 (1H, d, J = 9.3 Hz), 8.37 (1H, m), 8.91 (1H, d, J = 1.5 Hz), 13.28 (1H, br s).
EI-MSm/z: 312 (M⁺).

### 4) Methyl 1-(6-methoxy-3-pyridazinyl)-5-(5-methyl-2-pyrazinyl)-1H-pyrazole-3-carboxylate

Under argon atmosphere at 0°C, (trimethylsilyl)dizaomethane (2.0M solution in hexane, 6.72 ml) was added dropwise to a suspension of 1-(6-methoxy-3-pyridazinyl)-5-(5-methyl-2-pyrazinyl)-1H-pyrazole-3-carboxylic acid (3.50 g) in a mixture of dichloromethane (70 ml) and methanol (35ml), and the mixture was stirred for 1.5 hours. Another portion of the (trimethylsilyl) dizaomethane (2.0M solution in hexane, 6.72 ml) was added to the reaction liquid, and the mixture was stirred for 2 hours. The solvent was evaporated under reduced pressure, and the residue was purified by column chromatography on silica gel (ethyl acetate-chloroform) to give methyl 1-(6-methoxy-3-pyridazinyl)-5-(5-methyl-2-pyrazinyl)-1H-pyrazole-3-carboxylate (3.41 g, 93%) as a solid.

¹H-NMR (400 MHz, CDCl₃)δ: 2.59 (3H, s), 4.00 (3H, s), 4.11 (3H, s), 7.18 (1H, d, J = 9.3 Hz), 7.27-7.29 (1H, m), 7.98-8.01 (1H, m), 8.29 (1H, m), 8.71 (1H, d, J = 1.5 Hz).
EI-MSm/z: 326 (M⁺).

### 5) Methyl 5-(5-carboxy-2-pyrazinyl)-1-(6-methoxy-3-pyridazinyl)-1H-pyrazole-3-carboxylate

The procedure of Referential Example 57 (1) was repeated by using the methyl 1-(6-methoxy-3-pyridazinyl)-5-(5-methyl-2-pyrazinyl)-1H-pyrazole-3-carboxylate (3.40 g) and selenium dioxide (4.62 g) to give methyl 5-(5-carboxy-2-pyrazinyl)-1-(6-methoxy-3-pyridazinyl)-1H-pyrazole -3-carboxylate (2.57 g, 69%) as a solid.

¹H-NMR (400 MHz, DMSO-d₆)δ: 3.92 (3H, s), 4.05 (3H, s), 7.52 (1H, d, J = 9.3 Hz), 7.77 (1H, s), 8.10 (1H, d, J = 9.3 Hz), 8.98 (1H, d, J = 1.5 Hz), 9.21 (1H, d, J = 1.5 Hz).
EI-MSm/z: 356 (M⁺).

### 6) Methyl 5-(5-tert-butoxycarbonylamino-2-pyrazinyl)-1-(6-methoxy-3-pyridazinyl)-1H-pyrazole-3-carboxylate

The procedure of Referential Example 57 (2) was repeated by using 5-(5-carboxy-2-pyrazinyl)-1-(6-methoxy-3-pyridazinyl)-1H-pyrazole -3-carboxylic acid (2.56 g), diphenylphosphorylazide (1.70 ml), and tert-butanol (1.51 ml) to give methyl 5-[5-(tert-butoxycarbonylamino)-2-pyrazinyl]-1-(6-methoxy-3-pyrid azinyl)-1H-pyrazole-3-carboxylate (0.886 g, 29%) as a solid.

¹H-NMR (400 MHz, CDCl₃)δ: 1.54 (9H, s), 4.00 (3H, s), 4.12 (3H, s), 7.17 (1H, d, J = 9.3 Hz), 7.24-7.27 (1H, m), 7.57 (1H, s), 7.95 (1H, d, J = 9.3 Hz), 8.48 (1H, d, J = 1.5 Hz), 9.11 (1H, d, J = 1.5 Hz). EI-MSm/z: 427 (M⁺).

### 7) The title compound

To a suspension of methyl 5-[5-(tert-butoxycarbonylamino)-2-pyrazinyl]-1-(6-methoxy-3-pyrid azinyl)-1H-pyrazole-3-carboxylate (0.880 g) in a mixture of tetrahydrofuran (18 ml) and methanol (18 ml) was added 1N aqueous solution of sodium hydroxide (5.15 ml) at room temperature for 4 hours. The reaction liquid was neutralized by adding 1N aqueous hydrochloric acid (5.15 ml), and water (250 ml) was added to the reaction liquid. The solid precipitate was collected by filtration to give the title compound (0.732 g, 86%) as a solid.

¹H-NMR (400 MHz, DMSO-d₆)δ: 1.48 (9H, s), 4.05 (3H, m), 7.44 (1H, m), 7.49 (1H, dd, J = 9.3, 1.0 Hz), 8.02 (1H, dd, J = 9.3, 1.0 Hz), 8.71 (1H, m), 8.82 (1H, m), 10.39 (1H, s).
EI-MSm/z: 413 (M⁺).

[Example 1] N-tert-Butyl-1-(6-methoxy-3-pyridazinyl)-5-(2-pyridyl)-1H-pyrazole-3-carboxamide

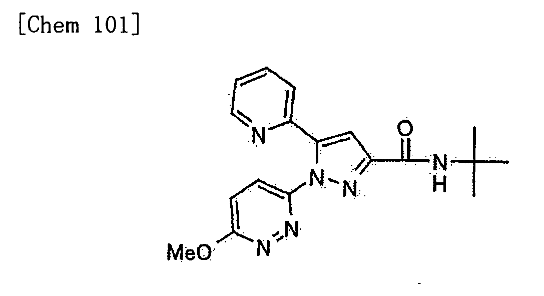

To a solution of the 1-(6-methoxy-3-pyridazinyl)-5-(2-pyridyl)-1H-pyrazole-3-carboxyli c acid (0.250 g) of Referential Example 9, 1-ethyl-3-(3-dimethylaminopropyl)carbodiimide hydrochloride (0.177 g), and 1-hydroxybenzotriazole (0.125 g) in N,N-dimethylformamide (5 ml) was added tert-butylamine (0.0967 ml) and triethylamine (0.129 ml), and the mixture was stirred at room temperature for 43 hours. Water and ethyl acetate were added to the reaction liquid and the phases were separated, and the organic layer was dried over anhydrous sodium sulfate. After filtration, the solvent was evaporated under reduced pressure, and the residue was purified by thin layer chromatography on silica gel (ethyl acetate-chloroform) to give the title compound (0.185 g, 63%) as a solid.

¹H-NMR (400 MHz, CDCl₃)δ: 1.48 (9H, s), 4.12 (3H, s), 6.83 (1H, s), 7.12 (1H, d, J = 9.0 Hz), 7.18-7.21 (1H, m), 7.20 (1H, s), 7.59-7.61 (1H, m), 7.72-7.76 (2H, m), 8.35-8.37 (1H, m).
EI-MSm/z: 352 (M⁺).

| Elementary analysis: for C₁₈H₂₀N₆O₂ | | | |
|---|---|---|---|
| Calculated: | C, 61.35; | H, 5.72; | N, 23.85. |
| Found: | C, 61.19; | H, 5.48; | N, 23.70. |

### [Example 2] N-tert-Butyl-5-(4-carbamoyl-2-pyridyl)-1-(6-methoxy-3-pyridyl)-1H-pyrazole-3-carboxamide

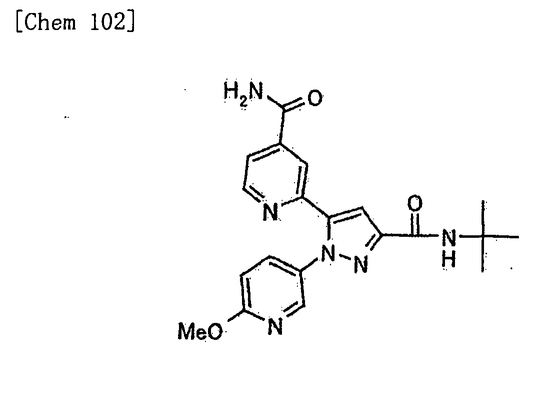

A suspension of the ethyl 5-(4-carbamoyl-2-pyridyl)-1-(6-methoxy-3-pyridyl)-1H-pyrazole-3-c arboxylate (286 mg) of Referential Example 20 and lithium hydroxide monohydrate (99.2 mg) in tetrahydrofuran (20 ml), ethanol (20 ml), and water (20 ml) was stirred at room temperature for 18 hours. The reaction solvent was evaporated under reduced pressure, and 1N aqueous hydrochloric acid (1.56 ml) was added to the residue, and the reaction solvent was evaporated under reduced pressure to give the crude lithium 5-(4-carbamoyl-2-pyridyl)-1-(6-methoxy-3-pyridyl)-1H-pyrazole-3-c arboxylate. To a solution of this lithium carboxylate and 1-hydroxybenzotriazole (286 mg) in N,N-dimethylformamide (20ml) were added tert-butylamine (0.278 ml) and 1-ethyl-(3-dimethylaminopropyl)carbodiimide hydrochloride (358 mg) at room temperature, and the mixture was stirred for 40 hours. The reaction solvent was evaporated under reduced pressure, and ethyl acetate and water were added to the residue and the phases were separated. The organic layer was dried over anhydrous sodium sulfate. After filtration, the solvent was evaporated under reduced pressure, and the residue was purified by column chromatography on silica gel (ethyl acetate-hexane) to give the title compound (255 mg, 76%) as a solid.

¹H-NMR (400 MHz, DMSO-d₆)δ: 1.41 (9H, s), 3.89 (3H, s), 6.88 (1H, dd, J = 8.8, 0.5 Hz), 7.37 (1H, s), 7.39 (1H, br s), 7.70 (1H, dd, J = 5.0, 1.6 Hz), 7.74 (1H, dd, J = 8.8, 2.7 Hz), 7.79 (1H, br s), 8.15-8.15 (1H, m), 8.19 (1H, dd, J = 2.7, 0.5 Hz), 8.30 (1H, br s), 8.54 (1H, dd, J = 5.1, 0.7 Hz).
ESI-MSm/z: 395 (M+H)⁺.

[Example 3] N-tert-Butyl-5-(4-carbamoyl-2-pyridyl)-1-(6-methoxy-3-pyridazinyl)-1H-pyrazole-3-carboxamide

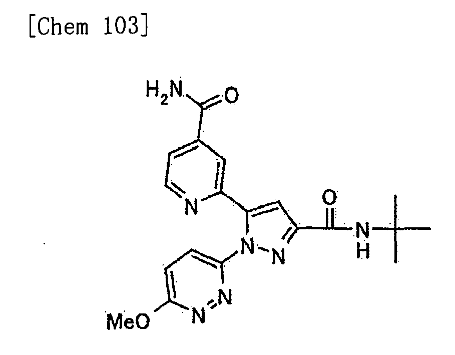

Lithium hydroxide monohydrate (6 mg) was added to a solution of the methyl 5-(4-carbamoyl-2-pyridyl)-1-(6-methoxy-3-pyridazinyl)-1H-pyrazole -3-carboxylate (29 mg) of Referential Example 30(4) in a mixture of tetrahydrofuran (4 ml) and water (1 ml) at room temperature, and the mixture was stirred for 1.5 hours. 1N aqueous hydrochloric acid (0.07 ml) was added to the reaction liquid, and the reaction solvent was evaporated under reduced pressure. The procedure of Example 1 was repeated by using the residue and tert-butylamine (51 µl) to give the title compound (10 mg, 30%).

¹H-NMR (400MHz, CDCl₃)δ: 1.49 (9H, s), 4.11 (3H, s), 6.82 (1H, s), 7.16 (1H, d, J = 9.38 Hz), 7.57 (1H, dd, J = 5.01, 1.71 Hz), 7.82 (1H, d, J = 9.38 Hz), 7.93 (1H, d, J = 1.71 Hz), 8.53 (1H, d, J = 5.01 Hz). FAB-MSm/z: 396 (M+H)⁺.

[Example 4] N-tert-Butyl-5-(4-hydroxymethyl-2-pyridyl)-1-(6-methoxy-3-pyridyl)-1H-pyrazole-3-carboxamide

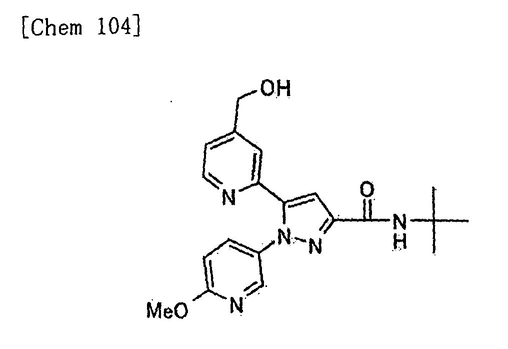

The procedure of Example 1 was repeated by using the 5-(4-hydroxymethyl-2-pyridyl)-1-(6-methoxy-3-pyridyl)-1H-pyrazole -3-carboxylic acid (232 mg) of Referential Example 29 and tert-butylamine (0.3 ml) to give the title compound (13 mg, 5%) as a solid.

¹H-NMR (400 MHz, CDCl₃)δ: 1.48 (9H, s), 3.95 (3H, s), 4.74 (2H, s), 6.75 (1H, d, J = 8.79 Hz), 6.86 (1H, br), 7.14 (1H, s), 7.20 (1H, d, J = 5.01 Hz), 7.48 (1H, s), 7.59 (1H, dd, J = 8.79, 2.56 Hz), 8.09 (1H, d, J = 2.56 Hz), 8.40 (1H, d, J = 5.01 Hz).
FAB-MSm/z: 382 (M+H)⁺.

[Example 5] N-tert-Butyl-1-(5-methoxy-2-pyridyl)-5-(2-pyridyl)-1H-pyrazole-3-carboxamide

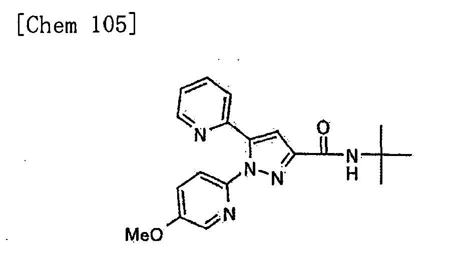

The procedure of Example 1 was repeated by using the 1-(5-methoxy-2-pyridyl)-5-(2-pyridyl)-1H-pyrazole-3-carboxylic acid (0.250 g) of Referential Example 8 and tert-butylamine (0.194 ml) to give the title compound (0.154 g, 51%) as a solid.

¹H-NMR (400 MHz, CDCl₃)δ: 1.48 (9H, s), 3.89 (3H, s), 6.87 (1H, br s), 7.16-7.20 (2H, m), 7.31 (1H, dd, J = 8.8, 3.2 Hz), 7.43-7.47 (2H, m), 7.67-7.72 (1H, m), 8.03 (1H, d, J = 2.9 Hz), 8.41-8.42 (1H, m).
EI-MSm/z: 351 (M⁺).

[Example 6] N-tert-Butyl-5-(5-hydroxy-2-pyridyl)-1-(6-methoxy-3-pyridyl)-1H-pyrazole-3-carboxamide

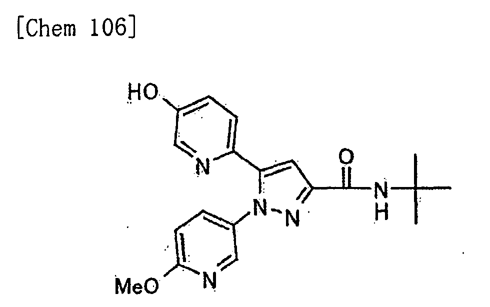

### 1) N-tert-Butyl-5-(5-benzyloxy-2-pyridyl)-1-(6-methoxy-3-pyridyl)-1H-pyrazole-3-carboxamide

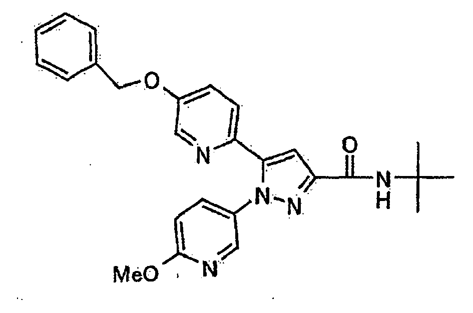

The procedure of Example 1 was repeated by using the 5-(5-benzyloxy-2-pyridyl)-1-(6-methoxy-3-pyridyl)-1H-pyrazole-3-c arboxylic acid (0.203 g) of Referential Example 14 and tert-butylamine (0.105 ml) to give N-tert-butyl-5-(5-benzyloxy-2-pyridyl)-1-(6-methoxy-3-pyridyl)-1H -pyrazole-3-carboxamide (0.195 g, 84%) as an amorphous product. ¹H-NMR (400 MHz, CDCl₃)δ: 1.48 (9H, s), 3.96 (3H, s), 5.10 (2H, s), 6.75 (1H, dd, J = 8.8, 2.7 Hz), 6.82 (1H, s), 7.11-7.12 (1H, m), 7.23-7.41 (7H, m), 7.60 (1H, dd, J=8.8, 2.7 Hz), 8.12 (1H, d, J=2.7 Hz), 8.23-8.24 (1H, m).
EI-MSm/z: 457 (M⁺).

### 2) The title compound

To a solution of N-tert-butyl-5-(5-benzyloxy-2-pyridyl)-1-(6-methoxy-3-pyridyl)-1H -pyrazole-3-carboxamide (0.190 g) in methanol (3.8 ml) was added 10% palladium on carbon (50% wet, 0.19 g), and the mixture was stirred at room temperature for 8 hours under hydrogen atmosphere. After the reaction liquid was filtered, the solvent was evaporated under reduced pressure, and the residue was purified by thin layer chromatography on silica gel (methanol-chloroform) to give the title compound (0.115 g, 75%) as a solid.

¹H-NMR (400 MHz, CDCl₃)δ: 1.49 (9H, s), 3.94 (3H, s), 6.74 (1H, d, J = 8.8 Hz), 6.96 (1H, br s), 7.05 (1H, s), 7.16-7.26 (2H, m), 7.58 (1H, dd, J = 8.8, 2.7 Hz), 8.11 (2H, m), 8.91 (1H, br s).
EI-MSm/z: 367 (M⁺).

| Elementary analysis: for C₁₉H₂₁N₅O₃·0.25H₂O | | | |
|---|---|---|---|
| Calculated: | C, 61.36; | H, 5.83; | N, 18.83. |
| Found: | C, 61.35; | H, 5.65; | N, 18.74. |

[Example 7] N-(2-Hydroxy-1,1-dimethylethyl)-1-(6-methoxy-3-pyridyl)-5-(2-pyridyl)-1H-pyrazole-3-carboxamide

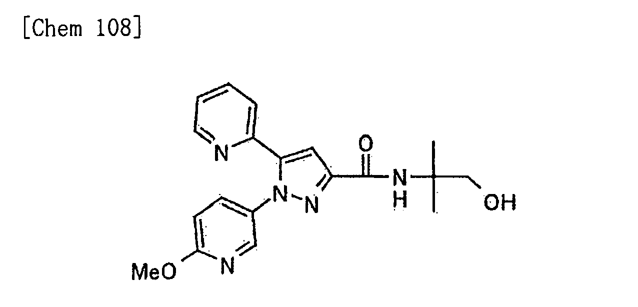

Triethylamine (0.3 ml) was added to a solution of the 1-(6-methoxy-3-pyridyl)-5-(2-pyridyl)-1H-pyrazole-3-carboxylic acid (200 mg) of Referential Example 4, 1-ethyl-3-(3-dimethylaminopropyl)carbodiimide hydrochloride (0.195 g), 1-hydroxybenzotriazole (0.137 g), and 1,1-dimethyl-2-hydroxyethylamine (67 mg) in dichloromethane (10 ml), and the mixture was stirred at room temperature for 24 hours. Water and dichloromethane were added to the reaction liquid and the phases were separated, and the organic layer was dried over anhydrous magnesium sulfate. After filtration, the solvent was evaporated under reduced pressure, and the residue was purified by thin layer chromatography on silica gel to give the title compound (180 mg, 66%) as a solid.

¹H-NMR (400 MHz, CDCl₃)δ: 1.26 (6H, s), 3.72 (2H, d, J = 6.3 Hz), 3.96 (3H, s), 4.90 (1H, t, J = 6.1 Hz), 6.77 (1H, d, J = 8.8 Hz), 7.03 (1H, br s), 7.21-7.26 (2H, m), 7.43 (1H, dd, J = 7.8, 0.7 Hz), 7.60 (1H, dd, J = 8.8, 2. 7 Hz), 7.71-7.74 (1H, m), 8.12 (1H, d, J=2.7Hz), 8.47-8.50 (1H, m).
EI-MSm/z: 367 (M⁺).

| Elementary analysis: for C₁₉H₂₁N₅O₃·0.25H₂O | | | |
|---|---|---|---|
| Calculated: | C, 61.36; | H, 5.83; | N, 18.83. |
| Found: | C, 61.52; | H, 5.56; | N, 18.95. |

Example 8] N-tert-Butyl-1-(6-methyl-3-pyridyl)-5-(2-pyridyl)-1H-pyrazole-3-carboxamide

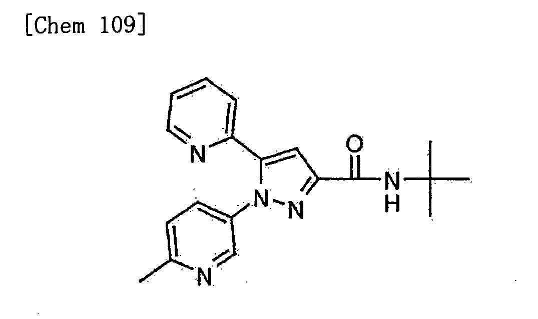

Lithium hydroxide monohydrate (40.7 mg) was added to a solution of the ethyl 1-(6-methyl-3-pyridyl)-5-(2-pyridyl)-1H-pyrazole-3-carboxylate (0.236 g) of Referential Example 12 in a mixture of tetrahydrofuran (2 ml), ethanol (0.5 ml), and water (1 ml) at room temperature, and the mixture was stirred for 1 hour. 1N aqueous hydrochloric acid (0.070 ml) was added to the reaction liquid, and the reaction solvent was evaporated under reduced pressure to give lithium 1-(6-methyl-3-pyridyl)-5-(2-pyridyl)-1H-pyrazole-3-carboxylate, which was used in the subsequent reaction without further purification.
The thus obtained lithium carboxylate was dissolved in N, N-dimethylformamide (4.0ml), and 1-hydroxybenzotriazole (0.166g), 1-ethyl-3-(3-dimethylaminopropyl)carbodiimide hydrochloride (0.229 g), and tert-butylamine (0.241 ml) were added to this solution at room temperature, and the mixture was stirred for 3 days. Water and a mixed solvent of chloroform and methanol (15:1) were added to the reaction liquid and the phases were separated, and the organic layer was dried over anhydrous sodium sulfate. After filtration, the solvent was evaporated under reduced pressure, and the residue was purified by column chromatography on silica gel (chloroform-methanol) to give the title compound (0.137 g, 54%) as an amorphous product.

¹H-NMR (400 MHz, CDCl₃)δ: 1.49 (9H, s), 2.60 (3H, s), 6.84 (1H, br), 7.18-7.30 (3H, m), 7.47 (1H, d like, J = 7.0 Hz), 7.63 (1H, dd, J = 8.1, 2.7 Hz), 7.72 (1H, t like, J = 8.1 Hz), 8.40 (1H, d, J = 2.7 Hz), 8.45-8.55 (1H, m).
ESI-MSm/z: 336 (M+H)⁺.

[Example 9] N-tert-Butyl-5-(5-cyano-2-pyridyl)-1-(6-methoxy-3-pyridyl)-1H-pyrazole-3-carboxamide

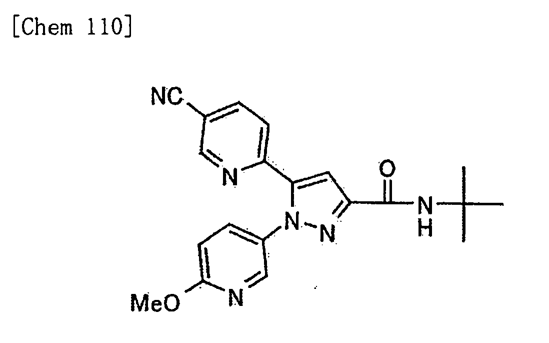

### 1) 6-[5-tert-Butylcarbamoyl-2-(6-methoxy-3-pyridyl)-2H-pyrazol-3-yl]-3-pyridyl trifluoromethanesulfonate

Under argon atmosphere, trifluoromethanesulfonic anhydride (1.24 ml) was added dropwise to a solution of the N-tert-butyl-5-(5-hydroxy-2-pyridyl)-1-(6-methoxy-2-pyridyl)-1H-p yrazole-3-carboxamide (2.47 g) of Example 6 and pyridine (1.79 ml) in dichloromethane (49 ml) at room temperature, and the mixture was stirred for 1 hour. Water and chloroform were added to the reaction liquid and the phases were separated, and the organic layer was dried over anhydrous sodium sulfate. After filtration, the solvent was evaporated under reduced pressure, and the residue was purified by column chromatography on silica gel (ethyl acetate-hexane) to give trifluoromethanesulfonate derivative (2.83 g, 84%) as a solid.

¹H-NMR (400 MHz, CDCl₃)δ: 1.47 (9H, s), 3.97 (3H, s), 6.79 (1H, dd, J = 8.8, 0.7 Hz), 6.81 (1H, br s), 7.25-7.27 (1H, m), 7.56-7.66 (3H, m), 8.11-8.12 (1H, m), 8.41 (1H, d, J = 2.4 Hz).
EI-MSm/z: 499 (M⁺).

### 2) The title compound

Under argon atmosphere, and at room temperature, a suspension of tri-n-butyltin cyanide (5.62 g) and tetrakis (triphenylphosphine)palladium(0) (7.70 g) in 1,2-dichloroethane (61 ml) was heated under reflux for 2 hours. After cooling with air, a solution of 6-[5-tert-butylcarbamoyl-2-(6-methoxy-3-pyridyl)-2H-pyrazol-3-yl] -3-pyridyl trifluoromethanesulfonate (2.22 g) in 1,2-dichloroethane (50 ml) was added dropwise to the reaction liquid, and the mixture was heated under reflux for another 22 hours. After cooling with air, saturated aqueous sodium bicarbonate was added to the reaction liquid, and the mixture was stirred. The reaction liquid was filtered through celite, and the chloroform was added to the filtrate and the phases were separated. The organic layer was dried over anhydrous sodium sulfate. After filtration, the solvent was evaporated under reduced pressure, and the residue was purified by column chromatography on silica gel (ethyl acetate-hexane) to give the title compound (1.50 g, 90%) as a solid.

¹H-NMR (400 MHz, CDCl₃)δ: 1.47 (9H, s), 3.97 (3H, s), 6.79-6.81 (2H, m), 7.33 (1H, s), 7.57-7.60 (2H, m), 7.95-7.97 (1H, m), 8.09 (1H, d, J = 2.9 Hz), 8.68 (1H, d, J = 2.2 Hz).
EI-MSm/z: 376 (M⁺).

| Elementary analysis: for C₂₀H₂₀N₆O₃ | | | |
|---|---|---|---|
| Calculated: | C, 63.82; | H, 5.36; | N, 22.33. |
| Found: | C, 63.76; | H, 5.32; | N, 22.36. |

[Example 10] N-tert-Butyl-5-(5-carbamoyl-2-pyridyl)-1-(6-methoxy-3-pyridyl)-1H-pyrazole-3-carboxamide

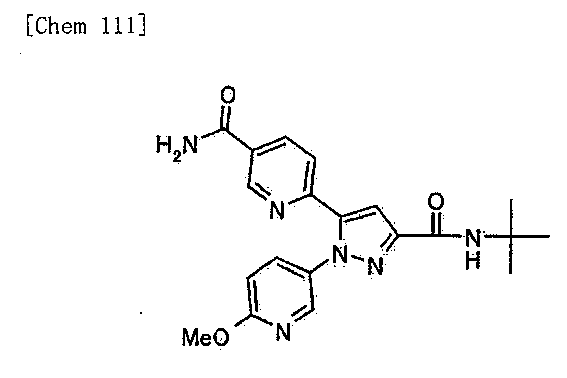

### 1) 6-[5-(N-tert-Butyl)carbamoyl-2-(6-methoxy-3-pyridyl)-2H-pyrazol-3-yl]nicotinic acid

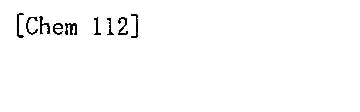

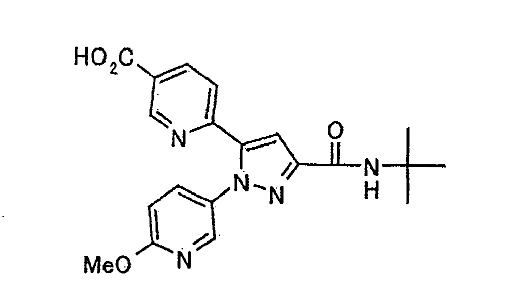

To a solution of the N-tert-butyl-5-(5-cyano-2-pyridyl)-1-(6-methoxy-3-pyridyl)-1H-pyr azole-3-carboxamide (0.70 g) of Example 9 in a mixture of methanol (14 ml) and tetrahydrofuran (14 ml) was added 1N sodium hydroxide (9.30 ml) at room temperature, and the mixture was heated under reflux for 7 hours. After cooling with air, water and chloroform were added to the reaction liquid and the aqueous layer was separated. The aqueous layer was acidified by adding 1N aqueous hydrochloric acid, and chloroform was added and the phases were separated. The organic layer was dried over anhydrous sodium sulfate. After filtration, the solvent was evaporated under reduced pressure to give 6-[5-(N-tert-butyl)carbamoyl-2-(6-methoxy-3-pyridyl)-2H-pyrazole-3-yl]nicotinic acid (0.572 g, 78%) as a solid.

¹H-NMR (400 MHz, CDCl₃)δ: 1.48 (9H, s), 3.97 (3H, s), 6.80 (1H, d, J = 8.8 Hz), 6.87 (1H, s), 7.36 (1H, s), 7.56 (1H, d, J = 8.3 Hz), 7.64 (1H, dd, J = 8.8, 2.7 Hz), 8.11 (1H, d, J = 2.7 Hz), 8.32-8.35 (1H, m), 9.08 (1H, d, J = 1.5 Hz).
EI-MSm/z: 395 (M⁺).

### 2) The title compound

The procedure of Example 1 was repeated by using 6-[5-(N-tert-butyl)carbamoyl-2-(6-methoxy-3-pyridyl)-2H-pyrazole-3-yl]nicotinic acid (0.250 g) and ammonium chloride (0.169 g) to give the title compound (0.116 g, 47%) as a solid.

¹H-NMR (400 MHz, CDCl₃)δ: 1.48 (9H, s), 3.96 (3H, s), 6.78 (1H, d, J = 8.8 Hz), 6.85 (1H, br s), 7.30 (1H, s), 7.52-7.54 (1H, m), 7.59 (1H, dd, J = 8.8, 2.7 Hz), 8.10 (1H, d, J = 2.7 Hz), 8.19-8.21 (1H, m), 8.90 (1H, d, J = 1.7 Hz).
EI-MSm/z: 394 (M⁺).

| Elementary analysis: for C₂₀H₂₂N₆O₃ | | | |
|---|---|---|---|
| Calculated: | C, 60.90; | H, 5.62; | N, 21.31. |
| Found: | C, 60.74; | H, 5.62; | N, 21.18. |

[Example 11] N-tert-Butyl-5-(5-hydroxymethyl-2-pyridyl)-1-(6-methoxy-3-pyridyl)-1H-pyrazole-3-carboxamide

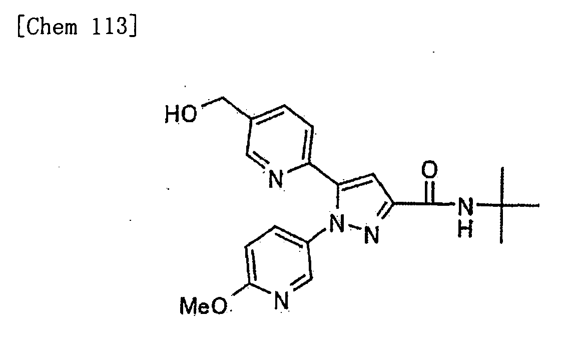

Under argon atmosphere, borane-methyl sulfide complex (0.141 ml) was added dropwise to a solution of the 6-[5-tert-butylcarbamoyl-2-(6-methoxy-3-pyridyl)-2H-pyrazol-3-yl] nicotinic acid (0.245 g) of Example 10 (1) in tetrahydrofuran (4. 9 ml) at room temperature, and the mixture was stirred for 2 hours. After adding another portion of the borane-methyl sulfide complex (0.282 ml), the mixture was stirred for another 4 hours. Saturated aqueous sodiumbicarbonate and a mixed solvent of chloroform and methanol (10:1) were added to the reaction liquid and the phases were separated, and the organic layer was dried over anhydrous sodium sulfate. After filtration, the solvent was evaporated under reduced pressure, and the residue was purified by column chromatography on silica gel (chloroform-methanol) to give the title compound (94.0 mg, 39%) as a solid.

¹H-NMR (400 MHz, CDCl₃)δ: 1.47 (9H, s), 3.94 (3H, s), 4.72 (2H, s), 6.75 (1H, d, J = 8.8 Hz), 6.83 (1H, br s), 7.14 (1H, s), 7.40 (1H, d, J = 8.1 Hz), 7.59 (1H, dd, J = 8.8, 2.7 Hz), 7.73 (1H, dd, J = 8.1, 2.2 Hz), 8.09 (1H, d, J = 2.7 Hz), 8.44 (1H, d, J = 2.2 Hz).
FAB-MSm/z: 382 (M+H)⁺.

| Elementary analysis: for C₂₀H₂₀N₆O₂·0.5H₂O | | | |
|---|---|---|---|
| Calculated: | C, 61.53; | H, 6.20; | N, 17.94. |
| Found: | C, 61.75; | H, 6.30; | N, 17.79. |

[Example 12] N-tert-Butyl-1-(6-methoxy-3-pyridyl)-5-(3-pyridazinyl)-1H-pyrazole-3-carboxamide

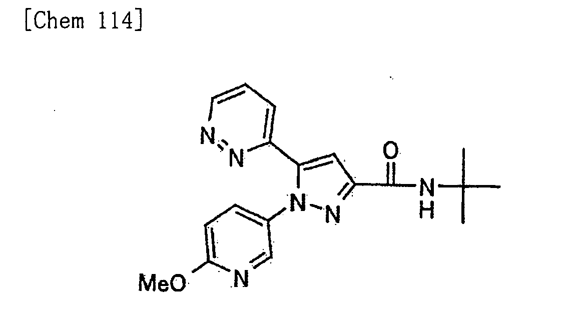

The procedure of Example 1 was repeated by using the lithium 1-(6-methoxy-3-pyridyl)-5-(3-pyridazinyl)-1H-pyrazole-3-carboxyla te (162 mg) of Referential Example 13 and tert-butylamine (112 µl) to give the title compound (81 mg, 40%) as a solid.

¹H-NMR (400 MHz, CDCl₃)δ: 1.50 (9H, s), 3.95 (3H, s), 6.80 (1H, dd, J = 8.8, 0.5 Hz), 6.85 (1H, br s), 7.33 (1H, s), 7.51 (1H, dd, J = 8.5, 4.9 Hz), 7.59 (1H, dd, J = 8.5, 1.7 Hz), 7.68 (1H, dd, J = 8.8, 2.7 Hz), 8.12 (1H, dd, J = 2.7, 0.5 Hz), 9.12 (1H, dd, J = 4.9, 1.7 Hz). ESI-MSm/z: 353 (M+H)⁺.

[Example 13] N-tert-Butyl-1-(6-methoxy-3-pyridyl)-5-(2-pyrazinyl)-1H-pyrazole-3-carboxamide

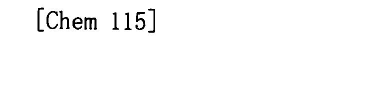

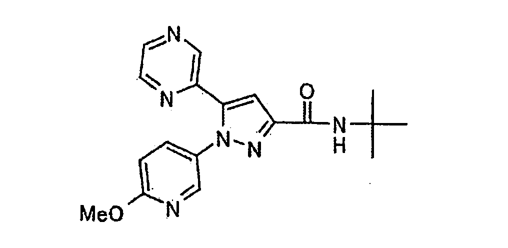

The procedure of Example 1 was repeated by using the 1-(6-methoxy-3-pyridyl)-5-(2-pyrazinyl)-1H-pyrazole-3-carboxylic acid (0.232 g) of Referential Example 22 and tert-butylamine (0.148 ml) to give the title compound (141 mg, 49%) as an amorphous product.

¹H-NMR (400 MHz, CDCl₃)δ: 1.49 (9H, s), 3.97 (3H, s), 6.80 (1H, d, J = 8.8 Hz), 6.82 (1H, br s), 7.33 (1H, s), 7.61 (1H, dd, J = 8.8, 2.7 Hz), 8.13 (1H, d, J = 2.4 Hz), 8.44 (1H, dd, J = 2. 4, 1.7 Hz), 8.49 (1H, d, J = 2.4 Hz), 8.74 (1H, d, J = 1.5 Hz).
ESI-MSm/z: 353 (M+H)⁺.

[Example 14] N-tert-Butyl-5-(4-dimethylaminophenyl)-1-(6-methoxy-3-pyridazinyl)-1H-pyrazole-3-carboxamide

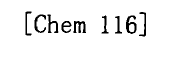

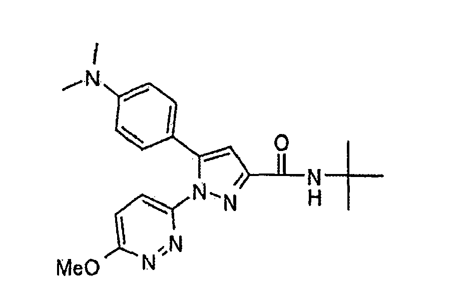

The procedure of Example 7 was repeated by using the 5-(4-dimethylaminophenyl)-1-(6-methoxy-3-pyridazinyl)-1H-pyrazole -3-carboxylic acid (203 mg) of Referential Example 10 and tert-butylamine (126 µl) to give the title compound (202 mg, 84%) as a solid.

¹H-NMR (400 MHz, CDCl₃)δ: 1.47 (9H, s), 2.96 (6H, s), 4.17 (3H, s), 6.62 (2H, d, J = 8.8 Hz), 6.89 (1H, br s), 6.94 (1H, s), 6.99 (1H, d, J = 9.3 Hz), 7.12 (2H, d, J = 8.8 Hz), 7.35 (1H, d, J = 9.3 Hz).
ESI-MSm/z: 395 (M+H)⁺.

| Elementary analysis: for C₂₁H₂₆N₆O₂·0.25H₂O | | | |
|---|---|---|---|
| Calculated: | C, 63.22; | H, 6.69; | N, 21.06. |
| Found: | C, 63.33; | H, 6.42; | N, 20.94. |

[Example 15] N-tert-Butyl-1-(5-methoxy-2-pyrazinyl)-5-(2-pyridyl)-1H-pyrazole-3-carboxamide

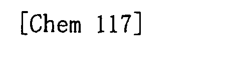

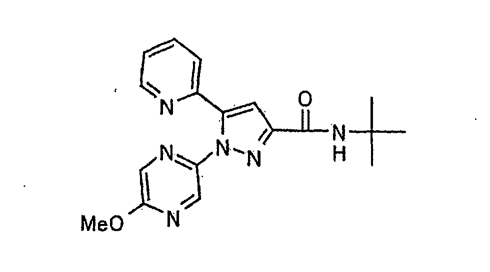

The procedure of Example 1 was repeated by using the 1-(5-methoxy-2-pyrazinyl)-5-(2-pyridyl)-1H-pyrazole-3-carboxylic acid (0.227 g) of Referential Example 11 and tert-butylamine (0.160 ml) to give the title compound (0.197 g, 73%) as a solid.

¹H-NMR (400MHz, CDCl₃)δ: 1.49 (9H, s), 4.03 (3H, s), 6.87 (1H, s), 7.18-7.21 (1H, m), 7.23 (1H, s), 7.55-7.57 (1H, m), 7.71-7.76 (1H, m), 7.98 (1H, d, J = 1.2 Hz), 8.33 (1H, d, J = 1.2 Hz), 8.37-8.38 (1H, m).
EI-MSm/z: 352 (M⁺).

| Elementary analysis: for C₁₈H₂₀N₆O₂ | | | |
|---|---|---|---|
| Calculated: | C, 61.35; | H, 5.72; | N, 23.85. |
| Found: | C, 61.27; | H, 5.68; | N, 23.98. |

[Example 16] N-tert-Butyl-5-(4-dimethylaminophenyl)-1-(6-methoxy-3-pyridyl)-1H-pyrazole-3-carboxamide

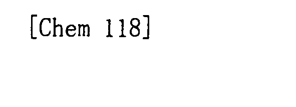

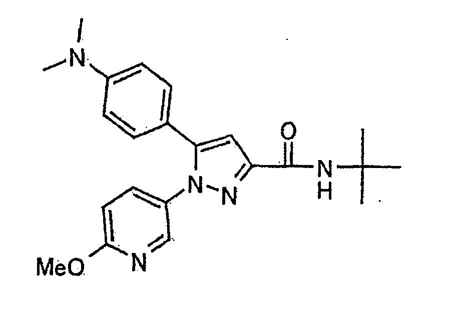

The procedure of Example 7 was repeated by using the 5-(4-dimethylaminophenyl)-1-(6-methoxy-3-pyridyl)-1H-pyrazole-3-c arboxylic acid (253 mg) of Referential Example 17 and tert-butylamine (126 µl) to give the title compound (226 mg, 76%) as a solid.

¹H-NMR (400 MHz, CDCl₃) δ: 1.48 (9H, s), 2.96 (6H, s), 3.95 (3H, s), 6.62 (2H, d, J= 9.0 Hz), 6.73 (1H, d, J = 8.8 Hz), 6.83 (1H, br s), 6.90 (1H, s), 7.06 (2H, d, J = 9.0 Hz), 7.51 (1H, dd, J = 8. 8, 2.7 Hz), 8.19 (1H, d, J = 2.7 Hz).
ESI-MSm/z: 394 (M+H)⁺.

| Elementary analysis: for C₂₂H₂₇N₅O₂ | | | |
|---|---|---|---|
| Calculated: | C, 67.15; | H, 6.92; | N, 17.80. |
| Found: | C, 66.96; | H, 6.90; | N, 17.87. |

[Example 17] N-tert-Butyl-5-(3-dimethylaminophenyl)-1-(6-methoxy-3-pyridyl)-1H-pyrazole-3-carboxamide

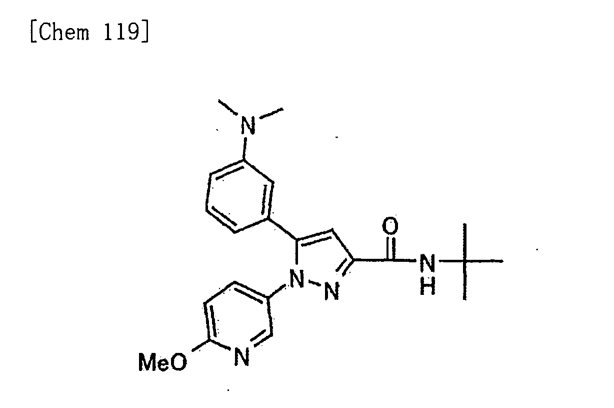

The procedure of Example 1 was repeated by using the 5-(3-dimethylaminophenyl)-1-(6-methoxy-3-pyridyl)-1H-pyrazole-3-c arboxylic acid (264 mg) of Referential Example 15 and tert-butylamine (0.139 ml) to give the title compound (194 mg, 62%) as an amorphous product.
¹H-NMR (400MHz, CDCl₃)δ: 1.49 (9H, s), 2.87 (6H, s), 3.94 (3H, s), 6.46-6.49 (1H, m), 6.56-6.57 (1H, m), 6.68 (1H, dd, J = 8.3, 2.4 Hz), 6.73 (1H, d, J = 8.8 Hz), 6.84 (1H, br s), 7.00 (1H, s), 7.15 (1H, dd, J = 8.3, 7.6 Hz), 7.51 (1H, dd, J = 8.8, 2.9 Hz), 8.17 (1H, d, J = 2.2 Hz).
ESI-MSm/z: 394 (M+H)⁺.

[Example 18] N-tert-Butyl-1-(6-methoxy-3-pyridazinyl)-5-(4-methyl-2-pyridyl)-1H-pyrazole-3-carboxamide

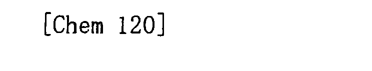

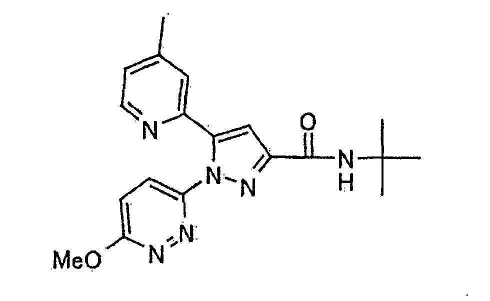

The procedure of Example 7 was repeated by using the 1-(6-methoxy-3-pyridazinyl)-5-(4-methyl-2-pyridyl)-1H-pyrazole-3-carboxylic acid (560 mg) of Referential Example 17 and tert-butylamine (167 µl) to give the title compound (340 mg, 52%) as a solid.

¹H-NMR (400 MHz, CDCl₃)δ: 1.49 (9H, s), 2.39 (3H, s), 4.12 (3H, s), 6.85 (1H, br), 7.02 (1H, d, J = 5.00 Hz), 7.12 (1H, d, J = 9.28 Hz), 7.17 (1H, s), 7.44 (1H, s), 7.74 (1H, d, J = 9.28 Hz), 8.20 (1H, d, J = 5.00 Hz).
FAB-MSm/z: 367 (M+H)⁺.

[Example 19] N-tert-Butyl-1-(6-methoxy-3-pyridyl)-5-(2-pyridyl)-1H-pyrazole-3-carboxamide

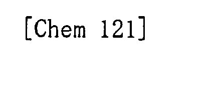

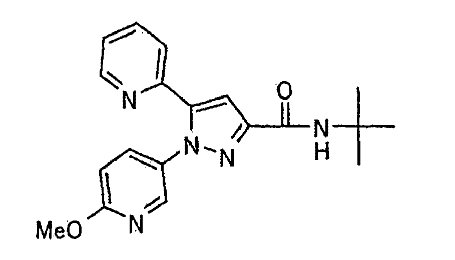

The procedure of Example 7 was repeated by using the 1-(6-methoxy-3-pyridyl)-5-(2-pyridyl)-1H-pyrazole-3-carboxylic acid (880 mg) of Referential Example 4 and tert-butylamine (217 mg) to give the title compound (850 mg, 81%) as a solid.

¹H-NMR (400 MHz, CDCl₃)δ: 1.49 (9H, s), 3.96 (3H, s), 6.76 (1H, d, J = 8.8 Hz), 6.84 (1H, br s), 7.18-7.24 (2H, m), 7.44 (1H, d, J = 7.8 Hz), 7.60 (1H, dd, J = 6.8, 1.9 Hz), 7.70-7.73 (1H, m), 8.12 (1H, d, J = 2.4 Hz), 8.47 (1H, m).
EI-MSm/z: 351 (M⁺).

| Elementary analysis: for C₁₉H₂₁N₅O₂ | | | |
|---|---|---|---|
| Calculated: | C, 64.94; | H, 6.02; | N, 19.93. |
| Found: | C, 64.97; | H, 6.06; | N, 19.86. |

[Example 20] N-tert-Butyl-1-(6-cyano-3-pyridyl)-5-(2-pyridyl)-1H-pyrazole-3-carboxamide

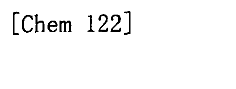

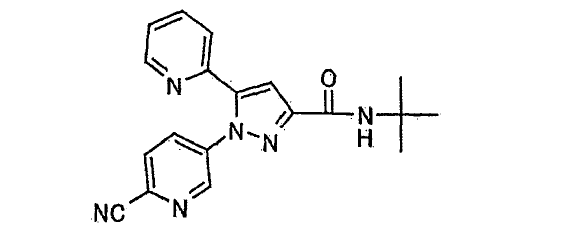

### 1) N-tert-Butyl-1-(6-hydroxy-3-pyridyl)-5-(2-pyridyl)-1H-pyrazole-3-carboxamide

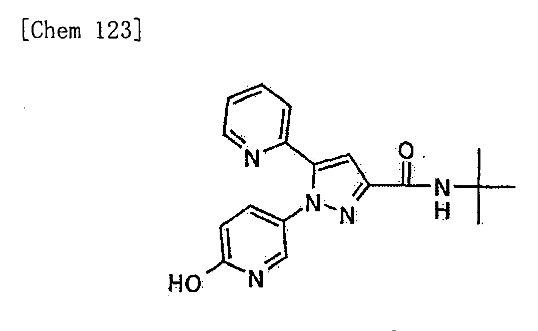

A solution of the N-tert-butyl-1-(6-methoxy-3-pyridyl)-5-(2-pyridyl)-1H-pyrazole-3-carboxamide (4.71 g) of Example 19 in a 1N solution of hydrochloric acid in ethanol (94 ml) was heated under reflux for 1.5 hours, and then the mixture was stirred at room temperature for 14 hours, and heated under reflux for another 11 hours, and stirred at room temperature for 13 hours. The reaction solvent was evaporated under reduced pressure, and saturated aqueous sodium carbonate and chloroform were' added to the residue and the phases were separated. The organic layer was dried over anhydrous sodium sulfate. After filtration, the solvent was evaporated under reduced pressure, and the residue was purified by column chromatography on silica gel (methanol-chloroform) to give N-tert-butyl-1-(6-hydroxy-3-pyridyl)-5-(2-pyridyl)-1H-pyrazole-3-carboxamide (4.17 g, 92%) as a solid.

¹H-NMR (400 MHz, CDCl₃)δ: 1.49 (9H, s), 6.54 (1H, d, J = 9.5 Hz), 6.80 (1H, br s), 7.20 (1H, s), 7.23-7.26 (1H, m), 7.46-7.59 (3H, m), 7.73-7.78 (1H, m), 8.50-8.52 (1H, m), 13.29 (1H, br s).
EI-MSm/z: 337 (M⁺).

### 2) 5-[3-(N-tert-Butyl)carbamoyl-5-(2-pyridyl)-1H-pyrazol-1-yl]-2-pyridyl trifluoromethanesulfonate

The procedure of Example 9(1) was repeated by using N-tert-butyl-1-(6-hydroxy-3-pyridyl)-5-(2-pyridyl)-1H-pyrazole-3-carboxamide (3.00 g) and trifluoromethanesulfon anhydride (1.80 ml) to give 5-[3-(N-tert-butyl) carbamoyl-5-(2-pyridyl)-1H-pyrazole-1-yl]-2-pyridyl trifluoromethanesulfonate (3.29 g, 79%) as a solid.

¹H-NMR (400 MHz, CDCl₃)δ: 1.50 (9H, s), 6.82 (1H, br s), 7.22-7.27 (3H, m), 7.61-7.63 (1H, m), 7.77-7.82 (1H, m), 7.94-7.97 (1H, m), 8.34-8.35 (1H, m), 8.40-8.41 (1H, m).
EI-MSm/z: 469 (M⁺).

### 3) The title compound

The procedure of Example 9(2) was repeated by using 5-[3-(N-tert-Butyl)carbamoyl-5-(2-pyridyl)-1H-pyrazol-1-yl]-2-pyr idyl trifluoromethanesulfonate (3.28 g) to give the title compound (1.89 g, 78%) as a solid.

¹H-NMR (400 MHz, CDCl₃)δ: 1.50 (9H, s), 6.80 (1H, br s), 7.26-7.30 (2H, m), 7.63-7.65 (1H, m), 7.75 (1H, dd, J = 8.3, 0.7 Hz), 7.79-7.84 (1H, m), 7.89-7.91 (1H, m), 8.40-8.42 (1H, m), 8.62 (1H, dd, J = 2.4, 0.7 Hz).
EI-MSm/z: 346 (M⁺).

| Elementary analysis: for C₁₉H₁₈N₆O | | | |
|---|---|---|---|
| Calculated: | C, 65.88; | H, 5.24; | N, 24.26. |
| Found: | C, 65.93; | H, 5.32; | N, 24.15. |

[Example 21] N-tert-Butyl-1-(6-carbamoyl-3-pyridyl)-5-(2-pyridyl)-1H-pyrazole-3-carboxamide

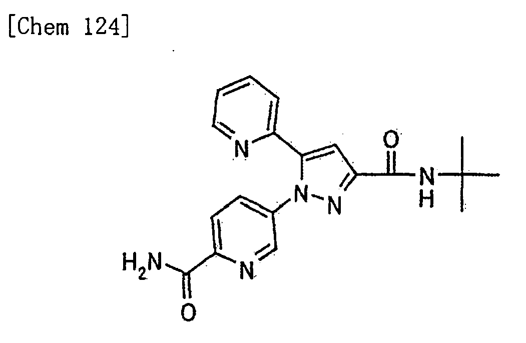

To a solution of the N-tert-butyl-1-(6-cyano-3-pyridyl)-5-(2-pyridyl)-1H-pyrazole-3-ca rboxamide (0.50 g) of Example 20 in a mixture of methanol (10 ml) and tetrahydrofuran (10 ml) was added 1N sodium hydroxide (7.22 ml) at room temperature, and the mixture was stirred at 80°C for 1 hour. After cooling with air, water and chloroform were added to the reaction liquid and the phases were separated, and the organic layer was dried over anhydrous sodium sulfate. After filtration, the solvent was evaporated under reduced pressure, and the residue was purified by column chromatography on silica gel (methanol-chloroform) to give the title compound (0.323 g, 60%) as a solid.

¹H-NMR (400 MHz, DMSO-d₆)δ: 1.40 (9H, s), 7.33 (1H, s), 7.35-7.39 (1H, m), 7.46 (1H, br s), 7.72 (1H, br s), 7.79-7.81 (1H, m), 7.89-7.94 (1H, m), 7.97-8.00 (1H, m), 8.09-8.11 (1H, m), 8.13 (1H, br s), 8.39-8.41 (1H, m), 8.56 (1H, m).
FAB-MSm/z: 365 (M+H)⁺.

[Example 22] N-tert-Butyl-5-(5-hydroxy-2-pyridyl)-1-(3-pyridyl)-1H-pyrazole-3-carboxamide

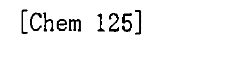

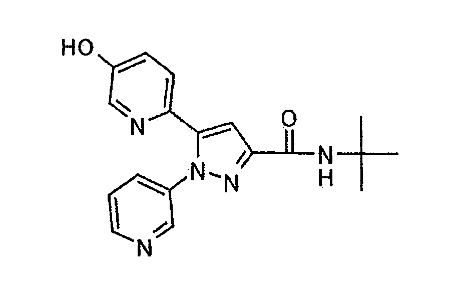

### 1) N-tert-Butyl-5-(5-benzyloxy-2-pyridyl)-1-(3-pyridyl)-1H-pyrazole-3-carboxamide

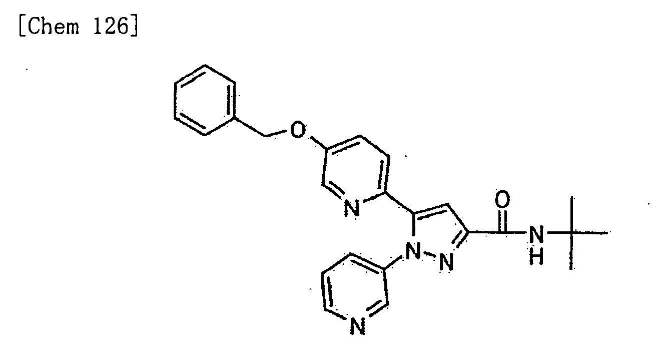

The procedure of Example 1 was repeated by using the 5-(5-benzyloxy-2-pyridyl)-1-(3-pyridyl)-1H-pyrazole-3-carboxylic acid (4.60 g) of Referential Example 18 and tert-butylamine (2.58 ml) to give N-tert-butyl-5-(5-benzyloxy-2-pyridyl)-1-(3-pyridyl)-1H-pyrazole-3-carboxamide (4.64 g, 73%) as a solid.

¹H-NMR (400 MHz, CDCl₃)δ: 1.48 (9H, s), 5.09 (2H, s), 6.84 (1H, s), 7.13 (1H, s), 7.26-7.43 (8H, m), 7.73-7.75 (1H, m), 8.19 (1H, d, J = 2.9 Hz), 8.53 (1H, d, J = 2.4 Hz), 8.58 (1H, dd, J = 4. 9, 1.5 Hz).
EI-MSm/z: 427 (M⁺).

### 2) The title compound

The procedure of Example 6(2) was repeated by using N-tert-butyl-5-(5-benzyloxy-2-pyridyl)-1-(3-pyridyl)-1H-pyrazole-3-carboxamide (4.63 g) to give the title compound (3.55 g, 96%) as a solid.

¹H-NMR (400MHz, CDCl₃)δ: 1.49 (9H, s), 6.86 (1H, s), 7.13 (1H, s), 7.20-7.23 (1H, m), 7.44-7.50 (2H, m), 7.91 (1H, d, J = 2.9 Hz), 7.99-8.02 (1H, m), 8.42 (1H, d, J = 2.4 Hz), 8.50 (1H, dd, J = 4.9, 1.5 Hz), 10.84 (1H, br s).
EI-MSm/z: 337 (M⁺).

| Elementary analysis: for C₁₈H₁₉N₅O₂·0.25H₂O | | | |
|---|---|---|---|
| Calculated: | C, 63.24; | H, 5.75; | N, 20.48. |
| Found: | C, 63.16; | H, 5.47; | N, 20.22. |

[Example 23] N-tert-Butyl-5-(5-cyano-2-pyridyl)-1-(3-pyridyl)-1H-pyrazol-3-carboxamide

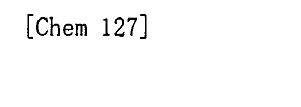

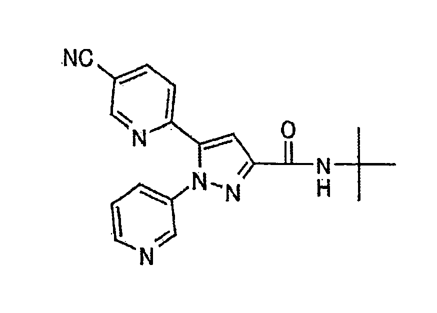

### 1) 6-[5-(N-tert-Butyl)carbamoyl-2-(3-pyridyl)-2H-pyrazol-3-yl]-3-pyridyl trifluoromethanesulfonate

The procedure of Example 9(1) was repeated by using the N-tert-butyl-5-(5-hydroxy-2-pyridyl)-1-(3-pyridyl)-1H-pyrazole-3-carboxamide (2.42 g) of Example 22 to give 6-[5-(N-tert-butyl)carbamoyl-2-(3-pyridyl)-2H-pyrazole-3-yl]-3-py ridyl trifluoromethanesulfonate (2.92 g, 88%) as a solid.

¹H-NMR (400 MHz, CDCl₃)δ: 1.49 (9H, s), 6.82 (1H, s), 7.30 (1H, s), 7.39 (1H, dd, J = 8.2, 4.8 Hz), 7.63-7.74 (3H, m), 8.36 (1H, d, J = 2.7 Hz), 8.57 (1H, d, J = 2.4 Hz), 8.64 (1H, dd, J = 4.8, 1.6 Hz).
EI-MSm/z: 469 (M⁺).

### 2) The title compound

The procedure of Example 9(2) was repeated by using 6-[5-(N-tert-butyl)carbamoyl-2-(3-pyridyl)-2H-pyrazol-3-yl]-3-pyr idyl trifluoromethanesulfonate (2.51 g) to give the title compound (1.53 g, 83%) as a solid.

¹H-NMR (400MHz, CDCl₃)δ: 1.49 (9H, s), 6.82 (1H, s), 7.36 (1H, s), 7.39-7.43 (1H, m), 7.66 (1H, dd, J = 8.3, 1.0 Hz), 7.72-7.75 (1H, m), 7.99-8.02 (1H, m), 8.55 (1H, d, J = 2.4 Hz), 8.63 (1H, m), 8.66 (1H, dd, J = 4.9, 1.5 Hz).
FAB-MSm/z: 347 (M+H)⁺.

| Elementary analysis: for C₁₉H₁₈N₆O | | | |
|---|---|---|---|
| Calculated: | C, 65.88; | H, 5.24; | N, 24.26. |
| Found: | C, 65.70; | H, 5.17; | N, 24.09. |

[Example 24] N-tert-Butyl-5-(5-carbamoyl-2-pyridyl)-1-(3-pyridyl)-1H-pyrazole-3-carboxamide

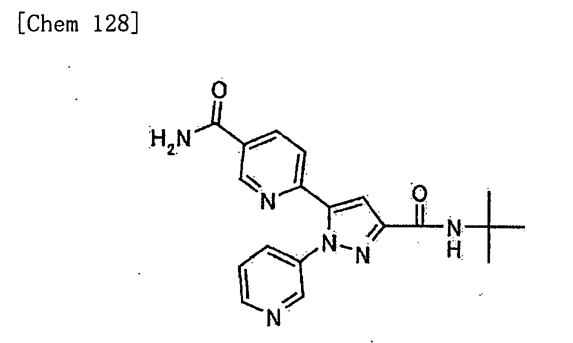

The procedure of Example 21 was repeated by using the N-tert-butyl-5-(5-cyano-2-pyridyl)-1-(3-pyridyl)-1H-pyrazole-3-ca rboxamide (0.40 g) of Example 23 to give the title compound (0.216 g, 51%) as a solid.

¹H-NMR (400 MHz, DMSO-d₆)δ: 1.39 (9H, s), 7.39 (1H, s), 7.43 (1H, br s), 7.48-7.51 (1H, m), 7.61 (1H, br s), 7.81-7.87 (2H, m), 8.13 (1H, br s), 8.26-8.28 (1H, m), 8.57-8.61 (2H, m), 8.79-8.80 (1H, m).
FAB-MSm/z: 365 (M+H)⁺.

| Elementary analysis: for C₁₉H₂₀N₆O₂·0.25H₂O | | | |
|---|---|---|---|
| Calculated: | C, 61.86; | H, 5.60; | N, 22.78. |
| Found: | C, 61.57; | H, 5.33; | N, 22.58. |

[Example 25] N-tert-Butyl-5-(5-methoxy-2-pyridyl)-1-(3-pyridyl)-1H-pyrazole-3-carboxamide

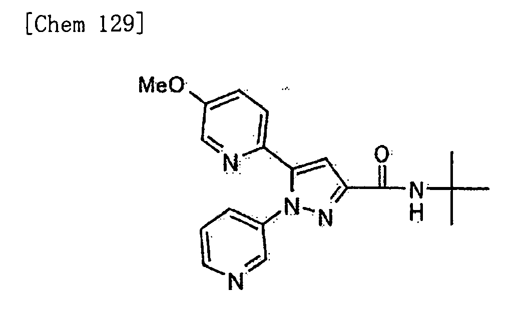

Under argon atmosphere, a 2.0M solution of (trimethylsilyl)diazomethane in hexane (0.489 ml) was added dropwise to a suspension of the N-tert-butyl-5-(5-hydroxy-2-pyridyl)-1-(3-pyridyl)-1H-pyrazole-3-carboxamide (0.30 g) of Example 22 in tetrahydrofuran (6 ml) at room temperature, and the mixture was stirred at room temperature for 20 minutes. After adding triethylamine (0.136ml) to the reaction liquid, the mixture was stirred for 10 minutes. To the solution was added dichloromethane (6 ml), and then, a 2.0M solution of (trimethylsilyl)diazomethane in hexane (0.489 ml) was added dropwise at room temperature, and the mixture was stirred for 15 minutes. Methanol (3 ml) was added to the reaction liquid, and the mixture was stirred at room temperature for 2 hours. The reaction solvent was evaporated under reduced pressure, and the residue was purified by column chromatography on silica gel (ethyl acetate-chloroform) to give the title compound (0.145 g, 45%) as a solid.

¹H-NMR (400 MHz, CDCl₃)δ: 1.49 (9H, s), 3.85 (3H, s), 6.84 (1H, br s), 7.14 (1H, s), 7.20-7.23 (1H, m), 7.33-7.37 (1H, m), 7.42-7.44 (1H, m), 7.73-7.76 (1H, m), 8.12 (1H, m), 8.57-8.58 (1H, m).
FAB-MSm/z: 352 (M+H)⁺.

[Example 26] N-tert-Butyl-5-(5-hydroxymethyl-2-pyridyl)-1-(3-pyridyl)-1H-pyrazole-3-carboxamide

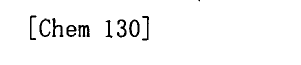

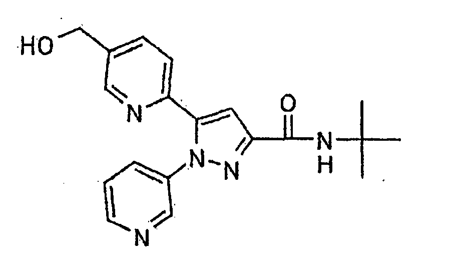

### 1) The title compound

Under argon atmosphere, a 1.01M solution of diisobutylaluminum hydride in toluene (4.25 ml) was added dropwise to a suspension of the N-tert-butyl-5-(5-cyano-2-pyridyl)-1-(3-pyridyl)-1H-pyrazole-3-ca rboxamide (0.929 g) of Example 23 in tetrahydrofuran (19 ml) at 0°C, and the mixture was stirred for 70 minutes. Another portion of the 1.01M solution of diisobutylaluminum hydride in toluene (4.25 ml) was added dropwise, and the mixture was stirred for 80 minutes. A 4N aqueous hydrochloric acid (10 ml) was added to the reaction liquid, and the mixture was stirred at room temperature for 45 minutes, and water and chloroform were added to the reaction liquid and the phases were separated. The organic layer was dried over anhydrous sodium sulfate. The aqueous layer obtained in this phase separation process was designated [Solution A], and it was used in the next step (2). After filtration, the solvent was evaporated under reduced pressure, and to a solution of the residue in methanol (19 ml) was added sodium borohydride (0.223 g) at room temperature under argon atmosphere, and the mixture was stirred at room temperature for 13 hours. Saturated aqueous sodium bicarbonate and chloroform were added to the reaction liquid and the phases were separated, and the organic layer was dried over anhydrous sodium sulfate. After filtration, the solvent was evaporated under reduced pressure, and the residue was purified by thin layer chromatography on silica gel (methanol-chloroform) to give the title compound (0.152 g, 16%) as a solid.

¹H-NMR (400 MHz, CDCl₃)δ: 1.48 (9H, s), 4.71-4.72 (2H, m), 6.85 (1H, br s), 7.18 (1H, d, J = 0.7 Hz), 7.34-7.37 (1H, m), 7.48 (1H, d, J = 8.1 Hz), 7.73-7.78 (2H, m), 8.39-8.40 (1H, m), 8.51 (1H, d, J = 2.7 Hz), 8.57 (1H, d, J = 4.2 Hz).
FAB-MSm/z: 352 (M+H)⁺.

| Elementary analysis: for C₁₉H₂₁N₅O₂⁻0.25H₂O | | | |
|---|---|---|---|
| Calculated: | C, 64.12; | H, 6.09; | N, 19.68. |
| Found: | C, 64.23; | H, 6.10; | N, 19.68. |

### 2) N-tert-Butyl-1-(3-pyridyl)-5-(5-tert-butoxycarbonylaminomethyl-2-pyridyl)-1H-pyrazole-3-carboxamide

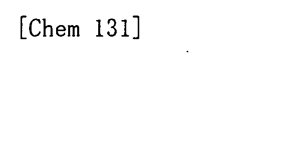

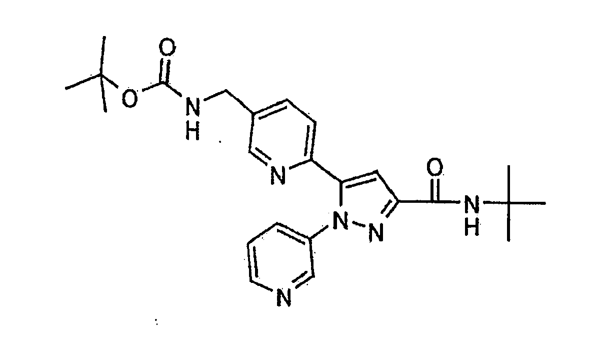

To the "Solution A" obtained in the previous step was added 1N aqueous sodium hydroxide (60 ml) and chloroform, and the phases were separated, and the organic layer was dried over anhydrous sodium sulfate. After filtration, the solvent was evaporated under reduced pressure, andasolutionoftriethylamine (0.601ml) anddi-tert-butyldicarbonate (0.471 g) in methanol (6.3 ml) was added to a solution of the residue in methanol (6.3 ml) at room temperature, and the mixture was stirred for 20 hours. The reaction solvent was evaporated under reduced pressure, and the residue was purified by column chromatography on silica gel (ethyl acetate-chloroform) to give N-tert-butyl-1-(3-pyridyl)-5-(5-tert-butoxycarbonylaminomethyl-2-pyridyl)-1H-pyrazole-3-carboxamide (84.0 mg, 10%) as an amorphous product.

¹H-NMR (400 MHz, CDCl₃)δ: 1.45 (9H, s), 1.49 (9H, s), 4.31-4.32 (2H, m), 4.91 (1H, br s), 6.84 (1H, br s), 7.21 (1H, s), 7.34-7.37 (1H, m), 7.47 (1H, d, J = 8.1 Hz), 7.67-7.69 (1H, m), 7.72-7.75 (1H, m), 8.33-8.34 (1H, m), 8.54 (1H, d, J = 2.4 Hz), 8.58-8.60 (1H, m). FAB-MSm/z: 451 (M+H)⁺.

[Example 27] N-tert-Butyl-5-(5-aminomethyl-2-pyridyl)-1-(3-pyridyl)-1H-pyrazole-3-carboxamide hydrochloride

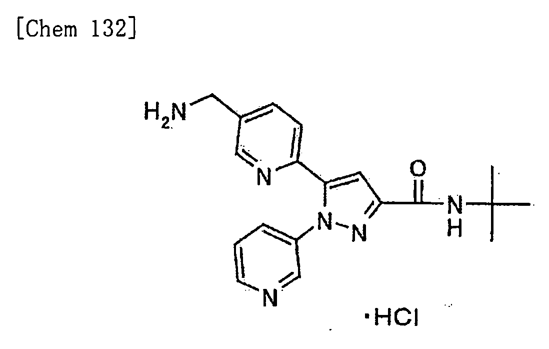

A 1N solution of hydrochloric acid in ethanol (6 ml) was added to a solution of the N-tert-butyl-1-(3-pyridyl)-5-(5-tert-butoxycarbonylaminomethyl-2-pyridyl)-1H-pyrazole-3-carboxamide (74.0 mg) of Example 26(2), and the mixture was stirred at 60°C for 4 hours. After cooling with air, the reaction solvent was evaporated under reduced pressure, and water and ethyl acetate were added to the residue and the aqueous layer was separated. The water was evaporated under reduced pressure to give the title compound (55.1 mg, 77%) as a solid.

¹H-NMR (400 MHz, DMSO-d₆)δ: 1.39 (9H, s), 7.33 (1H, s), 7.41 (1H, s), 7.51-7.55 (1H, m), 7.83 (1H, d, J=8.1Hz), 7.88-7.90 (1H, m), 8.00-8.03 (1H, m), 8.40 (2H, br s), 8.45 (1H, s), 8.55 (1H, d, J = 2.2 Hz), 8.61 (1H, d, J = 4.9 Hz).
FAB-MSm/z: 351 (M+H)⁺.

[Example 28] N-tert-Butyl-5-(5-chloro-2-pyridyl)-1-(3-pyridyl)-1H-pyrazole-3-carboxamide

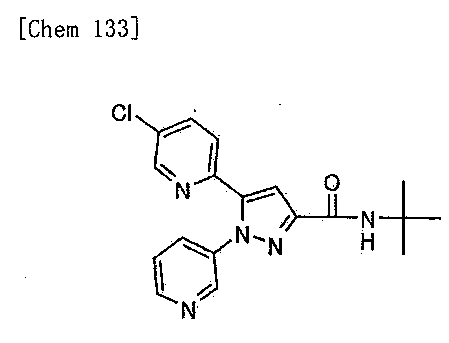

The procedure of Example 1 was repeated by using the 5-(5-chloro-2-pyridyl)-1-(3-pyridyl)-1H-pyrazole-3-carboxylic acid (348 mg) of Referential Example 25 and tert-butylamine (195 µl) to give the title compound (173 mg, 42%) as a solid.

¹H-NMR (400 MHz, CDCl₃)δ: 1.49 (9H, s), 6.84 (1H, br), 7.27 (1H, s), 7.38 (1H, dd, J= 8.18, 4.76 Hz), 7.49 (1H, d, J= 8.18 Hz), 7.74 (2H, m), 8.36 (1H, d, J = 2.08 Hz), 8.54 (1H, d, J = 2.08 Hz), 8.62 (1H, dd, J = 4.76, 1.47 Hz).
FAB-MSm/z: 356 (M+H)⁺.

[Example 29] N-tert-Butyl-5-(4-carbamoylphenyl)-1-(3-pyridyl)-1H-pyrazole-3-carboxamide

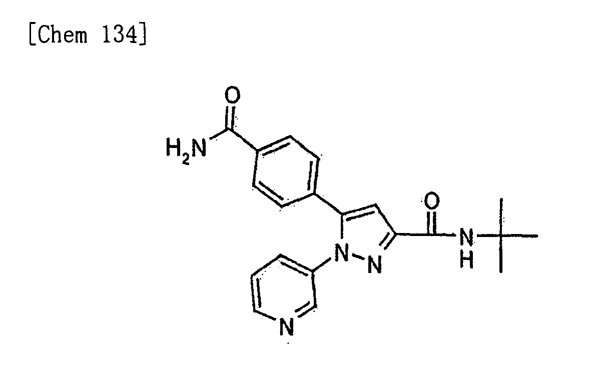

### 1) N-tert-Butyl-5-(4-cyanophenyl)-1-(3-pyridyl)-1H-pyrazole-3-carboxamide

The procedure of Example 1 was repeated by using the 5-(4-cyanophenyl)-1-(3-pyridyl)-1H-pyrazole-3-carboxylic acid (0.374 g) of Referential Example 24 and tert-butylamine (0.269 ml) to give N-tert-butyl-5-(4-cyanophenyl)-1-(3-pyridyl)-1H-pyrazole-3-carbox amide (0.413 g, 93%) as a solid.

¹H-NMR (400 MHz, CDCl₃)δ: 1.49 (9H, s), 6.82 (1H, br s), 7.12 (1H, s), 7.31-7.33 (2H, m), 7.38 (1H, dd, J = 8.1, 4.9 Hz), 7.61-7.65 (3H, m), 8.58 (1H, d, J = 2.4 Hz), 8.65 (1H, dd, J = 4. 9, 1.5 Hz). EI-MSm/z: 345 (M⁺).

| Elementary analysis: for C₂₀H₁₉N₅O | | | |
|---|---|---|---|
| Calculated: | C, 69.55; | H, 5.54; | N, 20.28. |
| Found: | C, 69.53; | H, 5.53; | N, 20.17. |

### 2) The title compound

The procedure of Example 21 was repeated by using N-tert-butyl-5-(4-cyanophenyl)-1-(3-pyridyl)-1H-pyrazole-3-carbox amide (0.314 g) to give the title compound (0.191 g, 57%) as a solid.

¹H-NMR (400 MHz, DMSO-d₆)δ: 1.40 (9H, s), 7.08 (1H, s), 7.33-7.43 (4H, m), 7.50 (1H, dd, J = 8.2, 4.8 Hz), 7.79-7.86 (3H, m), 8.01 (1H, br s), 8.57 (1H, d, J = 2.7 Hz), 8.61 (1H, dd, J = 4.8, 1.3 Hz).
EI-MSm/z: 363 (M⁺).

| Elementary analysis: for C₂₀H₂₁N₅O₂·0.25H₂O | | | |
|---|---|---|---|
| Calculated: | C, 65.29; | H, 5.89; | N, 19.03. |
| Found: | C, 65.45; | H, 5.77; | N, 18.91. |

[Example 30] N-tert-Butyl-1-(5-methoxy-1,3,4-thiadiazole-2-yl)-5-(2-pyridyl)-1H-pyrazole-3-carboxamide

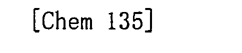

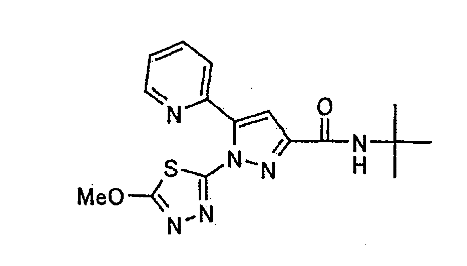

### 1) N-tert-Butyl-1-(5-ethylthio-1,3,4-thiadiazole-2-yl)-5-(2-pyridyl)-1H-pyrazole-3-carboxamide

The procedure of Example 1 was repeated by using the 1-(5-ethylthio-1,3,4-thiadiazole-2-yl)-5-(2-pyridyl)-1H-pyrazole-3-carboxylic acid (0.919 g) of Referential Example 19 and tert-butylamine (0.576 ml) to give N-tert-butyl-1-(5-ethylthio-1,3,4-thiadiazole-2-yl)-5-(2-pyridyl) -1H-pyrazole-3-carboxamide (1.05 g, 98%) as an amorphous product.

¹H-NMR (400 MHz, CDCl₃)δ: 1.44-1.48 (3H, m), 1.48 (9H, s), 3.30-3.36 (2H, m), 6.75 (1H, br s), 7.16 (1H, s), 7.28-7.31 (1H, m), 7.61 (1H, d, J = 7.8 Hz), 7.75-7.80 (1H, m), 8.54-8.55 (1H, m).
EI-MSm/z: 388 (M⁺).

### 2) N-tert-Butyl-1-(5-ethylsulfinyl-1,3,4-thiadiazol-2-yl)-5-(2-pyridyl)-1H-pyrazole-3-carboxamide

Under argon atmosphere, 3-chlorochloroperbenzoic acid (0.508 g) was added to a solution of N-tert-butyl-1-(5-ethylthio-1,3,4-thiadiazol-2-yl)-5-(2-pyridyl)-1H-pyrazole-3-carboxamide (1.04 g) in dichloromethane (21 ml) at room temperature, and the mixture was stirred for 30 minutes. Saturated aqueous sodium thiosulfate (40 ml), saturated aqueous sodium bicarbonate (40 ml), and chloroform were added to the reaction liquid and the phases were separated, and the organic layer was dried over anhydrous sodium sulfate. After filtration, the solvent was evaporated under reduced pressure, and the residue was purified by column chromatography on silica gel (ethyl acetate-chloroform) to give N-tert-butyl-1-(5-ethylsulfinyl-1,3,4-thiadiazol-2-yl)-5-(2-pyrid yl)-1H-pyrazole-3-carboxamide (0.860 g, 80%) as a solid.

¹H-NMR (400 MHz, CDCl₃)δ: 1.38-1.41 (3H, m), 1.49 (9H, s), 3.20-3.38 (2H, m), 6.76 (1H, br s), 7.20 (1H, s), 7.31-7.35 (1H, m), 7.63 (1H, d, J = 7.8 Hz), 7.79-7.83 (1H, m), 8.53-8.54 (1H, m).
EI-MSm/z: 404 (M⁺).

### 3) The title compound

To a solution of N-tert-butyl-1-(5-ethylsulfinyl-1,3,4-thiadiazol-2-yl)-5-(2-pyrid yl)-1H-pyrazole-3-carboxamide (0.850 g) in a mixture of methanol (17 ml) and tetrahydrofuran (17 ml) was added 1N sodium hydroxide (8.5 ml) at room temperature, and the mixture was stirred for 10 minutes. Water and chloroform were added to the reaction liquid and the phases were separated, and the organic layer was dried over anhydrous sodium sulfate. After filtration, the solvent was evaporated under reduced pressure, and the residue was purified by column chromatography on silica gel (ethyl acetate-chloroform) to give the title compound (0.379 g, 50%) as a solid.

¹H-NMR (400 MHz, CDCl₃)δ: 1.48 (9H, s), 4.21 (3H, s), 6.76 (1H, br s), 7.16 (1H, s), 7.27-7.30 (1H, m), 7.60-7.62 (1H, m), 7.75-7.79 (1H, m), 8.54-8.56 (1H, m).
EI-MSm/z: 358 (M⁺).

| Elementary analysis: for C₁₆H₁₈N₆O₂S | | | | |
|---|---|---|---|---|
| Calculated: | C, 53.62; | H, 5.06; | N, 23.45; | S, 8.95. |
| Found: | C, 53.53; | H, 4.90; | N, 23.45; | S, 8.94. |

[Example 31] N-tert-Butyl-1-(6-methoxy-3-pyridyl)-5-(1-methyl-1H-pyrrol-2-yl)-1H-pyrazole-3-carboxamide

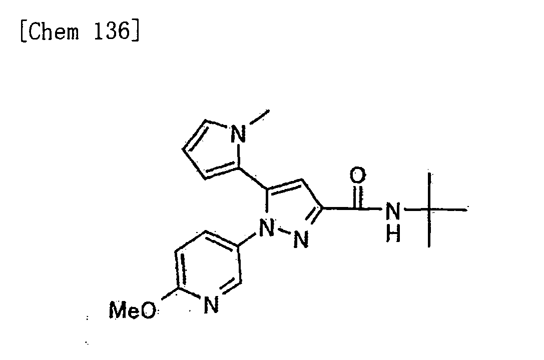

The procedure of Example 1 was repeated by using the 1-(6-methoxy-3-pyridyl)-5-(1-methyl-1H-pyrrol-2-yl)-1H-pyrazole-3
- carboxylic acid (0.232g) of Referential Example 23 and tert-butylamine (0.148 ml) to give the title compound (147 mg, 53%) as a solid.

¹H-NMR (400 MHz, CDCl₃)δ: 1.49 (9H, s), 3.40 (3H, s), 3.95 (3H, s), 6.01 (1H, dd, J = 3.7, 1.7 Hz), 6.12 (1H, dd, J = 3.7, 2.7 Hz), 6.69-6.73 (2H, m), 6.85 (1H, br s), 6.96 (1H, s), 7.44 (1H, dd, J = 8.8, 2.7 Hz), 8.12 (1H, d, J = 2.7 Hz).
ESI-MSm/z: 354 (M+H)⁺.

| Elementary analysis: for C₁₉H₂₃N₅O₂ | | | |
|---|---|---|---|
| Calculated: | C, 64.57; | H, 6.56; | N, 19.82. |
| Found: | C, 64.57; | H, 6.55; | N, 19.71. |

[Example 32] N-(cis-2-Aminocyclopropyl)-1-(6-methoxy-3-pyridyl)-5-phenyl-1H-pyrazole-3-carboxamide

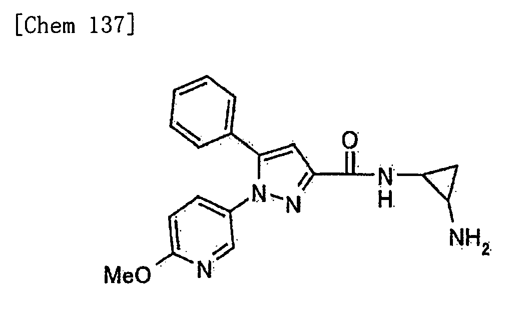

To a solution of the 1-(6-methoxy-3-pyridyl)-5-phenyl-1H-pyrazole-3-carboxamide (295mg) of Referential Example 5, N-hydroxy succinimide (115 mg), and triethylamine (446 µl) in dichloromethane (10 ml) was added 1-ethyl-3-(3-dimethylaminopropyl)carbodiimide hydrochloride (230 mg), and the mixture was stirred at room temperature for 2 hours. This reaction liquid was added dropwise to a suspension of cis-1,2-diaminocyclopropane dihydrochloride (290 mg) and triethylamine (557 µl) in dichloromethane (20 ml) over 5 minutes, and the mixture was stirred at room temperature for 13 hours. Saturated aqueous sodium bicarbonate and dichloromethane were added to the reaction liquid and the phases were separated, and the organic layer was dried over anhydrous sodium sulfate. After filtration, the solvent was evaporated under reduced pressure, and the residue was purified by thin layer chromatography on silica gel (chloroform-methanol) to give the title compound (157 mg, 44%) as an oily product.

¹H-NMR (400 MHz, CDCl₃)δ: 0.44-0.51 (1H, m), 1.04-1.12 (1H, m), 2.59-2.66 (1H, m), 2.97-3.06 (1H, m), 3.94 (3H, s), 6.71 (1H, d, J = 8.8 Hz), 7.04 (1H, s), 7.18-7.25 (2H, m), 7.28-7.37 (3H, m), 7.37-7.43 (1H, br), 7.48 (1H, dd, J = 8.8, 2.7 Hz), 8.12 (1H, d, J = 2.7 Hz).
ESI-MSm/z: 350 (M+H)⁺.

[Example 33] N-(cis-2-Dimethylaminocyclopropyl)-1-(6-methoxy-3-pyridyl)-5-phenyl-1H-pyrazole-3-carboxamide hydrochloride

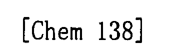

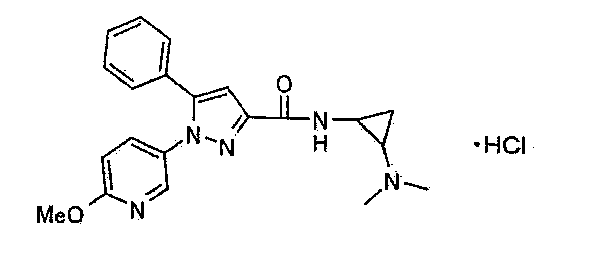

Cyano sodium borohydride (66 mg) was added to a solution of the N-(cis-2-aminocyclopropyl)-1-(6-methoxy-3-pyridyl)-5-phenyl-1H-py razole-3-carboxamide (92 mg) of Example 32, 35% aqueous formaldehyde (208 µl), and acetic acid (150 µl) in ethanol (10 ml), and the mixture was stirred at room temperature for 2 hours. Saturated aqueous sodium bicarbonate and ethyl acetate were added to the reaction liquid and the phases were separated, and the organic layer was washed with water and brine, and dried over anhydrous sodium sulfate. After filtration, the solvent was evaporated under reduced pressure, and the residue was purified by thin layer chromatography on silica gel (chloroform-methanol) to give N-(cis-2-dimethylaminocyclopropyl)-1-(6-methoxy-3-pyridyl)-5-phen yl-1H-pyrazole-3-carboxamide (93mg) asanoilyproduct. To a solution of this carboxamide derivative inmethanol (10ml) was added a 1M solution of hydrochloric acid in ethanol (0.25 ml), and the mixture was stirred. The reaction solvent was evaporated under reduced pressure to give the title compound (97 mg, 82%) as a solid.

¹H-NMR (400 MHz, DMSO-d₆)δ: 1.28-1.38 (1H, m), 1.43-1.52 (1H, m), 2.83 (3H, br s), 2.92 (3H, br s), 2.99-3.11 (1H, m), 3.87 (3H, s), 6.91 (1H, d, J = 8.8 Hz), 7.16 (1H, s), 7.26-7.33 (2H, m), 7.36-7.45 (3H, m), 7.72 (1H, dd, J = 8.8, 2.7 Hz), 8.18 (1H, d, J = 2.7 Hz), 8.60-8.69 (1H, br), 9.72-9.88 (1H, br).
ESI-MSm/z: 378 (M+H)⁺.

[Example 34] N-(Pyrrolidin-3-yl)-1-(6-methoxy-3-pyridyl)-5-phenyl-1H-pyrazole-3-carboxamide hydrochloride

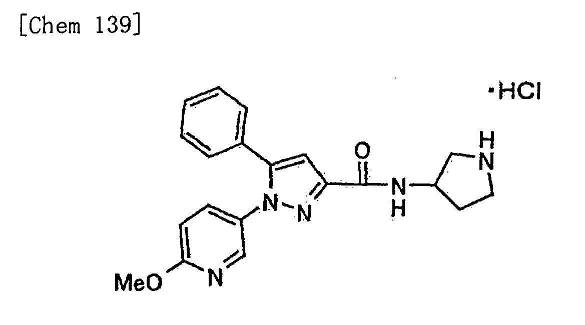

### 1) N-(N'-Benzyloxycarbonyl pyrrolidin-3-yl)-1-(6-methoxy-3-pyridyl)-5-phenyl-1H-pyrazole-3-carboxamide

The procedure of Example 7 was repeated by using the 1-(6-methoxy-3-pyridyl)-5-phenyl-1H-pyrazole-3-carboxylicacid(443 mg) of Referential Example 5 and the 3-aminopyrrolidine-1-carboxylic acid benzyl ester trifluoroacetate (501 mg) of Referential Example 27 to give N-(N'-benzyloxycarbonyl pyrrolidin-3-yl)-1-(6-methoxy-3-pyridyl)-5-phenyl-1H-pyrazole-3-c arboxamide (633 mg, 84%) as an oily product.

¹H-NMR (400 MHz, CDCl₃)δ: 1.93-2.08 (1H, br m), 2.21-2.32 (1H, br m), 3.36-3.47 (1H, m), 3.50-3.64 (2H, m), 3.76-3.84 (1H, m), 3.95 (3H, s), 4.65-4.73 (1H, m), 5.14 (2H, s), 6.74 (1H, d, J = 8.8 Hz), 7.00 (1H, d, J = 7.3 Hz), 7.03 (1H, s), 7.18-7.27 (2H, m), 7.28-7.41 (8H, m), 7.50 (1H, dd, J = 8.8, 2.7 Hz), 8.11 (1H, d, J = 2.7 Hz).
ESI-MSm/z: 498 (M+H)⁺.

### 2) The title compound

To a solution of N-(N'-benzyloxycarbonyl pyrrolidin-3-yl)-1-(6-methoxy-3-pyridyl)-5-phenyl-1H-pyrazole-3-c arboxamide (622 mg) in ethanol (25 ml) were added a 1M solution of hydrochloric acid in ethanol (1.25 ml) and 10% palladium on carbon (50% wet, 124 mg), and the mixture was stirred at room temperature for 4 hours under hydrogen atmosphere. After removing the catalyst, the solvent was evaporated under reduced pressure, and the residue was purified by gel filtration column chromatography (methanol) to give the title compound (388 mg, 73%) as a solid.

¹H-NMR (400MHz, DMSO-d₆)δ: 1.96-2.07 (1H, m), 2.15-2.26 (1H, m), 3.16-3.28 (2H, m), 3.32-3.45 (2H, m), 3.87 (3H, s), 4.57-4.65 (1H, m), 6.90 (1H, d, J = 8.8 Hz), 7.05 (1H, d, J = 2.7 Hz), 7.24-7.31 (2H, m), 7.35-7.42 (3H, m), 7.68-7.75 (1H, m), 8.16 (1H, d, J = 2.4 Hz), 8.66 (1H, d, J = 7.1 Hz), 8.92-9.16 (2H, br).
ESI-MSm/z: 364 (M+H)⁺.

[Example 35] N-(N'-Methylpyrrolidin-3-yl)-1-(6-methoxy-3-pyridyl)-5-phenyl-1H-pyrazole-3-carboxamide hydrochloride

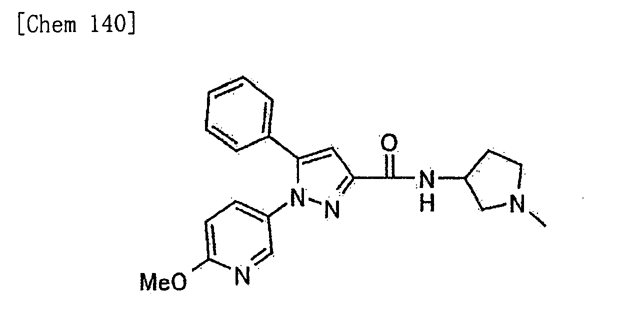

Cyano sodium borohydride (70 mg) was added to a solution of the N-(pyrrolidin-3-yl)-1-(6-methoxy-3-pyridyl)-5-phenyl-1H-pyrazole-3-carboxamide hydrochloride (222 mg) of Example 34, 35% aqueous formaldehyde (220 µl), and acetic acid (159 µl) in ethanol (10 ml), and the mixture was stirred at room temperature for 2 hours. Saturated aqueous sodium bicarbonate and ethyl acetate were added to the reaction liquid and the phases were separated, and the organic layer was washed with water and brine, and dried over anhydrous sodium sulfate. After filtration, the solvent was evaporated under reduced pressure, and the residue was purified by thin layer chromatography on silica gel (chloroform-methanol) to give N-(N'-methylpyrrolidine-3-yl)-1-(6-methoxy-3-pyridyl)-5-phenyl-1H -pyrazole-3-carboxamide (187 mg) as an oily product. This product was dissolved in a solution of diethylether and methanol (several drops), and a 1M solution of hydrochloric acid in ethanol (0.55 ml) was added to the mixture. The mixture was stirred, and the solid precipitate was collected by filtration to give the title compound (197 mg, 84%).

¹H-NMR (400 MHz, DMSO-d₆)δ: 1.97-3.92 (6H, m), 2.83 and 2.84 (3H, s), 3.87 (3H, s), 4.59-4.69 and 4.71-4.81 (1H, m), 6.90 (1H, d, J = 8.8 Hz), 7.07 (1H, d, J = 2.4 Hz), 7.23-7.31 (2H, m), 7.35-7.44 (3H, m), 7.69-7.78 (1H, m), 8.17 (1H, d, J = 2.7 Hz), 8.74 and 8.83 (1H, d, J = 7.5 Hz), 10.57-10.73 and 10.79-10.95 (1H, br).
ESI-MSm/z: 378 (M+H)⁺.

[Example 36] N-(2-Dimethylamino-1,1-dimethylethyl)-1-(6-methoxy-3-pyridazinyl)-5-(2-pyridyl)-1H-pyrazole-3-carboxamide

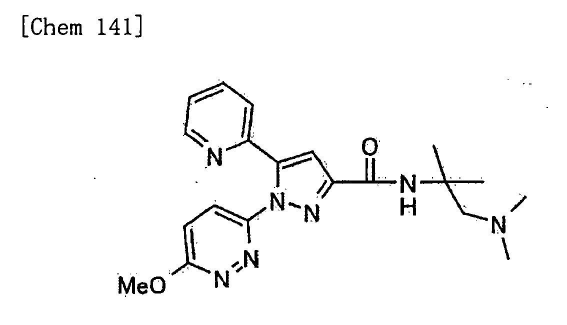

The procedure of Example 1 was repeated by using a solution of N-benzyloxycarbonyl-2-methylalanine (2.16 g) and 2.0M solution of dimethylamine in tetrahydrofuran (9.09 ml) to give N-(1-dimethylcarbamoyl-1-methylethyl)carbamic acid benzyl ester (2.33 g, 96%) as a solid. The procedure of Example 6(2) was repeated by using a solution of this benzyl ester derivative (2.33 g) in ethanol (50 ml) to give 2-amino-2, N,N-trimethylpropionamide. This amide derivative was dissolved in tetrahydrofuran (100 ml), and to this solution was added lithium aluminum hydride (454 mg), and the mixture was heated under reflux for 16 hours. After cooling with air, anhydrous magnesium sulfate and methanol were added to the reaction liquid, and the mixture was stirred. After filtration, the solvent was evaporated under reduced pressure, and dichloromethane was added to the residue, and the mixture was dried over anhydrous magnesium sulfate. After filtration, the solvent was evaporated under reduced pressure to give 2, N¹, N¹-trimethyl-1,2-propanediamine (0.600 g, 58%) as an oilyproduct. The procedure of Example 1 was repeated by using this propanediamine derivative (0.600 g) and the 1-(6-methoxy-3-pyridazinyl)-5-(2-pyridyl)-1H-pyrazole-3-carboxyli c acid (0.232 g) of Referential Example 9 to give the title compound (102 mg, 33%) as a solid.

¹H-NMR (400 MHz, CDCl₃)δ: 1.47 (6H, s), 2.36 (6H, s), 2.60 (2H, s), 4.11 (3H, s), 7.14 (1H, d, J = 9.3 Hz), 7.18-7.22 (2H, m), 7.29 (1H, br s), 7.60 (1H, d, J = 7.8 Hz), 7.73-7.77 (1H, m), 7.80 (1H, d, J = 9.3 Hz), 8.37-8.39 (1H, m).
ESI-MSm/z: 396 (M+H)⁺.

| Elementary analysis: for C₂₀H₂₅N₇O₂ | | | |
|---|---|---|---|
| Calculated: | C, 60.74; | H, 6.37; | N, 24.79. |
| Found: | C, 60.55; | H, 6.25; | N, 24.55. |

[Example 37] N-(1-Dimethylaminomethyl-1-cyclopentyl)-1-(6-methoxy-3-pyridyl)-5-(2-pyridyl)-1H-pyrazole-3-carboxamide

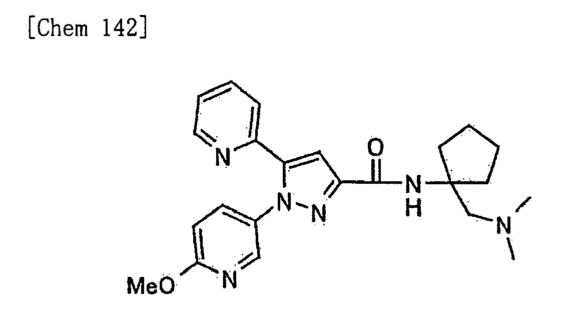

The procedure of Example 1 was repeated by using a solution of 1-(N-benzyloxycarbonyl)aminocyclopentanecarboxylic acid (2.63 g) and 2.0M solution of dimethylamine in tetrahydrofuran (10 ml) to give N-(1-dimethylcarbamoyl-1-cyclopentyl)carbamic acid benzyl ester (2.00 g, 68%) as a solid. The procedure of Example 6(2) was repeated by using a solution of this benzyl ester derivative in ethanol (50 ml) to give 1-amino-N,N-dimethylcyclopentylcarboxamide. Lithium aluminum hydride (355 mg) was added to a suspension of this carboxamide derivative in tetrahydrofuran (75 ml), and the mixture was heated under reflux for 14.5 hours. After cooling with air, anhydrous magnesium sulfate, methanol, and dichloromethane were added, and the mixture was stirred. After filtration, the solvent was evaporated under reduced pressure, and a solution of the residue in dichloromethane was dried over anhydrous magnesium sulfate. After filtration, the solvent was evaporated under reduced pressure to give
1- (dimethylaminomethyl) cyclopentylamine (1.14 g, measured) as an oily product. The procedure of Example 1 was repeated by using this amine derivative (0.470 g) and 1-(6-methoxy-3-pyridyl)-5-(2-pyridyl)-1H-pyrazole-3-carboxylic acid (0.231 g) of Referential Example 4 to give the title compound (104 mg, 31%) as a solid.

¹H-NMR (400MHz, CDCl₃)δ: 1.62-1.69 (2H, m), 1.78-1.86 (4H, m), 2.12-2.19 (2H, m), 2.34 (6H, s), 2.74 (2H, br s), 3.96 (3H, s), 6.76 (1H, dd, J = 8.8, 0.5 Hz), 7.20 (1H, s), 7.20-7.24 (2H, m), 7.42 (1H, ddd, J = 7.8, 1.0, 1.0 Hz), 7.60 (1H, dd, J = 8.8, 2.7 Hz), 7.70 (1H, ddd, J = 7.8, 7.8, 2.0 Hz), 8.12 (1H, d, J = 2.2 Hz), 8.48-8.50 (1H, m). ESI-MSm/z: 421 (M+H)⁺.

| Elementary analysis: for C₂₃H₂₈N₆O₂ | | | |
|---|---|---|---|
| Calculated: | C, 65.69; | H, 6.71; | N, 19.99. |
| Found: | C, 65.71; | H, 6.73; | N, 20.12. |

[Example 38] N-(1,3-Dimethylpyrrolidin-3-yl)-1-(6-methoxy-3-pyridazinyl)-5-(2-pyridyl)-1H-pyrazole-3-carboxamide

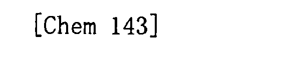

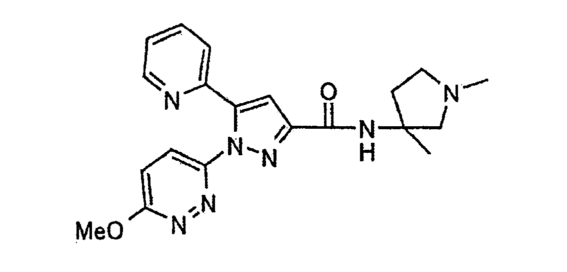

A suspension of 2-methylaminoacetic acid (2.00 g), paraformaldehyde (2.00 g), and 2-methylmethacrylate (1.07 ml) in benzene (250 ml) was heated under reflux for 3 hours. After cooling with air, the reaction liquid was filtered, and the solvent was evaporated under reduced pressure to give methyl 1,3-dimethyl-3-pyrrolidinecarboxylate (0.770 g, 21%) as an oily product. To a solution of this methyl ester derivative (0.770 g) in ethanol (15 ml) was added 1N aqueous sodium hydroxide (15.0 ml), and the mixture was stirred at room temperature for 19 hours. 1N aqueous hydrochloric acid (15.0 ml) was added to the reaction liquid, and the reaction solvent was evaporated under reduced pressure, and to this residue were added 2-methyl-2-propanol (20 ml), triethylamine (1.02 ml), and diphenylphosphorylazide (1.58 ml), and the mixture was heated under reflux for 17.5 hours. After cooling with air, the reaction solvent was evaporated under reduced pressure, and saturated aqueous sodium bicarbonate and dichloromethane were added to the residue and the phases were separated. The organic layer was dried over anhydrous sodium sulfate. After filtration, the solvent was evaporated under reduced pressure, and a 4N solution of hydrochloric acid in dioxane (10 ml) was added to the residue, and the mixture was stirred at room temperature for 4 hours. The reaction solvent was evaporated under reduced pressure to give 3-amino-1,3-dimethylpyrrolidine hydrochloride. The procedure of Example 1 was repeated by using a half of this aminopyrrolidine hydrochloride derivative and the 1-(6-methoxy-3-pyridazinyl)-5-(2-pyridyl)-1H-pyrazole-3-carboxyli c acid (0.232 g) of Referential Example 10 to give the title compound (169 mg, 17%) as a solid.

¹H-NMR (400 MHz, CDCl₃)δ: 1.63 (3H, s), 1.99-2.06 (1H, m), 2.24-2.32 (1H, m), 2.37 (3H, s), 2.58-2.67 (2H, m), 2.74-2.80 (1H, m), 2.98 (1H, d, J = 9.8 Hz), 4.12 (3H, s), 7.08 (1H, br s), 7.13 (1H, d, J = 9.3 Hz), 7.19-7.22 (2H, m), 7.60 (1H, d, J = 7.8 Hz), 7.73-7.77 (2H, m), 8.36-8.38 (1H, m).
ESI-MSm/z: 394 (M+H)⁺.

[Example 39] N-(2-Hydroxy-1,1-dimethylethyl)-1-(6-methoxy-3-pyridazinyl)-5-(2-pyridyl)-1H-pyrazole-3-carboxamide

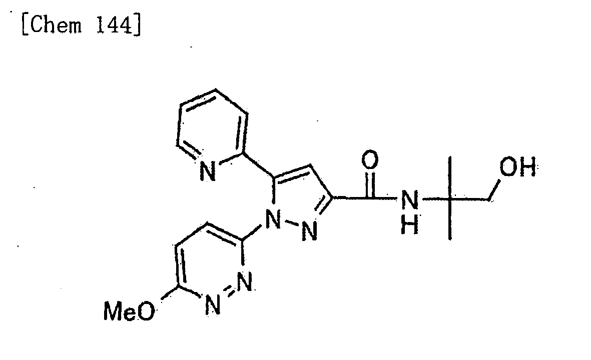

The procedure of Example 7 was repeated by using the 1-(6-methoxy-3-pyridazinyl)-5-(2-pyridyl)-1H-pyrazole-3-carboxyli c acid (300 mg) of Referential Example 10 and 2-amino-2-methyl-1-propanol (115 µl) to give the title compound (293 mg, 79%) as a solid.

¹H-NMR (400 MHz, CDCl₃)δ: 1.42 (6H, s), 3.71 (2H, d, J = 6.23 Hz), 4.13 (3H, s), 4.80 (1H, t, J = 6.23 Hz), 7.06 (1H, br), 7.13 (1H, d, J = 9.16 Hz), 7.22 (2H, m), 7.60 (1H, d, J = 7.81 Hz), 7.73 (1H, d, J = 9.16 Hz), 7.76 (1H, m), 8.36 (1H, d, J = 4.03 Hz).
FAB-MSm/z: 369 (M+H)⁺.

[Example 40] N-(2-Methoxy-1,1-dimethylethyl)-1-(6-methoxy-3-pyridazinyl)-5-(2-pyridyl)-1H-pyrazole-3-carboxamide

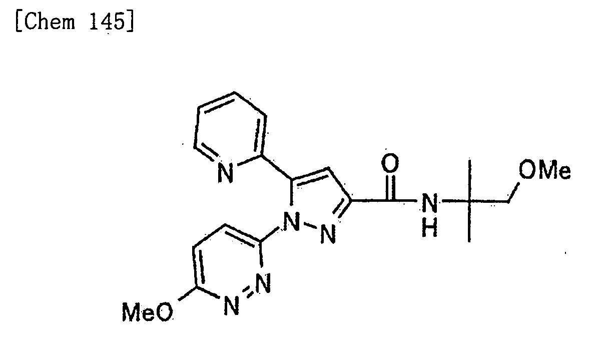

To a suspension of 60% sodium hydride (7 mg) in tetrahydrofuran (1 ml) was added the N-(2-hydroxy-1,1-dimethylethyl)-1-(6-methoxy-3-pyridazinyl)-5-(2-pyridyl)-1H-pyrazole-3-carboxamide (50 mg) of Example 39 at room temperature, and the mixture was stirred. Methyl iodide (26 µl) was added to the reaction liquid, and the mixture was stirred for 4 hours. Water and chloroform were added to the reaction liquid and the phases were separated. The organic layer was dried over anhydrous magnesium sulfate. After filtration, the solvent was evaporated under reduced pressure, and the residue was purified by thin layer chromatography on silica gel (chloroform-methanol) to give the title compound (40 mg, 70%) as a solid.

¹H-NMR (400 MHz, CDCl3)δ: 1.48 (6H, s), 3.41 (3H, s), 3.52 (2H, s), 4.12 (3H, s), 7.04 (1H, br), 7.12 (1H, d, J = 9.28 Hz), 7.20 (2H, m), 7.73 (1H, dd, J = 7.81, 1.47 Hz), 7.78 (1H, d, J = 9.16 Hz), 7.76 (1H, m), 8.36 (1H, d, J = 4.52 Hz).
FAB-MSm/z: 383 (M+H)⁺.

[Example 41] N-[(3R)-3-Tetrahydrofuranyl]-1-(6-methoxy-3-pyridyl)-5-(2-pyridyl)-1H-pyrazole-3-carboxamide

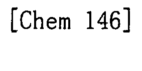

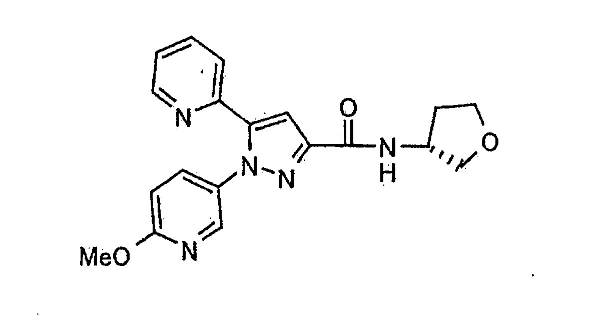

The procedure of Example 1 was repeated by using the 1-(6-methoxy-3-pyridyl)-5-(2-pyridyl)-1H-pyrazole-3-carboxylic acid (296 mg) of Referential Example 4 and (R)-(+)-3-aminotetrahydrofuran p-toluenesulfonate (311 mg) to give the title compound (144 mg, 39%) as a solid.

¹H-NMR (400MHz, CDCl₃)δ: 1.96-1.97 (1H, m), 2.33-2.37 (1H, m), 3.78-3.88 (2H, m), 3.96-4.01 (5H, m), 4.73-4.76 (1H, m), 6.78 (1H, dd, J = 8.8, 0.5 Hz), 7.11 (1H, d, J = 7.6 Hz), 7.21-7.24 (1H, m), 7.62 (1H, dd, J = 8.8, 2.7 Hz), 7.71 (1H, td, J = 7.7, 1.8 Hz), 8.11 (1H, dd, J = 2.7, 0.5 Hz), 8.49 (1H, dq, J = 4.9, 0.9 Hz).
ESI-MSm/z: 366 (M+H)⁺.

| Elementary analysis: for C₁₉H₁₉N₅O₃ | | | |
|---|---|---|---|
| Calculated: | C, 62.46; | H, 5.24; | N, 19.17. |
| Found: | C, 62.44; | H, 5.25; | N, 18.95. |

[Example 42] N-(4-Tetrahydropyranyl)-1-(6-methoxy-3-pyridyl)-5-(2-pyridyl)-1H-pyrazole-3-carboxamide

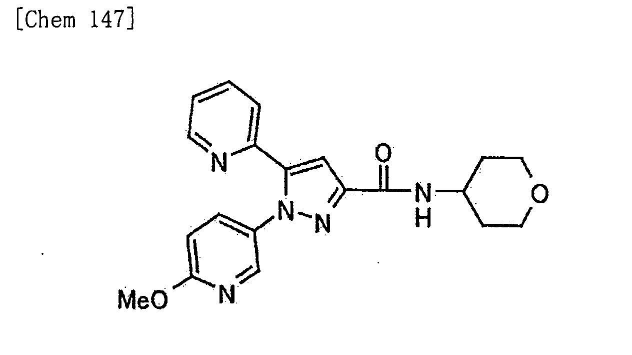

The procedure of Example 1 was repeated by using the 1-(6-methoxy-3-pyridyl)-5-(2-pyridyl)-1H-pyrazole-3-carboxylic acid (296 mg) of Referential Example 4 and 4-aminotetrahydropyran (121 mg) to give the title compound (184 mg, 48%) as a solid.

¹H-NMR (400 MHz, CDCl₃)δ: 1.99-2.05 (2H, m), 3.96 (3H, s), 4.00-4.02 (1H, m), 3.54 (2H, td, J = 11.8, 2.2 Hz), 4.19-4.24 (1H, m), 6.78 (1H, dd, J = 8.7, 0.6 Hz), 6.87 (1H, d, J= 8.1 Hz), 7.22-7.24 (1H, m), 7.44 (1H, dt, J = 7.9, 1.0 Hz), 7.62 (1H, dd, J = 8.7, 2.8 Hz), 7.70-7.72 (1H, m), 8.13-8.15 (1H, m), 8.48-8.50 (1H, m).
ESI-MSm/z: 380 (M+H)⁺.

[Example 43] N-(1-Hydroxymethyl-1-cyclopentyl)-1-(6-methoxy-3-pyridazinyl)-5-(2-pyridyl)-1H-pyrazole-3-carboxamide

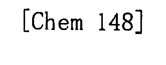

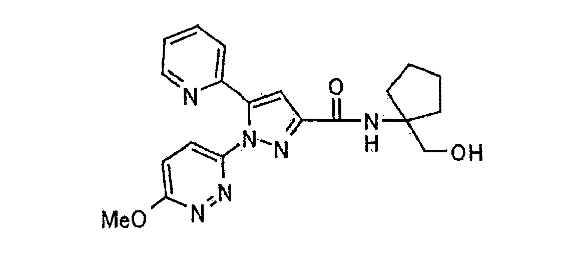

Triethylamine (1.90 ml) and isobutyl chloroformate (1.77 ml) were added to a solution of 1-(tert-butoxycarbonylamino)cyclopentanecarboxylic acid (3.12 g) in tetrahydrofuran (20 ml) at -10°C, and the mixture was stirred for 10 minutes. A solution of sodium borohydride (1.55 g) in water (15 ml) was added to the reaction liquid, and the mixture was stirred for 30 minutes. Water and ethyl acetate were added to the reaction liquid and the phases were separated, and the organic layer was dried over anhydrous magnesium sulfate. After filtration, the solvent was evaporated under reduced pressure, and the residue was purified by column chromatography on silica gel (ethyl acetate-hexane) to give N-(1-hydroxymethyl-1-cyclopentyl)carbamic acid tert-butyl ester (0.750 g, 25%) as a solid. To this butyl ester derivative (0.750 g) was added a 4N solution of hydrochloric acid in dioxane (10 ml), and the mixture was stirred at room temperature for 3.5 hours. The reaction solvent was evaporated under reduced pressure to give 1-hydroxymethylcyclopentylamine hydrochloride. The procedure of Example 1 was repeated by using this amine derivative and the 1-(6-methoxy-3-pyridazinyl)-5-(2-pyridyl)-1H-pyrazole-3-carboxyli c acid (0.696 g) of Referential Example 9 to give the title compound (592 mg, 43%) as an amorphous product.

¹H-NMR (400 MHz, CDCl₃)δ: 1.67-1.94 (6H, m), 1.98-2.05 (2H, m), 3.78 (2H, d, J = 5.9 Hz), 4.13 (3H, s), 4.63 (1H, t, J = 6.0 Hz), 7.14 (1H, d, J = 9.3Hz), 7.15 (1H, br s), 7.19-7.23 (1H, m), 7.23 (1H, s), 7.60-7.62 (1H, m), 7.73 (1H, d, J = 9.3 Hz), 7.75 (1H, ddd, J = 7.8, 7.6, 1.7 Hz), 8.36-8.38 (1H, m).
ESI-MSm/z: 395 (M+H)⁺.

| Elementary analysis: for C₂₀H₂₂N₆O₃ | | | |
|---|---|---|---|
| Calculated: | C, 60.90; | H, 5.62; | N, 21.31. |
| Found: | C, 60.80; | H, 5.53; | N, 21.31. |

[Example 44] N-(Tetrahydro-2-furanylmethyl)-1-(6-methoxy-3-pyridyl)-5-(2-pyridyl)-1H-pyrazole-3-carboxamide

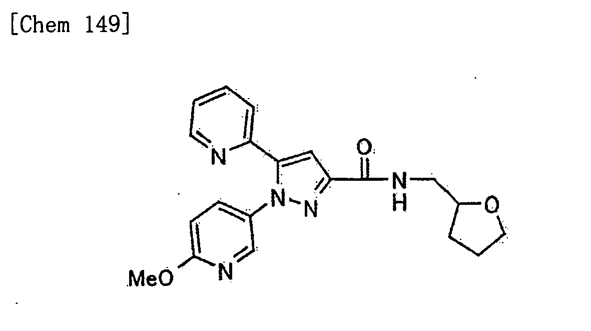

The procedure of Example 1 was repeated by using the 1-(6-methoxy-3-pyridyl)-5-(2-pyridyl)-1H-pyrazole-3-carboxylic acid (0.231 g) of Referential Example 4 and tetrahydro-2-furanylmethylamine (0.121 ml) to give the title compound (250 mg, 84%) as a solid.

¹H-NMR (400MHz, CDCl₃)δ: 1.60-1.69 (1H, m), 1.87-1.95 (2H, m), 1.99-2.07 (1H, m), 3.39-3.45 (1H, m), 3.71-3.80 (2H, m), 3.87-3.93 (1H, m), 3.96 (3H, s), 4.06-4.12 (1H, m), 6.77 (1H, d, J = 8.8 Hz), 7.21-7.28 (3H, m), 7.43 (1H, d, J = 8.1 Hz), 7.62 (1H, dd, J = 8.8, 2.7 Hz), 7.71 (1H, ddd, J = 7.8, 7.6, 1.7 Hz), 8.11 (1H, d, J = 2.9 Hz), 8.49-8.50 (1H, m).
ESI-MSm/z: 380 (M+H)⁺.

[Example 45] N-[(1R,2S)-2-Fluorocyclopropyl]-1-(6-methoxy-3-pyridyl)-5-(2-pyridyl)-1H-pyrazole-3-carboxamide

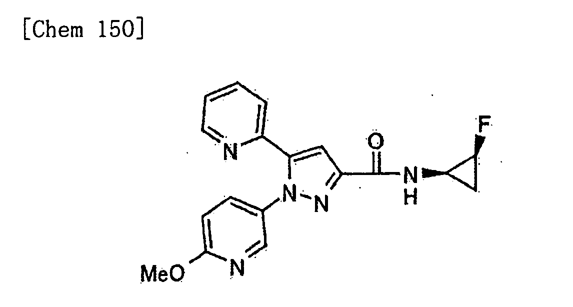

The procedure of Example 7 was repeated by using the 1-(6-methoxy-3-pyridyl)-5-(2-pyridyl)-1H-pyrazole-3-carboxylic acid (250 mg) of Referential Example 4 and (1R, 2S)-2-fluorocyclopropylamine tosylate (250 mg) to give the title compound (80 mg, 27%) as a solid.

¹H-NMR (400 MHz, CDCl₃)δ: 1.03 (0.5H, m), 1.10 (0.5H, m), 1.26 (1H, m), 3.05 (1H, q, J = 5.62 Hz), 3.96 (3H, s), 4.66 (0.5H, dt, J = 5.62, 3.05 Hz), 4.82 (0.5H, dt, J = 5.62, 3.05 Hz), 6.77 (1H, dd, J = 8.79, 0.49 Hz), 7.15 (1H, br), 7.23 (1H, m), 7.27 (1H, s), 7.43 (1H, d, J = 7.94 Hz), 7. 61 (1H, dd, J = 8.79, 2. 69 Hz), 7. 71 (1H, dt, J = 7.94, 1.71 Hz), 8.11 (1H, d, J = 2.21 Hz), 8.49 (1H, d, J = 4.76 Hz).

| Elementary analysis: for C₁₈H₁₆N₅O₂F | | | |
|---|---|---|---|
| Calculated: | C, 61.18; | H, 4.56; | N, 19.82. |
| Found: | C, 61.12; | H, 4.63; | N, 19.68. |

[Example 46] N-(1-Carbamoyl-1-methylethyl)-1-(6-methoxy-3-pyridyl)-5-(2-pyridyl)-1H-pyrazole-3-carboxamide

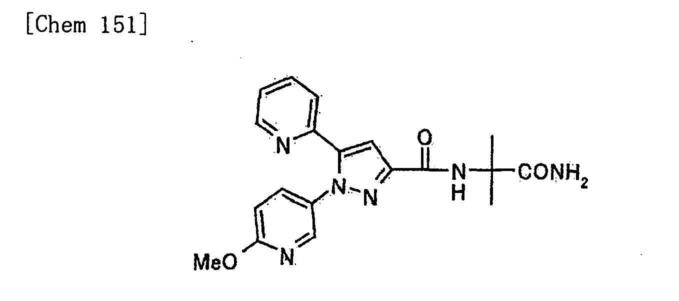

### 1) N-[1-(6-Methoxy-3-pyridyl)-5-(2-pyridyl)-1H-pyrazole-3-carbonyl]-1,1-dimethylglycine ethyl ester

The procedure of Example 7 was repeated by using the 1-(6-methoxy-3-pyridyl)-5-(2-pyridyl)-1H-pyrazole-3-carboxylic acid (3.0 g) of Referential Example 4 and 2,2-dimethylglycine ethyl ester hydrochloride (1.9 g) to give N-[1-(6-methoxy-3-pyridyl)-5-(2-pyridyl)-1H-pyrazole-3-carbonyl]-1,1-dimethylglycine ethyl ester (3.8 g, 91.6%) as an oily product.

¹H-NMR (400 MHz, CDCl₃)δ: 1.29 (3H, t, J = 7.1 Hz), 1.68 (6H, s), 3.93 (3H, s), 4.24 (2H, q, J = 7.1 Hz), 6.77 (1H, d, J = 8.8 Hz), 7.20-7.26 (1H, m), 7.22 (1H, s), 7.41-7.45 (1H, m), 7.63 (1H, dd, J = 8.8, 2.9 Hz), 7.69-7.73 (1H, m), 8.12 (1H, d, J = 2.7 Hz), 8.48-8.50 (1H, m). EI-MSm/z: 409 (M⁺).

### 2) N-[1-(6-Methoxy-3-pyridyl)-5-(2-pyridyl)-1H-pyrazole-3-carbonyl]-1,1-dimethylglycine

Lithium hydroxide monohydrate (410 mg) was added to a solution of the N-[1-(6-methoxy-3-pyridyl)-5-(2-pyridyl)-1H-pyrazole-3-carbonyl]-1,1-dimethylglycine ethyl ester (2.0 g) in tetrahydrofuran (95 ml) and water (25 ml), and the mixture was stirred at room temperature for 24 hours. The reaction liquid was neutralized by adding conc. hydrochloric acid, and dichloromethane was added and the phases were separated. The organic layer was dried over anhydrous magnesium sulfate. After filtration, the solvent was evaporated under reduced pressure to give N-[1-(6-methoxy-3-pyridyl)-5-(2-pyridyl)-1H-pyrazole-3-carbonyl]-1,1-dimethylglycine(1.8 g, 95%).

¹H-NMR (400 MHz, CDCl₃)δ: 1.71 (3H, s), 3.96 (3H, s), 6.77 (1H, d, J = 9.0 Hz), 7.36 (1H, brs), 7.42 (1H, d, J = 7.8 Hz), 7.61 (1H, dd, J = 8.8, 2.7 Hz), 8.13 (1H, d, J = 2.7 Hz), 8.51 (1H, d, J = 4.6 Hz). EI-MSm/z: 381 (M⁺).

### 3) The title compound

The procedure of Example 7 was repeated by using N-[1-(6-methoxy-3-pyridyl)-5-(2-pyridyl)-1H-pyrazole-3-carbonyl]-1, 1-dimethylglycine (350 mg) and 28% aqueous ammonia (0. 6 ml) to give the title compound (220 mg, 62%) as a solid.

¹H-NMR (400 MHz, CDCl₃)δ: 1.70 (6H, s), 3.97 (3H, s), 6.78 (1H, d, J = 8.8 Hz), 7.22-7.29 (2H, m), 7.42 (1H, d, J = 7.8 Hz), 7.62 (1H, dd, J = 8.8, 2.4 Hz), 7.69-7.73 (1H, m), 8.12 (1H, d, J = 2.7 Hz), 8.50 (1H, d, J = 4.9 Hz).
EI-MSm/z: 380 (M⁺).

[Example 47] N-Carbamoylmethyl-N-tert-butyl-1-(6-methoxy-3-pyridyl)-5-(2-pyridyl)-1H-pyrazole-3-carboxamide

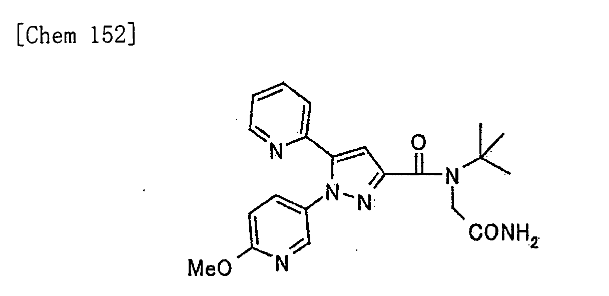

The procedure of Example 7 was repeated by using the 1-(6-methoxy-3-pyridyl)-5-(2-pyridyl)-1H-pyrazole-3-carboxylic acid (230 mg) of Referential Example 4 and 1-tert-butylaminoacetamide (100 mg) of Referential Example 28 to give the title compound (190 mg, 60%) as a solid.

¹H-NMR (400 MHz, CDCl₃)δ: 1.57 (9H, s), 3.94 (3H, s), 6.73-6.75 (1H, m), 7.08-7.28 (2H, m), 7.41-7.76 (3H, m), 8.05-8.52 (1H, m).
EI-MSm/z: 408 (M⁺).

| Elementary analysis: for C₂₁H₂₄N₆O₃ 0.25H₂O | | | |
|---|---|---|---|
| Calculated: | C, 61.08; | H, 5.98; | N, 20.35. |
| Found: | C, 60.96; | H, 5.86; | N, 20.63. |

[Example 48] N-(1-Carbamoyl-1-cyclopentyl)-1-(6-methoxy-3-pyridyl)-5-(2-pyridyl)-1H-pyrazole-3-carboxamide

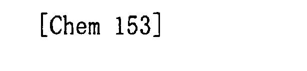

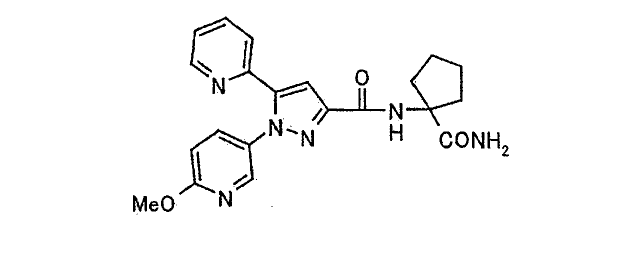

The procedure of Example 7 was repeated by using the 1-(6-methoxy-3-pyridyl)-5-(2-pyridyl)-1H-pyrazole-3-carboxylic acid (306 mg) of Referential Example 4 and 1-amino-1-cyclopentanecarboxamide trifluoroacetate (250 mg) of Referential Example 26 to give the title compound (390 mg, 92%) as a solid.

¹H-NMR (400MHz, CDCl₃)δ: 1.75-1.90 (4H, m), 2.15-2.20 (2H, m), 2.40-2.50 (2H, m), 3.97 (3H, s), 5.35 (1H, br s), 6.78 (1H, dd, J = 8.8, 0.5 Hz), 7.01 (1H, br), 7.17-7.26 (3H, m), 7.41-7.43 (1H, m), 7.59-7.61 (1H, m), 7.69-7.74 (1H, m), 8.12-8.13 (1H, m), 8.48-8.51 (1H, m).
EI-MSm/z: 406 (M⁺).

| Elementary analysis: for C₂₁H₂₂N₆O₃·0.25H₂O | | | |
|---|---|---|---|
| Calculated: | C, 61.38; | H, 5.52; | N, 20.45. |
| Found: | C, 61.12; | H, 5.38; | N, 20.18. |

[Example 49] N-Carbamoylmethyl-N-(tert-butyl)-1-(5-methoxy-2-pyridyl)-5-phenyl-1H-pyrazole-3-carboxamide

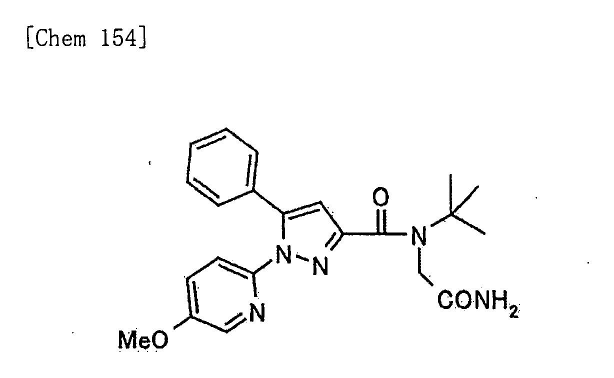

The procedure of Example 15(3) was repeated by using the 1-(5-methoxy-2-pyridyl)-5-phenyl-1H-pyrazole-3-carboxylic acid(230 mg) of Referential Example 7 and the 1-tert-butylaminoacetamide (100 mg) of Referential Example 28 to give the title compound (175 mg, 52%) as an amorphous product.

¹H-NMR (400 MHz, CDCl₃)δ: 1.57 (9H, s), 3.87 (3H, s), 4.27 (1.5H, br s), 6.88 (1H, s), 7.18-7.37 (7H, m), 8.00 (1H, d, J = 2.9 Hz).
EI-MSm/z: 407 (M⁺).

[Example 50] N-(cis-2-Carbamoyl-1-cyclopentyl)-1-(6-methoxy-3-pyridyl)-5-(2-pyridyl)-1H-pyrazole-3-carboxamide

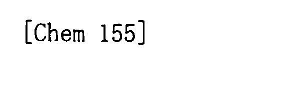

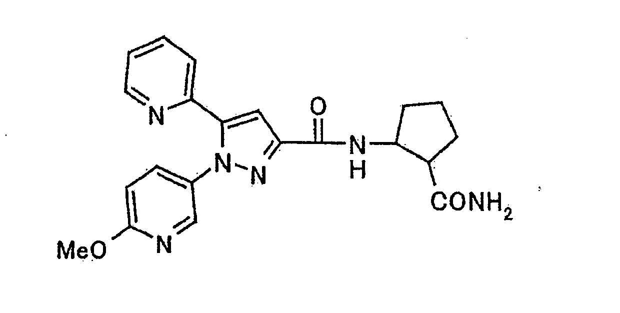

The procedure of Example 1 was repeated by using the 1-(6-methoxy-3-pyridyl)-5-(2-pyridyl)-1H-pyrazole-3-carboxylic acid (296 mg) of Referential Example 4 and (±)-cis-2-aminocyclopentanecarboxamide (153.8 mg) to give the title compound (137 mg, 34%) as a solid.

¹H-NMR (400 MHz, CDCl₃)δ: 1.83-2.17 (6H, m), 3.09 (1H, q, J = 7.5 Hz), 3.95 (3H, s), 4.62-4.64 (1H, m), 5.26 (1H, br s), 5.80 (1H, br s), 6.77 (1H, d, J = 8.8 Hz), 7.23-7.25 (1H, m), 7.37-7.39 (2H, m), 7.62-7.67 (1H, m), 7.65-7.72 (1H, m), 8.08 (1H, d, J = 3.4 Hz), 8.50-8.52 (1H, m),
ESI-MSm/z: 406 (M+H)⁺.

[Example 51] N-Cyclobutyl-1-(6-methoxy-3-pyridyl)-5-(2-pyridyl)-1H-pyrazole-3-carboxamide

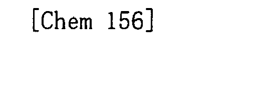

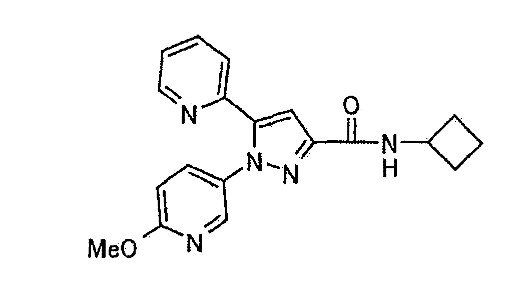

The procedure of Example 1 was repeated by using the 1-(6-methoxy-3-pyridyl)-5-(2-pyridyl)-1H-pyrazole-3-carboxylic acid (296 mg) of Referential Example 4 and cyclobutylamine (85.3 mg) to give the title compound (230 mg, 65%) as a solid.

¹H-NMR (400MHz, CDCl₃)δ: 1.74-1.77 (2H, m), 1.97-2.07 (2H, m), 2.39-2.46 (2H, m), 3.96 (3H, s), 4.59-4.63 (1H, m), 6.78 (1H, d, J = 8.8 Hz), 7.09 (1H, d, J = 8.1 Hz), 7.21-7.26 (1H, m), 7.43-7.45 (1H, m), 7.62 (1H, dd, J = 8.8, 2.7 Hz), 7.71 (1H, td, J = 7.7, 1.8 Hz), 8.13 (1H, d, J = 2.7 Hz), 8.48-8.49 (1H, m).
ESI-MSm/z: 350 (M+H)⁺.

[Example 52] N-(exo-2-Bicyclo [2.2.1]heptyl)-1-(6-methoxy-3-pyridyl)-5-(2-pyridyl)-1H-pyrazole-3-carboxamide

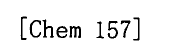

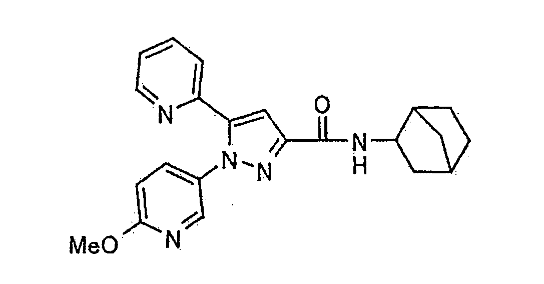

The procedure of Example 1 was repeated by using the 1-(6-methoxy-3-pyridyl)-5-(2-pyridyl)-1H-pyrazole-3-carboxylic acid (296 mg) of Referential Example 4 and exo-2-aminobicyclo [2.2.1]heptane hydrochloride (177 mg) to give the title compound (145 mg, 37%) as a solid.

¹H-NMR (400 MHz, CDCl₃)δ: 0.89-0.93 (1H, m), 1.30-1.67 (11H, m), 2.19-2.24 (2H, m), 2.56-2.58 (1H, m), 3.96 (3H, d, J = 2.0 Hz), 6.78 (1H, d, J = 8.8 Hz), 7.01 (1H, d, J = 7.4 Hz), 7.20-7.26 (1H, m), 7.43-7.45 (1H, m), 7.62 (1H, dd, J = 8.8, 2.9 Hz), 7.70-7.72 (1H, m), 8.15 (1H, d, J = 2.0 Hz), 8.48-8.49 (1H, m).
ESI-MSm/z: 390 (M+H)⁺.

[Example 53] N-Cyclohexylmethyl-1-(6-methoxy-3-pyridyl)-5-(2-pyridyl)-1H-pyrazole-3-carboxamide

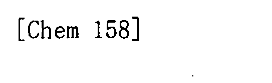

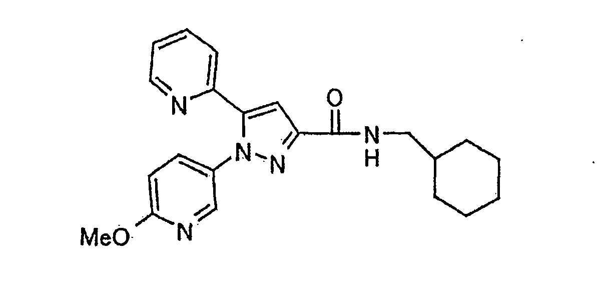

The procedure of Example 1 was repeated by using the 1-(6-methoxy-3-pyridyl)-5-(2-pyridyl)-1H-pyrazole-3-carboxylic acid (296 mg) of Referential Example 4 and cyclohexylmethylamine (156 µl) to give the title compound (188 mg, 48%) as a solid.

¹H-NMR (400MHz, CDCl₃)δ: 0.96-1.04 (2H, m), 1.12-1.30 (4H, m), 1.61-1.76 (5H, m), 3.30 (2H, t, J = 6.6 Hz), 3.94 (3H, s), 6.77 (1H, d, J = 8.8 Hz), 7.01-7.02 (1H, m), 7.21-7.31 (1H, m), 7.44-7.45 (1H, m), 7.61 (1H, dd, J = 8.8, 2.5 Hz), 7.71 (1H, td, J = 7.7, 1.8 Hz), 8.13 (1H, d, J = 2.5 Hz), 8.48-8.49 (1H, m).
ESI-MSm/z: 392 (M+H)⁺.

[Example 54] N-Methyl-N-tert-butyl-1-(6-methoxy-3-pyridazinyl)-5-(2-pyridyl)-1H-pyrazole-3-carboxamide

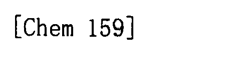

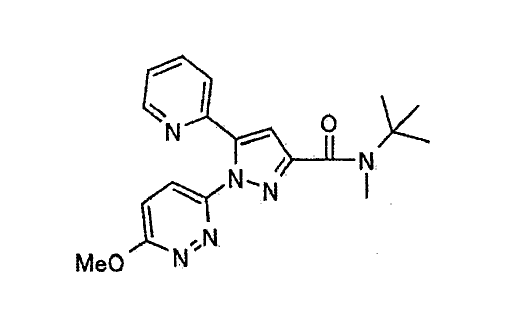

The procedure of Example 7 was repeated by using the 1-(6-methoxy-3-pyridazinyl)-5-(2-pyridyl)-1H-pyrazole-3-carboxyli c acid (300 mg) of Referential Example 9 and N-tert-butylmethylamine (145 µl) to give the title compound (200 mg, 54%) as a solid.

¹H-NMR (400 MHz, CDCl₃)δ: 1.54 (9H, s), 1.62 (3H, s), 3.19 (3H, s), 4.10 (3H, s), 6.99 (1H, s), 7.12 (1H, d, J = 9.03 Hz), 7.21 (1H, dd, J = 7.81, 4.64 Hz), 7.55 (1H, d), J = 8.06 Hz), 7.74 (1H, dt, J = 7.81, 1.71 Hz), 7.83 (1H, d, J = 9.03 Hz), 8.41 (1H, d, J = 4.64 Hz).
FAB-MSm/z: 367 (M+H)⁺.

[Example 55] N-Neopentyl-1-(6-methoxy-3-pyridazinyl)-5-(2-pyridyl)-1H-pyrazole-3-carboxamide

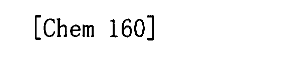

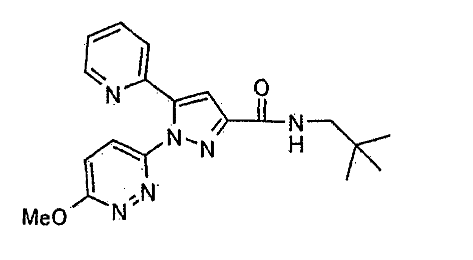

The procedure of Example 7 was repeated by using the 1-(6-methoxy-3-pyridazinyl)-5-(2-pyridyl)-1H-pyrazole-3-carboxyli c acid (200 mg) of Referential Example 9 and neopentylamine (59 mg) to give the title compound (155 mg, 58%) as a solid.

¹H-NMR (400 MHz, CDCl₃)δ: 0.99 (9H, s), 3.27 (2H, d, J = 6.6 Hz), 4.128 (1/2 x 3H, s), 4.130 (1/2 x 3H, s), 7.04-7.09 (1H, m), 7.14 (1H, dd, J = 9.3, 0.5 Hz), 7.19-7.30 (1H, m), 7.62 (1H, d, J = 7.8 Hz), 7.73-7.78 (2H, m), 8.37-8.38 (1H, m).
EI-MSm/z: 366 (M⁺).

[Example 56] N-Cyclopentyl-1-(6-methoxy-3-pyridazinyl)-5-(2-pyridyl)-1H-pyrazole-3-carboxamide

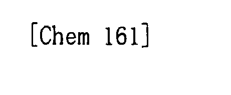

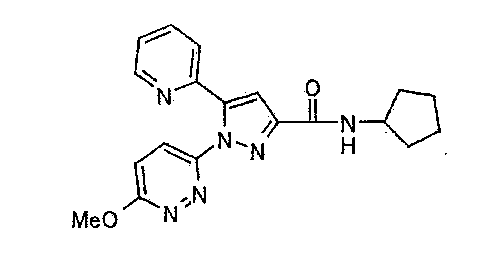

The procedure of Example 7 was repeated by using the 1-(6-methoxy-3-pyridazinyl)-5-(2-pyridyl)-1H-pyrazole-3-carboxyli c acid (297 mg) of Referential Example 9 and cyclopentylamine (109 µl) to give the title compound (274 mg, 75%) as a solid.

¹H-NMR (400 MHz, CDCl₃)δ: 1.50-1.75 (6H, m), 2.05-2.13 (2H, m), 4.13 (3H, s), 4.42 (1H, q, J = 7.2 Hz), 6.90 (1H, d, J = 10.0 Hz), 7.13 (1H, d, J= 9.3 Hz), 7.20-7.21 (1H, m), 7.26 (1H, s), 7.61 (1H, d, J= 7.8 Hz), 7.74-7.76 (2H, m), 8.37 (1H, d, J = 3.9 Hz).
ESI-MSm/z: 365 (M+H)⁺.

[Example 57] N-Phenyl-1-(6-methoxy-3-pyridyl)-5-(2-pyridyl)-1H-pyrazole-3-carboxamide

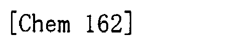

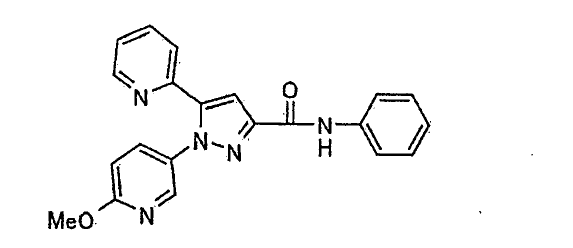

The procedure of Example 1 was repeated by using the 1-(6-methoxy-3-pyridyl)-5-(2-pyridyl)-1H-pyrazole-3-carboxylic acid (296 mg) of Referential Example 4 and aniline (311 mg) to give the title compound (158 mg, 43%) as a solid.

¹H-NMR (400MHz, CDCl₃)δ: 3.98 (3H, s), 6.80 (1H, d, J=8.6Hz), 7.12-7.16 (1H, m), 7.22-7.30 (1H, m), 7.36-7.39 (3H, m), 7.46-7.52 (1H, m), 7.65 (1H, dd, J = 8.8, 2.7 Hz), 7.70-7.76 (3H, m), 8.17 (1H, d, J = 2.7 Hz), 8.51-8.52 (1H, m), 8.77 (1H, s).
ESI-MSm/z: 372 (M+H)⁺.

[Example 58] N-(2-Pyridyl)-1-(6-methoxy-3-pyridyl)-5-(2-pyridyl)-1H-pyrazole-3-carboxamide

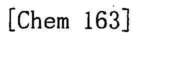

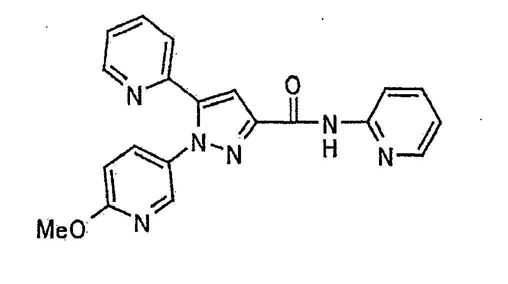

The procedure of Example 1 was repeated by using the 1-(6-methoxy-3-pyridyl)-5-(2-pyridyl)-1H-pyrazole-3-carboxylic acid (296 mg) of Referential Example 4 and 2-aminopyridine (311 mg) to give the title compound (178 mg, 48%) as a solid.

¹H-NMR (400 MHz, CDCl₃)δ: 3.96 (3H, s), 6.80 (1H, d, J = 8.8 Hz), 7.07 (1H, ddd, J= 7.4, 4. 9, 1. 0 Hz), 7.24-7.29 (1H, m), 7.35 (1H, s), 7.44-7.46 (1H, m), 7.69-7.75 (3H, m), 8.11 (1H, d, J = 2.9 Hz), 8.33-8.34 (1H, m), 8.38-8.40 (1H, m), 8.53-8.54 (1H, m), 9.42 (1H, s).
ESI-MSm/z: 373 (M+H)⁺.

[Example 59] N-Cyclopentyl-5-(5-chloro-2-pyridyl)-1-(3-pyridyl)-1H-pyrazole-3-carboxamide

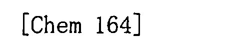

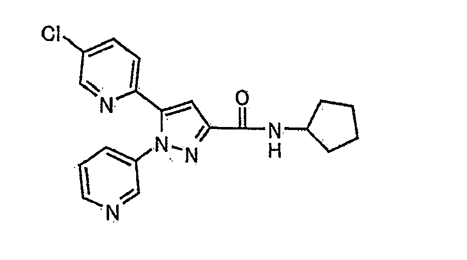

The procedure of Example 1 was repeated by using the 5-(5-chloro-2-pyridyl)-1-(3-pyridyl)-1H-pyrazole-3-carboxylic acid (300 mg) of Referential Example 25 and cyclopentylamine (150 µl) to give the title compound (127 mg, 35%) as a solid.

¹H-NMR (400 MHz, CDCl₃)δ: 1.53-1.75 (6H, m), 2.09 (2H, m), 4.42 (1H, q, J = 7.57 Hz), 6.89 (1H, d, J = 7.57 Hz), 7.28 (1H, s), 7.39 (1H, dd, J = 8.18, 4.76 Hz), 7.48 (1H, dd, J = 8.42, 0.61 Hz), 7.72 (1H, dd, J = 8.42, 2.44 Hz), 7.75 (1H, dd, J = 8.18, 2.44 Hz), 8.37 (1H, dd, J = 2.56, 0.61 Hz), 8.56 (1H, d, J = 2.56 Hz), 8.63 (1H, dd, J = 4.76, 1.47 Hz).
FAB-MSm/z: 368 (M+H)⁺.

[Example 60] N-tert-Butyl-1-(6-methoxy-3-pyridyl)-5-(1H-pyrrol-2-yl)-1H-pyrazole-3-carboxamide

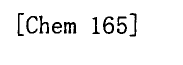

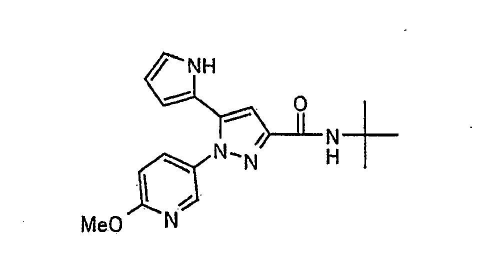

The procedure of Example 1 was repeated by using the 1-(6-methoxy-3-pyridyl)-5-(1H-pyrrol-2-yl)-1H-pyrazole-3-carboxyl ic acid (0.222 g) of Referential Example 46 and tert-butylamine (0.148 ml) to give the title compound (220 mg, 83%) as a solid.

¹H-NMR (400 MHz, CDCl₃)δ: 1.49 (9H, s), 4.00 (3H, s), 5.80-5.82 (1H, m), 6.13-6.15 (1H, m), 6.83 (1H, d, J = 8.8 Hz), 6.84-6.86 (2H, m), 7.13 (1H, s), 7.60 (1H, dd, J = 8.8, 2.4 Hz), 8.28 (1H, d, J = 2.7 Hz), 9.01 (1H, br s).
ESI-MSm/z: 340 (M+H)⁺.

[Example 61] N-(2,2-Dimethylpropyl)-5-(5-cyano-2-pyridyl)-1-(3-pyridyl)-1H-pyrazole-3-carboxamide

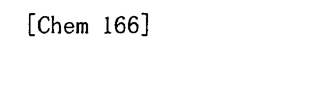

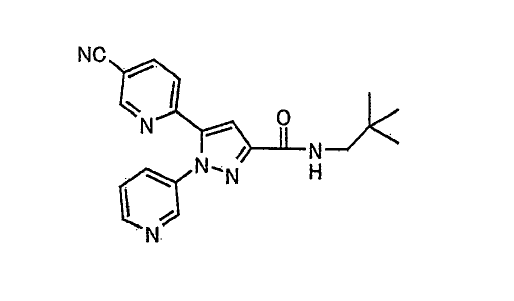

The procedure of Example 1 was repeated by using the 5-(5-cyano-2-pyridyl)-1-(3-pyridyl)-1H-pyrazole-3-carboxylic acid (0.350 g) of Referential Example 73 and neopentylamine (0.212 ml) to give the title compound (0.395 g, 91%) as a solid.

¹H-NMR (400 MHz, CDCl₃)δ: 0.99 (9H, s), 3.28 (2H, d, J = 6.6 Hz), 7.04 (1H, t, J= 6.6 Hz), 7.40-7.44 (2H, m), 7.68 (1H, dd, J= 8.3, 1.0 Hz), 7.74-7.77 (1H, m), 8.01 (1H, dd, J = 8.3, 2.2Hz), 8.57 (1H, m), 8.64-8.65 (1H, m), 8.67 (1H, dd, J = 4.9, 1.5 Hz).
EI-MSm/z: 360 (M⁺).

[Example 62] N-(2,2-Dimethylpropyl)-5-(5-carbamoyl-2-pyridyl)-1-(3-pyridyl)-1H-pyrazole-3-carboxamide

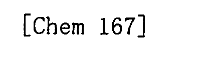

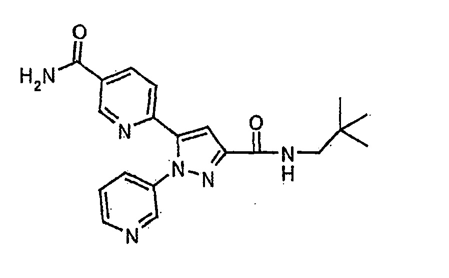

The procedure of Example 21 was repeated by using the N-(2,2-dimethylpropyl)-5-(5-cyano-2-pyridyl)-1-(3-pyridyl)-1H-pyr azole-3-carboxamide (0.333 g) of Example 61 to give the title compound (0.250 g, 71%) as a solid.

¹H-NMR (400 MHz, DMSO-d₆)δ: 0.91 (9H, s), 3.13 (2H, d, J = 6.6 Hz), 7.45 (1H, s), 7.52 (1H, dd, J = 8.1, 4.9 Hz), 7.63 (1H, br s), 7.84-7.89 (2H, m), 8.15 (1H, br s), 8.19 (1H, t, J = 6.6 Hz), 8.28-8.30 (1H, m), 8.61-8.63 (2H, m), 8.81 (1H, d, J = 2.2 Hz).
EI-MSm/z: 378 (M⁺).

[Example 63] N-tert-Butyl-5-(5-methyl-2-pyridyl)-1-(2-pyridyl)-1H-pyrazole-3-carboxamide

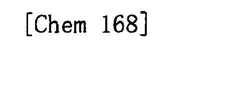

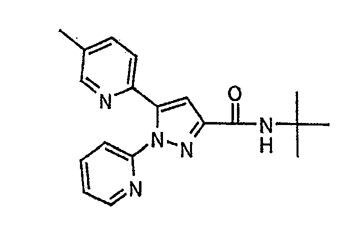

The procedure of Example 7 of was repeated by using the 5-(5-methyl-2-pyridyl)-1-(2-pyridyl)-1H-pyrazole-3-carboxylic acid (1.17 g) of Referential Example 35 and tert-butylamine (0.9 ml) to give the title compound (501 mg, 36%) as a solid.

¹H-NMR (400 MHz, CDCl₃)δ: 1.49 (9H, s), 2.33 (3H, s), 6.89 (1H, br), 7.16 (1H, s), 7.28 (1H, m), 7.38 (1H, d, J = 7.94 Hz), 7.53 (2H, m), 7.81 (1H, dt, J = 7.94, 1.83 Hz), 8. 24 (1H, s), 8.35 (1H, ddd, J = 4.76, 1.83, 0.73 Hz).
FAB-MSm/z: 336 (M+H)⁺.

[Example 64] N-(1-Hydroxymethylcyclohexan-1-yl)-1-(6-methoxy-3-pyridazinyl)-5-(2-pyridyl)-1H-pyrazole-3-carboxamide

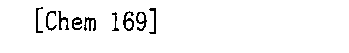

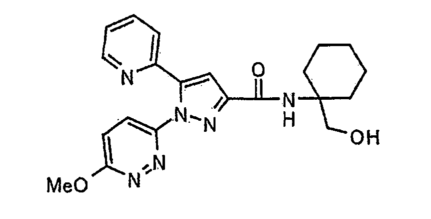

Triethylamine (1.40ml) and isobutylchloroformate (1.30ml) were added to a solution of 1-[(tert-butoxycarbonyl)amino]cyclohexanecarboxylic acid (2.43 g) in tetrahydrofuran (20 ml) at -10°C, and the mixture was stirred for 20 minutes. A solution of sodium borohydride (1.14 g) in water (15 ml) was added to the reaction liquid, and the mixture was stirred for 1.5 hours. Water and ethyl acetate were added to the reaction liquid and the phases were separated, and the organic layer was dried over anhydrous magnesium sulfate. After filtration, the solvent was evaporated under reduced pressure, and the residue was purified by column chromatography on silica gel (ethyl acetate-n-hexane) to give 1-(hydroxymethyl)cyclohexylcarbamic acid tert-butyl ester (1.50 g, 65%) as a solid. This carbamic acid derivative (1.50 g) was dissolved in a 4N solution of hydrochloric acid in 1,4-dioxane (20 ml), and the mixture was stirred at room temperature for 7.5 hours. The reaction solvent was evaporated under reduced pressure, and to the residue were added the 1-(6-methoxy-3-pyridazinyl)-5-(2-pyridyl)-1H-pyrazole-3-carboxyli c acid (1.30 g) of Referential Example 9, 1-hydroxybenzotriazole (0.803 g), and N,N-dimethylformamide (30 ml). Next, triethylamine (1.83 ml) and 1-(3-dimethylaminopropyl)-3-ethylcarbodiimide hydrochloride (1.01 g) were added to this suspension at room temperature, and the mixture was stirred for 18.5 hours. The reaction solvent was evaporated under reduced pressure, and ethyl acetate and saturated aqueous sodium bicarbonate were added to the residue and the phases were separated. The aqueous layer was extracted with ethyl acetate, and the combined organic layers were dried over anhydrous sodium sulfate. After filtration, the solvent was evaporated under reduced pressure, and the residue was purified by column chromatography on silica gel (hexane-ethyl acetate) to give the title compound (991 mg, 36%) as a solid.

¹H-NMR (400 MHz, CDCl₃) δ: 1.38-1.66 (8H, m), 1.96-2.02 (2H, m), 3.79 (2H, d, J = 6.1 Hz), 4.13 (3H, s), 4.84 (1H, t, J = 6.3 Hz), 7.07 (1H, br s), 7.15 (1H, d, J = 9.3 Hz), 7.20-7.23 (2H, m), 7.61 (1H, d, J = 7.6 Hz), 7.74-7.78 (2H, m), 8.36-8.38 (1H, m).
ESI-MSm/z: 409 (M+H)⁺.

[Example 65] N-tert-Butyl-5-(5-methyl-2-pyrazinyl)-1-(3-pyridyl)-1H-pyrazole-3-carboxamide

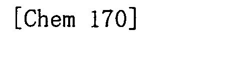

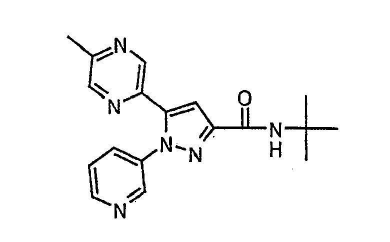

The procedure of Example 1 was repeated by using the 5-(5-methyl-2-pyrazinyl)-1-(3-pyridyl)-1H-pyrazole-3-carboxylic acid (0.250 g) of Referential Example 36 and tert-butylamine (0.186 ml) to give the title compound (0.198 g, 65%) as a solid.

¹H-NMR (400 MHz, CDCl₃)δ: 1.49 (9H, s), 2.57 (3H, s), 6.83 (1H, br s), 7.30 (1H, s), 7.37-7.41 (1H, m), 7.75-7.78 (1H, m), 8.27-8.28 (1H, m), 8.56 (1H, d, J = 2.7 Hz), 8.63 (1H, dd, J = 4.9, 1.5 Hz), 8.67 (1H, d, J = 1.5 Hz).
EI-MSm/z: 336 (M⁺).

[Example 66] N-tert-Butyl-5-(5-chloro-2-pyridyl)-1-(2-pyridyl)-1H-pyrazole-3-carboxamide

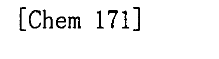

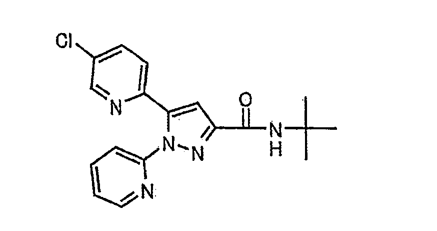

The procedure of Example 1 was repeated by using the 5-(5-chloro-2-pyridyl)-1-(2-pyridyl)-1H-pyrazole-3-carboxylic acid (939 mg) of Referential Example 38 and tert-butylamine (0.65 ml) to give the title compound (288 mg, 26%) as a solid.

¹H-NMR (400 MHz, CDCl₃)δ: 1.49 (9H, s), 6.87 (1H, br), 7.18 (1H, s), 7.29 (1H, m), 7.45 (1H, d, J = 8.42 Hz), 7.62 (1H, dd, J = 8.06, 0.98 Hz), 7.70 (1H, dd, J = 8.42, 2.44 Hz), 7.85 (1H, dt, J = 8.06, 1.83 Hz), 8.32 (1H, td, J = 4.76, 0.98 Hz), 8.35 (1H, d, J = 2.44 Hz).
FAB-MSm/z: 356 (M+H)⁺.

[Example 67] N-tert-Butyl-5-(5-methyl-2-pyridyl)-1-(3-pyridyl)-1H-pyrazole-3-carboxamide

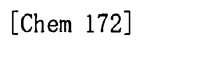

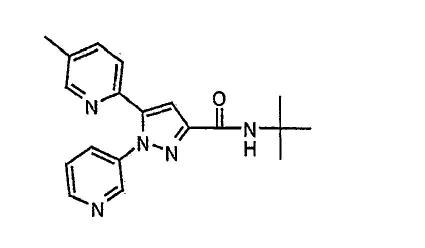

The procedure of Example 1 was repeated by using the 5-(5-methyl-2-pyridyl)-1-(3-pyridyl)-1H-pyrazole-3-carboxylic acid (665 mg) of Referential Example 37 and tert-butylamine (0.5 ml) to give the title compound (110 mg, 14%) as a solid.

¹H-NMR (400 MHz, CDCl₃)δ: 1.49 (9H, s), 2.33 (3H, s), 6.84 (1H, br), 7.19 (1H, s), 7.36 (1H, ddd, J = 8.18, 4.76, 0.73 Hz), 7.39 (1H, d, J = 7.93 Hz), 7.53 (1H, ddd, J = 7.93, 2.20, 0.73 Hz), 7.76 (1H, ddd, J = 8.18, 2.56, 1.47 Hz), 8.27 (1H, d, J = 2.20 Hz), 8.53 (1H, d, J = 2.56 Hz), 8.58 (1H, dd, J = 4.76, 1. 47 Hz).
FAB-MSm/z: 336 (M+H)⁺.

[Example 68] N-tert-Butyl-5-(5-cyano-2-pyridyl)-1-(2-pyridyl)-1H-pyrazole-3-carboxamide

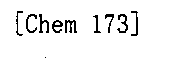

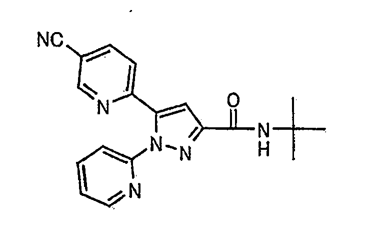

The procedure of Example 7 was repeated by using the 5-(5-cyano-2-pyridyl)-1-(2-pyridyl)-1H-pyrazole-3-carboxylic acid (339 mg) of Referential Example 39 and tert-butylamine (0.135 ml) to give the title compound (203 mg, 50%) as a solid.

¹H-NMR (400 MHz, CDCl₃)δ: 1.49 (9H, s), 6.86 (1H, s), 7.27 (1H, s), 7.32 (1H, t, J = 6. 2 Hz), 7. 62 (1H, dd, J = 8.2, 0.9 Hz), 7.72 (1H, d, J = 8.1 Hz), 7.88-7.92 (1H, m), 7.99 (1H, dd, J = 8.2, 2.1 Hz), 8.24-8.27 (1H, m), 8.65 (1H, d, J = 1.2 Hz).
ESI-MSm/z: 347 (M+H)⁺.

[Example 69] N-tert-Butyl-5-(5-cyano-2-pyridyl)-1-phenyl-1H-pyrazole-3-carboxamide

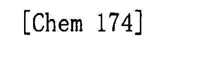

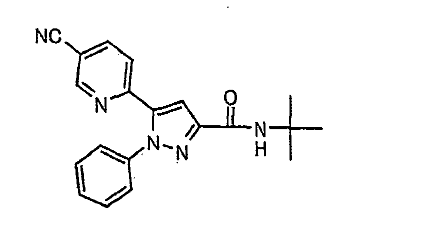

The procedure of Example 7 was repeated by using the 5-(5-cyano-2-pyridyl)-1-phenyl-1H-pyrazole-3-carboxylic acid (940 mg) of Referential Example 40 and tert-butylamine (0.374 ml) to give the title compound (629 mg, 56%) as a solid.

¹H-NMR (400 MHz, CDCl₃)δ: 1.48 (9H, s), 6.84 (1H, s), 7.30-7.45 (7H, m), 7.90 (1H, dd, J = 8.2, 2.1 Hz), 8.73 (1H, s).
ESI-MSm/z: 346 (M+H)⁺.

[Example 70] N-tert-Butyl-5-[5-(tert-butoxycarbonylamino)-2-pyridyl]-1-(3-pyridyl)-1H-pyrazole-3-carboxamide

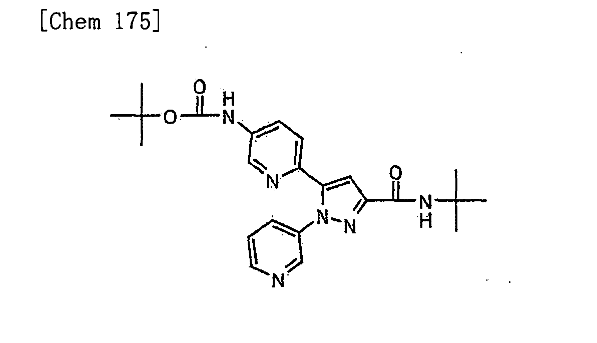

To a solution of the 5-[5-(tert-butoxycarbonylamino)-2-pyridyl]-1-(3-pyridyl)-1H-pyraz ole-3-carboxylic acid (250 mg) of Referential Example 41, tert-butylamine (138 µl), 1-hydroxybenzotriazole (88 mg), and triethylamine (365 µl) in dichloromethane (10 ml) was added 1-ethyl-3-(3-dimethylaminopropyl)carbodiimide hydrochloride (251 mg) at room temperature, and the mixture was stirred for 40 hours. Dichloromethane (10 ml), N, N-dimethylformamide (6 ml), triethylamine (365 µl), and tert-butylamine (138 µl) were further added to the reaction liquid, and the mixture was stirred for 96 hours. The dichloromethane in the reaction liquid was evaporated under reduced pressure, and water and ethyl acetate were added to the residue and the phases were separated, and the organic layer was washed with water and brine, and dried over anhydrous sodium sulfate. After filtration, the solvent was evaporated under reduced pressure, and the residue was purified by thin layer chromatography on silica gel (methanol-chloroform) to give the title compound (273 mg, 95%) as an oily product.

¹H-NMR (400 MHz, CDCl₃)δ: 1.49 (9H, s), 1.52 (9H, s), 6.56 (1H, br s), 6.83 (1H, br s), 7.17 (1H, s), 7.34 (1H, ddd, J = 8.3, 4.6, 0.7 Hz), 7.43 (1H, d, J = 8.5 Hz), 7.72 (1H, ddd, J = 8.3, 2.4, 1.7 Hz), 7.99-8.05 (1H, m), 8.25 (1H, d, J = 2.4 Hz), 8.56 (1H, d, J = 2.4 Hz), 8.58 (1H, dd, J = 4.9, 1.5 Hz).
ESI-MSm/z: 437 (M+H)⁺.

[Example 71] N-tert-Butyl-5-(5-amino-2-pyridyl)-1-(3-pyridyl)-1H-pyrazole-3-carboxamide

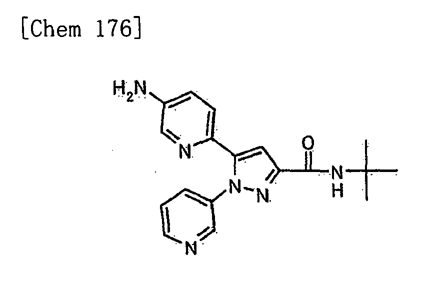

To a solution of the N-tert-butyl-5-[5-(tert-butoxycarbonylamino)-2-pyridyl]-1-(3-pyri dyl)-1H-pyrazole-3-carboxamide (273 mg) of Example 70 in dichloromethane (5 ml) was added a 4N solution of hydrochloric acid in 1,4-dioxane (5 ml) at room temperature, and the mixture was stirred for 2 hours. The solvent in the reaction liquid was evaporated under reduced pressure, and the residue was dissolved in a small amount of methanol, and an excessive amount of diethylether was poured into this solution. The solid precipitate was collected by filtration to give the title compound (212 mg, 75%).

¹H-NMR (400 MHz, CD₃OD)δ: 1.49 (9H, s), 7.31 (1H, s), 7.55-7.57 (2H, m), 7.90-7.95 (1H, m), 8.05-8.07 (1H, m), 8.30-8.35 (1H, m), 8.80-8.85 (1H, m), 9.01-9.07 (1H, m).
ESI-MSm/z: 337 (M+H)⁺.

[Example 72] N-tert-Butyl-5-(5-methyl-2-pyrazinyl)-1-(6-methyl-3-pyridyl)-1H-pyrazole-3-carboxamide

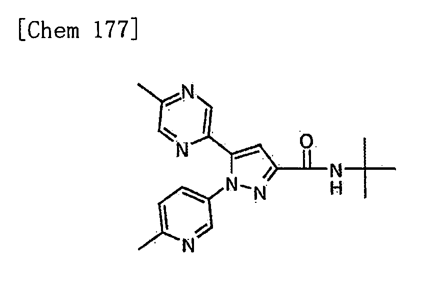

The procedure of Example 1 was repeated by using the 5-(5-methyl-2-pyrazinyl)-1-(6-methyl-3-pyridyl)-1H-pyrazole-3-car boxylic acid (0.228 g) of Referential Example 42 and tert-butylamine (0.161 ml) to give the title compound (0.165 g, 61%) as a solid.

¹H-NMR (400 MHz, CDCl₃)δ: 1.50 (9H, s), 2.57 (3H, s), 2.62 (3H, s), 6.83 (1H, br s), 7.24 (1H, d, J = 8.3 Hz), 7.29 (1H, s), 7.63-7.66 (1H, m), 8.31 (1H, m), 8.42 (1H, d, J = 2.7 Hz), 8.62 (1H, d, J = 1.5 Hz).
EI-MSm/z: 350 (M⁺).

[Example 73] N-(2-Hydroxy-1,1-dimethylethyl)-5-(5-chloro-2-pyridyl)-1-(3-pyridyl)-1H-pyrazole-3-carboxamide

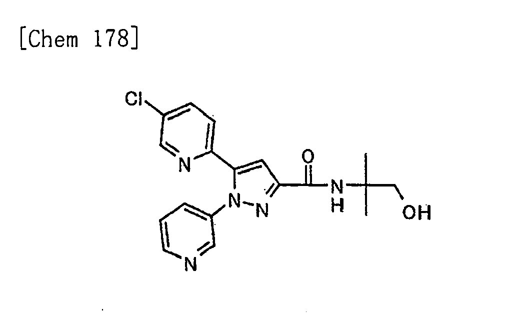

The procedure of Example 7 was repeated by using the 5-(5-chloro-2-pyridyl)-1-(3-pyridyl)-1H-pyrazole-3-carboxylic acid (0.269 g) of Referential Example 25 and 2-amino-2-methyl-1-propanol (0.170 ml) to give the title compound (0.312 g, 94%) as a solid.

¹H-NMR (400 MHz, CDCl₃)δ: 1.42 (6H, s), 3.72 (2H, d, J = 6.4 Hz), 4.68 (1H, t, J = 6.4 Hz), 7.02 (1H, br s), 7.20-7.30 (1H, m), 7.39 (1H, dd, J = 8.3, 4.7 Hz), 7.46 (1H, d, J = 8.3 Hz), 7.70-7.78 (2H, m), 8.37 (1H, d like, J = 2.4 Hz), 8.55 (1H, d like, J= 2.4 Hz), 8.62-8.67 (1H, m).
ESI-MSm/z: 372 (M+H)⁺.

[Example 74] N-tert-Butyl-5-(5-aminomethyl-2-pyridyl)-1-phenyl-1H-pyrazole-3-carboxamide

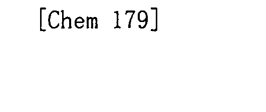

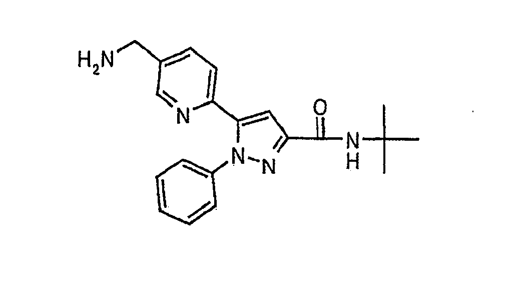

Under hydrogen atmosphere (8 atm), a suspension of the N-tert-butyl-5-(5-cyano-2-pyridyl)-1-phenyl-1H-pyrazole-3-carboxa mide (221 mg) of Example 69 and nickel-silica /alumina (about 65%, 100 mg) in a 2M solution of ammonia in ethanol (30 ml) was stirred overnight at 120°C. The reaction liquid was filtered through celite, and the solvent was evaporated under reduced pressure. The residue was purified by thin layer chromatography on silica gel (methanol-chloroform) to give the title compound (81 mg, 36%) as a solid.

¹H-NMR (400 MHz, DMSO-d₆)δ: 1.40 (9H, s), 1.90 (2H, s), 3.72 (2H, s), 7.13 (1H, s), 7.32-7.35 (3H, m), 7.42-7.43 (4H, m), 7.77 (1H, d, J = 8.1 Hz), 8.41 (1H, s).
ESI-MSm/z: 350 (M+H)⁺.

[Example 75] N-tert-Butyl-1-(6-methoxy-3-pyridyl)-5-(4-pyrimidinyl)-1H-pyrazole-3-carboxamide

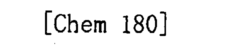

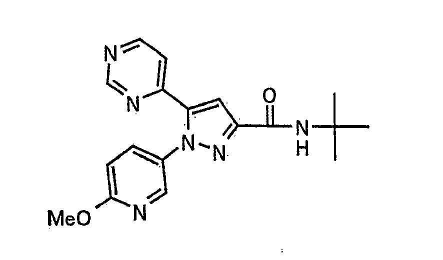

The procedure of Example 7 was repeated by using the 1-(6-methoxy-3-pyridyl)-5-(4-pyrimidinyl)-1H-pyrazole-3-carboxyli c acid (50 mg) of Referential Example 44 and tert-butylamine (27 µl) to give the title compound (46 mg, 77%) as a solid.

¹H-NMR (400 MHz, CDCl₃)δ: 1.49 (9H, s), 3.99 (3H, s), 6.81 (1H, br s), 6.82 (1H, d, J = 8.8 Hz), 7.40 (1H, s), 7.41 (1H, dd, J = 5.1, 1.2 Hz), 7.62 (1H, dd, J = 8.8, 2.7 Hz), 8.15 (1H, d, J = 2.7 Hz), 8.75 (1H, d, J = 5.1 Hz), 9.05 (1H, d, J = 1.2 Hz).
ESI-MSm/z: 353 (M+H)⁺.

[Example 76] N-tert-Butyl-5-(5-methyl-2-pyridyl)-1-(2-pyrazinyl)-1H-pyrazole-3-carboxamide

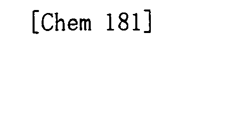

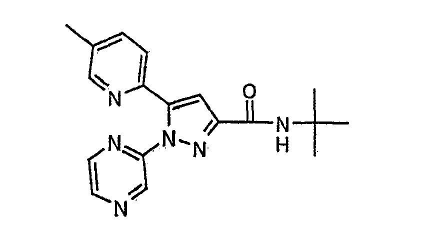

The procedure of Example 7 was repeated by using the 5-(5-methyl-2-pyridyl)-1-(2-pyrazinyl)-1H-pyrazole-3-carboxylic acid (0.253 g) of Referential Example 45 and tert-butylamine (0.190 ml) to give the title compound (0.145 g, 48%) as a solid.

¹H-NMR (400 MHz, CDCl₃)δ: 1.49 (9H, s), 2.33 (3H, s), 6.87 (1H, br s), 7.19 (1H, s), 7.49 (1H, d, J = 8.1 Hz), 7.56 (1H, dd, J = 8.1, 1.0 Hz), 8.18 (1H, d, J = 1.0 Hz), 8.32-8.37 (1H, m), 8.55 (1H, d, J = 2.7 Hz), 8.85 (1H, d, J = 1.4 Hz).
ESI-MSm/z: 337 (M+H)⁺.

[Example 77] N-(Tetrahydro-2H-pyran-4-yl)-1-(6-methoxy-3-pyridyl)-5-(1H-pyrrol-2-yl)-1H-pyrazole-3-carboxamide

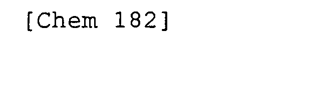

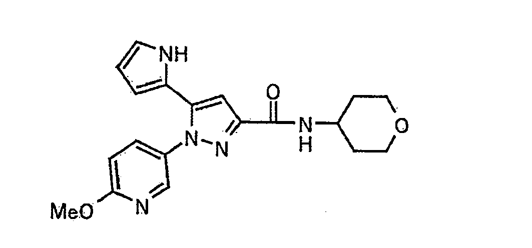

The procedure of Example 1 was repeated by using the 1-(6-methoxy-3-pyridyl)-5-(1H-pyrrol-2-yl)-1H-pyrazole-3-carboxyl ic acid (0.222 g) of Referential Example 46 and tetrahydro-2H-pyran-4-ylamine (0.142 g) to give the title compound (219 mg, 76%) as a solid.

¹H-NMR (400 MHz, CDCl₃)δ: 1.58-1.68 (2H, m), 1.97-2.02 (2H, m), 3.54 (2H, ddd, J = 11.7, 11.7, 2.2 Hz), 3.98-4.03 (2H, m), 4.01 (3H, s), 4.16-4.25 (1H, m), 5.80-5.82 (1H, m), 6.14-6.16 (1H, m), 6.83-6.86 (2H, m), 6.89 (1H, d, J = 8.1 Hz), 7.21 (1H, s), 7.61 (1H, dd, J = 8.8, 2.7 Hz), 8.28 (1H, dd, J = 2.7, 0.5 Hz), 9.18 (1H, br s).
ESI-MSm/z: 368 (M+H)⁺.

[Example 78] N-tert-Butyl-5-(5-methyl-2-pyridyl)-1-(3-pyridazinyl)-1H-pyrazole-3-carboxamide

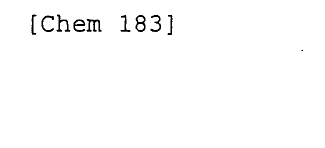

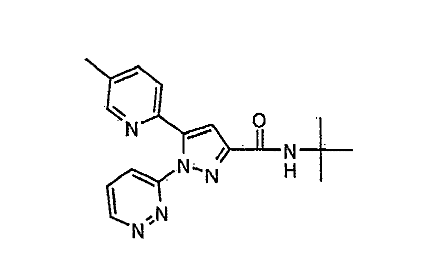

The procedure of Example 7 was repeated by using the 5-(5-methyl-2-pyridyl)-1-(3-pyridazinyl)-1H-pyrazole-3-carboxylic acid (0.223 g) of Referential Example 47 and tert-butylamine (0.250 ml) to give the title compound (0.202 g, 77%) as a solid.

¹H-NMR (400 MHz, CDCl₃)δ: 1.49 (9H, s), 2.31 (3H, s), 6.84 (1H, br s), 7.19 (1H, s), 7.50-7.60 (2H, m), 7.64 (1H, dd, J = 8.6, 4.9H), 7.91 (1H, dd, J = 8.6, 1.5 Hz), 8.12-8.23 (1H, m), 9.14 (1H, dd, J = 4.9, 1.5 Hz).
ESI-MSm/z: 337 (M+H)⁺.

[Example 79] N-(1-Carbamoyl-1-cyclopentyl)-1-(6-methoxy-3-pyridyl)-5-(1H-pyrrol-2-yl)-1H-pyrazole-3-carboxamide

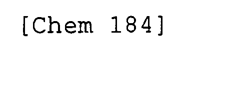

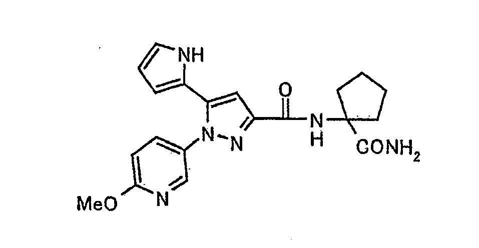

The procedure of Example 1 was repeated by using the 1-(6-methoxy-3-pyridyl)-5-(1H-pyrrol-2-yl)-1H-pyrazole-3-carboxyl ic acid (0.222 g) of Referential Example 46 and the 1-amino-1-cyclopentanecarboxamide trifluoroacetate (0.341 g) of Referential Example 26 to give the title compound (253 mg, 82%) as a solid.

¹H-NMR (400MHz, CDCl₃)δ: 1.75-1.88 (4H, m), 2.09-2.16 (2H, m), 2.41-2.49 (2H, m), 4.02 (3H, s), 5.33 (1H, br s), 5.77-5.79 (1H, m), 6.12-6.14 (1H, m), 6.77 (1H, br s), 6.85 (1H, d, J = 8.8 Hz), 6.96-6.98 (1H, m), 7.22 (1H, br s), 7.23 (1H, s), 7.60 (1H, dd, J = 8.8, 2.9 Hz), 8.28 (1H, d, J = 2.7 Hz), 9.21 (1H, br s).
ESI-MSm/z: 395 (M+H)⁺.

[Example 80] N-tert-Butyl-1-(6-methoxy-3-pyridyl)-5-(1H-pyrrol-1-yl)-1H-pyrazole-3-carboxamide

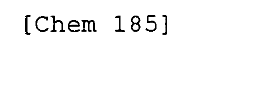

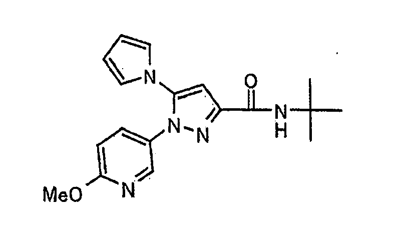

The procedure of Example 1 was repeated by using the 1-(6-methoxy-3-pyridyl)-5-(1H-pyrrol-1-yl)-1H-pyrazole-3-carboxyl ic acid (0.221 g) of Referential Example 48 and tert-butylamine (0.148 ml) to give the title compound (222 mg, 83%) as a solid.

¹H-NMR (400 MHz, CDCl₃)δ: 1.49 (9H, s), 3.95 (3H, s), 6.28-6.29 (2H, m), 6.63-6.64 (2H, m), 6.72 (1H, d, J = 9.0 Hz), 6.82 (1H, br s), 6.89 (1H, s), 7.29 (1H, dd, J = 8.9, 2.8 Hz), 8.03 (1H, d, J = 2.7 Hz).
ESI-MSm/z: 340 (M+H)⁺.

[Example 81] N-tert-Butyl-5-(5-methoxy-2-pyridyl)-1-(6-methyl-3-pyridyl)-1H-pyrazole-3-carboxamide

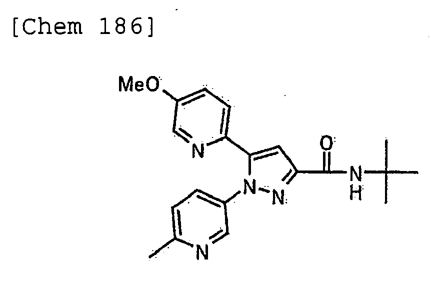

The procedure of Example 1 was repeated by using the 5-(5-methoxy-2-pyridyl)-1-(6-methyl-3-pyridyl)-1H-pyrazole-3-carb oxylic acid (0.143 g) of Referential Example 49 and tert-butylamine (96.3 µl) to give the title compound (0.130 g, 76%) as a solid.

¹H-NMR (400 MHz, CDCl₃)δ: 1.49 (9H, s), 2.60 (3H, s), 3.86 (3H, s), 6.84 (1H, br s), 7.13 (1H, m), 7.19-7.22 (2H, m), 7.40 (1H, d, J = 8.5 Hz), 7.63 (1H, dd, J = 8.3, 2.7 Hz), 8.15 (1H, d, J = 2.9 Hz), 8.40 (1H, d, J = 2.7 Hz).
FAB-MSm/z: 366 (M+H)⁺.

[Example 82] N-tert-Butyl-5-(5-methyl-2-pyridyl)-1-(2-pyrimidinyl)-1H-pyrazole-3-carboxamide

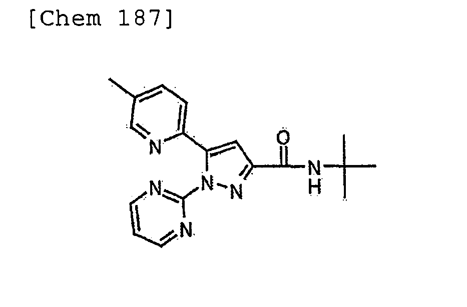

The procedure of Example 1 was repeated by using the 5-(5-methyl-2-pyridyl)-1-(2-pyrimidinyl)-1H-pyrazole-3-carboxylic acid (0.250 g) of Referential Example 50 and tert-butylamine (0.186 ml) to give the title compound (0.245 g, 81%) as a solid.

¹H-NMR (400 MHz, CDCl₃)δ: 1.48 (9H, s), 2.33 (3H, s), 6.96 (1H, s), 7.18 (1H, s), 7.29-7.31 (1H, m), 7.49 (1H, d, J = 7.8 Hz), 7.55-7.57 (1H, m), 8.18 (1H, m), 8.72 (2H, d, J = 4.9 Hz).
EI-MSm/z: 336 (M⁺).

[Example 83] N-tert-Butyl-1-(6-methoxy-3-pyridazinyl)-5-(2-pyrazinyl)-1H-pyrazole-3-carboxamide

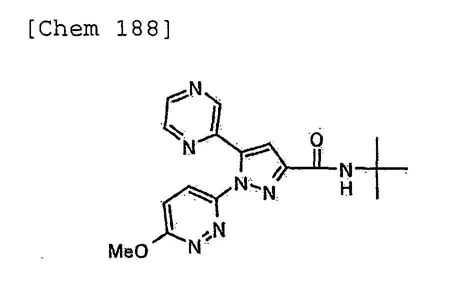

The procedure of Example 1 was repeated by using the 1-(6-methoxy-3-pyridazinyl)-5-(2-pyrazinyl)-1H-pyrazole-3-carboxy lic acid (0.232 g) of Referential Example 51 and tert-butylamine (0.148 ml) to give the title compound (210 mg, 75%) as a solid.

¹H-NMR (400 MHz, CDCl₃)δ: 1.50 (9H, s), 4.12 (3H, s), 6.81 (1H, br s), 7.18 (1H, d, J = 9.3 Hz), 7.30 (1H, s), 7.86 (1H, d, J = 9.3 Hz), 8.39 (1H, dd, J = 2.3, 1.8 Hz), 8.51 (1H, d, J = 2.7 Hz), 8.85 (1H, d, J = 1.5 Hz).
ESI-MSm/z: 354 (M+H)⁺.

[Example 84] N-(2,2-Dimethylpropyl)-1-(6-methoxy-3-pyridazinyl)-5-(2-pyrazinyl)-1H-pyrazole-3-carboxamide

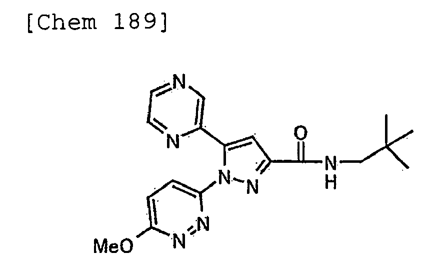

The procedure of Example 1 was repeated by using the 1-(6-methoxy-3-pyridazinyl)-5-(2-pyrazinyl)-1H-pyrazole-3-carboxy lic acid (0.232 g) of Referential Example 51 and neopentylamine (0.166 ml) to give the title compound (223 mg, 77%) as a solid.

¹H-NMR (400 MHz, CDCl₃)δ: 1.00 (9H, s), 3.29 (2H, d, J = 6.6 Hz), 4.13 (3H, s), 7.00-7.04 (1H, m), 7.19 (1H, d, J = 9.3 Hz), 7.35 (1H, s), 7.87 (1H, d, J = 9.0 Hz), 8.40 (1H, dd, J = 2.4, 1. 5 Hz), 8.52 (1H, d, J = 2.7 Hz), 8.87 (1H, d, J = 1.5 Hz).
ESI-MSm/z: 368 (M+H)⁺.

[Example 85] N-tert-Butyl-5-(5-fluoro-2-pyridyl)-1-(3-pyridyl)-1H-pyrazole-3-carboxamide

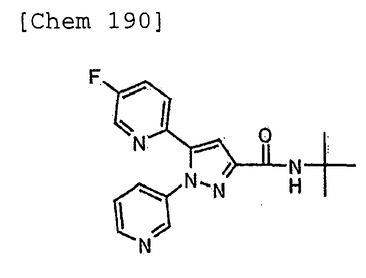

The procedure of Example 7 was repeated by using the 5-(5-fluoro-2-pyridyl)-1-(3-pyridyl)-1H-pyrazole-3-carboxylic acid (150 mg) of Referential Example 52 and tert-butylamine (111 µl) to give the title compound (56 mg, 31%) as a solid.

¹H-NMR (400 MHz, CDCl₃)δ: 1.49 (9H, s), 6.84 (1H, br s), 7.21 (1H, s), 7.37 (1H, ddd, J = 8.1, 4.9, 0.7 Hz), 7.46 (1H, ddd, J = 8.8, 7.8, 2.9 Hz), 7.54 (1H, ddd, J = 8.8, 4.4, 0.5 Hz), 7.74 (1H, ddd, J = 8.1, 2.4, 1.5 Hz), 8.28 (1H, d, J = 2.9 Hz), 8. 54 (1H, dd, J = 2.4, 0.5 Hz), 8. 61 (1H, dd, J = 4.9, 1.5 Hz).
ESI-MSm/z: 340 (M+H)⁺.

### [Example 86]

### N-(2-Fluoro-1,1-dimethylethyl)-1-(6-methoxy-3-pyridazinyl)-5-(2-p yridyl)-1H-pyrazole-3-carboxamide

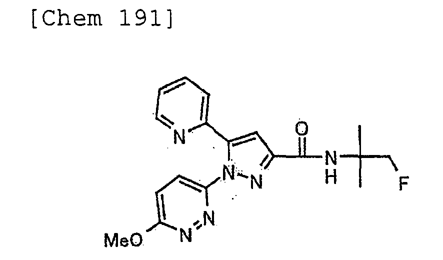

The procedure of Example 7 was repeated by using the 1-(6-methoxy-3-pyridazinyl)-5-(2-pyridyl)-1H-pyrazole-3-carboxyli c acid (297 mg) of Referential Example 9 and the 2-amino-1-fluoro-2-methylpropane hydrochloride (128 mg) of Referential Example 71 to give the title compound (207 mg, 56%) as a solid.

¹H-NMR (400 MHz, CDCl₃)δ: 1.49 (6H, d, J = 2.0 Hz), 4.13 (3H, s), 4.59 (2H, d, J = 47.4 Hz), 6.89 (1H, s), 7.14 (1H, d, J= 9.3 Hz), 7.18-7.22 (2H, m), 7.58-7.62 (1H, m), 7.73-7.77 (2H, m), 8.36-8.38 (1H, m).
ESI-MSm/z: 371 (M+H)⁺.

[Example 87] N-(2-Fluoro-1,1-dimethylethyl)-5-(5-chloro-2-pyridyl)-1-(3-pyridyl)-1H-pyrazole-3-carboxamide

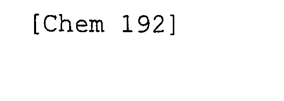

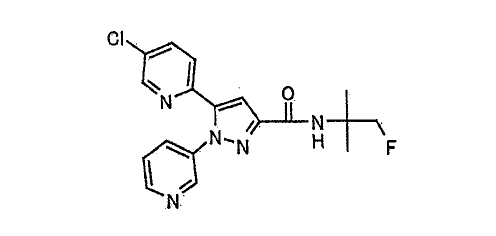

The procedure of Example 7 was repeated by using the 5-(5-chloro-2-pyridyl)-1-(3-pyridyl)-1H-pyrazole-3-carboxylic acid (301 mg) of Referential Example 25 and the 2-amino-1-fluoro-2-methylpropane hydrochloride (128 mg) of Referential Example 71 to give the title compound (186 mg, 50%) as a solid.

¹H-NMR (400 MHz, CDCl₃)δ: 1.50 (6H, d, J = 2.2 Hz), 4.59 (2H, d, J = 47.6 Hz), 6.88 (1H, s), 7.23 (1H, s), 7.39 (1H, dd, J = 4.2, 2.1 Hz), 7.46 (1H, d, J = 8.5 Hz), 7.70-7.77 (2H, m), 8.37 (1H, d, J = 2.0 Hz), 8.54 (1H, d, J = 2.4 Hz), 8.63 (1H, dd, J = 4.9, 1.5 Hz).
ESI-MSm/z: 374 (M+H)⁺.

[Example 88] N-(2-Fluoro-1,1-dimethylethyl)-5-(5-methyl-2-pyridyl)-1-(3-pyridazinyl)-1H-pyrazole-3-carboxamide

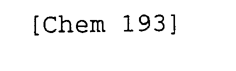

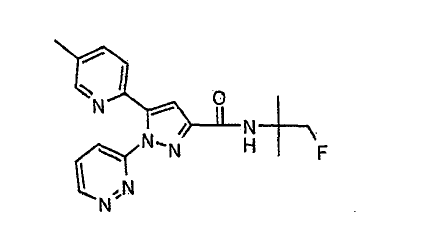

The procedure of Example 7 was repeated by using the 5-(5-methyl-2-pyridyl)-1-(3-pyridazinyl)-1H-pyrazole-3-carboxylic acid (167 mg) of Referential Example 47 and the 2-amino-1-fluoro-2-methylpropane hydrochloride (75.7 mg) of Referential Example 71 to give the title compound (102 mg, 48%) as a solid.

¹H-NMR (400 MHz, CDCl₃)δ: 1.53 (6H, d, J = 2.2 Hz), 2.32 (3H, s), 4.59 (2H, d, J = 47.4 Hz), 6.90 (1H, s), 7.19 (1H, s), 7.52-7.58 (2H, m), 7.66 (1H, dd, J = 8.5, 4.9 Hz), 7.94 (1H, dd, J = 8.7, 1.3 Hz), 8.17 (1H, s), 9.15 (1H, dd, J = 4.9, 1.5 Hz).
ESI-MSm/z: 355 (M+H)⁺.

[Example 89] N-(2-Fluoro-1,1-dimethylethyl)-5-(5-methyl-2-pyridyl)-1-(3-pyridyl)-1H-pyrazole-3-carboxamide

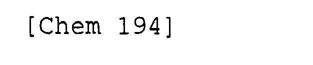

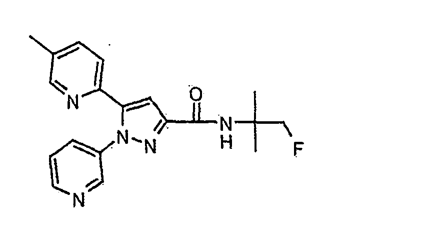

The procedure of Example 7 was repeated by using the 5-(5-methyl-2-pyridyl)-1-(3-pyridyl)-1H-pyrazole-3-carboxylic acid (102 mg) of Referential Example 37 and the 2-amino-1-fluoro-2-methylpropane hydrochloride (46.4 mg) of Referential Example 71 to give the title compound (36 mg, 28%) as a solid.

¹H-NMR (400 MHz, CDCl₃)δ: 1.50 (6H, d, J = 2.2 Hz), 2.31 (3H, d, J = 6.1 Hz), 4.58 (2H, dd, J = 47.5, 6.2 Hz), 6.89 (1H, s), 7.18 (1H, d, J = 6. 6 Hz), 7.24 (1H, s), 7.50-7.58 (2H, dd, J = 18.1, 7.3 Hz), 7.65 (1H, dt, J = 10.5, 4.3 Hz), 7.91-7.95 (1H, m), 8.16 (1H, d, J = 3.9 Hz), 9.13-9.16 (1H, m).
ESI-MSm/z: 354 (M+H)⁺.

[Example 90] N-tert-Butyl-1-(6-methoxy-3-pyridyl)-5-(4-methoxy-2-pyridyl)-1H-pyrazole-3-carboxamide

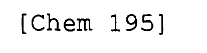

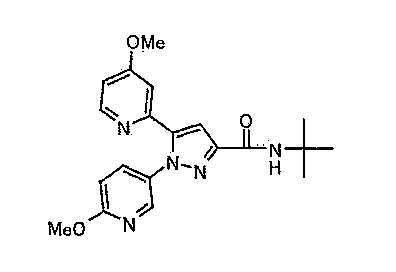

The procedure of Example 7 was repeated by using the 1-(6-methoxy-3-pyridyl)-5-(4-methoxy-2-pyridyl)-1H-pyrazole-3-car boxylic acid (300 mg) of Referential Example 53 and tert-butylamine (144 µl) to give the title compound (152 mg, 43%) as an amorphous product.

¹H-NMR (400 MHz, CDCl₃)δ: 1.49 (9H, s), 3.84 (3H, s), 3.96 (3H, s), 6.74 (1H, dd, J = 5.74, 2.44 Hz), 6.75 (1H, d, J = 8.79 Hz), 6.82 (1H, br), 6.97 (1H, d, J = 2.44 Hz), 7.18 (1H, s), 7.61 (1H, dd, J = 8.79, 2.69 Hz), 8.12 (1H, d, J = 2.69 Hz), 8.29 (1H, d, J = 5.74 Hz).
FAB-MSm/z: 382 (M+H)⁺.

[Example 91] N-tert-Butyl-1-(6-methoxy-3-pyridyl)-5-(4-methyl-2-pyridyl)-1H-pyrazole-3-carboxamide

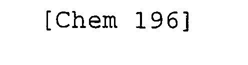

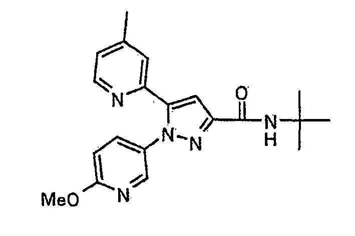

The procedure of Example 7 was repeated by using the 1-(6-methoxy-3-pyridyl)-5-(4-methyl-2-pyridyl)-1H-pyrazole-3-carb oxylic acid (300 mg) of Referential Example 54 and tert-butylamine (152 µl) to give the title compound (315 mg, 89%) as a solid.

¹H-NMR (400 MHz, CDCl₃)δ: 1.49 (9H, s), 2.36 (3H, s), 3.95 (3H, s), 6.75 (1H, d, J = 8.79 Hz), 6.83 (1H, br), 7.03 (1H, d, J = 5.01 Hz), 7.17 (1H, s), 7.29 (1H, s), 7.60 (1H, dd, J = 8.79, 2.69 Hz), 8.11 (1H, d, J = 2.69 Hz), 8.31 (1H, d, J = 5.01 Hz).
FAB-MSm/z: 366 (M+H)⁺.

[Example 92] N-tert-Butyl-5-(6-methoxy-3-pyridyl)-1-(3-pyridyl)-1H-pyrazole-3-carboxamide

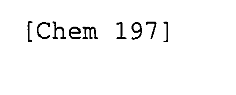

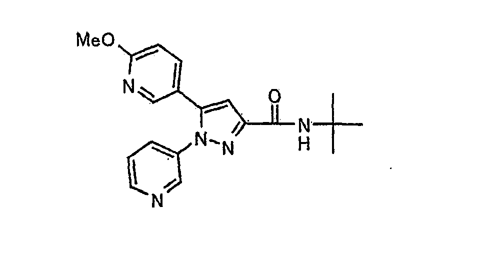

The procedure of Example 7 was repeated by using the 5-(6-methoxy-3-pyridyl)-1-(3-pyridyl)-1H-pyrazole-3-carboxylic acid (0.255 g) of Referential Example 55 and tert-butylamine (0.275 ml) to give the title compound (0.164 g, 54%) as a solid.

¹H-NMR (400 MHz, CDCl₃)δ: 1.49 (9H, s), 3.93 (3H, s), 6.70 (1H, d, J = 8.8 Hz), 6.83 (1H, br), 7.01 (1H, s), 7.32-7.40 (2H, m), 7.62-7.68 (1H, m), 8.07 (1H, d, J = 2.5 Hz), 8.61 (1H, d like, J = 2.9 Hz).
ESI-MSm/z: 352 (M+H)⁺.

[Example 93] N-tert-Butyl-5-(6-methoxy-3-pyridyl)-1-(2-pyridyl)-1H-pyrazole-3-carboxamide

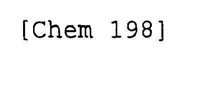

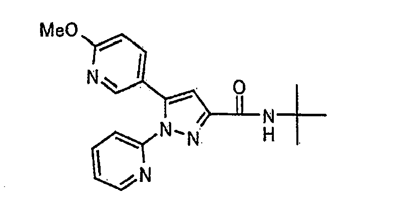

The procedure of Example 7 was repeated by using the 5-(6-methoxy-3-pyridyl)-1-(2-pyridyl)-1H-pyrazole-3-carboxylic acid (0.132 g) of Referential Example 56 and tert-butylamine (0.150 ml) to give the title compound (0.117 g, 75%) as a solid.

¹H-NMR (400 MHz, CDCl₃)δ: 1.48 (9H, s), 3.94 (3H, s), 6.69 (1H, d, J = 8.8 Hz), 6.87 (1H, br s), 6.99 (1H, s), 7.25-7.34 (1H, m), 7.42 (1H, dd, J = 8.8, 2.4 Hz), 7.52 (1H, d, J = 8.1 Hz), 7.79-7.86 (1H, m), 8.09 (1H, d, J = 2.4 Hz), 8.41 (1H, dd, J = 4.9, 2.0 Hz).
ESI-MSm/z: 352 (M+H)⁺.

[Example 94] N-(2-Fluoro-1,1-dimethylethyl)-5-(5-methyl-2-pyrazinyl)-1-(3-pyridyl)-1H-pyrazole-3-carboxamide

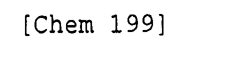

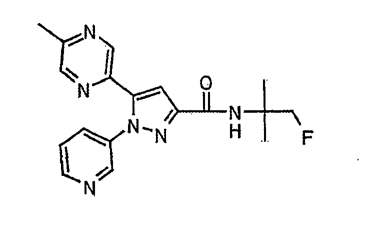

The procedure of Example 1 was repeated by using the 5-(5-methyl-2-pyrazinyl)-1-(3-pyridyl)-1H-pyrazole-3-carboxylic acid (0.250 g) of Referential Example 36 and the 2-amino-1-fluoro-2-methylpropane hydrochloride (0.125 g) of Referential Example 71 to give the title compound (0.281 g, 89%) as a solid.

¹H-NMR (400MHz, CDCl₃)δ: 1.50 (6H, m), 2.57 (3H, s), 4.54 (1H, s), 4.66 (1H, s), 6.89 (1H, s), 7.30 (1H, s), 7.40 (1H, dd, J = 8.2, 4.8 Hz), 7.76-7.79 (1H, m), 8.28 (1H, m), 8.56 (1H, d, J = 2.4 Hz), 8.63-8.65 (1H, m), 8.66 (1H, d, J = 1.5 Hz).
EI-MSm/z: 354 (M⁺).

[Example 95] N-tert-Butyl-5-(5-amino-2-pyrazinyl)-1-(3-pyridyl)-1H-pyrazole-3-carboxamide

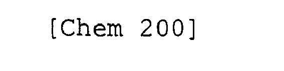

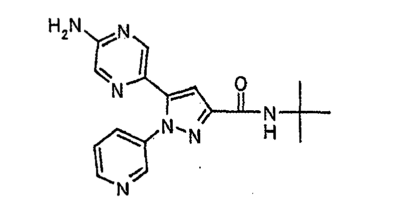

### 1) N-tert-Butyl-5-[5-(tert-butoxycarbonylamino)-2-pyrazinyl]-1-(3-py ridyl)-1H-pyrazole-3-carboxamide

The procedure of Example 1 was repeated by using the 5-[5-(tert-butoxycarbonylamino)-2-pyrazinyl]-1-(3-pyridyl)-1H-pyr azole-3-carboxylic acid (0.279 g) of Referential Example 57 and tert-butylamine (0.152 ml) to give N-tert-butyl-5-[5-(tert-butoxycarbonylamino)-2-pyrazinyl]-1-(3-py ridyl)-1H-pyrazole-3-carboxamide (0.236 g, 74%) as a solid.

¹H-NMR (400 MHz, CDCl₃)δ: 1.50 (9H, s), 1.53 (9H, s), 6.83 (1H, s), 7.24 (1H, m), 7.36-7.39 (2H, m), 7.74-7.77 (1H, m), 8.40 (1H, d, J = 1.0 Hz), 8.58 (1H, d, J = 2.4 Hz), 8.63 (1H, dd, J = 4.9, 1.0 Hz), 9.09-9.10 (1H, m).
EI-MSm/z: 437 (M⁺).

### 2) The title compound

Trifluoroacetic acid (2.3 ml) was added to a solution of N-tert-butyl-5-[5-(tert-butoxycarbonylamino)-2-pyrazinyl]-1-(3-py ridyl)-1H-pyrazole-3-carboxamide (0.230 g) in dichloromethane (4.6 ml) at 0°C, and the mixture was stirred for 90 minutes, and the mixture was stirred for another 90 minutes at room temperature. Saturated aqueous sodium bicarbonate and chloroform were added to the reaction liquid and the phases were separated, and the organic layer was dried over anhydrous sodium sulfate. After filtration, the solvent was evaporated under reduced pressure, and the residue was purified by column chromatography on silica gel (methanol-chloroform) to give the title compound (0.160 g, 89%) as a solid.

¹H-NMR (400 MHz, CDCl₃)δ: 1.49 (9H, s), 4.78 (2H, s), 6.83 (1H, s), 7.14 (1H, m), 7.37 (1H, dd, J = 8.2, 4.8 Hz), 7.75-7.80 (2H, m), 8.19 (1H, d, J = 1.2 Hz), 8.58-8.61 (2H, m).
EI-MSm/z: 337 (M⁺).

[Example 96] N-tert-Butyl-5-(5-dimethylamino-2-pyridyl)-1-(3-pyridyl)-1H-pyrazole-3-carboxamide

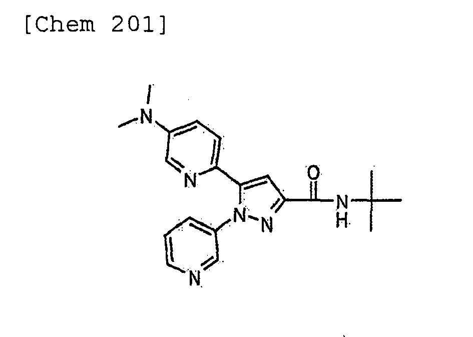

To a solution of the N-tert-butyl-5-(5-amino-2-pyridyl)-1-(3-pyridyl)-1H-pyrazole-3-ca rboxamide (95mg) of Example 71 in ethanol (10 ml) were added 1N aqueous hydrochloric acid (127 µl), 37% aqueous formalin (361 µl), and platinum oxide (IV) (32 mg), and the mixture was vigorously shaken at room temperature for 1 hour under hydrogen (3.5 atm) atmosphere. The catalyst in the reaction liquid was removed by filtration, and the solvent was evaporated under reduced pressure. Ethyl acetate and saturated aqueous sodium bicarbonate were added to the residue and the phases were separated, and the organic layer was washed with brine and dried over anhydrous sodium sulfate. After filtration, the solvent was evaporated under reduced pressure, and the residue was purified by thin layer chromatography on silica gel (methanol-chloroform) to give the title compound (32 mg, 41%) as a solid.

¹H-NMR (400 MHz, CDCl₃)δ: 1.49 (9H, s), 2.99 (6H, s), 6.84 (1H, br s), 6.94 (1H, dd, J = 8.8, 3.2 Hz), 7.08 (1H, s), 7.31 (1H, d, J = 8.8 Hz), 7.34 (1H, ddd, J = 8.1, 4.9, 0.7 Hz), 7.77 (1H, ddd, J = 8.1, 2.4, 1.5 Hz), 7.93 (1H, d, J = 2.9 Hz), 8.55-8.58 (2H, m).
ESI-MSm/z: 365 (M+H)⁺.

[Example 97] N-tert-Butyl-5-(4-methyl-2-pyridyl)-1-(3-pyridyl)-1H-pyrazole-3-carboxamide

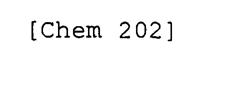

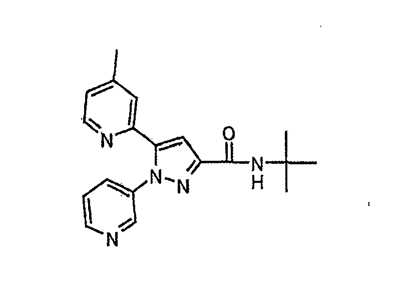

The procedure of Example 7 was repeated by using the 5-(4-methyl-2-pyridyl)-1-(3-pyridyl)-1H-pyrazole-3-carboxylic acid (280 mg) of Referential Example 58 and tert-butylamine (209 µl) to give the title compound (64.7 mg, 19%) as an amorphous product.

¹H-NMR (400 MHz, CDCl₃)δ: 1.50 (9H, s), 2.38 (3H, s), 6.85 (1H, br), 7.05 (1H, d, J = 5.01 Hz), 7.20 (1H, s), 7.34 (1H, s), 7.35 (1H, dd, J = 8.18, 4.76 Hz), 7.75 (1H, ddd, J = 8.18, 2.56, 1.47 Hz), 8.28 (1H, d, J = 5.01 Hz), 8.53 (1H, d, J = 2.56 Hz), 8.58 (1H, dd, J = 4.76, 1.47 Hz).
FAB-MSm/z: 336 (M+H)⁺.

[Example 98] N-(2-Fluoro-1,1-dimethylethyl)-5-(5-amino-2-pyridyl)-1-(3-pyridyl)-1H-pyrazole-3-carboxamide

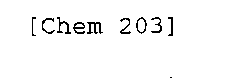

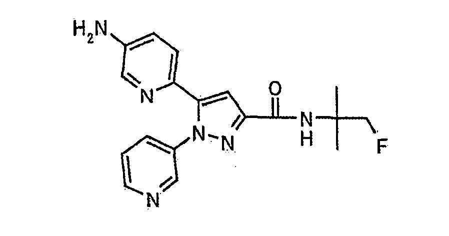

### 1) N-(2-Fluoro-1,1-dimethylethyl)-5-[5-(tert-butoxycarbonylamino)-2-pyridyl]-1-(3-pyridyl)-1H-pyrazole-3-carboxamide

The procedure of Example 7 was repeated by using the 5-[5-(tert-butoxycarbonylamino)-2-pyridyl]-1-(3-pyridyl)-1H-pyraz ole-3-carboxylic acid (250 mg) of Referential Example 41 and the 2-amino-1-fluoro-2-propane hydrochloride (100 mg) of Referential Example 71 to give N-(2-fluoro-1,1-dimethylethyl)-5-[5-(tert-butoxycarbonylamino)-2-pyridyl]-1-(3-pyridyl)-1H-pyrazole-3-carboxamide (217 mg, 73%) as a solid.

¹H-NMR (400 MHz, CDCl₃)δ: 1.52-1.60 (15H, m), 4.60 (2H, d, J = 47.6 Hz), 6.66 (1H, s), 6.89 (1H, s), 7.17 (1H, s), 7.33-7.43 (2H, m), 7.72-7.76 (1H, m), 8.04 (1H, s), 8.26 (1H, d, J = 2.0 Hz), 8.55-8.60 (2H, m).

### 2) The title compound

The procedure of Example 71 was repeated by using N-(2-fluoro-1,1-dimethylethyl)-5-[5-(tert-butoxycarbonylamino)-2-pyridyl]-1-(3-pyridyl)-1H-pyrazole-3-carboxamide (212 mg) to give the title compound (113 mg, 68%) as a solid.

¹H-NMR (400 MHz, CDCl₃)δ: 1.49 (6H, d, J = 10.0 Hz), 3.85 (2H, s), 4.60 (2H, d, J = 47.4 Hz), 6.89 (1H, s), 6.97 (1H, dd, J = 8.5, 2.9 Hz), 7.08 (1H, s), 7.26 (1H, s), 7.35 (1H, dd, J = 8.3, 4.9 Hz), 7.73-7.77 (1H, m), 7.89-7.90 (1H, m), 8.54-8.58 (2H, m).
ESI-MSm/z: 355 (M+H)⁺.

[Example 99] N-tert-Butyl-5-(5-amino-2-pyridyl)-1-(2-pyrazinyl)-1H-pyrazole-3-carboxamide

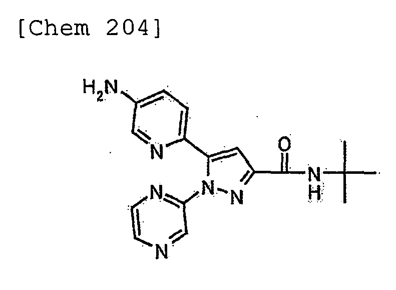

### 1) N-tert-Butyl-5-[5-(tert-butoxycarbonylamino)-2-pyridyl]-1-(2-pyrazinyl)-1H-pyrazole-3-carboxamide

The procedure of Example 1 was repeated by using the 5-[5-(tert-butoxycarbonylamino)-2-pyridyl]-1-(2-pyrazinyl)-1H-pyr azole-3-carboxylic acid (280 mg) of Referential Example 59 and tert-butylamine (307 µl) to give N-tert-butyl-5-[5-(tert-butoxycarbonylamino)-2-pyridyl]-1-(2-pyra zinyl)-1H-pyrazole-3-carboxamide (314 mg, 98%) as a solid.

¹H-NMR (400 MHz, CDCl₃)δ: 1.49 (9H, s), 1.52 (9H, s), 6.57 (1H, br s), 6.87 (1H, br s), 7.18 (1H, s), 7.53 (1H, d, J = 8.5 Hz), 7.98-8.04 (1H, m), 8.21 (1H, dd, J = 2.7, 0.7 Hz), 8.34 (1H, dd, J = 2.4, 1.5 Hz), 8.55 (1H, d, J = 2.4 Hz), 8.86 (1H, d, J = 1.5 Hz).
ESI-MSm/z: 438 (M+H)⁺.

### 2) The title compound

The procedure of Example 95 was repeated by using N-tert-butyl-5-[5-(tert-butoxycarbonylamino)-2-pyridyl]-1-(2-pyra zinyl)-1H-pyrazole-3-carboxamide (305 mg) to give the title compound (220 mg, 91%) as a solid.

¹H-NMR (400 MHz, CDCl₃)δ: 1.49 (9H, s), 3.74-3.96 (1H, br), 6.88 (1H, br s), 7.00 (1H, dd, J = 8.5, 2.7 Hz), 7.10 (1H, s), 7.38 (1H, dd, J = 8.5, 0.5 Hz), 7.81 (1H, dd, J = 2.7, 0.5 Hz), 8.38 (1H, dd, J = 2.4, 1.5 Hz), 8.54 (1H, d, J = 2.4 Hz), 8.78 (1H, d, J = 1.5 Hz).
ESI-MSm/z: 338 (M+H)⁺.

[Example 100] N-(2-Fluoro-1,1-dimethylethyl)-5-(5-amino-2-pyridyl)-1-(2-pyrazinyl)-1H-pyrazole-3-carboxamide

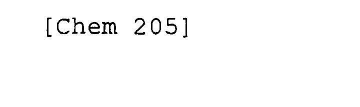

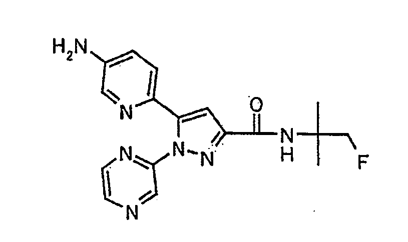

### 1) N-(2-Fluoro-1,1-dimethylethyl)-5-[5-(tert-butoxycarbonylamino)-2-pyridyl]-1-(2-pyrazinyl)-1H-pyrazole-3-carboxamide

The procedure of Example 7 was repeated by using the 5-[5-(tert-butoxycarbonylamino)-2-pyridyl]-1-(2-pyrazinyl)-1H-pyr azole-3-carboxylic acid (80 mg) of Referential Example 59 and the 2-fluoro-1,1-dimethylethylamine hydrochloride (80 mg) of Referential Example 71 to give N-(2-fluoro-1,1-dimethylethyl)-5-[5-(tert-butoxycarbonylamino)-2-pyridyl]-1-(2-pyrazinyl)-1H-pyrazole-3-carboxamide (89 mg, 93%) as a solid.

¹H-NMR (400 MHz, CDCl₃)δ: 1.50 (6H, d, J = 2.0 Hz), 1.52 (9H, s), 4.60 (2H, d, J = 47.6 Hz), 6.54 (1H, br s), 6.91 (1H, br s), 7.17 (1H, s), 7.53 (1H, d, J = 8.5 Hz), 7.99-8.05 (1H, m), 8.21 (1H, d, J = 2.7 Hz), 8.33 (1H, dd, J = 2.4, 1.5 Hz), 8.56 (1H, d, J = 2.4 Hz), 8.88 (1H, d, J = 1.5 Hz).
ESI-MSm/z: 456 (M+H)⁺.

### 2) The title compound

The procedure of Example 95 was repeated by using N-(2-fluoro-1,1-dimethylethyl)-5-[5-(tert-butoxycarbonylamino)-2-pyridyl]-1-(2-pyrazinyl)-1H-pyrazole-3-carboxamide (88 mg) to give the title compound (67 mg, 93%) as a solid.

¹H-NMR (400 MHz, CDCl₃)δ: 1.49 (6H, d, J = 2.0 Hz), 3.71-4.00 (2H, br), 4.60 (2H, d, J = 47.6 Hz), 6.92 (1H, br s), 7.00 (1H, dd, J = 8.5, 2.7 Hz), 7.10 (1H, s), 7.37 (1H, d, J = 8.5 Hz), 7.81 (1H, d, J = 2.7 Hz), 8.38 (1H, dd, J = 2.4, 1.5 Hz), 8.55 (1H, d, J = 2.4 Hz), 8.80 (1H, d, J = 1.5 Hz).
ESI-MSm/z: 356 (M+H)⁺.

[Example 101] N-tert-Butyl-1-(6-methoxy-3-pyridyl)-5-(1H-pyrazol-3-yl)-1H-pyrazole-3-carboxamide

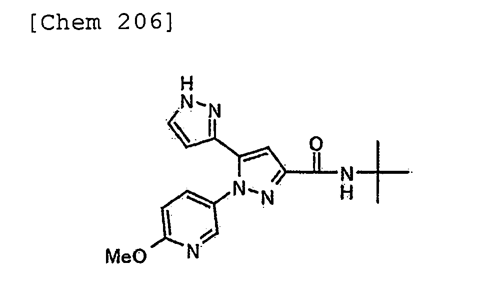

The procedure of Example 1 was repeated by using the 1-(6-methoxy-3-pyridyl)-5-(1H-pyrazol-3-yl)-1H-pyrazole-3-carboxy lic acid (0.222 g) of Referential Example 60 and tert-butylamine (0.148 ml) to give the title compound (124 mg, 46%) as a solid.

¹H-NMR (400 MHz, CDCl₃)δ: 1.49 (9H, s), 3.99 (3H, s), 6.08 (1H, d, J = 2.4 Hz), 6.81 (1H, d, J = 8.8 Hz), 6.86 (1H, br s), 7.49 (1H, s), 7.53 (1H, d, J = 2.4 Hz), 7.65 (1H, dd, J = 8.8, 2.7 Hz), 8.25 (1H, d, J = 2.7 Hz).
ESI-MSm/z: 341 (M+H)⁺.

[Example 102] N-Cyclopentyl-1-(6-methoxy-3-pyridazinyl)-5-(2-pyrazinyl)-1H-pyrazole-3-carboxamide

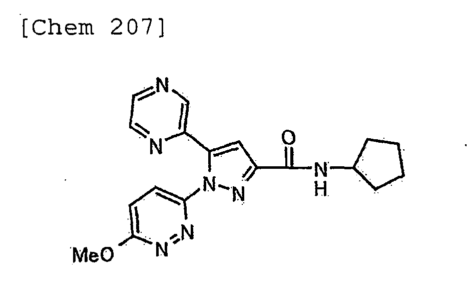

The procedure of Example 1 was repeated by using the 1-(6-methoxy-3-pyridazinyl)-5-(2-pyrazinyl)-1H-pyrazole-3-carboxy licacid (0.232 g) of Referential Example 51 and cyclopentylamine (0.139 ml) to give the title compound (247 mg, 86%) as a solid.

¹H-NMR (400MHz, CDCl₃)δ: 1.49-1.58 (2H, m), 1.63-1.78 (4H, m), 2.06-2.15 (2H, m), 4.12 (3H, s), 4.39-4.48 (1H, m), 6.86 (1H, d, J = 7.6 Hz); 7.18 (1H, d, J = 9.3 Hz), 7.34 (1H, s), 7.85 (1H, d, J = 9.0 Hz), 8.39 (1H, dd, J = 2.4, 1.5 Hz), 8.51 (1H, d, J = 2.4 Hz), 8.86 (1H, d, J = 1.5 Hz).
ESI-MSm/z: 366 (M+H)⁺.

[Example 103] N-(1-Carbamoyl-1-cyclopentyl)-5-(5-methyl-2-pyridyl)-1-(3-pyridyl)-1H-pyrazole-3-carboxamide

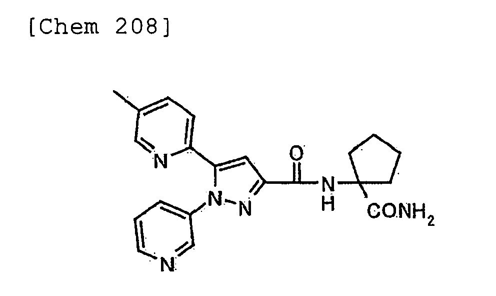

The procedure of Example 7 was repeated by using the 5-(5-methyl-2-pyridyl)-1-(3-pyridyl)-1H-pyrazole-3-carboxylic acid (0.253 g) of Referential Example 37 and the 1-amino-1-cyclopentanecarboxamide trifluoroacetate (0.335 g) of Referential Example 26 to give the title compound (0.180 g, 51%) as a solid.

¹H-NMR (400MHz, DMSO-d₆)δ: 1.60-1.75 (4H, m), 1.98-2.09 (2H, m), 2.10-2.20 (2H, m), 2.29 (3H, s), 6.84 (1H, br), 7.08 (1H, br), 7.28 (1H, s), 7.50 (1H, dd, J = 8.1, 4.6 Hz), 7.62 (1H, d, J = 8.1 Hz), 7.69-7.75 (1H, m), 7.79-7.83 (1H, m), 8.06 (1H, s), 8.25 (1H, d like, J = 1.5 Hz), 8.55 (1H, d, J = 2.4 Hz), 8.60 (1H, dd, J = 4.6, 1.5 Hz).
ESI-MSm/z: 391 (M+H)⁺.

[Example 104] N-tert-Butyl-5-(6-methyl-3-pyridazinyl)-1-(3-pyridyl)-1H-pyrazole-3-carboxamide

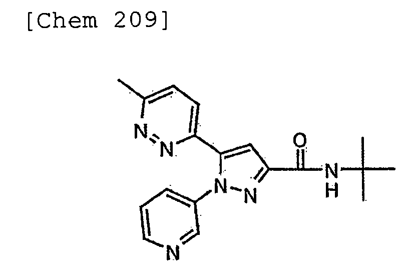

The procedure of Example 1 was repeated by using the 5-(6-methyl-3-pyridazinyl)-1-(3-pyridyl)-1H-pyrazole-3-carboxylic acid (0.247 g) of Referential Example 61 and tert-butylamine (0.280 ml) to give the title compound (0.148 g, 50%) as a solid.

¹H-NMR (400 MHz, CDCl₃)δ: 1.49 (9H, s), 2.71 (3H, s), 6.84 (1H, br s), 7.30 (1H, s), 7.36-7.42 (2H, m), 7.53 (1H, d, J = 8.6 Hz), 7.80-7.87 (1H, m), 8.55 (1H, d, J = 2.5 Hz), 8.62 (1H, dd, J = 4.8, 1.5 Hz).
ESI-MSm/z: 337 (M+H)⁺.

[Example 105] N-tert-Butyl-5-(1-methyl-1H-pyrrol-3-yl)-1-(3-pyridyl)-1H-pyrazole-3-carboxamide

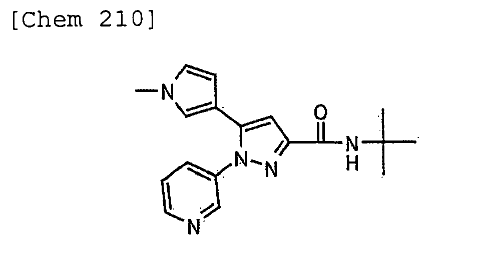

Water (1.0 ml) and lithium hydroxide monohydrate (14.2 mg) were added to a solution of the ethyl 5-(1-methyl-1H-pyrrol-3-yl)-1-(3-pyridyl)-1H-pyrazole-3-carboxyla te (100 mg) of Referential Example 62 in tetrahydrofuran (3.0 ml), and the mixture was sitrred overnight at room temperature. Another portion of lithium hydroxide monohydrate (14.2 mg) was added to the reaction liquid, and the mixture was stirred overnight. The solvent was evaporated under reduced pressure, and the residue was dissolved in N,N-dimethylformamide (3.0 ml), and tert-butylamine (71.0 µl), 1- (dimethylaminopropyl)-3-ethylcarbodiimide hydrochloride (97. 0 mg), and 1-hydroxybenzotriazole (68.5 mg) were added at room temperature, and the mixture was stirred for 3 days. The solvent was evaporated under reduced pressure, and water, saturated aqueous sodium bicarbonateand, and ethyl acetate were added to the residue and the phases were separated. The aqueous layer was extracted with ethyl acetate, and the combined organic layers were dried over anhydrous sodium sulfate. After filtration, the solvent was evaporated under reduced pressure, and the residue was purified by thin layer chromatography on silica gel (dichloromethane-ethyl acetate) to give the title compound (67.0 mg, 25%) as a solid.

¹H-NMR (400 MHz, CDCl₃)δ: 1.47 (9H, s), 3.60 (3H, s), 5.83-5.84 (1H, m), 6.47 (1H, t, J = 1.8 Hz), 6.50 (1H, t, J= 2.4 Hz), 6.80 (1H, s), 6.87 (1H, s), 7.37 (1H, dd, J = 8.2, 4.9 Hz), 7.79 (1H, dt, J = 8.2, 1.8 Hz), 8.63 (1H, dd, J = 4. 9, 1.1 Hz), 8.72 (1H, d, J = 2.4 Hz).
EI-MSm/z: 323 (M⁺).

[Example 106] N-tert-Butyl-1-(6-methyl-3-pyridazinyl)-5-(5-methyl-2-pyridyl)-1H-pyrazole-3-carboxamide

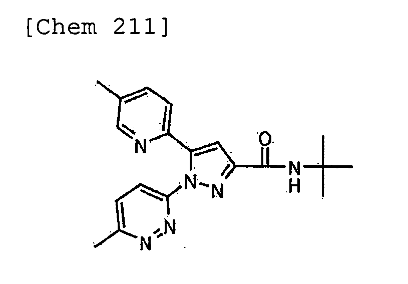

The procedure of Example 1 was repeated by using the 1-(6-methyl-3-pyridazinyl)-5-(5-methyl-2-pyridyl)-1H-pyrazole-3-c arboxylic acid (0.230 g) of Referential Example 64 and tert-butylamine (0.163 ml) to give the title compound (0.196 g, 72%) as a solid.

¹H-NMR (400 MHz, CDCl₃)δ: 1.49 (9H, s), 2.32 (3H, s), 2.75 (3H, s), 6.84 (1H, s), 7.17 (1H, s), 7.47-7.56 (3H, m), 7.80 (1H, d, J = 8.8 Hz), 8.18-8.19 (1H, m).
EI-MSm/z: 350 (M⁺).

[Example 107] N-tert-Butyl-5-(6-methoxy-3-pyridazinyl)-1-(3-pyridyl)-1H-pyrazole-3-carboxamide

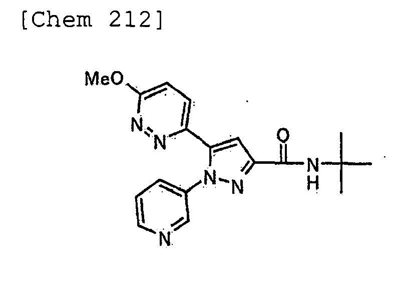

The procedure of Example 1 was repeated by using the 5-(6-methoxy-3-pyridazinyl)-1-(3-pyridyl)-1H-pyrazole-3-carboxyli c acid (0.303 g) of Referential Example 65 and tert-butylamine (0.321 ml) to give the title compound (0.185 g, 52%) as a solid.

¹H-NMR (400 MHz, CDCl₃)δ: 1.49 (9H, s), 4.11 (3H, s), 6.84 (1H, br s), 7.01 (1H, d, J = 9.0 Hz), 7.24 (1H, s), 7.39 (1H, dd, J = 8.0, 4.8 Hz), 7.49 (1H, d, J= 9.0 Hz), 7.78-7.84 (1H, m), 8.56 (1H, d, J = 2.5 Hz), 8.62 (1H, dd, J = 4.8, 1.5 Hz).
ESI-MSm/z: 353 (M+H)⁺.

[Example 108] N-tert-Butyl-1-(6-methoxy-3-pyridyl)-5-(1H-pyrrol-3-yl)-1H-pyrazole-3-carboxamide

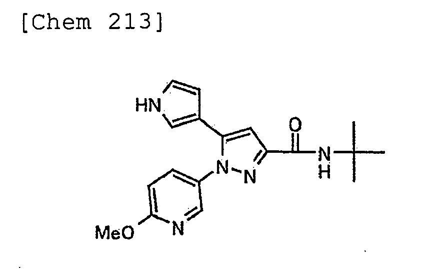

The ethyl 1-(6-methoxy-3-pyridyl)-5-(1H-pyrrol-3-yl)-1H-pyrazole-3-carboxyl ate (148 mg) produced by repeating the procedure of Referential Example 66 was suspended in tetrahydrofuran (2.0 ml), and to this suspension were added water (3.0 ml), methanol (2.0 ml), and lithium hydroxide monohydrate (40.0 mg) at room temperature, and the mixture was stirred overnight. To the reaction liquid was added 1N aqueous hydrochloric acid (948 µl), and the solvent was evaporated under reduced pressure. N,N-Dimethylformamide (3.0 ml), tert-butylamine (150 µl), 1- (dimethylaminopropyl) -3-ethylcarbodiimide hydrochloride (182 mg), and 1-hydroxybenzotriazole (130 mg) were added to the residue, and the mixture was stirred at room temperature for 18 days. The reaction solvent was evaporated under reduced pressure, and ethyl acetate, saturated aqueous sodiumbicarbonate, andwaterwere addedto the residue and the phases were separated. The aqueous layer was further extracted with ethyl acetate, and the combined organic layers were dried over anhydrous sodiumsulfate. After filtration, the solvent was evaporated under reduced pressure, and the residue was purified by column chromatography on silica gel (ethyl acetate-dichloromethane) to give the title compound (125 mg, 78%) as a solid.

¹H-NMR (400 MHz, CDCl₃)δ: 1.48 (9H, s), 3.99 (3H, s), 5.98-6.06 (1H, m), 6.62 (1H, d, J = 1.7 Hz), 6.70-6.75 (1H, m), 6.79 (1H, s), 6.81 (1H, s), 6.90 (1H, d, J = 0.7 Hz), 7.62 (1H, ddd, J = 8.8, 2.7, 0.7 Hz), 8.26 (1H, d, J = 2.7 Hz), 8.44 (1H, br s).
EI-MSm/z: 339 (M⁺).

[Example 109] N-Methoxy-N-methyl-1-(6-methoxy-3-pyridazinyl)-5-(5-methyl-2-pyridyl)-1H-pyrazole-3-carboxamide

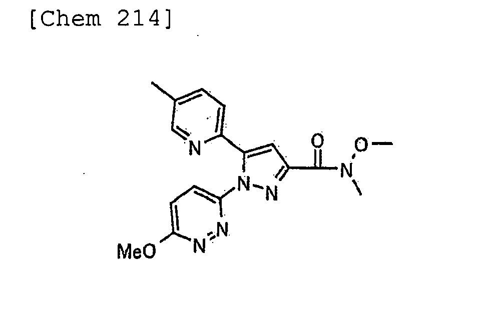

The procedure of Example 7 was repeated by using the 1-(6-methoxy-3-pyridazinyl)-5-(5-methyl-2-pyridyl)-1H-pyrazole-3-carboxylic acid (250 mg) Referential Example 67 and N,O-dimethylhydroxyamine hydrochloride (117 mg) to give the title compound (106 mg, 37%) as a solid.

¹H-NMR (400 MHz, CDCl₃)δ: 2.33 (3H, s), 3.49 (3H, br), 3.82 (3H, s), 4.11 (3H, s), 7.14 (1H, d, J = 8.91 Hz), 7.15 (1H, s), 7.46 (1H, d, J = 7.94 Hz), 7.54 (1H, dd, J = 7.94, 2.20 Hz), 7.90 (1H, d, J = 8.91 Hz), 8.25 (1H, s).
FAB-MSm/z: 355 (M+H)⁺.

[Example 110] N,N-Dimethyl-1-(6-methoxy-3-pyridazinyl)-5-(5-methyl-2-pyridyl)-1H-pyrazole-3-carboxamide

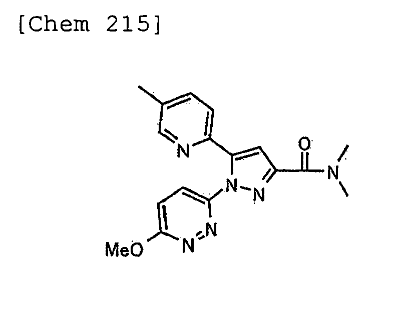

The procedure of Example 7 was repeated by using the 1-(6-methoxy-3-pyridazinyl)-5-(5-methyl-2-pyridyl)-1H-pyrazole-3-carboxylic acid (349 mg) of Referential Example 67 and dimethylamine hydrochloride (183 mg) to give the title compound (183 mg, 47%) as a solid.

¹H-NMR (400 MHz, CDCl₃)δ: 2.33 (3H, s), 3.15 (3H, s), 3.40 (3H, s), 4.11 (3H, s), 7.07 (1H, s), 7.12 (1H, d, J = 9.28 Hz), 7.47 (1H, d, J = 7.94 Hz), 7.54 (1H, dd, J = 7.94, 2.20 Hz), 7.80 (1H, d, J = 9.28 Hz), 8.24 (1H, s).
FAB-MSm/z: 339 (M+H)⁺.

[Example 111] N-[(2-Fluoro-1-fluoromethyl-1-methyl)ethyl]-5-(5-methyl-2-pyrazinyl)-1-(3-pyridyl)-1H-pyrazole-3-carboxamide

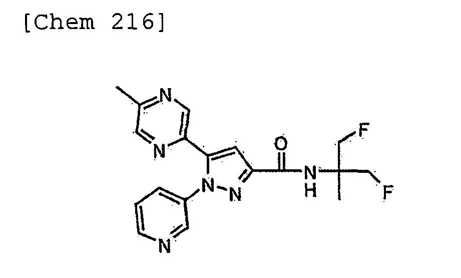

The procedure of Example 7 was repeated by using the 5-(5-methyl-2-pyrazinyl)-1-(3-pyridyl)-1H-pyrazole-3-carboxylic acid (281 mg) of Referential Example 36 and the 2-amino-1-fluoro-2-(fluoromethyl)propane hydrochloride (146 mg) of Referential Example 70 to give the title compound (171 mg, 46%) as a solid.

¹H-NMR (400 MHz, CDCl₃)δ: 1.58 (3H, s), 2.57 (3H, s), 4.59-4.83 (4H, m), 7.04 (1H, s), 7.29 (1H, s), 7.38-7.42 (1H, m), 7.76-7.79 (1H, m), 8.29 (1H, s), 8.55 (1H, d, J = 2.4 Hz), 8.64-8.66 (2H, m).
ESI-MSm/z: 373 (M+H)⁺.

[Example 112] N-(1-Carbamoyl-1-cyclopentyl)-1-(6-methyl-3-pyridyl)-5-(1-methyl-1H-pyrrol-3-yl)-1H-pyrazole-3-carboxamide

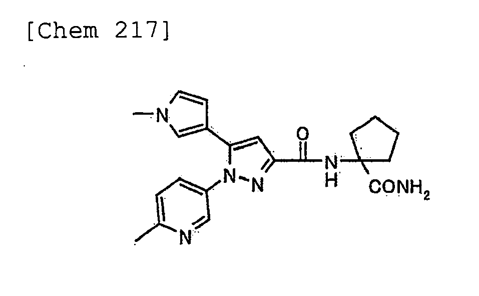

The procedure of Example 7 was repeated by using the 1-(6-methyl-3-pyridyl)-5-(1-methyl-1H-pyrrol-3-yl)-1H-pyrazole-3-carboxylic acid (0.248 g) Referential Example 43 and the 1-aminocyclopentane-1-carboxamide trifluoroacetate (0.322 g) of Referential Example 26 to give the title compound (0.298 g, 86%) as a solid.

¹H-NMR (400 MHz, DMSO-d₆)δ: 1.56-1.71 (4H, m), 1.94-2.17 (4H, m), 2.56 (3H, s), 5.73 (1H, t like, J= 2.0 Hz), 6.68 (1H, t like, J= 2.0 Hz), 6.73 (1H, t like, J = 2.0 Hz), 6.81 (1H, s), 6.84 (1H, br), 7.05 (1H, br), 7.42 (1H, d, J = 8.3 Hz), 7.78 (1H, dd, J = 8.3, 2.5 Hz), 7.87 (1H, s), 8.50 (1H, d, J = 2.5 Hz).
ESI-MSm/z: 393 (M+H)⁺.

[Example 113] N-(2-Fluoro-1,1-dimethylethyl)-5-(5-amino-2-pyrazinyl)-1-(3-pyridyl)-1H-pyrazole-3-carboxamide

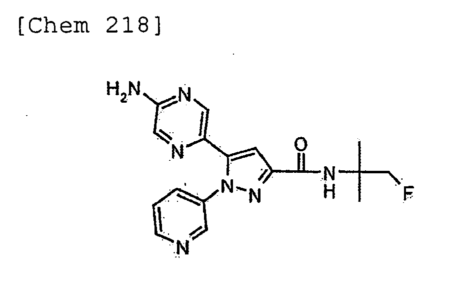

### 1) N-(2-Fluoro-1,1-dimethylethyl)-5-[5-(tert-butoxycarbonylamino)-2-pyrazinyl]-1-(3-pyridyl)-1H-pyrazole-3-carboxamide

The procedure of Example 1 was repeated by using the 5-[5-(tert-butoxycarbonylamino)-2-pyrazinyl]-1-(3-pyridyl)-1H-pyr azole-3-carboxylic acid (0.450 g) of Referential Example 57 and the 2-amino-1-fluoro-2-methylpropane hydrochloride (0.195 g) of Referential Example 71 to give N-(2-fluoro-1,1-dimethylethyl)-5-[5-(tert-butoxycarbonylamino)-2-pyrazinyl]-1-(3-pyridyl)-1H-pyrazole-3-carboxamide (0.470 g, 88%) as a solid.

¹H-NMR (400 MHz, CDCl₃)δ: 1.50 (6H, m), 1.53 (9H, s), 4.54 (1H, s), 4.66 (1H, s), 6.89 (1H, s), 7.24 (1H, s), 7.37-7.40 (1H, m), 7.66 (1H, s), 7.75-7.78 (1H, m), 8.41 (1H, d, J = 1.5 Hz), 8.57 (1H, d, J = 2.4 Hz), 8.63 (1H, dd, J = 4.9, 1.5 Hz), 9.11 (1H, d, J = 1.2 Hz).
EI-MSm/z: 455 (M⁺).

### 2) The title compound

The procedure of Example 95 was repeated by using N-(2-fluoro-1,1-dimethylethyl)-5-[5-(tert-butoxycarbonylamino)-2-pyrazinyl]-1-(3-pyridyl)-1H-pyrazole-3-carboxamide (0.460 g) to give the title compound (0.235 g, 65%) as a solid.

¹H-NMR (400 MHz, CDCl₃)δ: 1.49 (3H, s), 1.50 (3H, s), 4.54 (1H, s), 4.66 (1H, s), 4.79 (2H, s), 6.89 (1H, s), 7.14 (1H, s), 7.38 (1H, dd, J = 8.2, 4.8 Hz), 7.76-7.78 (1H, m), 7.80 (1H, m), 8.18 (1H, d, J = 1.2 Hz), 8.58 (1H, d, J = 2.7 Hz), 8.60-8.61 (1H, m).
EI-MSm/z: 355 (M⁺).

[Example 114] N-Cyanomethyl-N-methyl-1-(6-methoxy-3-pyridazinyl)-5-(5-methyl-2-pyridyl)-1H-pyrazole-3-carboxamide

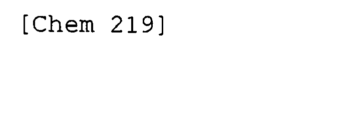

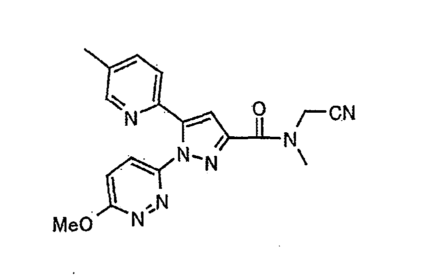

The procedure of Example 7 was repeated by using the 1-(6-methoxy-3-pyridazinyl)-5-(5-methyl-2-pyridyl)-1H-pyrazole-3-carboxylic acid (250 mg) of Referential Example 67 and (methylamino)acetonitrile hydrochloride (128 mg) to give the titl compound (128 mg, 44%) as a solid.

¹H-NMR (400 MHz, CDCl₃)δ: 2.34 (3H, s), 3.26 (1.5H, s), 3.60 (1.5H, s 4.13 (3H, s), 4.53 (1H, s), 5.02 (1H, s), 7.14 (1H, d, J = 9.16 Hz 7.18 (1H, s), 7.49 (1H, d, J = 7.69 Hz), 7.56 (1H, dd, J = 7.69, 2. Hz), 7.80 (1H, dd, J = 26.1, 9.16 Hz), 8.24 (1H, s).
FAB-MSm/z: 364 (M+H)⁺.

[Example 115] N,N-Dimethyl-1-(6-methoxy-3-pyridyl)-5-(1H-pyrrol-3-yl)-1H-pyrazole-5-carboxamide

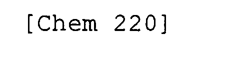

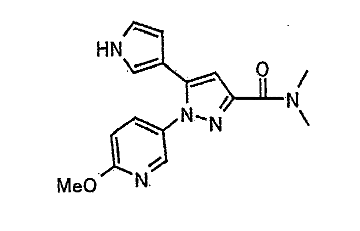

The procedure of Example 1 was repeated by using the 1-(6-methoxy-3-pyridyl)-5-(1H-pyrrol-3-yl)-1H-pyrazole-3-carboxyl ic acid (250 mg) of Referential Example 66 and dimethylamine hydrochloride (86 mg) to give the title compound (100 mg, 37%) as a solid.

¹H-NMR (400 MHz, CDCl₃)δ: 3.13 (3H, s), 3.40 (3H, s), 3.98 (3H, s), 6.05 (1H, s), 6.64 (1H, dd, J = 3.66, 2.69 Hz), 6.73 (1H, q, J = 2.32 Hz), 6.77 (1H, d, J = 8.79 Hz), 6.79 (1H, d, J = 2.69 Hz), 7.62 (1H, dd, J = 8.79, 2.69 Hz), 8.25 (1H, d, J = 2.69 Hz), 8.43 (1H, br).
FAB-MSm/z: 312 (M+H)⁺.

[Example 116] N,N-Dimethyl-1-(6-methoxy-3-pyridyl)-5-(1-methyl-1H-pyrrol-3-yl)-1H-pyrazole-3-carboxamide

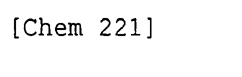

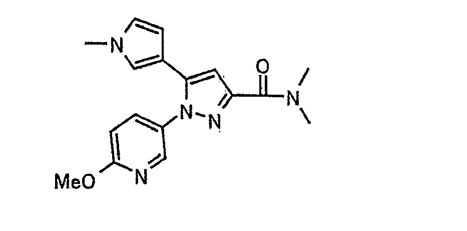

The procedure of Example 1 was repeated by using the 1-(6-methoxy-3-pyridyl)-5-(1-methyl-1H-pyrrol-3-yl)-1H-pyrazole-3 -carboxylic acid (200 mg) of Referential Example 68 and dimethylamine hydrochloride (82 mg) to give the title compound (47 mg, 21%) as a solid.

¹H-NMR (400 MHz, CDCl₃)δ: 3.12 (3H, s), 3.39 (3H, s), 3.60 (3H, s), 3.98 (3H, s), 5.90 (1H, dd, J = 2.69, 1.83 Hz), 6.47 (1H, t, J = 1.83 Hz), 6.51 (1H, t, J = 2.69 Hz), 6.76 (1H, s), 6.78 (1H, d, J = 8.79 Hz), 7. 62 (1H, dd, J = 8.79, 2.69 Hz), 8.25 (1H, dd, J = 2.69, 0.49 Hz). FAB-MSm/z: 326 (M+H)⁺.

[Example 117] N,N-Dimethyl-1-(6-methoxy-3-pyridyl)-5-(5-methyl-2-pyrazinyl)-1H-pyrazole-3-carboxamide

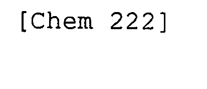

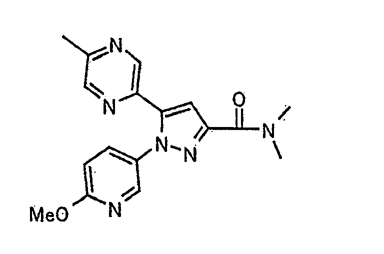

The procedure of Example 1 was repeated by using the 1-(6-methoxy-3-pyridyl)-5-(5-methyl-2-pyrazinyl)-1H-pyrazole-3-ca rboxylic acid (0.50 g) of Referential Example 69 and dimethylamine hydrochloride (0.393 g) to give the title compound (0.205 g, 37%) as a solid.

¹H-NMR (400 MHz, CDCl₃)δ: 2.58 (3H, s), 3.16 (3H, s), 3.42 (3H, s), 3.97 (3H, s), 6.79 (1H, d, J = 8.8 Hz), 7.21 (1H, s), 7.61-7.64 (1H, m), 8.12 (1H, d, J = 2.9 Hz), 8.36 (1H, m), 8.60 (1H, d, J = 1.5 Hz).
EI-MSm/z: 338 (M⁺).

[Example 118] 5-(5-Amino-2-pyrazinyl)-1-(3-pyridyl)-1H-pyrazole-3-carboxylic acid N,N-diisopropylamide

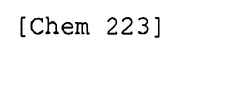

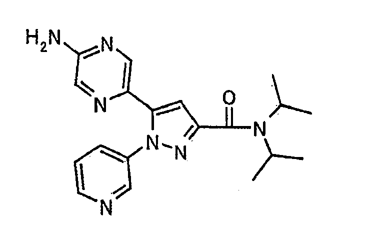

### 1) 5-[5-(tert-Butoxycarbonylamino)-2-pyrazinyl]-1-(3-pyridyl)-1H-pyrazole-3-carboxylic acid N,N-diisopropylamide

The procedure of Example 1 was repeated by using the 5-[5-(tert-butoxycarbonylamino)-2-pyrazinyl]-1-(3-pyridyl)-1H-pyr azole-3-carboxylic acid (0.675 g) of Referential Example 57 and diisopropylamine (0.496 ml) to give 5-[5-(tert-butoxycarbonylamino)-2-pyrazinyl]-1-(3-pyridyl)-1H-pyr azole-3-carboxylic acid N,N-diisopropylamide (0.504 g, 61%) as an amorphous product.

¹H-NMR (400 MHz, CDCl₃)δ: 1.25-1.72 (12H, m), 1.54 (9H, s), 3.60 (1H, m), 4.71 (1H, m), 7.02 (1H, s), 7.35-7.38 (1H, m), 7.48 (1H, s), 7.75 (1H, d, J = 7.8 Hz), 8.38 (1H, d, J= 1.2 Hz), 8.57-8.59 (2H, m), 9.15 (1H, s).
EI-MSm/z: 465 (M⁺).

### 2) The title compound

The procedure of Example 95(2) was repeated by using 5-[5-(tert-butoxycarbonylamino)-2-pyrazinyl]-1-(3-pyridyl)-1H-pyr azole-3-carboxylic acid N,N-diisopropylamide (0.494 g) to give the title compound (0.250 g, 63%) as a solid.

¹H-NMR (400 MHz, CDCl₃)δ: 1.26-1.27 (6H, m), 1.55-1.56 (6H, m), 3.59 (1H, m), 4.70 (1H, m), 4.84 (2H, br s), 6.91 (1H, s), 7.35 (1H, dd, J = 8.1, 4.6 Hz), 7.76 (1H, d, J = 8.1 Hz), 7.83-7.84 (1H, m), 8.16-8.17 (1H, m), 8.56-8.57 (2H, m).
FAB-MSm/z: 366 (M+H)⁺.

[Example 119] N-Isobutyl-N-methyl-5-(5-methyl-2-pyridyl)-1-(3-pyridyl)-1H-pyrazole-3-carboxamide

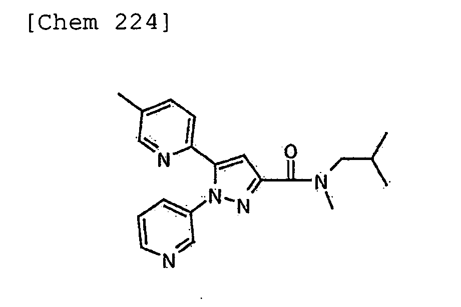

The procedure of Example 7 was repeated by using the 5-(5-methyl-2-pyridyl)-1-(3-pyridyl)-1H-pyrazole-3-carboxylic acid (100 mg) of Referential Example 37 and N-methylisobutylamine (64 µl) to give the title compound (66 mg, 53%) as a solid.

¹H-NMR (400 MHz, CDCl₃)δ: 0.89 (3H, d, J = 6.59 Hz), 0.98 (3H, d, J = 6.59 Hz), 2.09 (1H, m), 2.34 (3H, s), 3.13 (1.65H, s), 3.39 (1.35H, s), 3.42 (1H, d, J = 6.57 Hz), 3.70 (1H, d, J = 6.57 Hz), 7.10 (1H, d, J = 7.20 Hz), 7.34 (1H, dd, J = 8.18, 4.88 Hz), 7.39 (1H, dd, J = 10.99, 8.18 Hz), 7.54 (1H, t, J= 5.37 Hz), 7.74 (1H, t, J= 10.38 Hz), 8.31 (1H, s), 8.51 (1H, dd, J = 4.15, 2.56 Hz), 8.56 (1H, d, J = 4.52 Hz).
FAB-MSm/z: 350 (M+H)⁺.

[Example 120] N-Ethyl-N-methyl-1-(6-methoxy-3-pyridyl)-5-(1H-pyrrol-3-yl)-1H-pyrazole-3-carboxamide

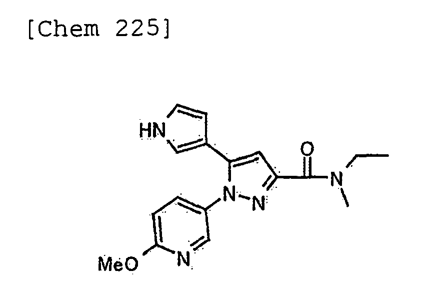

The procedure of Example 1 was repeated by using the 1-(6-methoxy-3-pyridyl)-5-(1H-pyrrol-3-yl)-1H-pyrazole-3-carboxyl ic acid (150 mg) of Referential Example 66 and N-ethylmethylamine (59 µl) to give the title compound (84 mg, 48%) as a solid.

¹H-NMR (400 MHz, CDCl₃)δ: 1.24 (3H, q, J = 7.08 Hz), 3.10 (1.5H, s), 3.36 (1.5H, s), 3.60 (1H, q, J = 7.08 Hz), 3.81 (1H, q, J = 7.08 Hz), 3.97 (3H, s), 6.05 (1H, s), 6.64 (1H, d, J = 5.74 Hz), 6.73 (2H, m), 6.77 (1H, d, J = 8.67 Hz), 7.61 (1H, dd, J = 8.67, 2.56 Hz), 8.25 (1H, d, J = 1.32 Hz), 8.43 (1H, br).
FAB-MSm/z: 326 (M+H)⁺.

[Example 121] N-(2-Fluoro-1,1-dimethylethyl)-5-(5-carbamoyl-2-pyridyl)-1-(3-pyridyl)-1H-pyrazole-3-carboxamide

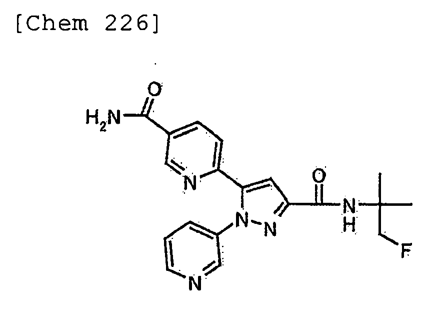

### 1) N-(2-Fluoro-1,1-dimethylethyl)-5-(5-cyano-2-pyridyl)-1-(3-pyridyl)-1H-pyrazole-3-carboxamide

The procedure of Example 7 was repeated by using the 5-(5-cyano-2-pyridyl)-1-(3-pyridyl)-1H-pyrazole-3-carboxylic acid (146 mg) of Referential Example 73 and the 2-fluoro-1,1-dimethylethylamine hydrochloride (96 mg) of Referential Example 71 to give N-(2-fluoro-1,1-dimethylethyl)-5-(5-cyano-2-pyridyl)-1-(3-pyridyl )-1H-pyrazole-3-carboxamide (137 mg, 75%) as a solid.

¹H-NMR (400 MHz, CDCl₃)δ: 1.50 (6H, d, J = 2.0 Hz), 4.59 (2H, d, J = 47.6 Hz), 6.88 (1H, s), 7.36 (1H, s), 7.42 (1H, dd, J = 8.4, 4.5 Hz), 7.66 (1H, dd, J = 8.3, 1.0 Hz), 7.73-7.74 (1H, m), 8.01 (1H, dd, J = 8.3, 2.2 Hz), 8.55 (1H, d, J = 2.0 Hz), 8.64 (1H, dd, J = 1.1, 0.5 Hz), 8.67 (1H, dd, J = 4.9, 1.5 Hz).

### 2) The title compound

The procedure of Example 21 was repeated by using N-(2-fluoro-1,1-dimethylethyl)-5-(5-cyano-2-pyridyl)-1-(3-pyridyl )-1H-pyrazole-3-carboxamide (175 mg) to give the title compound (140 mg, 76%) as a solid.

¹H-NMR (400 MHz, DMSO-d₆)δ: 1.39 (6H, d, J = 1.5 Hz), 4.60 (2H, d, J = 47.4 Hz), 7.44 (1H, s), 7.52 (1H, dd, J = 8.2, 4.8 Hz), 7.61 (1H, s), 7.63 (1H, s), 7.81-7.93 (2H, m), 8.15 (1H, s), 8.27-8.31 (1H, m), 8.60-8.64 (2H, m), 8.78-8.81 (1H, m).
ESI-MSm/z: 383 (M+H)⁺.

[Example 122] N-tert-Butyl-5-(1-methyl-1H-imidazol-4-yl)-1-(6-methyl-3-pyridyl)-1H-pyrazole-3-carboxamide

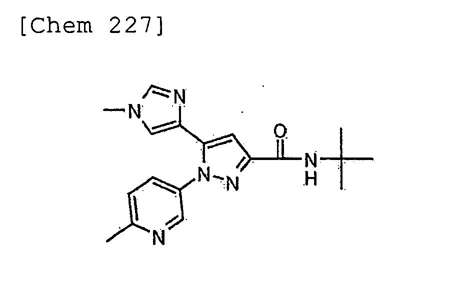

The procedure of Example 7 was repeated by using the 5-(1-methyl-1H-imidazol-4-yl)-1-(6-methyl-3-pyridyl)-1H-pyrazole-3-carboxylic acid (100 mg) of Referential Example 72 and tert-butylamine (45 µl) to give the title compound (88 mg, 74%) as a solid.

¹H-NMR (400 MHz, CDCl₃)δ: 1.48 (9H, s), 2.62 (3H, s), 3.66 (3H, s), 6.79 (1H, d, J= 1.2 Hz), 6.81 (1H, s), 7.04 (1H, s), 7.24 (1H, t, J = 6.7 Hz), 7.37 (1H, s), 7.75 (1H, dd, J = 8.3, 2.4 Hz), 8.54 (1H, d, J = 2.4 Hz).
ESI-MSm/z: 339 (M+H)⁺.
[Example 123] N-tert-Butyl-5-(5-cyano-2-pyridyl)-1-(3-pyridyl)-1H-pyrazole-3-carboxamide (An alternative for the synthesis of Example 23)

The procedure of Example 7 was repeated by using the 5-(5-cyano-2-pyridyl)-1-(3-pyridyl)-1H-pyrazole-3-carboxylic acid (3.50 g) of Referential Example 73 and tert-butylamine (2.53 ml) to give the title compound (3.40 g, 82%) as a solid.

¹H-NMR (400 MHz, CDCl₃)δ: 1.49 (9H, s), 6.82 (1H, s), 7.37 (1H, s), 7.41 (1H, q, J = 4.4 Hz), 7.67 (1H, d, J = 8.3 Hz), 7.74 (1H, d, J = 6.6 Hz), 8.01 (1H, dd, J = 8.2, 2.1 Hz), 8.55 (1H, d, J = 2.2 Hz), 8.63-8.67 (2H, m).
[Example 124] N-tert-Butyl-5-(5-hydroxymethyl-2-pyridyl)-1-(3-pyridyl)-1H-pyraz ole-3-carboxamide (An alternative for the synthesis of Example 26)

Nickel-silica/ alumina (content, about 65%; 1.0 g) was added to a solution of the N-tert-butyl-5-(5-cyano-2-pyridyl)-1-(3-pyridyl)-1H-pyrazole-3-ca rboxamide (2.04 g) of Example 123 in a 2M solution of ammonia in ethanol (50 ml), and the mixture was stirred in an autoclave under hydrogen atmosphere (8 atm) at 120°C for 2.5 hours. After cooling with air, the reaction liquid was filtered through celite, and the solvent was evaporated under reduced pressure. The residue was dissolved in water (100 ml) and acetic acid (20 ml), and sodium nitrite (2.03 g) was added at room temperature, and the mixture was stirred for 2 hours. After adding chloroform (150 ml) to the ice cold reaction liquid, sodium bicarbonate was gradually added until the bubbling stopped to render the solution basic. The organic layer was separated, and the aqueous layer was extracted with a mixed solvent of methanol-chloroform (1:10). The combined organic layers were dried over anhydrous sodium sulfate. After filtration, the solvent was evaporated under reduced pressure, and the residue was purified by column chromatography on silica gel (methanol-chloroform) to give the title compound (820 mg, 39%).

¹H-NMR (400 MHz, CDCl₃)δ: 1.49 (9H, s), 2.30 (1H, s), 4.73 (2H, s), 6.85 (1H, s), 7.19 (1H, s), 7.36 (1H, dd, J = 8.1, 4.9 Hz), 7.49 (1H, d, J = 8.1 Hz), 7.74-7.79 (2H, m), 8.40 (1H, s), 8.52 (1H, d, J = 2.4 Hz), 8.59 (1H, dd, J = 4.8, 1.6 Hz).

[Example 125] N-tert-Butyl-5-(5-formyl-2-pyridyl)-1-(3-pyridyl)-1H-pyrazole-3-carboxamide

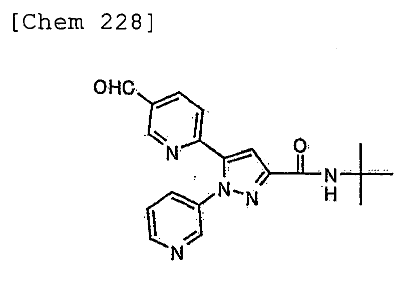

To a solution of the N-tert-butyl-5-(5-hydroxymethyl-2-pyridyl)-1-(3-pyridyl)-1H-pyraz ole-3-carboxamide (807 mg) of Example 124 in dichloromethane (50 ml) was added 1,1,1-tris(acetoxy)-1,1-dihydro-1,2-benziodoxol-3-(1H)-one (Dess-Martin reagent, 1.21 g) at room temperature, and the mixture was stirred overnight. 1N aqueous sodium hydroxide (50 ml) and chloroform were added to the reaction liquid and the phases were separated, and the organic layer was dried over anhydrous sodium sulfate. After filtration, the solvent was evaporated under reduced pressure, and the residue was purified by column chromatography on silica gel (hexane-ethyl acetate) to give the title compound (676 mg, 84%) as an amorphous product.

¹H-NMR (400 MHz, CDCl₃)δ: 1.50 (9H, s), 6.84 (1H, s), 7.38-7.42 (1H, m), 7.39 (1H, d, J = 4.9 Hz), 7.71 (1H, d, J = 8.1 Hz), 7.77 (1H, t, J = 4.2 Hz), 8.22 (1H, dd, J = 8.2, 2.1 Hz), 8.56 (1H, d, J = 2.4 Hz), 8.65 (1H, dd, J = 4.9, 1.5 Hz), 8.84 (1H, d, J = 2.2 Hz), 10.07 (1H, s).

[Example 126] N-tert-Butyl-5-[5-(1-hydroxyethyl)-2-pyridyl]-1-(3-pyridyl)-1H-pyrazole-3-carboxamide

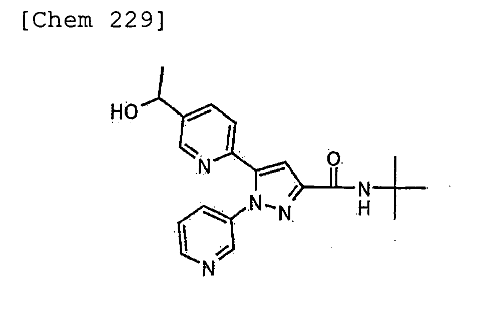

Methyl magnesium bromide (0.90M solution in tetrahydrofuran, 286 µl) was added dropwise to a solution of the N-tert-butyl-5-(5-formyl-2-pyridyl)-1-(3-pyridyl)-1H-pyrazole-3-c arboxamide (75.0 mg) of Example 125 in tetrahydrofuran (5 ml) at -78°C under argon atmosphere, and the mixture was stirred for 18 minutes. Water and chloroform were added to the reaction liquid and the phases were separated, and the organic layer was dried over anhydrous sodium sulfate. After filtration, the solvent was evaporated under reduced pressure, and the residue was purified by thin layer chromatography on silica gel (ethyl acetate) to give the title compound (34 mg, 43%) as an oily product.

¹H-NMR (400 MHz, CDCl₃)δ: 1.50 (9H, s), 1.51 (3H, d, J = 6.4 Hz), 2.55 (1H, s), 4.93 (1H, d, J = 6.3 Hz), 6.85 (1H, s), 7.18 (1H, s), 7.37 (1H, dd, J = 8.1, 4.9 Hz), 7.47 (1H, d, J = 8.1 Hz), 7.75-7.80 (2H, m), 8.40 (1H, s), 8.51 (1H, s), 8.58 (1H, d, J = 4.6 Hz).
ESI-MSm/z: 366 (M+H)⁺.

[Example 127] N-tert-Butyl-5-[5-(methylamino)methyl-2-pyridyl]-1-(3-pyridyl)-1H-pyrazole-3-carboxamide

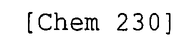

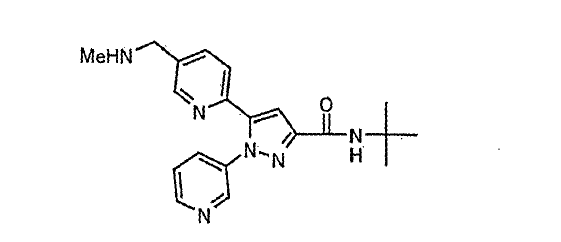

Cyano sodium borohydride (51 mg) was added to a solution of the N-tert-butyl-5-(5-formyl-2-pyridyl)-1-(3-pyridyl)-1H-pyrazole-3-c arboxamide (57mg) of Example 125, acetic acid (93.3µl), and methylamine hydrochloride (55 mg) in methanol (3 ml) at room temperature, and the mixture was stirred overnight. Saturated aqueous sodium bicarbonate and a mixed solvent of chloroform and methanol (10:1) were added to the reaction liquid and the phases were separated, and the organic layer was dried over anhydrous sodium sulfate. After filtration, the solvent was evaporated under reduced pressure, and the residue was purified by thin layer chromatography on silica gel (methanol-chloroform) to give the title compound (28 mg, 47%) as an oily product.

¹H-NMR (400 MHz, CDCl₃)δ: 1.49 (9H, s), 1.70 (3H, s), 2.67 (1H, s), 3.76 (2H, s), 6.85 (1H, s), 7.22 (1H, s), 7.36 (1H, dd, J = 8.1, 4. 9 Hz), 7.47 (1H, d, J = 8.1 Hz), 7.72-7.77 (2H, m), 8.37 (1H, s), 8.53-8.59 (2H, m).
ESI-MSm/z: 365 (M+H)⁺.

[Example 128] Methyl (2E)-3-{6-[2-(3-pyridyl)-5-(N-tert-butylcarbamoyl)-2H-pyrazol-3-yl]-3-pyridyl}acrylate

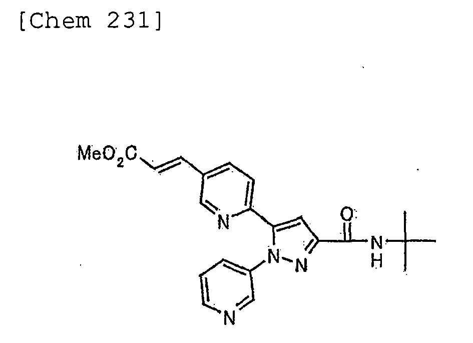

Under argon atmosphere and ice cooling, sodium hydride (55% in oil, 13 mg) was added to a solution of methyl phosphonoacetate (48 µl) intetrahydrofuran (3ml), and the mixture was stirred for 25 minutes. A solution of the N-tert-butyl-5-(5-formyl-2-pyridyl)-1-(3-pyridyl)-1H-pyrazole-3-c arboxamide (68.5 mg) of Example 125 in tetrahydrofuran (3 ml) was added to the reaction liquid under ice cooling, and the mixture was stirred for 15 minutes. Water and chloroform were added to the reaction liquid and the phases were separated, and the organic layer was dried over anhydrous sodium sulfate. After filtration, the solvent was evaporated under reduced pressure, and the residue was purified by thin layer chromatography on silica gel (hexane-ethyl acetate) to give the title compound (41 mg, 50%) as a solid.

¹H-NMR (400 MHz, CDCl₃)δ: 1.50 (9H, s), 3.82 (3H, s), 6.49 (1H, d, J = 16 Hz), 6.84, (1H, s), 7.29 (1H, s), 7.39 (1H, dd, J = 8.0, 4.8 Hz), 7.55 (1H, d, J = 8.4 Hz), 7.62 (1H, d, J = 16 Hz), 7.75-7.78 (1H, m), 7.87 (1H, dd, J = 8.0, 2.0 Hz), 8.52 (1H, d, J = 1.6 Hz), 8.56 (1H, d, J = 2.4 Hz), 8.63 (1H, dd, J = 5.2, 1.6 Hz).
ESI-MSm/z: 406 (M+H)⁺.

[Example 129] Methyl 3-{6-[2-(3-pyridyl)-5-(N-tert-butylcarbamoyl)-2H-pyrazol-3-yl]-3-pyridyl}propionate

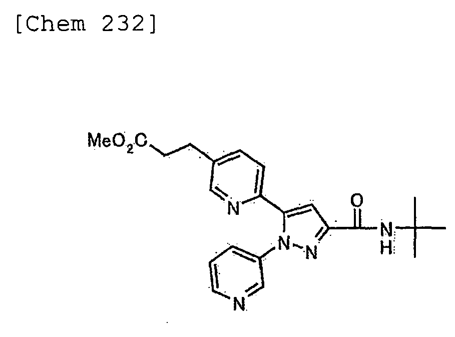

To a solution of the methyl (2E)-3-{6-[2-(3-pyridyl)-5-(N-tert-butylcarbamoyl)-2H-pyrazole-3-yl]-3-pyridyl}acrylate (35 mg) of Example 128 in methanol (7 ml) was added 10% palladium on carbon (water content 52.8%, 71 mg), and the mixture was stirred overnight at room temperature under hydrogen atmosphere. The reaction liquid was filtered, and the solvent was evaporated under reduced pressure to give the title compound (32 mg, " 91%) as an oily product.

¹H-NMR (400 MHz, CDCl₃)δ: 1.49 (9H, t, J = 9.9 Hz), 2.64 (2H, t, J = 7.6 Hz), 2.94 (2H, t, J = 7.6 Hz), 3.68 (3H, s), 6.84 (1H, s), 7.21 (1H, s), 7.37 (1H, dd, J = 8.2, 4.8Hz), 7.44 (1H, d, J = 8.1Hz), 7.58-7.60 (1H, m), 7.75-7.78 (1H, m), 8.29 (1H, d, J = 1.7 Hz), 8.54 (1H, d, J = 2.2 Hz), 8.59 (1H, d, J = 4.6 Hz).
ESI-MSm/z: 408 (M+H)⁺.

[Example 130] N-tert-Butyl-5-[5-(2-carbamoylethyl)-2-pyridyl]-1-(3-pyridyl)-1H-pyrazole-3-carboxamide

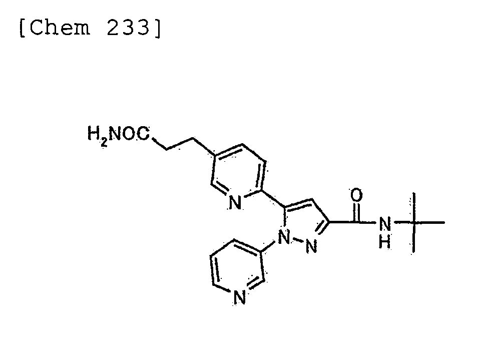

Lithium hydroxide monohydrate (3.6 mg) was added to a solution of the methyl 3-{6-[1-(3-pyridyl)-3-(N-tert-butylcarbamoyl)-1H-pyrazol-5-yl]-3-pyridyl}propionate (32 mg) of Example 129 in tetrahydrofuran (3 ml) and water (1 ml) at room temperature, and the mixture was stirred for 4 hours. After adding 1N aqueous hydrochloric acid (86.4 µl) to the reaction liquid, the solvent was evaporated under reduced pressure, and the residue was dissolved in N,N-dimethylformamide (5 ml). To this solution ammonium chloride (42 mg), 1-hydroxy benzotriazole (11 mg), triethylamine (109 µl), and 1-ethyl-3- (3-dimethylaminopropyl) carbodiimide hydrochloride (30 mg) were added at room temperature, and the mixture was stirred for 2 days. The reaction liquid was evaporated under reduced pressure, and the residue was purified by thin layer chromatography on silica gel (methanol-chloroform) to give the title compound (21 mg, 68%) as a solid.

¹H-NMR (400 MHz, CDCl₃)δ: 1.49 (9H, s), 2.53 (2H, t, J = 7.6 Hz), 2.97 (2H, t, J = 7.4 Hz), 5.58 (1H, s), 5.71 (1H, s), 6.86 (1H, s), 7.18 (1H, s), 7.37 (1H, dd, J = 8.3, 4.9 Hz), 7.41 (1H, d, J = 8.0 Hz), 7.60 (1H, dd, J = 8.1, 2.2 Hz), 7.76-7.79 (1H, m), 8.30 (1H, d, J = 2.2 Hz), 8.48 (1H, d, J = 2.4 Hz), 8.58 (1H, dd, J = 4.9, 1.5 Hz).
ESI-MSm/z: 393 (M+H)⁺.
[Example 131] N-tert-Butyl-5-(5-aminomethyl-2-pyridyl)-1-(3-pyridyl)-1H-pyrazole-3-carboxamide hydrochloride (An alternative for the synthesis of Example 27)

Nickel-silica/alumina (content, about 65%; 0.70 g) was added to a solution of the N-tert-butyl-5-(5-cyano-2-pyridyl)-1-(3-pyridyl)-1H-pyrazole-3-ca rboxamide (1.10 g) of Example 123 in 2M ammonia-ethanol (30 ml), and the mixture was stirred in an autoclave under hydrogen atmosphere (8 atm) at 120°C for 1.5 hours. After cooling with air, the reaction liquid wasfilteredthroughcelite, and the solvent was evaporatedunder reduced pressure to give N-tert-butyl-5-(5-aminomethyl-2-pyridyl)-1-(3-pyridyl)-1H-pyrazol e-3-carboxamide (1.06 g). Triethylamine (0.5 ml) and di-tert-butoxy dicarbonate (0.760 g) were added to a solution of this aminomethyl derivative (1.16 g) in dichloromethane (50 ml) at room temperature, and the mixture was stirred for 3 hours. The solvent was evaporated under reduced pressure, and the residue was purified by column chromatography on silica gel (methanol-dichloromethane) to give N-tert-butyl-5-[5-(tert-butoxycarbonyl)aminomethyl-2-pyridyl]-1-( 3-pyridyl)-1H-pyrazole-3-carboxamide (890 mg, 65%) as an amorphous product.

¹H-NMR (400 MHz, CDCl₃)δ: 1.45 (9H, s), 1.49 (9H, s), 4.31-4.32 (2H, m), 5.06 (1H, br s), 6.85 (1H, br s), 7.21 (1H, s), 7.34-7.37 (1H, m), 7.47 (1H, d, J = 8.1 Hz), 7.67-7.69 (1H, m), 7.72-7.75 (1H, m), 8.28-8.34 (1H, m), 8.54 (1H, d, J = 2.4 Hz), 8.58-8.60 (1H, m).
The procedure of Example 27 was repeated by using this tert-butoxycarbonylaminomethyl derivative (1.1 g) and 1M hydrochloric acid-ethanol (90 ml) to give the title compound (980 mg, 90%) as a solid.FAB-MSm/z: 351 (M+H)⁺.

[Example 132] 5-(5-Ethynyl-2-pyridyl)-N-(2-fluoro-1,1-dimethylethyl)-1-(3-pyridyl)-1H-pyrazole-3-carboxamide

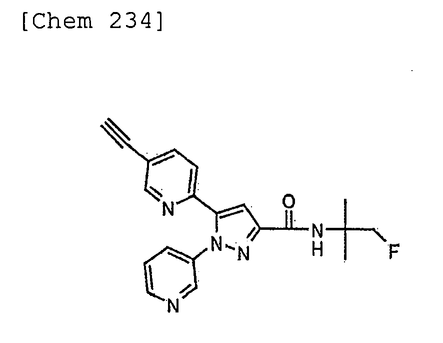

To a solution of the ethyl 1-(3-pyridyl)-5-{5-[2-(trimethylsilyl)ethynyl]-2-pyridyl}-1H-pyra zole-3-carboxylate (0.260 g) of Referential Example 76 in a mixture of ethanol (10 ml) and tetrahydrofuran (5 ml) was added 1N aqueous sodium hydroxide (2.00 ml) at room temperature, and the mixture was stirred for 2 hours. 1N aqueous hydrochloric acid (2.00 ml) was added to the reaction liquid, and the solvent was evaporated under reduced pressure, and the 2-amino-1-fluoro-2-methylpropane hydrochloride (0.153 g) of Referential Example 71, 1-hydroxybenzotriazole (0.122 g), and N,N-dimethylformamide (15 ml) were added to the residue at room temperature, and to this suspensionwere added triethylamine (0.280 ml) and 1- (3-dimethylaminopropyl) -3-ethylcarbodiimide hydrochloride (0.153 g), and the mixture was stirred for 4.5 days. The solvent was evaporated under reduced pressure, and ethyl acetate and saturated aqueous sodium bicarbonate were added to the residue and the phases' were separated. The aqueous layer was further extracted with ethyl acetate, and the combined organic layers were dried over anhydrous sodium sulfate. After filtration, the solvent was evaporated under reduced pressure, and the residue was purifiedby column chromatography on silica gel (ethyl acetate-hexane) to give the title compound (0.183 g, 75%) as a solid.

¹H-NMR (400 MHz, CDCl₃)δ: 1.50 (3H, s), 1.50 (3H, s), 3.27 (1H, s), 4.54 (1H, s), 4.66 (1H, s), 6.89 (1H, br s), 7.26 (1H, s), 7.39 (1H, ddd, J = 8.1, 4.8, 0.7 Hz), 7.48 (1H, dd, J = 8.2, 1.0 Hz), 7.75 (1H, ddd, J = 8.2, 2.4, 1.5 Hz), 7.81 (1H, dd, J = 8.2, 2.1 Hz), 8.50 (1H, dd, J = 2.2, 0.7 Hz), 8.55-8.56 (1H, m), 8.63 (1H, dd, J = 4.9, 1.5 Hz). ESI-MSm/z: 364 (M+H)⁺.

[Example 133] N-(2-Fluoro-1,1-dimethylethyl)-5-(5-acetyl-2-pyridyl)-1-(3-pyridyl)-1H-pyrazole-3-carboxamide

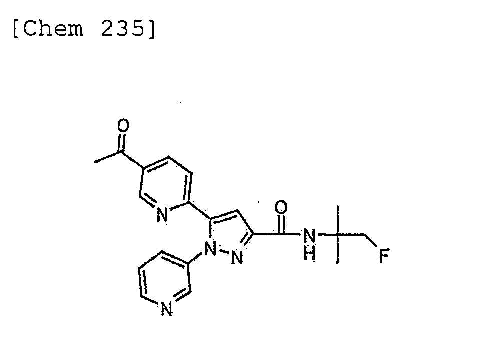

Sulfuric acid (0.0360 ml) was added to a suspension of the 5-(5-ethynyl-2-pyridyl)-N-(2-fluoro-1,1-dimethylethyl)-1-(3-pyrid yl)-1H-pyrazole-3-carboxamide (0.120 g) of Example 132 and mercuric sulfate (97.9 mg) in 75% aqueous acetone(2 ml), and the mixture was heated under reflux for 2 hours. After cooling with air, dichloromethane and potassium carbonate were added to the reaction liquid. The mixture was filtered and the solvent was evaporated under reduced pressure, and the residue was purifiedby column chromatography on silica gel (hexane-ethyl acetate) to give the title compound (64.5 mg, 51%) as an amorphous solid.

¹H-NMR (400 MHz, CDCl₃)δ: 1.50 (3H, s), 1.50 (3H, s), 2.61 (3H, s), 4.54 (1H, s), 4.66 (1H, s), 6.89 (1H, br s), 7.34 (1H, s), 7.41 (1H, dd, J = 8.1, 4.9 Hz), 7.64 (1H, d, J = 8.3 Hz), 7.78 (1H, ddd, J = 8.1, 2.4, 1.5 Hz), 8.27 (1H, dd, J = 8.3, 2.2 Hz), 8.55 (1H, d, J = 2.4 Hz), 8.64-8.66 (1H, m), 8.93 (1H, d, J = 1.7 Hz).
ESI-MSm/z: 382 (M+H)⁺.

[Example 134] 1-(6-Methoxy-3-pyridyl)-5-(2-pyridyl)-1H-pyrazole-3-carboxylic acid N-piperidin-1-ylamide

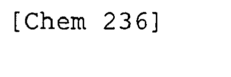

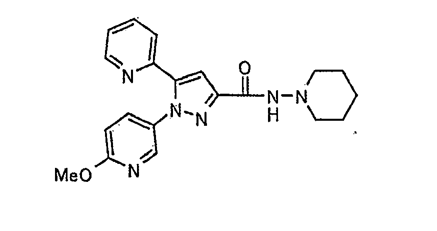

The procedure of Example 1 was repeated by using the 1-(6-methoxy-3-pyridyl)-5-(2-pyridyl)-1H-pyrazole-3-carboxylic acid (200 mg) of Referential Example 4 and N-aminopiperidine (68 mg) to give the title compound (230 mg, 89.8%) as a solid.

¹H-NMR (400 MHz, CDCl₃)δ: 1.40-1.50 (2H, m), 1.75-1.80 (4H, m), 2.86-2.95 (4H, m), 3.96 (3H, s), 6.76 (1H, d, J = 8.8 Hz), 7.20-7.23 (1H, m), 7.44 (1H, d, J = 7.8 Hz), 7.59-7.72 (3H, m), 8.12 (1H, d, J = 2.7 Hz), 8.46-8.48 (1H, m).
ESI-MSm/z: 379 (M+H)⁺.
Hydrochloride of the title compound (150mg, 53%) was alsoproduced by adding a 1.0M solution of hydrochloric acid in ethanol (0.6 ml) to a solution of the title compound in diethylether, and collecting the resulting crystal precipitate by filtration.

¹H-NMR (400 MHz, DMSO-d₆)δ: 1.40-1.50 (2H, m), 3.20-3.30 (4H, m), 3.9 (3H, s).
EI-MSm/z: 378 (M⁺).

[Example 135] 1-(6-Methoxy-3-pyridyl)-5-phenyl-1H-pyrazole-3-carboxylic acid N-piperidin-1-ylamide

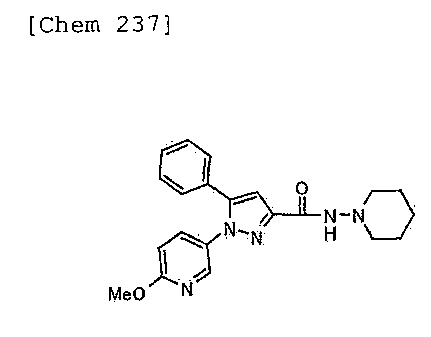

The procedure of Example 7 was repeated by using the 1-(6-methoxy-3-pyridyl)-5-phenyl-1H-pyrazole-3-carboxylicacid(300 mg) of Referential Example 5 and N-aminopiperidine (102 mg) to give the title compound (250 mg, 65%) as a solid.

¹H-NMR (400MHz, CDCl₃)δ: 1.40-1.50 (2H, m), 1.75-1.85 (4H, m), 2.80-3.00 (4H, m), 3.95 (3H, s), 6.73 (1H, dd, J = 8.8, 0.7 Hz), 7.09 (1H, s), 7.20-7.26 (2H, m), 7.30-7.35 (2H, m), 7.49 (1H, dd, J = 8.8, 2.7 Hz), 7.65 (1H, s), 8.13 (1H, bs).
EI-MSm/z: 377 (M⁺).

[Example 136] N-tert-Butyl-5-[5-(methanesulfonylamino)methyl-2-pyridyl]-1-phenyl-1H-pyrazole-3-carboxamide

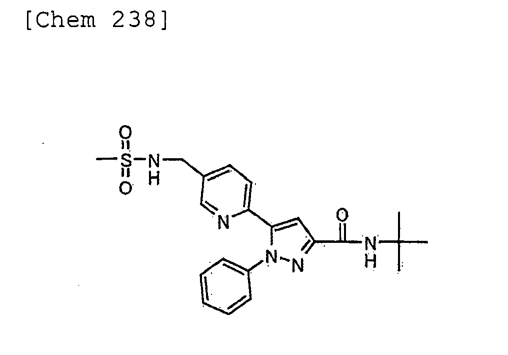

Triethylamine (0.028 ml) and methanesulfonyl chloride (0.018 ml) were added to a solution of the N-tert-butyl-5-(5-aminomethyl-2-pyridyl)-1-phenyl-1H-pyrazole-3-c arboxamide (67 mg) of Example 74 in dichloromethane (5 ml) at room temperature, and the mixture was stirred for 16 hours. Water and dichloromethane were added to the reaction liquid and the phases were separated, and the organic layer was dried over anhydrous magnesium sulfate. After filtration, the solvent was evaporated under reduced pressure, and the residue was purified by thin layer chromatography on silica gel (methanol-dichloromethane) to give the title compound (62 mg, 75%) as an amorphous product.

¹H-NMR (400 MHz, CDCl₃)δ: 1.48 (9H, s), 2.95 (3H, s), 4.33 (2H, d, J = 6.35 Hz), 5.32 (1H, t, J = 6.35 Hz), 6.88 (1H, br s), 7.13 (1H, s), 7.25-7.34 (3H, m), 7.36-7.39 (3H, m), 7.71 (1H, dd, J = 8.06, 2.20 Hz), 8.46 (1H, br s).
EI-MSm/z: 427 (M⁺).

[Example 137] N-tert-Butyl-5-[5-(methanesulfonylamino)methyl-2-pyridyl]-1-(3-pyridyl)-1H-pyrazole-3-carboxamide

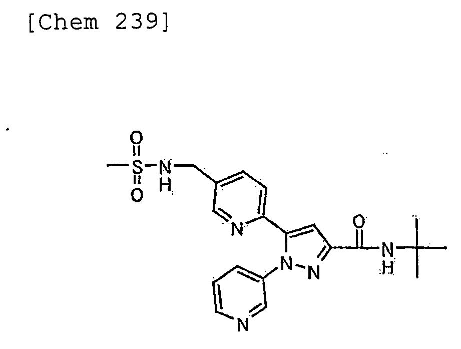

Triethylamine (0.070 ml) and methanesulfonyl chloride (0.014 ml) were added to a suspension of the N-tert-butyl-5-(5-aminomethyl-2-pyridyl)-1-(3-pyridyl)-1H-pyrazol e-3-carboxamidehydrochloride (70mg) of Example 131 in dichloromethane (5 ml) at room temperature, and the mixture was stirred for 16 hours. Water and dichloromethane were added to the reaction liquid and the phases were separated, and the organic layer was dried over anhydrous magnesium sulfate. After filtration, the solvent was evaporated under reduced pressure, and the residue was purified by thin layer chromatography on silica gel (methanol-dichloromethane) to give the title compound (60 mg, 88%) as an amorphous product.

¹H-NMR (400 MHz, CDCl₃)δ: 1.46 (9H, s), 2.96 (3H, s), 4.43 (2H, d, J = 6.35 Hz), 5.32-5.40 (1H, m), 6.85 (1H, br s), 7.17 (1H, s), 7.35-7.39 (1H, m), 7.49 (1H, dd, J = 8.06, 0.49 Hz), 7.76-7.82 (2H, m), 8.38 (1H, d, J = 1.47 Hz), 8.47 (1H, t, J = 0.49 Hz), 8.58 (1H, dd, J = 4.88, 1.47 Hz).
FAB-MSm/z: 429 (M+H)⁺.

[Example 138] N-tert-Butyl-5-[5-(acetylamino)methyl-2-pyridyl]-1-(3-pyridyl)-1H-pyrazole-3-carboxamide

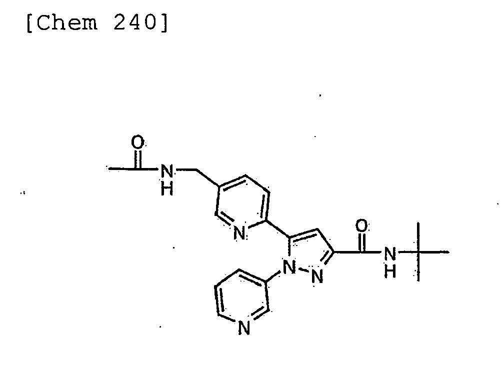

Triethylamine (0.035 ml) and acetyl chloride (0.017 ml) were added to a suspension of the N-tert-butyl-5-(5-aminomethyl-2-pyridyl)-1-(3-pyridyl)-1H-pyrazol e-3-carboxamide hydrochloride (70mg) of Example 131 in dichloromethane (5 ml) at room temperature, and the mixture was stirred for 16 hours. Water and dichloromethane were added to the reaction liquid and the phases were separated, and the organic layer was dried over anhydrous magnesium sulfate. After filtration, the solvent was evaporated under reduced pressure, and the residue was purified by thin layer chromatography on silica gel (methanol-dichloromethane) to give the title compound (60 mg, 96%) as an amorphous product.

¹H-NMR (400 MHz, CDCl₃)δ: 1.49 (9H, s), 2.04 (3H, s), 4.43 (2H, d, J = 6.10 Hz), 6.25-6.27 (1H, m), 6.85 (1H, br s), 7.14 (1H, s), 7.35-7.39 (1H, m), 7.42 (1H, d, J = 8.06 Hz), 7.68 (1H, dd, J = 8.06, 2.20 Hz), 7.73-7.76 (1H, m), 8.30 (1H, d, J = 1.95 Hz), 8.51 (1H, d, J = 2.44 Hz), 8.59 (1H, dd, J = 4.88, 1.46 Hz).
FAB-MSm/z: 393 (M+H)⁺.

[Example 139] Methyl {6-[5-(N-tert-butylcarbamoyl)-2-(3-pyridyl)-2H-pyrazol-3-yl]-3-pyridylmethyl}carbamate

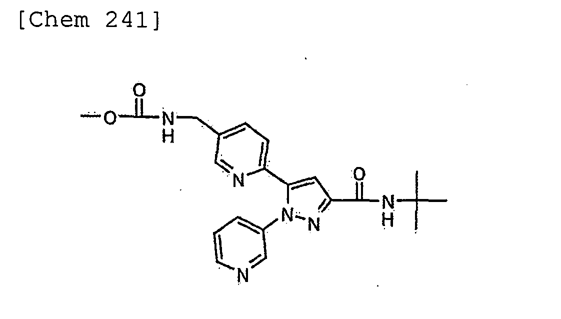

Triethylamine (0.070 ml) and methyl chloroformate (0.014 ml) were added to a suspension of the N-tert-butyl-5-(5-aminomethyl-2-pyridyl)-1-(3-pyridyl)-1H-pyrazol e-3-carboxamide hydrochloride (70 mg) of Example 131 in dichloromethane (5 ml) at room temperature for 16 hours. Water and dichloromethane were added to the reaction liquid and the phases were separated, and the organic layer was dried over anhydrous magnesium sulfate. After filtration, the solvent was evaporated under reduced pressure, and the residue was purified by thin layer chromatography on silica gel (methanol-dichloromethane) to give the title compound (55 mg, 85%) as an amorphous product.

¹H-NMR (400 MHz, CDCl₃)δ: 1.49 (9H, s), 3.70 (3H, s), 4.37 (2H, d, J = 6.10 Hz), 5.25-5.30 (1H, m), 6.85 (1H, br s), 7.20 (1H, s), 7.35-7.39 (1H, m), 7.46 (1H, d, J = 8.06 Hz), 7.68-7.77 (2H, m), 8.34 (1H, d, J = 1.71 Hz), 8.53 (1H, d, J = 1.46 Hz), 8.59 (1H, dd, J = 4.88, 1.47 Hz).
FAB-MSm/z: 409 (M+H)⁺.

[Example 140] N-tert-Butyl-5-[5-(cyclopentanecarbonylamino)methyl-2-pyridyl]-1-phenyl-1H-pyrazole-3-carboxamide

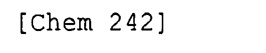

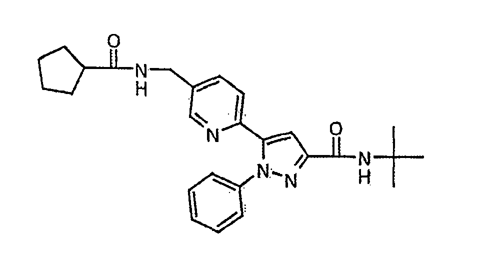

Triethylamine (0.030 ml) and cyclopentanecarbonyl chloride (19 mg) were added to a solution of the N-tert-butyl-5-(5-aminomethyl-2-pyridyl)-1-phenyl-1H-pyrazole-3-c arboxamide (50 mg) of Example 74 in dichloromethane (5 ml) at room temperature, and the mixture was stirred for 2 days. Methanol was added to the reaction liquid, and the mixture was stirred for 10 minutes. The reaction solvent was then evaporated under reduced pressure, and the residue was purified by thin layer chromatography on silica gel (methanol-chloroform) to give the title compound (53 mg, 83%) as an amorphous product.

¹H-NMR (400 MHz, CDCl₃)δ: 1.48 (9H, s), 1.50-1.90 (8H, m), 2.53-2.62 (1H, m), 4.44 (2H, d, J = 6.10 Hz), 6.25-6.30 (1H, m), 6.88 (1H, br s), 7.12 (1H, s), 7.19 (1H, d, J = 8.06 Hz), 7.26-7.37 (6H, m), 7.56 (1H, dd, J = 8.06, 2.20 Hz), 8.40 (1H, d, J = 1.71 Hz).
EI-MSm/z: 445 (M⁺).

[Example 141] Phenyl {6-[5-(N-tert-butylcarbamoyl)-2-phenyl-2H-pyrazol-3-yl]-3-pyridylmethyl}carbamate

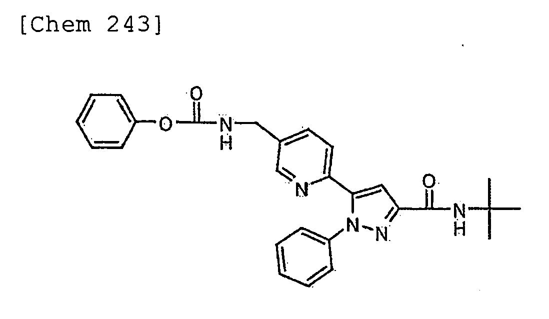

Triethylamine (0.030 ml) and phenyl chloroformate (0.018 ml) were added to a solution of the N-tert-butyl-5-(5-aminomethyl-2-pyridyl)-1-phenyl-1H-pyrazole-3-c arboxamide (50 mg) of Example 74 in dichloromethane (5 ml) at room temperature, and the mixture was stirred for 2 days. Methanol was added to the reaction liquid, and the mixture was stirred for 10 minutes. The reaction solvent was evaporated under reduced pressure, and the residue was purified by thin layer chromatography on silica gel (methanol-chloroform) to give the title compound (48 mg, 70%) as an amorphous product.

¹H-NMR (400MHz, CDCl₃)δ: 1.48 (9H, s), 4.45 (2H, d, J = 6.10 Hz), 5.75-5.78 (1H, m), 6.88 (1H, br s), 7.10-7.40 (7H, m), 7.66 (1H, dd, J = 8.06, 2.20 Hz), 8.49 (1H, br s). FAB-MSm/z: 470 (M+H)⁺.

[Example 142] Morpholine-4-carboxylic acid{6-[5-(N-tert-butylcarbamoyl)-2-phenyl-2H-pyrazol-3-yl]-3-pyridylmethyl}amide

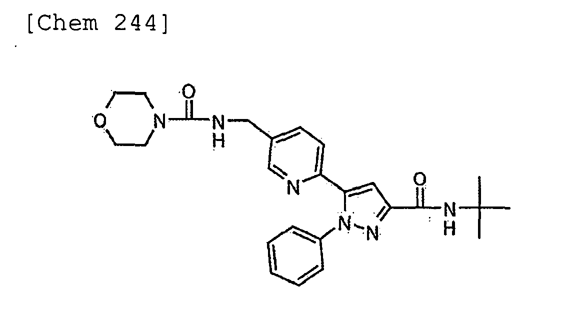

Triethylamine (0.030 ml) and 4-nitrophenyl chloroformate (30 mg) were added to a solution of the N-tert-butyl-5-(5-aminomethyl-2-pyridyl)-1-phenyl-1H-pyrazole-3-c arboxamide (50 mg) of Example 74 in dichloromethane (5 ml) under ice cooling, and the mixture was stirred for 30 minutes. To the reaction liquidwasaddedmorpholine (0.030 ml) under ice cooling, and the mixture was stirred at room temperature for 2 days. Methanol was added to the reaction liquid, and the mixture was stirred for 10 minutes. The reaction solvent was evaporated under reducedpressure, and the residue was purified by thin layer chromatography on silica gel (methanol-chloroform) to give the title compound (40 mg, 60%) as an amorphous product.

¹H-NMR (400 MHz, CDCl₃)δ: 1.47 (9H, s), 3.35-3.46 (4H, s), 3.65-3.72 (4H, m), 4.43 (2H, d, J = 5.62 Hz), 5.25-5.32 (1H, m), 6.88 (1H, br s), 7.09 (1H, s), 7.18 (1H, d, J = 8.06 Hz), 7.20-7.40 (5H, m), 7.61 (1H, d, J = 6.35 Hz), 8.42 (1H, s).
EI-MSm/z: 462 (M⁺).

[Example 143] N-tert-Butyl-1-phenyl-5-(5-{[3-(tetrahydro-2H-pyran-4-yl)ureido]methyl}-2-pyridyl)-1H-pyrazole-3-carboxamide

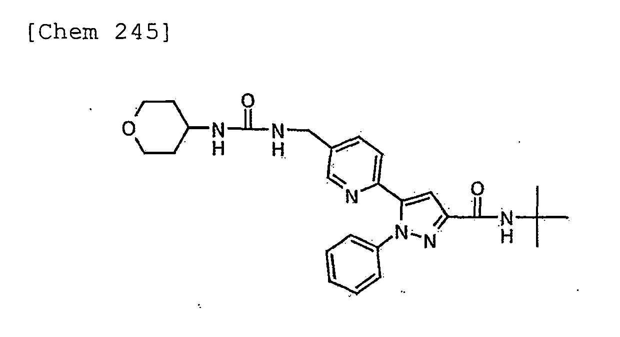

Triethylamine (0.030 ml) and 4-nitrophenyl chloroformate (30 mg) were added to a solution of the N-tert-butyl-5-(5-aminomethyl-2-pyridyl)-1-phenyl-1H-pyrazole-3-c arboxamide (50 mg) of Example 74 in dichloromethane (5 ml) under ice cooling, and the mixture was stirred for 30 minutes. Tetrahydro-2H-pyran-4-ylamine (0.030 ml) was added to the reaction mixture under ice cooling, and the mixture was stirred at room temperature for 2 days. Methanol was added to the reaction liquid, and the mixture was stirred for 10 minutes. The reaction solvent was evaporated under reduced pressure, and the residue was purified by thin layer chromatography on silica gel (methanol-dichloromethane) to give the title compound (45mg, 66%) as a colorless amorphous product.

¹H-NMR (400 MHz, CDCl₃)δ: 1.49 (9H, s), 1.67-1.92 (4H, s), 3.42-3.48 (2H, m), 3.75-3.88 (1H, m), 3.91-3.94 (2H, m), 4.39 (2H, d, J = 6.10 Hz), 5.35 (1H, d, J = 8.06 Hz), 6.05 (1H, t, J = 6.10 Hz), 6.96 (1H, s), 6.96 (1H, d, J = 3.17 Hz), 7.02 (1H, d, J = 8.06 Hz), 7.26-7.28 (2H, m), 7.35-7.39 (3H, m), 7.37 (1H, dd, J= 6.59, 2.93 Hz), 8.42 (1H, d, J = 1.71 Hz).
EI-MSm/z: 476 (M⁺).

[Example 144] 5-(5-Cyano-2-pyridyl)-1-(6-methyl-3-pyridyl)-1H-pyrazole-3-carboxylic acid N-methyl-N-isopropylamide

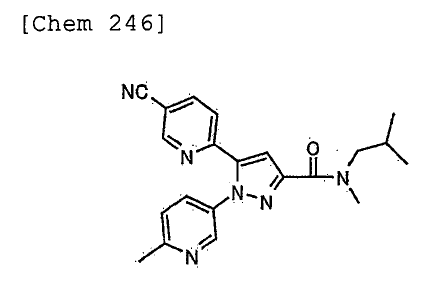

The procedure of Example 7 was repeated by using the 5-(5-cyano-2-pyridyl)-1-(6-methyl-3-pyridyl)-1H-pyrazole-3-carbox ylic acid (150 mg) of Referential Example 75 and isopropylamine (47 mg) to give the title compound (170 mg, 92%) as a solid.

¹H-NMR (400 MHz, CDCl₃)δ: 0.89 (1/2 x 3H, d, J = 6.59 Hz), 0.98 (1/2 x 3H, d, J = 6.84 Hz), 2.00-2.20 (1H, m), 2.63 (1/2 x 3H, s), 2.63 (1/2 x 3H, s), 3.13 (1/2 x 3H, s), 3.39 (1/2 x 3H, s), 3.42 (1/2 x 2H, d, J = 7.81 Hz), 3.70 (1/2 x 3H, d, J = 7.57 Hz), 7.24 (1H, d, J = 8.30 Hz), 7.29 (1H, d, J = 8.06 Hz), 7.57-7.64 (2H, m), 7.98-8.01 (1H, m), 8.41 (1H, d, J = 2.44 Hz), 8.69-8.70 (1H, m).
EI-MSm/z: 374 (M⁺).

[Example 145] 5-(5-Carbamoyl-2-pyridyl)-1-(6-methyl-3-pyridyl)-1H-pyrazole-3-carboxylic acid N-methyl-N-isopropylamide

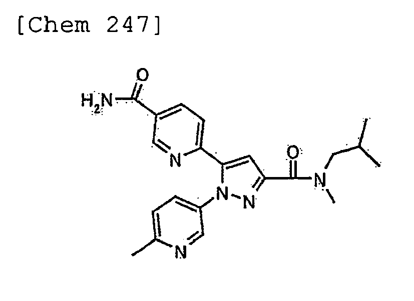

The procedure of Example 21 was repeated by using the 5-(5-cyano-2-pyridyl)-1-(6-methyl-3-pyridyl)-1H-pyrazole-3-carbox ylic acid N-methyl-N-isopropylamide (170 mg) of Example 144 to give the title compound (110 mg, 61%) as a solid.

¹H-NMR (400 MHz, CDCl₃)δ: 0.83 (1/2 x 3H, d, J = 6.59 Hz), 0.90 (1/2 x 3H, d, J = 6.59 Hz), 1.95-2.05 (1H, m), 7.30 (1H, d, J = 10.99 Hz), 7.35 (1H, d, J = 8.30 Hz), 7.60-7.65 (2H, m), 7.65-7.70 (1H, m), 7.84 (1H, d, J = 8.30 Hz), 8.16 (1H, br s), 8.28 (1H, dd, J = 8.30, 2.20 Hz), 8.39-8.41 (1H, m), 8.84 (1H, d, J = 1.95 Hz).
EI-MSm/z: 392 (M⁺).
[Example 146] 5-(4-Cyanophenyl)-1-(3-pyridyl)-1H-pyrazole-3-carboxylic acid N-ethyl-N-methylamide

The procedure of Example 7 was repeated by using the 5-(4-cyanophenyl)-1-(3-pyridyl)-1H-pyrazole-3-carboxylic acid (450 mg) of Referential Example 24 and N-methylethylamine (120 mg) to give the title compound (500 mg, 97%) as an amorphous product.

¹H-NMR (400 MHz, CDCl₃)δ: 1.24-1.33 (3H, m), 3.13 (1/2 x 3H, s), 3.41 (1/2 x 3H, s), 3.63 (1/2 x 3H, q, J = 7.32 Hz), 3.84 (1/2 x 3H, q, J = 6.84 Hz), 7.02 (1H, d, J = 8.55 Hz), 7.35-7.38 (3H, m), 7.60-7.67 (3H, m), 8.55-8.65 (2H, m).
EI-MSm/z: 331 (M⁺).

[Example 147] 5-[4-(Methanesulfonylamino)methylphenyl]-1-(3-pyridyl)-1H-pyrazole-3-carboxylic acid N-ethyl-N-methylamide

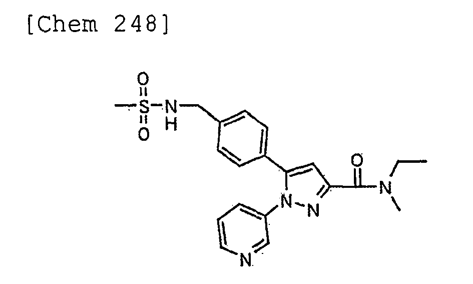

### 1) 5-(4-Aminomethylphenyl)-1-(3-pyridyl)-1H-pyrazole-3-carboxylic acid N-ethyl-N-methylamide

To a solution of the 5-(4-cyanophenyl)-1-(3-pyridyl)-1H-pyrazole-3-carboxylic acid N-ethyl-N-methylamide (500 mg) of Example 146 in methanol (10 ml) were added 1N aqueous hydrochloric acid (2.8 ml) and 10% palladium on carbon (200 mg) at room temperature, and the mixture was stirred for 5 hours under hydrogen atmosphere. The reaction liquid was filtered, and the solvent was evaporated under reduced pressure. Saturated aqueous sodium bicarbonate and dichloromethane were added to the residue and the phases were separated, and the organic layer was dried over anhydrous magnesium sulfate. After filtration, the solvent was evaporated under reduced pressure to give 5-(4-aminomethylphenyl)-1-(3-pyridyl)-1H-pyrazole-3-carboxylic acid N-ethyl-N-methylamide (270 mg, 53%) as an amorphous product.
EI-MS (m/z) : 336(M⁺).

### 2) The title compound

The procedure of Example 136 was repeated by using the 5-(4-aminomethylphenyl)-1-(3-pyridyl)-1H-pyrazole-3-carboxylic acid N-ethyl-N-methylamide (270 mg) and methanesulfonyl chloride (110 mg) to give the title compound (141 mg, 42%) as an amorphous product.

¹H-NMR (400 MHz, CDCl₃)δ: 1.23-1.32 (3H, m), 2.92 (3H, s), 3.12 (1/2 x 3H, s), 3.40 (1/2 x 3H, s), 3.62 (1/2 x 3H, q, J = 7.08 Hz), 3.83 (1/2 x 3H, q, J = 7.08 Hz), 4.32 (2H, d, J = 6.35 Hz), 5.43-5.50 (1H, m), 6.85 (1/2 x 1H, s), 6.88 (1/2 x 1H, s), 7.19-7.36 (5H, m), 7.61-7.65 (1H, m), 8.50-8.55 (2H, m).
EI-MSm/z: 413 (M⁺).

[Example 148] N-tert-Butyl-5-[4-(methanesulfonylamino)methylphenyl]-1-(3-pyridyl)-1H-pyrazole-3-carboxamide

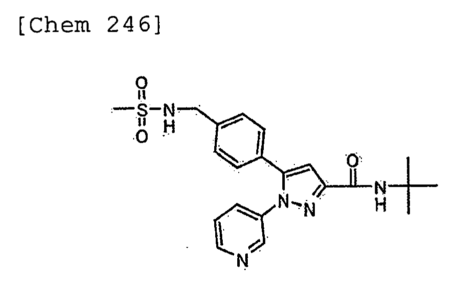

### 1) N-tert-Butyl-5-(4-aminomethylphenyl)-1-(3-pyridyl)-1H-pyrazole-3-carboxamide

The procedure of Example 147(1) was repeated by using the N-tert-butyl-5-(4-cyanophenyl)-1-(3-pyridyl)-1H-pyrazole-3-carbox amide (495 mg) of Example 29(1) to give N-tert-butyl-5-(4-aminomethylphenyl)-1-(3-pyridyl)-1H-pyrazole-3-carboxamide (435 mg, 86%) as an amorphous product.
FAB-MS(m/z): 350 (M+H)⁺.

### 2) The title compound

The procedure of Example 136 was repeated by using tert-butyl-5-(4-aminomethylphenyl)-1-(3-pyridyl)-1H-pyrazole-3-ca rboxamide (210 mg) and methanesulfonyl chloride (83 mg) to give the title compound (141 mg, 54%) as an amorphous product.

¹H-NMR (400 MHz, CDCl₃)δ: 1.49 (9H, s), 2.93 (3H, s), 4.33 (2H, d, J = 5.86 Hz), 5.11 (1H, t, J = 5.86 Hz), 6.86 (1H, br s), 6.99 (1H, d, J = 0.73 Hz), 7.18 (2H, d, J = 8.06 Hz), 7.32-7.35 (3H, m), 7.65 (1H, d, J = 8.30 Hz), 8.53 (1H, d, J = 2.44 Hz), 8.57 (1H, d, J = 4.64 Hz). EI-MSm/z: 427 (M⁺).

[Example 149] Methyl {6-[5-(N-tert-butylcarbamoyl)-2-(3-pyridyl)-2H-pyrazol-3-yl]-4-phenylmethyl}carbamate

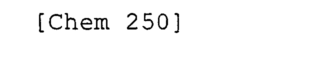

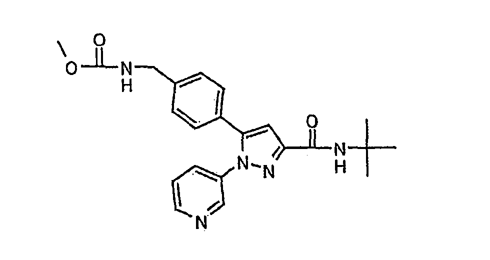

The procedure of Example 139 was repeated by using the tert-butyl-5-(4-aminomethylphenyl)-1-(3-pyridyl)-1H-pyrazole-3-ca rboxamide (210 mg) of Example 148 (1) and methyl chloroformate (68 mg) to give the title compound (92 mg, 37%) as an amorphous product.

¹H-NMR (400 MHz, CDCl₃)δ: 1.49 (9H, s), 3.71 (3H, s), 4.38 (2H, d, J = 6.10 Hz), 5.05-5.09 (1H, m), 6.84 (1H, br s), 7.01 (1H, s), 7.16 (2H, d, J = 8.30 Hz), 7.25 (2H, d, J = 8.30 Hz), 7.31-7.35 (1H, m), 7.63 (1H, d, J = 8.06 Hz), 8.58-8.60 (2H, m).
EI-MSm/z: 407 (M⁺).

[Example 150] N-tert-Butyl-5-(5-carbamoylmethyloxy-2-pyridyl)-1-(3-pyridyl)-1H-pyrazole-3-carboxamide

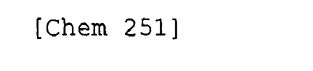

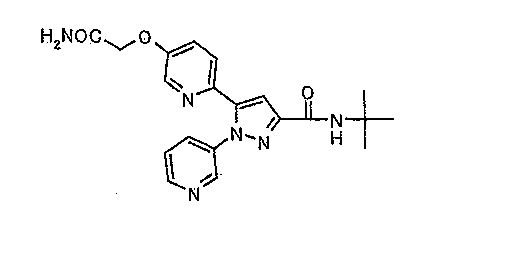

To a solution of the ethyl 5-(5-carbamoylmethyloxy-2-pyridyl)-1-(3-pyridyl)-1H-pyrazole-3-ca rboxylate (0.240 g) of Referential Example 77 in a mixture of ethanol (5 ml) and tetrahydrofuran (5 ml) was added 1N aqueous sodium hydroxide (2.00 ml) at room temperature, and the mixture was stirred for 3.5 hours. The solid generated in the reaction liquid was collected by filtration. tert-Butylamine (0.380 ml) and 1-(3-dimethylaminopropyl)-3-ethylcarbodiimide hydrochloride (0.276 g) were added to a suspension of the resulting solid and 1-hydroxybenzotriazole (0.220 g) in N,N-dimethylformamide (10 ml) at room temperature, and the mixture was stirred for 2.5 days. To the reaction liquid were further added 1-hydroxybenzotriazole (0.220 g), tert-butylamine (0.380 ml), 1-(3-dimethylaminopropyl)-3-ethylcarbodiimide hydrochloride (0.276 g), and N,N-dimethylformamide (10 ml), and the mixture was stirred at 60°C for 6.5 hours. After coolingwith air, the solvent in the reaction liquid was evaporated under reduced pressure, and ethyl acetate and saturated aqueous sodium bicarbonate were added to the residue and the phases were separated, and the aqueous layer was further extracted with ethyl acetate. The combined organic layers were dried over anhydrous sodium sulfate. After filtration, the sol vent was evaporated under reduced pressure, and the residue was purified by column chromatography on silica gel (methanol-dichloromethane) to give the title compound (0.101 g, 39%) as a solid. The di-tert-butylcarbamoyl derivative of Example 151 was also produced from different fraction.

¹H-NMR (CDCl₃)δ: 1.49 (9H, s), 4.54 (2H, s), 5.62 (1H, br s), 6.45 (1H, br s), 6.84 (1H, br s), 7.18 (1H, s), 7.25-7.29 (1H, m), 7.39 (1H, dd, J = 8.2, 4.8 Hz), 7.50 (1H, d, J = 8.8 Hz), 7.78 (1H, ddd, J = 8.1, 2.4, 1.7 Hz), 8.18 (1H, d, J = 2.7 Hz), 8.51 (1H, br s), 8.60 (1H, d, J = 4.4 Hz).
ESI-MSm/z: 395 (M+H)⁺.

[Example 151] N-tert-Butyl-5-[5-(N-tert-butyl)carbamoylmethyloxy-2-pyridyl]-1-(3-pyridyl)-1H-pyrazole-3-carboxamide

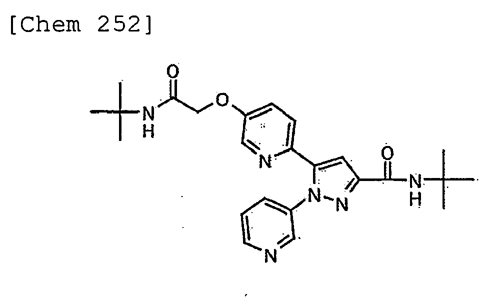

The title compound (93.6 mg, 31%) was produced as a solid from the different fraction of the column chromatography on silica gel (methanol-dichloromethane) used for purification in Example 150.

¹H-NMR (400 MHz, CDCl₃)δ: 1.41 (9H, s), 1.49 (9H, s), 4.40 (2H, s), 6.23 (1H, br s), 6.84 (1H, br s), 7.17 (1H, s), 7.24-7.27 (1H, m), 7.37 (1H, dd, J = 8.1, 4. 9 Hz), 7.49 (1H, d, J = 8.5 Hz), 7.75-7.79 (1H, m), 8.16 (1H, d, J = 2.9 Hz), 8.51 (1H, br s), 8.60 (1H, d, J = 4.4 Hz).
ESI-MSm/z: 451 (M+H)⁺.

[Example 152] N-(2-Hydroxy-1,1-dimethylethyl)-5-(5-methyl-2-pyridyl)-1-(3-pyridyl)-1H-pyrazole-3-carboxamide

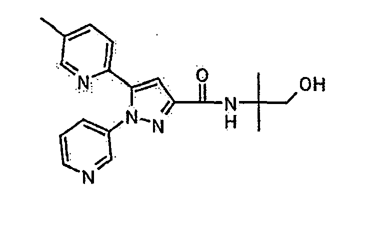

The procedure of Example 7 was repeated by using the 5-(5-methyl-2-pyridyl)-1-(3-pyridyl)-1H-pyrazole-3-carboxylic acid (150 mg) of Referential Example 37 and 2-amino-2-methyl-1-propanol (52 mg) to give the title compound (125 mg, 66%) as a solid.

¹H-NMR (400 MHz, CDCl₃)δ: 1.43 (6H, s), 2.34 (3H, s), 3.56 (2H, d, J = 6.35 Hz), 4.80-4.90 (1H, m), 7.04 (1H, br s), 7.20 (1H, s), 7.35-7.39 (2H, m), 7.53-7.56 (1H, m), 7.74-7.77 (1H, m), 8.27 (1H, d, J = 1.47 Hz), 8.53-8.60 (1H, m), 6.61 (1H, d, J = 0.98 Hz). EI-MSm/z: 351 (M⁺).

[Example 153] N-Ethyl-N-isopropyl-1-(6-methoxy-3-pyridazinyl)-5-(2-pyridyl)-1H-pyrazole-3-carboxamide

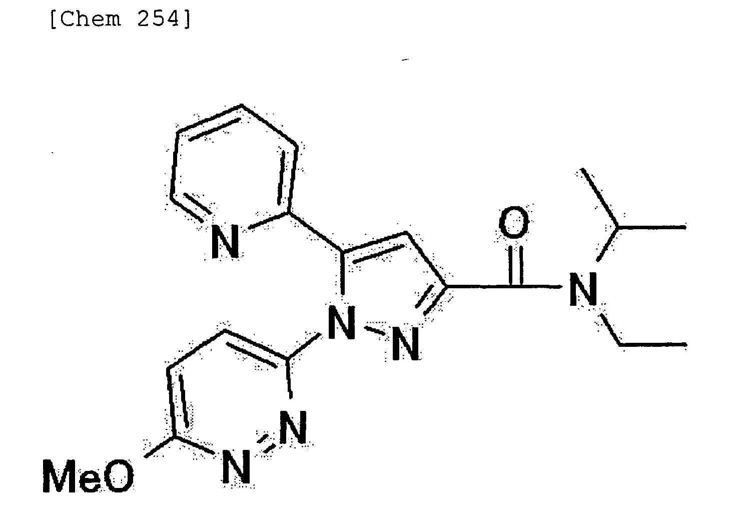

The procedure of Example 1 was repeated by using the 1-(6-methoxy-3-pyridazinyl)-5-(2-pyridyl)-1H-pyrazole-3-carboxylic acid (0.232 g) of Referential Example 9 and N-ethyl-N-isopropylamine (0.171 ml) to give the title compound (0.259 g, 90%) as a solid.

¹H-NMR (400MHz, CDCl₃)δ: 1.24-1.33 (9H, m), 3.44-3.50 (4/3H, m), 3.66-3.72 (2/3H, m), 4.10 (3H, s), 4.75-4.84 (1H, m), 7.00 (2/3H, br s), 7.08 (1/3H, br s), 7.13 (1H, d, J = 9.0 Hz), 7.21-7.24 (1H, m), 7.55-7.60 (1H, m), 7.73-7.77 (1H, m), 7.81-7.86 (1H, m), 8.43 (1H, br s).
ESI-MSm/z: 367 (M+H)⁺.

[Example 154] N-Ethyl-N-methyl-5-(5-amino-2-pyrazinyl)-1-(6-methoxy-3-pyridyl)-1H-pyrazole-3-carboxamide

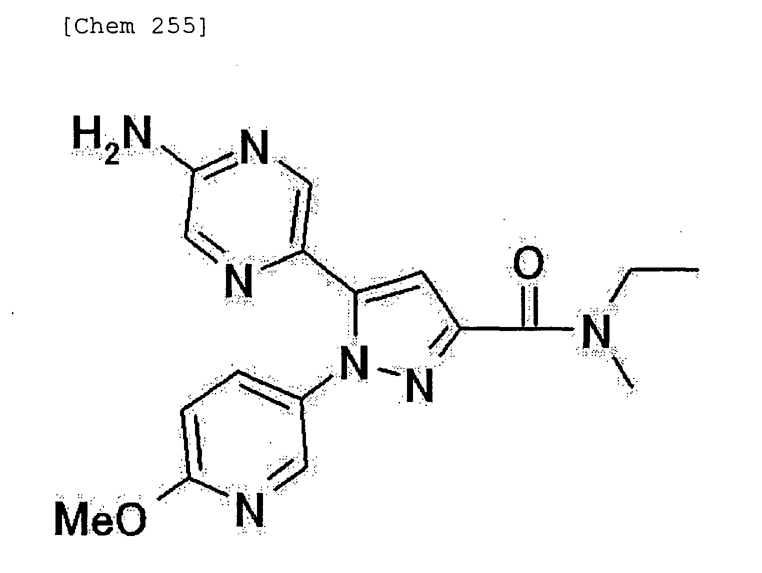

### 1) N-Ethyl-N-methyl-5-[5-(tert-butoxycarbonyl)amino-2-pyrazinyl]-1-(6-methoxy-3-pyridyl)-1H-pyrazole-3-carboxamide

The procedure of Example 1 was repeated by using the 5-[5-(tert-butoxycarbonyl)amino-2-pyrazinyl]-1-(6-methoxy-3-pyrid yl)-1H-pyrazole-3-carboxylic acid (0.420 g) of Referential Example 80 and N-ethylmethylamine (0.175 ml) to give N-ethyl-N-methyl-5-[5-(tert-butoxycarbonyl)amino-2-pyrazinyl]-1-( 6-methoxy-3-pyridyl)-1H-pyrazole-3-carboxamide (0.417 g, 90%) as a solid.

¹H-NMR (400 MHz, CDCl₃)δ: 1.23-1.29 (3H, m), 1.54 (9H, s), 3.12-3.38 (3H, m), 3.59-3.85 (2H, m), 3.97 (3H, s), 6.77 (1H, d, J = 8.8 Hz), 7.13-7.15 (1H, m), 7.49 (1H, m), 7.59-7.64 (1H, m), 8.12 (1H, d, J = 2.7 Hz), 8.34 (1H, d, J = 1.2 Hz), 9.17 (1H, m). EI-MSm/z: 453 (M⁺).

### 2) The title compound

The procedure of Example 95 was repeated by using the N-ethyl-N-methyl-5-[5-(tert-butoxycarbonyl)amino-2-pyrazinyl]-1-( 6-methoxy-3-pyridyl)-1H-pyrazole-3-carboxamide (0.407 g) to give the title compound (0.211 g, 66%) as a solid.

¹H-NMR (400MHz, CDCl₃)δ: 1.19-1.28 (3H, m), 3.11-3.37 (3H, m), 3.58-3.84 (2H, m), 3.95 (3H, s), 4.88 (2H, br s), 6.76 (1H, d, J = 8.8 Hz), 7.01-7.03 (1H, m), 7.59-7.63 (1H, m), 7.88 (1H, m), 8.09 (1H, m), 8.14 (1H, d, J = 2.0 Hz).
FAB-MSm/z: 354 (M+H)⁺.

[Example 155] N,N-Diethyl-5-(5-amino-2-pyrazinyl)-1-(6-methoxy-3-pyridyl)-1H-pyrazole-3-carboxamide

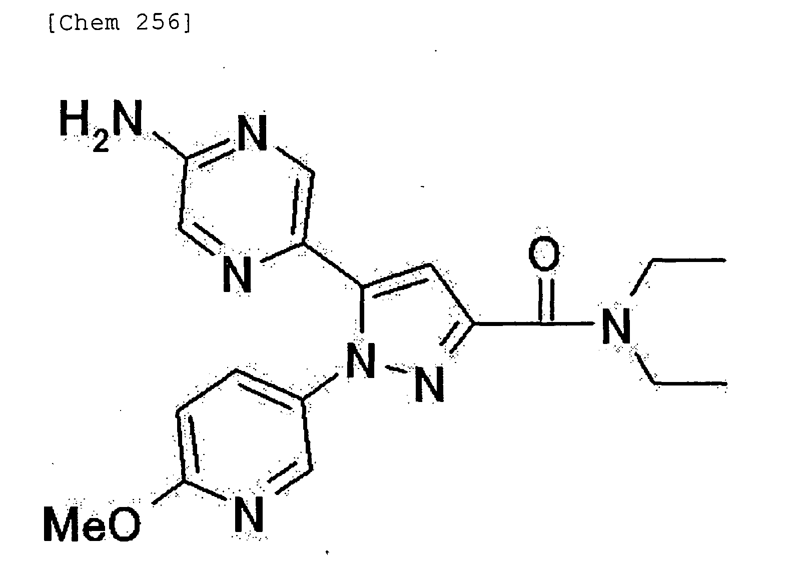

### 1) N,N-Diethyl-5-[5-(tert-butoxycarbonyl)amino-2-pyrazinyl]-1-(6-methoxy-3-pyridyl)-1H-pyrazole-3-carboxamide

The procedure of Example 1 was repeated by using the 5-[5-(tert-butoxycarbonyl)amino-2-pyrazinyl]-1-(6-methoxy-3-pyrid yl)-1H-pyrazole-3-carboxylic acid (0.420 g) of Referential Example 80 and diethylamine (0.213 ml) to give N,N-diethyl-5-[5-(tert-butoxycarbonyl)amino-2-pyrazinyl]-1-(6-met hoxy-3-pyridyl)-1H-pyrazole-3-carboxamide (0.452 g, 95%) as a solid.

¹H-NMR (400 MHz, CDCl₃)δ: 1.25-1.28 (6H, m), 1.54 (9H, s), 3.55-3.60 (2H, m), 3.77-3.82 (2H, m), 3.96 (3H, s), 6.77 (1H, d, J = 8.8 Hz), 7.13 (1H, s), 7.59-7.62 (2H, m), 8.12 (1H, d, J = 2.2 Hz), 8.35 (1H, d, J = 1.5 Hz), 9.17 (1H, d, J = 1.5 Hz).
EI-MSm/z: 467 (M⁺).

### 2) The title compound

The procedure of Example 95 was repeated by using N,N-diethyl-5-[5-(tert-butoxycarbonyl)amino-2-pyrazinyl]-1-(6-met hoxy-3-pyridyl)-1H-pyrazole-3-carboxamide (0.443g) to give the title compound (0.192 g, 55%) as a solid.

¹H-NMR (400MHz, CDCl₃)δ: 1.24-1.28 (6H, m), 3.54-3.59 (2H, m), 3.75-3.81 (2H, m), 3.95 (3H, s), 4.88 (2H, br s), 6.75-6.77 (1H, m), 7.02 (1H, s), 7.60 (1H, dd, J = 8.8, 2.7 Hz), 7.88 (1H, d, J = 1.5 Hz), 8.10 (1H, d, J = 1.5 Hz), 8.14 (1H, m).
FAB-MSm/z: 368 (M+H)⁺.

[Example 156] N-Ethyl-N-methyl-1-(6-methoxy-3-pyridyl)-5-(5-methyl-2-pyrazinyl)-1H-pyrazole-3-carboxamide

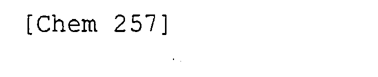

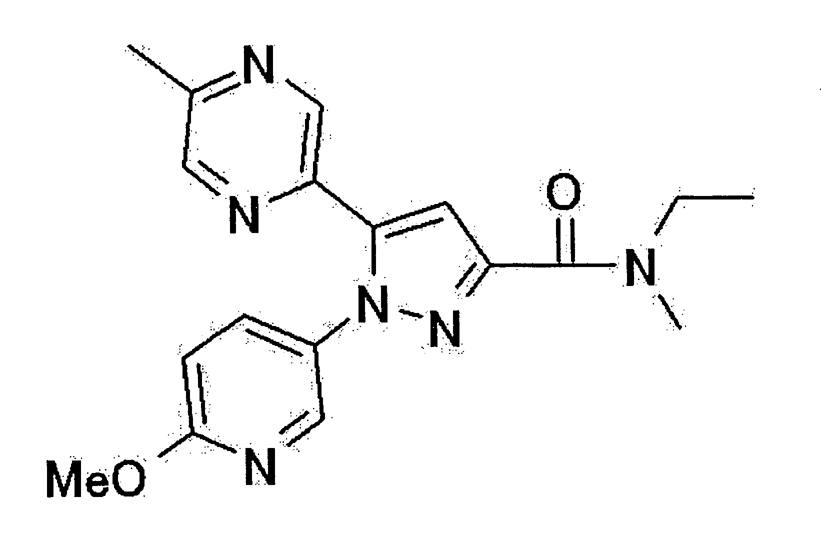

The procedure of Example 1 was repeated by using the 1-(6-methoxy-3-pyridyl)-5-(5-methyl-2-pyrazinyl)-1H-pyrazole-3-ca rboxylic acid (0.250g) of Referential Example 69 andN-ethylmethylamine (0.138 ml) to give the title compound (93.0 mg, 33%) as a solid.

¹H-NMR (400 MHz, CDCl₃)δ: 1.22-1.28 (3H, m), 2.57 (3H, s), 3.12-3.37 (3H, m), 3.59-3.84 (2H, m), 3.96 (3H, s), 6.78 (1H, d, J = 8.8 Hz), 7.18-7.20 (1H, m), 7.59-7.63 (1H, m), 8.11 (1H, d, J = 2.7 Hz), 8.35 (1H, m), 8.59 (1H, d, J = 1.2 Hz).
EI-MSm/z: 352 (M⁺).

[Example 157] N,N-Diethyl-1-(6-methoxy-3-pyridazinyl)-5-(5-methyl-2-pyrazinyl)-1H-pyrazole-3-carboxamide

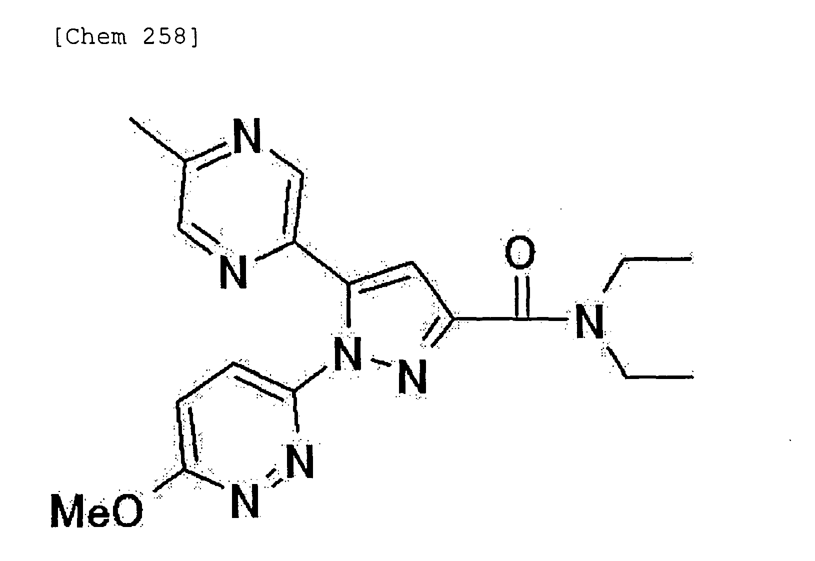

The procedure of Example 1 was repeated by using the 1-(6-methoxy-3-pyridazinyl)-5-(5-methyl-2-pyrazinyl)-1H-pyrazole-3-carboxylic acid (0.250 g) of Referential Example 81 and diethylamine (0.167 ml) to give the title compound (0.198 g, 67%) as a solid.

¹H-NMR (400 MHz, CDCl₃)δ: 1.26-1.30 (6H, m), 2.59 (3H, s), 3.56-3.78 (4H, m), 4.11 (3H, s), 7.14 (1H, s), 7.17 (1H, d, J = 9.3 Hz), 7.89 (1H, d, J = 9.3 Hz), 8.30-8.31 (1H, m), 8.70 (1H, d, J = 1.5 Hz).
EI-MSm/z: 367 (M⁺).

[Example 158] N-Ethyl-N-methyl-1-(6-methoxy-3-pyridazinyl)-5-(5-methyl-2-pyridyl)-1H-pyrazole-3-carboxamide

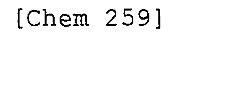

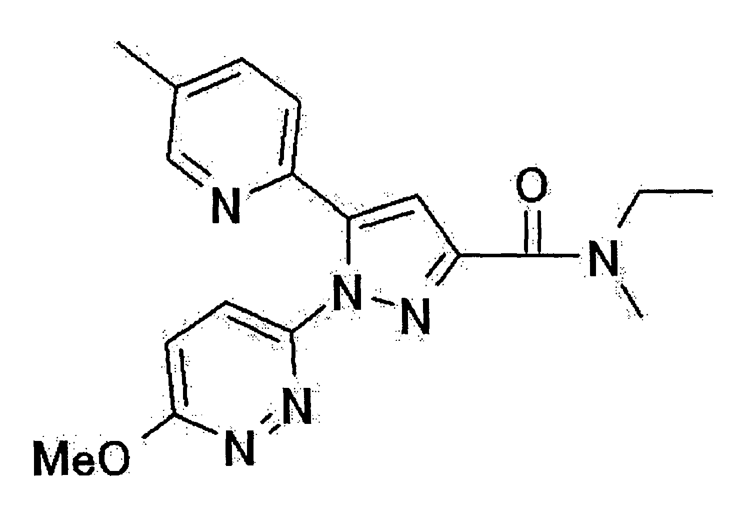

The procedure of Example 7 was repeated by using the 1-(6-methoxy-3-pyridazinyl)-5-(5-methyl-2-pyridyl)-1H-pyrazole-3-carboxylic acid (70mg) of Referential Example 67 and N-ethylmethylamine (25 µl) to give the title compound (32 mg, 40%) as a solid.

¹H-NMR (400 MHz, CDCl₃)δ: 1.25 (3H, t, J = 7.08 Hz), 2.33 (3H, s), 3.12 (1.8H, s), 3.36 (1.2H, s), 3.62 (1H, q, J = 7.08 Hz), 3.79 (1H, q, J = 7.08 Hz), 4.11 (3H, s), 7.05 (1H, d, J = 10.5 Hz), 7.12 (1H, dd, J = 9.16, 3.30 Hz), 7.47 (1H, d, J = 8.06 Hz), 7.54 (1H, d, J = 8.06 Hz), 7.81 (1H, dd, J = 9.16, 3.42 Hz), 8.25 (1H, s).
FAB-MSm/z: 353 (M+H)⁺.

[Example 159] N-Isopropyl-N-methyl-1-(6-methoxy-3-pyridazinyl)-5-(5-methyl-2-pyridyl)-1H-pyrazole-3-carboxamide

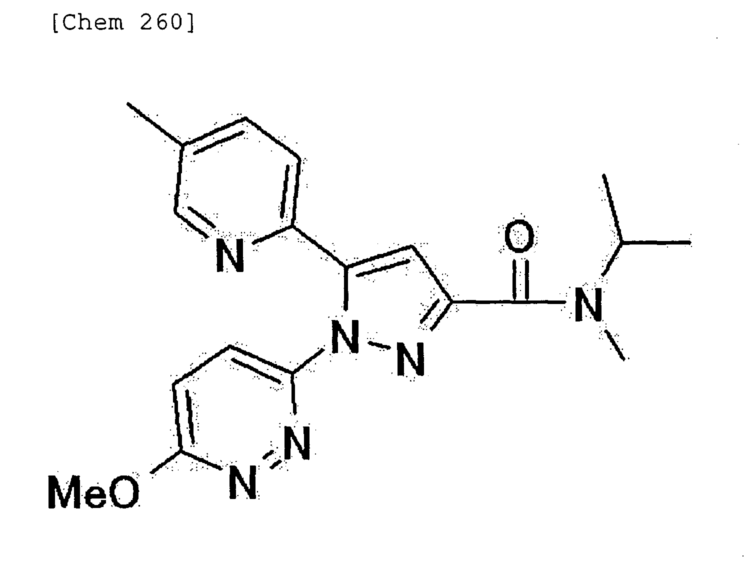

The procedure of Example 7 was repeated by using the 1-(6-methoxy-3-pyridazinyl)-5-(5-methyl-2-pyridyl)-1H-pyrazole-3-carboxylic acid (50 mg) of Referential Example 67 and N-isopropylmethylamine (22 µl) to give the title compound (23 mg, 39%) as a solid.

¹H-NMR (400 MHz, CDCl₃)δ: 1.22 (3H, d, J = 6.71 Hz), 1.24 (3H, d, J = 6.59 Hz), 2.33 (3H, s), 2.99 (1.8H, s), 3.17 (1.2H, s), 4.11 (3H, s), 4.79 (0.5H, q, J = 6.59 Hz), 5.01 (0.5H, q, J = 6.71 Hz), 6.97-7.04 (1H, d, J = 28.3 Hz), 7. 12 (1H, dd, J = 9.40, 3.78 Hz), 7.46 (1H, m), 7.53 (1H, d, J = 7.69 Hz), 7.83 (1H, t, J = 8.91 Hz), 8.25 (1H, d, J = 7.20 Hz).
FAB-MSm/z: 367 (M+H)⁺.

[Example 160] N,N-Diethyl-1-(6-methoxy-3-pyridazinyl)-5-(5-methyl-2-pyridyl)-1H-pyrazole-3-carboxamide

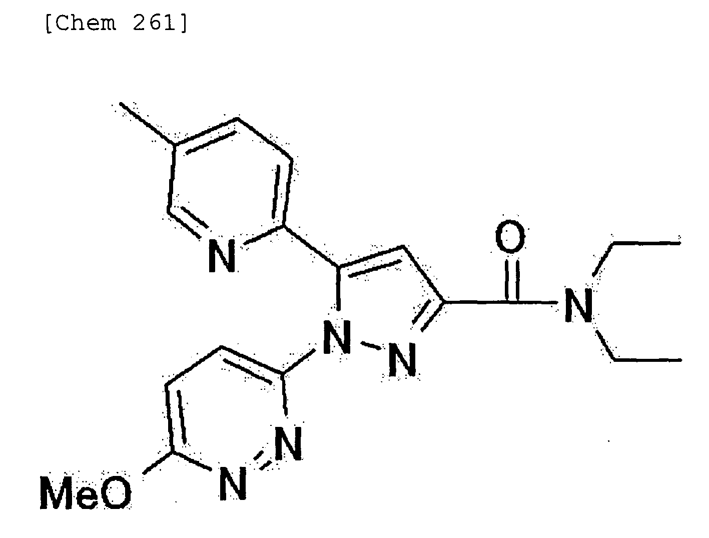

The procedure of Example 7 was repeated by using the 1-(6-methoxy-3-pyridazinyl)-5-(5-methyl-2-pyridyl)-1H-pyrazole-3-carboxylic acid (50 mg) of Referential Example 67 and diethylamine (22 µl) to give the title compound (20 mg, 34%) as a solid.

¹H-NMR (400 MHz, CDCl₃)δ: 1.27 (6H, t, J = 7.08 Hz), 2.33 (3H, s), 3.57 (2H, q, J = 7.08 Hz), 3.76 (2H, q, J = 7.08 Hz), 4.11 (3H, s), 7.05 (1H, s), 7.12 (1H, d, J = 9.16 Hz), 7.47 (1H, d, J = 7.93 Hz), 7.54 (1H, d, J = 8.18 Hz), 7.82 (1H, d, J = 9.28 Hz), 8.25 (1H, s).
FAB-MSm/z: 367 (M+H)⁺.

[Example 161] N-Isobutyl-N-methyl-1-(6-methoxy-3-pyridazinyl)-5-(5-methyl-2-pyridyl)-1H-pyrazole-3-carboxamide

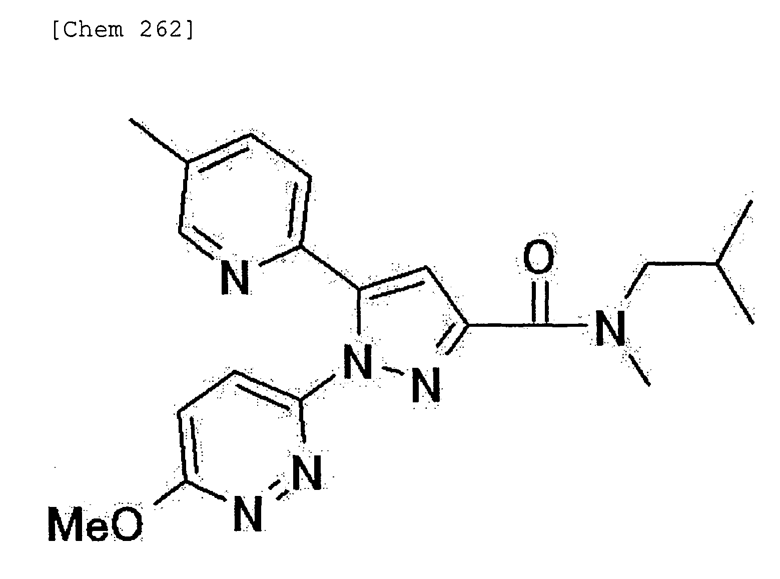

The procedure of Example 7 was repeated by using the 1-(6-methoxy-3-pyridazinyl)-5-(5-methyl-2-pyridyl)-1H-pyrazole-3-carboxylic acid (50 mg) of Referential Example 67 and N-isobutylmethylamine (25 µl) to give the title compound (30 mg, 49%) as a solid.

¹H-NMR (400 MHz, CDCl₃)δ: 0.88 (3H, d, J = 6.84 Hz), 0.98 (3H, d, J = 6.59 Hz), 2.08 (1H, m), 2.33 (3H, s), 3.12 (1.5H, s), 3.36 (1.5H, s), 3.41 (1H, d, J = 7.57 Hz), 3.66 (1H, d, J = 7.45 Hz), 4.11 (3H, s), 7.06 (1H, d, J = 11.85 Hz), 7.13 (1H, dd, J = 9.28, 5.13 Hz), 7.47 (1H, dd, J = 7.96, 3.41 Hz), 7.54 (1H, d, J = 7.93 Hz), 7.81 (1H, dd, J = 9.28, 3.18 Hz), 8.24 (1H, s).
FAB-MSm/z: 381 (M+H)⁺.

[Example 162] N,N-Dimethyl-1-(6-methoxy-3-pyridazinyl)-5-(1-methyl-1H-pyrrol-3-yl)-1H-pyrazole-3-carboxamide

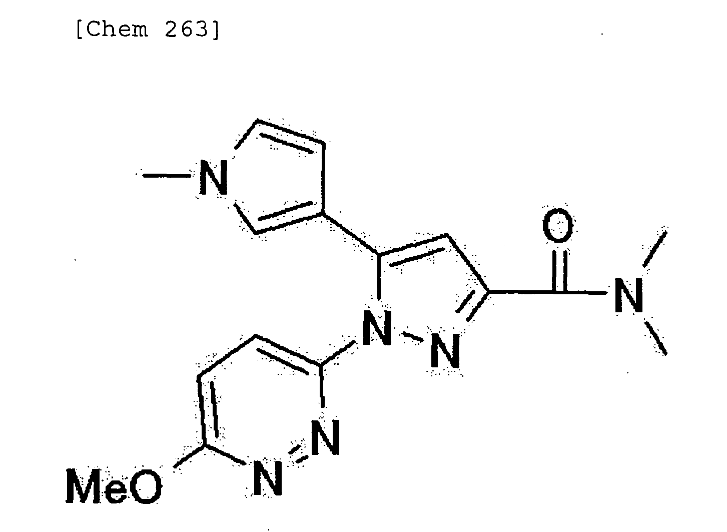

The procedure of Example 1 was repeated by using the 1-(6-methoxy-3-pyridazinyl)-5-(1-methyl-1H-pyrrol-3-yl)-1H-pyrazo le-3-carboxylic acid (200 mg) of Referential Example 78 and N,N-dimethylamine hydrochloride (82 mg) to give the title compound (66 mg, 30%) as a solid.

¹H-NMR (400 MHz, CDCl₃)δ: 3.13 (3H, s), 3.36 (3H, s), 3.63 (3H, s), 4.19 (3H, s), 6.08 (1H, dd, J = 2.56, 1.95 Hz), 6.53 (1H, t, J= 2.56 Hz), 6.78 (1H, s), 6.85 (1H, t, J = 1.95 Hz), 7.07 (1H, d, J = 9.28 Hz), 7.59 (1H, d, J = 9.28 Hz).
FAB-MSm/z: 327 (M+H)⁺.

[Example 163] N,N-Dimethyl-1-(6-methoxy-3-pyridyl)-5-(5-methyl-2-pyridyl)-1H-pyrazole-3-carboxamide

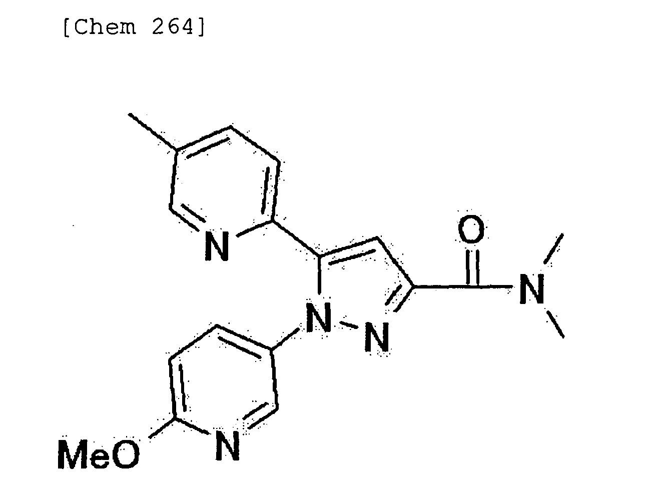

The procedure of Example 7 was repeated by using the 1-(6-methoxy-3-pyridyl)-5-(5-methyl-2-pyridyl)-1H-pyrazole-3-carb oxylic acid (200 mg) of Referential Example 79 and N,N-dimethylamine hydrochloride (79 mg) to give the title compound (117 mg, 54%) as a solid.

¹H-NMR (400 MHz, CDCl₃)δ: 2.34 (3H, s), 3.15 (3H, s), 3.40 (3H, s), 3.95 (3H, s), 6.75 (1H, d, J = 8.79 Hz), 7.08 (1H, s), 7.30 (1H, d, J = 8.06 Hz), 7.50 (1H, dd, J = 8.06, 2.20 Hz), 7.61 (1H, dd, J = 8.79, 2.69 Hz), 8.10 (1H, d, J = 2.69 Hz), 8.35 (1H, s).
FAB-MSm/z: 338 (M+H)⁺.

[Example 164] N-Ethyl-N-methyl-1-(6-methoxy-3-pyridyl)-5-(2-pyridyl)-1H-pyrazole-3-carboxamide

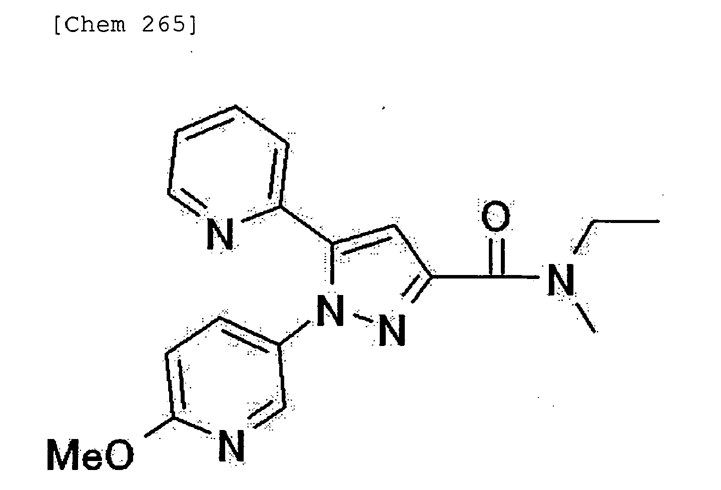

The procedure of Example 7 was repeated by using the 1-(6-methoxy-3-pyridyl)-5-(2-pyridyl)-1H-pyrazole-3-carboxylic acid (200 mg) of Referential Example 4 and N-ethylmethylamine (117 µl) to give the title compound (133 mg, 58%) as a solid.

¹H-NMR (400 MHz, CDCl₃)δ: 1.26 (3H, t, J = 7.33 Hz), 3.12 (1.7H, s), 3.37 (1.3H, s), 3.62 (1H, q, J = 7.08 Hz), 3.81 (1H, q, J = 7.08 Hz), 3.95 (3H, s), 6.75 (1H, d, J = 8.67 Hz), 7.12 (1H, d, J = 8.30 Hz), 7.23 (1H, br), 7.43 (1H, d, J = 7.20 Hz), 7.59 (1H, t, J = 6.84 Hz), 7.71 (1H, t, J = 7.20 Hz), 8.12 (1H, s), 8.52 (1H, s).
FAB-MSm/z: 338 (M+H)⁺.

[Example 165] N-(1-Hydroxymethyl cyclopentan-1-yl)-1-(3-pyridyl)-5-(5-methyl-2-pyrazinyl)-1H-pyrazole-3-carboxamide

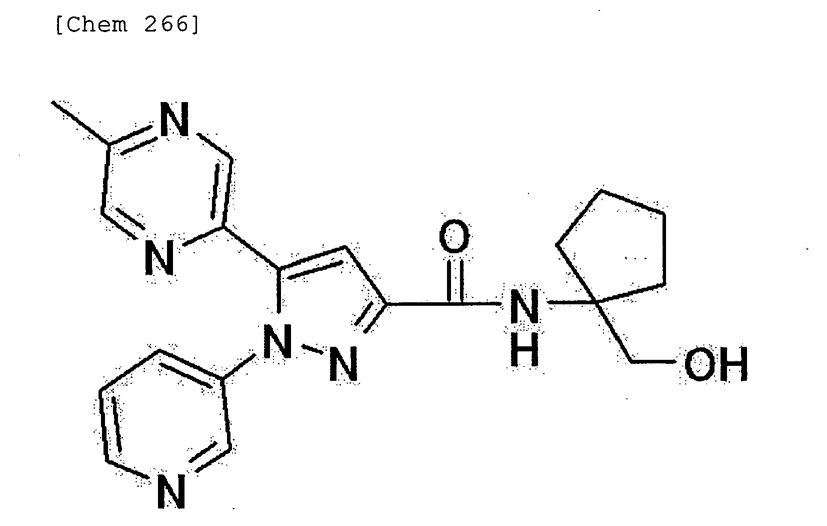

The procedure of Example 7 was repeated by using the 1-(3-pyridyl)-5-(5-methyl-2-pyrazinyl)-1H-pyrazole-3-carboxylic acid (262 mg) of Referential Example 36 and 1-amino-1-cyclopentane methanol (132 mg) to give the title compound (64 mg, 18%) as a solid.

¹H-NMR (400 MHz, CDCl₃)δ: 1.64-2.04 (8H, m), 2.57 (3H, s), 3.79 (2H, s), 4.57 (1H, s), 7.15 (1H, s), 7.31 (1H, d, J = 0.7 Hz), 7.34-7.42 (1H, m), 7.76 (1H, d, J = 8.1 Hz), 8.28 (1H, s), 8.57 (1H, d, J = 2.4 Hz), 8.65 (1H, d, J = 4.6 Hz), 8.67 (1H, s).
ESI-MSm/z: 379 (M+H)⁺.

[Example 166] N-Isopropyl-N-methyl-1-(6-methoxy-3-pyridazinyl)-5-(2-pyridyl)-1H-pyrazole-3-carboxamide

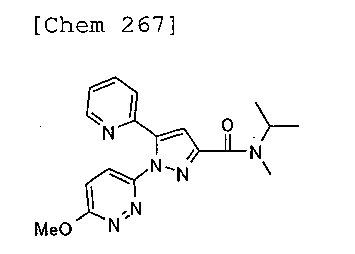

The procedure of Example 7 was repeated by using the 1-(6-methoxy-3-pyridazinyl)-5-(2-pyridyl)-1H-pyrazole-3-carboxyli c acid (0.248 g) of Referential Example 9 and N-isopropyl-N-methylamine (0.260 ml) to give the title compound (145 mg, 49%) as a solid.

¹H-NMR (400 MHz, CDCl₃)δ: 1.00-1.30 (6H, m), 2.99 (3Hx3/5, s), 3.18 (3Hx2/5, s), 4.06 (3H, s), 4.75-4.85 (1Hx3/5, m), 4.95-5.05 (1Hx2/5, m), 7.01 (1Hx3/5, s), 7.08 (1Hx2/5, s), 7.10-7.30 (2H, m), 7.53-7.62 (1H, m), 7.72-7.86 (2H, m), 8.37-8.46 (1H, m). ESI-MSm/z: 353 (M+H)⁺.

[Example 167] N,N-Dimethyl-5-(5-ethyl-2-pyridyl)-1-(6-methoxy-3-pyridazinyl)-1H-pyrazole-3-carboxamide

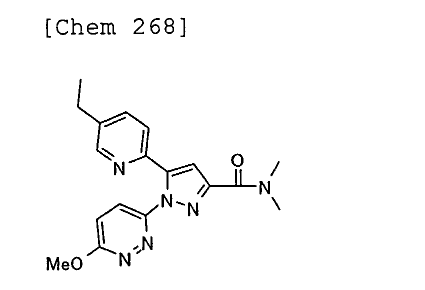

Sodium methoxide (81.0 mg) was added to a solution of the methyl 1-(6-chloro-3-pyridazinyl)-5-(5-ethyl-2-pyridyl)-1H-pyrazole-3-ca rboxylate (0.343 g) of Referential Example 82 in a mixture of methanol (5 ml) and tetrahydrofuran (10 ml) at room temperature, and the mixture was stirred for 25 hours. A 1N aqueous solution of sodium hydroxide (2.00 ml) was added to the reaction liquid, and the mixture was stirred at room temperature for 2.5 days. 1N aqueous hydrochloric acid (2.5 ml), ethanol, and ethyl acetate were added to the reaction liquid, and the mixture was dried over anhydrous sodium sulfate. After filtration, the solvent was evaporated under reduced pressure to give crude 5-(5-ethyl-2-pyridyl)-1-(6-methoxy-3-pyridazinyl)-1H-pyrazole-3-c arboxylic acid. The procedure of Example 1 was repeated by using this crude
5-(5-ethyl-2-pyridyl)-1-(6-methoxy-3-pyridazinyl)-1H-pyrazole-3-c arboxylic acid and dimethylamine hydrochloride (0.122 g) to give the title compound (0.279 g, 79%) as a solid.

¹H-NMR (400 MHz, CDCl₃)δ: 1.26 (3H, t, J = 7.6 Hz), 2.65 (2H, q, J = 7.6 Hz), 3.15 (3H, s), 3.40 (3H, s), 4.11 (3H, s), 7.08 (1H, s), 7.12 (1H, d, J = 9.3 Hz), 7.50 (1H, d, J = 8.1 Hz), 7.57 (1H, dd, J = 8.1, 2.0 Hz), 7.80 (1H, d, J = 9.3 Hz), 8.25 (1H, d, J = 2.0 Hz).
ESI-MSm/z: 353 (M+H)⁺.

[Example 168] N-Ethyl-N-methyl-1-(6-methoxy-3-pyridyl)-5-(6-methyl-3-pyridyl)-1H-pyrazole-3-carboxamide

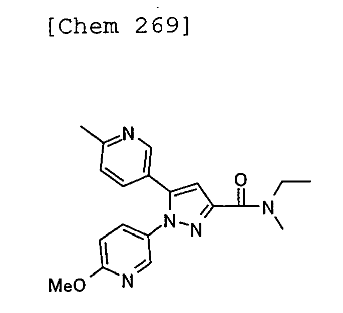

The procedure of Example 7 was repeated by using the 1-(6-methoxy-3-pyridyl)-5-(6-methyl-3-pyridyl)-1H-pyrazole-3-carb oxylic acid (0.250 g) of Referential Example 83 and N-ethylmethylamine (0.138 ml) to give the title compound (0.136 g, 48%) as a solid.

¹H-NMR (400 MHz, CDCl₃)δ: 1.19-1.30 (3H, m), 2.58 (3H, s), 3.12-3.39 (3H, m), 3.59-3.86 (2H, m), 3.95 (3H, s), 6.76 (1H, d, J = 8.8 Hz), 6.96 (1H, d, J = 8.8 Hz), 7.15 (1H, d, J = 8.1 Hz), 7.42-7.44 (1H, m), 7.50-7.54 (1H, m), 8.09 (1H, m), 8.41 (1H, m).
EI-MSm/z: 351 (M⁺).

[Example 169] N-Ethyl-N-methyl-5-(6-amino-3-pyridyl)-1-(6-methoxy-3-pyridyl)-1H-pyrazole-3-carboxamide

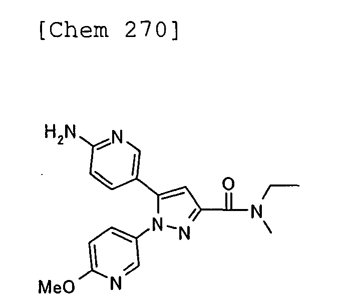

### 1) N-Ethyl-N-methyl-5-[6-(tert-butoxycarbonylamino)-3-pyridyl]-1-(6-methoxy-3-pyridyl)-1H-pyrazole-3-carboxamide

The procedure of Example 1 was repeated by using the 5-[6-(tert-butoxycarbonylamino)-3-pyridyl]-1-(6-methoxy-3-pyridyl )-1H-pyrazole-3-carboxylic acid (0.325 g) of Referential Example 84 and N-ethylmethylamine (0.136 ml) to give N-ethyl-N-methyl-5-[6-(tert-butoxycarbonylamino)-3-pyridyl]-1-(6-methoxy-3-pyridyl)-1H-pyrazole-3-carboxamide (0.348 g, 97%) as a solid.

¹H-NMR (400 MHz, CDCl₃)δ: 1.23-1.30 (3H, m), 1.52 (9H, s), 3.12-3.39 (3H, m), 3.59-3.86 (2H, m), 3.95 (3H, s), 6.76 (1H, d, J = 9.0 Hz), 6.92-6.94 (1H, m), 7.45-7.53 (2H, m), 7.94-7.96 (2H, m), 8.11-8.12 (1H, m), 8.20-8.21 (1H, m).
EI-MSm/z: 452 (M⁺).

### 2) The title compound

The procedure of Example 95(2) was repeated by using the N-ethyl-N-methyl-5-[6-(tert-butoxycarbonylamino)-3-pyridyl]-1-(6-methoxy-3-pyridyl)-1H-pyrazole-3-carboxamide (0.338 g) and trifluoroacetic acid (3.4 ml) to give the title compound (0.192 g, 73%) as a solid.

¹H-NMR (400MHz, CDCl₃)δ: 1.20-1.30 (3H, m), 3.12-3.39 (3H, m), 3.59-3.86 (2H, m), 3.96 (3H, s), 4.76 (2H, s), 6.48 (1H, d, J = 8.5 Hz), 6.77 (1H, d, J = 8.8 Hz), 6.86 (1H, d, J = 8.8 Hz), 7.26-7.29 (1H, m), 7.52-7.57 (1H, m), 7.99 (1H, s), 8.14-8.15 (1H, m).
EI-MSm/z: 352 (M⁺).

[Example 170] N-Ethyl-N-methyl-1-(6-methoxy-3-pyridazinyl)-5-(6-methyl-3-pyridyl)-1H-pyrazole-3-carboxamide

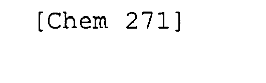

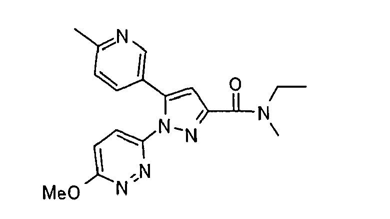

The procedure of Example 1 was repeated by using the 1-(6-methoxy-3-pyridazinyl)-5-(6-methyl-3-pyridyl)-1H-pyrazole-3-carboxylic acid (0.250 g) of Referential Example 85 and N-ethylmethylamine (0.138 ml) to give the title compound (0.167 g, 58%) as a solid.

¹H-NMR (400 MHz, CDCl₃)δ: 1.20-1.31 (3H, m), 2.59 (3H, s), 3.14-3.37 (3H, m), 3.61-3.82 (2H, m), 4.12 (3H, s), 6.94 (1H, d, J = 8.8 Hz), 7.12-7.21 (2H, m), 7.63-7.66 (1H, m), 7.84-7.87 (1H, m), 8.43-8.44 (1H, m).
EI-MSm/z: 352 (M⁺).

[Example 171] N-Ethyl-N-methyl-5-(5-benzyloxy-2-pyridyl)-1-(6-methoxy-3-pyridyl)-1H-pyrazole-3-carboxamide

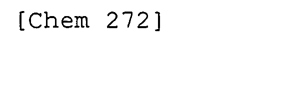

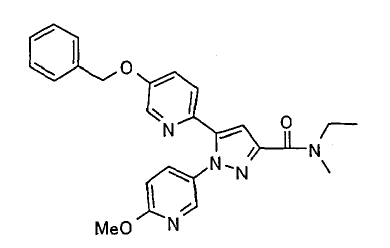

The procedure of Example 1 was repeated by using the 5-(5-benzyloxy-2-pyridyl)-1-(6-methoxy-3-pyridyl)-1H-pyrazole-3-c arboxylic acid (1.0 g) of Referential Example 14 and N-ethylmethylamine (0.176 g) to give the title compound (1.06 g, 95%) as a solid.

¹H-NMR (400 MHz, CDCl₃)δ: 1.22-1.28 (3H, m), 3.11 (1/2 x 3H, s), 3.36 (1/2 x 3H, s), 3.61 (1/2 x 2H, q, J = 7.3 Hz), 3.81 (1/2 x 2H, q, J = 7.3 Hz), 3.91 (3H, s), 5.12 (2H, s), 6.74 (1H, d, J = 8.8 Hz), 7.02 (1H, d, J = 8.8 Hz), 7.23-7.27 (1H, m), 7.33-7.42 (6H, m), 7.52-7.61 (1H, m), 8.11 (1H, d, J = 2.2 Hz), 8.27 (1H, d, J = 2.2 Hz).
EI-MSm/z: 443 (M⁺).

[Example 172] N-Ethyl-N-methyl-5-(5-carbamoylmethoxy-2-pyridyl)-1-(6-methoxy-3-pyridyl)-1H-pyrazole-3-carboxamide

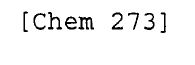

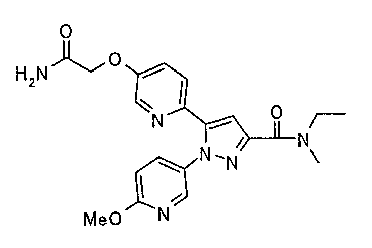

### 1) N-Ethyl-N-methyl-5-(5-hydroxy-2-pyridyl)-1-(6-methoxy-3-pyridyl)-1H-pyrazole-3-carboxamide

To a solution of the N-ethyl-N-methyl-5-(5-benzyloxy-2-pyridyl)-1-(6-methoxy-3-pyridyl )-1H-pyrazole-3-carboxamide (1.05 g) of Example 171 in ethanol (20 ml) and ethyl acetate (20 ml) was added 10% palladium on carbon (50% wet, 700 mg), and the mixture was stirred for 1 hour under hydrogen atmosphere. After removing the catalyst by filtration, the solvent was evaporated under reduced pressure to give N-ethyl-N-methyl-5-(5-hydroxy-2-pyridyl)-1-(6-methoxy-3-pyridyl)-1H-pyrazole-3-carboxamide (840 mg, measured) as a solid.

¹H-NMR (400 MHz, CDCl₃)δ: 1.20-1.28 (3H, m), 3.09 (1/2 x 3H, s), 3.35 (1/2 x 3H, s), 3.58 (1/2 x 2H, q, J = 7.1 Hz), 3.79 (1/2 x 2H, q, J = 7.1 Hz), 3.90 (1/2 x 3H, s), 3.91 (1/2 x 3H, s), 6.68 (1H, d, J = 8.8 Hz), 6.89 (1H, d, J = 6.8 Hz), 7.05-7.10 (2H, m), 7.46-7.50 (1H, m), 8.06-8.10 (2H, m).
EI-MSm/z: 353 (M⁺).

### 2) The title compound

Potassium carbonate (235 mg) and 2-bromoacetamide (141 mg) were added to a solution of the N-ethyl-N-methyl-5-(5-hydroxy-2-pyridyl)-1-(6-methoxy-3-pyridyl)-1H-pyrazole-3-carboxamide (300 mg) in N,N-dimethylformamide (5 ml) at room temperature, and the mixture was stirred for 20 hours. Water and ethyl acetate were added to the reaction liquid and the phases were separated, and the organic layer was dried over anhydrous magnesium sulfate. After filtration, the solvent was evaporated under reduced pressure, and the residue was purified by thin layer chromatography on silica gel (methanol-dichloromethane) to give the title compound (120 mg, 37%) as a solid.

¹H-NMR (400 MHz, DMSO-d₆)δ: 1.11-1.21 (3H, m), 2.99 (1/2 x 3H, s), 3.29 (1/2 x 3H, s), 3.47 (1/2 x 2H, q, J = 6.8 Hz), 3.71 (1/2 x 2H, q, J = 6.8 Hz), 3.89 (3H, s), 4.54 (2H, s), 6.87 (1H, d, J = 8.8 Hz), 7.06 (1H, d, J= 11.5 Hz), 7.40-7.43 (2H, m), 7.58-7.70 (3H, m), 8.13-8.30 (2H, m).
EI-MSm/z: 410 (M⁺).

[Example 173] N-Ethyl-N-methyl-5-(5-benzyloxy-2-pyridyl)-1-(6-methoxy-3-pyridazinyl)-1H-pyrazole-3-carboxamide

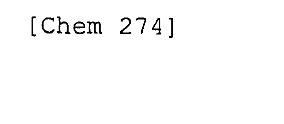

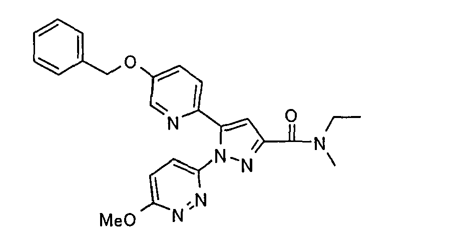

The procedure of Example 1 was repeated by using the 5-(5-benzyloxy-2-pyridyl)-1-(6-methoxy-3-pyridazinyl)-1H-pyrazole -3-carboxylic acid (1.0 g) of Referential Example 87 and N-ethylmethylamine (0.18 g) to give the title compound (0.782 g, 71%) as a solid.

¹H-NMR (400 MHz, CDCl₃)δ: 1.23-1.28 (3H, m), 3.11 (1/2 x 3H, s), 3.36 (1/x3H, s), 3.61 (1/2 x 2H, q, J = 7.1 Hz), 3.79 (1/2 x 2H, q, J = 7.1 Hz), 4.11 (3H, s), 5.10 (2H, s), 7.02 (1H, d, J = 10.0 Hz), 7.11-7.15 (1H, m), 7.26 (1H, d, J = 1.0 Hz), 7.28-7.45 (5H, m), 7.51 (1H, d, J = 8.8 Hz), 7.80 (1H, dd, J = 9.3, 3.4 Hz), 8.18 (1H, d, J = 2.9 Hz). EI-MSm/z: 444 (M⁺).

[Example 174] N-Ethyl-N-methyl-5-(5-carbamoylmethoxy-2-pyridyl)-1-(6-methoxy-3-pyridazinyl)-1H-pyrazole-3-carboxamide

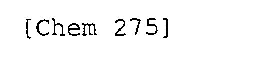

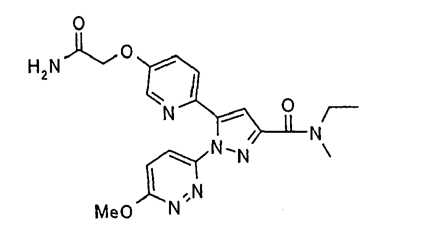

### 1) N-Ethyl-N-methyl-5-(5-hydroxy-2-pyridyl)-1-(6-methoxy-3-pyridazinyl)-1H-pyrazole-3-carboxamide

The procedure of Example 172(1) was repeated by using the N-ethyl-N-methyl-5-(5-benzyloxy-2-pyridyl)-1-(6-methoxy-3-pyridaz inyl)-1H-pyrazole-3-carboxamide (780 mg) of Example 173 to give N-ethyl-N-methyl-5-(5-hydroxy-2-pyridyl)-1-(6-methoxy-3-pyridazin yl)-1H-pyrazole-3-carboxamide (280 mg, 45%) as a solid.

¹H-NMR (400 MHz, DMSO-d₆)δ: 1.12-1.20 (3H, m), 2.99 (1/2 x 3H, s), 3.17 (1/2 x 3H, s), 3.46-3.52 (1/2 x 2H, m), 3.66-3.72 (1/2 x 2H, m), 4.05 (3H, s), 7.04 (1H, d, J = 12.7 Hz), 7.20-7.24 (1H, m), 7.41-7.47 (1H, m), 7.56 (1H, d, J = 8.6 Hz), 7.86-7.92 (1H, m), 10.20 (1H, s).
EI-MSm/z: 354 (M⁺).

### 2) The title compound

The procedure of Example 172(2) was repeated by using the N-ethyl-N-methyl-5-(5-hydroxy-2-pyridyl)-1-(6-methoxy-3-pyridazin yl) -1H-pyrazole-3-carboxamide (280 mg) to give the title compound (28 mg, 8.5%) as a solid.

¹H-NMR (400 MHz, CDCl₃)δ: 1.23-1.29 (3H, m), 3.12 (1/2 x 3H, s), 3.56 (1/2 x 3H, s), 3.62 (1/2 x 2H, q, J = 7.1 Hz), 3.79 (1/2 x 2H, q, J = 7.1 Hz), 4.10 (3H, s), 4.54 (2H, s), 5.88 (1H, br s), 6.54 (1H, s), 7.04 (1H, d, J = 9.5 Hz), 7.13-7.16 (1H, m), 7.26-7.29 (1H, m), 7.55 (1H, d, J = 8.6 Hz), 7.83 (1H, d, J = 9.3 Hz), 8.17 (1H, d, J = 2.9 Hz).
EI-MSm/z: 411 (M⁺).

[Example 175] N-Ethyl-N-methyl-5-(5-benzyloxy-2-pyridyl)-1-(6-methyl-3-pyridyl)-1H-pyrazole-3-carboxamide

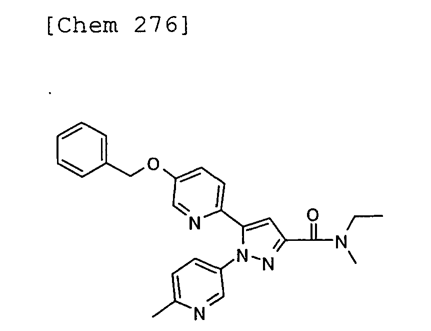

The procedure of Example 1 was repeated by using the 5-(5-benzyloxy-2-pyridyl)-1-(6-methyl-3-pyridyl)-1H-pyrazole-3-ca rboxylic acid (1.3 g) of Referential Example 88 and N-ethylmethylamine (0.24 g) to give the title compound (1.3 g, 90%) as a solid.

¹H-NMR (400 MHz, CDCl₃)δ: 1.23-1.29 (3H, m), 2.59 (3H, s), 3.11 (1/2 x 3H, s), 3.37 (1/2 x 3H, s), 3.61 (1/2 x 2H, d, J = 7.1 Hz), 3.81 (1/2 x 2H, d, J = 7.1 Hz), 5.11 (32H, s), 7.03 (1H, d, J = 8.8 Hz), 7.16-7.19 (1H, m), 7.25-7.27 (1H, m), 7.35-7.42 (6H, m), 7.60-7.64 (1H, m), 8.26 (1H, s), 8.40 (1H, s).
EI-MSm/z: 427 (M⁺).

[Example 176] Benzyl {6-[5-(ethylmethylcarbamoyl)-2-(6-methyl-3-pyridyl)-2H-pyrazol]-3-pyridyloxy}acetate

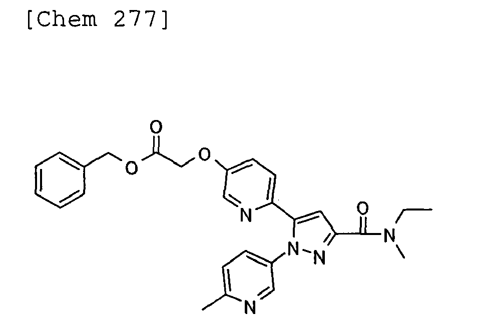

### 1) N-Ethyl-N-methyl-5-(5-hydroxy-2-pyridyl)-1-(6-methyl-3-pyridyl)-1H-pyrazole-3-carboxamide

The procedure of Example 172(1) was repeated by using the N-ethyl-N-methyl-5-(5-benzyloxy-2-pyridyl)-1-(6-methyl-3-pyridyl) -1H-pyrazole-3-carboxamide (1.3 g) of Example 175 to give N-ethyl-N-methyl-5-(5-hydroxy-2-pyridyl)-1-(6-methyl-3-pyridyl)-1 H-pyrazole-3-carboxamide (970 mg, 94%) as a solid.
EI-MSm/z: 337 (M⁺).

### 2) The title compound

The procedure of Example 172(2) was repeated by using the N-ethyl-N-methyl-5-(5-hydroxy-2-pyridyl)-1-(6-methyl-3-pyridyl)-1 H-pyrazole-3-carboxamide (970 mg), benzyl 2-bromoacetate (780 mg), and potassium carbonate (790 mg) to give the title compound (1.2 g, 86%) as a solid.

¹H-NMR (400 MHz, CDCl₃)δ: 1.21-1.29 (3H, m), 2.59 (3H, s), 3.11 (1/2 x 3H, s), 3.37 (1/2 x 3H, s), 3.61 (1/2 x 2H, q, J = 7.1 Hz), 3.81 (1/2 x 2H, q, J = 7.1 Hz), 4.71 (2H, s), 5.24 (2H, s), 7.04 (1H, d, J= 8.8 Hz), 7.15-7.17 (2H, m), 7.33-7.39 (6H, m), 7.53-7.61 (1H, m), 8.19-8.21 (1H, m), 8.41-8.44 (1H, m).
EI-MSm/z: 485 (M⁺).

[Example 177] {6-[5-(Ethylmethylcarbamoyl)-2-(6-methyl-3-pyridyl)-2H-pyrazol-3-yl]-3-pyridyloxy}acetic acid

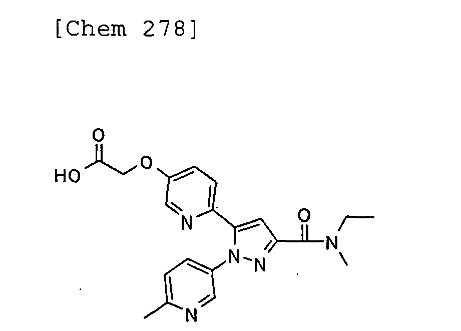

The procedure of Example 172 (1) was repeated by using the benzyl {6-[5-(ethylmethylcarbamoyl)-2-(6-methyl-3-pyridyl)-2H-pyrazol]-3 -pyridyloxy}acetate (1.2 g) of Example 176 and 10% palladium-carbon (50% wet, 500 mg) to give the title compound (1.0 g, measured) as an amorphous product.

¹H-NMR (400 MHz, CDCl₃)δ: 1.23-1.29 (3H, m), 2.61 (3H, s), 3.12 (1/2 x 3H, s), 3.37 (1/2 x 3H, s), 3.62 (1/2 x 2H, q, J = 7.3 Hz), 3.80 (1/2 x 2H, q, J = 7.3 Hz), 4.66 (2H, s), 6.63 (1H, br), 7.08 (1H, d, J = 9.8 Hz), 7.13-7.27 (2H, m), 7.41 (1H, d, J = 8.8 Hz), 7.77-7.80 (1H, m), 8.20-8.22 (1H, m), 8.36 (1H, s).
FAB-MSm/z: 396 (M+H)⁺.

[Example 178] N-Ethyl-N-methyl-5-(5-carbamoylmethoxy-2-pyridyl)-1-(6-methyl-3-pyridyl)-1H-pyrazole-3-carboxamide

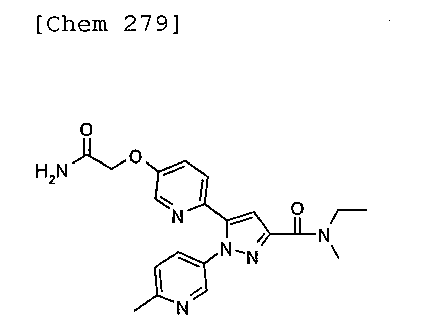

The procedure of Example 7 was repeated by using the {6-[5-(ethylmethylcarbamoyl)-2-(6-methyl-3-pyridyl)-2H-pyrazol-3-yl]-3-pyridyloxy}acetic acid (200 mg) of Example 177 and 28% aqueous ammonia (0.3 ml) to give the title compound (50 mg, 25%) as a solid.

¹H-NMR (400 MHz, CDCl₃)δ: 1.23-1.29 (3H, m), 2.59 (3H, s), 3.12 (1/2 x 3H, s), 3.38 (1/2 x 3H, s), 3.60 (1/2 x 2H, q, J = 7.1 Hz), 3.82 (1/2 x 2H, q, J = 7.1 Hz), 4.55 (2H, s), 5.71 (1H, br s), 6.48 (1H, br s), 7.07 (1H, d, J = 8.6 Hz), 7.19-7.26 (2H, m), 7.44 (1H, d, J = 8.8 Hz), 7.62-7.67 (1H, m), 8.24 (1H, d, J = 2.9 Hz), 8.36 (1H, s). EI-MSm/z: 394 (M⁺).

[Example 179] N-Ethyl-N-methyl-5-(5-cyano-2-pyridyl)-1-(6-methoxy-3-pyridyl)-1H-pyrazole-3-carboxamide

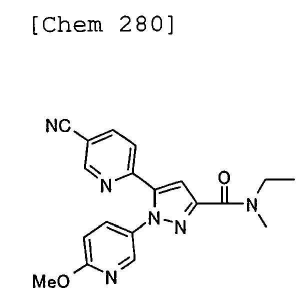

The procedure of Example 1 was repeated by using the 5-(5-cyano-2-pyridyl)-1-(6-methoxy-3-pyridyl)-1H-pyrazole-3-carbo xylic acid (1.2 g) of Referential Example 90 and N-ethylmethylamine (0.27 g) to give the title compound (0.982 g, 72.2%) as a solid.

¹H-NMR (400 MHz, CDCl₃)δ: 1.23-1.28 (3H, m), 3.12 (1/2 x 3H, s), 3.38 (1/2 x 3H, s), 3.61 (1/2 x 2H, q, J = 7.32 Hz), 3.82 (1/2 x 2H, q, = 7.3 Hz), 3.98 (3H, s), 6.79 (1H, d, J = 8.8 Hz), 7.27 (1H, d, J = 9.0 Hz), 7.57-7.61 (2H, m), 7.97 (1H, dd, J = 8.3, 2.0 Hz), 8.10 (1H, d, J = 2.7 Hz), 8.73 (1H, s).
EI-MSm/z: 362 (M⁺).

[Example 180] N-Ethyl-N-methyl-5-(5-cyano-2-pyridyl)-1-(6-methoxy-3-pyridazinyl)-1H-pyrazole-3-carboxamide

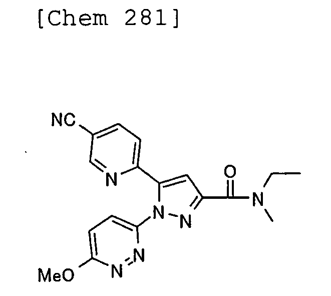

The procedure of Example 1 was repeated by using the 5-(5-cyano-2-pyridyl)-1-(6-methoxy-3-pyridazinyl)-1H-pyrazole-3-c arboxylic acid (0.97 mg) of Referential Example 89 and N-ethylmethylamine (0.21 g) to give the title compound (1.00 g, 91%) as a solid.

¹H-NMR (400 MHz, CDCl₃)δ: 1.24-1.30 (3H, m), 3.13 (1/2 x 3H, s), 3.37 (1/2 x 3H, s), 3.62 (1/2 x 2H, q, J = 7.1 Hz), 3.79 (1/2 x 2H, q, J = 7.1 Hz), 4.12 (3H, s), 7.17-7.21 (2H, m), 7.70 (1H, dd, J = 8.3, 1.0 Hz), 7.87 (1H, dd, J = 9.3, 2.2 Hz), 8.02 (1H, dd, J = 8.1, 1.95 Hz), 8.67 (1H, br s).
EI-MSm/z: 363 (M⁺).

[Example 181] N-Ethyl-N-methyl-5-[5-(methanesulfonylaminomethyl)-2-pyridyl]-1-(6-methoxy-3-pyridazinyl)-1H-pyrazole-3-carboxamide

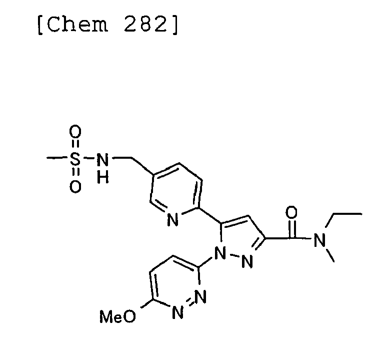

### 1) N-Ethyl-N-methyl-5-(5-aminomethyl-2-pyridyl)-1-(6-methoxy-3-pyridazinyl)-1H-pyrazole-3-carboxamide

Nickel-silica/alumina (content, about 65%; 800 mg) was added to a suspension of the N-ethyl-N-methyl-5-(5-cyano-2-pyridyl)-1-(6-methoxy-3-pyridazinyl )-1H-pyrazole-3-carboxamide (1.0 g) of Example 180 in a 2M solution of ammonia in ethanol (24ml), and the mixture was stirred in an autoclave under hydrogen atmosphere (8 atm) at 120°C for 3 hours. After cooling with air, the reaction liquid was filtered through celite, and the solvent was evaporated under reduced pressure to give N-ethyl-N-methyl-5-(5-aminomethyl-2-pyridyl)-1-(6-methoxy-3-pyrid azinyl)-1H-pyrazole-3-carboxamide (1.1 g, quantitative) as an amorphous product.
FAB-MSm/z: 368(M+H)⁺.

### 2) The title compound

The procedure of Example 136 was repeated by using the N-ethyl-N-methyl-5-(5-aminomethyl-2-pyridyl)-1-(6-methoxy-3-pyrid azinyl)-1H-pyrazole-3-carboxamide (370 mg) and methanesulfonyl chloride (93 µl) to give the title compound (226 mg, 50%) as a solid.

¹H-NMR (400 MHz, CDCl₃)δ: 1.23-1.29 (3H, m), 2.93 (3H, s), 3.12 (1/2 x 3H, s), 3.35 (1/2 x 3H, s), 3.61 (1/2 x 2H, q, J = 7.1 Hz), 3.78 (1/2 x 2H, q, J = 7.1 Hz), 4.08 (3H, s), 4.30 (2H, d, J = 5.9 Hz), 5.50-5.62 (1H, br), 7.02 (1H, d, J = 9.8 Hz), 7.14 (1H, dd, J = 9.3, 2.4 Hz), 7.50 (1H, d, J = 8.1 Hz), 7.75 (1H, d, J = 8.1 Hz), 7.82 (1H, d, J = 9.3 Hz), 8.37 (1H, br s).
EI-MSm/z: 445 (M⁺).

[Example 182] N-Ethyl-N-methyl-5-[5-(3,3-dimethylureidomethyl)-2-pyridyl]-1-(6-methoxy-3-pyridazinyl)-1H-pyrazole-3-carboxamide

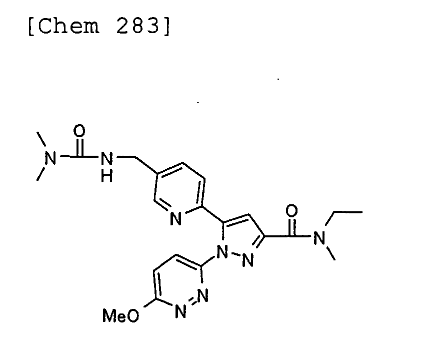

Triethylamine (0.16 ml) and N,N-dimethylcarbamic chloride (110 µl) were added to a solution of the N-ethyl-N-methyl-5-(5-aminomethyl-2-pyridyl)-1-(6-methoxy-3-pyrid azinyl)-1H-pyrazole-3-carboxamide (390 mg) of Example 181(1) in dichloromethane (20ml) at room temperature, and the mixture was stirred for 16 hours. To the reaction liquid were also added 4-dimethylaminopyridine (110 mg) and N,N-dimethylcarbamic chloride (110 µl), and the mixture was stirred for 6 hours. Water was added to the reaction liquid and the phases were separated, and the organic layer was dried over anhydrous magnesium sulfate. After filtration, the solvent was evaporated under reduced pressure, and the residue was purified by thin layer chromatography on silica gel (methanol-dichloromethane) to give the title compound (248 mg, 53%) as a solid.

¹H-NMR (400 MHz, CDCl₃)δ: 1.22-1.28 (3H, m), 2.93 (6H, s), 3.11 (1/2 x 3H, s), 3.36 (1/2 x 3H, s), 3.61 (1/2 x 2H, q, J = 7.1 Hz), 3.78 (1/2 x2H, J = 7.1 Hz), 4.10 (3H, s), 4.42 (2H, d, J = 5.6 Hz), 4.85-4.92 (1H, br), 7.06 (1H, d, J = 10.3 Hz), 7.13 (1H, dd, J = 9.3, 2.93 Hz), 7.51 (1H, d, J = 8.1 Hz), 7.71-7.81 (2H, m), 8.35 (1H, s).
EI-MSm/z: 438 (M⁺).

[Example 183] 4-Methylpiperazine-1-carboxylic acid{6-[5-(ethylmethylcarbamoyl)-2-(6-methoxy-3-pyridazinyl)-2H-pyrazol-3-yl]-3-pyridylmethyl}amide

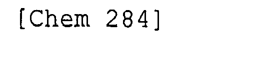

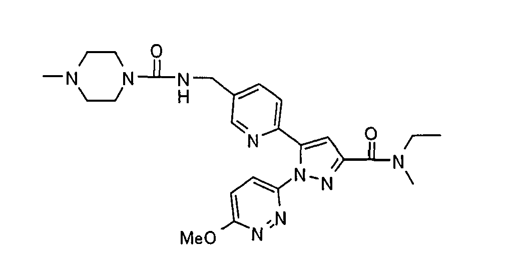

The procedure of Example 142 was repeated by using the N-ethyl-N-methyl-5-(5-aminomethyl-2-pyridyl)-1-(6-methoxy-3-pyrid azinyl)-1H-pyrazole-3-carboxamide (370 mg) of Example 181(1), 4-nitrophenyl chloroformate (210 mg), and N-methylpiperazine (111 mg) to give the title compound (240 mg, 48%) as an amorphous product.

¹H-NMR (400 MHz, CDCl₃)δ: 1.23-1.28 (3H, m), 2.40-2.50 (4H, m), 3.12 (1/2 x 3H, s), 3.35 (1/2 x 3H, s), 3.40-3.50 (4H, m), 3.61 (1/2 x 2H, q, J = 7.1 Hz), 3.78 (1/2 x 2H, q, J = 7.1 Hz), 4.10 (3H, s), 4.41 (2H, d, J = 5.6 Hz), 4.95-5.05 (1H, br), 7.05 (1H, d, J = 9.5 Hz), 7.14 (1H, dd, J = 9.3, 3.0 Hz), 7.51 (1H, d, J = 8.3 Hz), 7.81 (1H, dd, J = 9.3, 3.4 Hz), 8.34 (1H, s).
EI-MSm/z: 493 (M⁺).

[Example 184] N-Ethyl-N-methyl-5-[5-(methanesulfonylaminomethyl)-2-pyridyl]-1-(6-methoxy-3-pyridyl)-1H-pyrazole-3-carboxamide

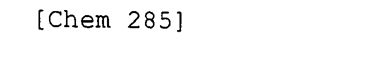

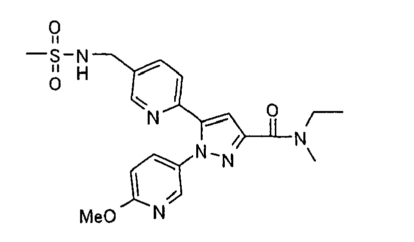

### 1) N-Ethyl-N-methyl-5-(5-aminomethyl-2-pyridyl)-1-(6-methoxy-3-pyridyl)-1H-pyrazole-3-carboxamide

The procedure of Example 181(1) was repeated by using the N-ethyl-N-methyl-5-(5-cyano-2-pyridyl)-1-(6-methoxy-3-pyridyl)-1H -pyrazole-3-carboxamide (980 mg) of Example 179 to give N-ethyl-N-methyl-5-(5-aminomethyl-2-pyridyl)-1-(6-methoxy-3-pyrid yl)-1H-pyrazole-3-carboxamide (1.0 g, measured) as an amorphous product.
EI-MSm/z: 366 (M⁺).

### 2) The title compound

The procedure of Example 136 was repeated by using N-ethyl-N-methyl-5-(5-aminomethyl-2-pyridyl)-1-(6-methoxy-3-pyrid yl)-1H-pyrazole-3-carboxamide (326 mg) and methanesulfonyl chloride (69 µl) to give the title compound (240 mg, 60%) as a solid.

¹H-NMR (400 MHz, CDCl₃)δ: 1.23-1.28 (3H, m), 2.96 (3H, s), 3.11 (1/2 x 3H, s), 3.37 (1/2 x 3H, s), 3.61 (1/2 x 2H, q, J = 7.1 Hz), 3.81 (1/2 x 2H, q, J = 7.1 Hz), 3.95 (3H, s), 4.34 (2H, d, J = 6.4 Hz), 5.45-5.55 (1H, m), 6.74 (1H, d, J=8.8 Hz), 7.03 (1H, d, J= 9.8 Hz), 7.37 (1H, d, J = 8.1 Hz), 7.54-7.59 (1H, m), 7.75 (1H, d, J = 8.1 Hz), 8.06 (1H, d, J = 2.2 Hz), 8.46 (1H, s).
EI-MSm/z: 444 (M⁺).

[Example 185] N-Ethyl-N-methyl-5-[5-(3,3-dimethylureidomethyl)-2-pyridyl]-1-(6-methoxy-3-pyridyl)-1H-pyrazole-3-carboxamide

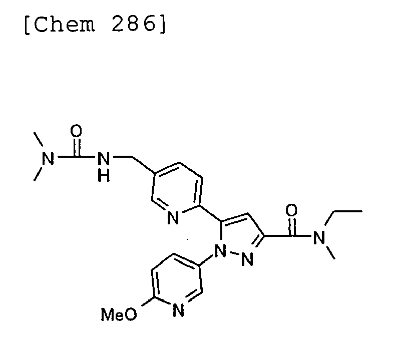

Triethylamine (0.15 ml) and N,N-dimethylcarbamic chloride (110 µl) were added to a solution of the N-ethyl-N-methyl-5-(5-aminomethyl-2-pyridyl)-1-(6-methoxy-3-pyrid yl)-1H-pyrazole-3-carboxamide (340 mg) of Example 184(1) in dichloromethane (20ml) at room temperature, and the mixture was stirred for 15 hours. Water was added to the reaction liquid and the phases were separated, and the organic layer was dried over anhydrous magnesium sulfate. After filtration, the solvent was evaporated under reduced pressure, and the residue was purified by thin layer chromatography on silica gel (methanol-dichloromethane) to give the title compound (190 mg, 46%) as a solid.

¹H-NMR (400 MHz, CDCl₃)δ: 1.22-1.28 (3H, m), 2.91 (6H, s), 3.11 (1/2 x 3H, s), 3.36 (1/2 x 3H, s), 3.61 (1/2 x 2H, q, J = 7.3 Hz), 3.81 (1/2 x 2H, J=7.3Hz), 3.95 (3H, s), 4.44 (2H, d, J = 5.6 Hz), 4.85-4.95 (1H, br), 6.75 (1H, d, J = 8.8 Hz), 7.08 (1H, d, J = 9.3 Hz), 7.36 (1H, d, J = 8.1 Hz), 7.59 (1H, dd, J = 8.79, 2.7 Hz), 7.62-7.71 (1H, m), 8.11 (1H, s), 8.44 (1H, s).
EI-MSm/z: 437 (M⁺).

[Example 186] 4-Methylpiperazine-1-carboxylic acid{6-[5-(ethylmethylcarbamoyl)-2-(6-methoxy-3-pyridyl)-2H-pyrazol-3-yl]-3-pyridylmethyl}amide

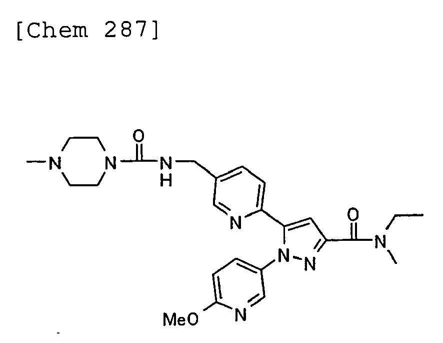

The procedure of Example 142 was repeated by using the N-ethyl-N-methyl-5-(5-aminomethyl-2-pyridyl)-1-(6-methoxy-3-pyrid yl)-1H-pyrazole-3-carboxamide (350 mg) of Example 184(1), 4-nitrophenyl chloroformate (195 mg), and N-methylpiperazine (105 mg) to give the title compound (290 mg, 61%) as an amorphous product.

¹H-NMR (400 MHz, CDCl₃)δ: 1.22-1.28 (3H, m), 2.40-2.50 (4H, m), 3.11' (1/2 x 3H, s), 3.36 (1/2 x 3H, s), 3.40-3.50 (4H, m), 3.60 (1/2 x 2H, q, J = 7.1 Hz), 3.80 (1/2 x 2H, q, J = 7.1 Hz), 3.95 (3H, s), 4.43 (2H, d, J = 5.6 Hz), 5.00-5.10 (1H, br), 6.75 (1H, d, J = 8.8 Hz), 7.04 (1H, d, J = 8.1 Hz), 7.35 (1H, d, J = 7.7 Hz), 7.57-7.68 (2H, m), 8.10 (1H, s), 8.42 (1H, s).
FAB-MSm/z: 493 (M+H)⁺.

[Example 187] N-Ethyl-N-methyl-5-(5-cyano-2-pyridyl)-1-(6-methyl-3-pyridyl)-1H-pyrazole-3-carboxamide

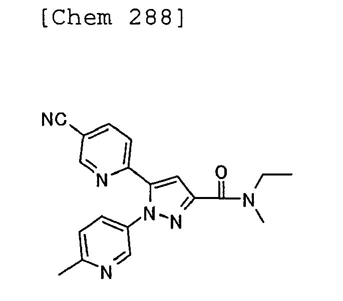

The procedure of Example 7 was repeated by using the 5-(5-cyano-2-pyridyl)-1-(6-methyl-3-pyridyl)-1H-pyrazole-3-carbox ylic acid (200 mg) of Referential Example 75 and N-ethylmethylamine (43 mg) to give the title compound (200 mg, 88%) as a solid.

¹H-NMR (400 MHz, CDCl₃)δ: 1.23-1.29 (3H, m), 2.63 (3H, s), 3.12 (1/2 x 3H, s), 3.39 (1/2 x 3H, s), 3.62 (1/2 x 2H, q, J = 7.1 Hz), 3.82 (1/2 x 2H, q, J = 7.1 Hz), 7.23-7.29 (2H, m), 7.58-7.63 (2H, m), 7.99 (1H, dd, J = 8.3, 2.0 Hz), 8.41 (1H, s), 8.70 (1H, s).
EI-MSm/z: 346 (M⁺).

[Example 188] N-Ethyl-N-methyl-5-[5-(methanesulfonylaminomethyl)-2-pyridyl]-1-(6-methyl-3-pyridyl)-1H-pyrazole-3-carboxamide

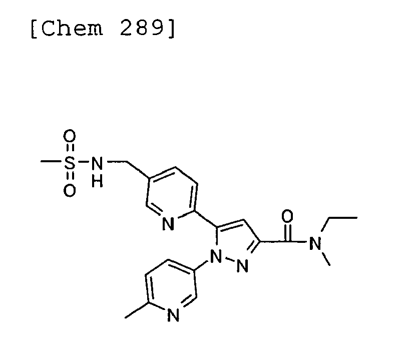

### 1) N-Ethyl-N-methyl-5-(5-aminomethyl-2-pyridyl)-1-(6-methyl-3-pyridyl)-1H-pyrazole-3-carboxamide

The procedure of Example 181(1) was repeated by using the N-ethyl-N-methyl-5-(5-cyano-2-pyridyl)-1-(6-methyl-3-pyridyl)-1H-pyrazole-3-carboxamide (2.0 g) of Example 187 to give N-ethyl-N-methyl-5-(5-aminomethyl-2-pyridyl)-1-(6-methyl-3-pyridy 1)-1H-pyrazole-3-carboxamide (2.2 g, quantitative) as an amorphous product.
FAB-MSm/z: 351(M+H)⁺.

### 2) The title compound

The procedure of Example 136 was repeated by using the N-ethyl-N-methyl-5-(5-aminomethyl-2-pyridyl)-1-(6-methyl-3-pyridy 1)-1H-pyrazole-3-carboxamide (220 mg) and methanesulfonyl chloride (59 µl) to give the title compound (220 mg, 81%) as a solid.

¹H-NMR (400 MHz, CDCl₃)δ: 1.21-1.29 (3H, m), 2.42 (3H, s), 2.96 (3H, s), 3.11 (1/2 x 3H, s), 3.37 (1/2 x 3H, s), 3.61 (1/2 x 2H, q, J = 7.3 Hz), 3.81 (1/2 x 2H, q, J = 7.3 Hz), 4.33 (2H, d, J = 6.4 Hz), 5.70-5.80 (1H, m), 7.03 (1H, d, J = 10.3 Hz), 7.18 (1H, dd, J = 8.3, 2.4 Hz), 7.40 (1H, d, J = 8.3 Hz), 7.57-7.62 (1H, m), 7.77 (1H, d, J = 8.1 Hz), 8.33-8.35 (1H, m), 8.44 (1H, s).
FAB-MSm/z: 429 (M⁺).

[Example 189] N-Ethyl-N-methyl-5-[5-(3,3-dimethylureidomethyl)-2-pyridyl]-1-(6-methyl-3-pyridyl)-1H-pyrazole-3-carboxamide

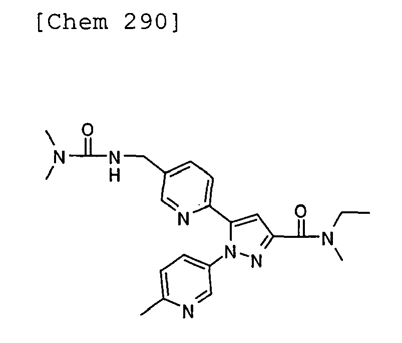

The procedure of Example 185 was repeated by using the N-ethyl-N-methyl-5-(5-aminomethyl-2-pyridyl)-1-(6-methyl-3-pyridy 1)-1H-pyrazole-3-carboxamide (220 mg) of Example 188(1) and N,N-dimethylcarbamic chloride (69 µl) to give the title compound (185 mg, 70%) as a solid.

¹H-NMR (400 MHz, CDCl₃)δ: 1.23-1.29 (3H, m), 2.59 (3H, s), 2.92 (6H, s), 3.11 (1/2 x 3H, s), 3.37 (1/2 x 3H, s), 3.61 (1/2 x 2H, q, J = 7.1 Hz), 3.81 (1/2 x 2H, J = 7.1 Hz), 4.43 (2H, d, J = 5.6 Hz), 4.86-4.90 (1H, m), 7.08 (1H, d, J = 8.8 Hz), 7.18 (1H, d, J = 8.3 Hz), 7.24-7.26 (1H, m), 7.39 (1H, d, J = 8.1 Hz), 7.60-7.72 (2H, m), 8.39-8.42 (2H, m).
EI-MSm/z: 421 (M⁺).

[Example 190] 4-Methylpiperazine-1-carboxylic acid{6-[5-(ethylmethylcarbamoyl)-2-(6-methyl-3-pyridyl)-2H-pyrazol-3-yl]-3-pyridylmethyl}amide

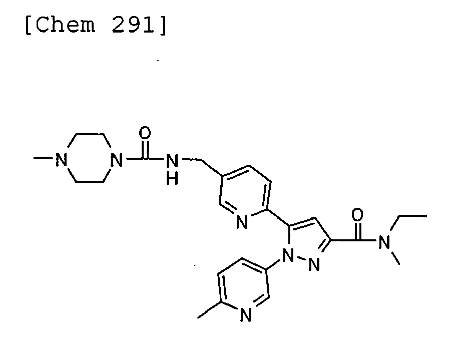

The procedure of Example 142 was repeated by using the N-ethyl-N-methyl-5-(5-aminomethyl-2-pyridyl)-1-(6-methyl-3-pyridy l)-1H-pyrazole-3-carboxamide (260mg) of Example 184 (1), 4-nitrophenyl chloroformate (164 mg), and N-methylpiperazine (89 mg) to give the title compound (159 mg, 45%) as a solid.

¹H-NMR (400 MHz, CDCl₃)δ: 1.23-1.29 (3H, m), 2.31 (3H, s), 2.39-2.42 (4H, m), 2.59 (3H, s), 3.11 (1/2 x 3H, s), 3.37 (1/2 x 3H, s), 3.41-3.43 (4H, m), 3.61 (1/2 x 2H, q, J = 7.1 Hz), 3.81 (1/2 x 2H, q, J = 7.1 Hz), 4.43 (2H, d, J = 5.6Hz), 5.03 (1H, t, J = 5.6 Hz), 7.06 (1H, d, J = 8.3 Hz), 7.18 (1H, d, J = 8.3 Hz), 7.39 (1H, d, J = 8.1 Hz), 7.61-7.70 (2H, m), 8.37-8.41 (2H, m).
EI-MSm/z: 476 (M⁺).

[Example 191] N-Ethyl-N-methyl-5-[5-(acetylaminomethyl)-2-pyridyl]-1-(6-methyl-3-pyridyl)-1H-pyrazole-3-carboxamide

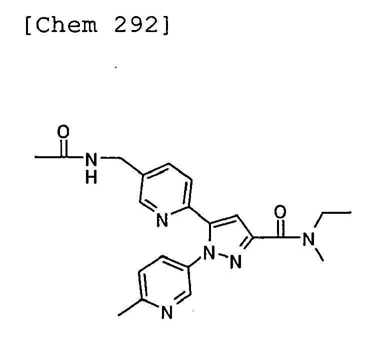

The procedure of Example 138 was repeated by using the N-ethyl-N-methyl-5-(5-aminomethyl-2-pyridyl)-1-(6-methyl-3-pyridy l)-1H-pyrazole-3-carboxamide (220 mg) of Example 184(1) and acetyl chloride (71 µl) to give the title compound (185 mg, 75%) as a solid.

¹H-NMR (400 MHz, CDCl₃)δ: 1.23-1.29 (3H, m), 2.05 (3H, s), 2.59 (3H, s), 3.11 (1/2 x 3H, s), 3.37 (1/2 x 3H, s), 3.61 (1/2 x 2H, q, J = 7.1 Hz), 3.81 (1/2 x 2H, J = 7.1 Hz), 4.44 (2H, d, J = 5.9 Hz), 6.17 (1H, br), 7.06 (1H, d, J = 8.3 Hz), 7.19 (1H, d, J = 8.3 Hz), 7.39 (1H, d, J = 7.8 Hz), 7.60-7.67 (2H, m), 8.36-8.39 (2H, m).
FAB-MSm/z: 393 (M+H)⁺.

[Example 192] N-Ethyl-N-methyl-5-[5-(cyclopentanecarbonylaminomethyl)-2-pyridyl]-1-(6-methyl-3-pyridyl)-1H-pyrazole-3-carboxamide

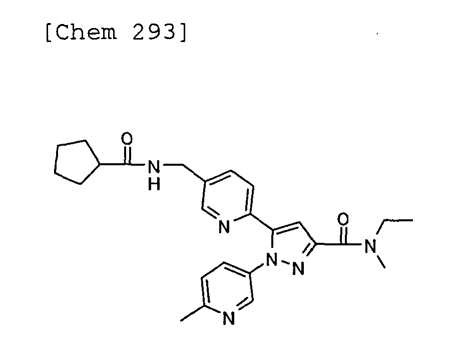

The procedure of Example 140 was repeated by using the N-ethyl-N-methyl-5-(5-aminomethyl-2-pyridyl)-1-(6-methyl-3-pyridy 1)-1H-pyrazole-3-carboxamide (220 mg) of Example 184(1) and cyclopentanecarbonyl chloride (91 µl) to give the title compound (196 mg, 70%) as a solid.

¹H-NMR (400MHz, CDCl₃)δ: 1.23-1.29 (3H, m), 1.55-1.90 (8H, m), 2.53-2.59 (1H, m), 2.59 (3H, s), 3.11 (1/2 x 3H, s), 3.37 (1/2 x 3H, s), 3.61 (1/2 x 2H, q, J = 7.1 Hz), 3.81 (1/2 x 2H, J = 7.1 Hz), 4.45 (2H, d, J = 6.1 Hz), 5.97 (1H, br), 7.08 (1H, d, J = 8.6 Hz), 7.18 (1H, d, J = 8.6 Hz), 7.40 (1H, d, J = 8.1 Hz), 7.40 (1H, d, J = 8.1 Hz), 7.60-7.66 (2H, m), 8.38 (2H, br s).
FAB-MSm/z: 447 (M+H)⁺.

[Example 193] Methyl {6-[5-(ethylmethylcarbamoyl)-2-(6-methyl-3-pyridyl)-2H-pyrazole-3-yl]-3-pyridylmethyl}carbamate

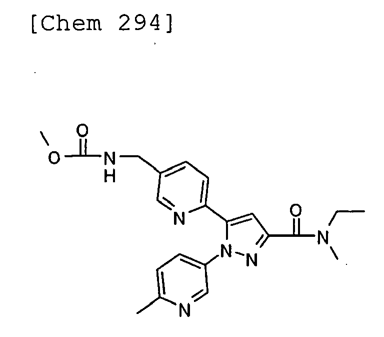

The procedure of Example 139 was repeated by using the N-ethyl-N-methyl-5-(5-aminomethyl-2-pyridyl)-1-(6-methyl-3-pyridy 1)-1H-pyrazole-3-carboxamide (220 mg) of Example 184(1) and methyl chloroformate (58 µl) to give the title compound (190 mg, 75%) as a solid.

¹H-NMR (400 MHz, CDCl₃)δ: 1.23-1.29 (3H, m), 2.59 (3H, s), 3.12 (1/2 x 3H, s), 3.38 (1/2 x 3H, s), 3.61 (1/2 x 2H, q, J = 7.1 Hz), 3.71 (3H, s), 3.81 (1/2 x 2H, J = 7.1 Hz), 4.38 (2H, d, J = 5.4 Hz), 5.19 (1H, br s), 7.10 (1H, d, J = 8.6 Hz), 7.19 (1H, d, J = 8.3 Hz), 7.42 (1H, d, J = 8.3 Hz), 7.60-7.69 (2H, m), 8.39-8.41 (2H, m).
FAB-MSm/z: 409 (M+H)⁺.

[Example 194] Morpholine-4-carboxylic acid{6-[5-(ethylmethylcarbamoyl)-2-(6-methyl-3-pyridyl)-2H-pyrazol-3-yl]-3-pyridylmethyl}amide

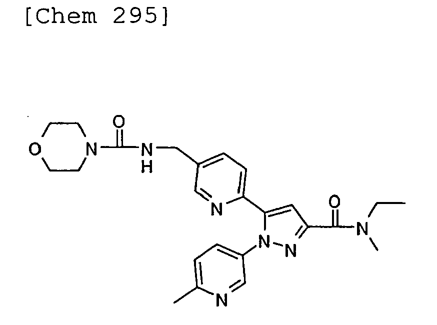

The procedure of Example 142 was repeated by using the N-ethyl-N-methyl-5-(5-aminomethyl-2-pyridyl)-1-(6-methyl-3-pyridy 1)-1H-pyrazole-3-carboxamide (260mg) of Example 184 (1), 4-nitrophenyl chloroformate (164mg), and morpholine (79mg) to give the title compound (220 mg, 65%) as a solid.

¹H-NMR (400 MHz, CDCl₃)δ: 1.25-1.29 (3H, m), 2.59 (3H, s), 3.11 (1/2 x 3H, s), 3.37 (1/2 x 3H, s), 3.37-3.40 (4H, m), 3.61 (1/2 x 2H, q, J = 7.1 Hz), 3.68-3.70 (4H, m), 3.81 (1/2 x 2H, q, J = 7.1 Hz), 4.44 (2H, d, J = 5.4 Hz), 5.10 (1H, br s), 7.00-7.07 (1H, m), 7.19 (1H, d, J = 8.1 Hz), 7.37-7.40 (1H, m), 8.62-8.68 (2H, m), 8.36 (1H, s), 8.41 (1H, s).
EI-MSm/z: 463 (M⁺).

[Example 195] 4,4-Difluoropiperidine-1-carboxylic acid{6-[5-(ethylmethylcarbamoyl)-2-(6-methyl-3-pyridyl)-2H-pyrazol-3-yl]-3-pyridylmethyl}amide

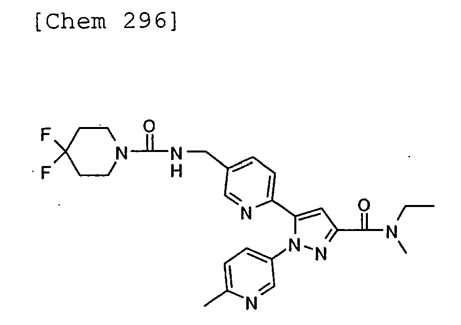

The procedure of Example 142 was repeated by using the N-ethyl-N-methyl-5-(5-aminomethyl-2-pyridyl)-1-(6-methyl-3-pyridy 1)-1H-pyrazole-3-carboxamide (260mg) of Example 184(1), 4-nitrophenyl chloroformate (164 mg), and the 4,4-difluoropiperidine hydrochloride (140 mg) of Referential Example 86 to give the title compound (258 mg, 70%) as a solid.

¹H-NMR (400 MHz, CDCl₃)δ: 1.22-1.29 (3H, m), 1.93-2.03 (4H, m), 2.59 (3H, s), 3.11 (1/2 x 3H, s), 3.37 (1/2 x 3H, s), 3.48-3.55 (4H, m), 3.61 (1/2 x 2H, q, J = 7.1 Hz), 3.81 (1/2 x 2H, q, J = 7.1 Hz), 4.42 (2H, d, J = 5.6 Hz), 5. 16 (1H, t, J = 5.6 Hz), 7.05 (1H, d, J = 7.8 Hz), 7.19 (1H, d, J = 8.1 Hz), 7.38 (1H, d, J = 8.1 Hz), 7.62-7.69 (2H, m), 8.35 (1H, s), 8.41 (1H, s).
EI-MSm/z: 497 (M⁺).

[Example 196] N-Ethyl-N-methyl-1-(6-methyl-3-pyridyl)-5-{5-[(3-methylureido)methyl]-2-pyridyl}-1H-pyrazole-3-carboxamide

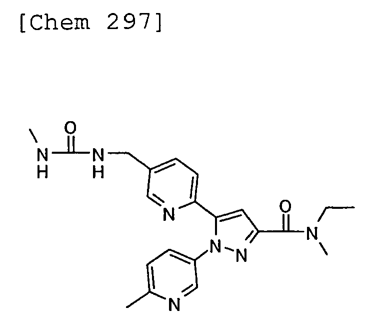

The procedure of Example 142 was repeated by using the N-ethyl-N-methyl-5-(5-aminomethyl-2-pyridyl)-1-(6-methyl-3-pyridy 1)-1H-pyrazole-3-carboxamide (340mg) ofExample184(1), 4-nitrophenyl chloroformate (215 mg), and methylamine (1.0M solution in tetrahydrofuran, 60 µl) to give the title compound (257 mg, 65%) as a solid.

¹H-NMR (400 MHz, CDCl₃)δ: 1.22-1.30 (3H, m), 2.57 (3H, s), 2.75 (3H, d, J = 4.6 Hz), 3.10 (1/2 x 3H, s), 3.37 (1/2 x 3H, s), 3.61 (1/2 x 2H, q, J = 7.1 Hz), 3.82 (1/2 x 2H, q, J = 7.1 Hz), 4.35 (2H, d, J = 5.9 Hz), 5.13 (1H, br s), 5.77 (1H, br s), 6.93 (1H, d, J = 8.8 Hz), 7.15 (1H, d, J = 8.3 Hz), 7.22 (1H, d, J = 7.8 Hz), 7.49-7.62 (2H, m), 8.35-8.37 (2H, m).
FAB-MSm/z: 408 (M⁺).

[Example 197] N,N-Diethyl-5-(5-amino-2-pyrazinyl)-1-(6-methoxy-3-pyridazinyl)-1H-pyrazole-3-carboxamide

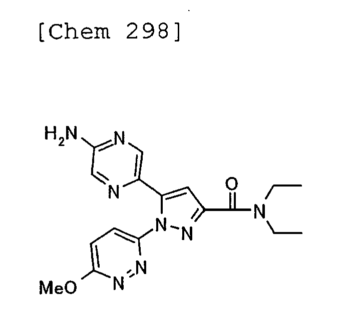

### 1) N,N-Diethyl-5-[5-(tert-butoxycarbonylamino)-2-pyrazinyl]-1-(6-methoxy-3-pyridazinyl)-1H-pyrazole-3-carboxamide

The procedure of Example 1 was repeated by using the 5-[5-(tert-butoxycarbonylamino)-2-pyrazinyl]-1-(6-methoxy-3-pyrid azinyl) -1H-pyrazole-3-carboxylicacid (0. 366 g) of Referential Example 91 and diethylamine (0.185 ml) to give N,N-diethyl-5-[5-(tert-butoxycarbonylamino)-2-pyrazinyl]-1-(6-met hoxy-3-pyridazinyl)-1H-pyrazole-3-carboxamide (0.359 g, 87%) as a solid.

¹H-NMR (400 MHz, CDCl₃)δ: 1.26-1.30 (6H, m), 1.55 (9H, s), 3.56-3.62 (2H, m), 3.74-3.80 (2H, m), 4.13 (3H, s), 7.11 (1H, s), 7.16 (1H, d, J = 9.3 Hz), 7.70 (1H, s), 7.86 (1H, d, J = 9.3 Hz), 8.49 (1H, d, J = 1.5 Hz), 9.13 (1H, d, J = 1.5 Hz). EI-MSm/z: 468 (M⁺).

### 2) The title compound

The procedure of Example 95(2) was repeated by using N,N-diethyl-5-[5-(tert-butoxycarbonylamino)-2-pyrazinyl]-1-(6-met hoxy-3-pyridazinyl)-1H-pyrazole-3-carboxamide (0.349 g) and trifluoroacetic acid (3.5 ml) to give the title compound (0.225 g, 82%) as a solid.

¹H-NMR (400MHz, CDCl₃)δ: 1.25-1.29 (6H, m), 3.55-3.60 (2H, m), 3.72-3.77 (2H, m), 4.11 (3H, s), 4.94 (2H, br s), 7.01 (1H, s), 7.15 (1H, d, J = 9.3 Hz), 7.84 (1H, d, J = 1.2 Hz), 7.86 (1H, d, J = 9.3 Hz), 8.23 (1H, d, J = 1.2 Hz).
EI-MSm/z: 368 (M⁺).

### [Test Example 1] Inhibitory effect on platelet coagulation

Human blood was collected by using 1/10 volume of 3.13% sodium citrate as an anticoagulant, and the blood was centrifuged at 180 g for 10 minutes to separate platelet rich plasma (PRP), which was the upper layer. After collecting the upper layer PRP, the remaining lower layer was centrifuged at 1600 g for 10 minutes, and platelet poor plasma (PPP) in the upper layer was collected. 1 µl solution of the compound of the Examples was added to 200 µl of the PRP, and after allowing the sample to stand at 37°C for 2 minutes, 2 µl of collagen was added- in order to induce platelet coagulation. Platelet coagulation rate was measured with PAM-12C (SSR Engineering). Light transmittance of the PPP was used as the value indicating 100% coagulation, and the coagulation rate was measured at various concentrations of the compounds of the Examples to calculate the value of IC₅₀. The results are shown in Table 1.

### [Test Example 2] Inhibitory effects on cyclooxygenase-1 (COX-1) and cyclooxygenase-2 (COX-2)

Inhibitory activity against COX-1 and COX-2 of the compounds produced in the Examples was measured by using COX Inhibitor Screening AssayKit (CatalogNos. 560101 and 560121) purchased from Cayman Chemical Company.
Before starting the measurement, reaction buffer, heme, arachidonic acid, SnCl₂, EIA buffer, washing buffer, prostaglandin (PG) screening EIA standard, PG screening acetylcholineesterase (AchE), tracer (chromogenic enzyme HRP conjugate), and PG screening EIA antiserum were prepared ready for use.
(1) Production of PGF₂α by COX-1 or COX-2
   The reaction mixture containing the compound of the Examples (50 µM) and COX-1 or COX-2 was allowed to stand at 37°C for 10 minutes, and after adding 10 µl of arachidonic acid, the solution was again allowed to stand at 37°C for 2 minutes. After the reaction, the reaction was terminated by adding 50 µl of 1N-hydrochloricacid, and 100 µl solution of SnCl₂ was added, and the reaction mixture was allowed to stand at room temperature for 5 minutes.
(2) Quantitative determination of PGF₂α by ELISA
   50 µl of antiserum (rabbit anti-PGF₂α antibody) was placed in the wells of 96 well plate that had been coated with mouse anti-rabbit IgG, and 50 µl of the 2000-fold diluted PGF₂α-containing liquid described above and 50 µl of AchE tracer were added to the well in this order, and the mixture was allowed to stand at room temperature for 18 hours. The wells were washed 5 times with the washing buffer to remove an excessive AchE tracer, and 200 µl of Ellman reagent was added. After leaving the plate in a dark room for 60 minutes, absorbance was measured at 405 nm.
(3) Calculation of inhibitory activity of the compound of the Examples
   A calibration curve was obtained by using the PG screening EIA standard, and the amount of PGF₂α produced was determined from the absorption. Inhibitory rate at 50 µM of the compound of the Examples against COX-1 or COX-2 was calculated. The results are shown in Table 1.
   It is to be noted that the inhibitory rate was calculated in relation to the amount of the PGF₂α produced by the reaction liquid free from the compounds of the Examples, which was taken 100%.

**Table 1**

| Compound (Example No.) | inhibition of collagen-induced platelet coagulation, IC₅₀ (µM) | Inhibitory effect against COX-1 at 50 µM (% inhibition) | Inhibitory effect against COX-2 at 50 µM (% inhibition) |
|---|---|---|---|
| 4 | 0.12 | -1. 5 | -1. 9 |
| 8 | 0.018 | -3. 3 | 3. 9 |
| 13 | 0.018 | 13.9 | 9. 2 |
| 15 | 0.029 | 39.5 | 6.9 |
| 22 | 0.21 | 0.8 | 4. 2 |
| 25 | 0.018 | 4.7 | 8. 2 |
| 26 | 0.065 | 5. 5 | 8.2 |
| 28 | 0.0089 | 5.4 | 7.6 |
| 40 | 0.065 | 72 | 14. 8 |
| 44 | 0.14 | 4. 3 | 3. 6 |
| 48 | 0.19 | 3. 6 | 8. 7 |
| 56 | 0.092 | -1.2 | -1.7 |
| 65 | 0.02 | 11.6 | -1.6 |
| 71 | 0.0231 | 7.9 | N.T. |
| 74 | 0.17 | 38. 8 | N.T. |
| 85 | 0.054 | N.T. | N. T. |
| 95 | 0.16 | -9. 1 | N.T. |
| 98 | 0.043 | -20. 4 | N.T. |
| 113 | 0.15 | -2. 8 | N. T. |
| 116 | 0.02 | N.T. | N. T. |
| 122 | 0.1 | N.T. | N.T. |
| 132 | 0.032 | N.T. | N. T. |
| 137 | 0.23 | - 5. 1 | N.T. |
| 138 | 0.16 | 0. 7 | N.T. |
| 139 | 0.071 | -25. 8 | N.T. |
| 152 | 0.11 | -8. 7 | N.T. |

As demonstrated in Table 1, the compound (I) of the present invention, its salt or solvate, or a solvate of such salt potently inhibited platelet coagulation without inhibiting neither COX-1 nor COX-2.

## Claims

1. A compound represented by general formula (I): 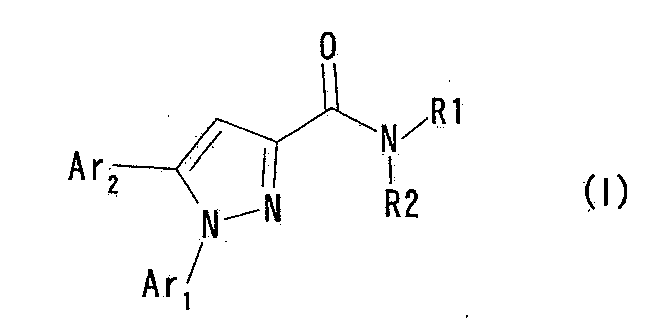 wherein Ar₁ and Ar₂ independently represent a 5- or 6-membered aromatic heterocyclic group optionally substituted with 1 to 3 substituents, or a phenyl group optionally substituted with 1 to 3 substituents, with the proviso that, when Ar₁ is a 5- or 6-membered aromatic heterocyclic group substituted with 1 to 3 substituents, Ar₂ is a 5- or 6-membered aromatic heterocyclic group optionally substituted with 1 to 3 substituents, and when Ar₁ is an unsubstituted 5- or 6-membered aromatic heterocyclic group, Ar₂ is a 5- or 6-membered aromatic heterocyclic group substituted with 1 to 3 substituents or a phenyl group substituted with a carbamoyl group having 1 or 2 substituents or with a lower alkyl group having 1 or 2 substituents, and when Ar₁ is a phenyl group optionally substituted with 1 to 3 substituents, Ar₂ is a 5- or 6-membered aromatic heterocyclic group substituted with 1 to 3 substituents;
R1 represents a lower acyl group, a lower alkoxycarbonyl group, a lower alkoxy group, a lower alkyl group optionally substituted with 1 or 2 substituents, a carbamoyl group optionally substituted with 1 or 2 substituents, an oxamoyl group optionally substituted with 1 or 2 substituents, an amino group optionally substituted with 1 or 2 substituents, a 4-to 7-membered alicyclic heterocyclic group optionally substituted with 1 or 2 substituents, a phenyl group optionally substituted with 1 to 3 substituents, or a 5- or 6-membered aromatic heterocyclic group optionally substituted with 1 to 3 substituents; and
R2 represents a hydrogen atom or a lower alkyl group optionally substituted with 1 or 2 substituents; or a salt thereof, or a solvate thereof.

2. A compound, a salt thereof, or a solvate thereof according to claim 1, wherein Ar₁ is a 5- or 6-membered aromatic heterocyclic group substituted with 1 to 3 substituents, and Ar₂ is a 5- or 6-membered aromatic heterocyclic group optionally substituted with 1 to 3 substituents.

3. A compound, a salt thereof, or a solvate thereof according to claim 1, wherein Ar₁ is an unsubstituted 5- or 6-membered aromatic heterocyclic group, and Ar₂ is an aromatic heterocyclic group substituted with 1 to 3 substituents.

4. A compound, a salt thereof, or a solvate thereof according to claim 1, wherein Ar₁ is an unsubstituted 5- or 6-membered aromatic heterocyclic group, and Ar₂ is a phenyl group substituted with a carbamoyl group having 1 or 2 substituents or with a lower alkyl group having 1 or 2 substituents.

5. A compound, a salt thereof, or a solvate thereof according to claim 1, wherein Ar₁ is a phenyl group optionally substituted with 1 to 3 substituents, and Ar₂ is a 5- or 6-membered aromatic heterocyclic group substituted with 1 to 3 substituents.

6. A drug comprising a compound, a salt thereof, or a solvate thereof according to any one of claims 1 to 5.

7. A prophylactic and/or therapeutic composition for an ischemic disease comprising a compound, a salt thereof, or a solvate thereof according to any one of claims 1 to 5.

8. Use of a compound, a salt thereof, or a solvate thereof according to any one of claims 1 to 5 for producing a drug.

9. A method for preventing and/or treating an ischemic disease which comprises administering to a patient an effective amount of a compound, a salt thereof, or a solvate thereof according to any one of claims 1 to 5.
